(19) Europäisches Patentamt
European Patent Office
Office européen des brevets

(11) **EP 4 559 917 A1**

(12) **EUROPEAN PATENT APPLICATION**
published in accordance with Art. 153(4) EPC


(43) Date of publication:
**28.05.2025 Bulletin 2025/22**

(21) Application number: **23842446.9**

(22) Date of filing: **21.07.2023**

(51) International Patent Classification (IPC):
***C07D 487/04*** *(2006.01)* ***C07D 209/00*** *(2006.01)*
***C07D 235/00*** *(2006.01)* ***A61K 31/404*** *(2006.01)*
***A61K 31/454*** *(2006.01)* ***A61P 35/00*** *(2006.01)*

(52) Cooperative Patent Classification (CPC):
**A61K 31/404; A61K 31/454; A61P 35/00; C07D 209/00; C07D 235/00; C07D 487/04**

(86) International application number:
**PCT/CN2023/108762**

(87) International publication number:
**WO 2024/017384 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.07.2022 CN 202210866557**
**06.03.2023 CN 202310257457**



(71) Applicant: **Shenzhen Zhongge Biological Technology Co., Ltd.**
**Shenzhen, Guangdong 518000 (CN)**

(72) Inventors:
- **MOU, Jianfeng**
**Shenzhen, Guangdong 518000 (CN)**
- **NIU, Chunyi**
**Shenzhen, Guangdong 518000 (CN)**
- **FANG, Xiaoniu**
**Shenzhen, Guangdong 518000 (CN)**
- **WAN, Xiao**
**Shenzhen, Guangdong 518000 (CN)**
- **DONG, Guangping**
**Shenzhen, Guangdong 518000 (CN)**
- **YU, Qingfang**
**Shenzhen, Guangdong 518000 (CN)**
- **WANG, Shaohui**
**Shenzhen, Guangdong 518000 (CN)**
- **CHEN, Bin**
**Shenzhen, Guangdong 518000 (CN)**
- **ZHANG, Peiyu**
**Shenzhen, Guangdong 518000 (CN)**

(74) Representative: **Meissner Bolte Partnerschaft mbB**
**Patentanwälte Rechtsanwälte**
**Postfach 86 06 24**
**81633 München (DE)**


(54) **COMPOUND FOR RECOVERING P53 MUTATION FUNCTION AND USE THEREOF**


(57) Provided are a compound for recoverying a p53 mutation function and use thereof, particularly relating to a compound represented by Formula M, or an enantiomer, a diastereomer, a racemate, a tautomer, a stereoisomer, a geometric isomer, a crystal form, a nitrogen oxide, a metabolite or pharmaceutically acceptable salt, ester, solvate, hydrate, isotope-labeled compound or prodrug of the compound.

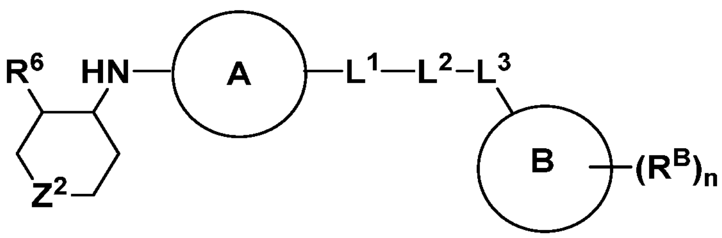

Formula M


EP 4 559 917 A1

**Description**

The present application claims the benefit of priority to Chinese patent application CN202210866557.8 filed on July 22, 2022 and Chinese patent application CN202310257457.X filed on March 6, 2023, the entire contents of which are incorporated by reference herein.

## Technical Field

The present invention relates to the field of medicine, and specifically to a compound for recovering p53 mutation function and uses thereof.

## Background Art

p53 is an important tumor suppressor gene, which was first reported in 1979. When it was first discovered, it was generally believed that the P53 gene was an oncogene. With the continuous deepening of research, the function of p53 as a tumor suppressor gene was gradually revealed. p53 is called the guardian of the genome. It plays a key role in cell function and can maintain the stability of the genome during cell stress.

p53 encodes p53 protein, which is a sequence-specific transcription factor that can regulate and participate in gene expression in many cellular processes. The functions of these target genes mainly include inducing cell cycle arrest, DNA repair, regulating cell metabolism, cell senescence, cell apoptosis, and the recently discovered induction of cell ferroptosis.

p53 has a mutation frequency far ahead of any other protein in human cancer, and mutations exist in 50% of cancer patients. p53 mutations are most common in ovarian (47.8%), colon (43.2%), esophageal (43.1%), head and neck (40.6%) and laryngeal (40.4%) tumors, while the p53 mutation frequency is lowest in cervical (5.8%), hematopoietic (12.7%) and endocrine (14.6%) tumors.

p53 is considered one of the most difficult undruggable targets, and there is currently no effective therapy targeting p53 in the world. The difficulties in the development of p53-targeted therapy are: 1) unlike traditional inhibitors developed, drugs targeting p53 need to recover rather than inhibit the normal activity of the protein; 2) the vast number of p53 mutants that lose tumor suppression activity; 3) p53 is not an enzyme, so it has not evolved a hydrophobic pocket for binding with a small molecule ligand.

The tyrosine at position 220 of the p53 Y220C mutant is replaced by cysteine, and this mutation creates a crevice in the protein, making the mutant protein unable to maintain the conformation and function of wild-type p53.

Therefore, compounds that can recover the normal function of the p53 Y220C mutant need further investigation.

## Contents of the Invention

The inventors of the present application have obtained new compounds that can recover the normal function of the p53 Y220C mutant through in-depth research and creative discovery. These compounds recover the normal function of the p53 Y220C mutant by targeting the small crevice on the protein surface caused by the p53 Y220C mutation, thereby exerting a tumor suppressor effect. These compounds can be used to treat a variety of diseases caused by the p53 Y220C mutation (e.g., gastric adenocarcinoma, pancreatic cancer, etc.).

To this end, in the first aspect of the present invention, the present invention provides a compound represented by Formula M, or an enantiomer, diastereomer, racemate, tautomer, stereoisomer, geometric isomer, crystal form, N-oxide, metabolite of the compound, or a pharmaceutically acceptable salt, ester, solvate, hydrate, isotope-labeled (preferably deuterated) compound or prodrug thereof,

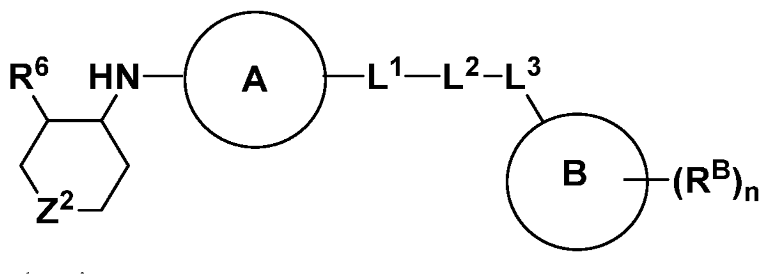

Formula M

wherein:

Ring A is selected from

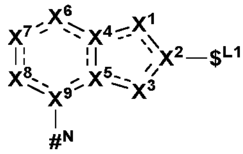, 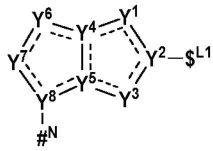,

**wherein** $\#^N$ represents a connection point between Ring A and NH, $\$^{L1}$ is a connection point between Ring A and $L^1$; each ------ is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C(=O), N, NH, and S;
$Y^1$, $Y^2$, $Y^3$, $Y^4$, $Y^5$, $Y^6$, $Y^7$, $Y^8$ are each independently selected from the group consisting of S, N, C, CH, $CR^d$, and $NR^d$;
$R^a$, $R^d$ are each independently selected from the group consisting of deuterium, C1-C6 alkyl, C3-C7 cycloalkyl, 4- to 8-membered heterocyclyl, and C5-C6 heteroaryl; wherein the C1-C6 alkyl, C3-C7 cycloalkyl, 4- to 8-membered heterocyclyl, C5-C6 heteroaryl are each independently substituted with deuterium, halogen, hydroxyl, amino, cyano, or C3-C7 cycloalkyl;
each $R^b$ is independently selected from the group consisting of hydrogen, deuterium, hydroxyl, halogen, and cyano;
each $R^c$ is independently selected from the group consisting of hydrogen, deuterium, hydroxyl, halogen, and cyano;
$L^1$ is selected from the group consisting of

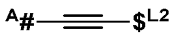,

and 5- to 6-membered heteroarylene; wherein, $\#^A$ represents a connection point between $L^1$ and Ring A, $\$^{L2}$ represents a connection point between $L^1$ and $L^2$;
$L^2$ is

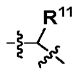;

$L^3$ is selected from the group consisting of NH, -NHC(=O)-, and -NHC(=O)NH-;
Ring B is selected from the group consisting of phenyl, and 5- to 10-membered heteroaryl;
each $R^B$ is independently selected from the group consisting of hydrogen, deuterium, halogen, cyano, amino, C1-C6 alkyl, $R^3O$-, $R^eR^fN$-C(=O)-, $R^gO$-C(=O)-, $R^hS(=O)_2$-, $R^iR^jN$-$S(=O)_2$-,

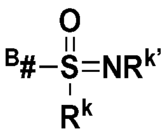, 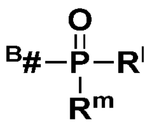,

5- to 6-membered heteroaryl, 4- to 8-membered saturated heterocyclyl, and C3-C7 cycloalkyl; wherein the C1-C6 alkyl, 5- to 6-membered heteroaryl, 4- to 8-membered saturated heterocyclyl, C3-C7 cycloalkyl are optionally substituted with 0, 1, 2, 3 substituents selected from the group consisting of deuterium, halogen, hydroxyl, amino, cyano, C1-C6 alkoxy, and phenyl; $\#^B$ represents a connection point between

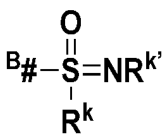

or

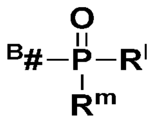

and Ring B;

n is selected from the group consisting of 0, 1, 2, and 3;

each $R^3$ is independently selected from the group consisting of hydrogen, deuterium, C1-C6 alkyl, and C1-C6 alkyl-C(=O)-; wherein the C1-C6 alkyl is optionally substituted with halogen, hydroxyl, cyano, amino, or C1-C6 alkoxy;

$R^e$, $R^f$ are each independently selected from the group consisting of hydrogen, deuterium, and C1-C6 alkyl; wherein the C1-C6 alkyl is optionally substituted with halogen, hydroxyl, cyano, amino, C1-C6 alkyl, C1-C6 alkoxy, or 4- to 8-membered heterocyclyl; or, $R^e$, $R^f$ together with the N atom to which they connect form a 4- to 8-membered saturated heterocyclic ring or a 7- to 11-membered spiro-heterocyclic ring, wherein the 4- to 8-membered saturated heterocyclic ring or 7- to 11-membered spiro-heterocyclic ring is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

each $R^g$ is selected from the group consisting of hydrogen, deuterium, C1-C6 alkyl, and C3-C7 cycloalkyl; wherein the C1-C6 alkyl, C3-C7 cycloalkyl are optionally substituted with deuterium, halogen, hydroxyl, amino, or cyano;

each $R^h$ is selected from the group consisting of hydrogen, deuterium, amino, C1-C6 alkyl, C3-C7 cycloalkyl, and 4- to 8-membered saturated heterocyclyl; or $R^h$ is connected to the ortho position of $R^hS(=O)_2$- to form a 5- to 6-membered heterocyclic ring;

$R^i$, $R^j$ are each independently selected from the group consisting of hydrogen, deuterium, C1-C6 alkyl, C1-C6 haloalkyl, C3-C7 cycloalkyl, C3-C7 halocycloalkyl, and 5- to 6-membered heteroaryl; or, $R^i$, $R^j$ together with the N atom to which they connect form a 4- to 8-membered saturated heterocyclic ring or a 7- to 11-membered spiro-heterocyclic ring;

$R^k$ is selected from the group consisting of hydrogen, deuterium, C1-C6 alkyl, cyano, C3-C7 cycloalkyl, and 4- to 8-membered heterocyclyl; $R^{k'}$ is selected from the group consisting of hydrogen, deuterium, C1-C6 alkyl, cyano, and C3-C7 cycloalkyl; or, $R^k$, $R^{k'}$ together with -S=N-to which they connect form a 4- to 8-membered heterocyclic ring; or $R^k$ is connected to the ortho position of

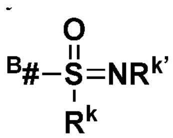

to form a 5- to 6-membered heterocyclic ring;

$R^l$, $R^m$ are each independently selected from the group consisting of hydrogen, deuterium, C1-C6 alkyl, and C3-C7 cycloalkyl; or, $R^l$, $R^m$ together with the P atom to which they connect form a 4- to 8-membered saturated heterocyclic ring; or one of $R^l$, $R^m$ is connected to the ortho position of

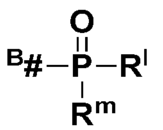

to form a 5- to 6-membered heterocyclic ring;

$R^6$ is selected from the group consisting of hydrogen, deuterium, hydroxyl, halogen, and cyano;

$R^{11}$ is selected from the group consisting of hydrogen, deuterium, and C1-C6 alkyl;

$Z^2$ is selected from the group consisting of $N(R^7)$, $C(R^8)_2$, O, and $S(O)_2$;

$R^7$ is selected from the group consisting of hydrogen, deuterium, and C1-C6 alkyl; wherein the C1-C6 alkyl is optionally substituted with halogen, hydroxyl, amino, cyano, C1-C6 alkoxy, halogen-substituted C1-C6 alkoxy, hydroxyl-substituted C1-C6 alkoxy, or amino-substituted C1-C6 alkoxy;

each $R^8$ is independently selected from the group consisting of hydrogen, deuterium, halogen, hydroxyl, amino, cyano, C1-C6 alkyl, 4- to 8-membered saturated heterocyclyl, 7- to 11-membered spiro-heterocyclyl, 5- to 10-membered bridged heterocyclyl, and $R^oR^pN$-; wherein the C1-C6 alkyl, 4- to 8-membered saturated heterocyclyl, 7- to 11-membered spiro-heterocyclyl, 5- to 10-membered bridged heterocyclyl are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or

amino-substituted C1-C6 alkoxy; provided that: at most one $R^8$ is selected from the group consisting of 4- to 8-membered saturated heterocyclyl, 7- to 11-membered spiro-heterocyclyl, and 5- to 10-membered bridged heterocyclyl; or two $R^8$ groups together with the carbon atom to which they connect form a 4- to 8-membered heterocyclyl; wherein the 4- to 8-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy; $R^o$, $R^p$ are each independently selected from the group consisting of hydrogen, deuterium, C1-C6 alkyl, and 4- to 8-membered saturated heterocyclyl; wherein the C1-C6 alkyl, 4- to 8-membered saturated heterocyclyl are optionally substituted with halogen, hydroxyl, amino, cyano, or C1-C6 alkoxy; or, $R^o$ and $R^p$ together with the N atom to which they connect form a 4-to 8-membered saturated heterocyclic ring, a 7- to 11-membered spiro-heterocyclic ring or a 5- to 10-membered bridged heterocyclic ring; wherein the 4- to 8-membered heterocyclic ring, 7- to 11-membered spiro-heterocyclic ring, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

the compound of Formula M must simultaneously meet conditions 1) and 2):

1) when Ring A is not

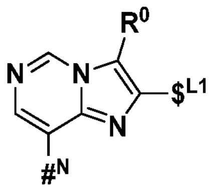 , 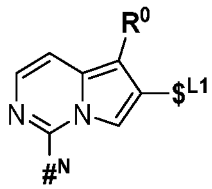 , 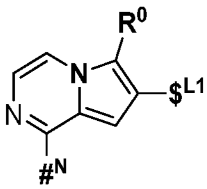 ,

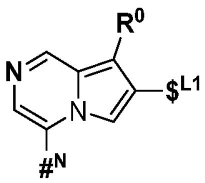 , 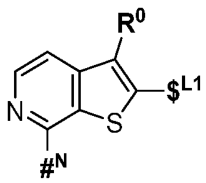 , 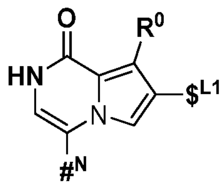 , 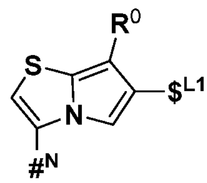 , 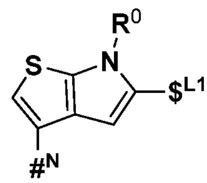 ,

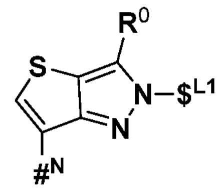 , 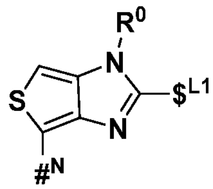 , 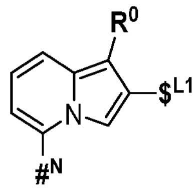 ,

$R^0$ is selected from the group consisting of C1-C6 alkyl, C3-C7 cycloalkyl, 4- to 8-membered heterocyclyl, and C5-C6 heteroaryl, wherein the C1-C6 alkyl, C3-C7 cycloalkyl, 4- to 8-membered heterocyclyl, C5-C6 heteroaryl are each independently optionally substituted with deuterium, halogen, hydroxyl, amino, or cyano, and at least one of the following conditions must be met:

a) $L^1$ is 5- to 6-membered heteroarylene;

b) $L^3$ is selected from the group consisting of -NHC(=O)-, and -NHC(=O)NH-;

c) Ring B is substituted with $R^hS(=O)_2$-, and $R^h$ is connected to the ortho position of $R^hS(=O)_2$- to form a 5- to 6-membered heterocyclic ring; or

Ring B is substituted with

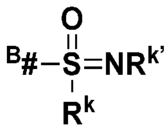

,

and $R^k$, $R^{k'}$ together with -S=N- to which they connect form a 4- to 8-membered heterocyclic ring; or

Ring B is substituted with

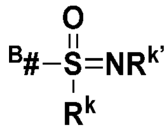

,

ring; or and $R^k$ is connected to the ortho position of

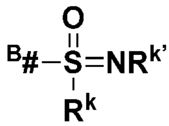

to form a 5- to 6-membered heterocyclic ring; or

Ring B is substituted with

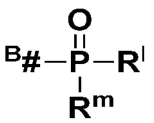

,

and $R^l$, $R^m$ together with the P atom to which they connect form a 4- to 8-membered saturated heterocyclic ring, or one of $R^l$, $R^m$ is connected to the ortho position of

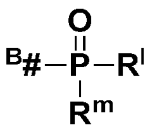

to form a 5- to 6-membered heterocyclic ring;

d) $Z^2$ is $C(R^8)_2$, and only one $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they connect form a 4- to 8-membered saturated heterocyclic ring, a 7-to 11-membered spiro-heterocyclic ring or a 5- to 10-membered bridged heterocyclic ring; wherein the 7- to 11-membered spiro-heterocyclyl, 7- to 11-membered spiro-heterocyclic ring, 5-to 10-membered bridged heterocyclyl, 5- to 10-membered bridged heterocyclic ring, 4- to 8-membered saturated heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

2) the compound represented by Formula M does not include the following compounds:

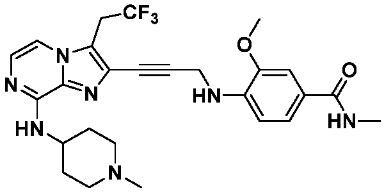
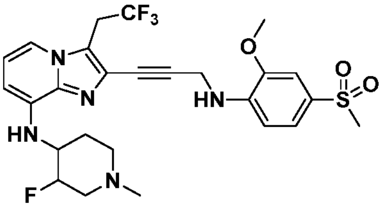
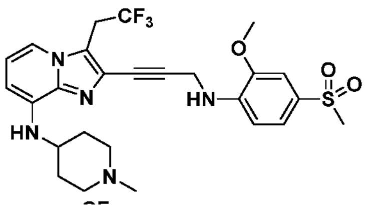

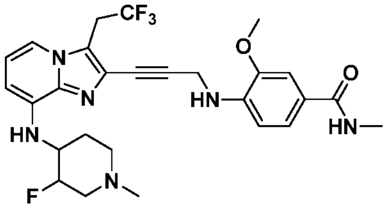
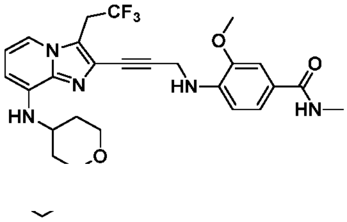
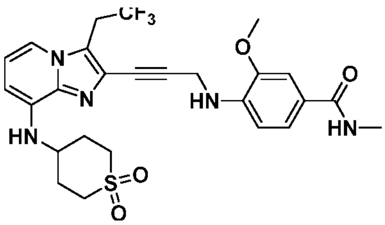

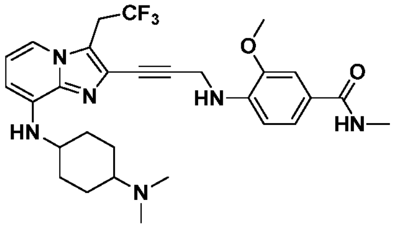
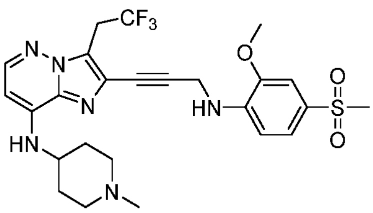
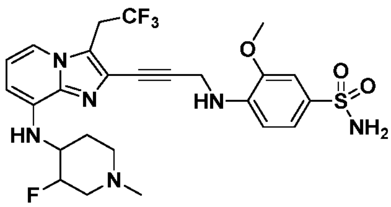

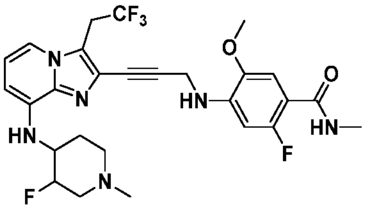
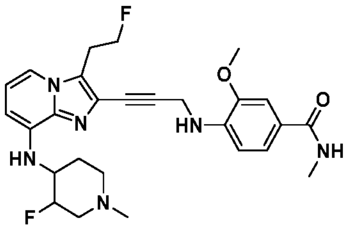
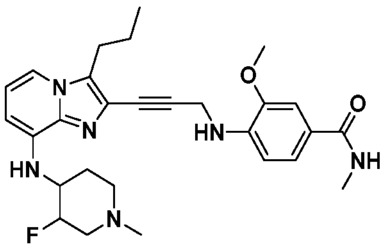

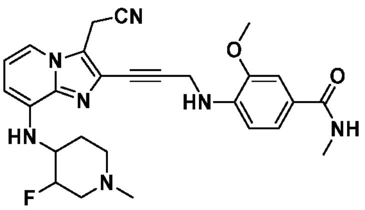
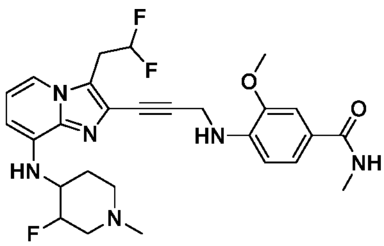

.

In some embodiments, the condition 2) is that: the compound represented by Formula M does not include the compounds specifically listed in the aforementioned condition 2), and their enantiomers, diastereomers, racemates, tautomers, stereoisomers, geometric isomers, for example:

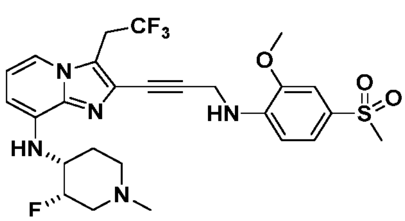
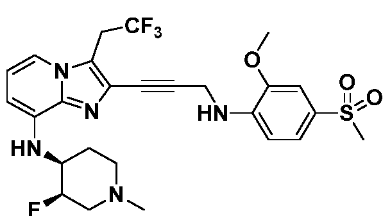
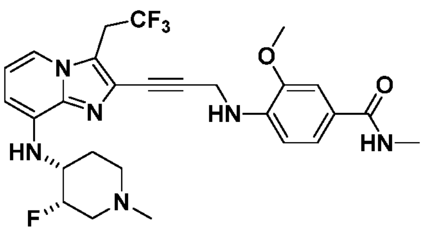

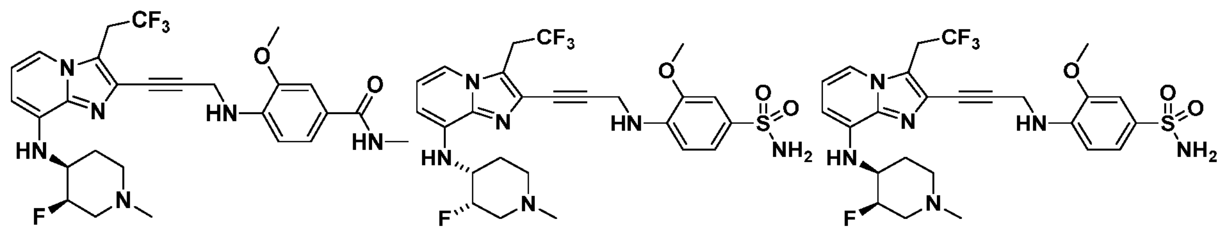

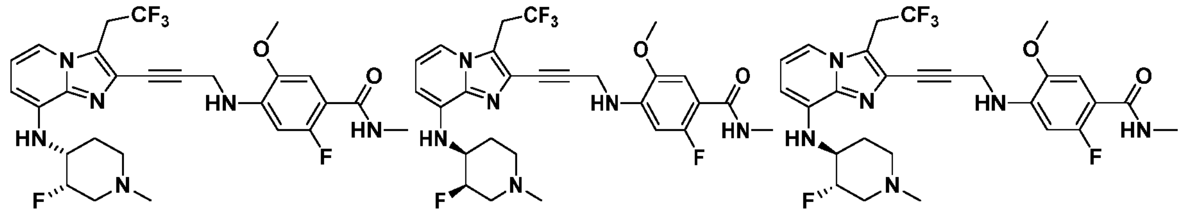

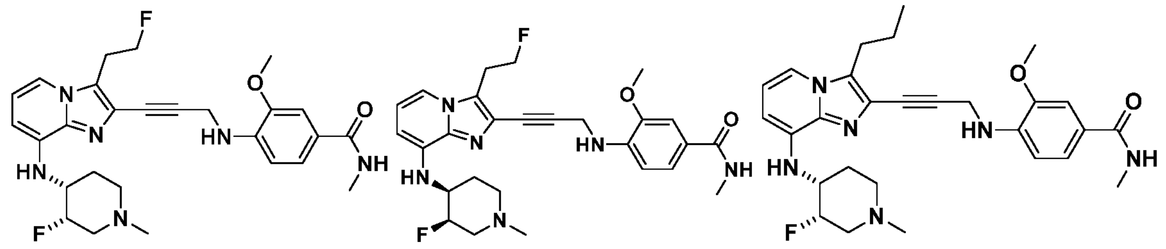

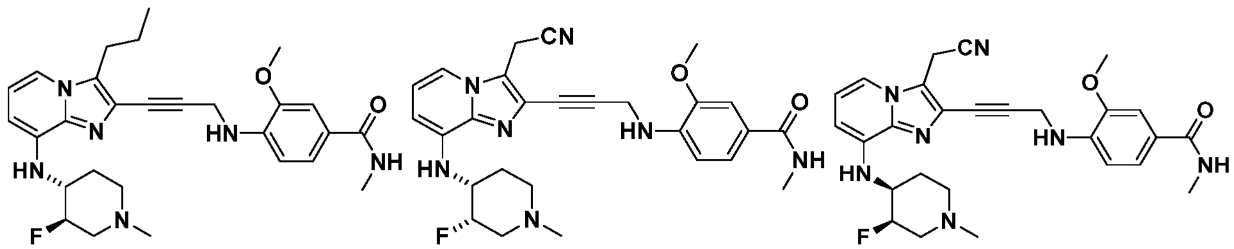

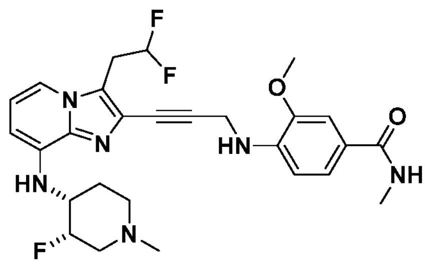 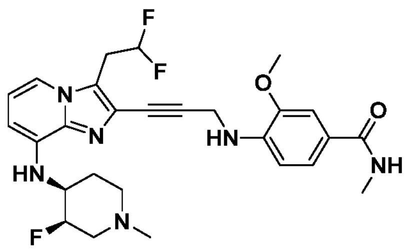.

In some embodiments, Ring A is selected from the group consisting of

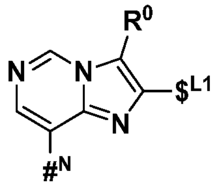,

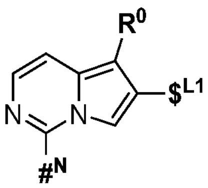, 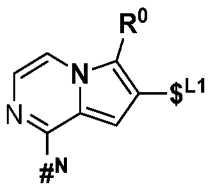, 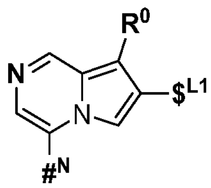, 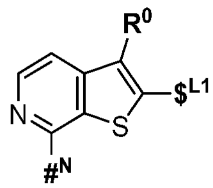, 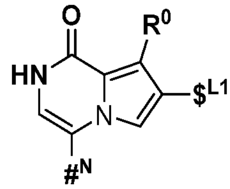,

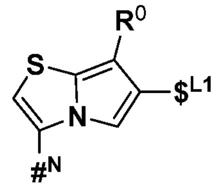, 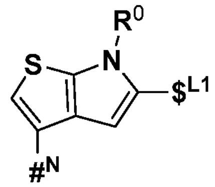, 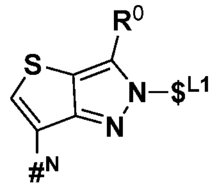, 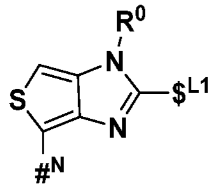, 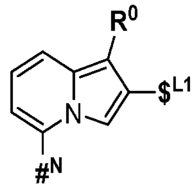,

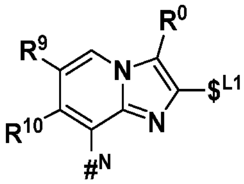, 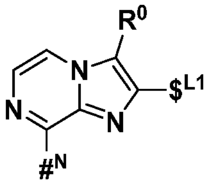, 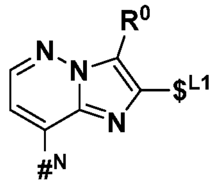, 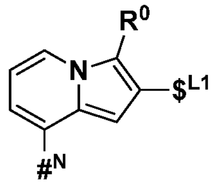, and 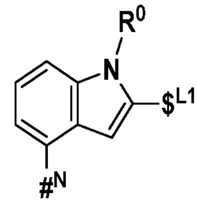;

$R^0$, $R^9$, $R^{10}$ have the definitions described herein.

Preferably, $R^0$ is selected from the group consisting of C1-C6 alkyl, C1-C6 haloalkyl, cyano-C1-C6 alkylene-, C3-C6 cycloalkyl, and C3-C6 halocycloalkyl; $R^9$ and $R^{10}$ are each independently selected from the group consisting of hydrogen, deuterium, hydroxyl, amino, halogen, cyano, and C1-C3 alkyl.

More preferably, $R^0$ is selected from the group consisting of C1-C4 alkyl, C1-C2 haloalkyl, and cyano-C1-C2 alkylene-; $R^9$ is selected from the group consisting of hydrogen, and hydroxyl; $R^{10}$ is selected from the group consisting of hydrogen, and fluorine.

Most preferably, $R^0$ is selected from the group consisting of $CF_3CH_2$-, $CH_2FCH_2$-, $CH_3CH_2CH_2$-, $CNCH_2$-, and $CHF_2CH_2$-.

In some embodiments, Ring A is selected from the group consisting of

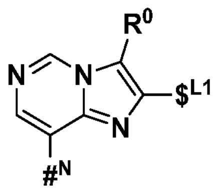,

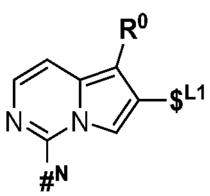, 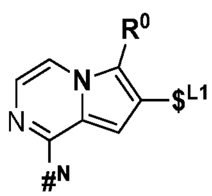, 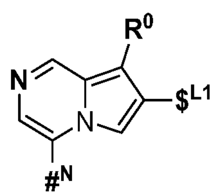, 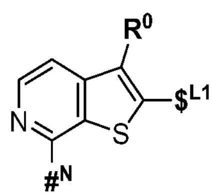, 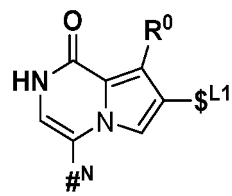,

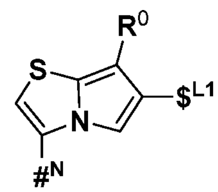, 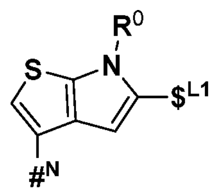, 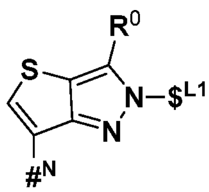, 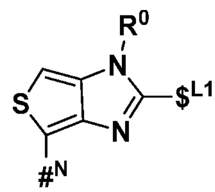, and 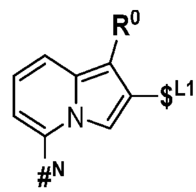; $R^0$ has the definitions described herein.

Preferably, $R^0$ is selected from the group consisting of C1-C6 alkyl, C1-C6 haloalkyl, cyano-C1-C6 alkylene-, C3-C6 cycloalkyl, and C3-C6 halocycloalkyl.

More preferably, $R^0$ is selected from the group consisting of C1-C4 alkyl, C1-C2 haloalkyl, and cyano-C1-C2 alkylene-.

Most preferably, $R^0$ is selected from the group consisting of $CF_3CH_2$-, $CH_2FCH_2$-, $CH_3CH_2CH_2$-, $CNCH_2$-, and $CHF_2CH_2$-.

In some embodiments, Ring A is selected from the group consisting of

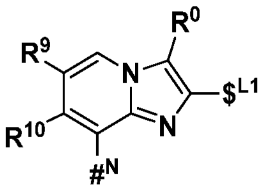
,
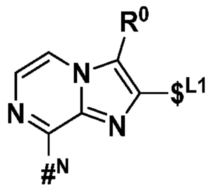
,
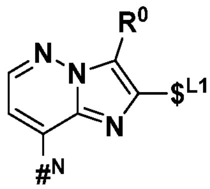
,
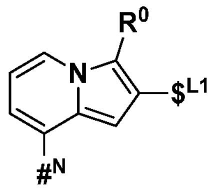
,

and

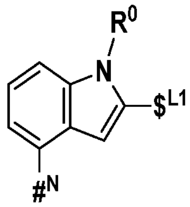
;

$R^0$, $R^9$, $R^{10}$ have the definitions described herein.

Preferably, $R^9$ and $R^{10}$ are each independently selected from the group consisting of hydrogen, deuterium, hydroxyl, amino, halogen, cyano, and C1-C3 alkyl.

More preferably, $R^9$ and $R^{10}$ are each independently selected from the group consisting of hydrogen, hydroxyl, and halogen.

Preferably, Ring A is selected from the group consisting of

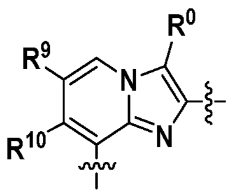
,

and

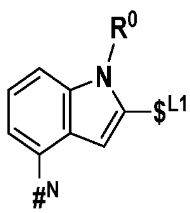
;

$R^0$ is selected from the group consisting of C1-C6 alkyl, C1-C6 haloalkyl, cyano-C1-C6 alkylene-, C3-C6 cycloalkyl, and C3-C6 halocycloalkyl; $R^9$ is selected from the group consisting of hydrogen, and hydroxyl; $R^{10}$ is selected from the group consisting of hydrogen, and fluorine.

More preferably, $R^0$ is selected from the group consisting of C1-C4 alkyl, C1-C2 haloalkyl, and cyano-C1-C2 alkylene-.

Most preferably, $R^0$ is selected from the group consisting of $CF_3CH_2$-, $CH_2FCH_2$-, $CH_3CH_2CH_2$-, $CNCH_2$-, and $CHF_2CH_2$-; $R^9$ and $R^{10}$ are hydrogen.

In some embodiments, Ring A is selected from the group consisting of

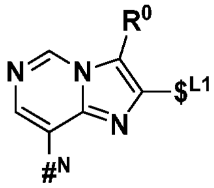,

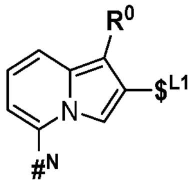, 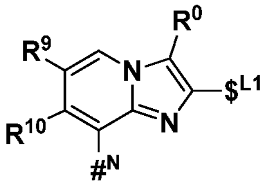, and 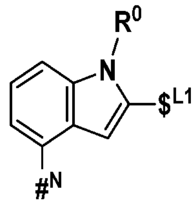;

$R^0$, $R^9$, $R^{10}$ have the definitions described herein.

Preferably, $R^0$ is selected from the group consisting of C1-C4 alkyl, C1-C2 haloalkyl, and cyano-C1-C2 alkylene-; $R^9$ and $R^{10}$ are each independently selected from the group consisting of hydrogen, deuterium, hydroxyl, amino, halogen, cyano, and C1-C3 alkyl.

More preferably, $R^0$ is selected from the group consisting of $CF_3CH_2$-, $CH_2FCH_2$-, $CH_3CH_2CH_2$-, $CNCH_2$-, and $CHF_2CH_2$-; $R^9$ and $R^{10}$ are hydrogen.

In some embodiments $L^1$ is selected from the group consisting of

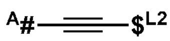,

and 5-membered heteroarylene.

Preferably, $L^1$ is selected from the group consisting of

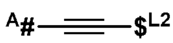,

1,3,4-thiadiazolylene, 1,2,4-oxadiazolylene, 1,3,4-oxadiazolylene, and thiazolylene.

More preferably, $L^1$ is selected from the group consisting of

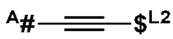, 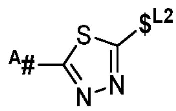,

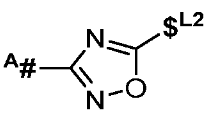, 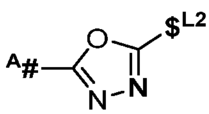, 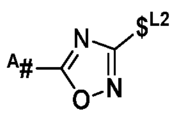, 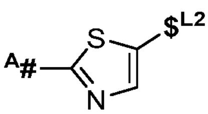, 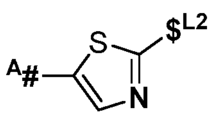,

and

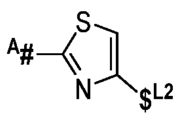.

Further preferably, $L^1$ is

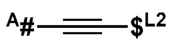.

Further preferably, $L^1$ is selected from the group consisting of

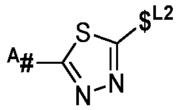

,

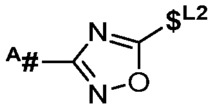 , 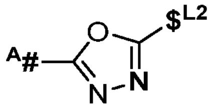 , 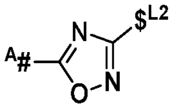 , 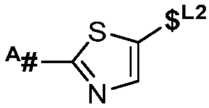 , 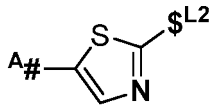

and

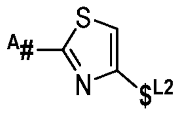

.

In some embodiments, $R^{11}$ is selected from the group consisting of hydrogen, deuterium, and C1-C4 alkyl.

Preferably, $R^{11}$ is selected from the group consisting of hydrogen and methyl.

In some embodiments, $L^3$ is selected from the group consisting of NH, $\#^{L2}$-NHC(=O)-$\$^B$, and $\#^{L2}$-NHC(=O)NH-$\$^B$, wherein $\#^{L2}$ represents a connection point between $L^3$ and $L^2$, and $\$^B$ represents a connection point between $L^3$ and Ring B.

Preferably, $L^3$ is NH.

In some embodiments, $R^3O$-, $R^eR^fN$-C(=O)-, $R^gO$-C(=O)-, $R^hS(=O)_2$-, $R^iR^jN$-$S(=O)_2$-,

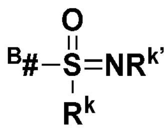 , 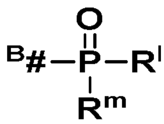 ,

5- to 6-membered heteroaryl, 4- to 8-membered saturated heterocyclyl, C3-C7 cycloalkyl as optional substituents $R^B$ on Ring B can only be selected once at most; that is, when there are multiple substituents $R^B$ on Ring B, $R^3O$-, $R^eR^fN$-C(=O)-, $R^gO$-C(=O)-, $R^hS(=O)_2$-, $R^iR^jN$-$S(=O)_2$-,

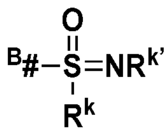 , 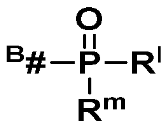 ,

5- to 6-membered heteroaryl, 4- to 8-membered saturated heterocyclyl, C3-C7 cycloalkyl as optional substituents can not be selected twice or more times independently.

In some embodiments Ring B is selected from the group consisting of phenyl, pyridyl, 5-membered heteroaromatic ring, and

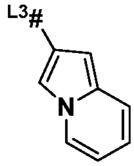

;

**and** $\#^{L3}$ represents a connection point between Ring B and $L^3$.

Preferably, Ring B is selected from the group consisting of phenyl, pyridyl, thienyl, pyrrolyl, thiazolyl, pyrazolyl, imidazolyl, and furanyl.

More preferably, Ring B is selected from the group consisting of phenyl and pyridyl.

More preferably, Ring B is selected from the group consisting of thienyl, pyrrolyl, thiazolyl, pyrazolyl, imidazolyl,

and furanyl.

Most preferably, Ring B is phenyl.

In some embodiments, Ring B is selected from the group consisting of phenyl, and

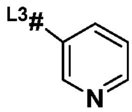
;

n is 2 or 3, and each $R^B$ is independently selected from the group consisting of halogen, $R^3O$-,$R^hS(=O)_2$-,

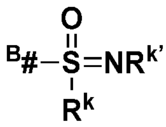
, and
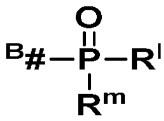
,

and each $R^B$ is different.

Preferably, $R^3$ is selected from the group consisting of hydrogen, C1-C4 alkyl, C1-C4 haloalkyl, cyano-C1-C2 alkylene-, hydroxy-C1-C2 alkylene-, C1-C2 alkoxy-C1-C2 alkylene-, and C1-C2 alkyl-C(=O)-.

Preferably, $R^h$ is connected to the ortho position of $R^hS(=O)_2$- to form a 5- to 6-membered heterocyclic ring;

$R^{k'}$ is hydrogen, and $R^k$ is connected to the ortho position of

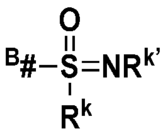

to form a 5- to 6-membered heterocyclic ring;

$R^l$ is C1-C6 alkyl (preferably C1-C3 alkyl), and $R^m$ is connected to the ortho position of

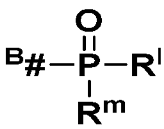

to form a 5- to 6-membered heterocyclic ring.

Preferably, $R^3O$-, $R^hS(=O)_2$-,

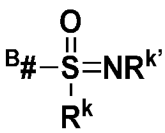
,
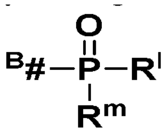

as optional substituents $R^B$ on Ring B can be selected at most once.

Preferably, Ring B is selected from the group consisting of phenyl and

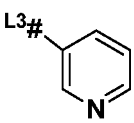
,

n is 2, one $R^B$ is $R^3O$-, and the other $R^B$ is selected from the group consisting of $R^hS(=O)_2$-,

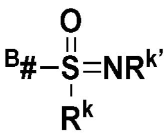, and 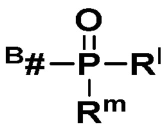.

Further preferably, Ring B is phenyl, n is 2, one $R^B$ is $R^3O$-, and the other $R^B$ is selected from the group consisting of $R^hS(=O)_2$-,

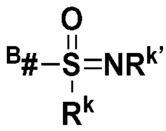, and 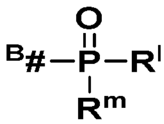.

More preferably, $R^3$ is selected from the group consisting of hydrogen, methyl, ethyl, trifluoromethyl, difluoromethyl, monofluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-.

Most preferably, $R^3$ is methyl.

Preferably, Ring B is selected from the group consisting of phenyl and

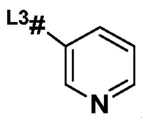,

n is 3 one $R^B$ is hydrogen or fluorine, one $R^B$ is $R^3O$-, and one $R^B$ is selected from the group consisting of $R^hS(=O)_2$-,

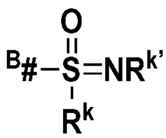, and 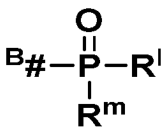.

Further preferably, $R^3$ is selected from the group consisting of hydrogen, methyl, ethyl, trifluoromethyl, difluoromethyl, monofluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-.

Most preferably, $R^3$ is methyl.

In some embodiments,

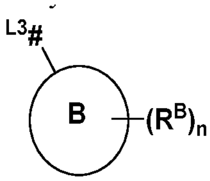

is selected from the group consisting of

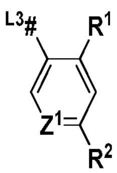,

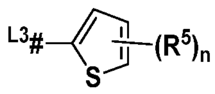, 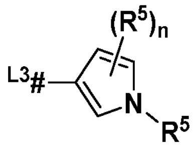, 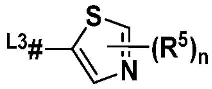, 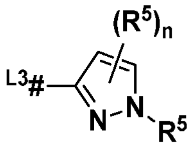, 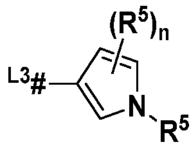,

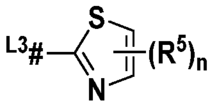, 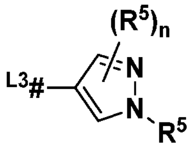, 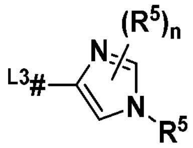, 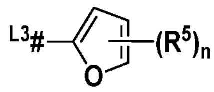, 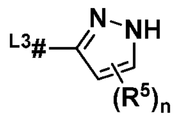, and

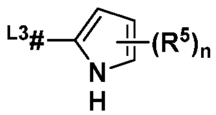;

n is selected from the group consisting of 0, 1, and 2;

$Z^1$ is selected from the group consisting of N and $CR^4$;

$R^1$ is selected from the group consisting of hydrogen, deuterium, hydroxyl, halogen, amino, C1-C6 alkyl, and $R^3O$-; wherein, the C1-C6 alkyl is optionally substituted with 1, 2 or 3 substituents selected from the group consisting of deuterium, halogen, hydroxyl and amino;

$R^2$ is selected from the group consisting of hydrogen, deuterium, cyano, halogen, C1-C6 alkyl, $R^eR^fN$-C(=O)-, $R^gO$-C(=O)-, $R^hS(=O)_2$-, $R^iR^jN$-$S(=O)_2$-,

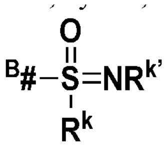, 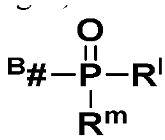,

5-to 6-membered heteroaryl, and 4- to 8-membered heterocyclyl; wherein, the C1-C6 alkyl, 5- to 6-membered heteroaryl and 4- to 8-membered heterocyclyl are optionally substituted with 0, 1, 2 and 3 substituents selected from the group consisting of deuterium, halogen, hydroxyl, amino, cyano, C1-C6 alkoxy, and phenyl;

$R^4$ is selected from the group consisting of hydrogen, deuterium, hydroxyl, halogen, and cyano;

$R^5$ is selected from the group consisting of hydrogen, deuterium, R'-NH-C(=O)-, C1-C6 alkyl, 4- to 8-membered saturated heterocyclyl, and C3-C7 cycloalkyl; wherein, the C1-C6 alkyl, 4- to 8-membered saturated heterocyclyl, C3-C7 cycloalkyl are optionally substituted with 0, 1, 2, 3 substituents selected from the group consisting of deuterium, halogen, hydroxyl, amino, cyano, C1-C3 alkyl, and phenyl;

R' is selected from the group consisting of hydrogen, deuterium, and C1-C6 alkyl.

Preferably, n is 0 or 1.

Preferably,

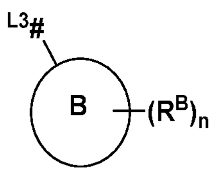

is

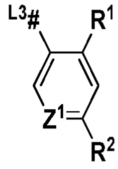;

$Z^1$ is selected from the group consisting of N, and $CR^4$;
$R^1$ is $R^3O$-;
$R^2$ is selected from the group consisting of hydrogen, cyano, halogen, C1-C6 alkyl, $R^eR^fN$-C(=O)-, $R^gO$-C(=O)-, $R^hS(=O)_2$-, $R^iR^jN$-$S(=O)_2$-,

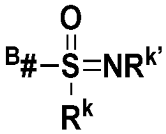, 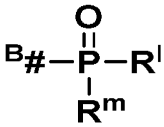,

5- to 6-membered heteroaryl, and 4- to 8-membered saturated heterocyclyl; wherein the C1-C6 alkyl, 5-to 6-membered heteroaryl, 4- to 8-membered saturated heterocyclyl are optionally substituted with 0, 1, 2, 3 substituents selected from the group consisting of deuterium, halogen, hydroxyl, amino, cyano, C1-C6 alkoxy, and phenyl;
$R^3$ is selected from the group consisting of hydrogen, C1-C6 alkyl, and C1-C6 alkyl-C(=O)-; wherein the C1-C6 alkyl is optionally substituted with halogen, hydroxyl, cyano, amino, or C1-C6 alkoxy;
$R^4$ is selected from the group consisting of hydrogen, and halogen;
further preferably, $Z^1$ is selected from the group consisting of N, and $CR^4$;
$R^1$ is $R^3O$-;
$R^2$ selected from the group consisting of cyano, halogen, C1-C6 alkyl, C1-C6 haloalkyl, hydroxyl-substituted C1-C6 alkyl, C1-C6 alkoxy-C1-C6 alkylene, cyano-C1-C6 alkylene, 4- to 8-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

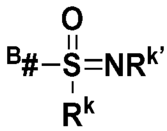, 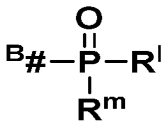,

5- to 6-membered heteroaryl, and 4- to 8-membered saturated heterocyclyl; wherein the 5- to 6-membered heteroaryl, 4- to 8-membered saturated heterocyclyl are optionally substituted with 0, 1, 2 or 3 substituents selected from the group consisting of halogen, hydroxyl, amino, cyano, and C1-C6 alkoxy;
$R^3$ is selected from the group consisting of hydrogen, methyl, ethyl, trifluoromethyl, difluoromethyl, monofluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-.
$R^4$ is selected from the group consisting of hydrogen and halogen;
more preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

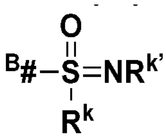, 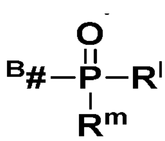,

and 5- to 6-membered heteroaryl (e.g., oxazolyl, thiazolyl, pyridyl).

Further preferably, $R^1$ is methoxy; $R^2$ is $CH_3NHC(=O)$-; $Z^1$ is selected from the group consisting of CF and CH.

In some embodiments,

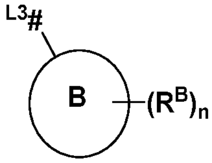

is selected from the group consisting of

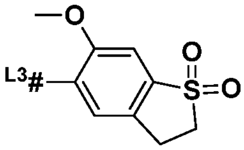, 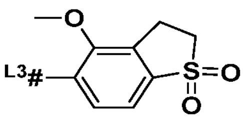, 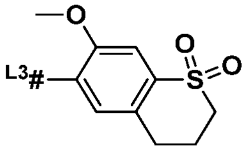, 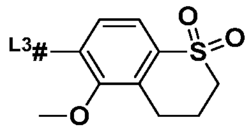,

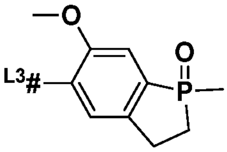, 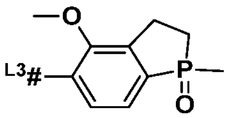, 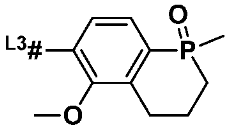, 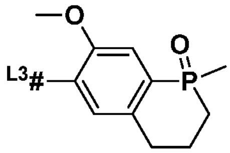,

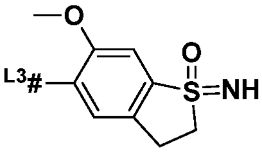, 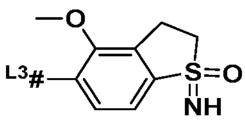, 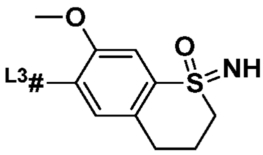, and 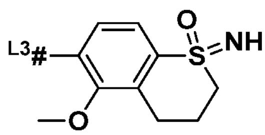.

Preferably,

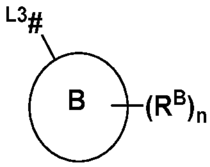

is selected from the group consisting of

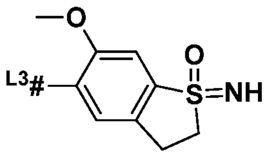,

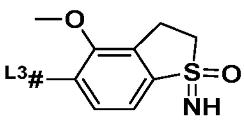, 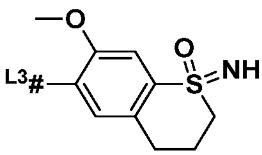, and 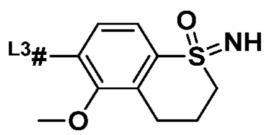.

In some embodiments, $R^5$ is selected from the group consisting of hydrogen, R'-NH-C(=O)-, C1-C6 alkyl, 4- to 6-membered saturated heterocyclyl, and C3-C6 cycloalkyl, wherein the C1-C6 alkyl, 4- to 6-membered saturated heterocyclyl, C3-C6 cycloalkyl are optionally substituted with 0, 1, 2, 3 substituents selected from the group consisting of halogen, cyano, C1-C3 alkyl, and phenyl;

R' is C1-C3 alkyl.

Preferably,

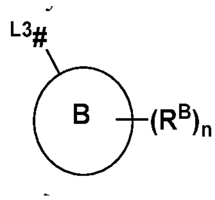

is selected from the group consisting of

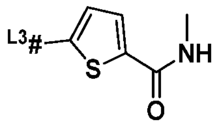 ,

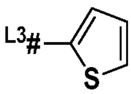 , 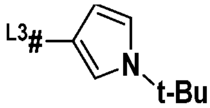 , 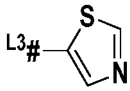 , 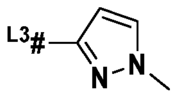 , 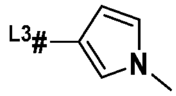 , 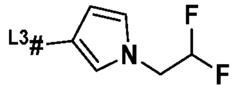 ,

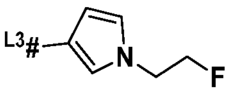 , 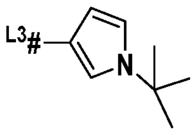 , 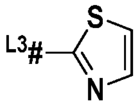 , 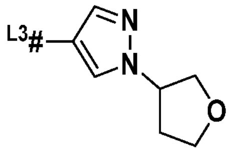 , 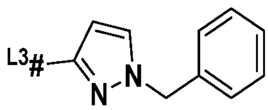 ,

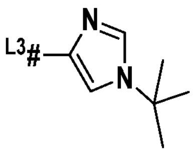 , 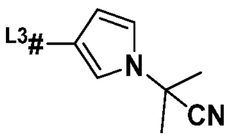 , 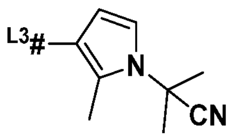 , 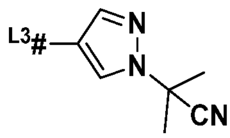 , 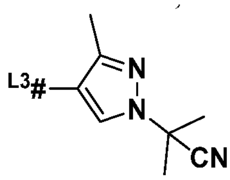 ,

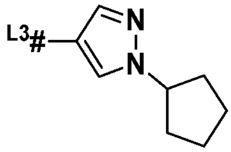 , 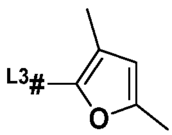 , 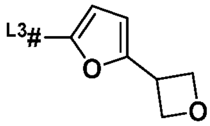 , 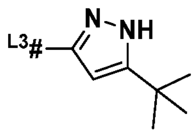 , 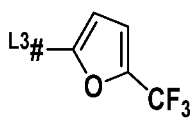 ,

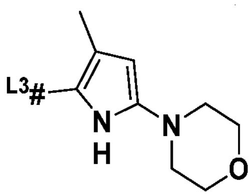 , and 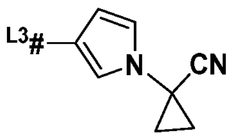 .

In some embodiments,

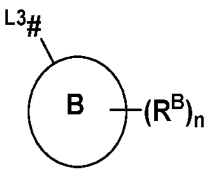 is 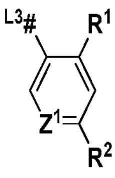 ;

$R^1$ is selected from the group consisting of hydroxyl, halogen, C1-C2 haloalkyl, and $R^3O$-;

$Z^1$ is selected from the group consisting of N, and $CR^4$;
$R^2$ is selected from the group consisting of cyano, halogen, C1-C6 alkyl, C1-C6 haloalkyl, hydroxyl-substituted C1-C6 alkyl, C1-C6 alkoxy-C1-C6 alkylene, cyano-C1-C6 alkylene, 4- to 8-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

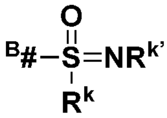 , 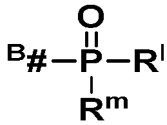 ,

and 5- to 6-membered heteroaryl;
$R^4$ is selected from the group consisting of hydrogen, deuterium, and fluorine.
preferably, $R^1$ is selected from the group consisting of hydroxyl, fluorine, trifluoromethyl, and $R^3O$-; $R^3$ is selected from the group consisting of hydrogen, methyl, ethyl, trifluoromethyl, difluoromethyl, monofluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-;
$R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

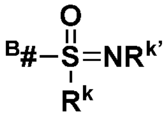 , 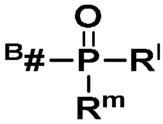 ,

and 5- to 6-membered heteroaryl;
$R^4$ is selected from the group consisting of hydrogen, and fluorine;
more preferably, $R^1$ is methoxy;
$R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-$S(=O)_2$-,

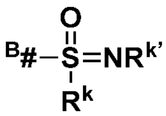 , 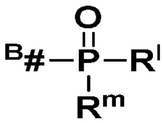 ,

oxazolyl, thiazolyl, and pyridyl.

Most preferably, $R^1$ is methoxy; $R^2$ is $CH_3NHC(=O)$-; $Z^1$ is selected from the group consisting of CF and CH.

In some embodiments, Ring B is phenyl, n is 2 or 3, and each $R^B$ is independently selected from the group consisting of halogen, $R^3O$-, and $R^eR^fN$-C(=O)-; $R^3$, $R^e$, $R^f$ have the definitions described herein.

Preferably, each $R^B$ is different.

Preferably, $R^3$ is selected from the group consisting of hydrogen, C1-C4 alkyl, C1-C4 haloalkyl, cyano-C1-C2 alkylene-, hydroxy-C1-C2 alkylene-, C1-C2 alkoxy-C1-C2 alkylene-, and C1-C2 alkyl-C(=O)-.

More preferably, $R^3$ is selected from the group consisting of hydrogen, methyl, ethyl, trifluoromethyl, monofluoromethyl, difluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-.

Most preferably, $R^3$ is methyl.

Preferably, $R^e$ and $R^f$ are each independently selected from the group consisting of hydrogen, deuterium, and C1-C6 alkyl; wherein the C1-C6 alkyl is optionally substituted with halogen, hydroxyl, cyano, amino, C1-C6 alkyl, C1-C6 alkoxy, or 4- to 8-membered heterocyclyl.

More preferably, $R^e$ and $R^f$ are each independently selected from the group consisting of hydrogen and C1-C6 alkyl.

Most preferably, $R^e$ and $R^f$ are each independently selected from the group consisting of hydrogen and methyl.

Preferably, n is 2 or 3, and each $R^B$ is independently selected from the group consisting of fluorine, methoxy, and $CH_3NHC(=O)$-, and each $R^B$ is different.

More preferably, n is 2, one $R^B$ is methoxy, and the other $R^B$ is $CH_3NHC(=O)$-.

More preferably, n is 3, and $R^B$ is fluorine, methoxy, or $CH_3NHC(=O)$-.

In some embodiments,

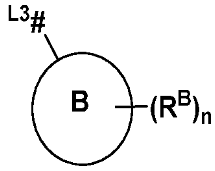

is selected from the group consisting of

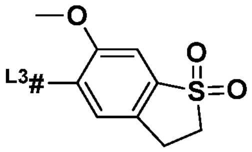 , 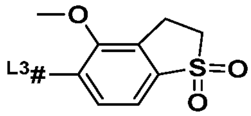 , 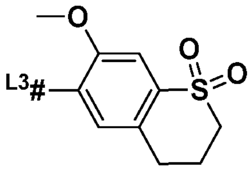 , 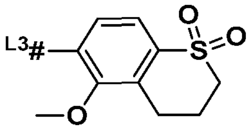 ,

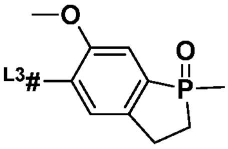 , 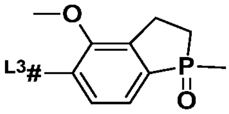 , 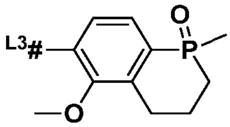 , 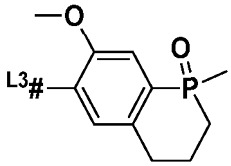 ,

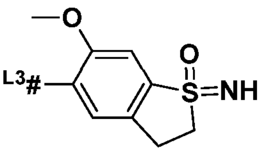 , 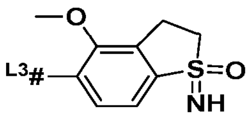 , 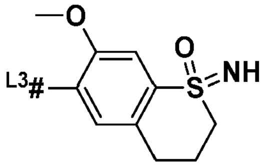 , 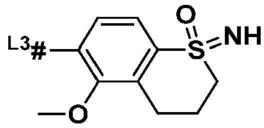 ,

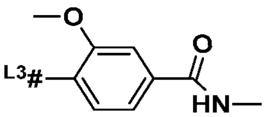 , 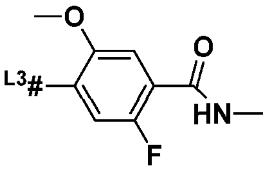 , 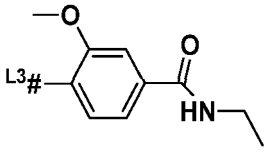 , 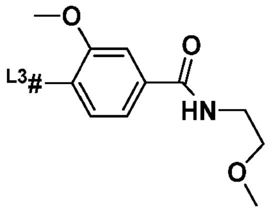 ,

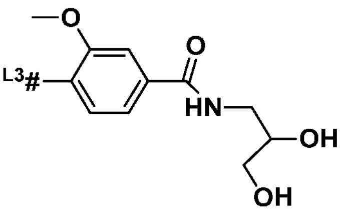 , 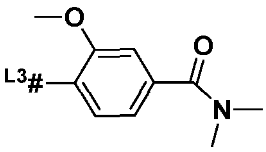 , 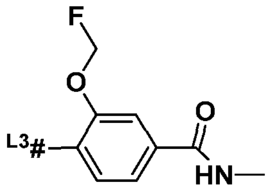 , 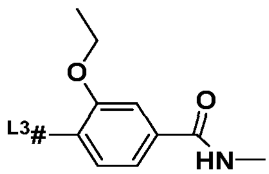 ,

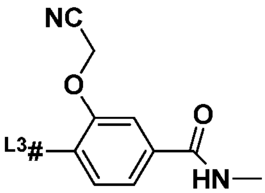 , 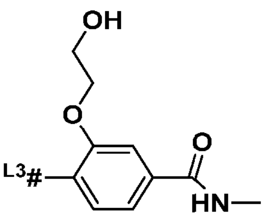 , 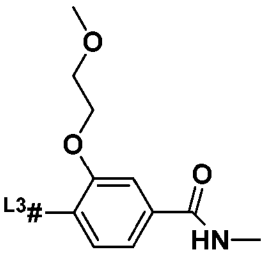 , 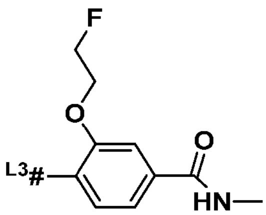 ,

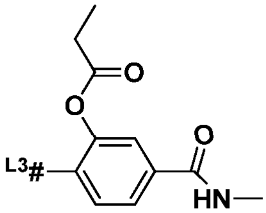, 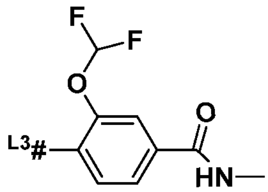, 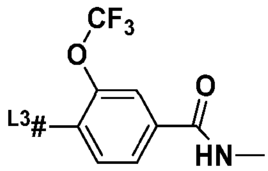, 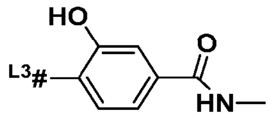,

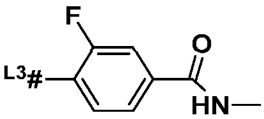, 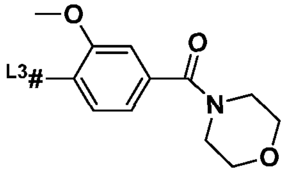, 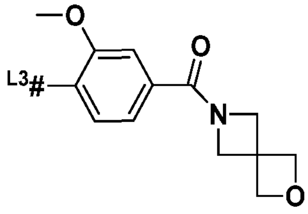, 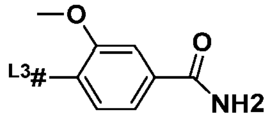,

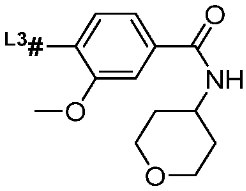, 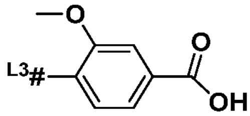, 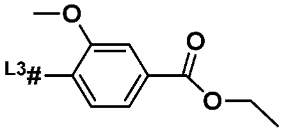, 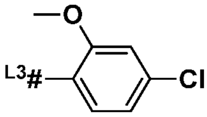,

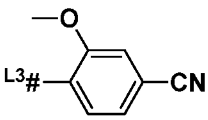, 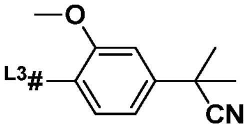, 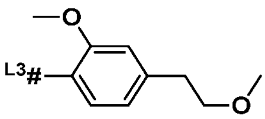, 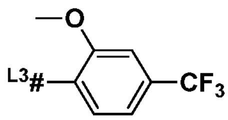,

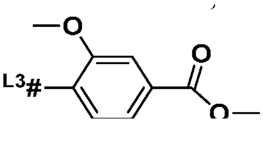, 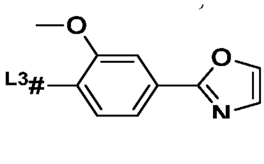, 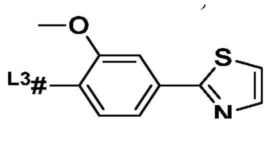, 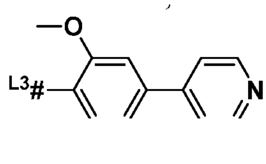,

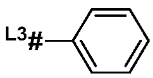, 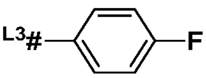, 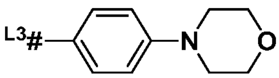, 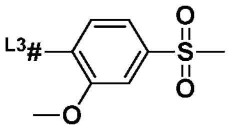,

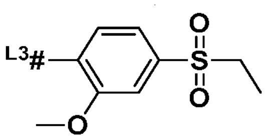, 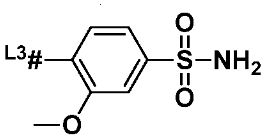, 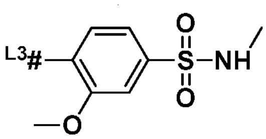, 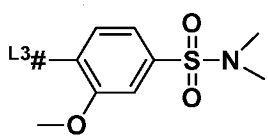,

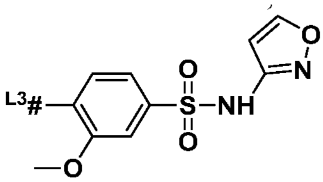, 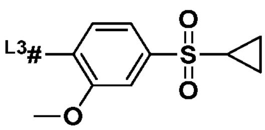, 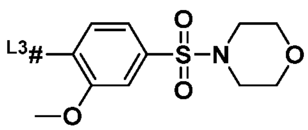,

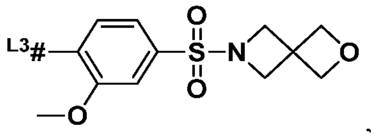, 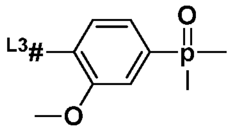, 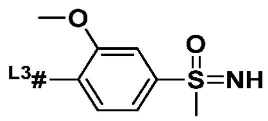, 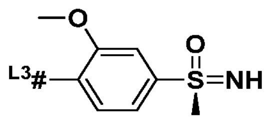,

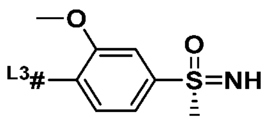, 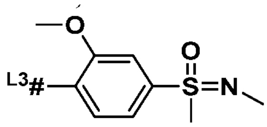, 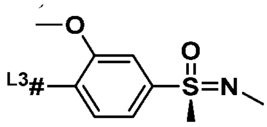, 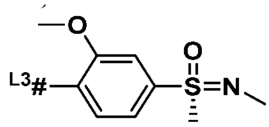,

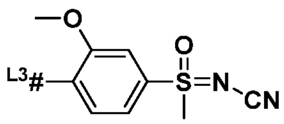, 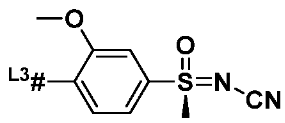, 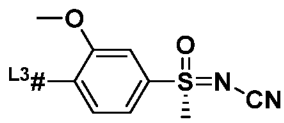, 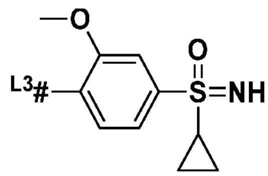,

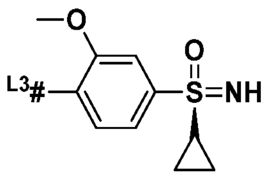, 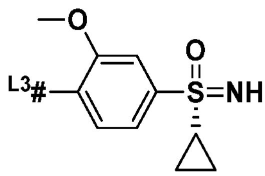, 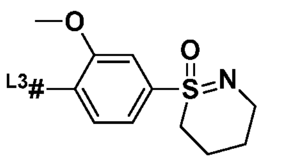, 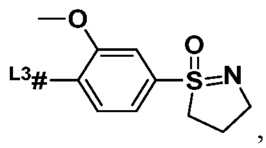,

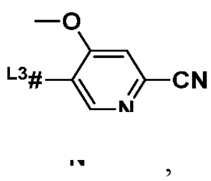, 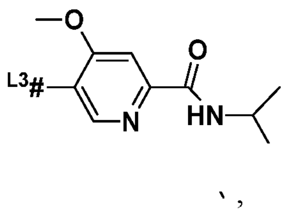, 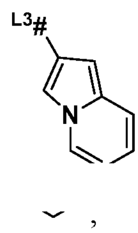, 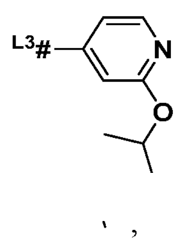, 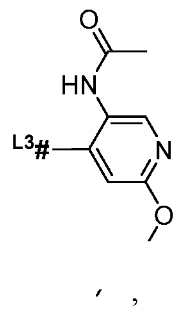,

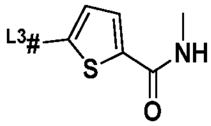, 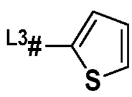, 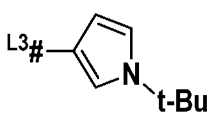, 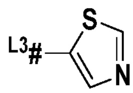, 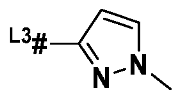, 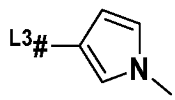,

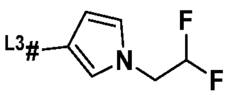, 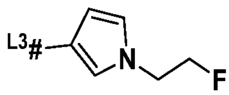, 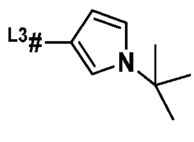, 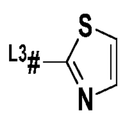, 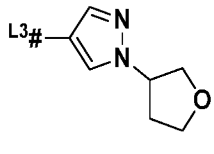,

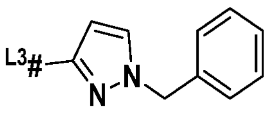, 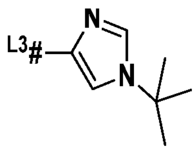, 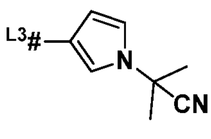, 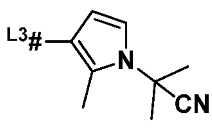, 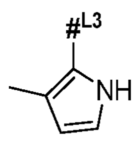,

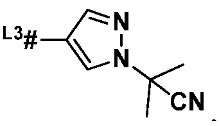,
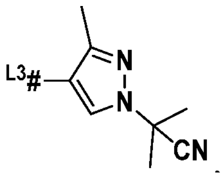,
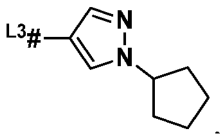,
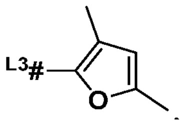,
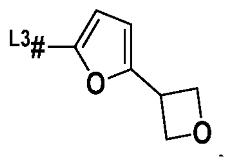,
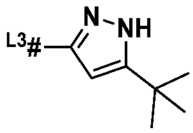
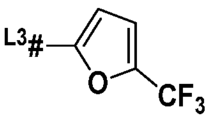
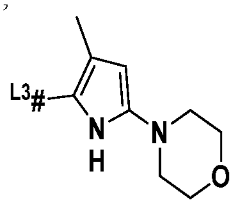 and
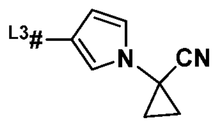
,

Preferably,

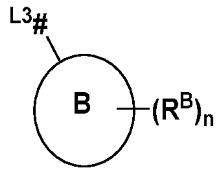

is selected from the group consisting of

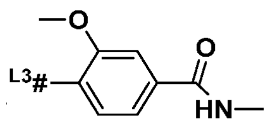,

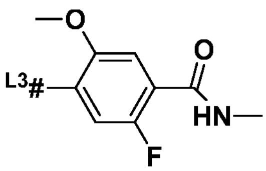,
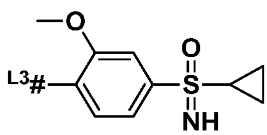,
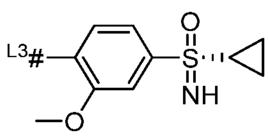,
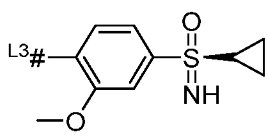,

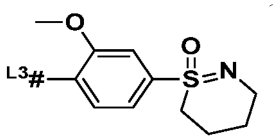,
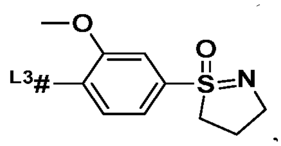,
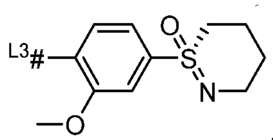, and
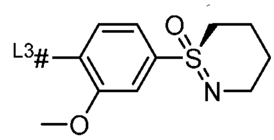.

In some embodiments, $Z^2$ is $C(R^8)_2$;
one $R^8$ is hydrogen, and the other $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they connect form a 4- to 8-membered saturated heterocyclic ring, a 7- to 11-membered spiro-heterocyclic ring or a 5- to 10-membered bridged heterocyclic ring; wherein the 7- to 11-membered spiro-heterocyclic ring, 7- to 11-membered spiro-heterocyclic ring, 5- to 10-membered bridged heterocyclyl, 5- to 10-membered bridged heterocyclic ring, 4- to 8-membered saturated heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy; or, two $R^8$ groups together with the carbon atoms to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably, the 7- to 11-membered spiro-heterocyclyl is selected from the group consisting of

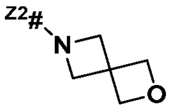, 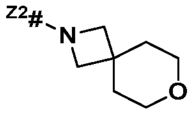, 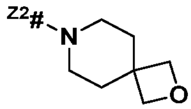, 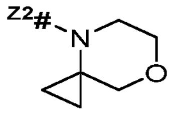, 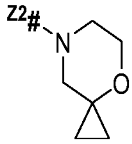, 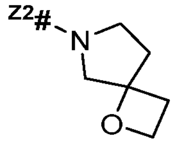,

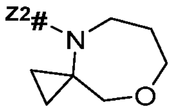, and 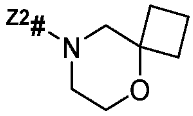;

the 5-to 10-member bridged heterocyclyl is selected from the group consisting of

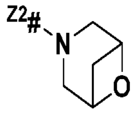, 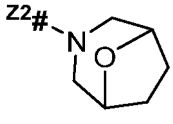, and 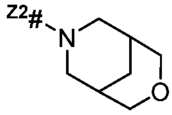;

wherein, $\#^{Z2}$ represents a connection point where $R^8$ is connected to the 6-membered ring where $Z^2$ is located.

Preferably, one $R^8$ is hydrogen, and the other $R^8$ is selected from the group consisting of 7-to 11-membered spiro-heterocyclyl, 5- to 10-membered bridged heterocyclyl, and $R^oR^pN$-; $R^o$ and $R^p$ together with the N atom to which they commonly connect form a 7- to 11-membered spiro-heterocyclic ring or a 5- to 10-membered bridged heterocyclic ring; wherein the 7- to 11-membered spiro-heterocyclyl, 7- to 11-membered spiro-heterocyclylic ring, 5- to 10-membered bridged heterocyclyl, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy; or, two $R^8$ groups together with the carbon atom to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably, one $R^8$ is hydrogen, and the other $R^8$ is selected from the group consisting of 7-to 11-membered spiro-heterocyclyl, and $R^oR^pN$-; $R^o$ and $R^p$ together with the N atom to which they commonly connect form a 7- to 11-membered spiro-heterocyclic ring; wherein the 7- to 11-membered spiro-heterocyclyl, 7- to 11-membered spiro-heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6, or amino-substituted C1-C6 alkoxy; or, two $R^8$ together with carbon atoms to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

More preferably, one $R^8$ is hydrogen, and the other $R^8$ is selected from the group consisting of 7- to 11-membered spiro-heterocyclyl, and $R^oR^pN$-; $R^o$ and $R^p$ together with the N atom to which they connect form a 7- to 11-membered spiro-heterocyclic ring; wherein the 7- to 11-membered spiro-heterocyclyl, 7- to 11-membered spiro-heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably, one $R^8$ is hydrogen, and the other $R^8$ is selected from the group consisting of 5-to 10-membered bridged heterocyclyl, and $R^oR^pN$-; $R^o$ and $R^p$ together with the N atom to which they connect form a 5- to 10-membered bridged heterocyclic ring; wherein the 5- to 10-membered bridged heterocyclyl, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably, one $R^8$ is hydrogen, and the other $R^8$ is $R^oR^pN$-, $R^o$ and $R^p$ together with the N atom to which they connect form a 4- to 8-membered saturated heterocyclic ring; wherein the 4-to 8-membered saturated heterocyclic ring is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably,

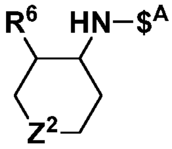

is selected from the group consisting of

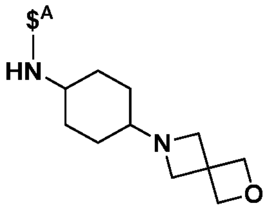,

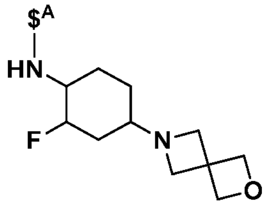, 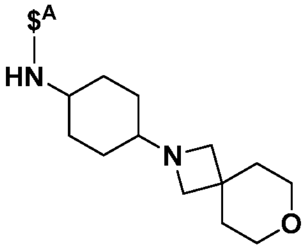, 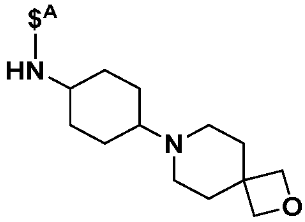, 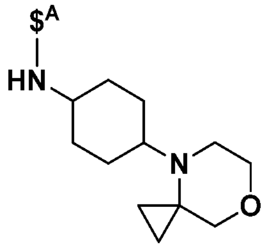,

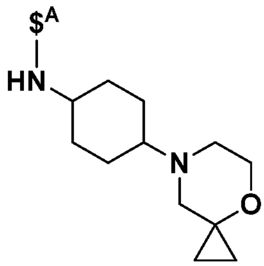, 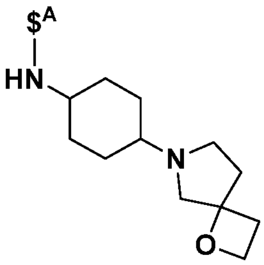, 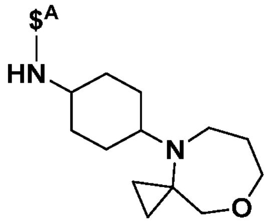, 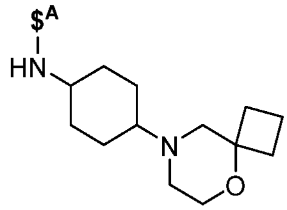,

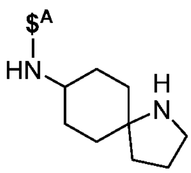, 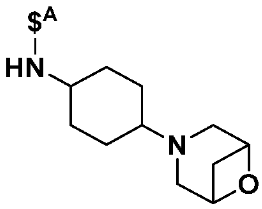, 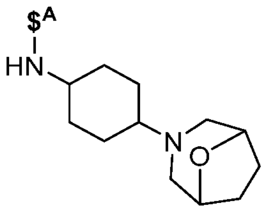, and 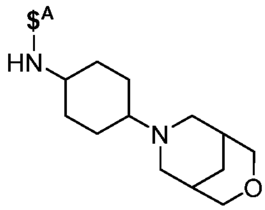;

wherein

$^A represents a connection point between NH and Ring A.

In some embodiments, $Z^2$ is $N(R^7)$;

$R^7$ is C1-C6 alkyl; wherein the C1-C6 alkyl is optionally substituted with hydroxyl, C1-C6 alkoxy, or hydroxyl-substituted C1-C6 alkoxy.

Preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl; more preferably, $R^7$ is selected from the group consisting of methyl,

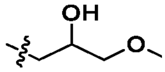

and

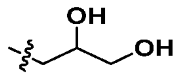.

In some embodiments, $R^6$ is selected from the group consisting of hydrogen and fluorine.

In some embodiments, each $R^a$ and $R^d$ is independently selected from the group consisting of C1-C4 alkyl, C1-C2 haloalkyl, and cyano-C1-C2 alkylene.

In some embodiments, each $R^b$ is independently selected from the group consisting of hydrogen, hydroxyl, halogen, and cyano.

In some embodiments, each $R^c$ is independently selected from the group consisting of hydrogen and halogen.

In some embodiments, each $R^a$ and $R^d$ is independently selected from the group consisting of $CF_3CH_2$-, $CH_2FCH_2$-, $CH_3CH_2CH_2$-, $CNCH_2$-, and $CHF_2CH_2$-.

In some embodiments, each $R^b$ is independently selected from the group consisting of hydrogen and hydroxyl.

In some embodiments, each $R^c$ is independently selected from the group consisting of hydrogen and fluorine.

In some embodiments,

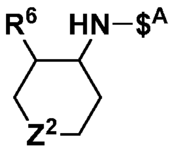

is selected from

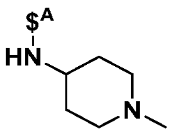, 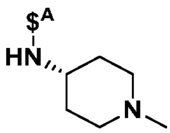,

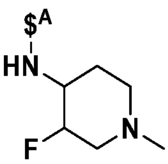, 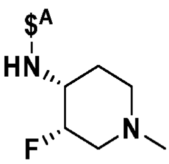, 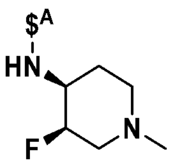, 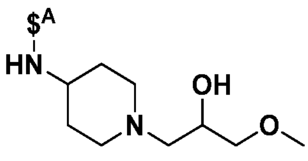, 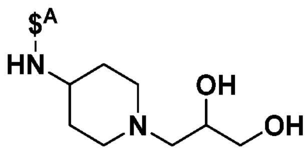,

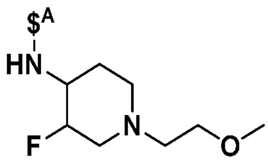, 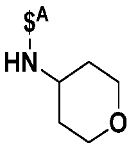, 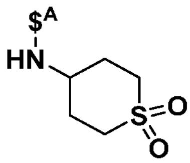, 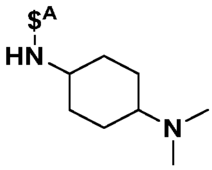, 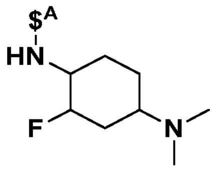,

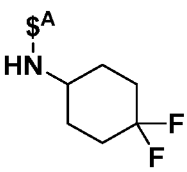. 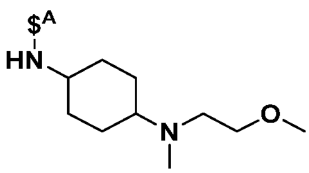. 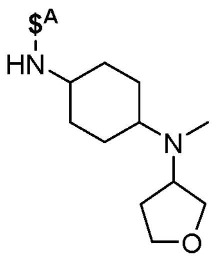. 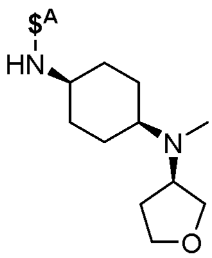. 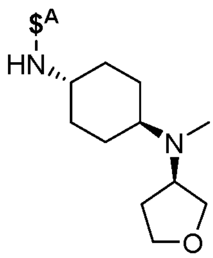.

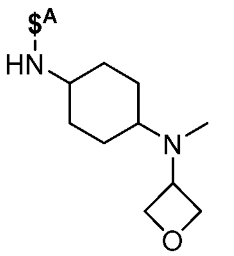, 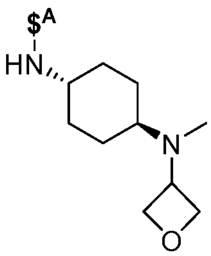, 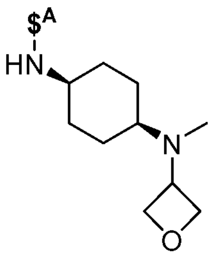, 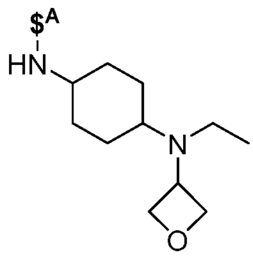, 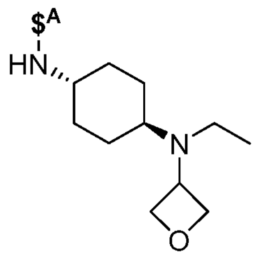,

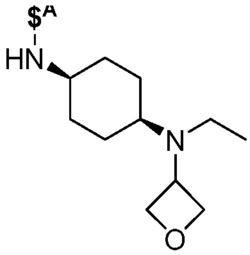, 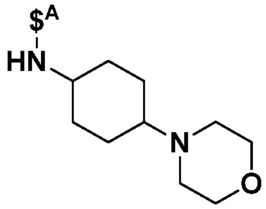, 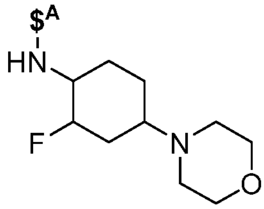, 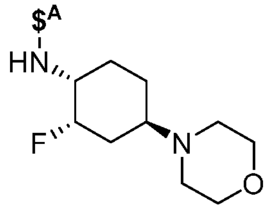,

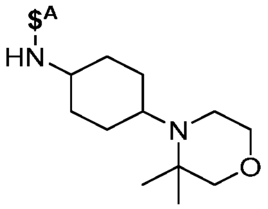, 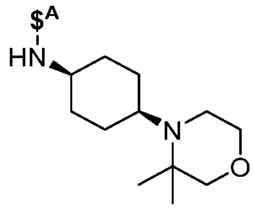, 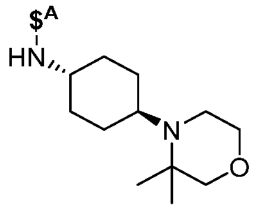, 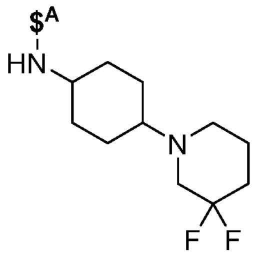,

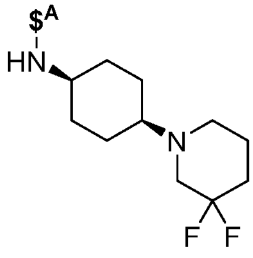, 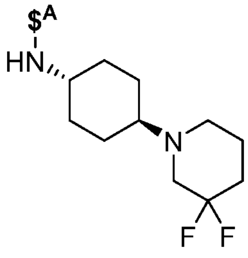, 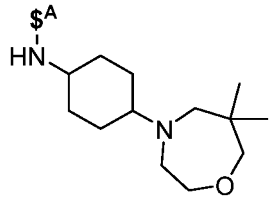, 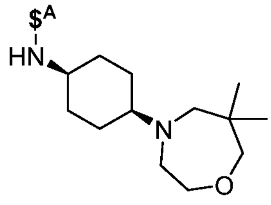,

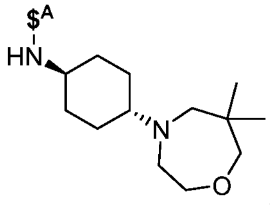, 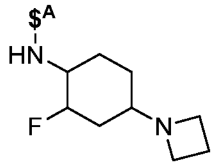, 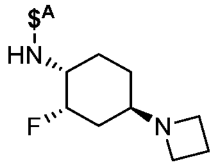, 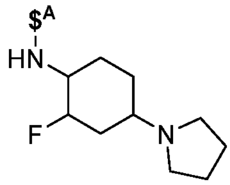, 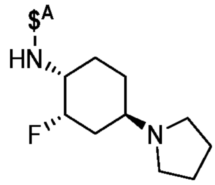,

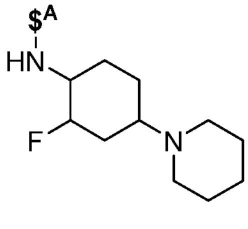, 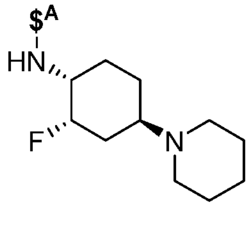, 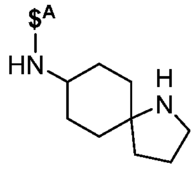, 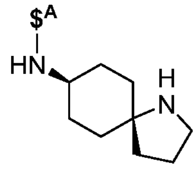, 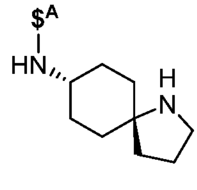,

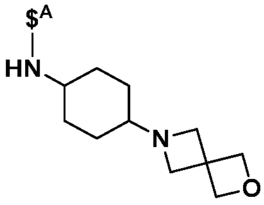, 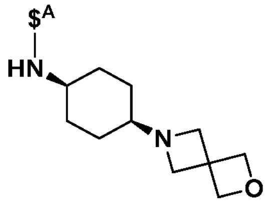, 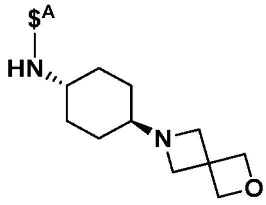, 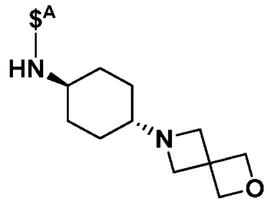,

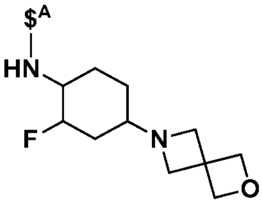, 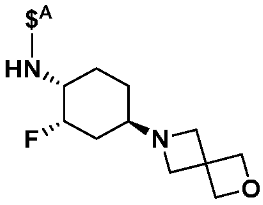, 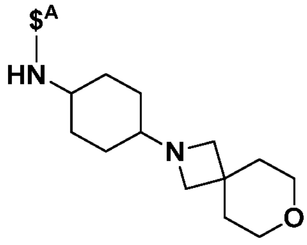, 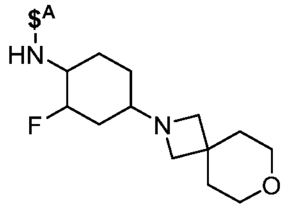,

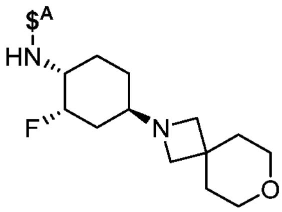, 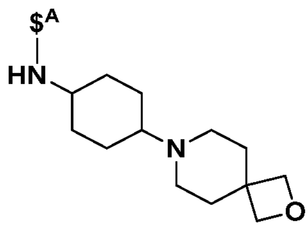, 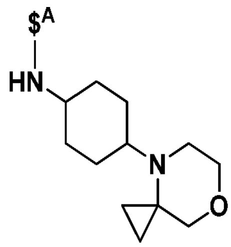, 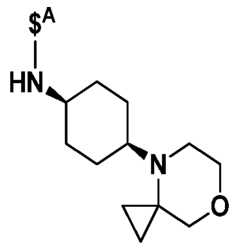,

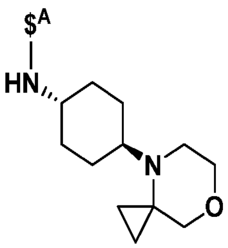, 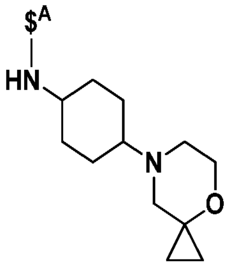, 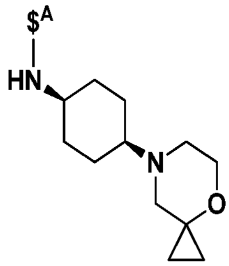, 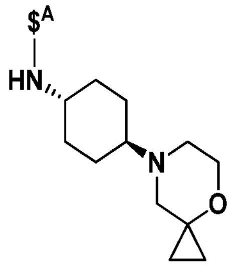,

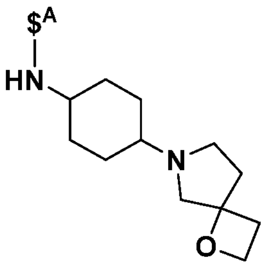, 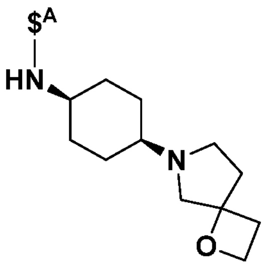, 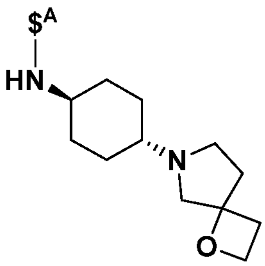, 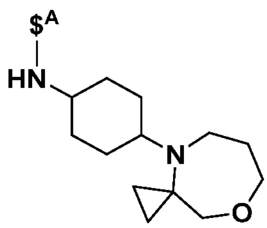,

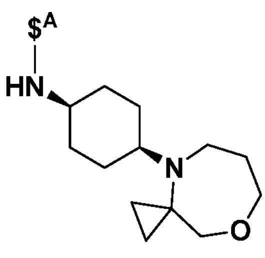, 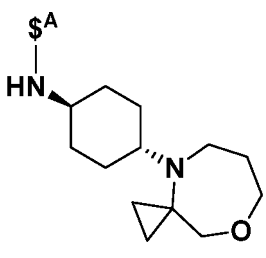, 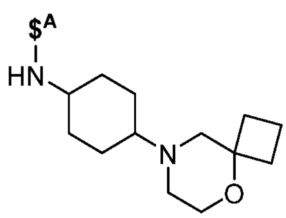, 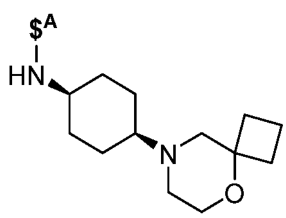,

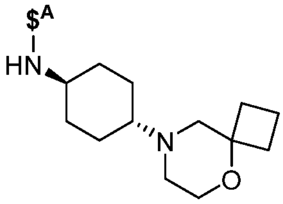, 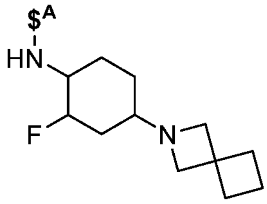, 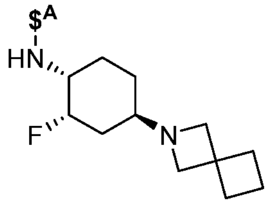, 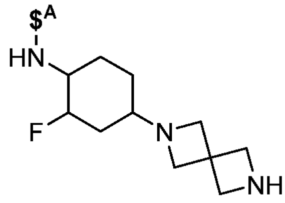, 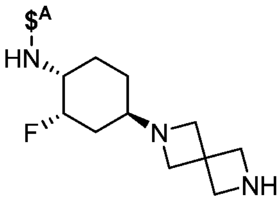, 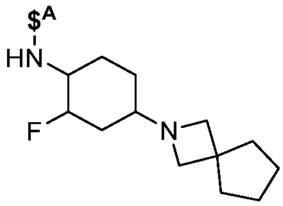, 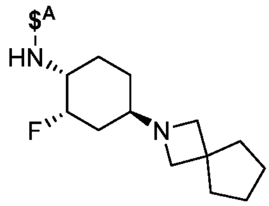, 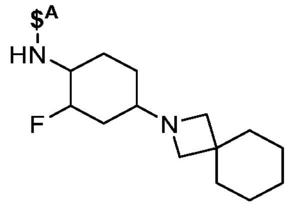, 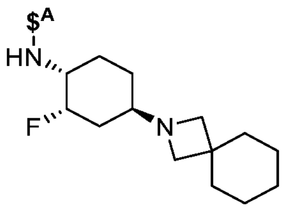, 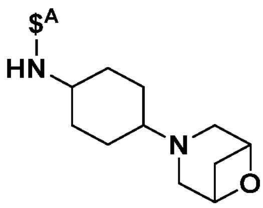, 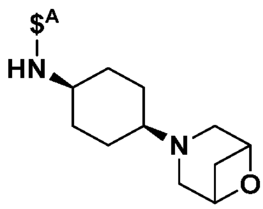, 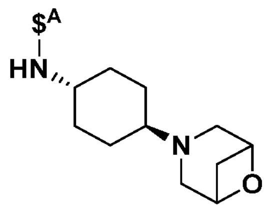, 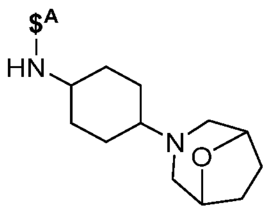, 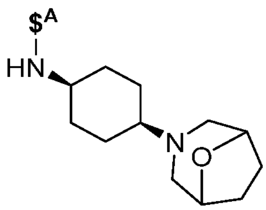, 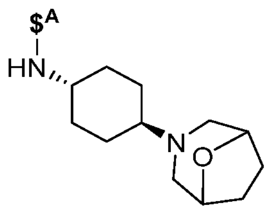, 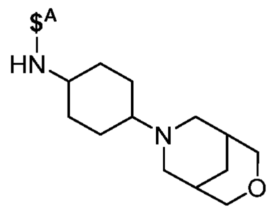, 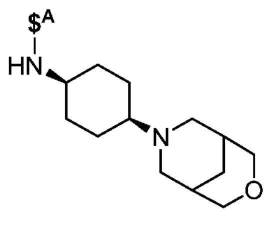, and 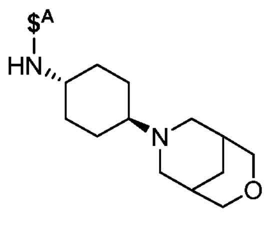.

Preferably, in

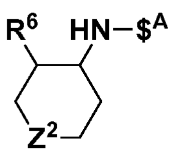 ,

$R^6$ is selected from the group consisting of hydrogen and fluorine; $Z^2$ is $NR^7$, wherein $R^7$ is C1-C6 alkyl, wherein the C1-C6 alkyl is substituted with a substituent selected from the group consisting of halogen, hydroxyl and/or C1-C6 alkoxy.

More preferably, the C1-C6 alkyl is substituted with a substituent selected from the group consisting of fluorine, hydroxyl, and/or C1-C3 alkoxy.

Preferably,

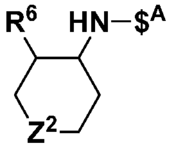

is selected from the group consisting of

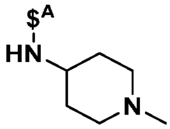,

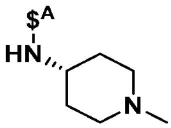, 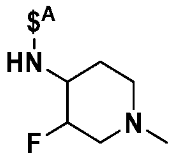, 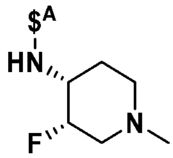, 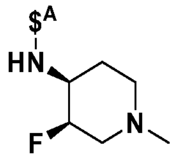, 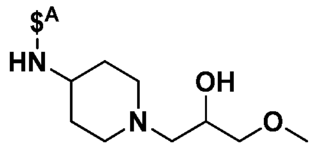,

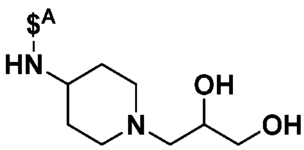, 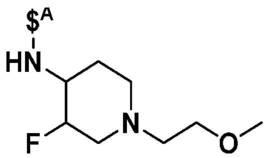, 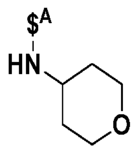, and 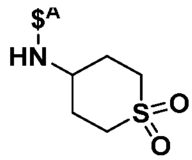.

Preferably,

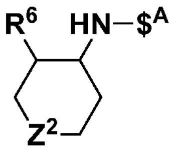

is selected from the group consisting of

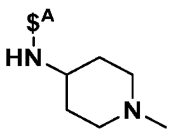,

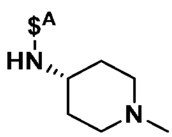, 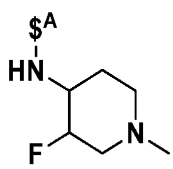, 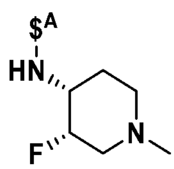, and 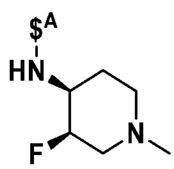.

Preferably, in

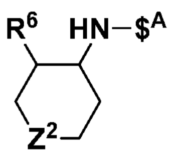

, $R^6$ is selected from the group consisting of hydrogen and fluorine; $Z^2$ is $C(R^8)_2$, and each $R^8$ is independently selected from the group consisting of hydrogen, deuterium, halogen, hydroxyl, amino, cyano, C1-C6 alkyl, 4- to 8-membered saturated heterocyclyl, 7- to 11-membered spiro-heterocyclyl, 5- to 10-membered bridged heterocyclyl, and $R^oR^pN$-; wherein the C1-C6 alkyl, 4- to 8-membered saturated heterocyclyl, 7- to 11-membered spiro-heterocyclyl, 5- to 10-membered bridged heterocyclyl are optionally substituted with halogen, hydroxyl, amino, cyano, C1-C6 alkyl, halogen-substituted C1-C6 alkyl, hydroxyl-substituted C1-C6 alkyl, or amino-substituted C1-C6 alkyl; provided that: at most one $R^8$ is selected from the group consisting of 4- to 8-membered saturated heterocyclyl, 7- to 11-membered spiro-heterocyclyl, and 5- to 10-membered bridged heterocyclyl; or two $R^8$ groups together with the carbon atom to which they connect form a 4- to 8-membered saturated heterocyclyl; wherein the 4- to 8-membered saturated heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

$R^o$, $R^p$ are each independently selected from the group consisting of hydrogen, deuterium, C1-C6 alkyl, and 4- to 8-membered saturated heterocyclyl; wherein the C1-C6 alkyl, 4- to 8-membered saturated heterocyclyl are optionally substituted with halogen, hydroxyl, amino, cyano, or C1-C6 alkoxy; or, $R^o$ and $R^p$ together with the N atom to which they connect form a 4-to 8-membered saturated heterocyclic ring, a 7- to 11-membered spiro-heterocyclic ring or a 5-to 10-membered bridged heterocyclic ring; wherein the 4- to 8-membered heterocyclic ring, 7- to 11-membered spiro-heterocyclic ring, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

More preferably, one $R^8$ is hydrogen or halogen, and the other $R^8$ is selected from the group consisting of halogen, C1-C6 alkyl, 4- to 8-membered saturated heterocyclyl, 7- to 11-membered spiro-heterocyclyl, 5- to 10-membered bridged heterocyclyl, and $R^oR^pN$-; or, two $R^8$ groups together with the carbon atom to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Preferably,

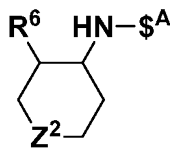

is selected from

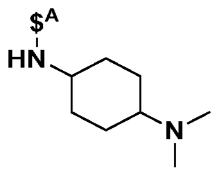, 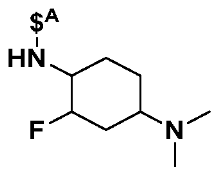,

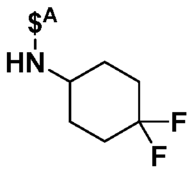, 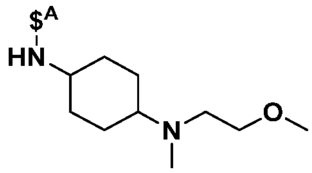, 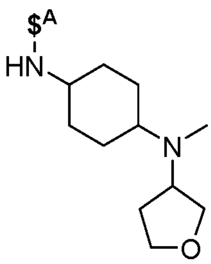, 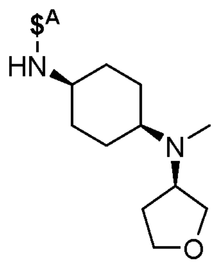, 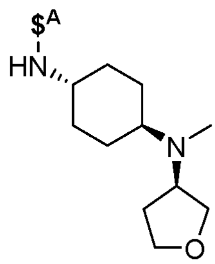.

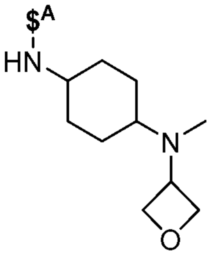, 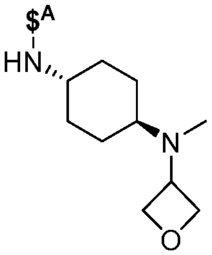, 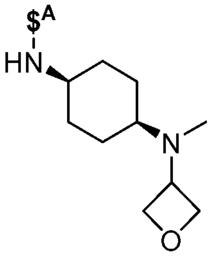, 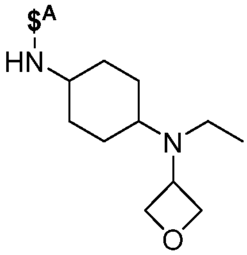, 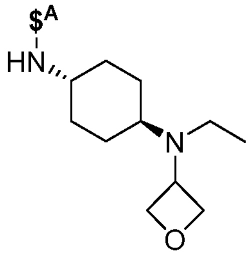,

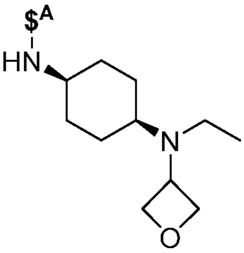, 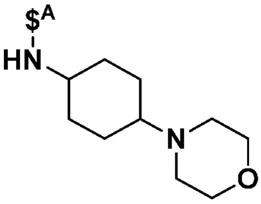, 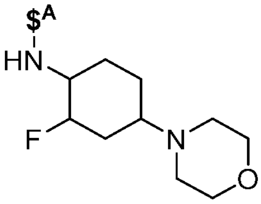, 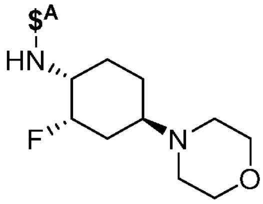,

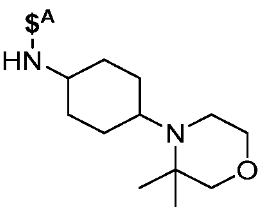, 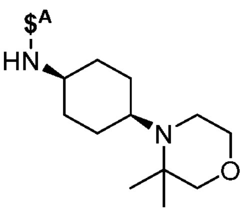, 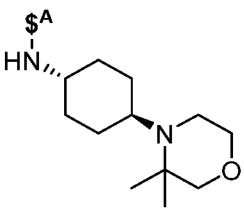, 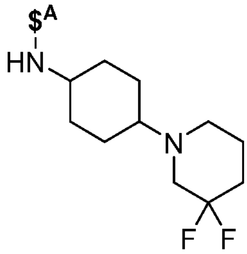,

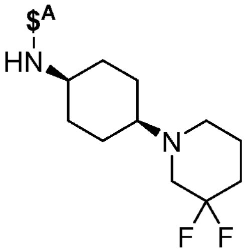, 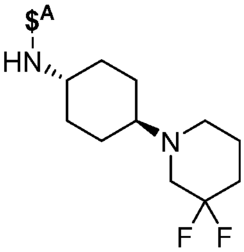, 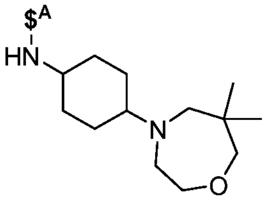, 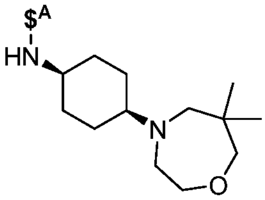,

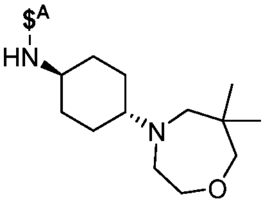, 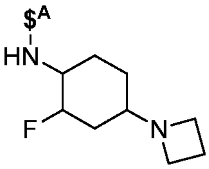, 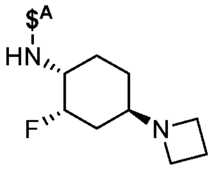, 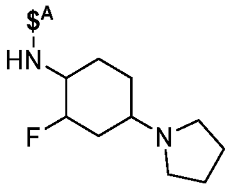, 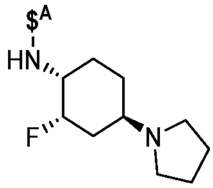,

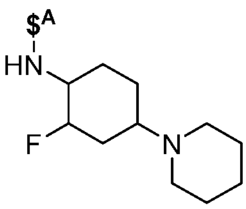, 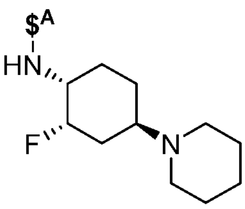, 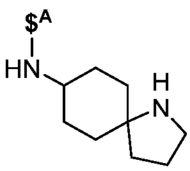, 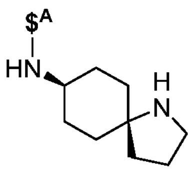, 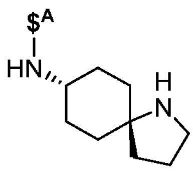,

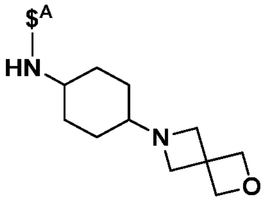, 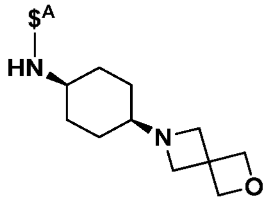, 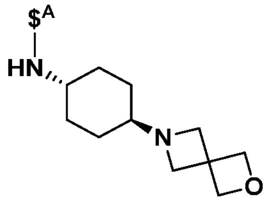, 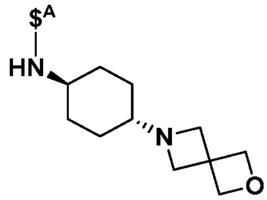,

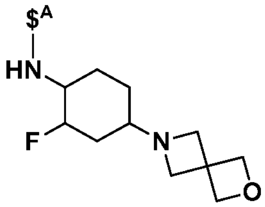, 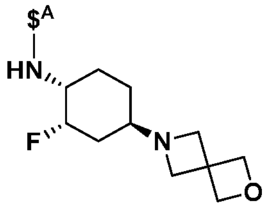, 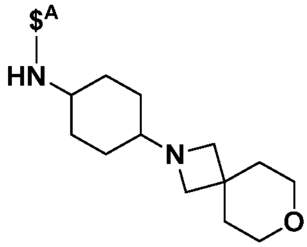, 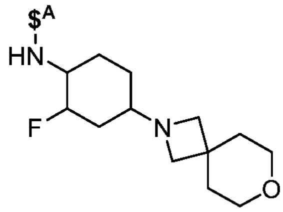,

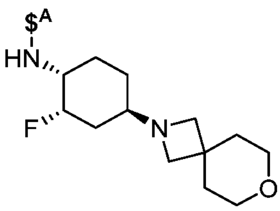, 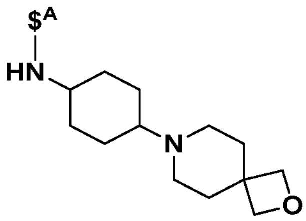, 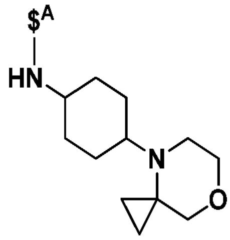, 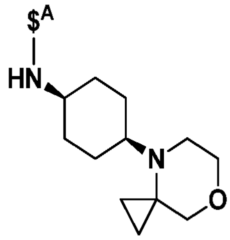,

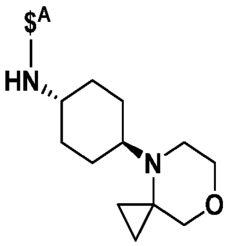, 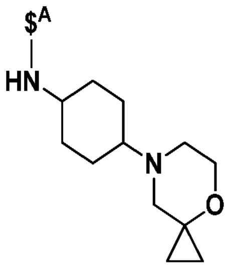, 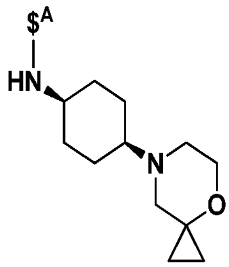, 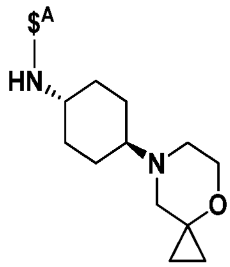,

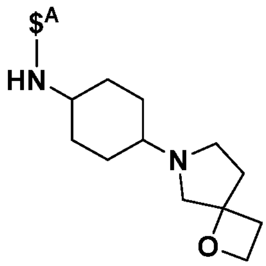, 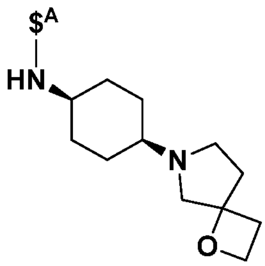, 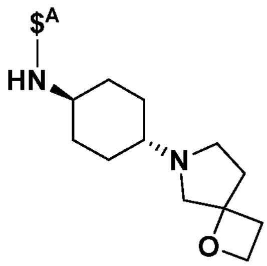, 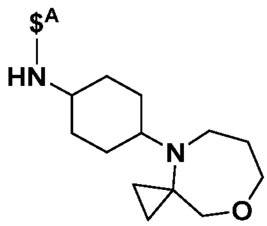,

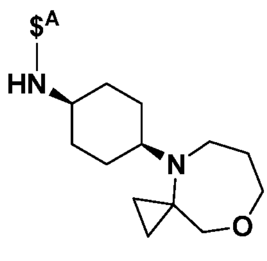, 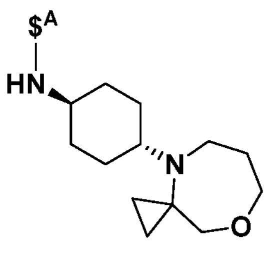, 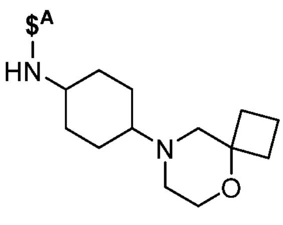, 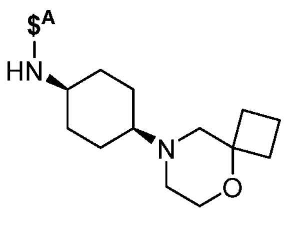,

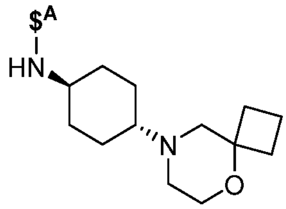, 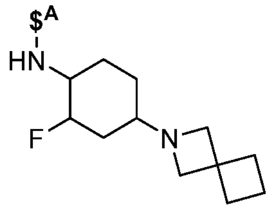, 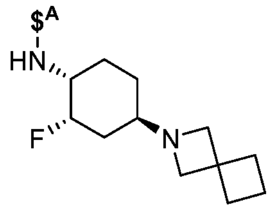, 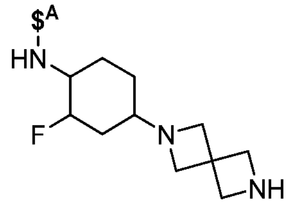,

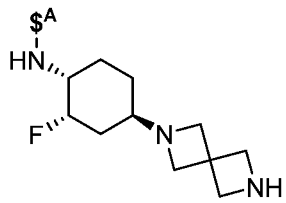, 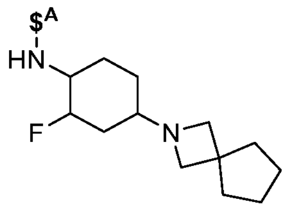, 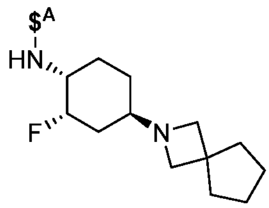, 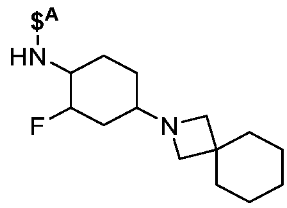,

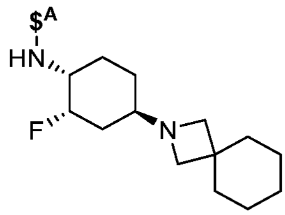, 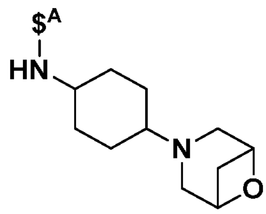, 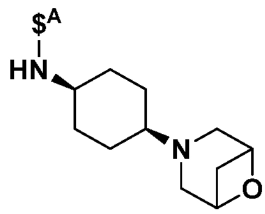, 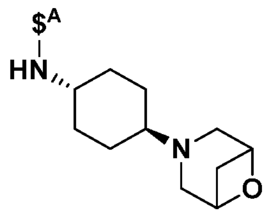,

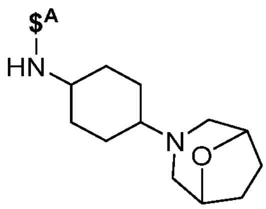, 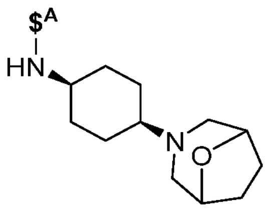, 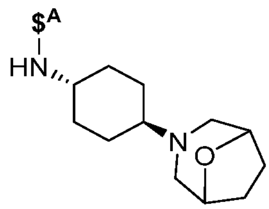, 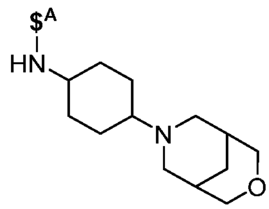,

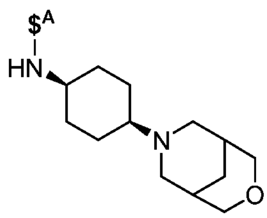, 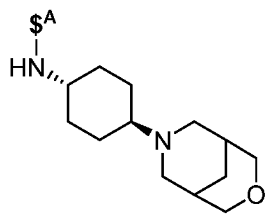.

Preferably, in

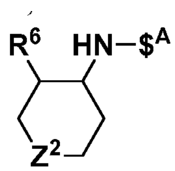,

$R^6$ is selected from the group consisting of hydrogen and fluorine; $Z^2$ is $C(R^8)_2$, and two $R^8$ groups together with the carbon atom to which they connect form a 4- to 8-membered heterocyclyl.

More preferably, two $R^8$ groups together with the carbon atom to which they connect form a 4- to 7-membered heterocyclyl.

More preferably, two $R^8$ groups together with the carbon atom to which they connect form a 4- to 6-membered heterocyclyl.

More preferably, two $R^8$ groups together with the carbon atom to which they connect form a 5-membered heterocyclyl.

More preferably,

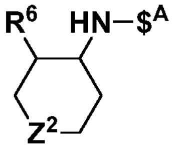

is selected from the group consisting of

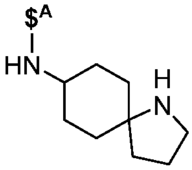
,

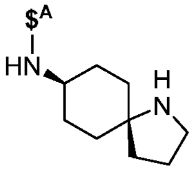
, and
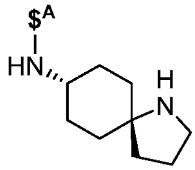
.

Preferably, in

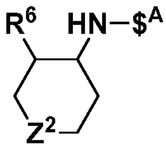
,

$R^6$ is selected from the group consisting of hydrogen and fluorine; $Z^2$ is $C(R^8)_2$, one $R^8$ is hydrogen, and the other $R^8$ is selected from the group consisting of 7- to 11-membered spiro-heterocyclyl, and $R^oR^pN$-; $R^o$ and $R^p$ together with and the N atom to which they connect form a 7- to 11-membered spiro-heterocyclic ring.

More preferably, the other $R^8$ is selected from the group consisting of 7- to 9-membered spiro-heterocyclyl, and $R^oR^pN$-; $R^o$ and $R^p$ together with the N atom to which they connect form a 7- to 9-membered spiro-heterocyclic ring.

Further preferably,

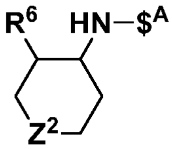

is selected from the group consisting of

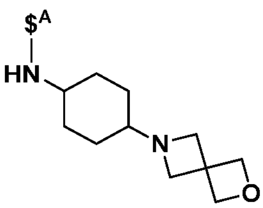
,
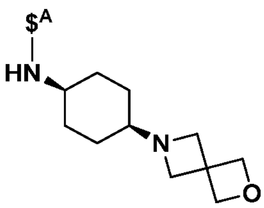
,
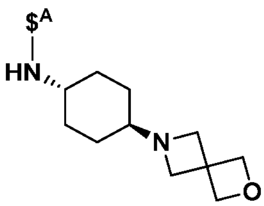
,
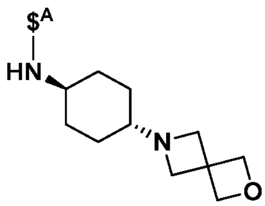
,

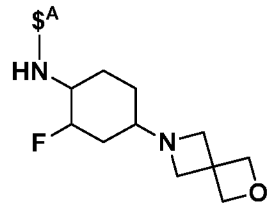, 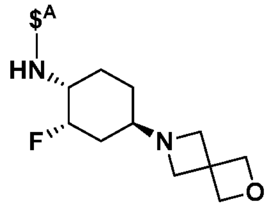, 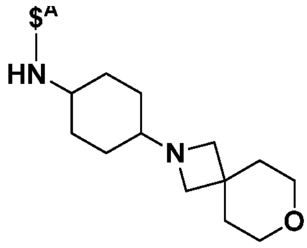, 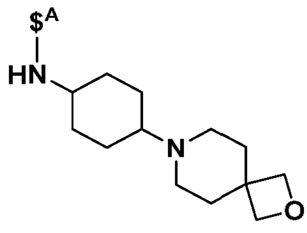,

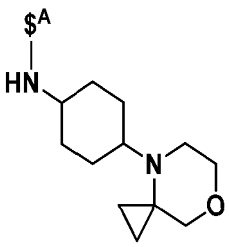, 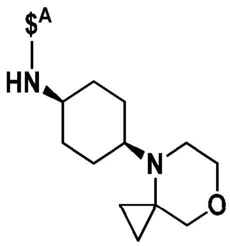, 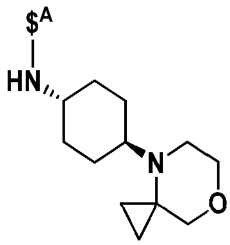, 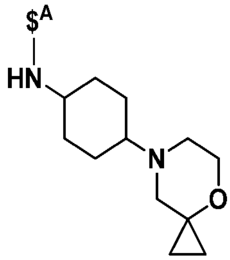,

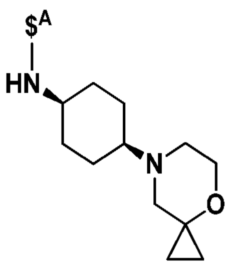, 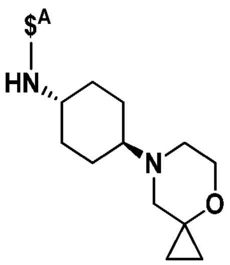, 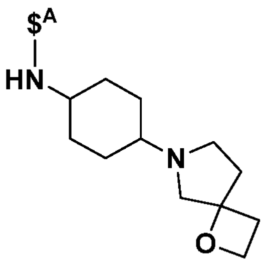, 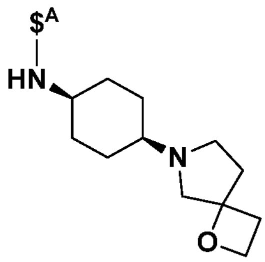,

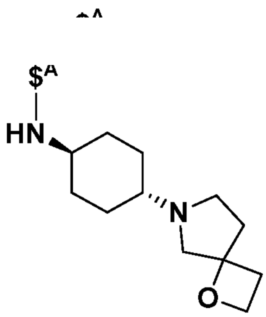, 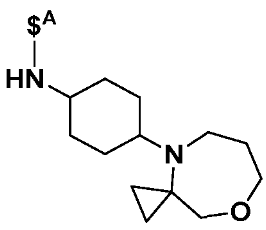, 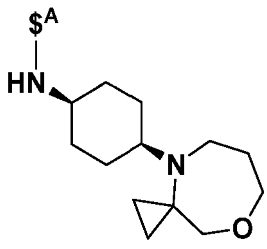, 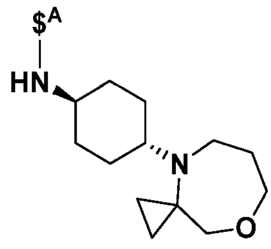,

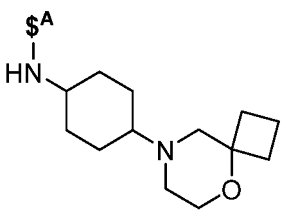, 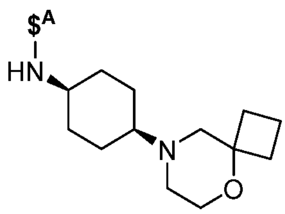, 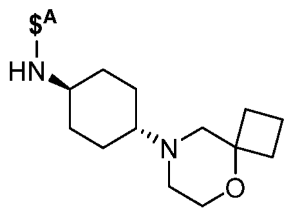, 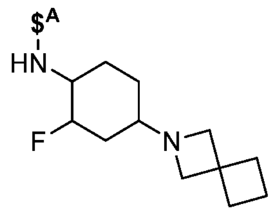,

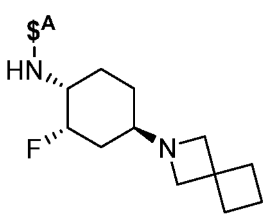, 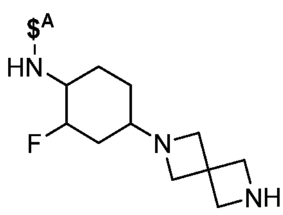, 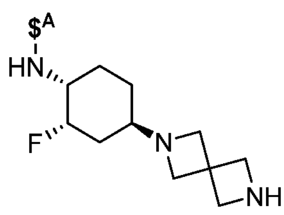, 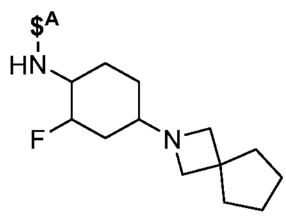,

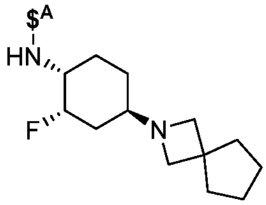, 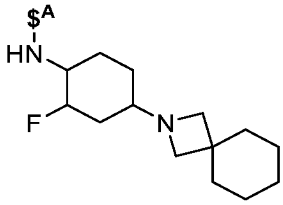, and 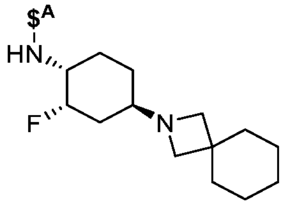.

More preferably,

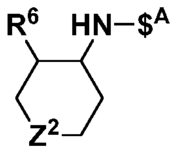

is selected from the group consisting of

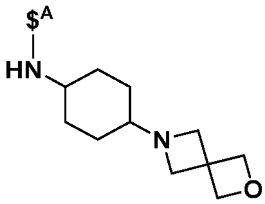, 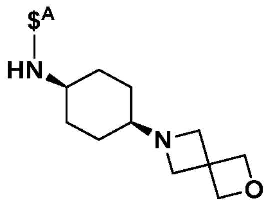, 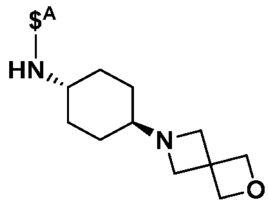, 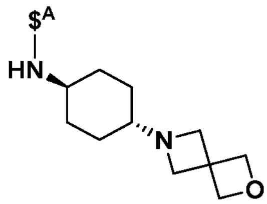, 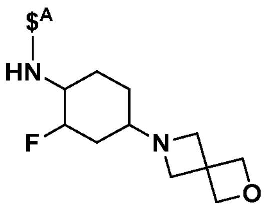, 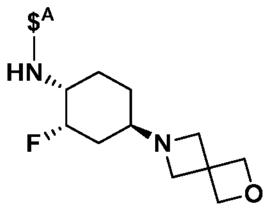, 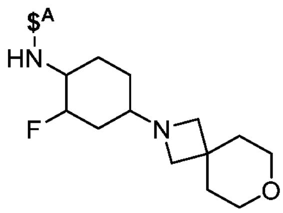, and 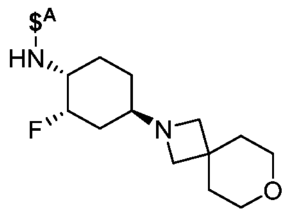.

Preferably, in

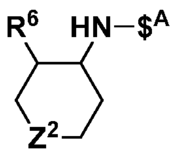

, $R^6$ is selected from the group consisting of hydrogen and fluorine; $Z^2$ is $C(R^8)_2$, one $R^8$ is hydrogen, and the other $R^8$ is selected from the group consisting of a 5- to 10-membered heterocyclyl, and $R^oR^pN$-; wherein $R^o$ and $R^p$ together with the N atom to which they connect form a 5- to 10-membered heterocyclic ring.

More preferably, the other $R^8$ is selected from the group consisting of a 7- to 9-membered heterocyclyl, and $R^oR^pN$-; wherein $R^o$ and $R^p$ together with the N atom to which they connect form a 7- to 9-membered heterocyclic ring.

Further preferably,

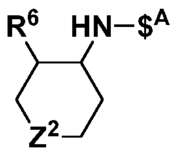

is selected from the group consisting of

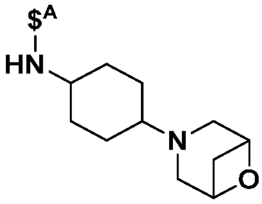, 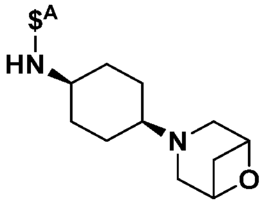, 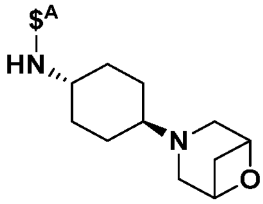, 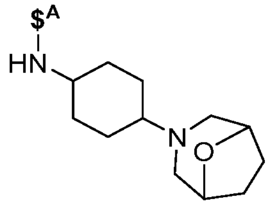,

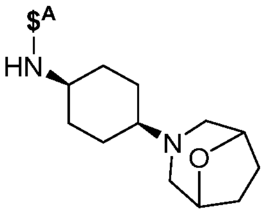, 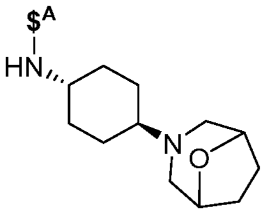, 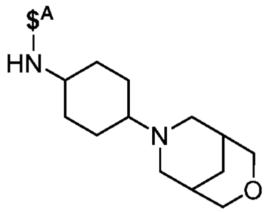, 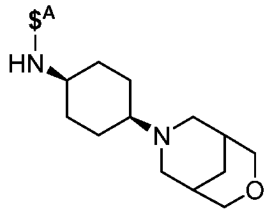, and

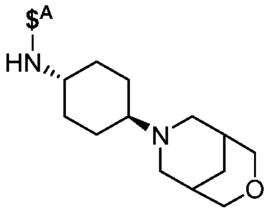.

Preferably, in

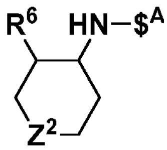

, $R^6$ is selected from the group consisting of hydrogen and fluorine; $Z^2$ is $C(R^8)_2$, wherein one $R^8$ is hydrogen, and the other $R^8$ is a 4- to 7-membered heterocyclyl, wherein the 4- to 7-membered heterocyclyl is substituted with halogen and/or C1-C6 alkyl.

More preferably, the 4- to 7-membered heterocyclyl is substituted with halogen and/or C1-C3 alkyl.

More preferably,

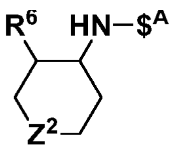

is selected from the group consisting of

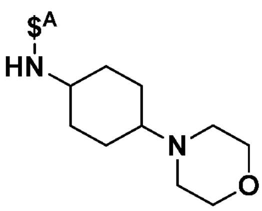, 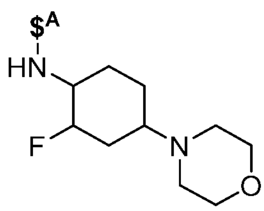, 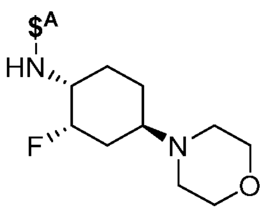, 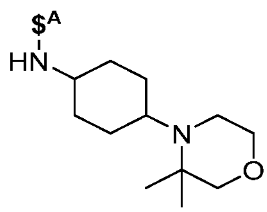,

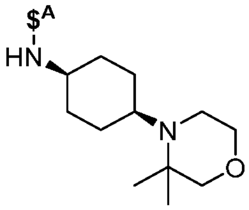, 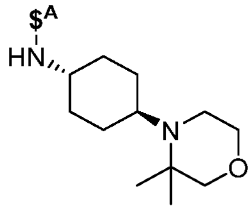, 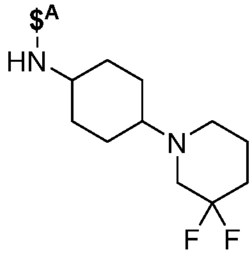, 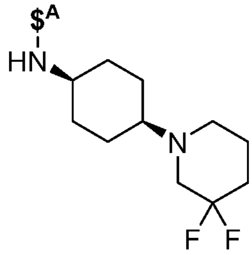,

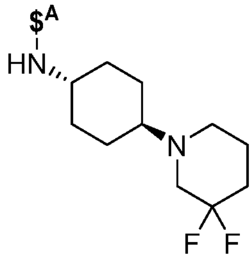, 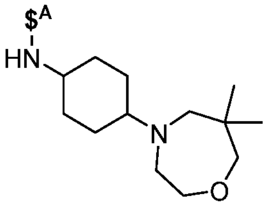, 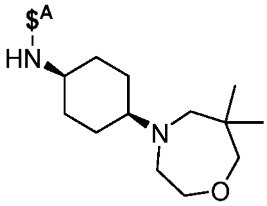, 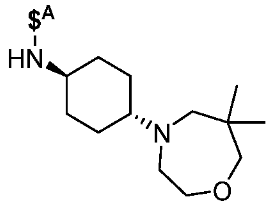,

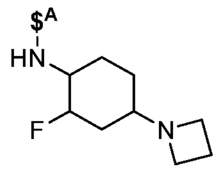, 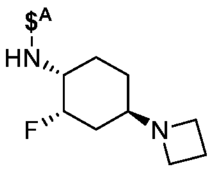, 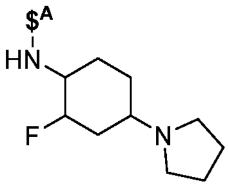, 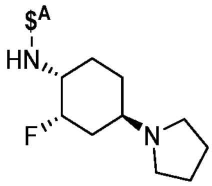, 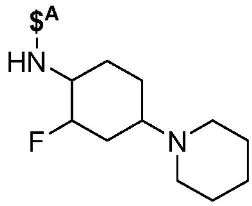,

and

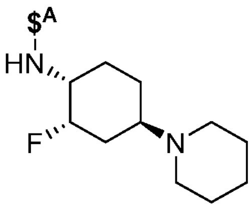.

In some embodiments, the compound is represented by Formula MI-1 and MI-2:

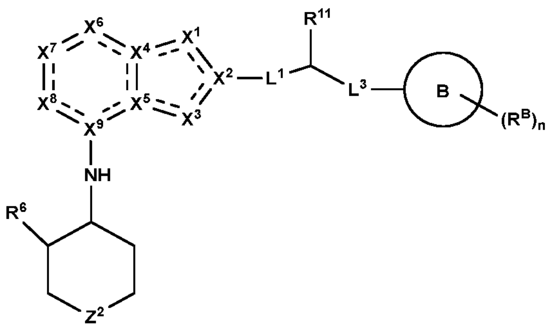

MI-1

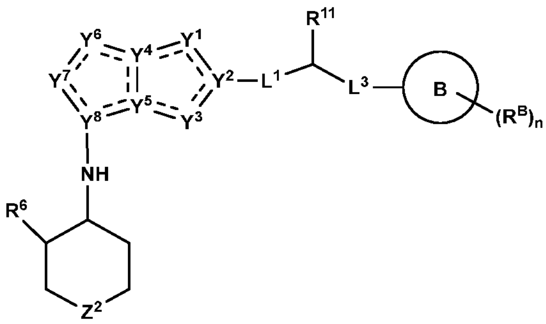

MI-2

wherein, $X^1$, $X^2$, $X^3$, $X^4$, $X^5$, $X^6$, $X^7$, $X^8$, $X^9$, $Y^1$, $Y^2$, $Y^3$, $Y^4$, $Y^5$, $Y^6$, $Y^7$, $Y^8$, $L^1$, $L^3$, Ring B, $R^B$, n, $R^6$, $R^{11}$, $Z^2$ have the definitions described herein.

Preferably, $L^1$ is

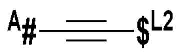

.

More preferably, $L^1$ is a 5-membered heteroarylene.

Further preferably, $L^1$ is selected from the group consisting of

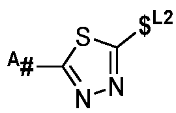

,

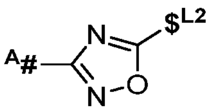

,

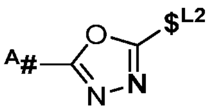

,

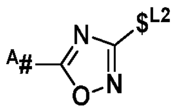

,

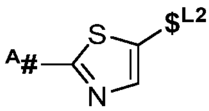

,

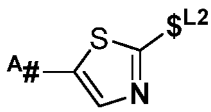

,

and

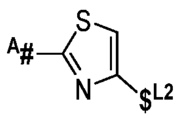

.

Preferably,

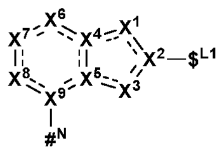

is selected from the group consisting of

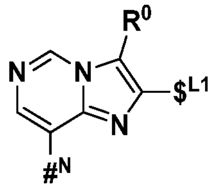

,

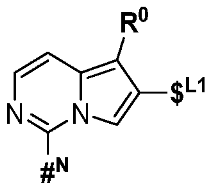, 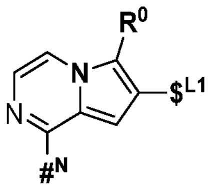, 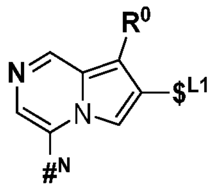, 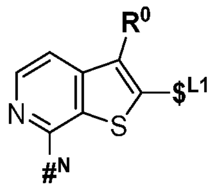, 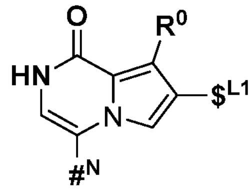,

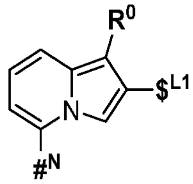, 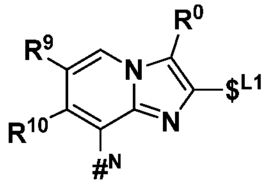, 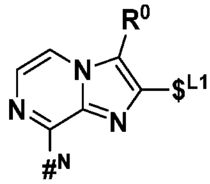, 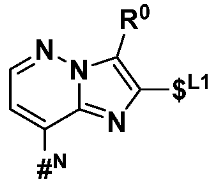, 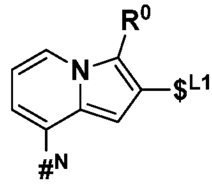,

and

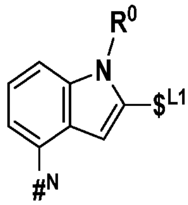;

$R^0$, $R^9$, $R^{10}$ have the definitions described herein.

More preferably,

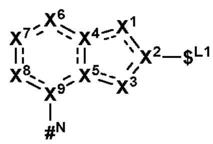

is selected from the group consisting of

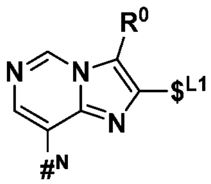, 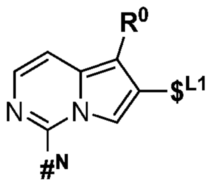, 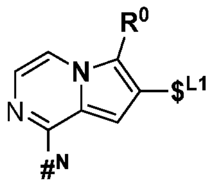, 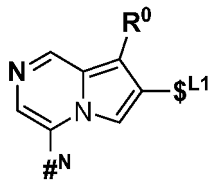, 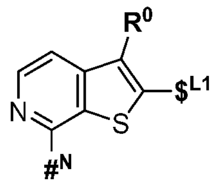,

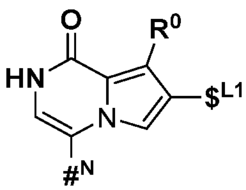, and 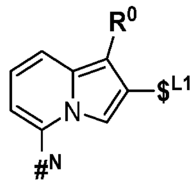;

$R^0$ has the definitions described herein.

More preferably,

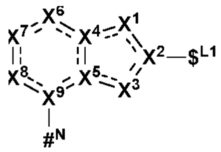

is selected from the group consisting of

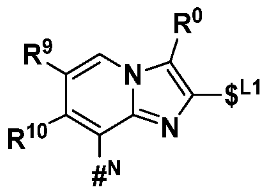 , 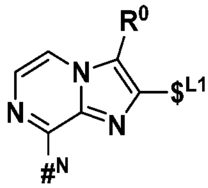 , 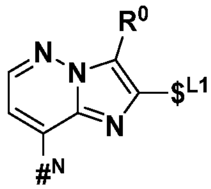 , 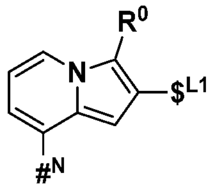 ,

and

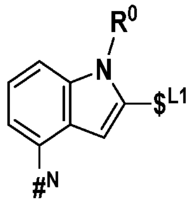 ;

$R^0$, $R^9$, $R^{10}$ have the definitions described herein.

Further preferably,

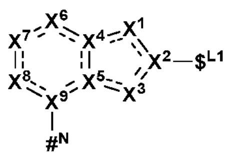

is selected from the group consisting of

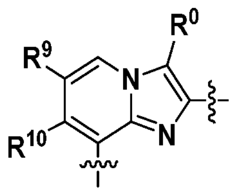 , and 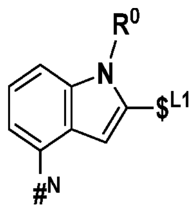 ;

$R^0$ is selected from the group consisting of C1-C6 alkyl, C1-C6 haloalkyl, cyano-C1-C6 alkylene-, C3-C6 cycloalkyl, and C3-C6 halocycloalkyl; $R^9$ is selected from the group consisting of hydrogen, and hydroxyl; $R^{10}$ is selected from the group consisting of hydrogen, and fluorine.

More preferably, $R^0$ is selected from the group consisting of C1-C4 alkyl, C1-C2 haloalkyl, and cyano-C1-C2 alkylene-.

Most preferably, $R^0$ is selected from the group consisting of $CF_3CH_2$-, $CH_2FCH_2$-, $CH_3CH_2CH_2$-, $CNCH_2$-, and $CHF_2CH_2$-; $R^9$ and $R^{10}$ are hydrogen.

Further preferably,

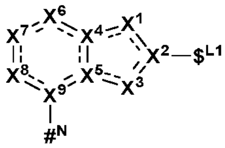

is selected from the group consisting of

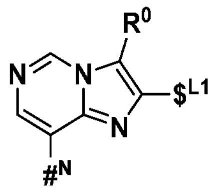 , 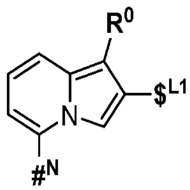 , 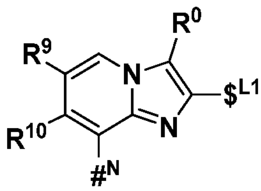 , and 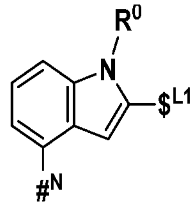 ;

$R^0$, $R^9$, $R^{10}$ have the definitions described herein.

More preferably, $R^0$ is selected from the group consisting of C1-C4 alkyl, C1-C2 haloalkyl, and cyano-C1-C2 alkylene-; $R^9$ and $R^{10}$ are each independently selected from the group consisting of hydrogen, deuterium, hydroxyl, amino, halogen, cyano, and C1-C3 alkyl.

More preferably, $R^0$ is selected from the group consisting of $CF_3CH_2$-, $CH_2FCH_2$-, $CH_3CH_2CH_2$-, $CNCH_2$-, and $CHF_2CH_2$-; $R^9$ and $R^{10}$ are hydrogen.

In some embodiments, the compound is represented by Formula MI-1-1:

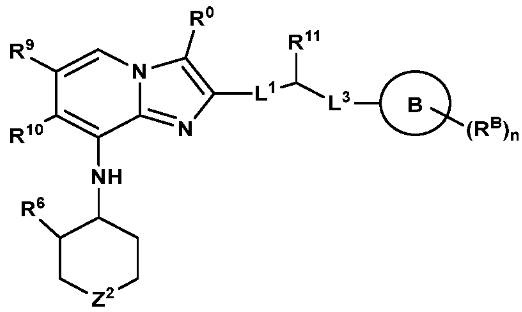

MI-1-1

wherein, $L^1$, $L^3$, Ring B, $R^B$, n, $R^0$, $R^6$, $R^{11}$, $Z^2$, $R^9$, $R^{10}$ have the definitions described herein.

In some embodiments, the compound is represented by Formula MI-1-1A or MI-1-1B:

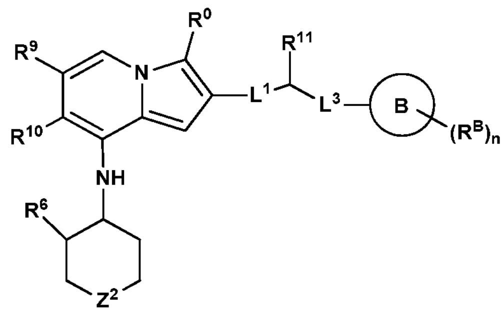

MI-1-1A

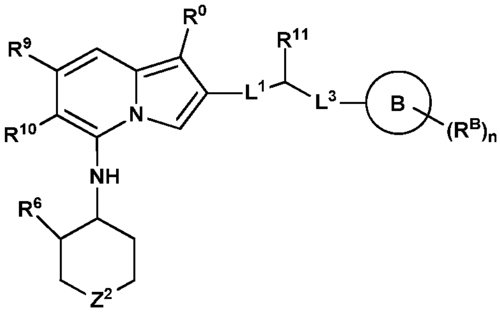

MI-1-1B

wherein, $L^1$, $L^3$, Ring B, $R^B$, n, $R^0$, $R^6$, $R^{11}$, $Z^2$, $R^9$, $R^{10}$ have the definitions described herein.

Preferably, $L^1$ is a 5-membered heteroarylene.

More preferably, $L^1$ is selected from the group consisting of

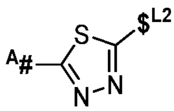

,

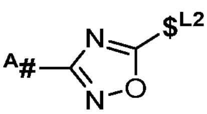 , 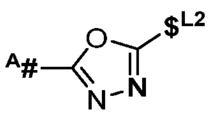 , 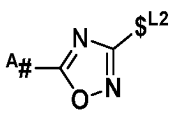 , 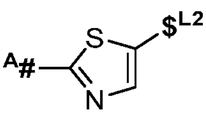 , 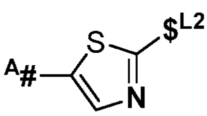 ,

and

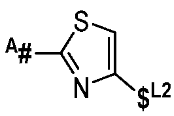

.

Preferably, $L^1$ is

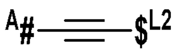

.

Preferably, $R^0$ is selected from the group consisting of C1-C4 alkyl, C1-C2 haloalkyl, and cyano-C1-C2 alkylene-.

Preferably, $R^9$ is selected from the group consisting of hydrogen and hydroxyl. Most preferably, $R^9$ is hydrogen.

Preferably, $R^{10}$ is selected from the group consisting of hydrogen and fluorine. Most preferably, $R^{10}$ is hydrogen.

Preferably, $R^{11}$ is selected from the group consisting of hydrogen and methyl.

Preferably, $L^3$ is NH.

Preferably, $Z^2$ is selected from the group consisting of $N(R^7)$, and $C(R^8)_2$;

$R^7$ is selected from the group consisting of hydrogen, and C1-C6 alkyl; wherein the C1-C6 alkyl is optionally substituted with halogen, hydroxyl, amino, cyano, C1-C6 alkoxy, halogen-substituted C1-C6 alkoxy, hydroxyl-substituted C1-C6 alkoxy, or amino-substituted C1-C6 alkoxy;

each $R^8$ is independently selected from the group consisting of hydrogen, halogen, hydroxyl, amino, cyano, C1-C6 alkyl, 4- to 8-membered saturated heterocyclyl, 7- to 11-membered spiro-heterocyclyl, 5- to 10-membered bridged heterocyclyl, and $R^oR^pN$-; wherein the C1-C6 alkyl, 4-to 8-membered saturated heterocyclyl, 7- to 11-membered spiro-heterocyclyl, 5- to 10-membered bridged heterocyclyl are optionally substituted with halogen, cyano, C1-C6 alkyl, halogen-substituted C1-C6 alkyl, hydroxyl-substituted C1-C6 alkyl, or amino-substituted C1-C6 alkyl; provided that: at most one $R^8$ is selected from the group consisting of 4- to 8-membered saturated heterocyclyl, 7- to 11-membered spiro-heterocyclyl, and 5- to 10-membered bridged heterocyclyl; or, two $R^8$ groups together with the carbon atom to which they connect form a 4- to 8-membered heterocyclyl; wherein the 4- to 8-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy,

hydroxyl-substituted C1-C6 alkoxy, or amino-substituted C1-C6 alkoxy;

$R^o$ and $R^p$ are each independently selected from the group consisting of hydrogen, deuterium, C1-C6 alkyl, and 4- to 8-membered saturated heterocyclyl; wherein the C1-C6 alkyl, 4- to 8-membered saturated heterocyclyl are optionally substituted with halogen, hydroxyl, amino, cyano, or C1-C6 alkoxy; or, $R^o$ and $R^p$ together with the N atom to which they connect form a 4- to 8-membered saturated heterocyclic ring, a 7- to 11-membered spiro-heterocyclic ring or a 5- to 10-membered bridged heterocyclic ring; wherein the 4- to 8-membered heterocyclic ring, 7- to 11-membered spiro-heterocyclic ring, 5- to 10-membered bridged heterocyclic ring are optionally substituted with halogen, cyano, C1-C6 alkyl, halogen-substituted C1-C6 alkyl, hydroxyl-substituted C1-C6 alkyl, or amino-substituted C1-C6 alkyl.

More preferably, $Z^2$ is selected from the group consisting of $N(R^7)$, and $C(R^8)_2$;

$R^7$ is selected from the group consisting of hydrogen, and C1-C3 alkyl;

one $R^8$ is hydrogen, and the other $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they connect form a 7- to 11-membered spiro-heterocyclic ring or a 5- to 10-membered bridged heterocyclic ring; wherein the 7- to 11-membered spiro-heterocyclyl, 7- to 11-membered spiro-heterocyclic ring, 5- to 10-membered bridged heterocyclyl, 5- to 10-membered bridged heterocyclic ring are optionally substituted with halogen, cyano, C1-C6 alkyl, halogen-substituted C1-C6 alkyl, hydroxyl-substituted C1-C6 alkyl, or amino-substituted C1-C6 alkyl; or, two $R^8$ groups together with the carbon atom to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy.

Further preferably, the 7- to 11-membered spiro-heterocyclyl is selected from the group consisting of

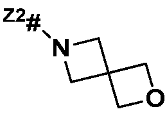, 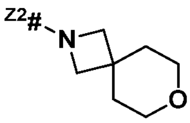, 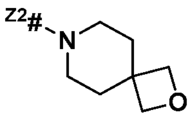, 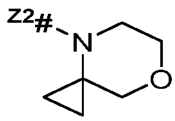, 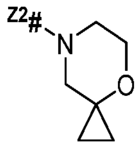,

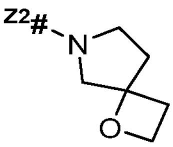, 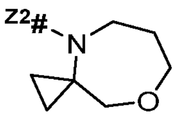, 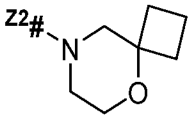, 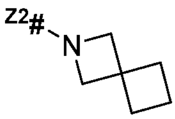, 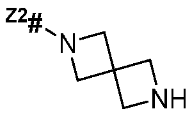, 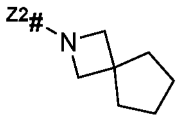,

and

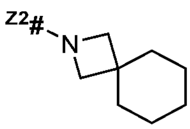;

the 5- to 10-membered bridged heterocyclyl is selected from the group consisting of

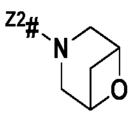, 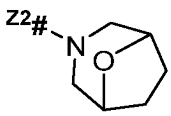, and 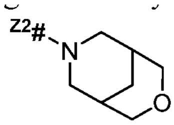,

**wherein,** $\#^{Z2}$ represents a connection point where $R^8$ is connected to the 6-membered ring where $Z^2$ is located.

Further preferably,

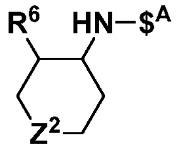

is selected from the group consisting of

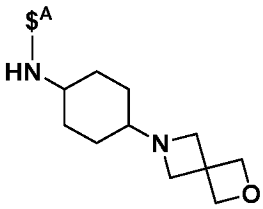, 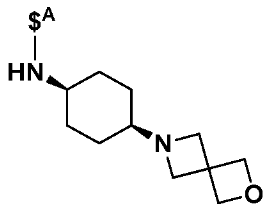, 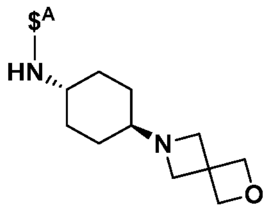, 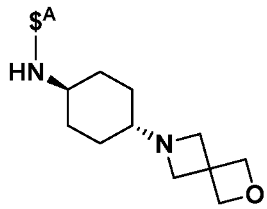,

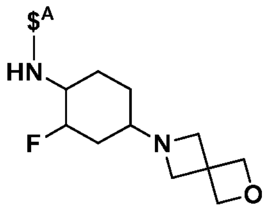, 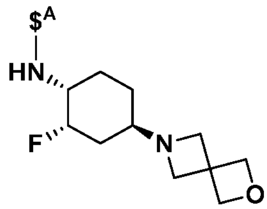, 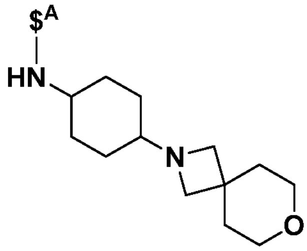, 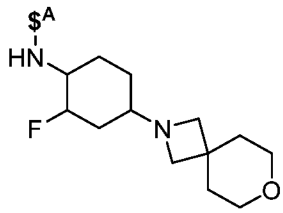,

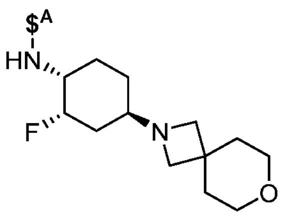, 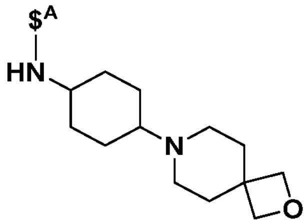, 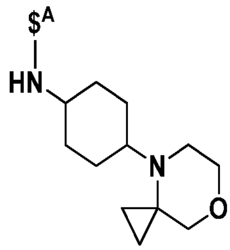, 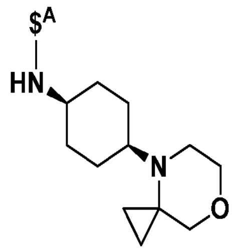,

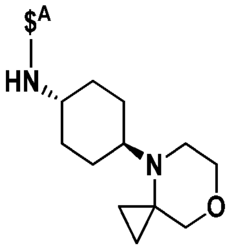, 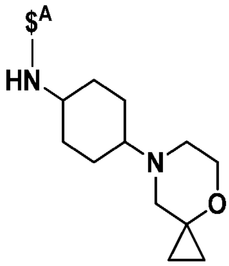, 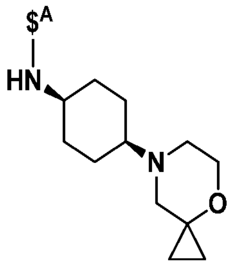, 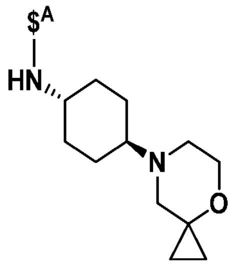,

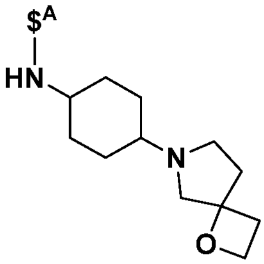, 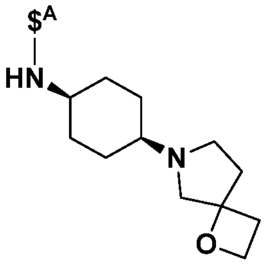, 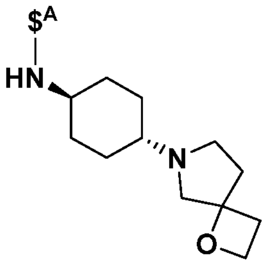, 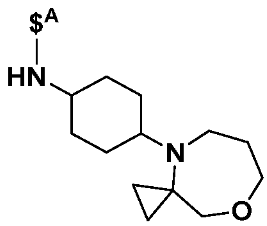,

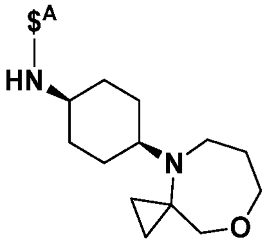, 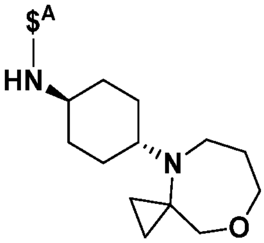, 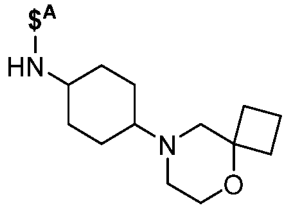, 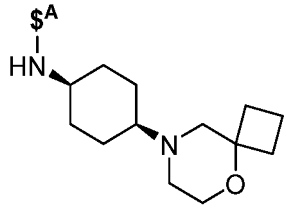,

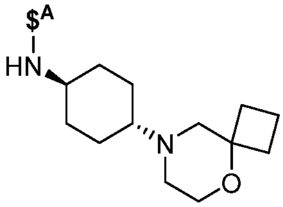, 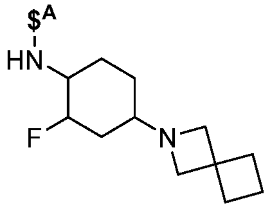, 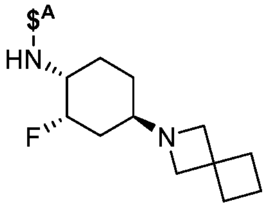, 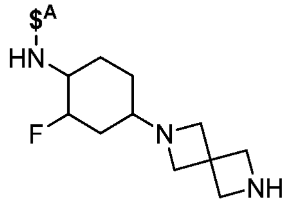,

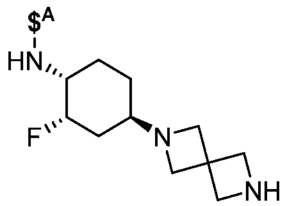, 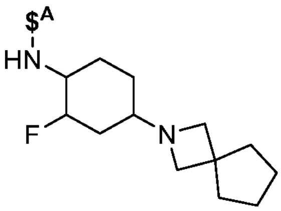, 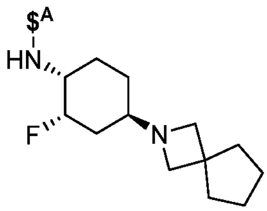, 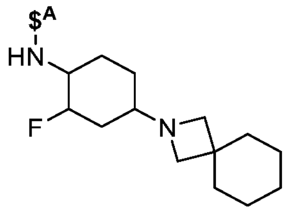,

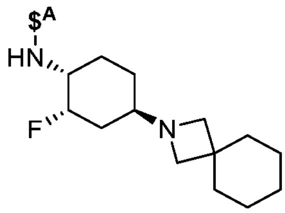, 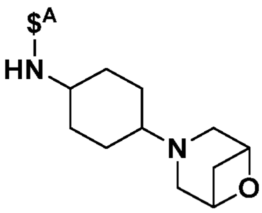, 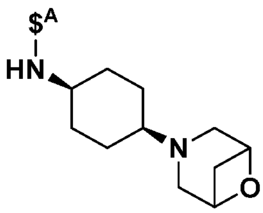, 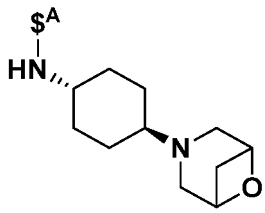,

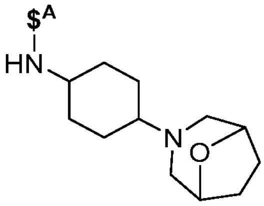, 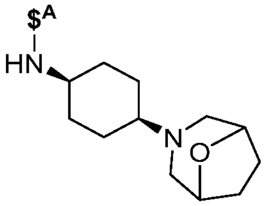, 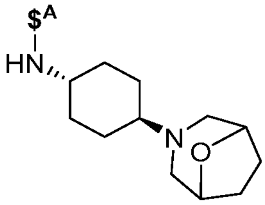, 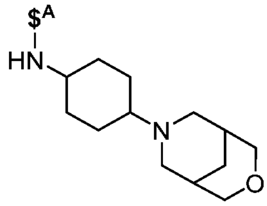,

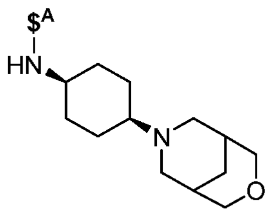, and 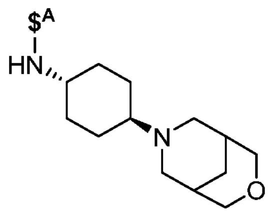;

wherein, $\$^A$ is a connection point between NH and Ring A.

Further preferably,

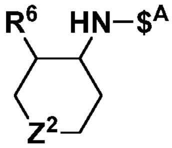

is selected from the group consisting of

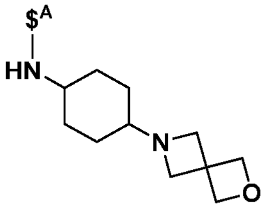, 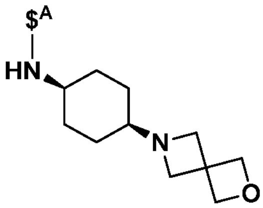, 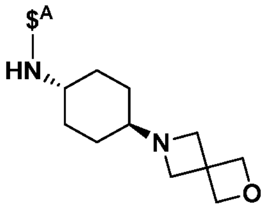, 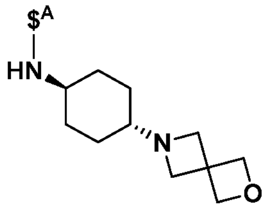,

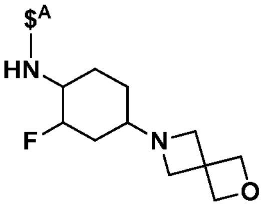, 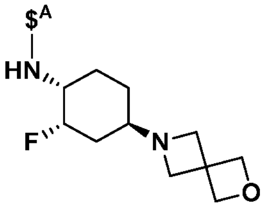, 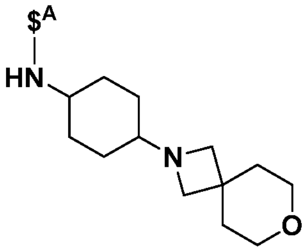, 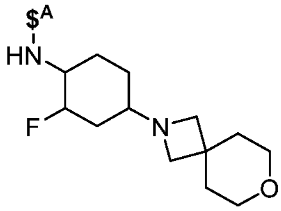,

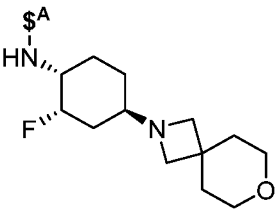, 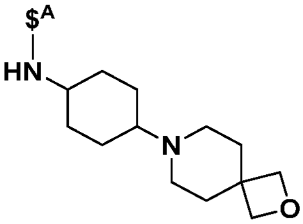, 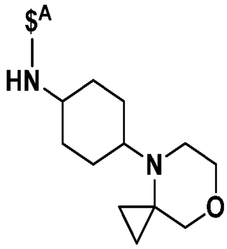, 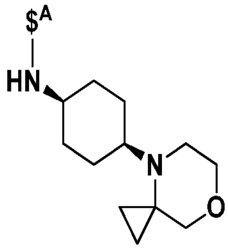,

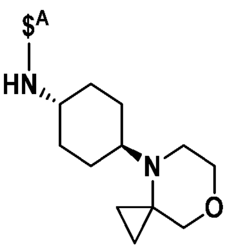, 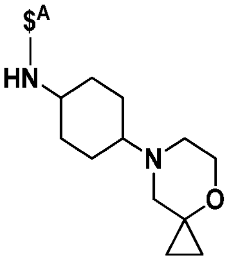, 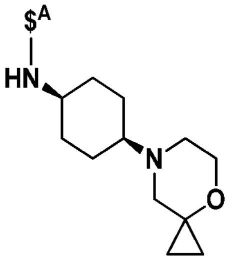, 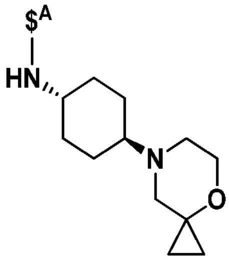,

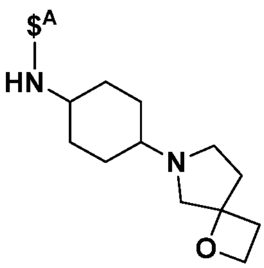, 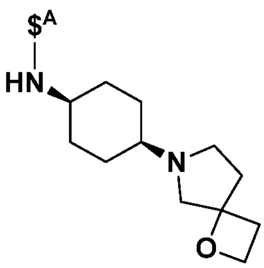, 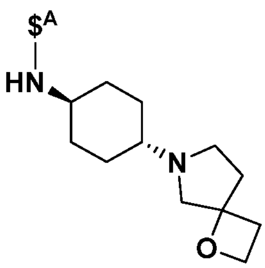, 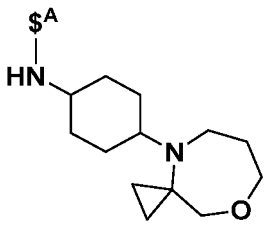,

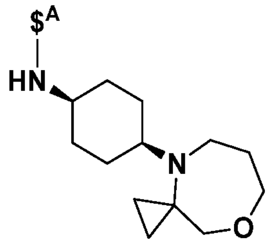, 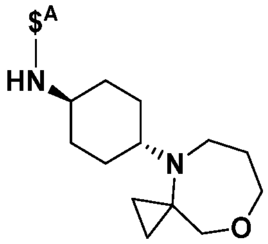, 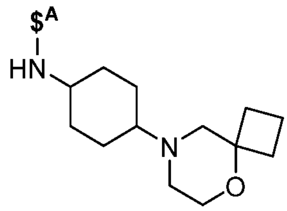, 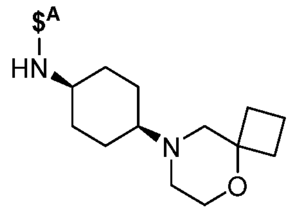,

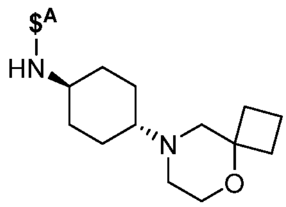, 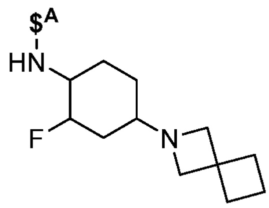, 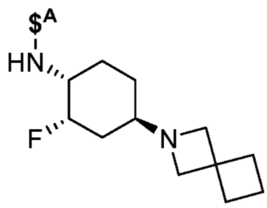, 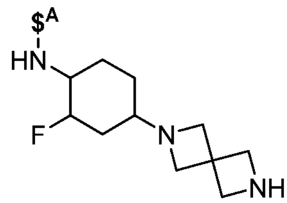,

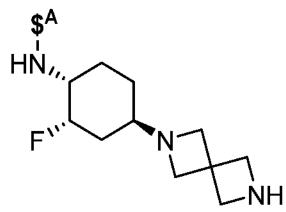, 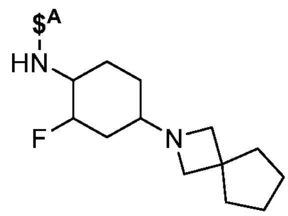, 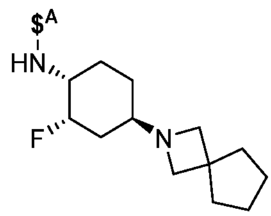, 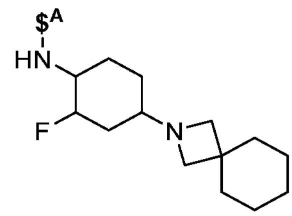,

and

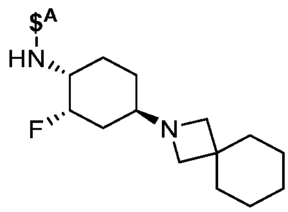

Further preferably,

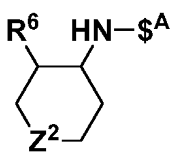

is selected from the group consisting of

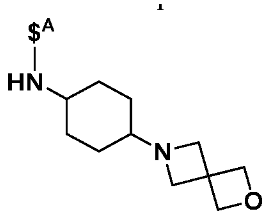, 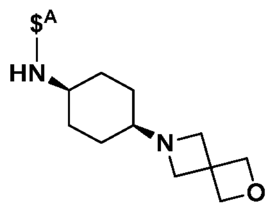, 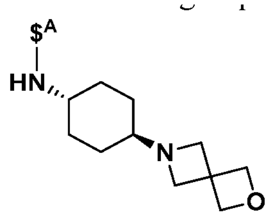, 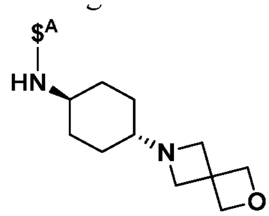,

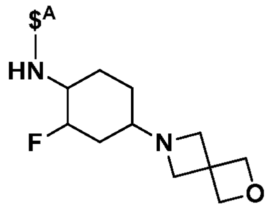, 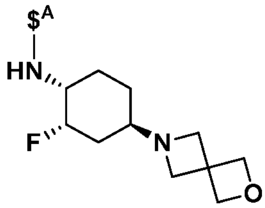, 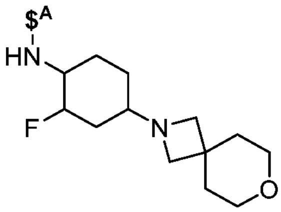, and 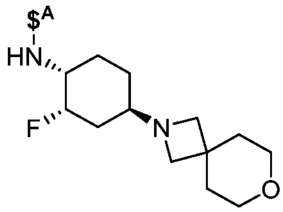.

Further preferably,

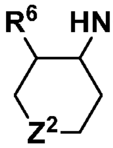

is selected from the group consisting of

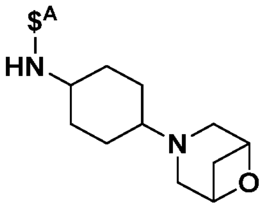, 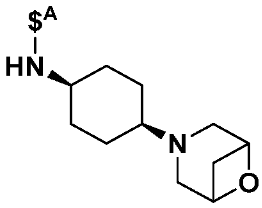, 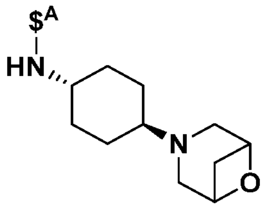, 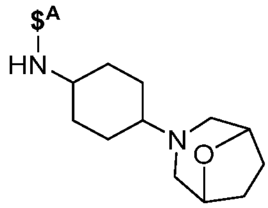,

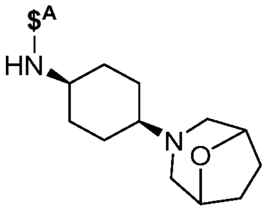, 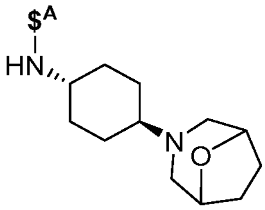, 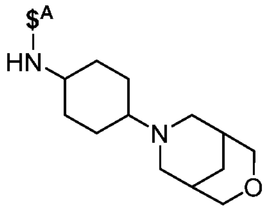, 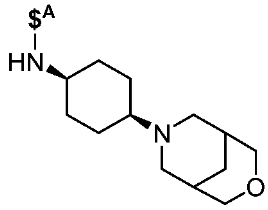,

and

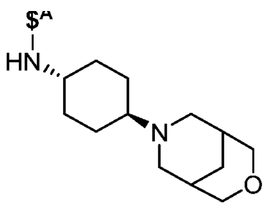.

Furder preferably, Ring B is selected from the grup consisting of phenyl, pyridyl, and 5-membered heteroaryl. More optimally, Ring B is selected from the group consisting of phenyl and pyridyl. Most preferably, Ring B is phenyl.

In some embodiments, the compound is represented by Formula MI-1-1-2, MI-1-1-2A, or MI-1-1-2B:

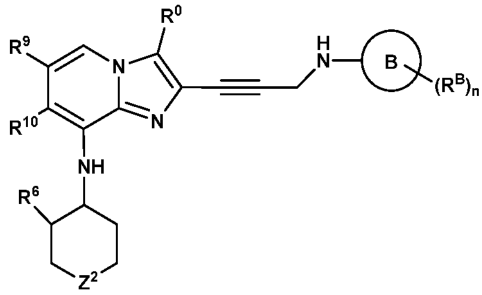

MI-1-1-2

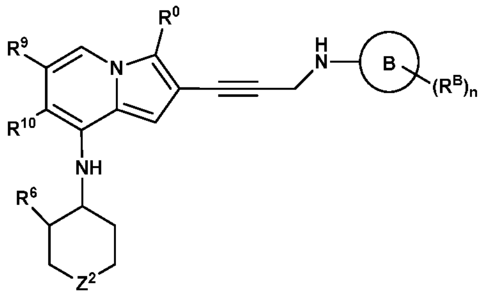

MI-1-1-2A

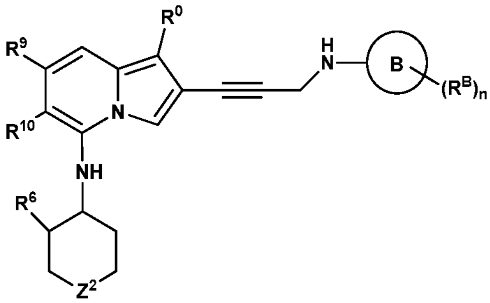

MI-1-1-2B

wherein, $R^0$, $R^6$, $R^9$, $R^{10}$, $Z^2$, n have the definitions described herein.

In some embodiments, the compound is represented by Formula MI-1-1-1, MI-1-1-1A or MI-1-1-1B:

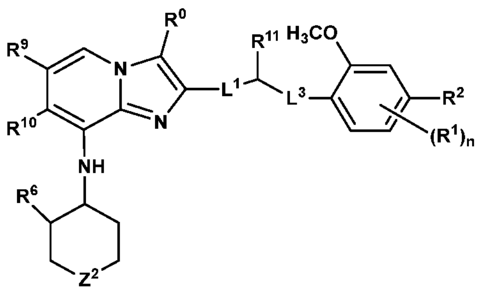

MI-1-1-1

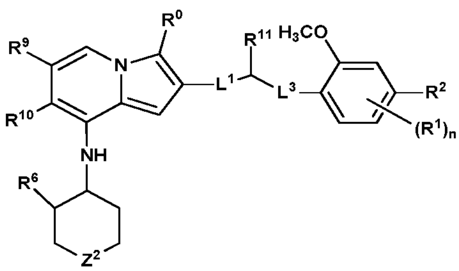

MI-1-1-1A

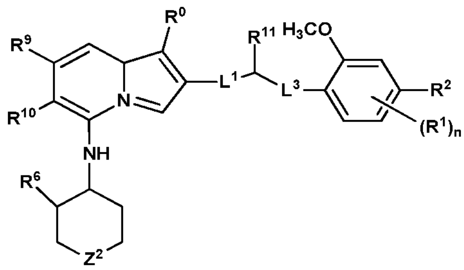

MI-1-1-1B

wherein, $R^0$, $R^1$, $R^2$, $R^6$, $R^9$, $R^{10}$, $R^{11}$, $L^1$, $L^3$, $Z^2$, n have the definitions described herein.

Preferably, $L^1$ is

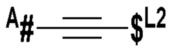

.

Preferably, $R^0$ is selected from the group consisting of C1-C4 alkyl, C1-C2 haloalkyl, and cyano-C1-C2 alkylene-.

Preferably, $R^1$ is selected from the group consisting of hydrogen, hydroxyl, halogen, C1-C2 haloalkyl, and $R^3O$-; $R^3$ is selected from the group consisting of hydrogen, C1-C4 alkyl, C1-C4 haloalkyl, cyano-C1-C2 alkylene-, hydroxyl-C1-C2 alkylene-, C1-C2 alkoxy-C1-C2 alkylene-, and C1-C2 alkyl-C(=O)-. More preferably, $R^1$ is selected from the group consisting of hydrogen, hydroxyl, and halogen. Most preferably, $R^1$ is selected from the group consisting of hydrogen and fluorine.

Preferably, $R^9$ is selected from the group consisting of hydrogen and hydroxyl. Most preferably, $R^9$ is hydrogen.

Preferably, $R^{10}$ is selected from the group consisting of hydrogen and fluorine. Most preferably, $R^{10}$ is hydrogen.

Preferably, $R^{11}$ is selected from the group consisting of hydrogen and methyl.

Preferably, $L^3$ is NH.

Preferably, $Z^2$ is $C(R^8)_2$;

each $R^8$ is independently selected from the group consisting of hydrogen, halogen, hydroxyl, amino, cyano, C1-C6 alkyl, 4- to 8-membered saturated heterocyclyl, 7- to 11-membered spiro-heterocyclyl, 5- to 10-membered bridged heterocyclyl, and $R^oR^pN$-; wherein the C1-C6 alkyl, 4-to 8-membered saturated heterocyclyl, 7- to 11-membered spiro-heterocyclyl, 5- to 10-membered bridged heterocyclyl are optionally substituted with halogen, cyano, C1-C6 alkyl, halogen-substituted C1-C6 alkyl, hydroxyl-substituted C1-C6 alkyl, or amino-substituted C1-C6 alkyl; provided that: at most one $R^8$ is selected from the group consisting of 4- to 8-membered saturated heterocyclyl, 7- to 11-membered spiro-heterocyclyl, and 5- to 10-membered bridged heterocyclyl; or, two $R^8$ groups together with the carbon atom to which they connect form a 4- to 8-membered heterocyclyl; wherein the 4- to 8-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

$R^o$, $R^p$ are each independently selected from the group consisting of hydrogen, deuterium, C1-C6 alkyl, and 4- to 8-membered saturated heterocyclyl; wherein the C1-C6 alkyl, 4- to 8-membered saturated heterocyclyl are optionally substituted with halogen, hydroxyl, amino, cyano, or C1-C6 alkoxy; or, $R^o$ and $R^p$ together with the N atom to which they connect form a 4-to 8-membered saturated heterocyclic ring, a 7- to 11-membered spiro-heterocyclic ring or a 5- to 10-membered bridged heterocyclic ring; wherein the 4- to 8-membered heterocyclic ring, 7- to 11-membered spiro-heterocyclic ring, 5- to 10-membered bridged heterocyclic ring are optionally substituted with halogen, cyano, C1-C6 alkyl, halogen-substituted C1-C6 alkyl, hydroxyl-substituted C1-C6 alkyl, or amino-substituted C1-C6 alkyl.

More preferably, $Z^2$ is $C(R^8)_2$;

one $R^8$ is hydrogen, and the other $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they connect form a 7- to 11-membered spiro-heterocyclic ring or a 5- to 10-membered bridged heterocyclic ring; wherein the 7- to 11-membered spiro-heterocyclyl, 7- to 11-membered spiro-heterocyclic ring, 5- to 10-membered bridged heterocyclyl, 5- to 10-membered bridged heterocyclic ring are optionally substituted with halogen, cyano, C1-C3 alkyl, halogen-substituted C1-C3 alkyl, hydroxyl-substituted C1-C3 alkyl, or amino-substituted C1-C3 alkyl; or, two $R^8$ groups together with the carbon atom to which they connect form a 4- to 7-membered heterocyclyl, wherein the 4- to 7-membered heterocyclyl is optionally substituted with halogen, hydroxyl, amino, cyano, C1-C3 alkyl, halogen-substituted C1-C3 alkyl, hydroxyl-substituted C1-C3 alkyl, amino-substituted C1-C3 alkyl, C1-C3 alkoxy, halogen-substituted C1-C3 alkoxy, hydroxyl-substituted C1-C3 alkoxy, or amino-substituted C1-C3 alkoxy.

Further preferably, the 7- to 11-membered spiro-heterocyclyl, 7- to 11-membered spiro-heterocyclic ring (i.e., the other $R^8$) is selected from the group consisting of

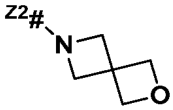,

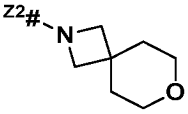, 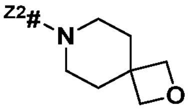, 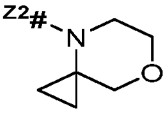, 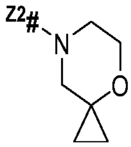, 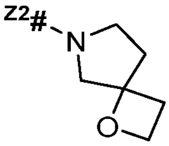, 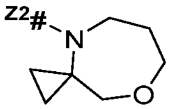,

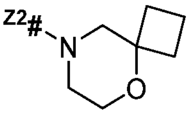, 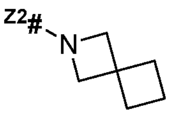, 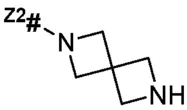, 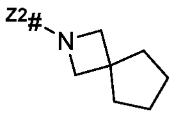, and 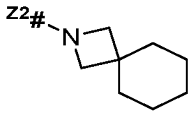;

the 5- to 10-membered bridged heterocyclyl, 5- to 10-membered bridged heterocyclic ring (i.e., the other $R^8$) is selected from the group consisting of

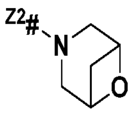, 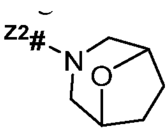,

and

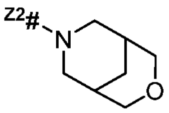;

wherein, $\#^{Z2}$ represents a connection point between $R^8$ and the 6-membered ring where $Z^2$ is located.

Further preferably,

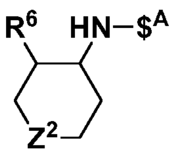

is selected from the group consisting of

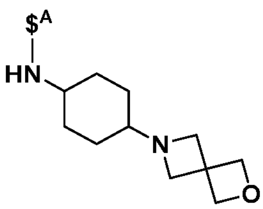, 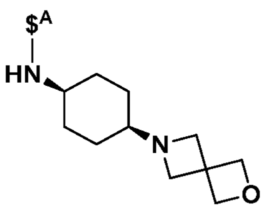, 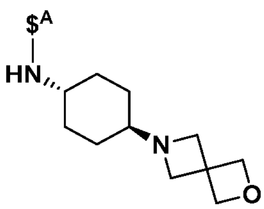, 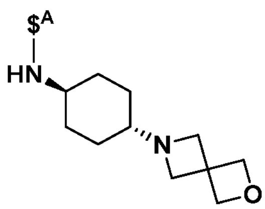,

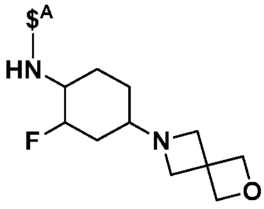, 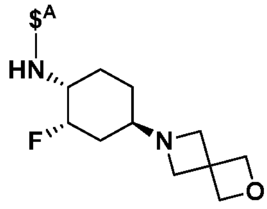, 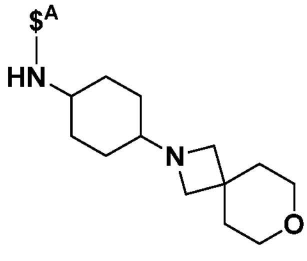, 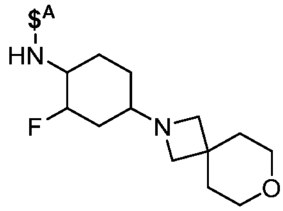,

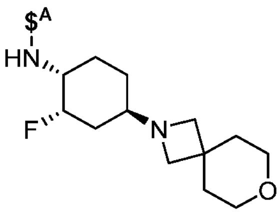, 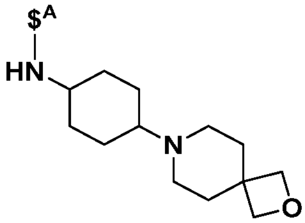, 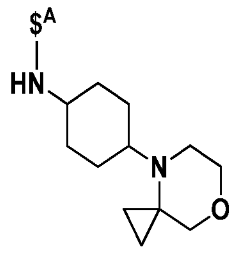, 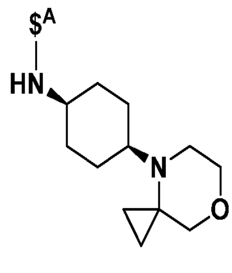,

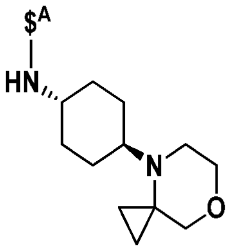, 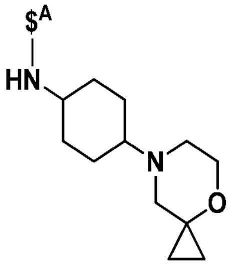, 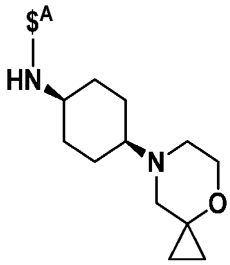, 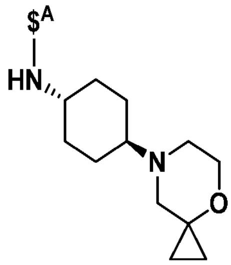,

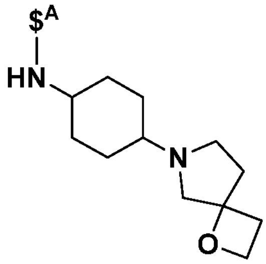, 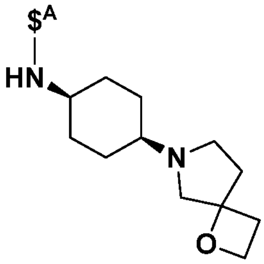, 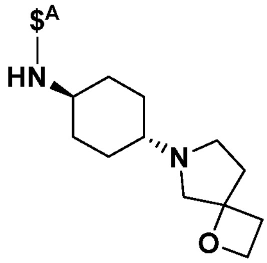, 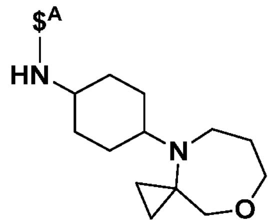,

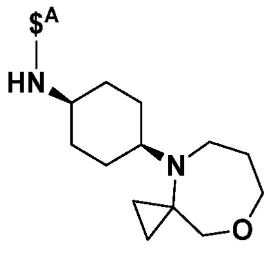, 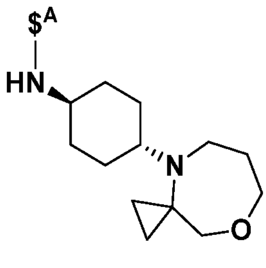, 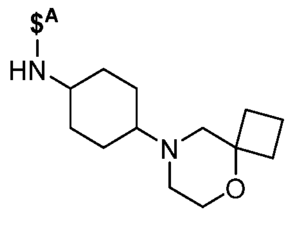, 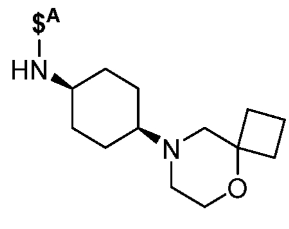,

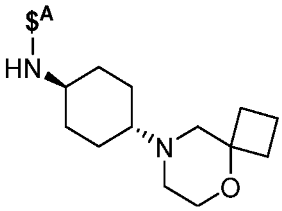 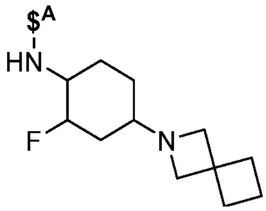 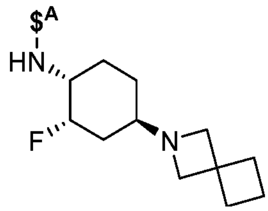 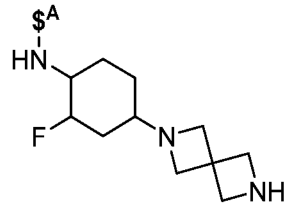

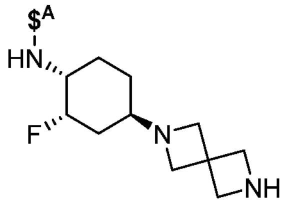, 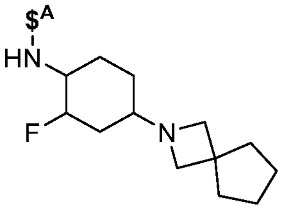, 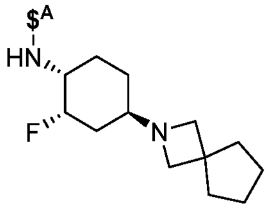, 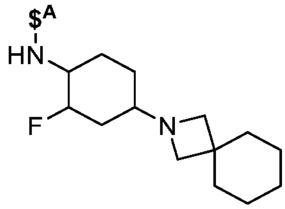,

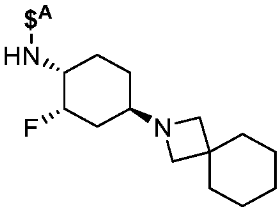, 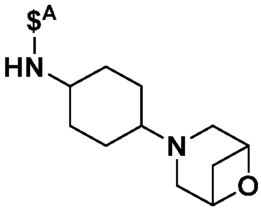, 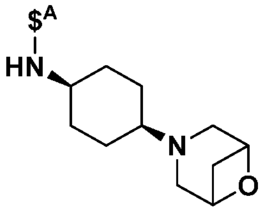, 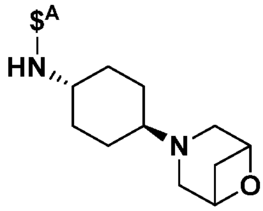,

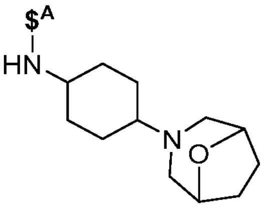, 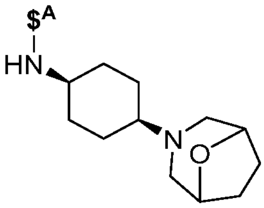, 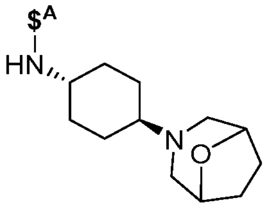, 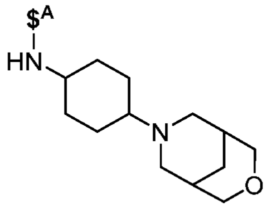,

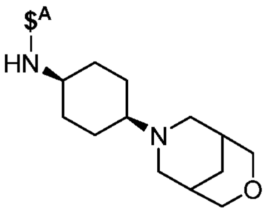, and 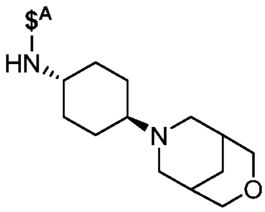;

wherein $^A represents a connection point between NH and Ring A.

More preferably,

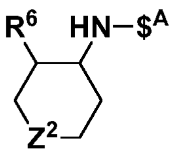

is selected from the group consisting of

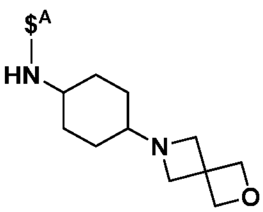, 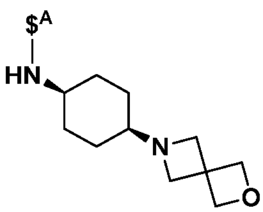, 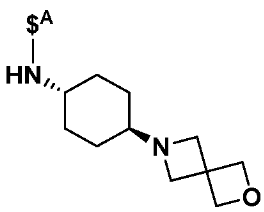, 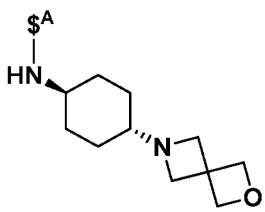,

, , , ,

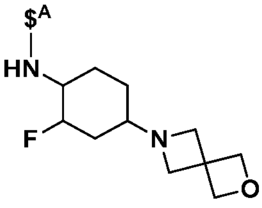, 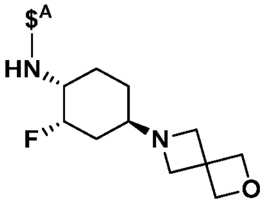, 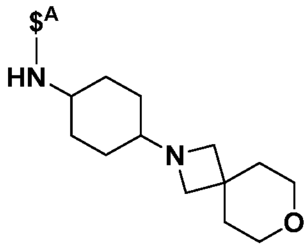, 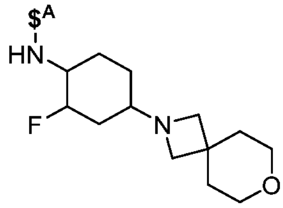,

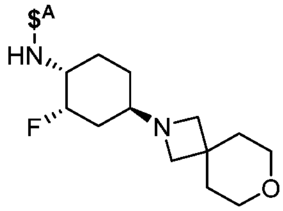, 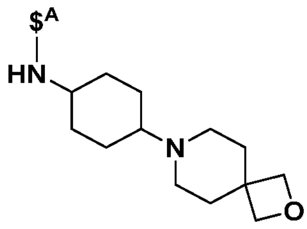, 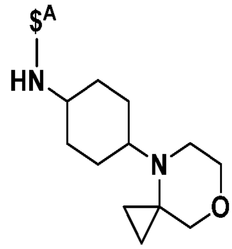, 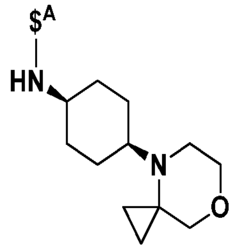,

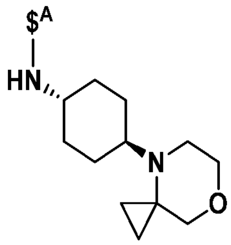, 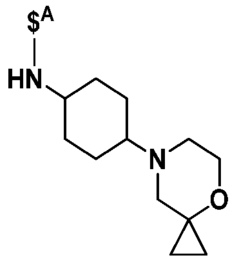, 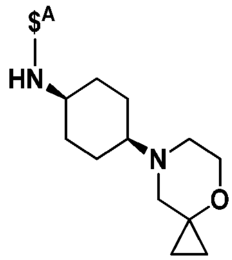, 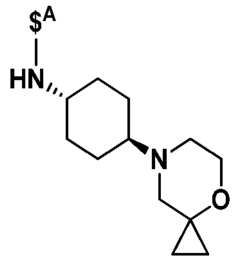,

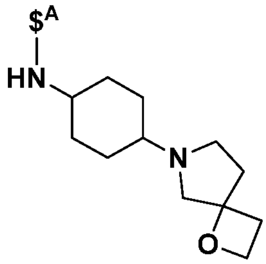, 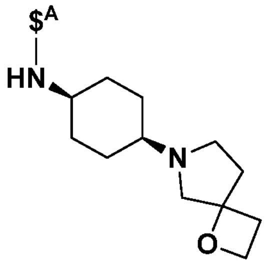, 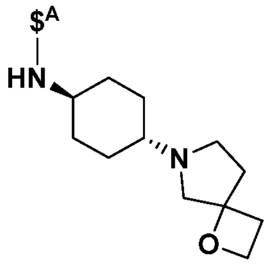, 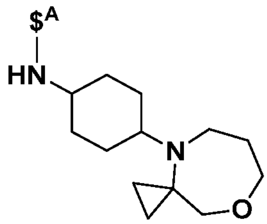,

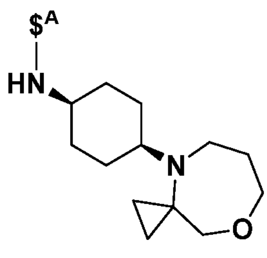, 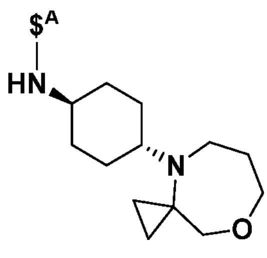, 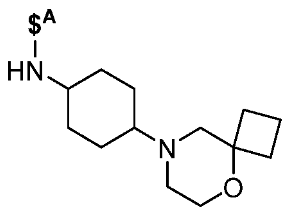, 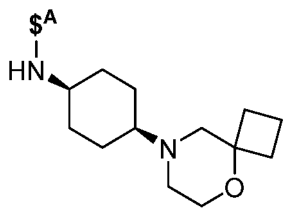,

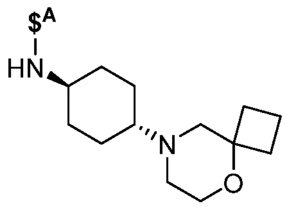, 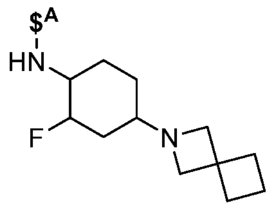, 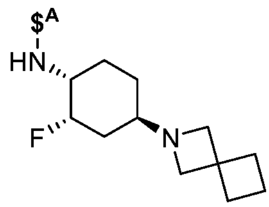, 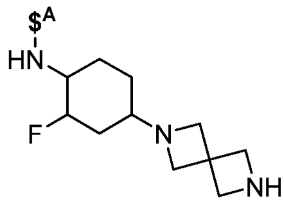,

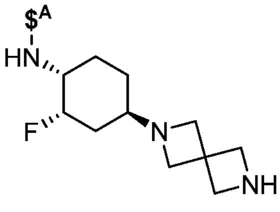, 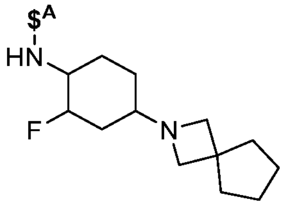, 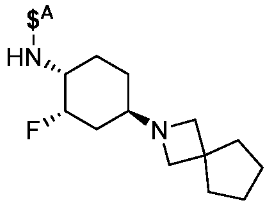, 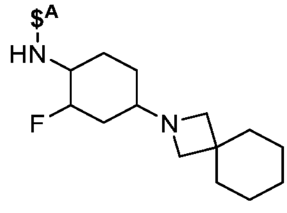,

and

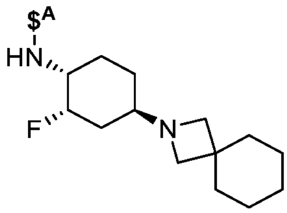.

More preferably,

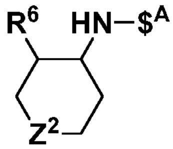

is selected from the group consisting of

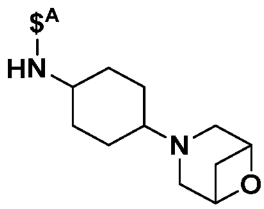, 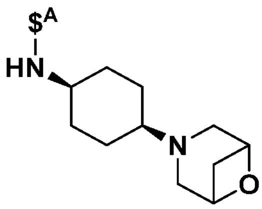, 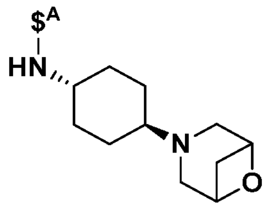, 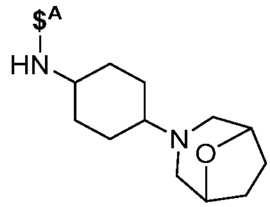,

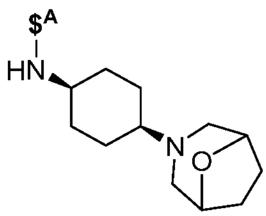, 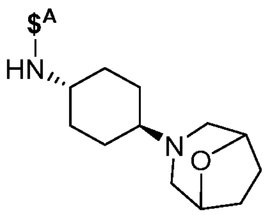, 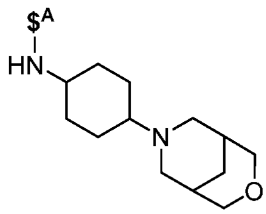, 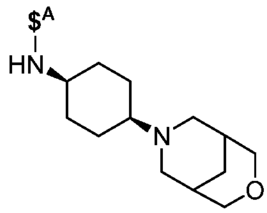,

and

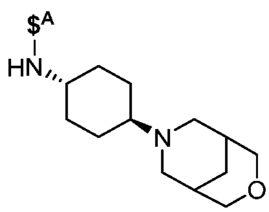.

More preferably,

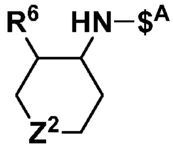

is selected from the group consisting of

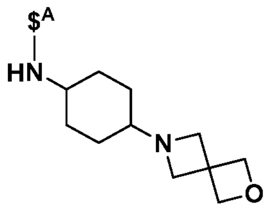, 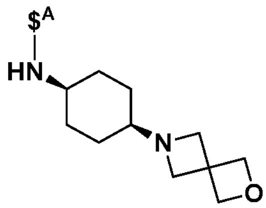, 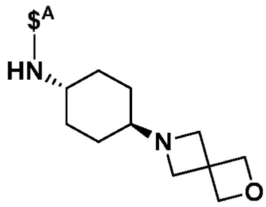, 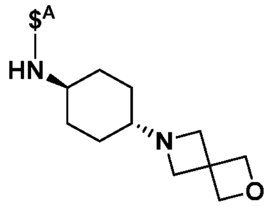,

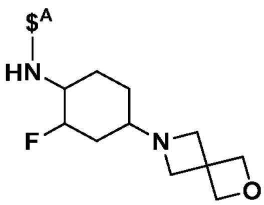, 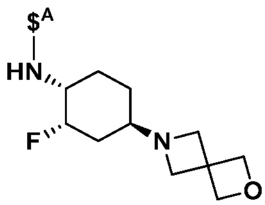, 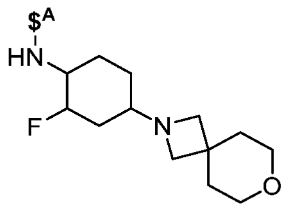, and 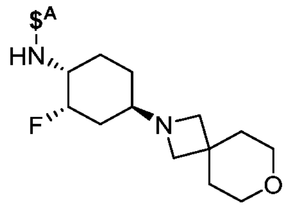.

Preferably, $R^2$ is selected from the group consisting of $R^hS(=O)_2$-,

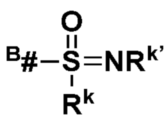,

and

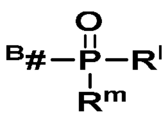;

$R^h$ is connected to the ortho position of $R^hS(=O)_2$- to form a 5- to 6-membered heterocyclic ring;

$R^k$, $R^{k'}$ together with -S=N- to which they connect form a 5- to 7-membered heterocyclic ring, or $R^{k'}$ is hydrogen, $R^k$ is connected to the ortho position of

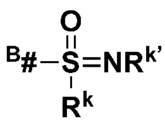

to form a 5- to 6-membered heterocyclic ring;

$R^l$ is C1-C6 alkyl (preferably C1-C3 alkyl), $R^m$ is connected at the ortho position of

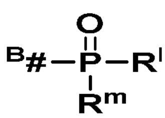

to form a 5- to 6-membered heterocyclic ring.

Preferably, $L^1$ is a 5-membered heteroarylene.

More preferably, $L^1$ is a 5-membered heteroarylene; $R^1$ is halogen, preferably fluorine and chlorine; n is 0 or 1; $R^2$ is selected from the group consisting of $R^hS(=O)_2$-,

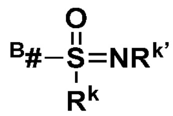
, and
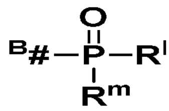
;

$R^h$ is connected to the ortho position of $R^hS(=O)_2$- to form a 5- to 6-membered heterocyclic ring;
$R^k$ $R^{k'}$ together with -S=N- to which they connect form a 5- to 7-membered heterocyclic ring, or $R^{k'}$ is hydrogen, and $R^k$ is connected to the ortho position of

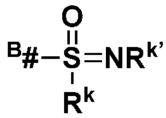

to form a 5-to 6-membered heterocyclic ring;
$R^1$ is C1-C6 alkyl (preferably C1-C3 alkyl), and $R^m$ is connected to the ortho position of

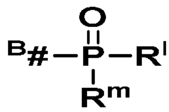

to form a 5- to 6-membered heterocyclic ring.

In some embodiments, the compound is represented by Formula T:

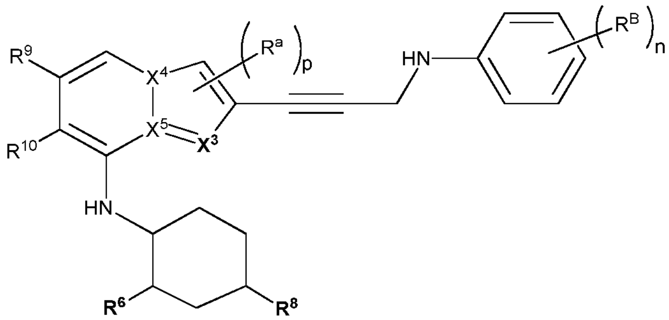

Formula T

In Formula T, $X^3$, $X^4$, $X^5$, $R^6$, $R^8$, $R^9$, $R^{10}$, $R^B$, $R^a$ are as defined in any one of claims 1-15, and n and p are independently 0, 1, 2 or 3; or

in Formula T, $X^3$ is CH or N, $X^4$ is CH or N, $X^5$ is CH or N, preferably, $X^4$ and/or $X^3$ are N;
$R^B$ is independently selected from the group consisting of -CONR$_{1-1}$R$_{1-2}$, -SO$_2$R$_{1-3}$, -O-C1-C6 alkyl, -O-C2-C6 alkenyl, -O-C1-C6 deuterated alkyl, -O-C1-C6 haloalkyl, -O-C1-C6 alkoxy, -O-C3-C6 cycloalkyl, -O-C3-C6 halocycloalkyl, and -O-C3-C6 heterocycloalkyl; preferably, $R^B$ is selected from the group consisting of -CONR$_{1-1}$R$_{1-2}$, -SO$_2$R$_{1-3}$, and O-C1-C6 deuterated alkyl;
$R_{1-1}$, $R_{1-2}$ are independently selected from the group consisting of H, deuterium, C1-C6 alkyl, -O-C1-C6 alkyl, -C1-C3 alkylene-O-C1-C3 alkyl, C1-C6 deuterated alkyl, C1-C6 haloalkyl, C3-C6 cycloalkyl, C3-C6 halocycloalkyl, 3- to 6-membered heterocycloalkyl, and 3- to 6-membered halogenated heterocycloalkyl; preferably H and C1-C6 deuterated alkyl; or $R_{1-1}$, $R_{1-2}$ together with the N atom they commonly connect form a 3- to 6-membered heterocycloalkyl, preferably, the 3- to 6-membered heterocycloalkyl is selected from the group consisting of

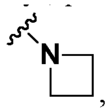,

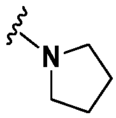, and 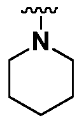;

$R_{1-3}$ is selected from the group consisting of H, C1-C6 alkyl, C1-C6 deuterated alkyl, C1-C6 haloalkyl, C3-C6 cycloalkyl, and C3-C6 halocycloalkyl;

$R^8$ is selected from the group consisting of $-NR_{2-1}R_{2-2}$, 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl;

$R_{2-1}$, $R_{2-2}$ are independently C1-C6 alkyl or 4- to 7-membered monoheterocycloalkyl;

$R^6$ is independently selected from the group consisting of H, halogen, preferably fluorine, C1-C6 alkyl, and C1-C6 haloalkyl;

$R^a$ is independently selected from the group consisting of C1-C6 alkyl, C1-C6 haloalkyl, cyano-C1-C6 alkylene-, C3-C6 cycloalkyl, and C3-C6 halocycloalkyl; preferably, $R^a$ is selected from the group consisting of C1-C4 alkyl, C1-C2 haloalkyl, and cyano-C1-C2 alkylene-; more preferably, $R^a$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;

$R^9$ and $R^{10}$ are each independently selected from the group consisting of hydrogen, deuterium, hydroxyl, amino, halogen, cyano, and C1-C3 alkyl.

In some embodiments, in Formula T, $R^B$ is independently selected from the group consisting of $-CONR_{1-1}R_{1-2}$, $-SO_2R_{1-3}$, -O-C1-C6 alkyl, -O-C1-C6 deuterated alkyl, -O-C1-C6 haloalkyl, - O-C3-C6 cycloalkyl, and -O-C3-C6 heterocycloalkyl;

$R_{1-1}$, $R_{1-2}$ are independently selected from the group consisting of H, C1-C6 alkyl, -C1-C3 alkyl-O-C1-C3 alkyl, -O-C1-C6 alkyl, C1-C6 deuterated alkyl, C1-C6 haloalkyl, C3-C6 cycloalkyl, and C3-C6 halocycloalkyl; or $R_{1-1}$, $R_{1-2}$ together with the N atom to which they connect form a 3- to 6-membered heterocycloalkyl, preferably, the 3- to 6-membered heterocycloalkyl is selected from the group consisting of

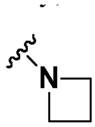, 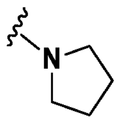, and 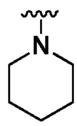;

$R_{1-3}$ is selected from the group consisting of H, and C1-C6 alkyl;

$R^8$ is selected from the group consisting of 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl;

$R^6$ is independently selected from the group consisting of H, halogen, preferably fluorine, C1-C6 alkyl, and C1-C6 haloalkyl;

$R^a$ is independently selected from the group consisting of C1-C6 alkyl, and C1-C6 haloalkyl; preferably, $R^a$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;

$R^9$ and $R^{10}$ are each independently selected from the group consisting of hydrogen and deuterium.

In some embodiments, in Formula T, $R^B$ is independently selected from the group consisting of $-CONR_{1-1}R_{1-2}$, $-SO_2R_{1-3}$, -O-C1-C6 alkyl, -O-C1-C6 deuterated alkyl, -O-C1-C6 haloalkyl, - O-C3-C6 cycloalkyl, and -O-C3-C6 halocycloalkyl; preferably, $R^1$ is selected from the group consisting of $-CONR_{1-1}R_{1-2}$, $-SO_2R_{1-3}$, and O-C1-C6 deuterated alkyl;

$R_{1-1}$, $R_{1-2}$ are independently selected from the group consisting of H, C1-C6 alkyl, -C1-C3 alkyl-O-C1-C3 alkyl, C1-C6 deuterated alkyl, C1-C6 haloalkyl, C3-C6 cycloalkyl, C3-C6 halocycloalkyl, 3- to 6-membered heterocycloalkyl, and 3- to 6-membered halogenated heterocycloalkyl; preferably H and C1-C6 deuterated alkyl; or $R_{1-1}$, $R_{1-2}$ together with the N atom to which they connect form a 3- to 6-membered heterocycloalkyl, preferably, the 3- to 6-membered

heterocycloalkyl is selected from the group consisting of

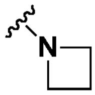, 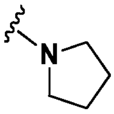,

and

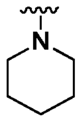;

$R_{1-3}$ is selected from the group consisting of H, C1-C6 alkyl, C1-C6 deuterated alkyl, C1-C6 haloalkyl, C3-C6 cycloalkyl, and C3-C6 halocycloalkyl alkyl;
$R^8$ is selected from the group consisting of $-NR_{2-1}R_{2-2}$, 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl;
$R_{2-1}$, $R_{2-2}$ are independently C1-C6 alkyl or 4- to 7-membered monoheterocycloalkyl;
$R^6$ is independently selected from the group consisting of H, halogen, preferably fluorine, C1-C6 alkyl, and C1-C6 haloalkyl;
$R^a$ is independently selected from the group consisting of C1-C6 alkyl, C1-C6 haloalkyl, cyano-C1-C6 alkylene-, C3-C6 cycloalkyl, and C3-C6 halocycloalkyl; preferably, $R^a$ is selected from the group consisting of C1-C4 alkyl, C1-C2 haloalkyl, and cyano-C1-C2 alkylene-; more preferably, $R^a$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;
$R^9$ and $R^{10}$ are each independently selected from the group consisting of hydrogen, deuterium, hydroxyl, amino, halogen, cyano, and C1-C3 alkyl.

In some embodiments, in Formula T,

$R^B$ is independently selected from the group consisting of $-CONR_{1-1}R_{1-2}$, $-SO_2R_{1-3}$, -O-C1-C6 alkyl, -O-C1-C6 deuterated alkyl, -O-C1-C6 haloalkyl, -O-C3-C6 cycloalkyl, and -O-C3-C6 heterocycloalkyl;
$R_{1-1}$, $R_{1-2}$ are independently selected from the group consisting of H, C1-C6 alkyl, -C1-C3 alkyl-O-C1-C3 alkyl, -O-C1-C6 alkyl, C1-C6 deuterated alkyl, C1-C6 haloalkyl, C3-C6 cycloalkyl, and C3-C6 halocycloalkyl; or $R_{1-1}$, $R_{1-2}$ together with the N atom to which they connect form a 3- to 6-membered heterocycloalkyl, preferably, the 3- to 6-membered heterocycloalkyl is selected from the group consisting of

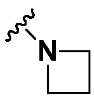, 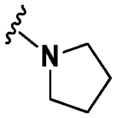, and 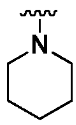;

$R_{1-3}$ selected from the group consisting of H, and C1-C6 alkyl;
$R^8$ is selected from the group consisting of 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl;
$R^6$ is independently selected from the group consisting of H, halogen, preferably fluorine, C1-C6 alkyl, and C1-C6 haloalkyl;
$R^a$ is independently selected from the group consisting of C1-C6 alkyl, and C1-C6 haloalkyl; preferably, $R^a$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;
$R^9$ and $R^{10}$ are each independently selected from the group consisting of hydrogen and deuterium.

In some embodiments, in Formula T,

$R^B$ is independently selected from the group consisting of $-CONR_{1-1}R_{1-2}$, $-SO_2R_{1-3}$, -O-C1-C6 alkyl, -O-C1-C6

deuterated alkyl, -O-C1-C6 haloalkyl, -O-C3-C6 cycloalkyl, and -O-C3-C6 halocycloalkyl; preferably, $R^B$ is selected from the group consisting of $-CONR_{1-1}R_{1-2}$, $-SO_2R_{1-3}$, and O-C1-C6 deuterated alkyl;

$R_{1-1}$, $R_{1-2}$ are independently selected from the group consisting of H, C1-C6 alkyl, -C1-C3 alkyl-O-C1-C3 alkyl, C1-C6 deuterated alkyl, C1-C6 haloalkyl, C3-C6 cycloalkyl, C3-C6 halocycloalkyl, 3- to 6-membered heterocycloalkyl, and 3- to 6-membered halogenated heterocycloalkyl; preferably H and C1-C6 deuterated alkyl; or $R_{1-1}$, $R_{1-2}$ together with the N atom to which they commonly connect form a 3- to 6-membered heterocycloalkyl, preferably, the 3- to 6-membered heterocycloalkyl is selected from the group consisting of

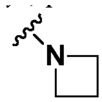,

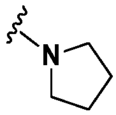, and 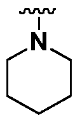;

$R_{1-3}$ is selected from the group consisting of H, C1-C6 alkyl, C1-C6 deuterated alkyl, C1-C6 halogenated alkyl, C3-C6 cycloalkyl, and C3-C6 halogenated cycloalkyl;

$R^8$ is selected from the group consisting of 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen.

Preferably, in Formula T,

$R^B$ is independently selected from the group consisting of $-CONR_{1-1}R_{1-2}$, $-SO_2R_{1-3}$, -O-C1-C3 alkyl, -O-C1-C3 deuterated alkyl, -O-C1-C3 haloalkyl, -O-C3-C6 cycloalkyl; preferably, $R^1$ is selected from the group consisting of $-CONR_{1-1}R_{1-2}$, $-SO_2R_{1-3}$, and O-C1-C3 deuterated alkyl;

$R_{1-1}$, $R_{1-2}$, $R_{1-3}$ are independently selected from the group consisting of H, C1-C3 alkyl, C1-C3 deuterated alkyl, C1-C3 haloalkyl, and C3-C6 cycloalkyl; preferably H and C1-C3 deuterated alkyl;

$R^6$ is independently selected from the group consisting of H, halogen, C1-C3 alkyl, and C1-C3 haloalkyl;

$R^a$ is independently selected from the group consisting of halogen, C1-C3 alkyl, and C1-C3 haloalkyl;

$R^9$ and $R^{10}$ are each independently selected from the group consisting of hydrogen and deuterium.

The Formula T is selected from the group consisting of Formula T-1 to T-9:

| | |
|---|---|
| 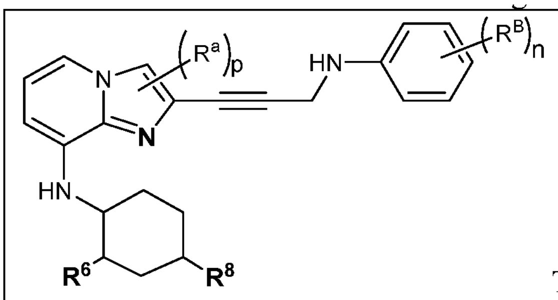 T-1 | 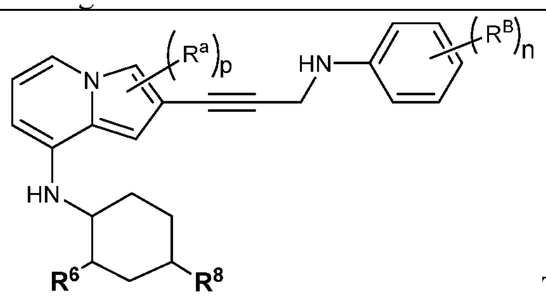 T-2 |
| 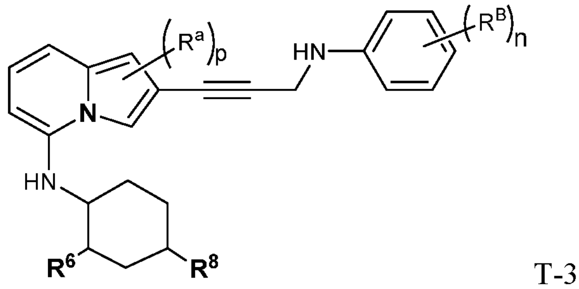 T-3 | 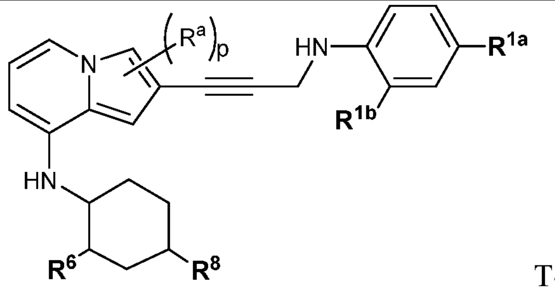 T-4 |
| 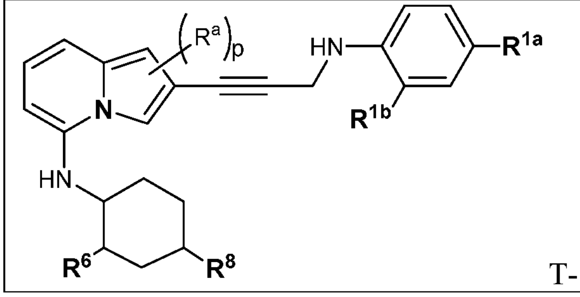 T-5 | 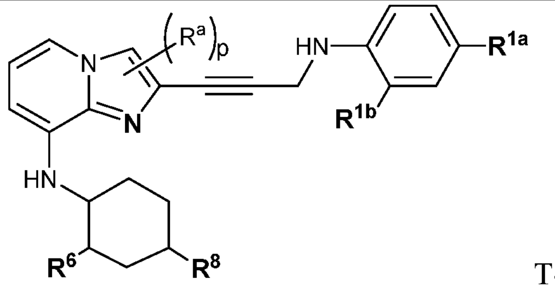 T-6 |
| 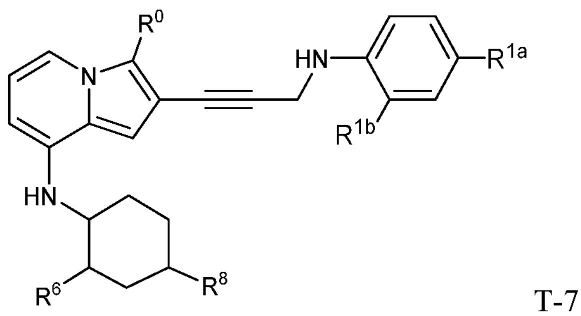 T-7 | 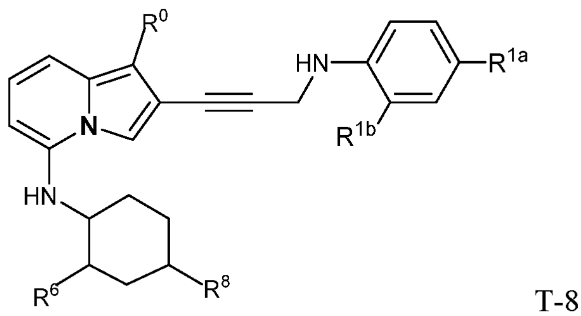 T-8 |
| 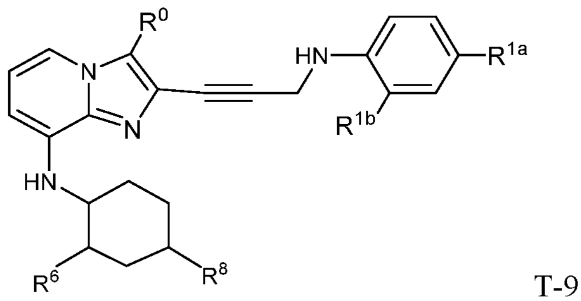 T-9 | |

In Formula T-1 to Formula T9, the definitions of symbols are the same as those of Formula T; $R^{1a}$ and $R^{1b}$ are defined as $R^B$ in Formula T, and $R^{1a}$ and $R^{1b}$ are different;

preferably, $R^{1a}$ is selected from the group consisting of $-CONR_{1-1}R_{1-2}$, and $-SO_2R_{1-3}$; further preferably, $R^{1a}$ is selected from the group consisting of $-CONHCD_3$, $-CONHCH_3$, $-SO_2CH_3$, and $-SO_2CD_3$;

preferably, $R^0$ is independently selected from the group consisting of C1-C6 alkyl, and C1-C6 haloalkyl; more preferably, $R^0$ is independently selected from the group consisting of halogen, C1-C3 alkyl, and C1-C3 haloalkyl; more

preferably, $R^0$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;

preferably, $R^{1b}$ is selected from the group consisting of -O-C1-C6 alkyl, -O-C1-C6 haloalkyl, -O-C1-C6 deuterated alkyl, and -O-C3-C6 cycloalkyl; further preferably, $R^{1b}$ is selected from the group consisting of $-OCH_3$, $-OD_3$, $-OCH_2F$, and

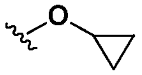.

In Formula T, Formula T-1 to Formula T9, preferably, $R^8$ is selected from the group consisting of

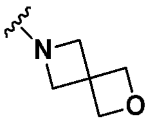, 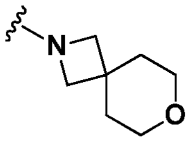, 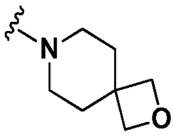, 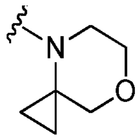, 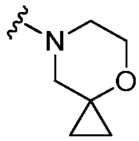, 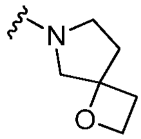,

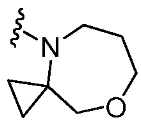, 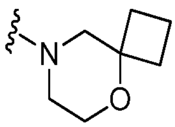, 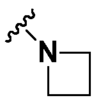, 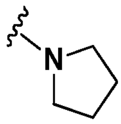, 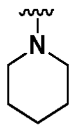, 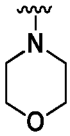, 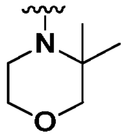, 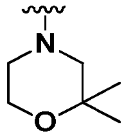, 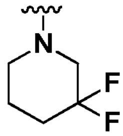,

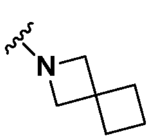, 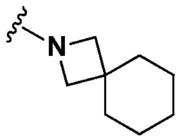, and 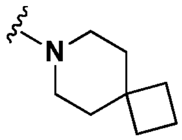;

in Formula T, Formula T-1 to Formula T11, preferably, $R^8$ is selected from the group consisting of

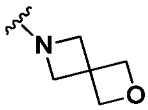, 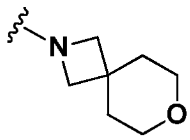, 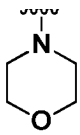, and 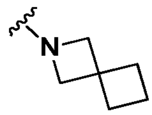;

in Formula T, Formula T-1 to Formula T11, preferably, $R^6$ is halogen, further preferably fluorine.

In some embodiments, the compound is represented by Formula MII:

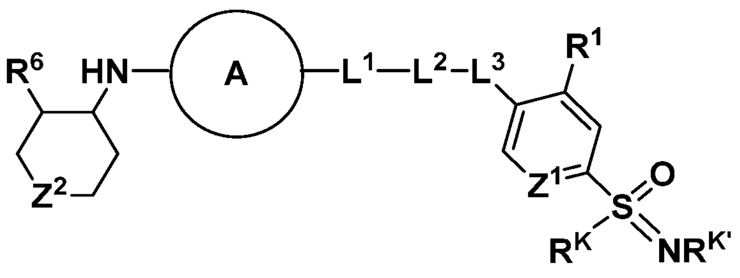
MII

wherein, Ring A, $L^1$, $L^2$, $L^3$, $R^1$, $R^k$, $R^{k'}$, $R^6$, $Z^1$, and $Z^2$ have the definitions described herein.

Preferably, $R^k$, $R^{k'}$ together with -S=N- to which they connect form a 4- to 8-membered heterocyclic ring, or $R^k$ is connected to the ortho position of

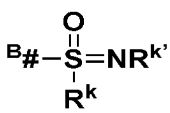

to form a 5- to 6-membered heterocyclic ring. Preferably, Ring A is selected from the group consisting of

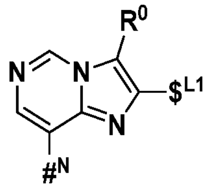,

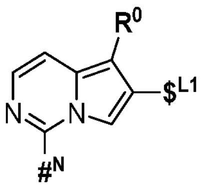, 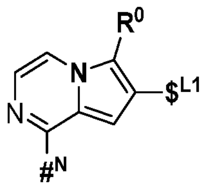, 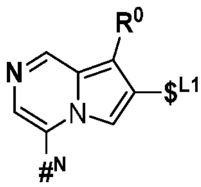, 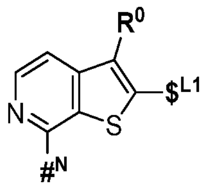, 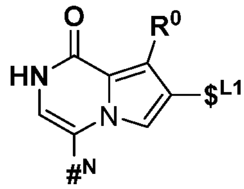,

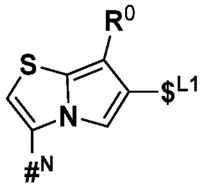, 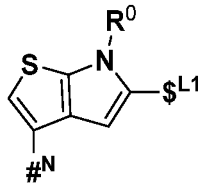, 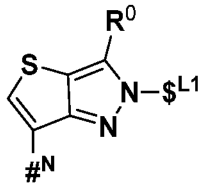, 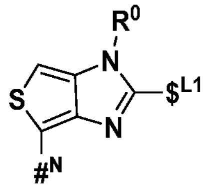, 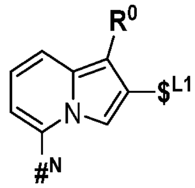,

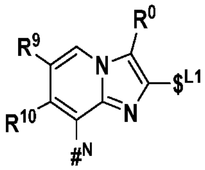, 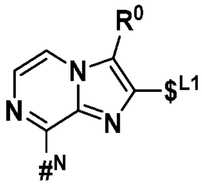, 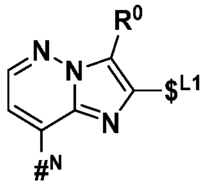, 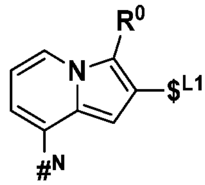, and 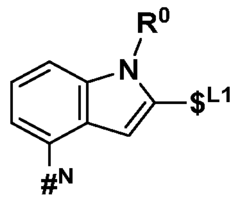;

$R^9$ and $R^{10}$ are each independently selected from the group consisting of hydrogen, deuterium, hydroxyl, amino, halogen, cyano, and C1-C3 alkyl.

More preferably, Ring A is selected from the group consisting of

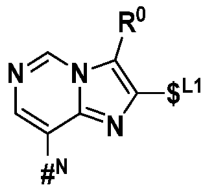,

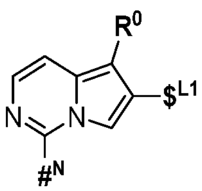, 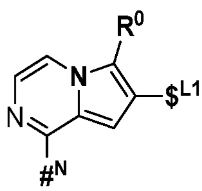, 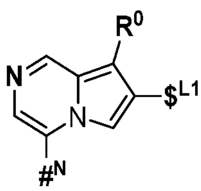, 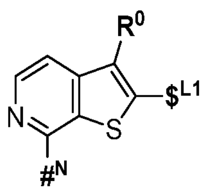, 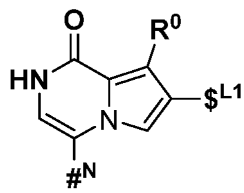,

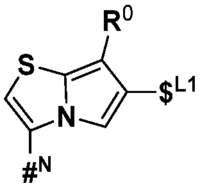, 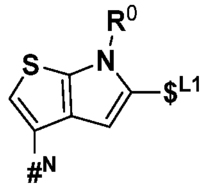, 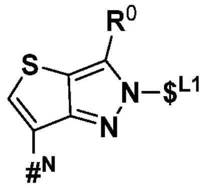, 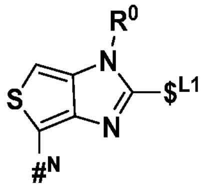, and 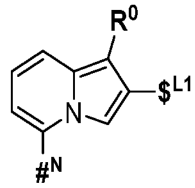.

More preferably, Ring A is selected from the group consisting of

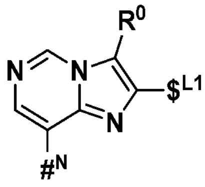,

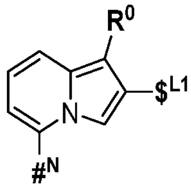, 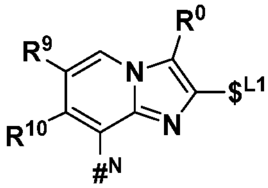, and 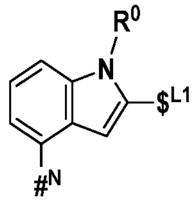.

Preferably, $L^1$ is

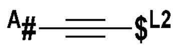;

preferably, $L^2$ is selected from the group consisting of $CH_2$, and $CH(CH_3)$;
preferably, $L^3$ is NH;
preferably, $Z^1$ is selected from the group consisting of N, and $CR^4$; $R^4$ is selected from the group consisting of hydrogen, and halogen;
more preferably, $Z^1$ is selected from the group consisting of N, CH, and CF;
preferably, $R^1$ is selected from the group consisting of hydrogen, hydroxyl, halogen, C1-C4 alkyl, and $R^3O$-; wherein the C1-C4 alkyl is optionally substituted with 1, 2 or 3 substituents selected from the group consisting of halogen and hydroxyl;
$R^3$ is independently selected from the group consisting of hydrogen, C1-C4 alkyl, and C1-C4 alkyl-C(=O)-; wherein the C1-C4 alkyl is optionally substituted with halogen, hydroxyl, cyano, amino, or C1-C4 alkoxy;
more preferably, $R^1$ is selected from the group consisting of hydrogen, hydroxyl, F, and $R^3O$-;
$R^3$ is independently selected from the group consisting of hydrogen, C1-C3 alkyl, and C1-C3 alkyl-C(=O)-; wherein the C1-C3 alkyl is optionally substituted with F, hydroxyl, cyano, or C1-C3 alkoxy;
most preferably, $R^1$ is methoxy.

In some embodiments, the compound is represented by Formula MII-1, MII-2 or MII-3:

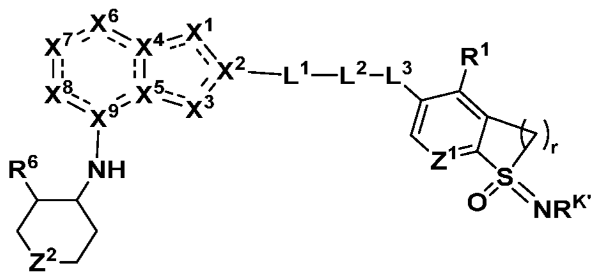

MII-1

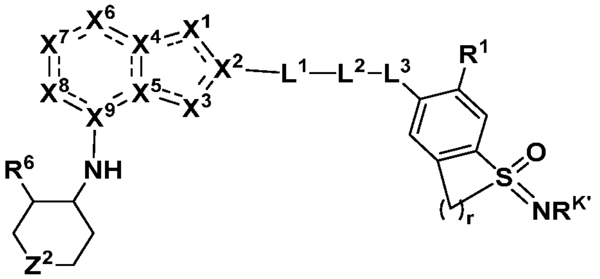

MII-2

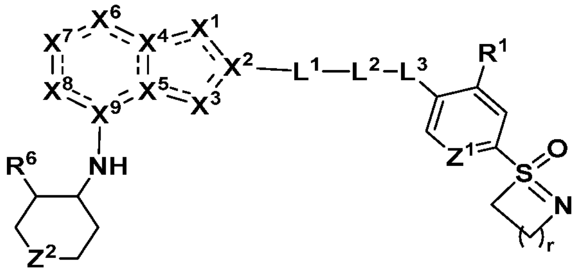

MII-3

wherein, $X^1$, $X^2$, $X^3$, $X^4$, $X^5$, $X^6$, $X^7$, $X^8$, $X^9$, $L^1$, $L^2$, $L^3$, $R^1$, $R^{k'}$, $R^6$, $Z^1$, $Z^2$ have the definitions described herein; r is selected from the group consisting of 1, 2, 3 and 4.

Preferably,

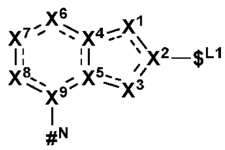

is selected from the group consisting of

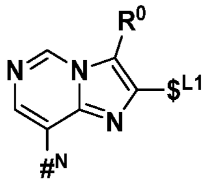

,

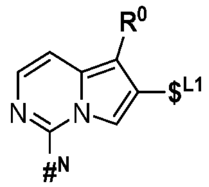

,

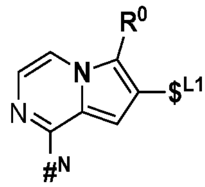

,

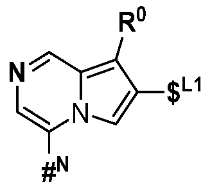

,

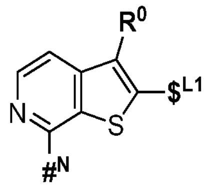

,

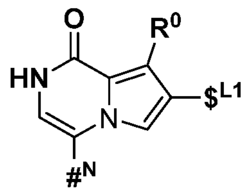

,

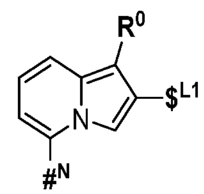

,

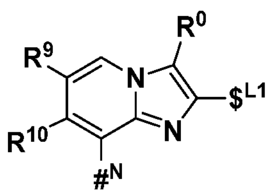

,

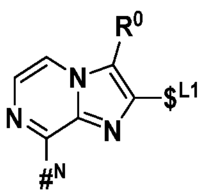

,

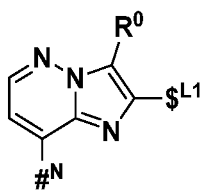

,

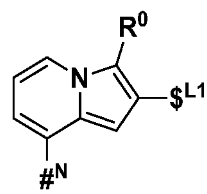

,

and

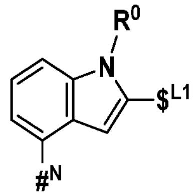
;

$R^0$, $R^9$, $R^{10}$ have the definitions described herein.

More preferably,

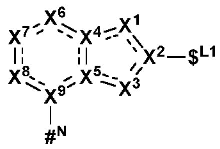

is selected from the group consisting of

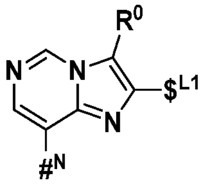
, 
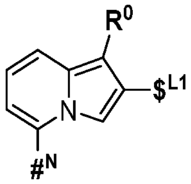
, 
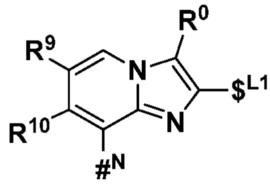
, and 
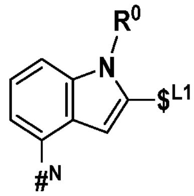
.

Preferably, $L^1$ is

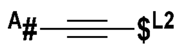
.

Preferably, $L^2$ is selected from the group consisting of $CH_2$, and $CH(CH_3)$.

Preferably, $L^3$ is NH.

Preferably, $Z^1$ is selected from the group consisting of N, and $CR^4$; $R^4$ is selected from the group consisting of hydrogen, and halogen.

More preferably, $Z^1$ is selected from the group consisting of N, CH, and CF.

Preferably, $R^1$ is selected from the group consisting of hydrogen, hydroxyl, halogen, C1-C4 alkyl, and $R^3O$-; wherein the C1-C4 alkyl is optionally substituted with 1, 2 or 3 substituents selected from the group consisting of halogen and hydroxyl;

$R^3$ is independently selected from the group consisting of hydrogen, C1-C4 alkyl, and C1-C4 alkyl-C(=0)-; wherein the C1-C4 alkyl is optionally substituted with halogen, hydroxyl, cyano, amino, or C1-C4 alkoxy.

More preferably, $R^1$ is selected from the group consisting of hydrogen, hydroxyl, F, and $R^3O$-;

$R^3$ is independently selected from the group consisting of hydrogen, C1-C3 alkyl, and C1-C3 alkyl-C(=0)-; wherein the C1-C3 alkyl is optionally substituted with F, hydroxyl, cyano, or C1-C3 alkoxy.

Most preferably, $R^1$ is methoxy.

Preferably, r is selected from the group consisting of 1, 2 and 3.

In some embodiments, the compound is represented by Formula MII-1-1, MII-1-2, MII-2-1, MII-2-2, MII-3-1 or MII-3-2:

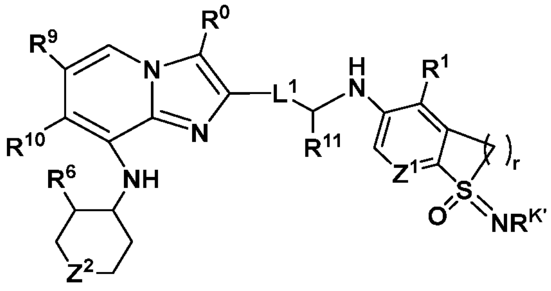

MII-1-1

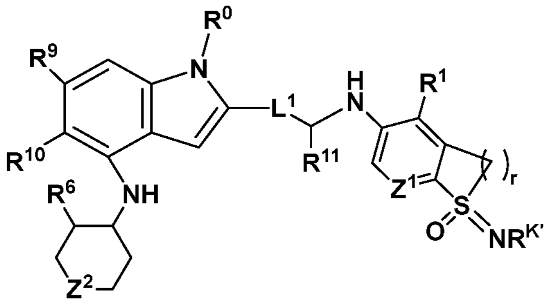

MII-1-2

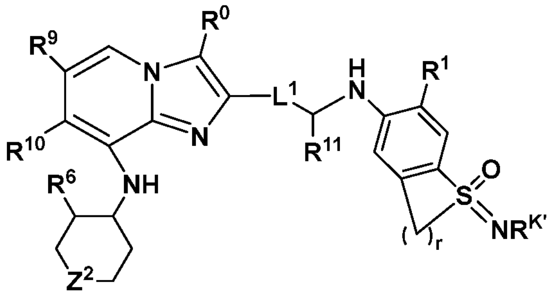

MII-2-1

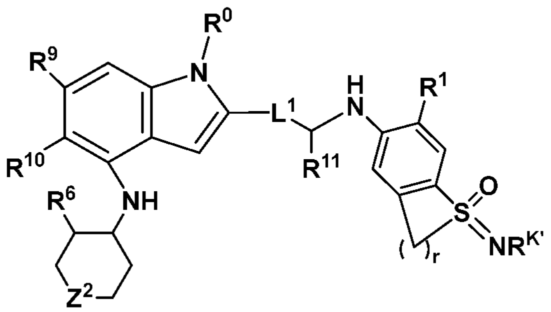

MII-2-2

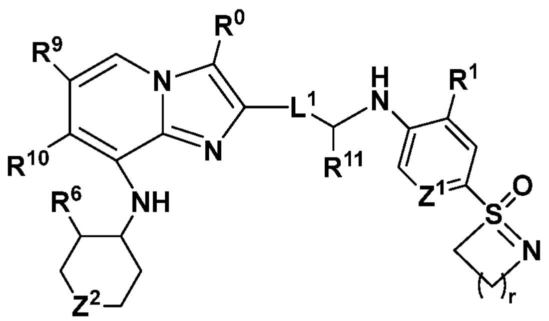

MII-3-1

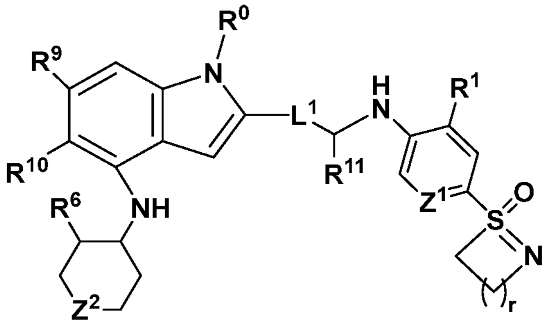

MII-3-2

wherein, $L^1$, $R^0$, $R^9$, $R^{10}$, $R^{11}$, $R^1$, $R^{k'}$, $R^6$, $Z^1$, $Z^2$, r have the definitions described herein.

Preferably, $L^1$ is

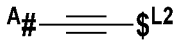

.

Preferably, $R^{11}$ is selected from the group consisting of hydrogen and methyl.

Preferably, $R^0$ is selected from the group consisting of C1-C4 alkyl, C1-C2 haloalkyl, and cyano-C1-C2 alkylene-.

Preferably, $R^9$ is selected from the group consisting of hydrogen and hydroxyl. Most preferably, $R^9$ is hydrogen.

Preferably, $R^{10}$ is selected from the group consisting of hydrogen and fluorine. Most preferably, $R^{10}$ is hydrogen.

Preferably, $Z^1$ is selected from the group consisting of N and $CR^4$; $R^4$ is selected from the group consisting of hydrogen and halogen.

More preferably, $Z^1$ is selected from the group consisting of N, CH, and CF.

Most preferably, $Z^1$ is CH.

Preferably, $R^1$ is selected from the group consisting of hydrogen, hydroxyl, halogen, C1-C4 alkyl, and $R^3O$-; wherein the C1-C4 alkyl is optionally substituted with 1, 2 or 3 substituents selected from the group consisting of halogen and hydroxyl;

$R^3$ is independently selected from the group consisting of hydrogen, C1-C4 alkyl, and C1-C4 alkyl-C(=0)-; wherein the C1-C4 alkyl is optionally substituted with halogen, hydroxyl, cyano, amino, or C1-C4 alkoxy.

More preferably, $R^1$ is selected from the group consisting of hydrogen, hydroxyl, F, and $R^3O$-;

$R^3$ is independently selected from the group consisting of hydrogen, C1-C3 alkyl, and C1-C3 alkyl-C(=0)-; wherein the C1-C3 alkyl is optionally substituted with F, hydroxyl, cyano, or C1-C3 alkoxy.

Most preferably, $R^1$ is methoxy.

Preferably, r is selected from the group consisting of 1, 2 and 3.

In some embodiments, the compound is selected from:

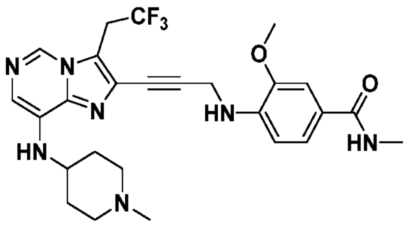
,
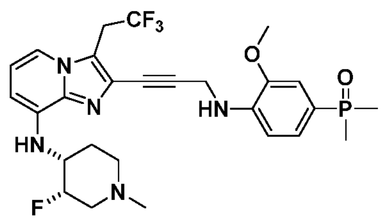
,
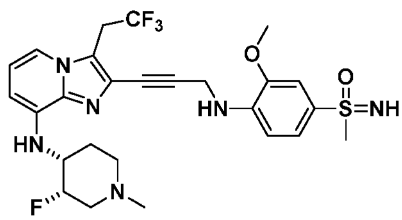
,

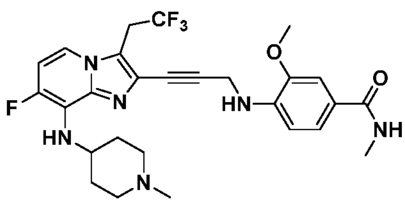
,
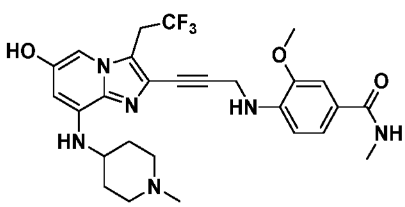
,
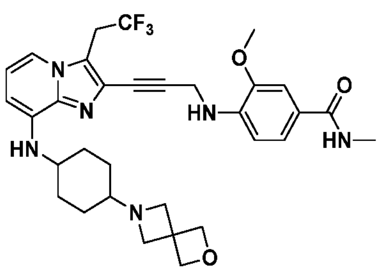
,

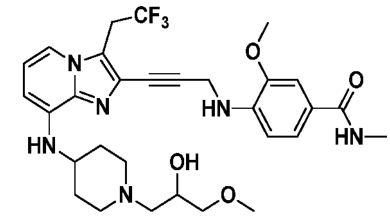,
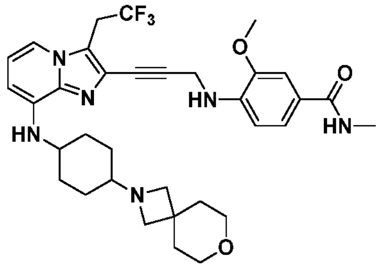,
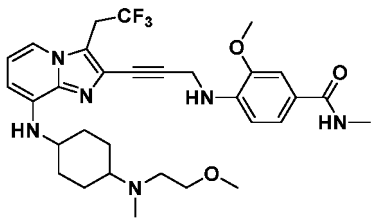,

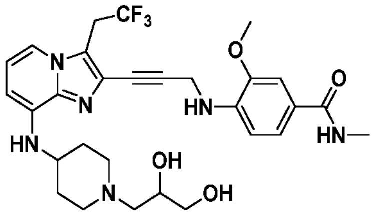,
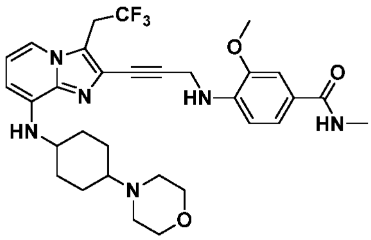,
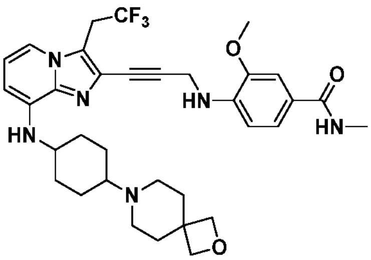,

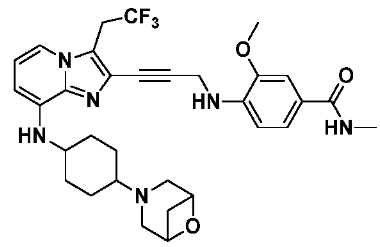,
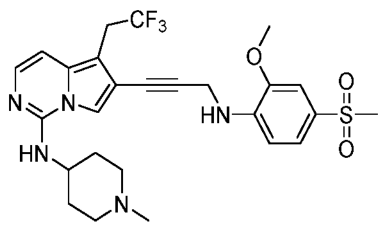,
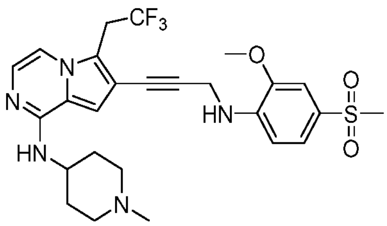,

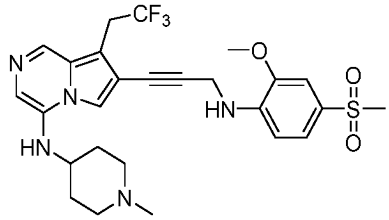,
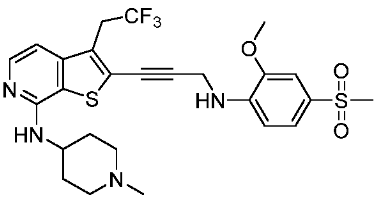,
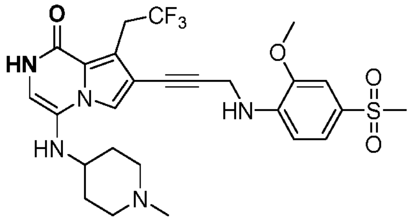,

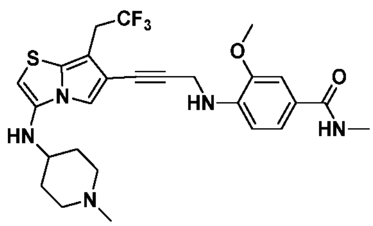,
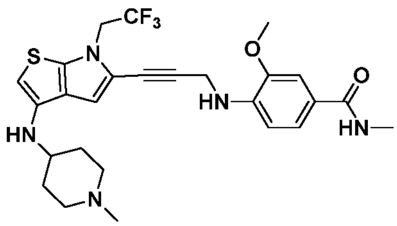,
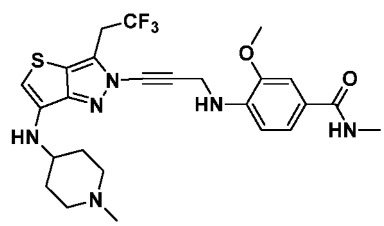,

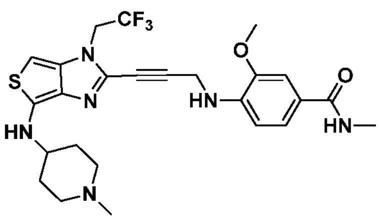,
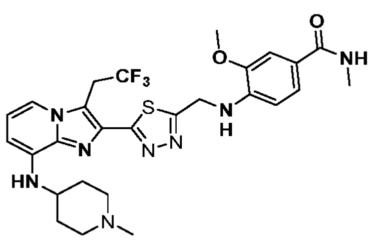,
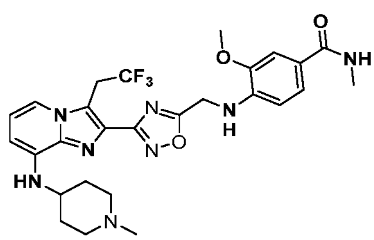,

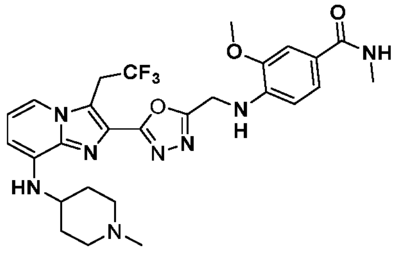,

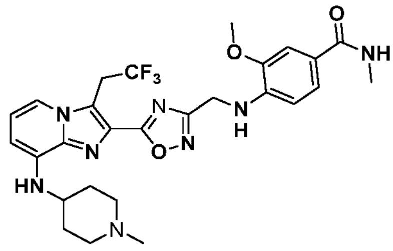,

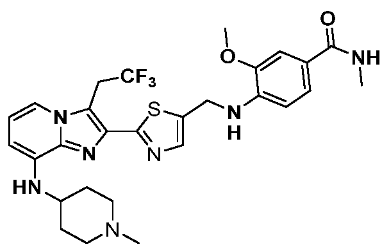,

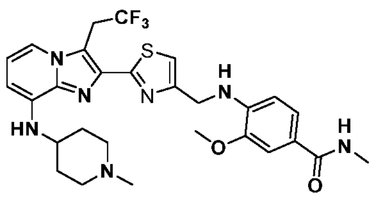,

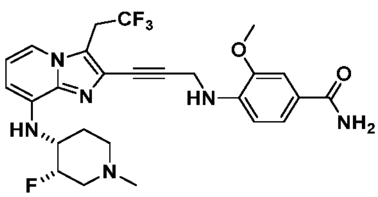,

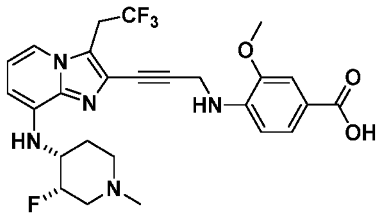,

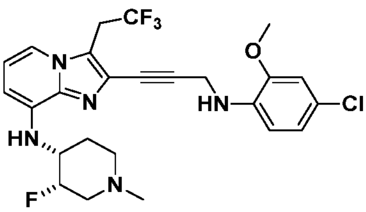,

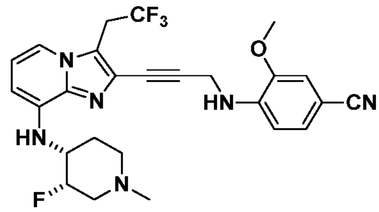,

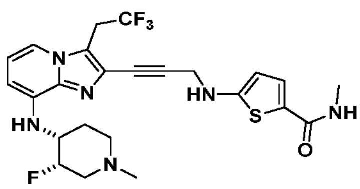,

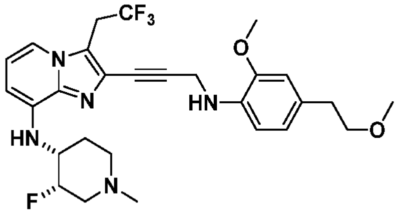,

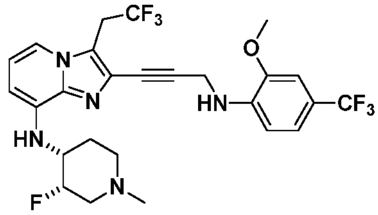,

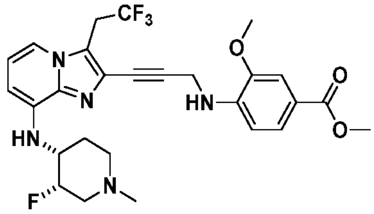,

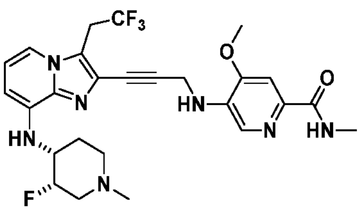,

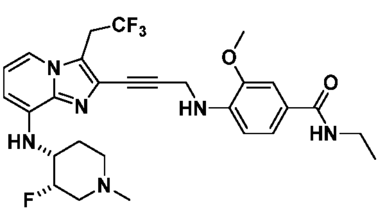,

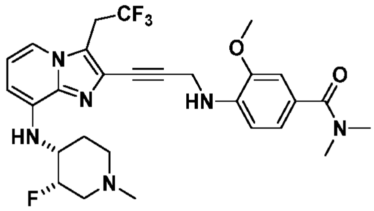,

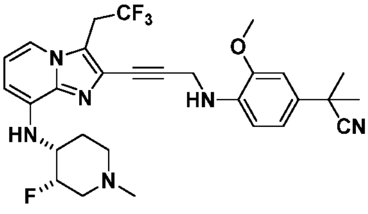,

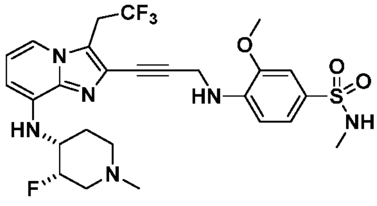,

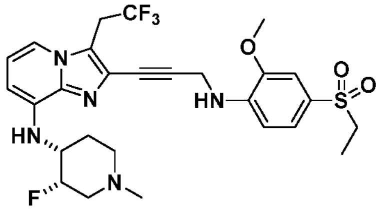,

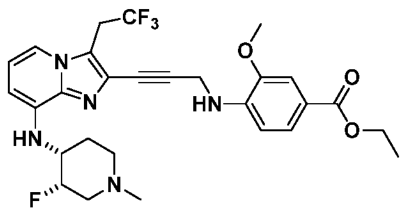, 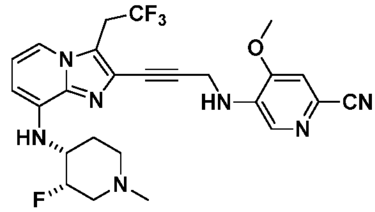, 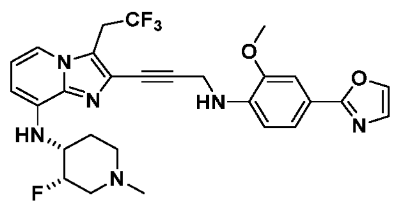,

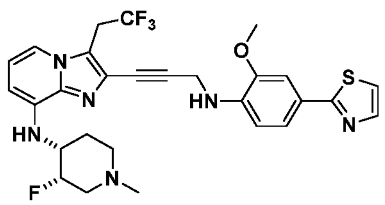, 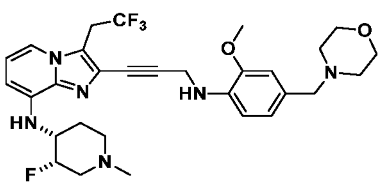, 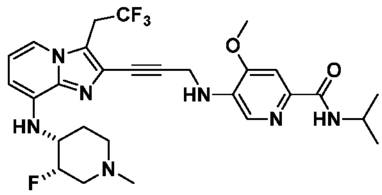,

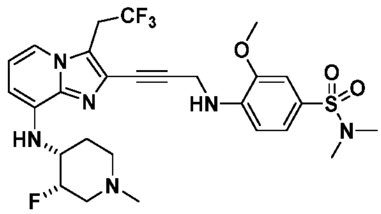, 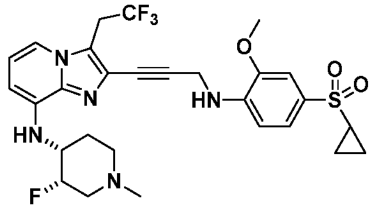, 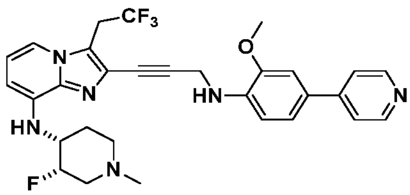,

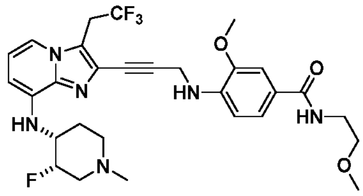, 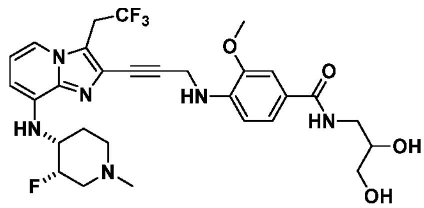, 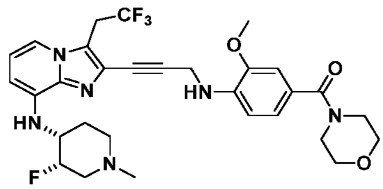,

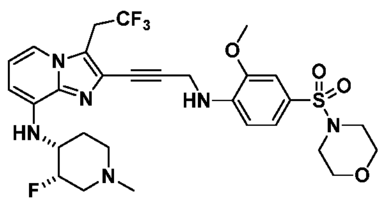, 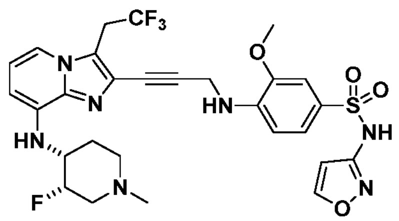, 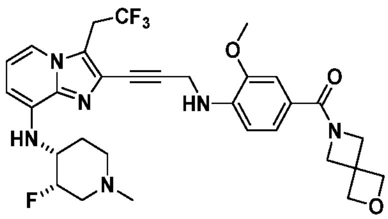,

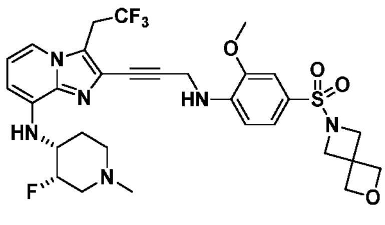, 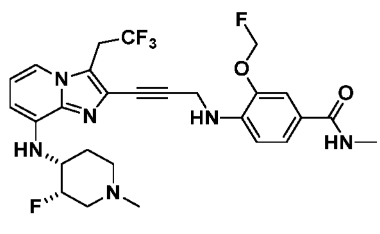, 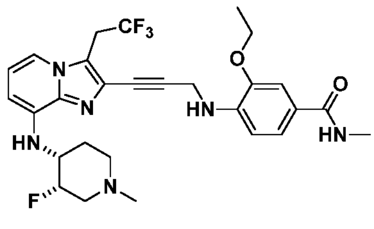,

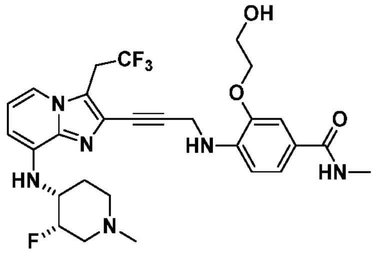,
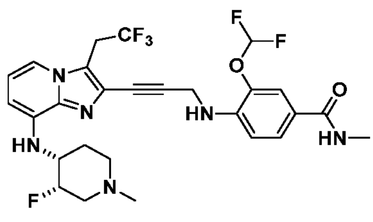,
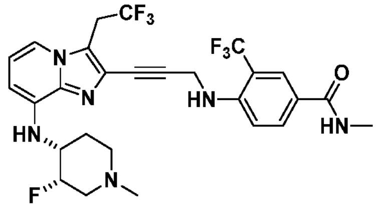,
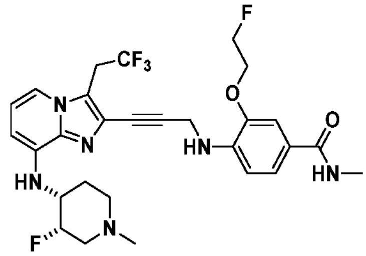,
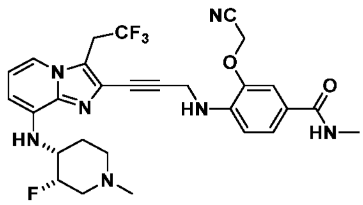,
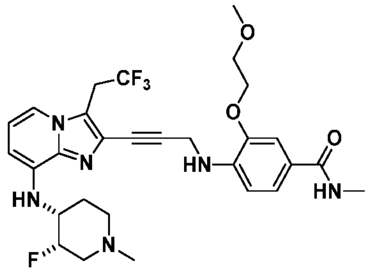,
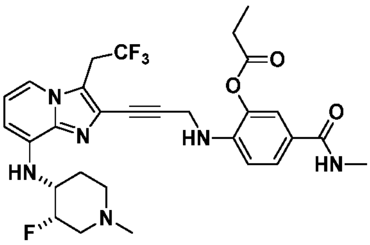,
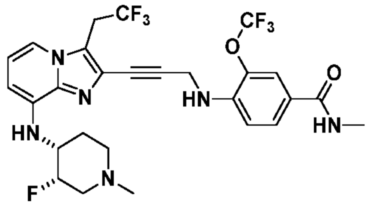,
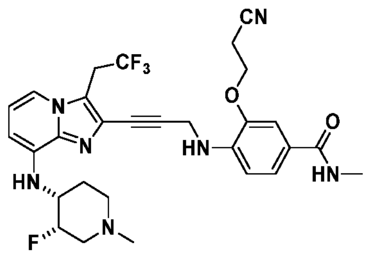,
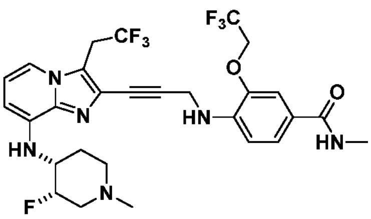,
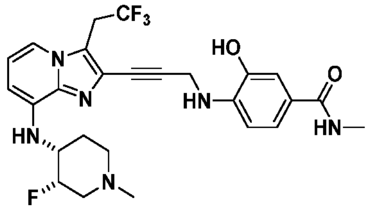,
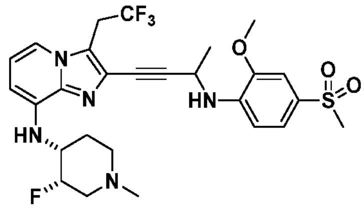,
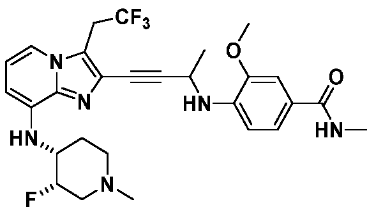,
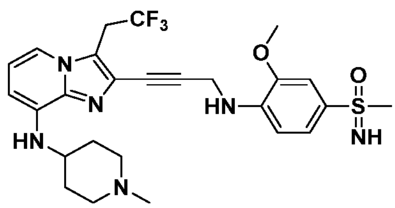,
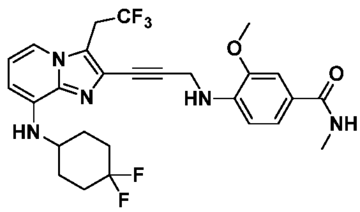,
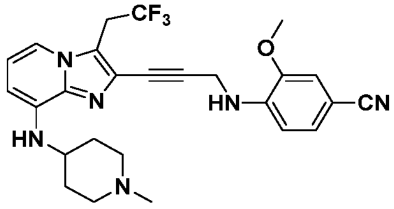,
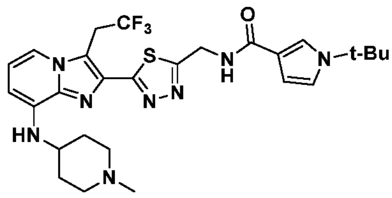,
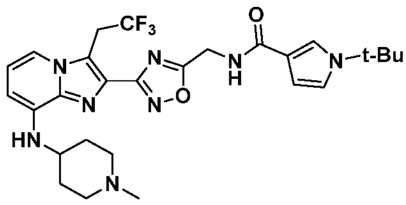,

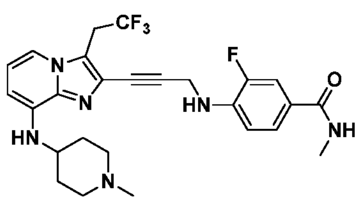,
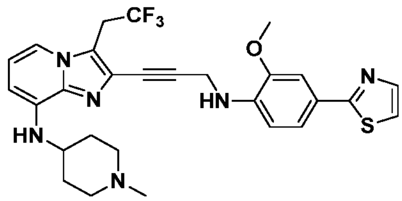,
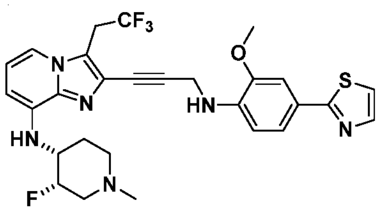,

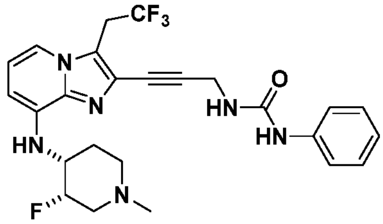,
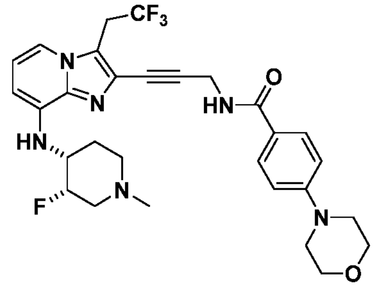,
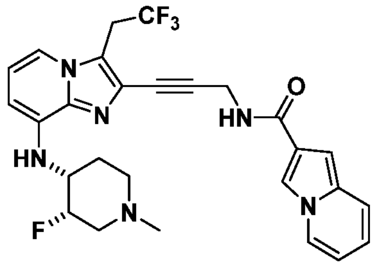,

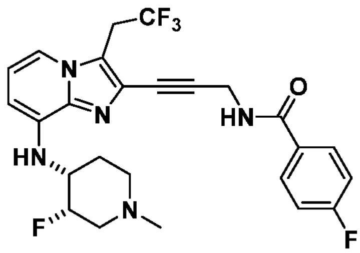,
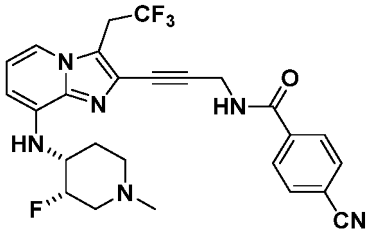,
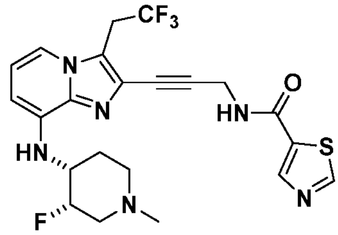,

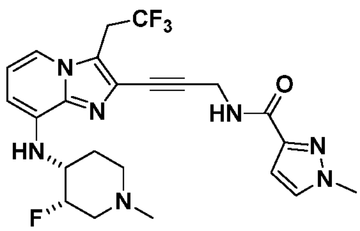,
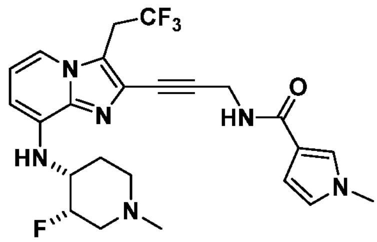,
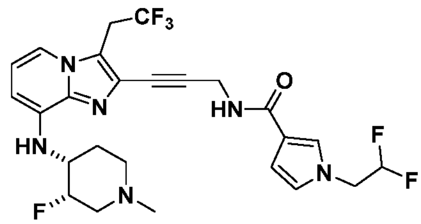,

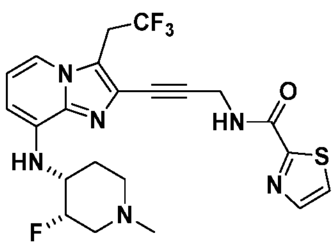,
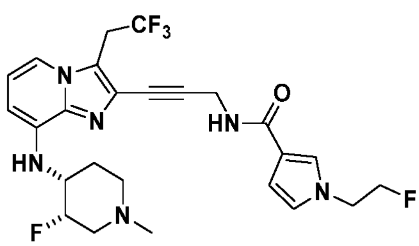,
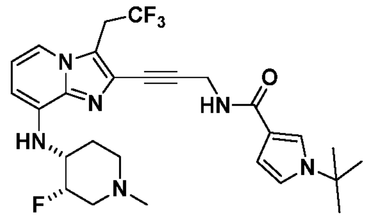,

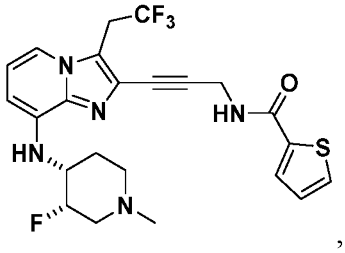,
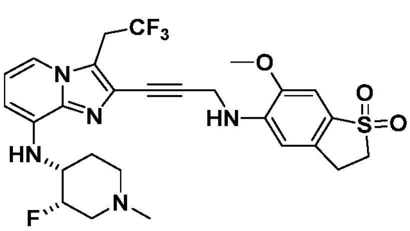,
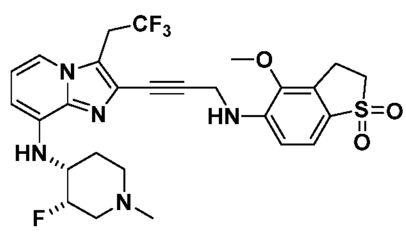,

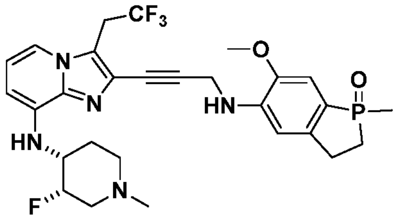,
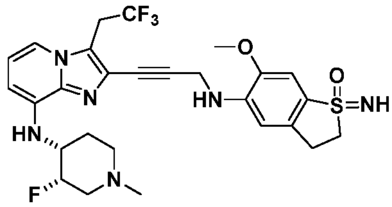,
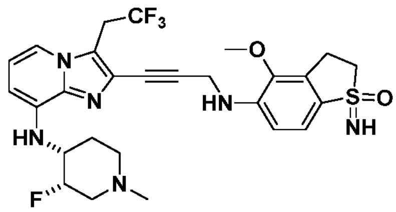,

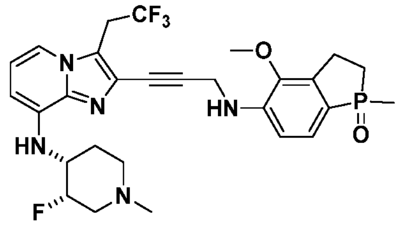,
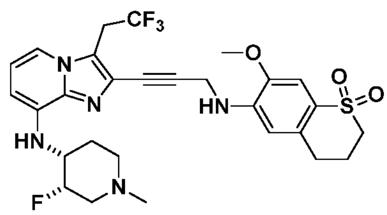,
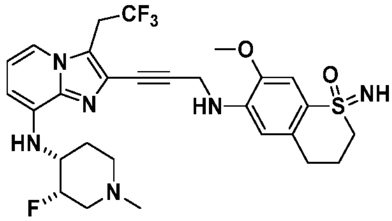,

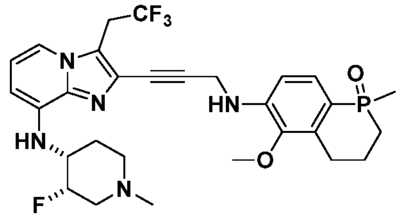,
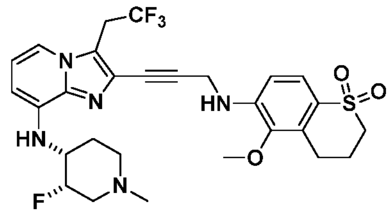,
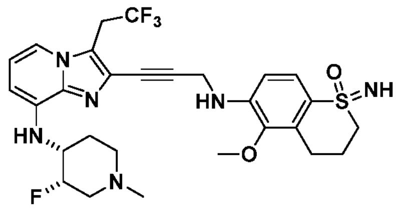,

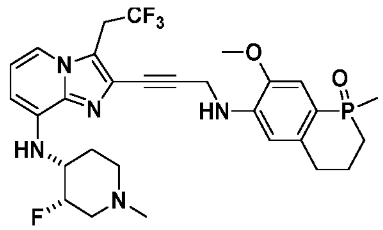,
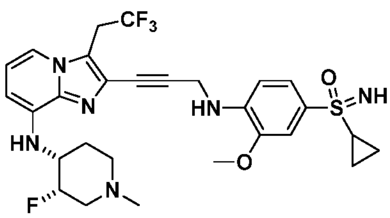,
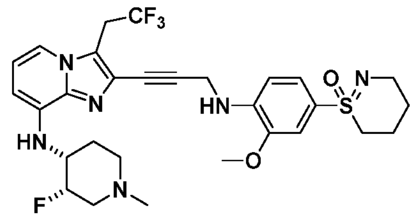,

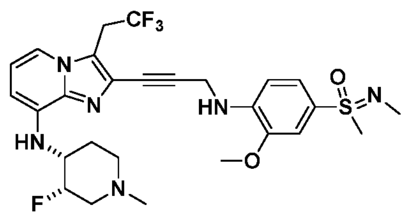,
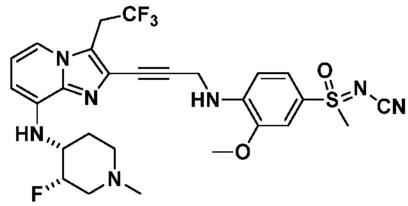,
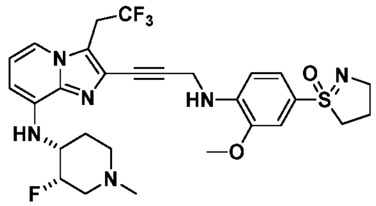,

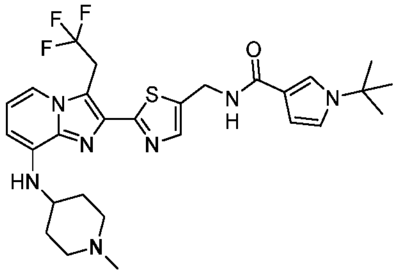,
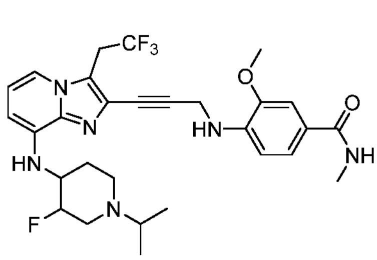,
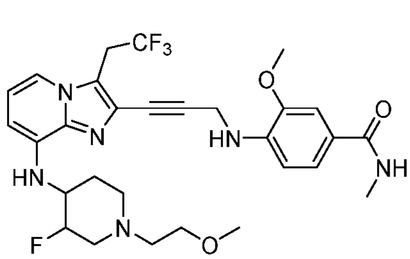,

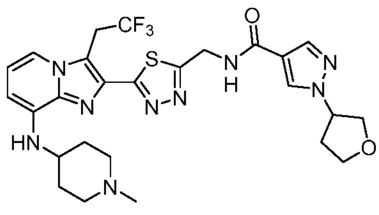,

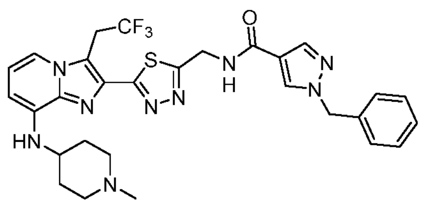,

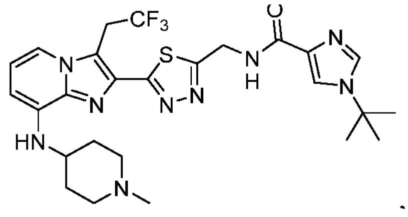,

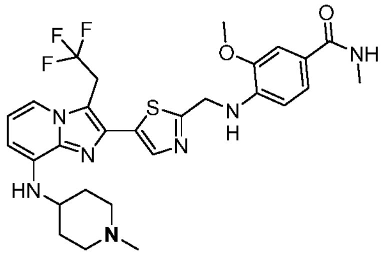,

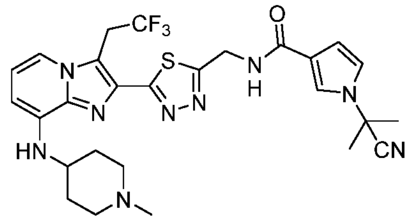,

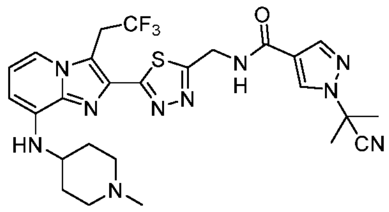,

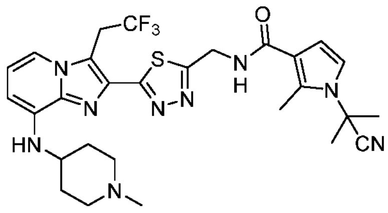,

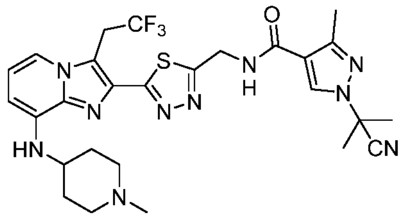,

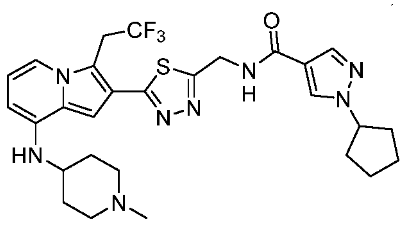,

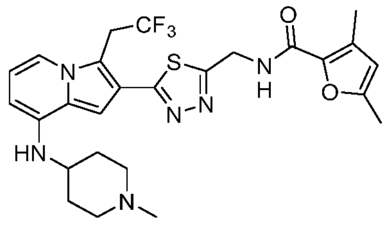,

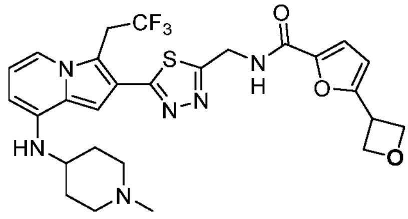,

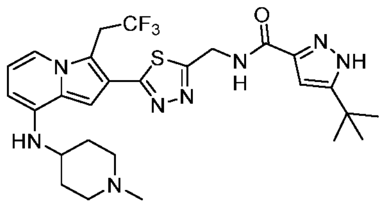,

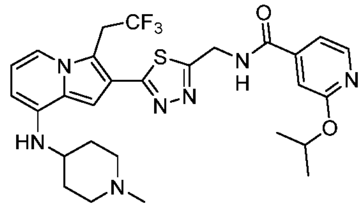,

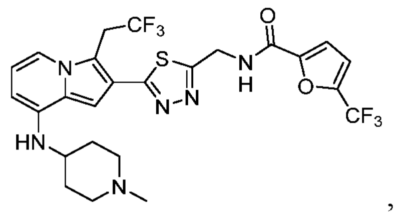,

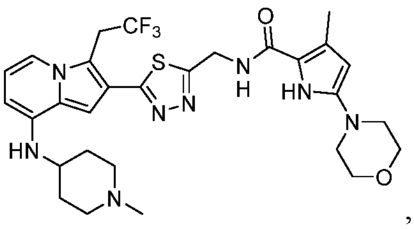,

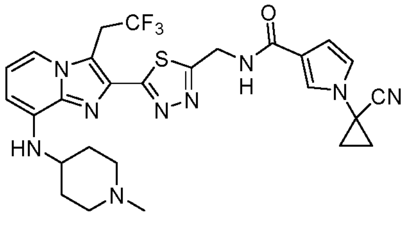,

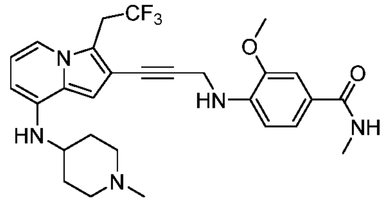,

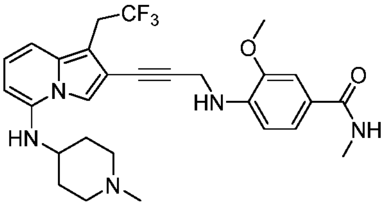,

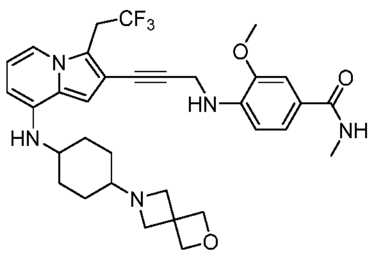 , 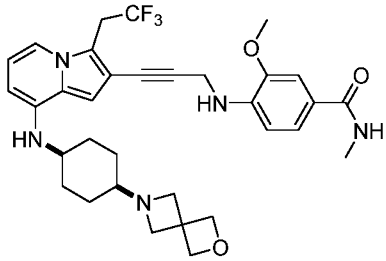 , 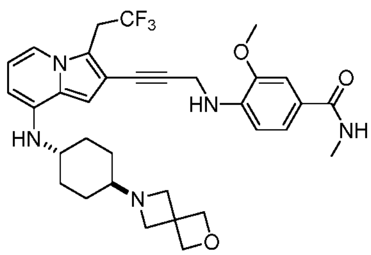 ,

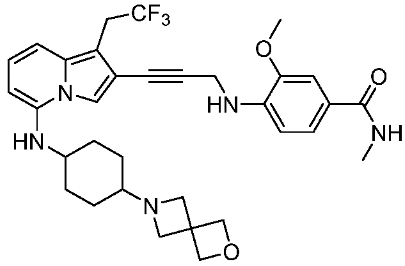 , 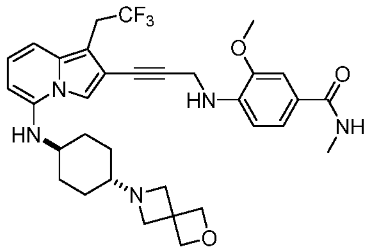 , 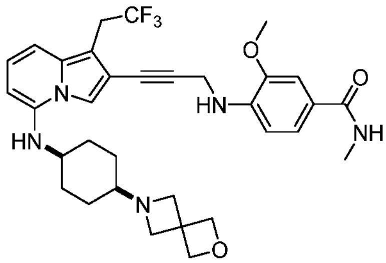 ,

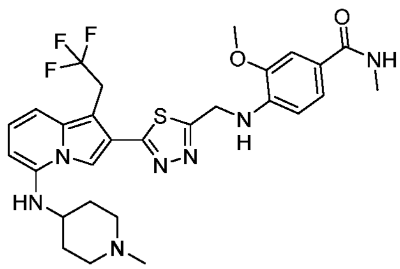 , 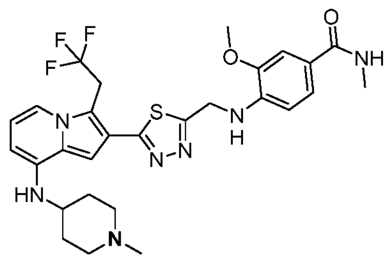 , 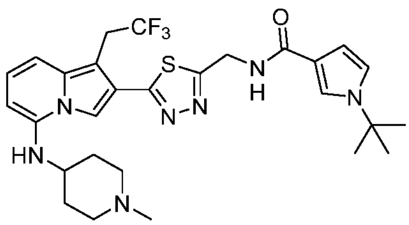 ,

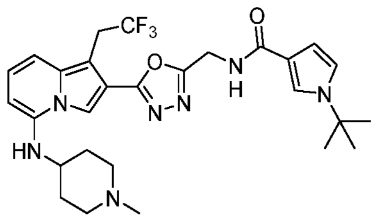 , 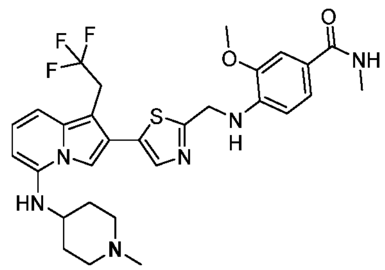 , 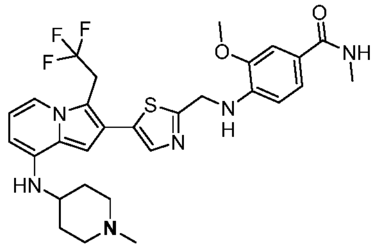 ,

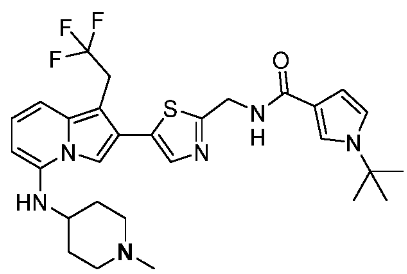 , 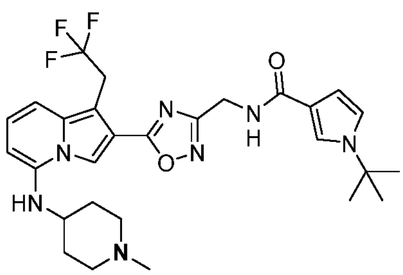 , 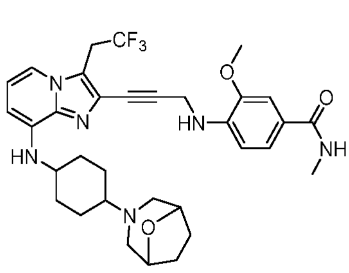 ,

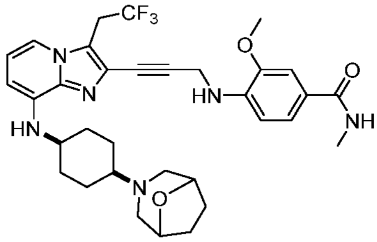,
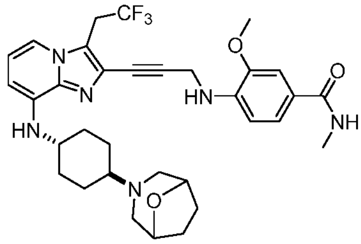,
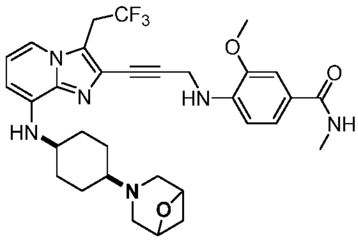,
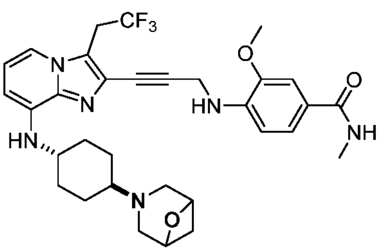,
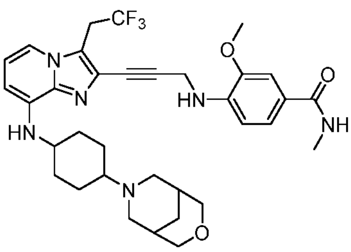,
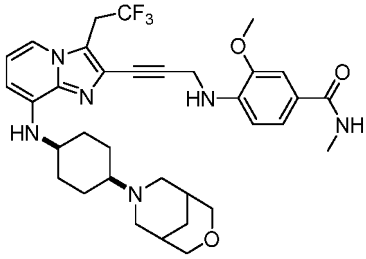,
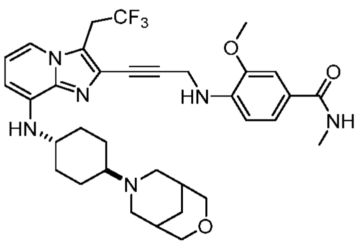,
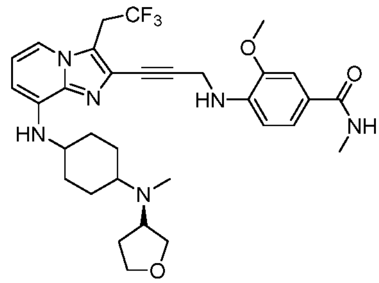,
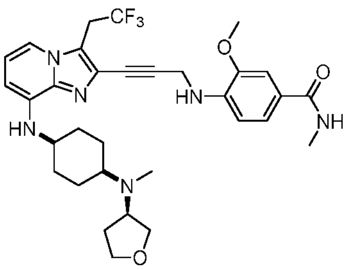,
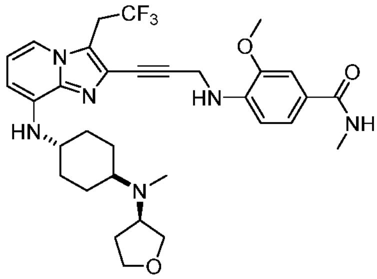,
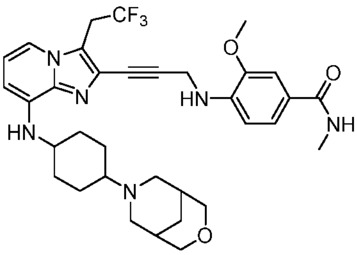,
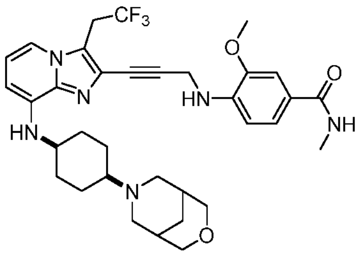,
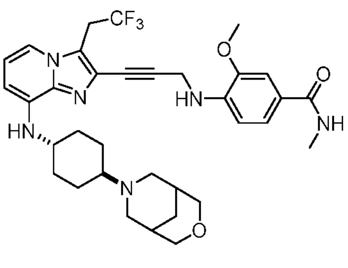,
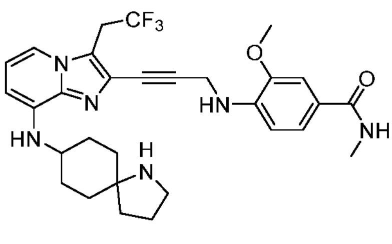,
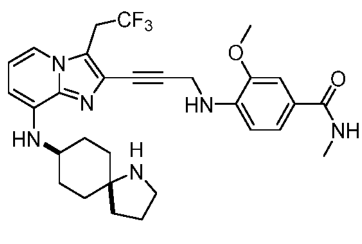,

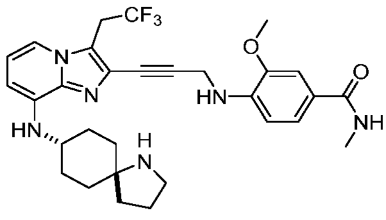,
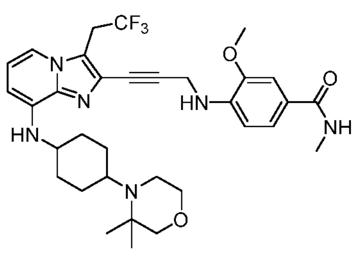,
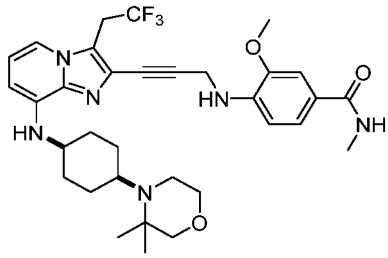,

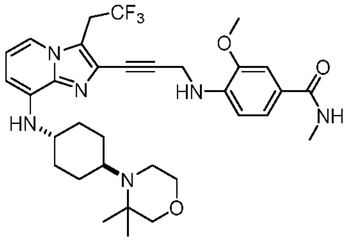,
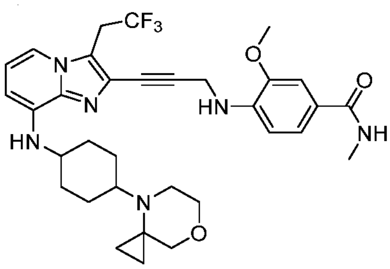,
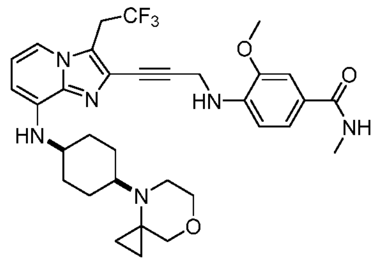,

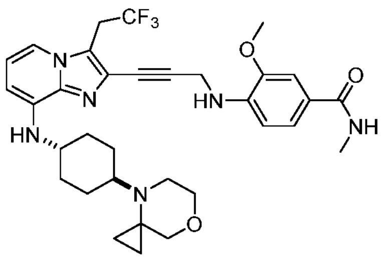,
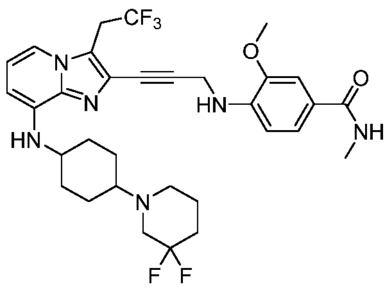,
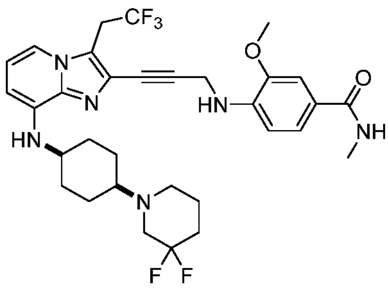,

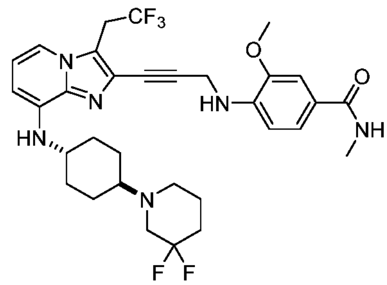,
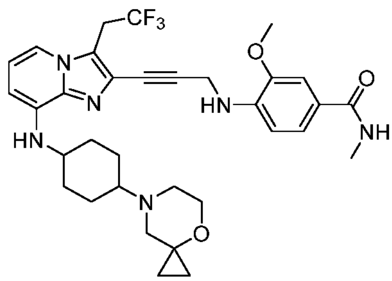,
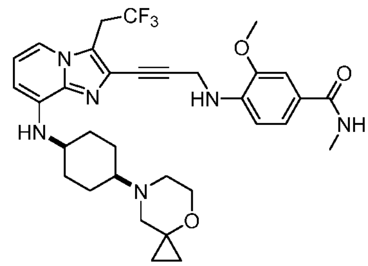,

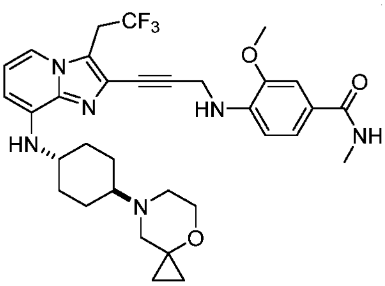,
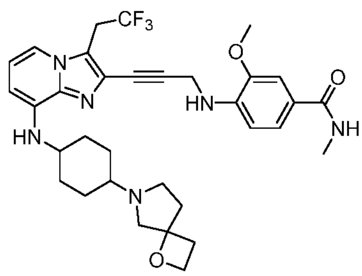,
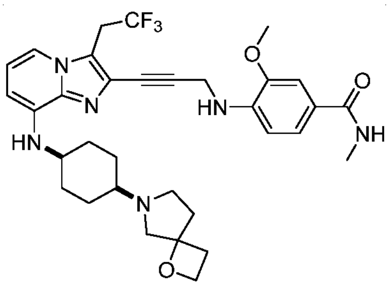,

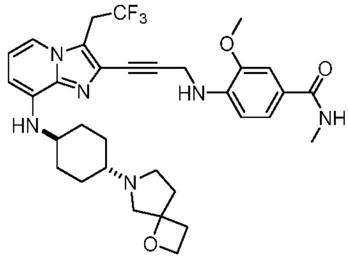, 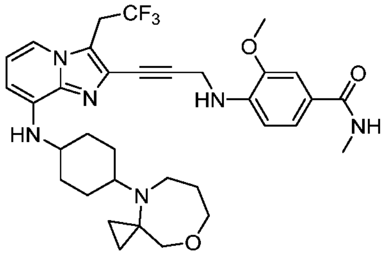, 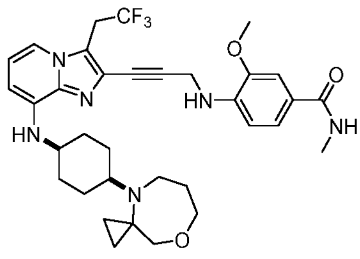,

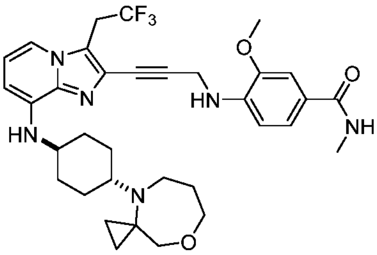, 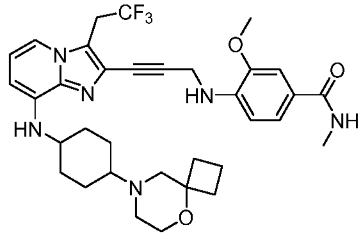, 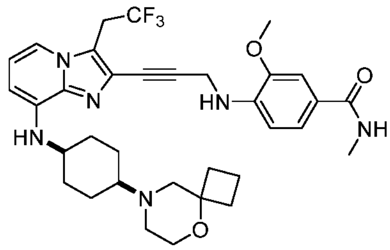,

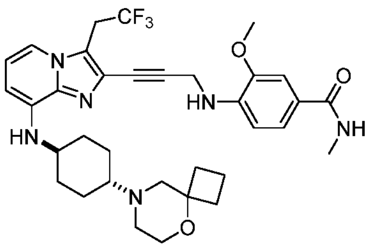, 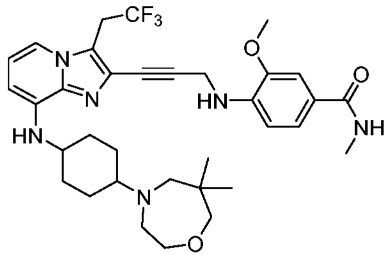, 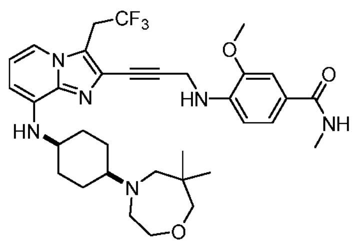,

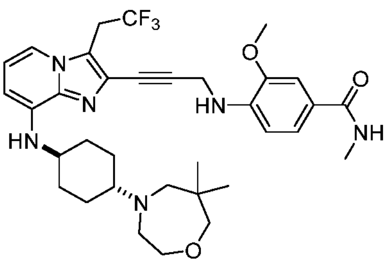, 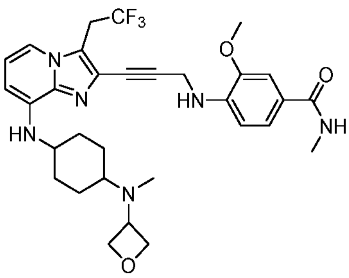, 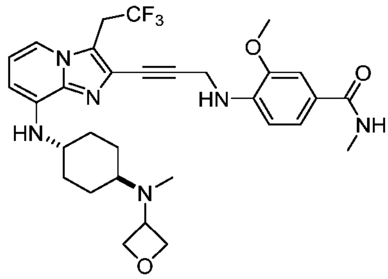,

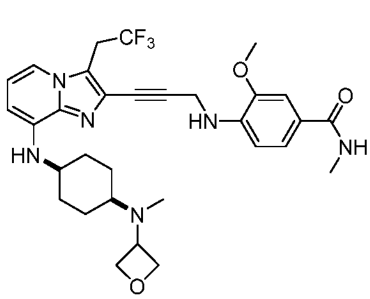, 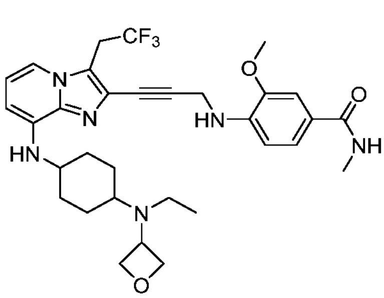, 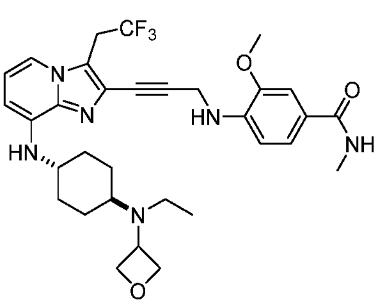,

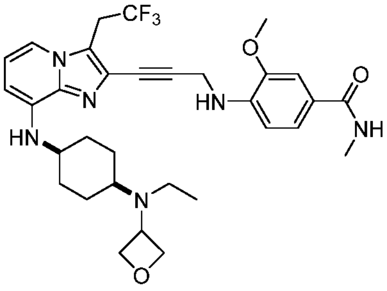
,
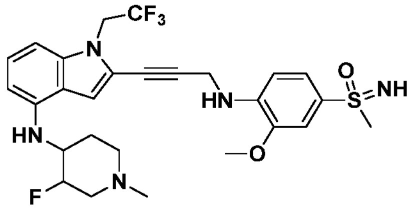
,
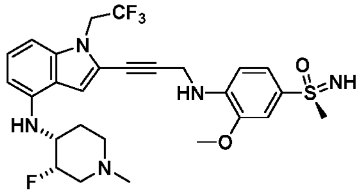
,
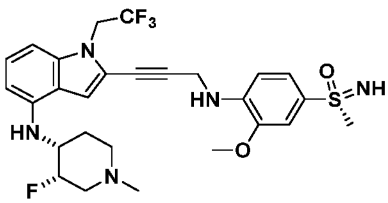
,
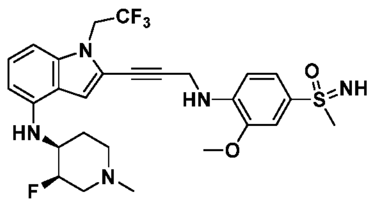
,
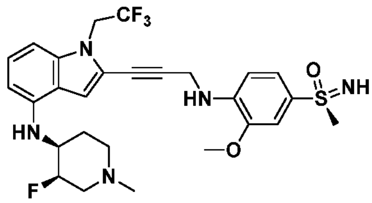
,
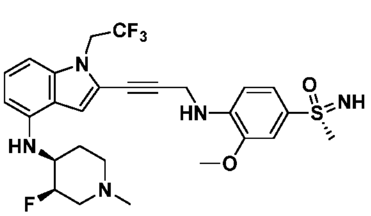
,
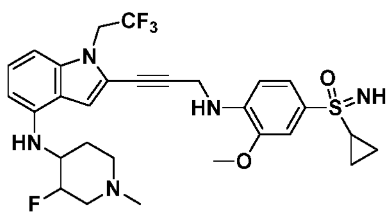
,
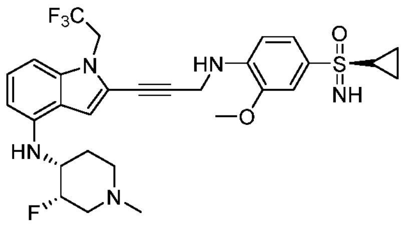
,
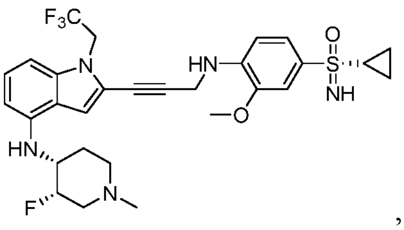
,
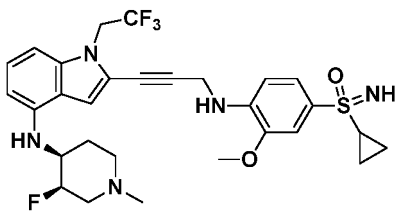
,
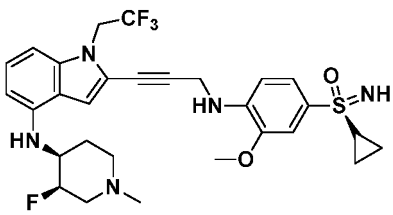
,
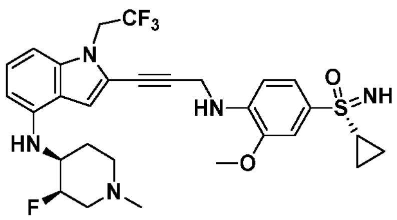
,
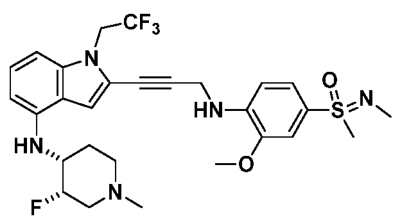
,
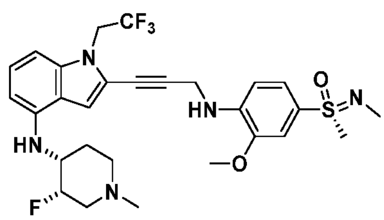
,
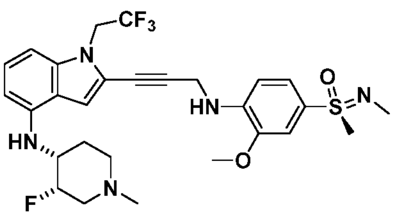
,
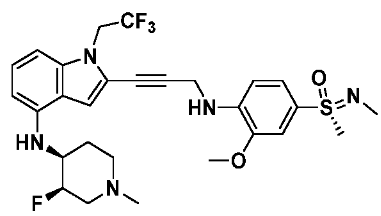
,
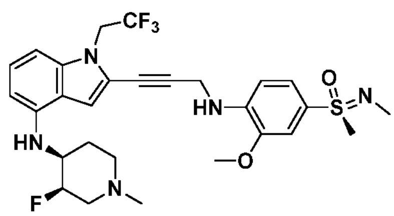
,

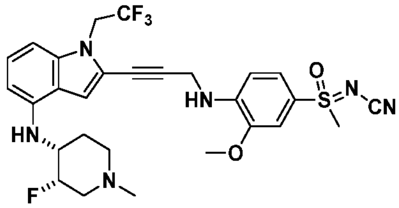,
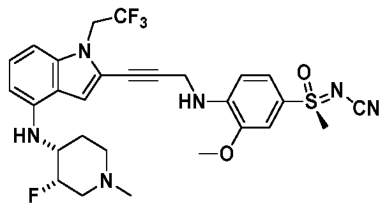,
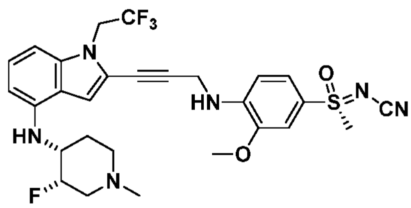,
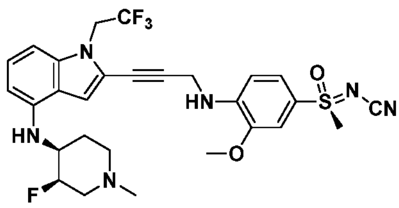,
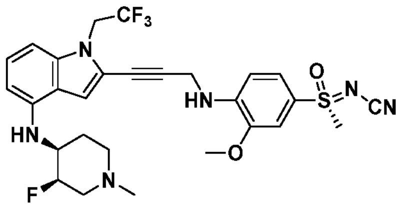,
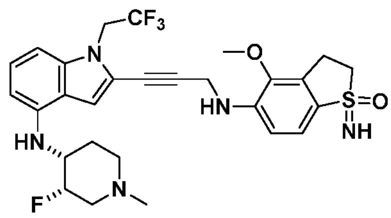,
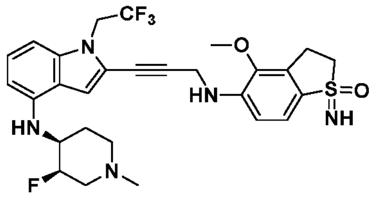,
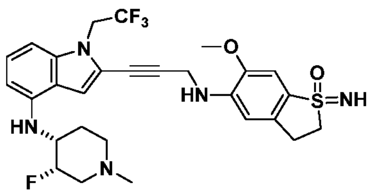,
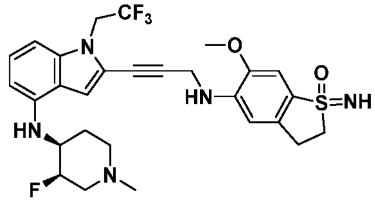,
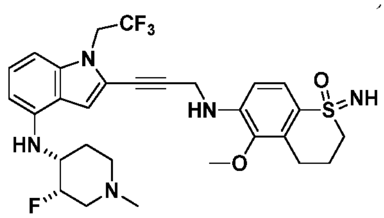,
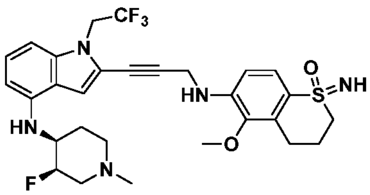,
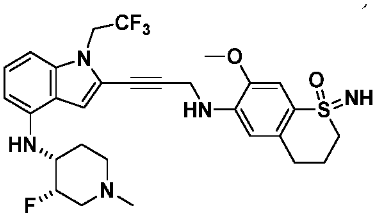,
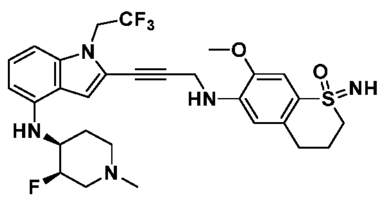,
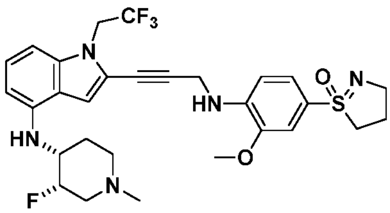,
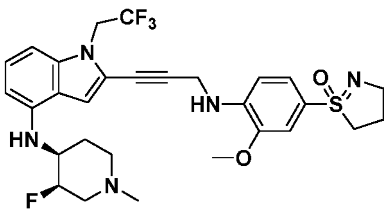,
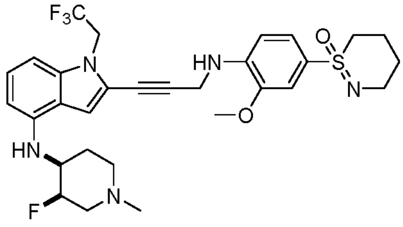,
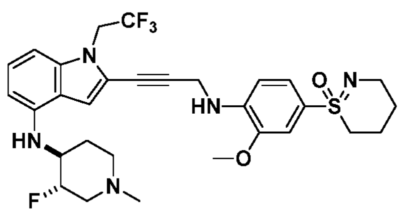,
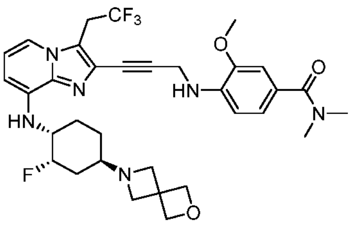,

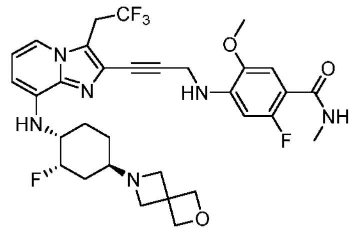 , 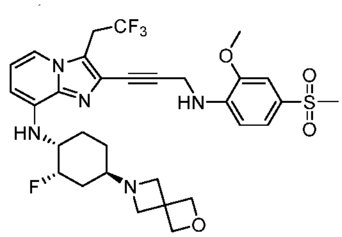 , 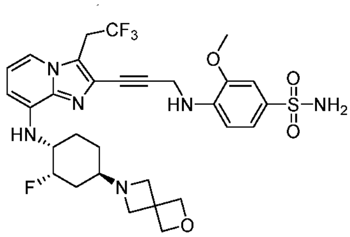 ,

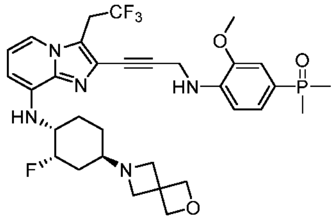 , 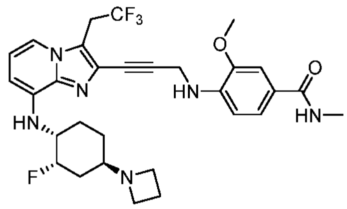 , 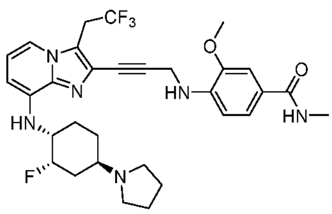 ,

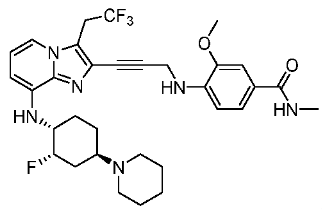 , 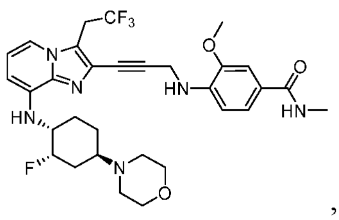 , 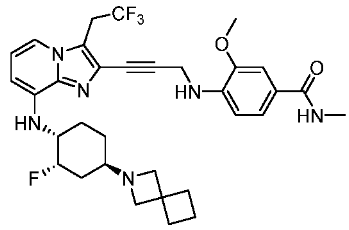 ,

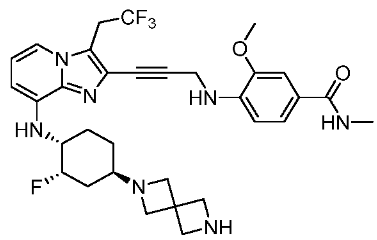 , 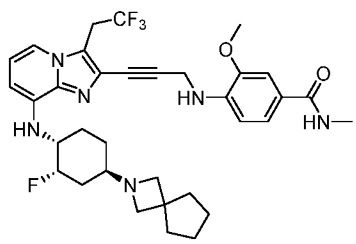 , 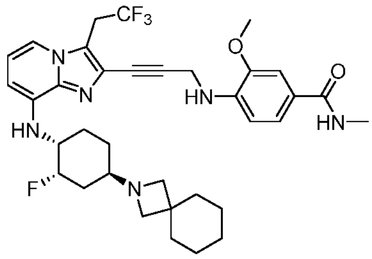 ,

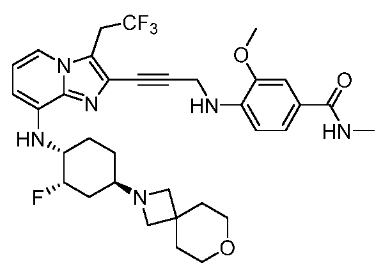 , 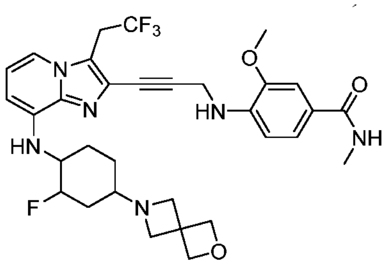 , 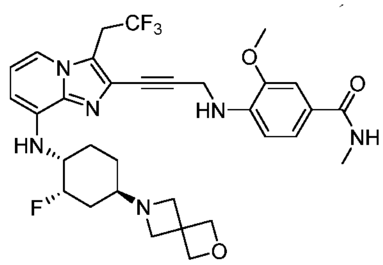 ,

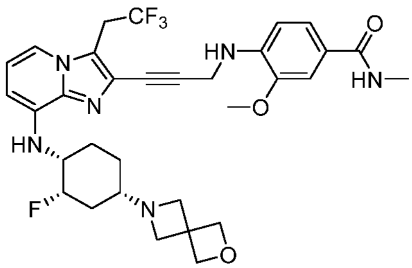 , 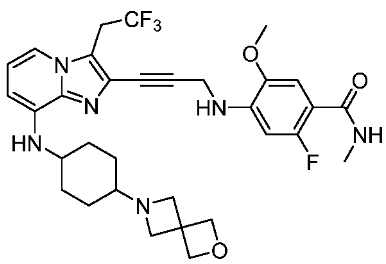 , 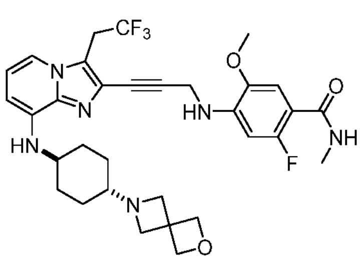 ,

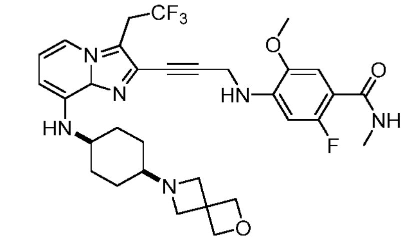 , 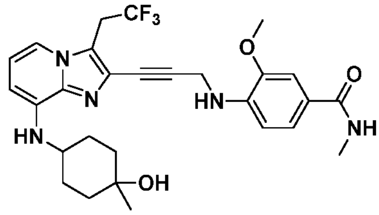 , 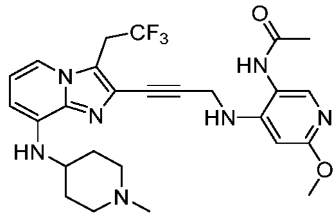 ,

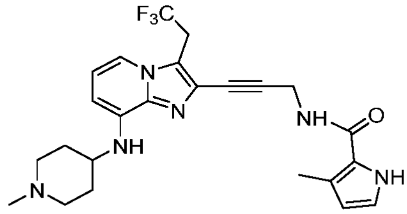 , 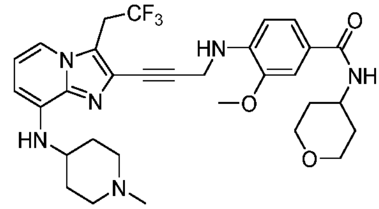 , 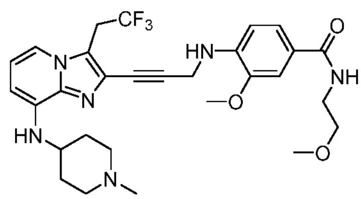 ,

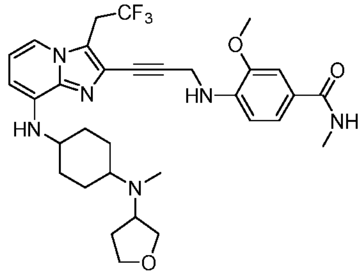 , 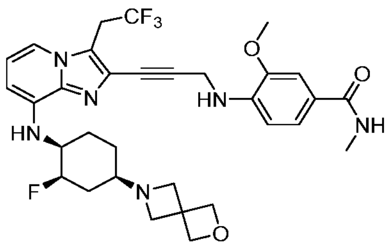 , 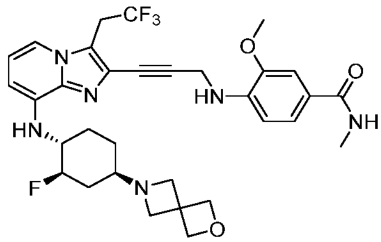 ,

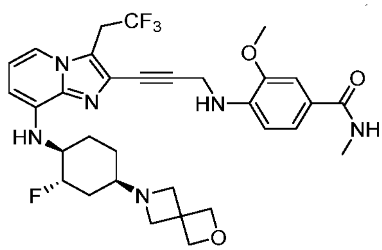 , 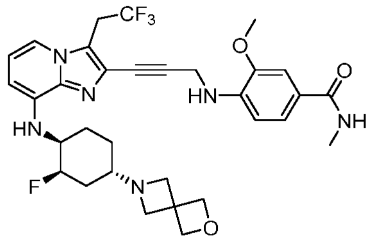 , 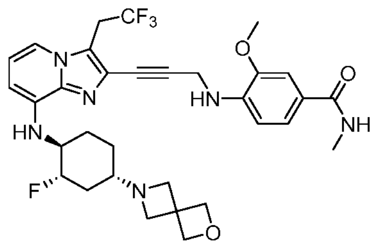 ,

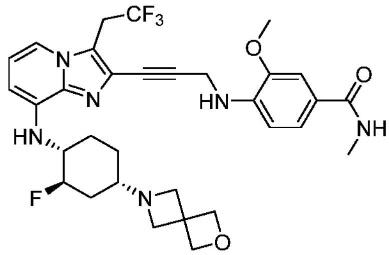 , 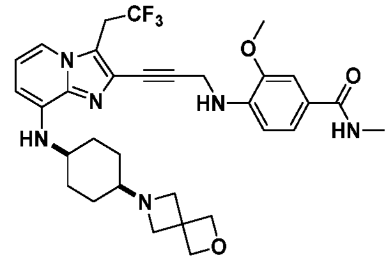 , 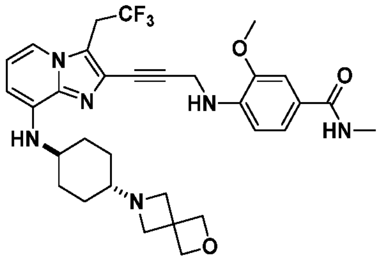 ,

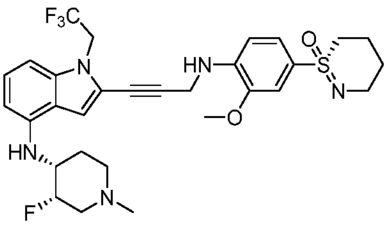 , 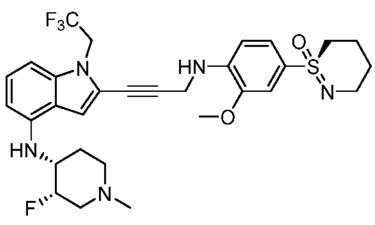 , 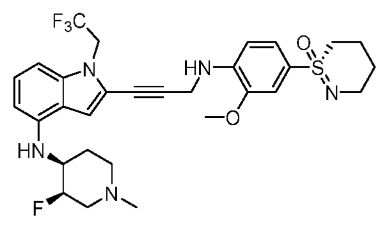 ,

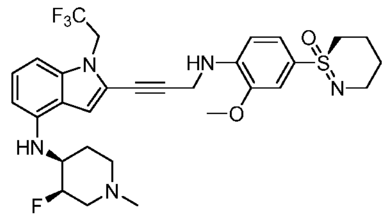, 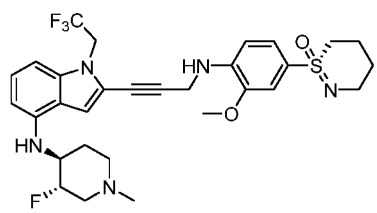, 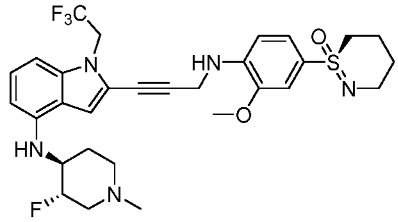,

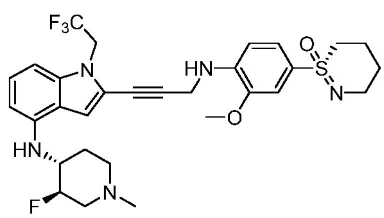, 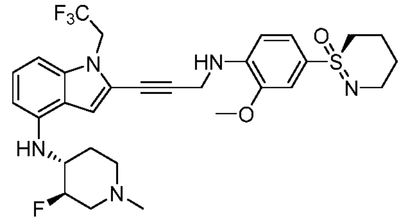, 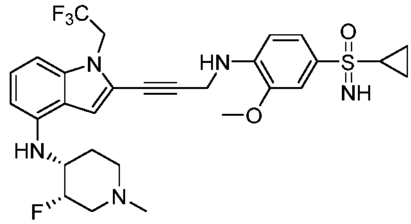,

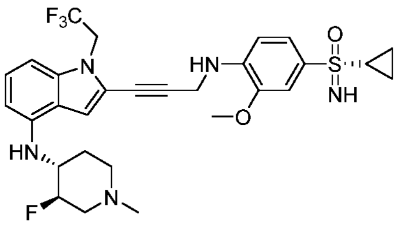, 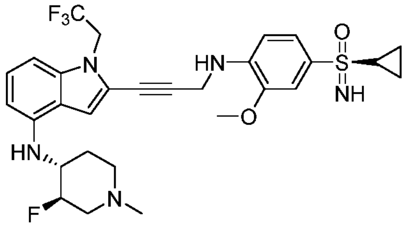, 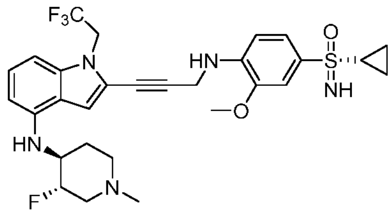,

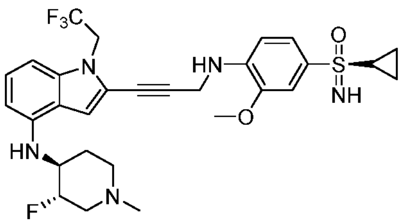, 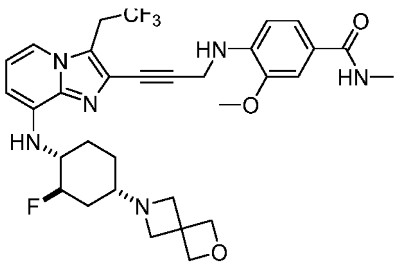, 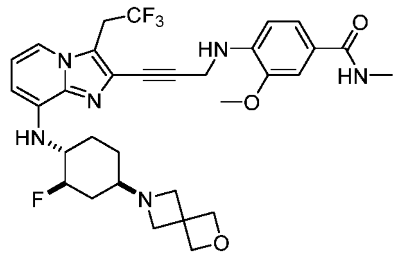,

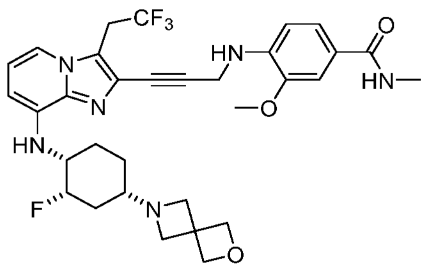, 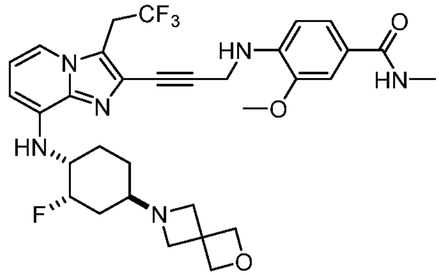.

On the other hand, the present invention provides a compound represented by Formula O, or a stereoisomer, tautomer, prodrug, crystal form, hydrate, isotope-labeled compound, metabolite, ester, pharmaceutically acceptable salt or pharmaceutically acceptable solvate of the compound,

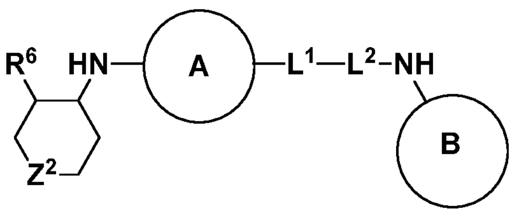

formula O wherein:

Ring A is selected from the group consisting of

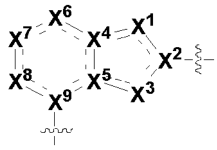

, and

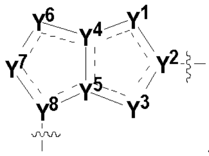

,

wherein the site at the lower left end is connected to NH, and the site at the upper right end is connected to $L^1$; each - - - - - - is independently a single bond or a double bond;

$X^1$, $X^2$, $X^3$, $X^4$, $X^5$, $X^6$, $X^7$, $X^8$, $X^9$ are independently selected from the group consisting of C, CH, $CR^a$, $CR^b$, $CR^c$, C=O, N, NH, and S, and meet the following conditions a) or b):

a) any one of $X^4$ and $X^5$ is N,

b) when neither of $X^4$ and $X^5$ is N, any one of $X^1$ and $X^3$ is S;

$Y^1$, $Y^2$, $Y^3$, $Y^4$, $Y^5$ $Y^6$, $Y^7$, $Y^8$ are each independently selected from the group consisting of S, N, C, CH, $CR^d$, and $NR^d$, and meet the following conditions c) or d):

c) any one of $Y^4$ and $Y^5$ is N,

d) when neither of $Y^4$ and $Y^5$ is N, any one of $Y^6$ and $Y^7$ is S;

$R^a$ is selected from the group consisting of C1-C6 alkyl, C1-C6 haloalkyl, cyano-C1-C6 alkylene, C3-C6 cycloalkyl, and C3-C6 halocycloalkyl;

preferably, $R^a$ is selected from the group consisting of C1-C6 alkyl, C1-C6 haloalkyl, and cyano-C1-C6 alkylene;

preferably, $R^a$ is selected from the group consisting of C1-C4 alkyl, C1-C2 haloalkyl, and cyano-C1-C2 alkylene;

more preferably, $R^a$ is selected from the group consisting of

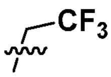

,

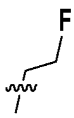

,

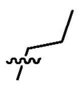

,

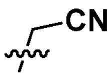

, and

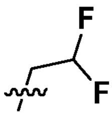

;

most preferably, $R^a$ is

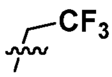

;

$R^b$ is selected from the group consisting of hydrogen, hydroxyl, halogen, and cyano;

preferably, $R^b$ is selected from the group consisting of hydrogen, and hydroxyl;

preferably, $R^b$ is hydrogen;

$R^c$ is selected from the group consisting of hydrogen, hydroxyl, halogen, and cyano;

preferably, $R^c$ is selected from the group consisting of hydrogen, and halogen;

preferably, $R^c$ is selected from the group consisting of hydrogen, and fluorine;

more preferably, $R^c$ is hydrogen;

$R^d$ is selected from the group consisting of C1-C6 alkyl, C1-C6 haloalkyl, cyano-C1-C6 alkylene, C3-C6 cycloalkyl, and C3-C6 halocycloalkyl;

preferably, $R^d$ is selected from the group consisting of C1-C6 alkyl, C1-C6 haloalkyl, and cyano-C1-C6 alkylene;

preferably, $R^d$ is selected from the group consisting of C1-C4 alkyl, C1-C2 haloalkyl, and cyano-C1-C2 alkylene;

more preferably, $R^d$ is selected from the group consisting of

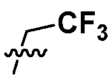, 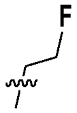, 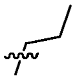,

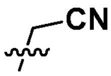, and 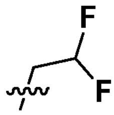;

most preferably, $R^d$ is

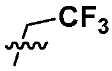;

preferably, Ring A is selected from the group consisting of

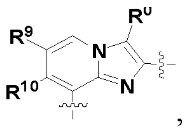, 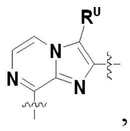,

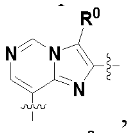, 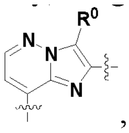, 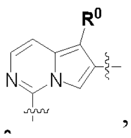, 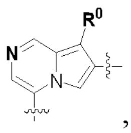, 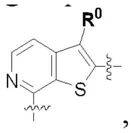, 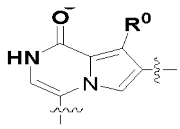, 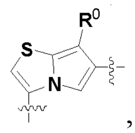, 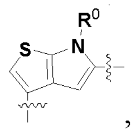,

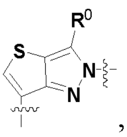, and 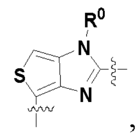,

wherein the site at lower left end is connected to NH, and the site at upper right end is connected to L; more preferably, Ring A is

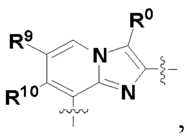,

wherein the site at lower left end is connected to NH, and the site at upper right end is connected to $L^1$; Ring B is selected from the group consisting of

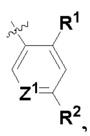, and 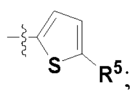;

preferably, Ring B is

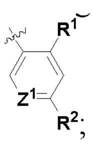;

$L^1$ is selected from the group consisting of

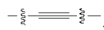,

and 5- to 6-membered heteroarylene;
preferably, $L^1$ is selected from the group consisting of

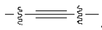,

and 5-membered heteroarylene;
more preferably, $L^1$ is selected from the group consisting of

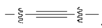,

1,3,4-thiadiazollene, 1,2,4-oxadiazolylene, 1,3,4-oxadiazolylene, and thiazolylene;
further preferably, $L^1$ is selected from the group consisting of

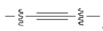, 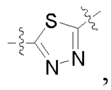, 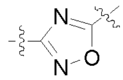,

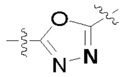, 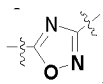, 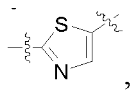, and 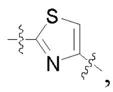,

wherein the left site is connected to Ring A, and the right site is connected to $L^2$;
most preferably, $L^1$ is

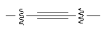;

$L^2$ is

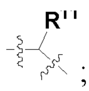;

$Z^1$ is selected from the group consisting of N and $CR^4$;
$Z^2$ is selected from the group consisting of

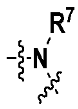, 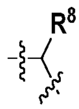, 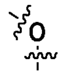, and 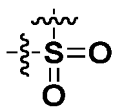;

preferably, $Z^2$ is selected from the group consisting of

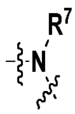, and 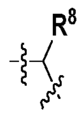;

$R^0$ is selected from the group consisting of C1-C6 alkyl, C1-C6 haloalkyl, cyano-C1-C6 alkylene, C3-C6 cycloalkyl, and C3-C6 halocycloalkyl;
preferably, $R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

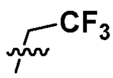, 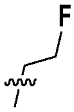, 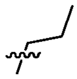,

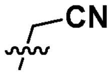, and 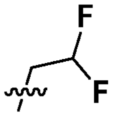;

most preferably, $R^0$ is

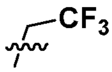;

$R^1$ is selected from the group consisting of C1-C6 alkyl, C1-C6 haloalkyl, and $R^3O$-;
preferably, $R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 alkyl, C1-C6 haloalkyl, hydroxyl-substituted C1-C6 alkyl, C1-C6 alkoxy-C1-C6 alkylene, cyano-C1-C6 alkylene, 4- to 8-membered saturated heterocyclyl-C1-C6 alkylene, $R^eR^fN$-C(=O)-, $R^8O$-C(=O)-, $R^hS(=O)_2$-, $R^iR^jN$-$S(=O)_2$-,

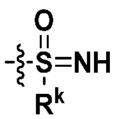, 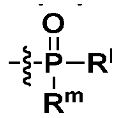,

,and 5- to 6-membered heteroaryl;
preferably, $R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

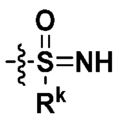, 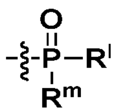,

and 5- to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^1R^jN$-$S(=O)_2$-,

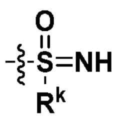, 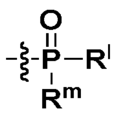,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^1R^jN$-$S(=O)_2$-,

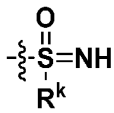, 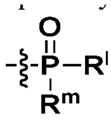,

oxazolyl, thiazolyl, and pyridyl;

$R^e$, $R^f$ are each independently selected from the group consisting of hydrogen, C1-C6 alkyl, cyano-C1-C6 alkylene, C1-C6 haloalkyl, C1-C6 alkoxy-C1-C6 alkylene, and C1-C6 alkyl substituted with 1 to 3 hydroxyl groups; or, $R^e$, $R^f$ together with the N atom to which they connect form a 4- to 8-membered saturated heterocyclic ring or a 7- to 11-membered spiro-heterocyclic ring;

preferably, $R^e$ and $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;

preferably, $R^e$ is selected from the group consisting of hydrogen, and C1-C6 alkyl, $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, and C1-C4 alkyl, $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, $R^f$ is selected from the group consisting of hydrogen, methyl, ethyl, isopropyl,

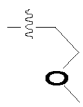, and 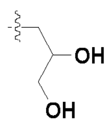;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which they connect form

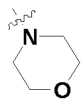,

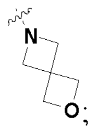;

$R^g$ is selected from the group consisting of hydrogen, C1-C6 alkyl, cyano-C1-C6 alkylene, C1-C6 haloalkyl, hydroxy-C1-C6 alkylene, C3-C6 cycloalkyl, C3-C6 halocycloalkyl, and hydroxy-C3-C6 cycloalkylene;

preferably, $R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, C1-C6 haloalkyl, C3-C6 cycloalkyl, C3-C6 halocycloalkyl, and 5- to 6-membered heteroaryl; or, $R^i$, $R^j$ together with the N atom to which they connect form a 4- to 8-membered saturated heterocyclic ring or a 7- to 11-membered spiro-heterocyclic ring;

preferably, $R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;

preferably, $R^i$ is selected from the group consisting of hydrogen, and C1-C6 alkyl, an $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the

group consisting of hydrogen, C1-C6 alkyl, and 5-membered heteroaryl;
more preferably, $R^i$ is selected from the group consisting of hydrogen, and C1-C6 alkyl, and $R^j$ is selected from the group consisting of hydrogen, C1-C6 alkyl, and isoxazolyl;
more preferably, $R^i$ is selected from the group consisting of hydrogen, and C1-C4 alkyl, and $R^j$ is selected from the group consisting of hydrogen, C1-C4 alkyl, and

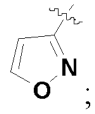;

most preferably, $R^i$ is selected from the group consisting of hydrogen, and methyl, and $R^j$ is selected from the group consisting of hydrogen, methyl, and

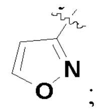;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

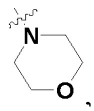,

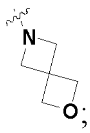;

$R^k$ is selected from the group consisting of hydrogen, C1-C6 alkyl, and C3-C6 cycloalkyl;
$R^l$, $R^m$ are each independently selected from the group consisting of hydrogen, C1-C6 alkyl, and C3-C6 cycloalkyl; or, $R^l$, $R^m$ together with the P atom to which they connect form a 4- to 6-membered heterocyclic ring;
preferably, $R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$, $R^l$, $R^m$ are methyl;
most preferably, $R^2$ is selected from the group consisting of chlorine, cyano, $F_3C$-,

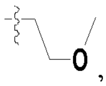,

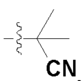, 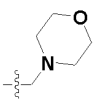, 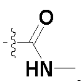, 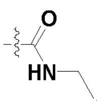, 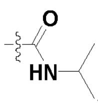, 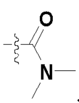, 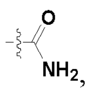, 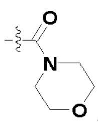, 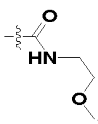, 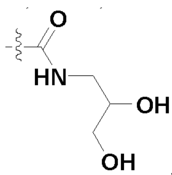,
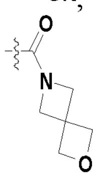, 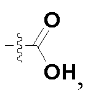, 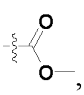, 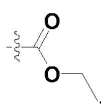, 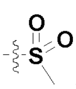, 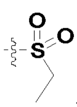, 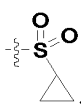, 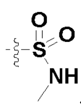, 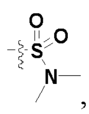, 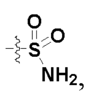, 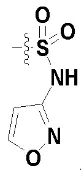, 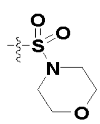,

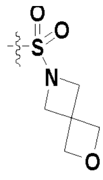, 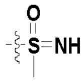, 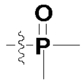, 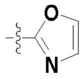, 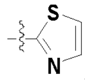, and 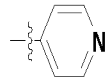;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;
more preferably, $R^3$ is selected from the group consisting of hydrogen, methyl,

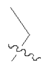,

$F_3C$-,

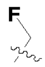, 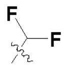, 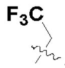, 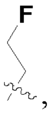, 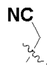, 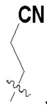, 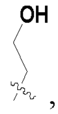, 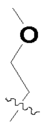, and 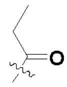;

most preferably, $R^3$ is methyl;
$R^4$ is selected from the group consisting of hydrogen, hydroxyl, halogen, and cyano;
preferably, $R^4$ is selected from the group consisting of hydrogen, and halogen;
preferably, $R^4$ is selected from the group consisting of hydrogen, and fluorine;
more preferably, $R^4$ is hydrogen;
$R^5$ is R'-NH-C(=O)-;
R' is C1-C6 alkyl;
preferably, $R^5$ is

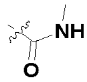;

$R^6$ is selected from the group consisting of hydrogen, hydroxyl, halogen halogen, and cyano;
preferably, $R^6$ is selected from the group consisting of hydrogen and halogen;
preferably, $R^6$ is selected from the group consisting of hydrogen and fluorine;
more preferably, $R^6$ is fluorine;
$R^7$ is selected from the group consisting of hydrogen, C1-C6 alkyl, cyano, cyano-C1-C6 alkylene, C1-C6 haloalkyl, C1-C6 alkyl substituted with 1 to 3 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, and the C1-C2 alkoxy-C1-C4 alkylene is substituted with hydroxyl;
further preferably, $R^7$ is selected from the group consisting of methyl,

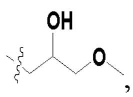,

and

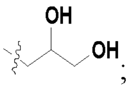;

most preferably, $R^7$ is methyl;

$R^8$ is $R^oR^pN$-;

$R^o$, $R^p$ are each independently selected from the group consisting of hydrogen, cyano-C1-C6 alkylene, C1-C6 haloalkyl, C1-C6 alkyl substituted with hydroxyl, C1-C6 alkyl, and C1-C6 alkoxy-C1-C6 alkylene; or, $R^o$ and $R^p$ together with the N atom to which they connect form a 4-to 7-membered saturated heterocyclic ring, a 7- to 11-membered spiro-heterocyclic ring or a 7-to 11-membered bridged heterocyclic ring;

preferably, $R^o$ and $R^p$ are each independently selected from the group consisting of C1-C6 alkyl, and C1-C6 alkoxy-C1-C6 alkylene; or, $R^o$ and $R^p$ together with the N atom to which they connect form a 5- to 6-membered saturated heterocyclic ring, a 7- to 9-membered spiro-heterocyclic ring, or a 7-membered bridged heterocyclic ring;

preferably, $R^o$ is C1-C6 alkyl, and $R^p$ is selected from the group consisting of C1-C6 alkyl, and C1-C6 alkoxy-C1-C6 alkylene;

more preferably, $R^o$ is C1-C4 alkyl, and $R^p$ is selected from the group consisting of C1-C4 alkyl, and C1-C2 alkoxy-C1-C2 alkylene;

most preferably, $R^o$ is methyl, and $R^p$ is selected from the group consisting of methyl, and

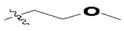;

or preferably, and $R^p$ together with the N atom to which they commonly connect form a 6-membered saturated heterocyclyl, a 7- to 9-membered spiro-heterocyclyl, or a 7-membered bridged heterocyclyl;

or more preferably, $R^o$ and $R^p$ together with the N atom to which they commonly connect form

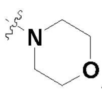, 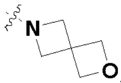, 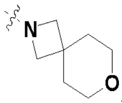, 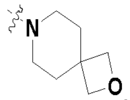, 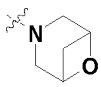;

preferably, $R^8$ is selected from the group consisting of

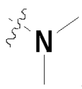, 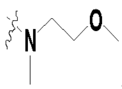, 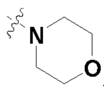, 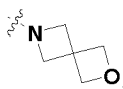,

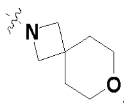, 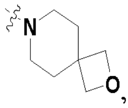, and 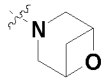;

$R^9$ is selected from the group consisting of hydrogen, hydroxyl, halogen, and cyano;

preferably, $R^9$ is selected from the group consisting of hydrogen, and hydroxyl;

preferably, $R^9$ is hydrogen;

$R^{10}$ is selected from the group consisting of hydrogen, hydroxyl, halogen, and cyano;

preferably, $R^{10}$ is selected from the group consisting of hydrogen, and halogen;

preferably, $R^{10}$ is selected from the group consisting of hydrogen, and fluorine;

more preferably, $R^{10}$ is hydrogen;

$R^{11}$ is selected from the group consisting of hydrogen, and C1-C6 alkyl;

preferably, $R^{11}$ is selected from the group consisting of hydrogen, and C1-C4 alkyl;

more preferably, $R^{11}$ is selected from the group consisting of hydrogen, and methyl;

most preferably, $R^{11}$ is hydrogen.

In the third aspect of the present invention, the present invention provides a compound of Formula I, or a stereoisomer, tautomer, prodrug, crystal form, hydrate, isotope-labeled compound, metabolite, ester, pharmaceutically acceptable salt or pharmaceutically acceptable solvate of the compound,

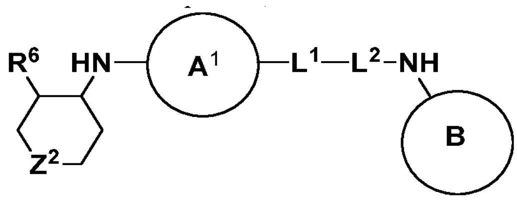

formula I

wherein:

Ring $A^1$ is

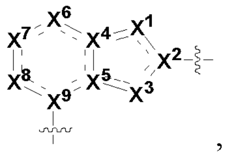

,

wherein the site at the lower left end is connected to NH, and the site at the upper right end is connected to L;
each ------ is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;
preferably, $R^a$ is selected from the group consisting of C1-C4 alkyl, C1-C2 haloalkyl, and cyano-C1-C2 alkylene;
more preferably, $R^a$ is selected from the group consisting of

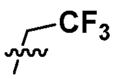 , 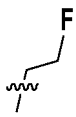 , 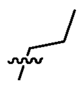 ,

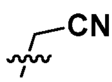 , and 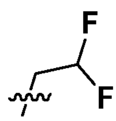 ;

most preferably, $R^a$ is

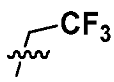 ;

$R^b$ is selected from the group consisting of hydrogen, and hydroxyl;
preferably, $R^b$ is hydrogen;
$R^c$ is selected from the group consisting of hydrogen and halogen;
preferably, $R^c$ is selected from the group consisting of hydrogen and fluorine;
more preferably, $R^c$ is hydrogen;
preferably, Ring $A^1$ is selected from the group consisting of

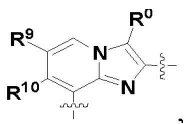 , 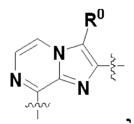 ,

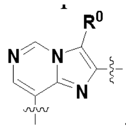, 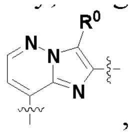, 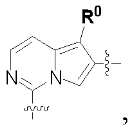, 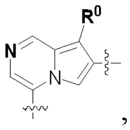, 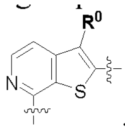, and 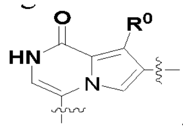,

wherein the site at the lower left end is connected to NH and the site at the upper right end is connected to $L^1$; more preferably, Ring $A^1$ is

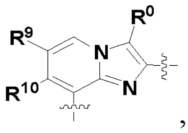,

wherein the site at the lower left end is connected to NH, and the site at the upper right end is connected to $L^1$; Ring B is selected from the group consisting of

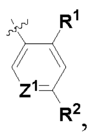, and 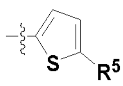;

preferably, Ring B is

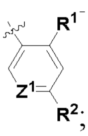;

$L^1$ is selected from the group consisting of

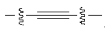,

and 5- to 6-membered heteroaryl; preferably, $L^1$ is selected from the group consisting of

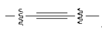,

and 5-membered heteroaryl; more preferably, $L^1$ is selected from the group consisting of

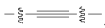,

1,3,4-thiadiazolylene , 1,2,4-oxadiazolylene, 1,3,4-oxadiazolylene, and thiazolylene; further preferably, $L^1$ is selected from the group consisting of

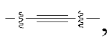, 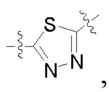, 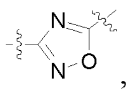,

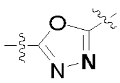, 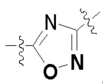, 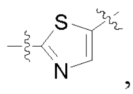, and 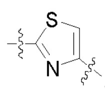,

wherein the site at the left side is connected to Ring $A^1$ and the site at the right side is connected to $L^2$; most preferably, $L^1$ is

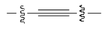;

$L^2$ is

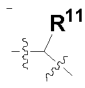;

$Z^2$ is selected from the group consisting of

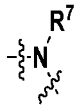, and 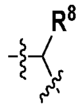;

$Z^1$ is selected from the group consisting of N, and $CR^4$;
$R^0$ is selected from the group consisting of C1-C6 alkyl, C1-C6 haloalkyl, and cyano-C1-C6 alkylene;
preferably, $R^0$ is selected from the group consisting of C1-C 4 alkyl, C1-C2 haloalkyl, and cyano-C1-C2 alkylene;
more preferably, $R^0$ is selected from the group consisting of

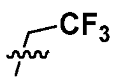, 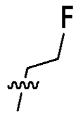, 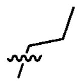,

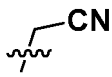, and 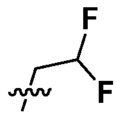;

most preferably, $R^0$ is

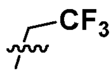;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

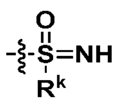, 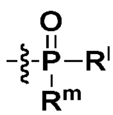,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

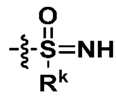, 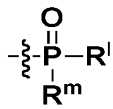,

and 5-to 6-membered heteroaryl;

more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$- C(=O)-, $R^gO$-C(=O)-, $R^hS(=O)_2$-, $R^iR^jN$-$S(=O)_2$-,

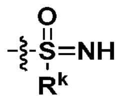, 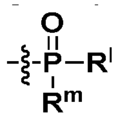,

oxazolyl, thiazolyl, and pyridyl;

$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;

preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, and $R^i$ is selected from the group consisting of hydrogen, methyl, ethyl, isopropyl,

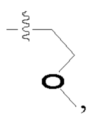, and 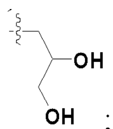;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which the connect form

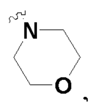,

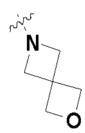;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated and heterocyclic ring or 7-membered spiro-heterocyclic ring;

preferably, $R^i$ is selected from the group consisting of hydrogen, and C1-C6 alkyl, and $R^j$ is selected from the group

consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, C1-C6 alkyl, and 5-membered heteroaryl;

further preferably, $R^i$ is selected from the group consisting of hydrogen, and C1-C6 alkyl, and $R^j$ is selected from the group consisting of hydrogen, C1-C6 alkyl, and isoxazolyl;

further preferably, $R^i$ is selected from the group consisting of hydrogen, and C1-C4 alkyl, and $R^j$ is selected from the group consisting of hydrogen, C1-C4 alkyl, and

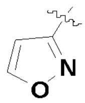;

most preferably, $R^1$ is selected from the group consisting of hydrogen, and methyl, and $R^j$ is selected from the group consisting of hydrogen, methyl, and

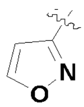;

or preferably, $R^i$, $R^j$ together with the N atom to which they connect form a 6-membered saturated heterocyclic ring or a 7-membered spiro-heterocyclic ring;

or more preferably, $R^1$, $R^j$ together with the N atom to which connect form

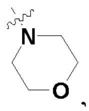,

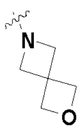;

$R^k$, $R^l$, $R^m$ are each independently selected from the group consisting of hydrogen, and C1-C6 alkyl;

preferably, $R^k$, $R^l$, $R^m$ are each independently C1-C6 alkyl;

more preferably, $R^k$, $R^l$, $R^m$ are each independently C1-C4 alkyl;

most preferably, $R^k$, $R^l$, $R^m$ are methyl;

most preferably, $R^2$ is selected from the group consisting of chlorine, cyano, $F_3C$-,

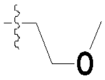,

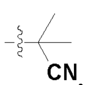, 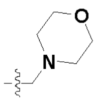, 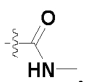, 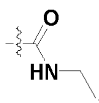, 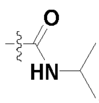, 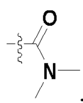, 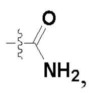, 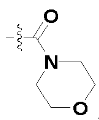, 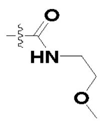, 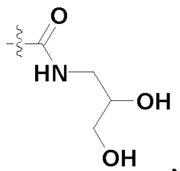,

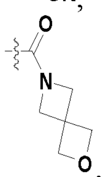, 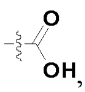, 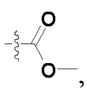, 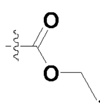, 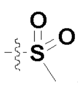, 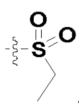, 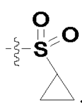, 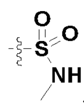, 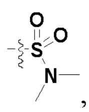, 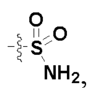, 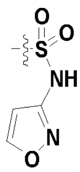, 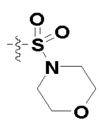,

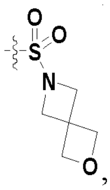, 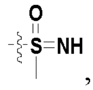, 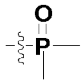, 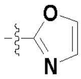, 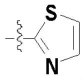, and 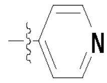;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=O)-;
more preferably, $R^3$ is selected from the group consisting of hydrogen, methyl,

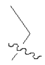,

$F_3C$-,

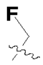, 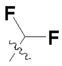, 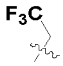, 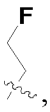, 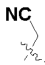, 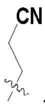, 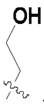, 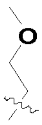, and 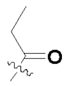;

most preferably, $R^3$ is methyl;
$R^4$ is selected from the group consisting of hydrogen and halogen;
preferably, $R^4$ is selected from the group consisting of hydrogen and fluorine;
preferably, $R^4$ is hydrogen;
$R^5$ is R'-NH-C(=O)-;
R' is C1-C6 alkyl;
preferably, R5 is

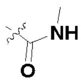;

$R^6$ is selected from the group consisting of hydrogen and halogen;
preferably, $R^6$ is selected from the group consisting of hydrogen and fluorine;
preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, and the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, and the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, and the C1-C2 alkoxy-C1-C4 alkylene is substituted with hydroxyl;
further preferably, $R^7$ is selected from the group consisting of methyl,

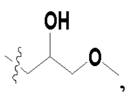, and

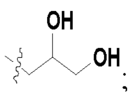;

most preferably, $R^7$ is methyl;
$R^8$ is $R^oR^pN$-;
$R^o$, $R^p$ are each independently selected from the group consisting of C1-C6 alkyl, and C1-C6 alkoxy-C1-C6 alkylene;

or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C6 alkyl, and C1-C6 alkoxy-C1-C6 alkylene;
more preferably, $R^o$ is C1-C4 alkyl, and $R^p$ is selected from the group consisting of C1-C4 alkyl, and C1-C2 alkoxy-C1-C2 alkylene;
most preferably, $R^o$ is methyl, and $R^p$ is selected from the group consisting of methyl, and

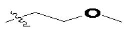;

or preferably, $R^o$ and $R^p$ together with the N atom to which they commonly connect form a 6-membered saturated heterocyclyl, 7- to 9-membered spiro-heterocyclyl, or 7-membered bridged heterocyclyl;
or more preferably, $R^o$ and $R^p$ together with the N atom to which they commonly connect form

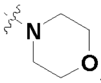, 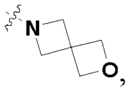, 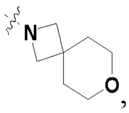, 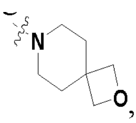, 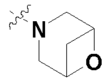;

preferably, $R^8$ is selected from the group consisting of

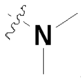, 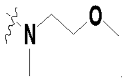, 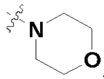, 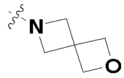,

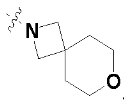, 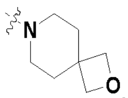, and 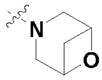;

$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the fourth aspect of the present invention, the present invention provides a compound of Formula I-1, or a stereoisomer, tautomer, prodrug, crystal form, hydrate, isotope-labeled compound, metabolite, ester, pharmaceutically acceptable salt or pharmaceutically acceptable solvate of the compound,

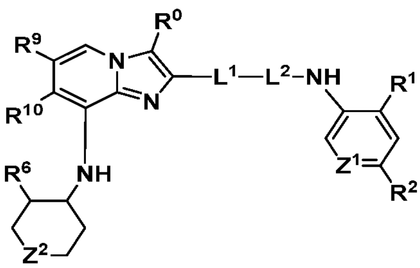

formula I-1

wherein:

$L^1$ is selected from the group consisting of

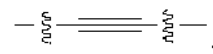,

and 5- to 6-membered heteroarylene;

preferably, $L^1$ is selected from the group consisting of

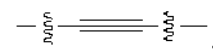,

and 5-membered heteroarylene;

more preferably, $L^1$ is selected from the group consisting of

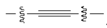,

1,3,4-thiadiazolylene, 1,2,4-oxadiazolylene, 1,3,4-oxadiazolylene, and thiazolylene;

further preferably, $L^1$ is selected from the group consisting of

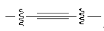, 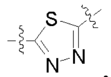, 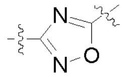,

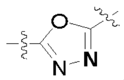, 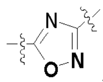, 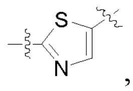, and 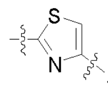,

wherein the site at the left side e is connected to the imidazole ring, and the site at the right side is connected to $L^2$;

most preferably, $L^1$ is

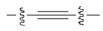;

$L^2$ is 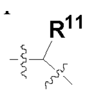;

$Z^2$ is selected from the group consisting of

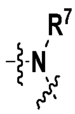, and 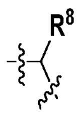;

$Z^1$ is selected from the group consisting of N, and $CR^4$;

$R^0$ is selected from the group consisting of C1-C6 alkyl, C1-C6 haloalkyl, and cyano-C1-C6 alkylene;

preferably, $R^0$ is selected from the group consisting of C1-C4 alkyl, C1-C2 haloalkyl, and cyano-C1-C2 alkylene;

more preferably, $R^0$ is selected from the group consisting of

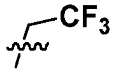, 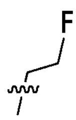, 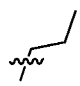,

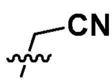, and 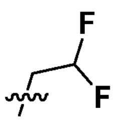;

most preferably, $R^0$ is

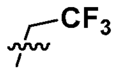
;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^1R^jN$-$S(=O)_2$-,

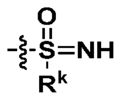, 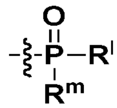,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS$(=O )2-, $R^iR^jN$-$S(=O)_2$-,

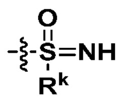, 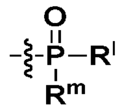,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^1R^jN$-$S(=O)_2$-,

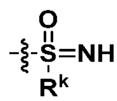, 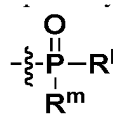,

oxazolyl, thiazolyl, and pyridyl;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;
preferably, $R^e$ is selected from the group consisting of hydrogen, and C1-C6 alkyl, $R^f$ is selected from the group consisting of hydrogen, C1-C6 alkyl, C1-C6 alkoxy-C1-C6 alkylene, and C1-C6 alkyl substituted with 2 hydroxyl groups;
more preferably, $R^e$ is selected from the group consisting of hydrogen, C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;
most preferably, $R^e$ is selected from the group consisting of hydrogen, and methyl, and $R^f$ is selected from the group consisting of hydrogen, methyl, ethyl, isopronyl,

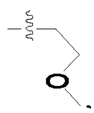, and 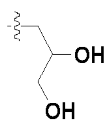;

or preferably, $R^e$, $R^f$ together with the N atom to which they connect form a 6-membered saturated heterocyclic ring or a 7-membered spiro-heterocyclic ring;
or more preferably, $R^e$, $R^f$ together with the N atom to which the connect form

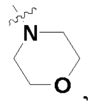
,

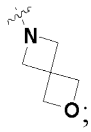
;

$R^g$ is selected from the group consisting of hydrogen, and C1-C6 alkyl;
preferably, $R^g$ is selected from the group consisting of hydrogen, and C1-C4 alkyl;
more preferably, $R^g$ is selected from the group consisting of hydrogen, methyl, and ethyl;
$R^h$ is selected from the group consisting of C1-C6 alkyl, and C3-C6 cycloalkyl;
preferably, $R^h$ is selected from the group consisting of C1-C4 alkyl, and C3-C6 cycloalkyl;
more preferably, $R^h$ is selected from the group consisting of methyl, ethyl, and cyclopropyl;
$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or 7-membered spiro-heterocyclic ring;
preferably, $R^i$ is selected from the group consisting of hydrogen, and C1-C6 alkyl, and $R^j$ is selected from the group consisting of hydrogen, C1-C6 alkyl, and 5- to 6-membered heteroaryl;
more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

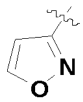
;

most preferably, $R^i$ is selected from the group consisting of hydrogen, and methyl, and $R^j$ is selected from the group consisting of hydrogen, methyl, and

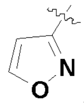
;

or preferably, R’, $R^j$ together with the N atom to which they connect form a 6-membered saturated heterocyclic ring or a 7-membered spiro-heterocyclic ring;
or more preferably, $R^i$, $R^j$ together with the N atom to which they connect form

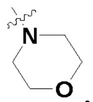
,

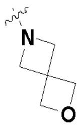
;

$R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, Rl and $R^m$ are each independently C1-C4 alkyl;

most preferably, $R^k$, $R^l$, and $R^m$ are methyl;

most preferably, $R^2$ is selected from the group consisting of chlorine, cyano, $F_3C$-,

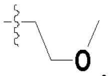,

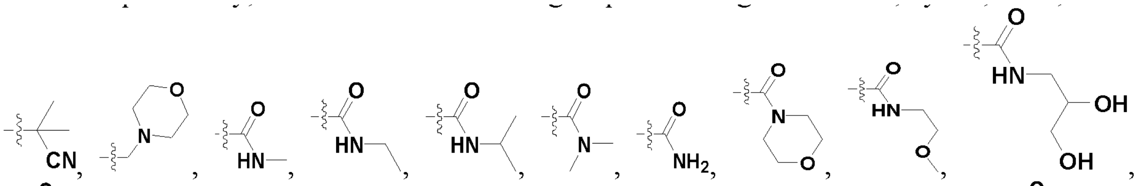,

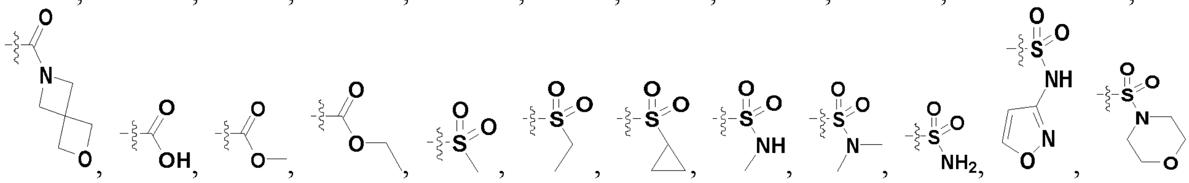,

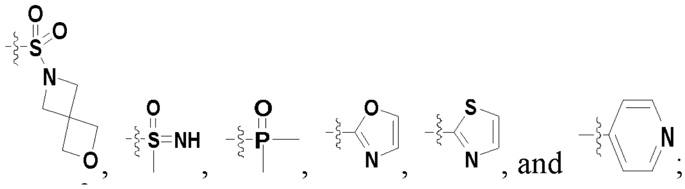, and ;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;

preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;

more preferably, $R^3$ is selected from the group consisting of hydrogen, methyl,

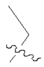, $F_3C$-,

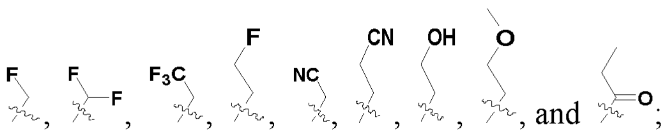, and ;

most preferably, $R^3$ is methyl;

$R^4$ is selected from the group consisting of hydrogen, and halogen;

preferably, $R^4$ is selected from the group consisting of hydrogen, and fluorine;

more preferably, $R^4$ is hydrogen;

$R^6$ is selected from the group consisting of hydrogen, and halogen;

preferably, $R^6$ is selected from the group consisting of hydrogen and fluorine;

more preferably, $R^6$ is fluorine;

$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

more preferably, $R^7$ is selected from the group consisting of C1-C4 alkyl, C1-C4 alkyl substituted with 2 hydroxyl groups, and C1-C2 alkoxy-C1-C4 alkylene, and the C1-C2 alkoxy-C1-C4 alkylene is substituted with hydroxyl;

further preferably, $R^7$ is selected from the group consisting of methyl,

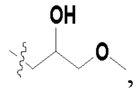,

and

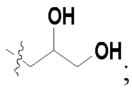;

most preferably, $R^7$ is methyl;

$R^8$ is $R^oR^pN$-;

$R^o$, $R^p$ are each independently selected from the group consisting of C1-C6 alkyl, and C1-C6 alkoxy-C1-C6 alkylene;

or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;

preferably, $R^o$ is C1-C6 alkyl, and $R^p$ is selected from the group consisting of C1-C6 alkyl, and C1-C6 alkoxy-C1-C6 alkylene;

more preferably, $R^o$ is C1-C4 alkyl, and $R^p$ is selected from the group consisting of C1-C4 alkyl, and C1-C2 alkoxy-C1-C2 alkylene;

most preferably, $R^o$ is methyl, and $R^p$ is selected from the group consisting of methyl, and

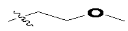;

or preferably, $R^o$ and $R^p$ together with the N atom to which they commonly connect form a 6-membered saturated heterocyclyl, a 7- to 9-membered spiro-heterocyclyl, or a 7-membered bridged heterocyclyl;

or more preferably, $R^o$ and $R^p$ together with the N atom to which they commonly connect form

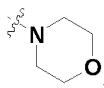, 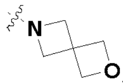, 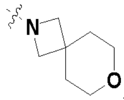, 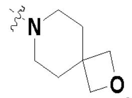, 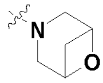;

preferably, $R^8$ is selected from the group consisting of

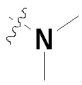, 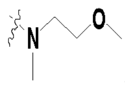, 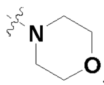, 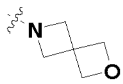,

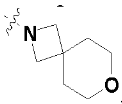, 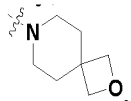, and 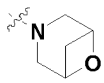;

$R^9$ is selected from the group consisting of hydrogen and hydroxyl;

preferably, $R^9$ is hydrogen;

$R^{10}$ is selected from the group consisting of hydrogen and halogen;

preferably, $R^{10}$ is selected from the group consisting of hydrogen and fluorine;

more preferably, $R^{10}$ is hydrogen;

$R^{11}$ is selected from the group consisting of hydrogen, and C1-C6 alkyl;

preferably, $R^{11}$ is selected from the group consisting of hydrogen, and C1-C4 alkyl;

more preferably, $R^{11}$ is selected from the group consisting of hydrogen, and methyl;

most preferably, $R^{11}$ is hydrogen.

In the fifth aspect of the present invention, the present invention provides a compound represented by Formula I-1-1, or a stereoisomer, tautomer, prodrug, crystalline form, hydrate, isotope-labeled compound, metabolite, ester, pharmaceutically acceptable salt or pharmaceutically acceptable solvate of the compound, wherein,

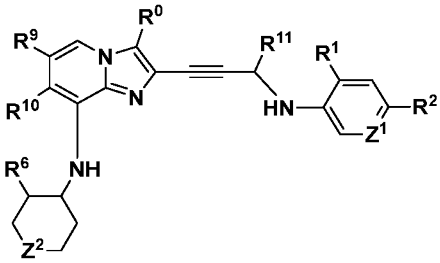

Formula I-1-1

wherein:

$Z^2$ is selected from the group consisting of

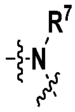, and 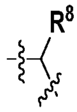;

$Z^1$ is selected from the group consisting of N, and $CR^4$;
$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

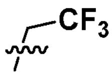, 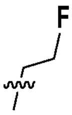, 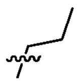,

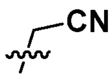, and 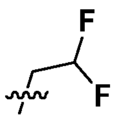;

most preferably, $R^0$ is

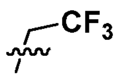;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(= O)-, $R^hS(=O)_2$-, $R^iR^jN$-$S(=O)_2$-,

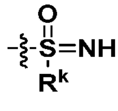, 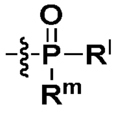,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

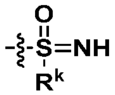, 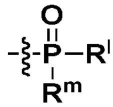,

and 5-to 6-membered heteroaryl;

more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^1R^jN$-$S(=O)_2$-,

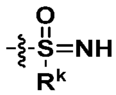, 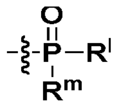,

oxazolyl, thiazolyl, and pyridyl;

$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;

preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, and $R^f$ is selected from the group consisting of hydrogen, methyl, ethyl, isopropyl,

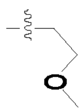, and 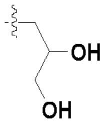;

or preferably, $R^e$, $R^f$ together with the N atom to which they connect form a 6-membered saturated heterocyclic ring or 7-membered spiro-heterocyclic ring;

or more preferably, $R^e$, $R^f$ together with the N atom to which the connect form

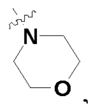,

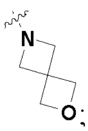;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;

preferably, $R^i$ is selected from the group consisting of hydrogen, and C1-C6 alkyl, and $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

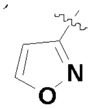 ;

most preferably, $R^i$ is selected from the group consisting of hydrogen, and methyl, and $R^j$ is selected from the group consisting of hydrogen, methyl, and

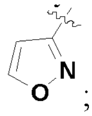 ;

or preferably, $R^i$, $R^j$ together with the N atom to which they commonly connect form a 6-membered saturated heterocyclic ring or a 7-membered spiro-heterocyclic ring;
or more preferably, $R^i$, $R^j$ together with the N atom to which they are commonly connect form

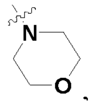, 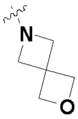;

$R^k$, $R^l$, and $R^m$ are each independently selected from the group consisting of hydrogen, and C1-C6 alkyl;
preferably, $R^k$, $R^l$, and $R^m$ are each independently C1-C6 alkyl;
more preferably, $R^k$, $R^l$, and $R^m$ are each independently C1-C4 alkyl;
most preferably, $R^k$ $R^l$, and $R^m$ are methyl;
most preferably, $R^2$ is selected from the group consisting of chlorine, cyano, $F_3C$-,

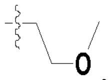,

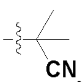, 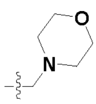, 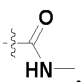, 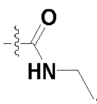, 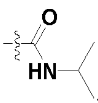, 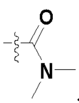, 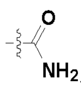, 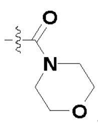, 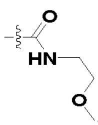, 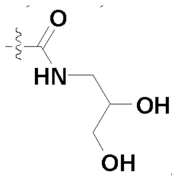,
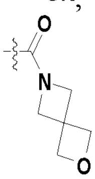, 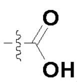, 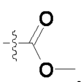, 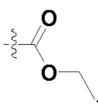, 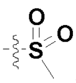, 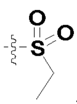, 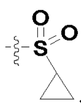, 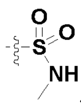, 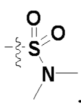, 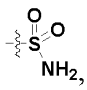, 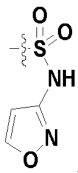, 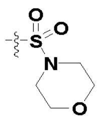,

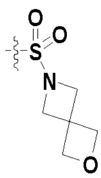, 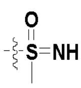, 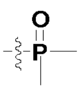, 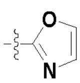, 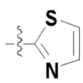, and 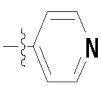;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=O)-;
more preferably, $R^3$ is selected from the group consisting of hydrogen, methyl,

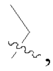,

$F_3C$-,

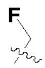, 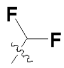, 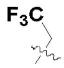, 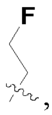, 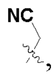, 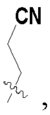, 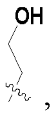, 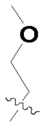, and 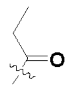;

most preferably, $R^3$ is methyl;
$R^4$ is selected from the group consisting of hydrogen and halogen;
preferably, $R^4$ is selected from the group consisting of hydrogen and fluorine;
more preferably, $R^4$ is hydrogen;
$R^6$ is selected from the group consisting of hydrogen and halogen;
preferably, $R^6$ is selected from the group consisting of hydrogen and fluorine;
more preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
further preferably, $R^7$ is selected from the group consisting of methyl,

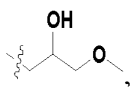,

and

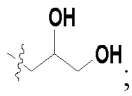;

most preferably, $R^7$ is methyl;
$R^8$ is $R^oR^pN$-;
$R^o$ and $R^p$ are independently selected from the group consisting of C1-C6 alkyl, and C1-C6 alkoxy-C1-C6 alkylene; or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C6 alkyl, and C1-C6 alkoxy-C1-C6 alkylene;
more preferably, $R^o$ is C1-C4 alkyl, and $R^p$ is selected from the group consisting of C1-C4 alkyl, and C1-C2 alkoxy -C1-C2 alkylene;
most preferably, $R^o$ is methyl, and $R^p$ is selected from the group consisting of methyl, and

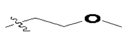;

or preferably, $R^o$ and $R^p$ together with the N atom to which they commonly connect form a 6-membered saturated heterocyclyl, a 7- to 9-membered spiro-heterocyclyl, or a 7-membered bridged heterocyclyl;
or more preferably, $R^o$ and $R^p$ together with the N atom to which they commonly connect form

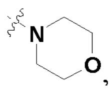, 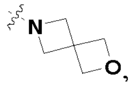, 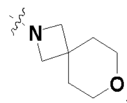, 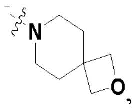, 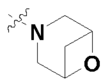;

preferably, $R^8$ is selected from the group consisting of

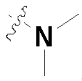, 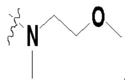, 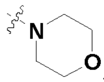, 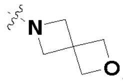,

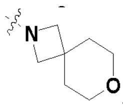, 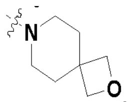, and 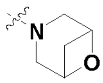;

$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the sixth aspect of the present invention, the present invention provides a compound represented by Formula I-1-1-1, or a stereoisomer, tautomer, prodrug, crystal form, hydrate, isotope-labeled compound, metabolite, ester, pharmaceutically acceptable salt or pharmaceutically acceptable solvate of the compound,

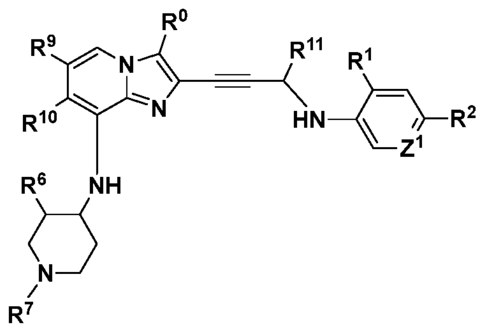

Formula I-1-1-1

wherein:

$Z^1$ is selected from the group consisting of N, and $CR^4$;
$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

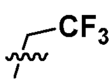, 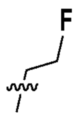, 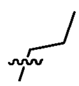,

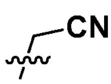, and 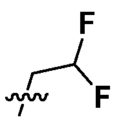;

most preferably, $R^0$ is

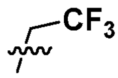
;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

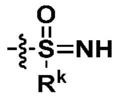, 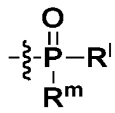,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

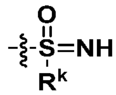, 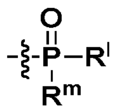,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-$S(=O)_2$-,

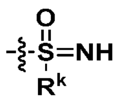, 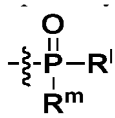,

oxazolyl, thiazolyl, and pyridyl;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;
preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;
more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;
most preferably, $R^e$ is selected from the group consisting of hydrogen, and methyl, and $R^f$ is selected from the group consisting of hydrogen, methyl, ethyl, isopropyl,

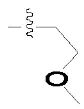, and 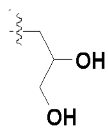;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which they connect form

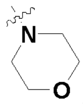
,

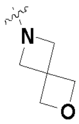
;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;

preferably, $R^i$ is selected from the group consisting of hydrogen, and C1-C6 alkyl, and $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, C1-C6 alkyl, and 5-membered heteroaryl;

further preferably, $R^i$ is selected from the group consisting of hydrogen, and C1-C6 alkyl, and $R^j$ is selected from the group consisting of hydrogen, C1-C6 alkyl, and isoxazolyl;

further preferably, $R^i$ is selected from the group consisting of hydrogen, and C1-C4 alkyl, and $R^j$ is selected from the group consisting of hydrogen, C1-C4 alkyl, and

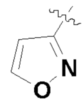
;

most preferably, $R^i$ is selected from the group consisting of hydrogen, and methyl, and $R^j$ is selected from the group consisting of hydrogen, methyl, and

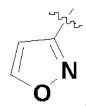
;

or preferably, $R^i$, $R^j$ together with the N atom to which they connect form a 6-membered saturated heterocyclic ring or a 7-membered spiro-heterocyclic ring;

or more preferably, $R^i$, $R^j$ together with the N atom to which they commonly connect form

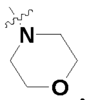
, 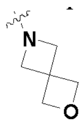
;

$R^k$, $R^l$, $R^m$ are each independently selected from the group consisting of hydrogen, and C1-C6 alkyl;

preferably, $R^k$, $R^l$, $R^m$ are each independently C1-C6 alkyl;

more preferably, $R^k$, $R^l$, $R^m$ are each independently C1-C4 alkyl;

most preferably, $R^k$, $R^l$, $R^m$ are methyl;

most preferably, $R^2$ is selected from the group consisting of chlorine, cyano, $F_3C$-,

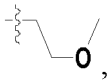,

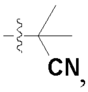, 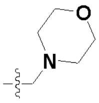, 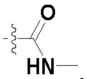, 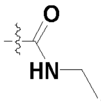, 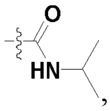, 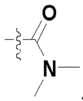, 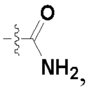, 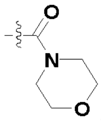, 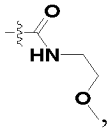, 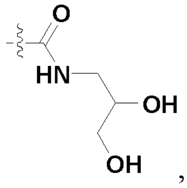,

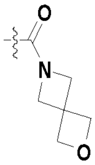, 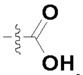, 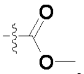, 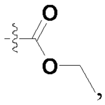, 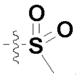, 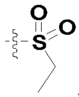, 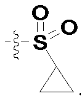, 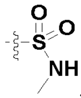, 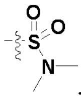, 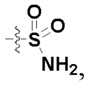, 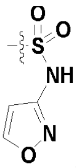, 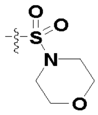,

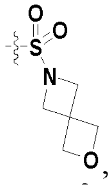, 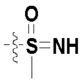, 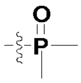, 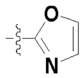, 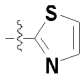, and 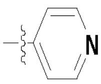;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=O)-;
more preferably, $R^3$ is selected from the group consisting of hydrogen, methyl,

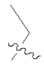,

$F_3C$-,

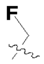, 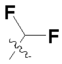, 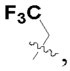, 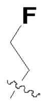, 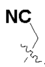, 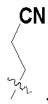, 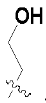, 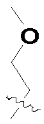, and 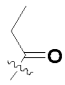;

most preferably, $R^3$ is methyl;
$R^4$ is selected from the group consisting of hydrogen, and halogen;
preferably, $R^4$ is selected from the group consisting of hydrogen, and fluorine;
more preferably, $R^4$ is hydrogen;
$R^6$ is selected from the group consisting of hydrogen, and halogen;
preferably, $R^6$ is selected from the group consisting of hydrogen, and fluorine;
more preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
further preferably, $R^7$ is selected from the group consisting of methyl,

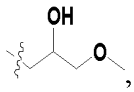,

and

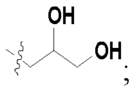;

most preferably, $R^7$ is methyl;
$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the seventh aspect of the present invention, the present invention provides a compound represented by Formula I-1-1-1a, or a stereoisomer, tautomer, prodrug, crystal form, hydrate, isotope-labeled compound, metabolite, ester, pharmaceutically acceptable salt or pharmaceutically acceptable solvate of the compound,

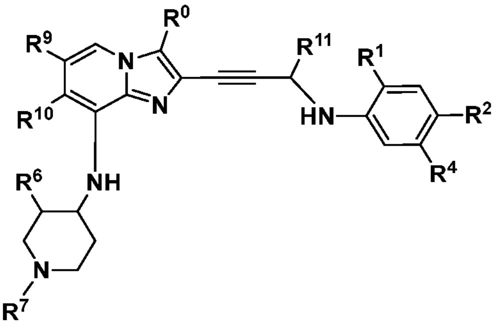

Formula I-1-1-1a

wherein:

$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

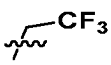, 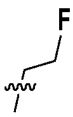, 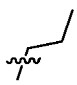,

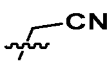, and 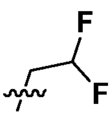;

most preferably, $R^0$ is

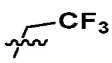;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

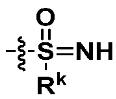, 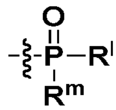,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^j$-$S(=O)_2$-,

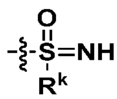, 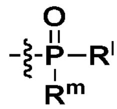,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^j$-$S(=O)_2$-,

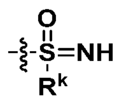, 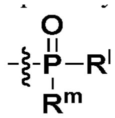,

oxazolyl, and thiazolyl, pyridyl;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;
preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;
more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;
most preferably, $R^e$ is selected from the group consisting of hydrogen, and methyl, and $R^f$ is selected from the group consisting of hydrogen, methyl, ethyl,

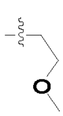, and 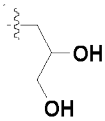;

or preferably, $R^e$, $R^f$ together with the N atom to which they connect form a 6-membered saturated heterocyclic ring or a 7-membered spiro-heterocyclic ring;
or more preferably, $R^e$, $R^f$ and the N atom to which they connect form

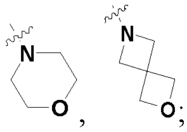

$R^g$ is selected from the group consisting of hydrogen, and C1-C6 alkyl;
preferably, $R^g$ is selected from the group consisting of hydrogen, and C1-C4 alkyl;
more preferably, $R^g$ is selected from the group consisting of hydrogen, methyl, and ethyl;
$R^h$ is selected from the group consisting of C1-C6 alkyl, and C3-C6 cycloalkyl;
preferably, $R^h$ is selected from the group consisting of C1-C4 alkyl, and C3-C6 cycloalkyl;
more preferably, $R^h$ is selected from the group consisting of methyl, ethyl, and cyclopropyl;
$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;
preferably, $R^i$ is selected from the group consisting of hydrogen, and C1-C6 alkyl, and $R^j$ is selected from the group consisting of hydrogen, C1-C6 alkyl, and 5- to 6-membered heteroaryl;
more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

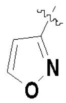

;

most preferably, $R^i$ is selected from the group consisting of hydrogen, and methyl, and $R^j$ is selected from the group consisting of hydrogen, methyl, and

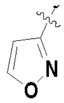

;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

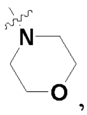

,

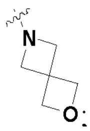

;

$R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$, $R^l$, $R^m$ are methyl;
most preferably, $R^2$ is selected from the group consisting of chlorine, cyano, $F_3C$-,

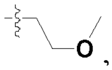

,

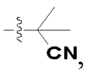, 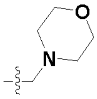, 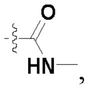, 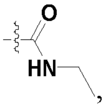, 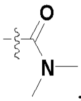, 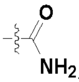, 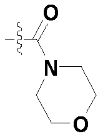, 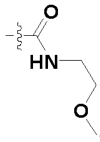, 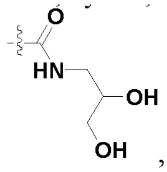, 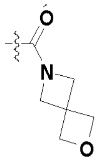,

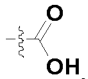, 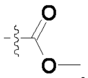, 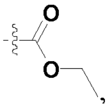, 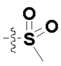, 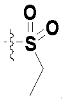, 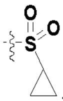, 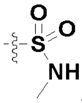, 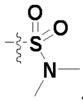, 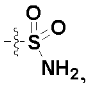, 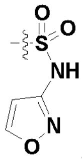, 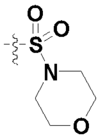, 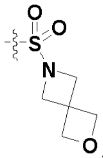,

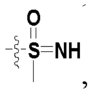, 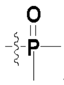, 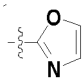, 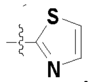, and 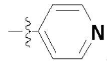;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=O)-;
more preferably, $R^3$ is selected from the group consisting of hydrogen, methyl,

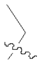,

$F_3C$-,

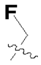, 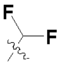, 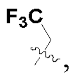, 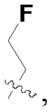, 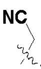, 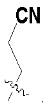, 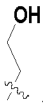, 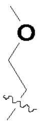, and 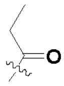;

most preferably, $R^3$ is methyl;
$R^4$ is selected from the group consisting of hydrogen, and halogen;
preferably, $R^4$ is selected from the group consisting of hydrogen and fluorine;
more preferably, $R^4$ is hydrogen;
$R^6$ is selected from the group consisting of hydrogen and halogen;
preferably, $R^6$ is selected from the group consisting of hydrogen and fluorine;
more preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
further preferably, $R^7$ is selected from the group consisting of methyl,

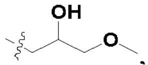,

and

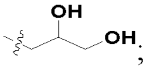;

most preferably, $R^7$ is methyl;

$R^9$ is selected from the group consisting of hydrogen, and hydroxyl groups;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the eighth aspect of the present invention, the present invention provides a compound represented by Formula I-1-1-1b, or a stereoisomer, tautomer, prodrug, crystal form, hydrate, isotope-labeled compound, metabolite, ester, pharmaceutically acceptable salt or pharmaceutically acceptable solvate of the compound,

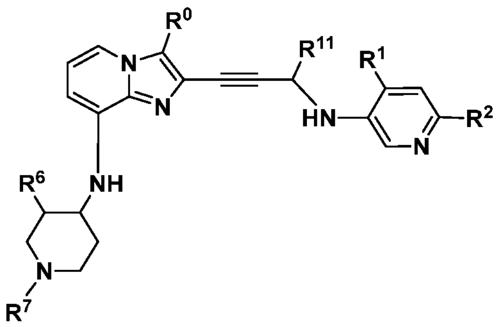

Formula I-1-1-1b

wherein:

$R^0$ is C1-C6 haloalkyl;
preferably. $R^0$ is C1-C2 haloalkyl;
more preferably, $R^0$ is

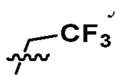;

$R^1$ is $R^3O$-;
$R^2$ is selected from the group consisting of cyano, and $R^eR^fN$-C(=O)-;
$R^e$, $R^f$ are each independently selected from the group consisting of hydrogen, and C1-C6 alkyl;
preferably, $R^e$ is hydrogen, and $R^f$ is C1-C6 alkyl;
more preferably, $R^e$ is hydrogen, and $R^f$ is C1-C4 alkyl;
most preferably, $R^e$ is hydrogen, and $R^f$ is selected from the group consisting of ethyl, and isopropyl;
preferably, $R^2$ is selected from the group consisting of cyano,

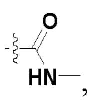, and 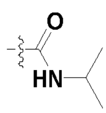;

$R^3$ is C1-C6 alkyl;
preferably, $R^3$ is C1-C4 alkyl;
more preferably, $R^3$ is methyl;
$R^6$ is halogen;
preferably, $R^6$ is fluorine;
$R^7$ is C1-C6 alkyl;
preferably, $R^7$ is C1-C4 alkyl;
more preferably, $R^7$ is methyl;
$R^{11}$ is selected from the group consisting of hydrogen, and C1-C6 alkyl;
preferably, $R^{11}$ is selected from the group consisting of hydrogen, and C1-C4 alkyl;

more preferably, $R^{11}$ is selected from the group consisting of hydrogen, and methyl;
most preferably, $R^{11}$ is hydrogen.

In the ninth aspect of the present invention, the present invention provides a compound represented by Formula 1-1-1-2, or a stereoisomer, tautomer, prodrug, crystal form, hydrate, isotope-labeled compound, metabolite, ester, pharmaceutically acceptable salt or pharmaceutically acceptable solvate of the compound,

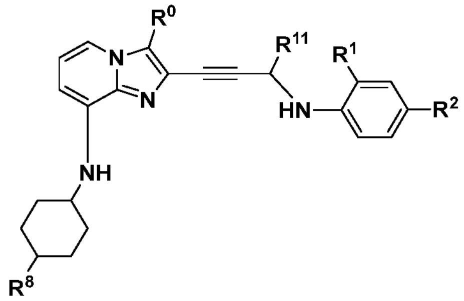

Formula I-1-1-2

wherein:

$R^0$ is C1-C6 haloalkyl;
preferably, $R^0$ is C1-C2 haloalkyl;
more preferably, $R^0$ is

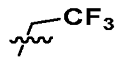
;

$R^1$ is $R^3O$-;
$R^2$ is $R^eR^fN$ -C(=O)-;
$R^e$, $R^f$ are each independently selected from the group consisting of hydrogen, and C1-C6 alkyl;
preferably, $R^e$ is hydrogen, and $R^f$ is C1-C6 alkyl;
more preferably, $R^e$ is hydrogen, and $R^f$ is C1-C4 alkyl;
most preferably, $R^e$ is hydrogen, and $R^f$ is methyl;
preferably, $R^2$ is

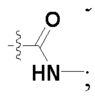
;

$R^3$ is C1-C6 alkyl;
preferably, $R^3$ is C1-C4 alkyl;
more preferably, $R^3$ is methyl;
$R^8$ is $R^oR^pN$-;
$R^o$, $R^p$ are each independently selected from the group consisting of C1-C6 alkyl, and C1-C6 alkoxy-C1-C6 alkylene;
or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C6 alkyl, and C1-C6 alkoxy-C1-C6 alkylene;
more preferably, $R^o$ is C1-C4 alkyl, and $R^p$ is selected from the group consisting of C1-C4 alkyl, and C1-C2 alkoxy-C1-C2 alkylene;
most preferably, $R^o$ is methyl, and R p is selected from the group consisting of methyl, and

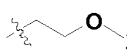
;

or preferably, $R^o$ and $R^p$ together with the N atom to which they commonly connect form a 6-membered saturated heterocyclyl, a 7- to 9-membered spiro-heterocyclyl, or a 7-membered bridged heterocyclyl;
or more preferably, $R^o$ and $R^p$ together with the N atom to which they commonly connect form

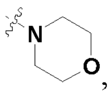, 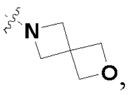, 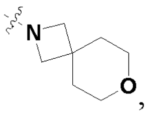, 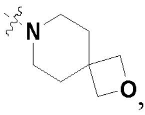, 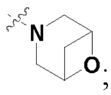;

preferably, $R^8$ is selected from the group consisting of

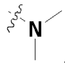, 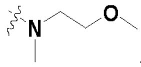, 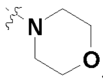, 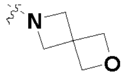,

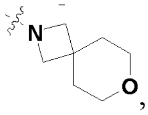, 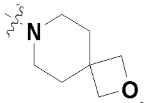, and 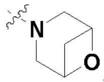;

$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the tenth aspect of the present invention, the present invention provides a compound represented by Formula 1-1-2, or a stereoisomer, tautomer, prodrug, crystal form, hydrate, isotope-labeled compound, metabolite, ester, pharmaceutically acceptable salt or pharmaceutically acceptable solvate of the compound,

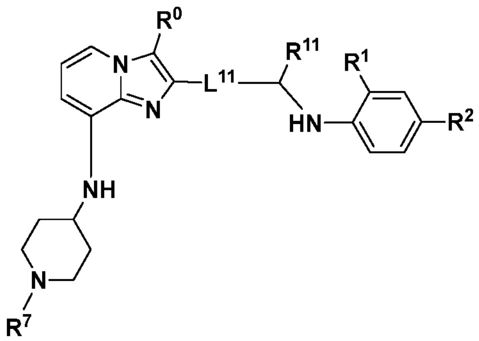

Formula I-1-2

wherein:

$L^{11}$ is 5- to 6-membered heteroarylene;
preferably, $L^{11}$ is 5-membered heteroarylene;
more preferably, $L^{11}$ is selected from the group consisting of 1,3,4-thiadiazolylene, 1,2,4-oxadiazolylene, 1,3,4-oxadiazolylene, and thiazolylene;
most preferably, $L^{11}$ is selected from the group consisting of

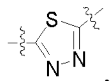, 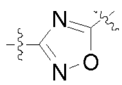, 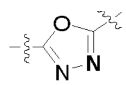,

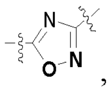, 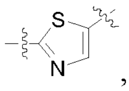, and 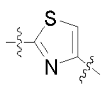,

wherein the site on the left side is connected to the imidazole ring, and the site on the right side is connected to the alkylene/alkylidene;
$R^0$ is C1-C6 haloalkyl;
preferably, $R^0$ is C1-C2 haloalkyl;
more preferably, $R^0$ is

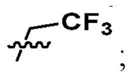

;

$R^1$ is $R^3O$-;
$R^2$ is $R^eR^fN$-C(=O)-;
$R^e$, $R^f$ are each independently selected from the group consisting of hydrogen, and C1-C6 alkyl;
preferably, $R^e$ is hydrogen, and $R^f$ is C1-C6 alkyl;
more preferably, $R^e$ is hydrogen, and $R^f$ is C1-C4 alkyl;
most preferably, $R^e$ is hydrogen, and $R^f$ is methyl;
preferably, $R^2$ is

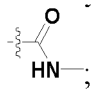

;

$R^3$ is C1-C6 alkyl;
preferably, $R^3$ is C1-C4 alkyl;
more preferably, $R^3$ is methyl;
$R^7$ is C1-C6 alkyl;
preferably, $R^7$ is C1-C4 alkyl;
more preferably, $R^7$ is methyl;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the eleventh aspect of the present invention, the present invention provides a compound represented by Formula 1-2, or a stereoisomer, tautomer, prodrug, crystal form, hydrate, isotope-labeled compound, metabolite, ester, pharmaceutically acceptable salt or pharmaceutically acceptable solvate of the compound,

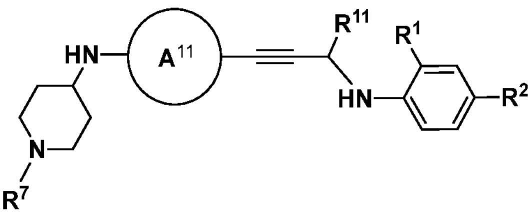

Formula I-2

wherein:

Ring $A^{11}$ is selected from the group consisting of

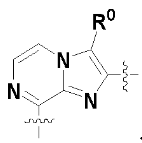
,
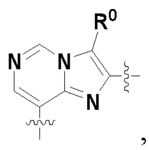
,
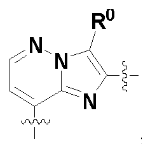
,

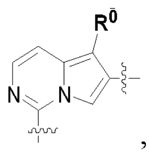
,
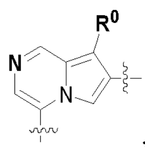
,
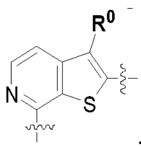
, and
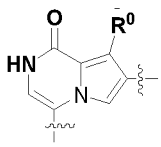
,

wherein the site at the lower left end is connected to NH, and the site at the upper right end is connected to the alkynyl;
$R^0$ is C1-C6 haloalkyl;
preferably, $R^0$ is C1-C2 haloalkyl;
more preferably, $R^0$ is

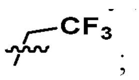;

$R^1$ is $R^3O$-;
$R^2$ is selected from the group consisting of $R^eR^fN$-C(=O)-, and $R^hS(=O)_2$-;
$R^e$, $R^f$ are each independently selected from the group consisting of hydrogen, and C1-C6 alkyl;
preferably, $R^e$ is hydrogen, and $R^f$ is C1-C6 alkyl;
more preferably, $R^e$ is hydrogen, and $R^f$ is C1-C4 alkyl;
most preferably, $R^e$ is hydrogen, and $R^f$ is methyl;
$R^h$ is C1-C6 alkyl;
preferably, $R^h$ is C1-C4 alkyl;
more preferably, $R^h$ is methyl;
preferably, $R^2$ is

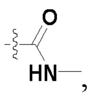, or 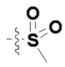;

$R^3$ is C1-C6 alkyl;
preferably, $R^3$ is C1-C4 alkyl;
more preferably, $R^3$ is methyl;
$R^7$ is C1-C6 alkyl;
preferably, $R^7$ is C1-C4 alkyl;
more preferably, $R^7$ is methyl;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the twelfth aspect of the present invention, the present invention provides a compound represented by Formula 1-3, or a stereoisomer, tautomer, prodrug, crystal form, hydrate, isotope-labeled compound, metabolite, ester, pharmaceutically acceptable salt or pharmaceutically acceptable solvate of the compound,

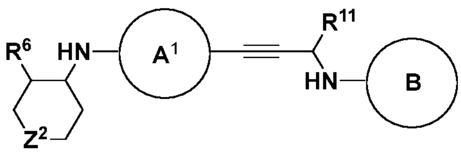

Formula I-3

wherein:

Ring $A^1$ is

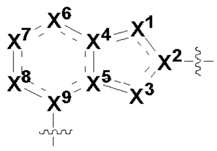,

wherein the site at the lower left end is connected to NH, and the site at the upper right end is connected to the alkynyl;
each ------ is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene; preferably, $R^a$ is selected from the group consisting of C1-C4 alkyl, C1-C2 haloalkyl, and cyano-C1-C2 alkylene; more preferably, $R^a$ is selected from the group consisting of

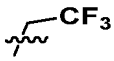, 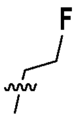, 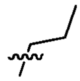,

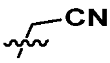, and 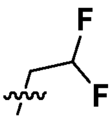;

most preferably, $R^a$ is

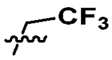;

$R^b$ is selected from the group consisting of hydrogen and hydroxyl;
preferably, $R^b$ is hydrogen;
$R^c$ is selected from the group consisting of hydrogen and halogen;
preferably, $R^c$ is selected from the group consisting of hydrogen and fluorine;
more preferably, $R^c$ is hydrogen;
preferably, Ring $A^1$ is selected from the group consisting of

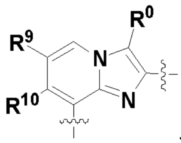, 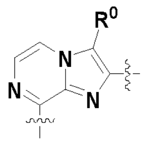,

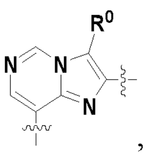, 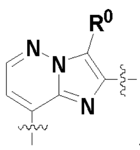, 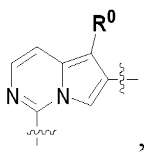, 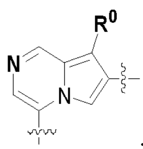, 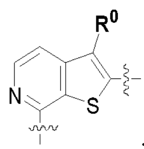, and 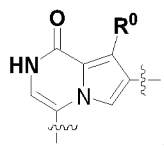,

wherein the site at the lower left end is connected to NH, and the site at the upper right end is connected to the alkynyl; more preferably, Ring $A^1$ is

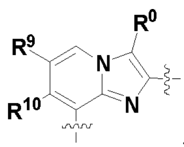,

wherein the site at the lower left end is connected to NH, and the site at the upper right end is connected to the alkynyl; Ring B is selected from the group consisting of

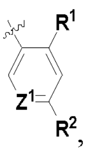, and 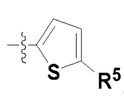;

preferably, Ring B is

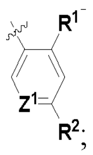,

$Z^2$ is selected from the group consisting of

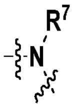, and 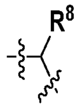;

$Z^1$ is selected from the group consisting of N, and $CR^4$;
$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

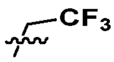, 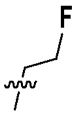, 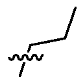,

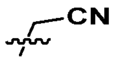, and 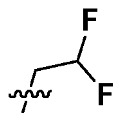;

most preferably, $R^0$ is

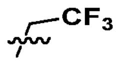;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^j$-$S(=O)_2$-,

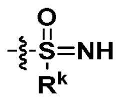, 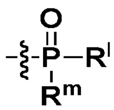,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

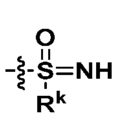, 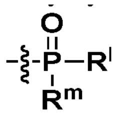,

and 5-to 6-membered heteroaryl;

more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-$S(=O)_2$-,

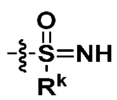, 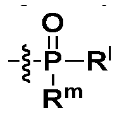,

oxazolyl, thiazolyl, and pyridyl;

$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or 7-membered spiro-heterocyclic ring;

preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, and $R^f$ is selected from the group consisting of hydrogen, methyl, ethyl, isopropyl,

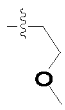, and 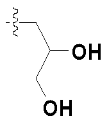;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which they connect form

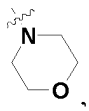,

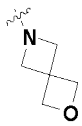;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;

preferably, $R^i$ is selected from the group consisting of hydrogen, and C1-C6 alkyl, and $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, C1-C6 alkyl, and 5-membered heteroaryl;

further preferably, $R^i$ is selected from the group consisting of hydrogen, and C1-C6 alkyl, and $R^j$ is selected from the group consisting of hydrogen, C1-C6 alkyl, and isoxazolyl;

further preferably, $R^i$ is selected from the group consisting of hydrogen, and C1-C4 alkyl, and $R^j$ is selected from the group consisting of hydrogen, C1-C4 alkyl, and

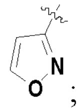

most preferably, $R^1$ is selected from the group consisting of hydrogen, and methyl, and $R^j$ is selected from the group consisting of hydrogen, methyl, and

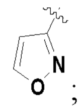

or preferably, $R^i$, $R^j$ together with the N atom to which they connect form a 6-membered saturated heterocyclic ring or a 7-membered spiro-heterocyclic ring;
or more preferably. $R^i$, $R^j$ together with the N atom to which the connect form

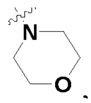

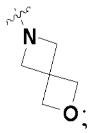

$R^k$, $R^l$, $R^m$ are each independently selected from the group consisting of hydrogen, and C1-C6 alkyl;
preferably, $R^k$, $R^l$, $R^m$ Each is independently C1-C6 alkyl;
more preferably, $R^k$, $R^l$, $R^m$ are each independently C1-C4 alkyl;
most preferably, $R^k$ $R^l$ $R^m$ are methyl;
most preferably, $R^2$ is selected from the group consisting of chlorine, cyano, $F_3C$-,

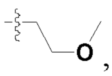,

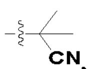, 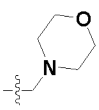, 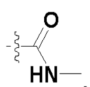, 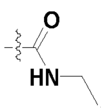, 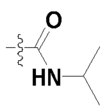, 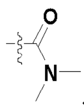, 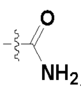, 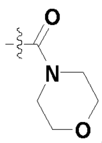, 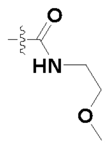, 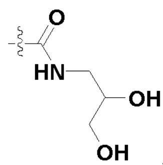,
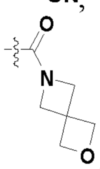, 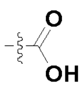, 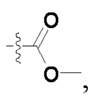, 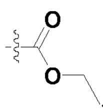, 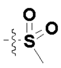, 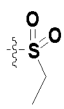, 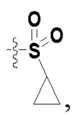, 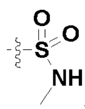, 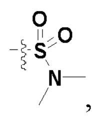, 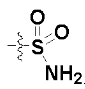, 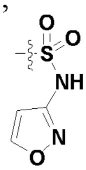, 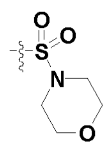,

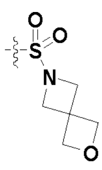, 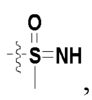, 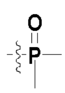, 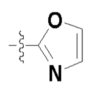, 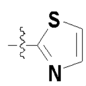, and 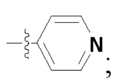;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;

more preferably, $R^3$ is selected from the group consisting of hydrogen, methyl,

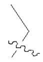,

$F_3C$-,

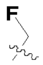, 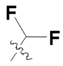, 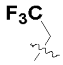, 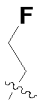, 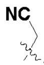, 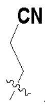, 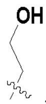, 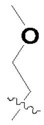, and 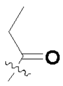;

most preferably, $R^3$ is methyl;

$R^4$ is selected from the group consisting of hydrogen, and halogen;

preferably, $R^4$ is selected from the group consisting of hydrogen, and fluorine;

more preferably, $R^4$ is hydrogen;

$R^5$ is R'-NH-C(=O)-;

R' is C1-C6 alkyl;

preferably, $R^5$ is

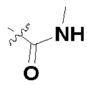;

$R^6$ is selected from the group consisting of hydrogen, and halogen;

preferably, $R^6$ is selected from the group consisting of hydrogen, and fluorine;

more preferably, $R^6$ is fluorine;

$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;

further preferably, $R^7$ is selected from the group consisting of methyl,

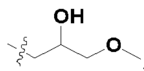,

and

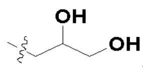;

most preferably, $R^7$ is methyl;

$R^8$ is $R^oR^pN$-;

$R^o$ and $R^p$ are each independently selected from the group consisting of C1-C6 alkyl, and C1-C6 alkoxy-C1-C6 alkylene; or, $R^o$ and $R^p$ together with the N atom to which they connect form a 5- to 6-membered saturated heterocyclic ring, a 7- to 9-membered spiro-heterocyclic ring or a 7-membered bridged heterocyclic ring;

preferably, $R^o$ is C1-C6 alkyl, and $R^p$ is selected from the group consisting of C1-C6 alkyl, and C1-C6 alkoxy-C1-C6 alkylene;

more preferably, $R^o$ is C1-C4 alkyl, and $R^p$ is selected from the group consisting of C1-C4 alkyl, and C1-C2 alkoxy-C1-C2 alkylene;

most preferably, $R^o$ is methyl, and $R^p$ is selected from the group consisting of methyl, and

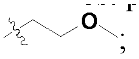;

or preferably, $R^o$ and $R^p$ together with the N atom to which they commonly connect form a 6-membered saturated heterocyclyl, a 7- to 9-membered spiro-heterocyclyl, or a 7-membered bridged heterocyclyl;
or more preferably, $R^o$ and $R^p$ together with the N atom to which they commonly connect form

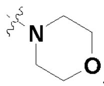, 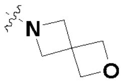, 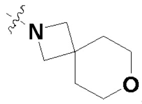, 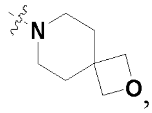, 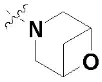;

preferably, $R^8$ is selected from the group consisting of

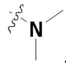, 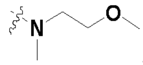, 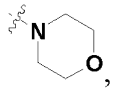, 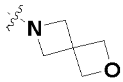,

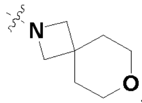, 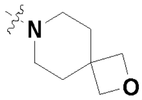, and 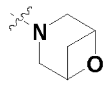;

$R^9$ is selected from the group consisting of hydrogen and hydroxyl;
preferably, $R^9$ is hydrogen;
$R^{10}$ is selected from the group consisting of hydrogen and halogen;
preferably, $R^{10}$ is selected from the group consisting of hydrogen and fluorine;
more preferably, $R^{10}$ is hydrogen;
$R^{11}$ is selected from the group consisting of hydrogen and C1-C6 alkyl;
preferably, $R^{11}$ is selected from the group consisting of hydrogen and C1-C4 alkyl;
more preferably, $R^{11}$ is selected from the group consisting of hydrogen and methyl;
most preferably, $R^{11}$ is hydrogen.

In the thirteenth aspect of the present invention, the present invention provides a compound represented by Formula I-4, or a stereoisomer, tautomer, prodrug, crystal form, hydrate, isotope-labeled compound, metabolite, ester, pharmaceutically acceptable salt or pharmaceutically acceptable solvate of the compound,

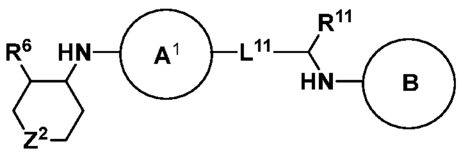

Formula I-4

wherein:

Ring $A^1$ is

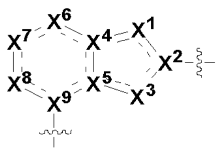,

wherein the site at the lower left end is connected to NH, and the site at the upper right end is connected to $L^{11}$;
each - - - - - - is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;
preferably, $R^a$ is selected from the group consisting of C1-C4 alkyl, C1-C2 haloalkyl, and cyano-C1-C2 alkylene;
more preferably, $R^a$ is selected from the group consisting of

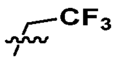 , 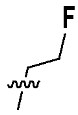 , 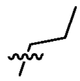 ,

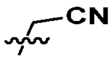 , and 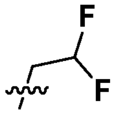 ;

most preferably, $R^a$ is

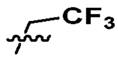 ;

$R^b$ is selected from the group consisting of hydrogen, and hydroxyl;
preferably, $R^b$ is hydrogen;
$R^c$ is selected from the group consisting of hydrogen, and halogen;
preferably, $R^c$ is selected from the group consisting of hydrogen, and fluorine;
more preferably, $R^c$ is hydrogen;
preferably, Ring $A^1$ is selected from the group consisting of

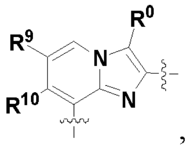 , 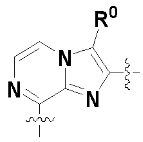 ,

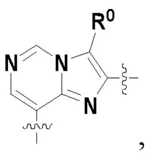 , 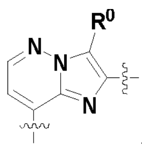 , 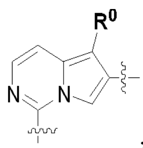 , 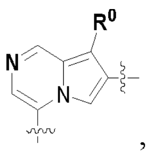 , 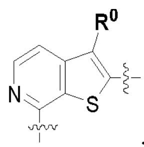 , and 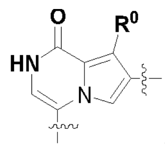 ,

wherein the site at the lower left end is connected to NH, and the site at the upper right end is connected to $L^{11}$;
more preferably, Ring $A^1$ is

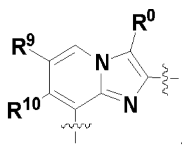 ,

wherein the site at the lower left end is connected to NH, and the site at the upper right end is connected to $L^{11}$;
most preferably, Ring $A^1$ is

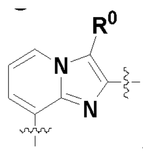 ,

wherein the site at the lower left end is connected to NH, and the site at the upper right end is connected to $L^{11}$;
Ring B is selected from the group consisting of

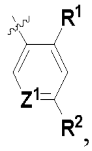, and 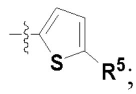;

preferably, Ring B is

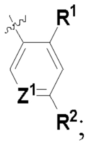;

$L^{11}$ is 5- to 6-membered heteroarylene;
preferably, $L^{11}$ is 5-membered heteroarylene;
preferably, $L^{11}$ is selected from the group consisting of 1,3,4-thiadiazolylene, 1,2,4-oxadiazolylene, 1,3,4-oxadiazolylene, and thiazolylene;
further preferably, $L^{11}$ is selected from the group consisting of

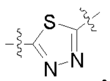, 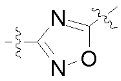,

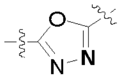, 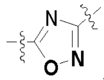, 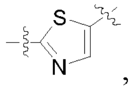, and 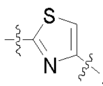,

wherein the site at the left side is connected to Ring $A^1$, and site at the right side is connected to the alkylene/alkylidene;
$Z^2$ is selected from the group consisting of

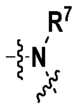, and 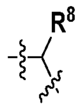;

preferably, $Z^2$ is

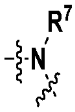;

$Z^1$ is selected from the group consisting of N, and $CR^4$;
preferably, $Z^1$ is $CR^4$;
$R^0$ is selected from the group consisting of C1-C6 alkyl, C1-C6 haloalkyl, and cyano-C1-C6 alkylene;
preferably, $R^0$ is C1-C6 haloalkyl;
preferably, $R^0$ is selected from the group consisting of C1-C4 alkyl, C1-C2 haloalkyl, and cyano-C1-C2 alkylene;
preferably, $R^0$ is C1-C2 haloalkylene;
more preferably, $R^0$ is selected from the group consisting of

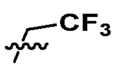, 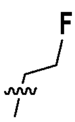, 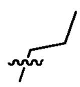,

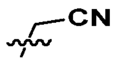, and 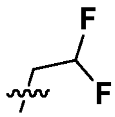;

most preferably, $R^0$ is

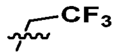;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^j$-$S(=O)_2$-,

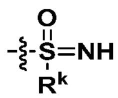, 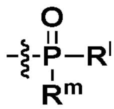,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^j$-$S(=O)_2$-,

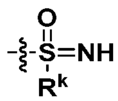, 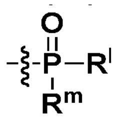,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^j$-$S(=O)_2$-,

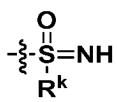, 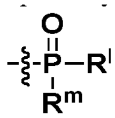,

oxazolyl, thiazolyl, and pyridyl;
further preferably, $R^2$ is $R^eR^fN$-C(=O)-;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form to form a 5- to 6-membered saturated heterocyclic ring or a 7-membered spiro-heterocyclic ring;
preferably, $R^e$ and $R^f$ are each independently selected from the group consisting of hydrogen and C1-C6 alkyl;
preferably, $R^e$ is selected from the group consisting of hydrogen and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;
more preferably, $R^e$ is selected from the group consisting of hydrogen and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;
more preferably, $R^e$ is hydrogen and $R^f$ is C1-C6 alkyl;
further preferably, $R^e$ is hydrogen, and $R^f$ is C1-C4 alkyl;

further preferably, $R^e$ is selected from the group consisting of hydrogen, and methyl, and $R^f$ is selected from the group consisting of hydrogen, methyl, ethyl, isopropyl,

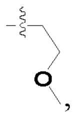, and 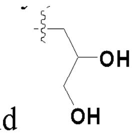;

most preferably, $R^e$ is hydrogen, and $R^f$ is methyl;
or preferably, $R^e$, $R^f$ together with the N atom to which they connect form a 6-membered saturated heterocyclic ring or a 7-membered spiro-heterocyclic ring;
or more preferably, $R^e$, $R^f$ and the N atom to which they connect form

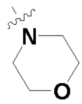, 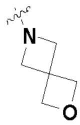;

$R^g$ is selected from the group consisting of hydrogen, and C1-C6 alkyl;
preferably, $R^g$ is selected from the group consisting of hydrogen, and C1-C4 alkyl;
more preferably, $R^g$ is selected from the group consisting of hydrogen, methyl, and ethyl;
$R^h$ is selected from the group consisting of C1-C6 alkyl, and C3-C6 cycloalkyl;
preferably, $R^h$ is selected from the group consisting of C1-C4 alkyl, and C3-C6 cycloalkyl;
more preferably, $R^h$ is selected from the group consisting of methyl, ethyl, and cyclopropyl;
$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;
preferably, $R^i$ is selected from the group consisting of hydrogen, and C1-C6 alkyl, and $R^j$ is selected from the group consisting of hydrogen, C1-C6 alkyl, and 5- to 6-membered heteroaryl;
more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

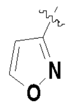;

most preferably, $R^i$ is selected from the group consisting of hydrogen, and methyl, and $R^j$ is selected from the group consisting of hydrogen, methyl, and

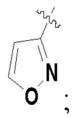;

or preferably, $R^i$, $R^j$ together with the N atom to which they connect form a 6-membered saturated heterocyclic ring or a 7-membered spiro-heterocyclic ring;
or more preferably, $R^i$, $R^j$ and the N atom to which they connect form

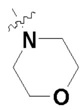, 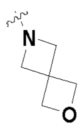;

$R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$, $R^l$, $R^m$ are methyl;
further preferably, $R^2$ is selected from the group consisting of chlorine, cyano, $F_3C$-,

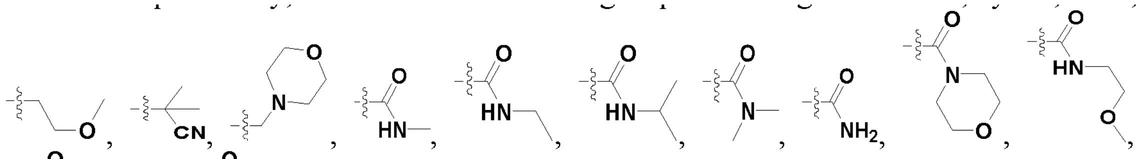

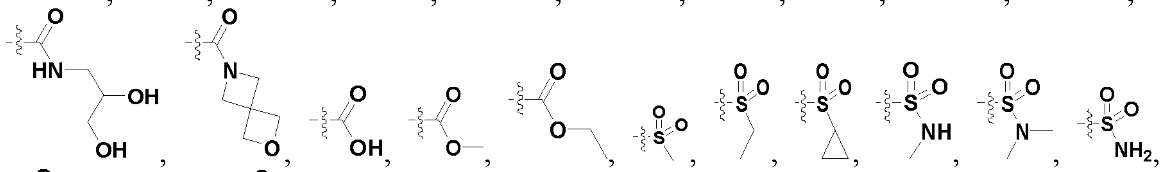

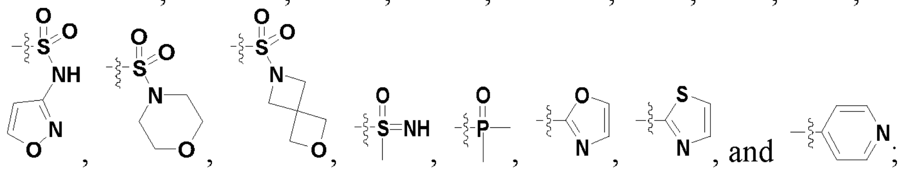

most preferably, $R^2$ is

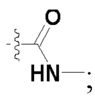

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;
preferably, $R^3$ is C1-C6 alkyl;
preferably, $R^3$ is C1-C4 alkyl;
more preferably, $R^3$ is selected from the group consisting of hydrogen, methyl,

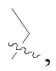

$F_3C$-,

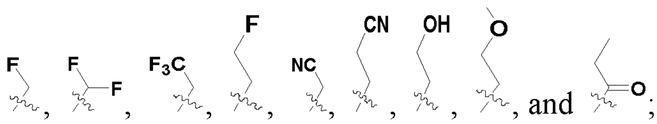

most preferably, $R^3$ is methyl;
$R^4$ is selected from the group consisting of hydrogen, and halogen;
preferably, $R^4$ is selected from the group consisting of hydrogen, and fluorine;
more preferably, $R^4$ is hydrogen;
$R^5$ is R'-NH-C(=O)-;
R' is C1-C6 alkyl;
preferably, $R^5$ is

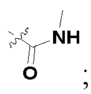

$R^6$ is selected from the group consisting of hydrogen and halogen;
preferably, $R^6$ is selected from the group consisting of hydrogen and fluorine;
more preferably, $R^6$ is fluorine;
or more preferably, $R^6$ is hydrogen;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy -C1-C6 alkylene, wherein the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
more preferably, $R^7$ is C1-C6 alkyl;
more preferably, $R^7$ is C1-C4 alkyl;
further preferably, $R^7$ is selected from the group consisting of methyl,

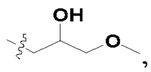

and

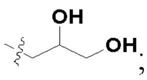

most preferably, $R^7$ is methyl;
$R^8$ is $R^oR^pN$-;
$R^o$, $R^p$ are each independently selected from the group consisting of C1-C6 alkyl, and C1-C6 alkoxy-C1-C6 alkylene; or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C6 alkyl, and C1-C6 alkoxy-C1-C6 alkylene;
more preferably, $R^o$ is C1-C4 alkyl, and $R^p$ is selected from the group consisting of C1-C4 alkyl, and C1-C2 alkoxy-C1-C2 alkylene;
most preferably, $R^o$ is methyl, and $R^p$ is methyl,
or preferably, $R^o$ and $R^p$ together with the N atom to which they connect form a 5- to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C4 alkyl, and C1-C2 alkoxy-C1-C2 alkylene;
most preferably, $R^o$ is methyl, and $R^p$ is selected from the group consisting of methyl, and

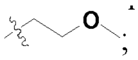

or preferably, $R^o$ and $R^p$ together with the N atom to which they connect form a 5- to 6-membered saturated heterocyclic ring, a 7- to 9-membered spiro-heterocyclic ring or a 7-membered bridged heterocyclic ring;
or more preferably, $R^o$ and $R^p$ together with the N atom to which they connect form

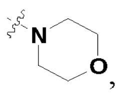

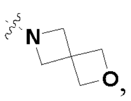 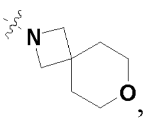 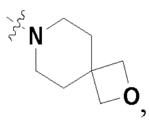 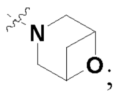

preferably, $R^8$ is selected from the group consisting of

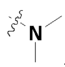, 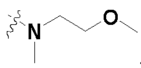, 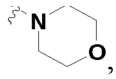,

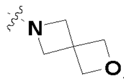, 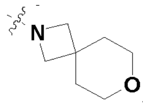, 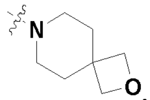, and 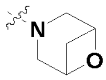;

$R^9$ is selected from the group consisting of hydrogen and hydroxyl;
preferably, $R^9$ is hydrogen;
$R^{10}$ is selected from the group consisting of hydrogen and halogen;
preferably, $R^{10}$ is selected from the group consisting of hydrogen and fluorine;
more preferably, $R^{10}$ is hydrogen;
$R^{11}$ is selected from the group consisting of hydrogen and C1-C6 alkyl;
preferably, $R^{11}$ is selected from the group consisting of hydrogen and C1-C4 alkyl;
more preferably, $R^{11}$ is selected from the group consisting of hydrogen and methyl;
most preferably, $R^{11}$ is hydrogen.

In the fourteenth aspect of the present invention, the present invention provides a compound represented by Formula I-5, or a stereoisomer, tautomer, prodrug, crystal form, hydrate, isotope-labeled compound, metabolite, ester, pharmaceutically acceptable salt or pharmaceutically acceptable solvate of the compound,

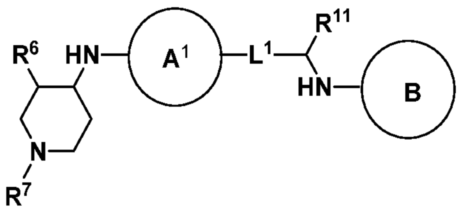

Formula I-5

wherein:

Ring $A^1$ is

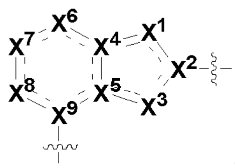

,

wherein the site at the lower left end is connected to NH, and the site at the upper right end is connected to $L^1$;
each - - - - - - is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;
preferably, $R^a$ is selected from the group consisting of C1-C4 alkyl, C1-C2 haloalkyl, and cyano-C1-C2 alkylene;
more preferably, $R^a$ is selected from the group consisting of

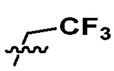, 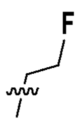, 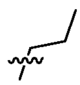,

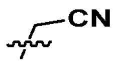, and 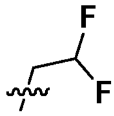;

most preferably, $R^a$ is

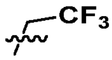;

$R^b$ is selected from the group consisting of hydrogen, and hydroxyl;
preferably, $R^b$ is hydrogen;
$R^c$ is selected from the group consisting of hydrogen, and halogen;
preferably, $R^c$ is selected from the group consisting of hydrogen, and fluorine;
more preferably, $R^c$ is hydrogen;
preferably, Ring $A^1$ is selected from the group consisting of

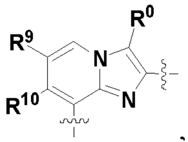, 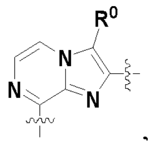,

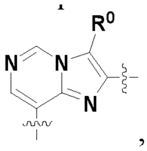, 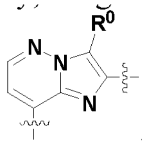, 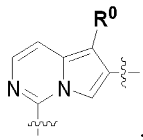, 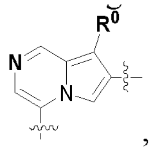, 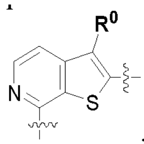, and 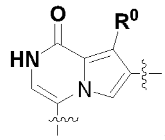,

wherein the site at the lower left end is connected to NH, and the site at the upper right end is connected to $L^1$;
more preferably, Ring $A^1$ is

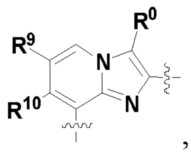,

wherein the site at the lower left end is connected to NH, and the site at the upper right end is connected to $L^1$;
Ring B is selected from the group consisting of

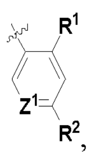, and 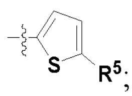;

preferably, Ring B is

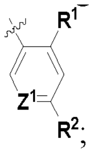;

$L^1$ is selected from the group consisting of

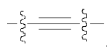,

and 5- to 6-membered heteroarylene;
preferably, $L^1$ is selected from the group consisting of

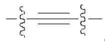,

and 5-membered heteroarylene;
more preferably, $L^1$ is selected from the group consisting of

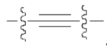,

1,3,4-thiadiazolylene, 1,2,4-oxadiazolylene, 1,3,4-oxadiazolylene, and thiazolylene;
further preferably, $L^1$ is selected from the group consisting of

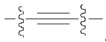, 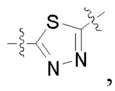, 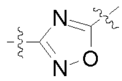,

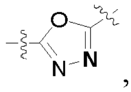, 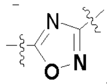, 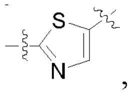, and 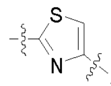,

wherein the site at the left side is connected to Ring $A^1$, and the site at the right side is connected to the alkylene/alkylidene;
most preferably, $L^1$ is

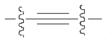;

$Z^1$ is selected from the group consisting of N, and $CR^4$;
$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

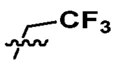, 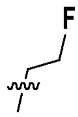, 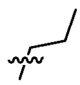,

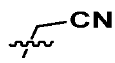, and 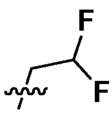;

most preferably, $R^0$ is

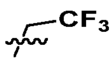;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^j$-$S(=O)_2$-,

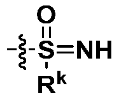, 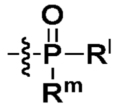,

and 5-to 6-membered heteroaryl;

preferably, $R^2$ is selected from the group consisting of cyano, halogen, C1-C2 haloalkyl, C1-C2 alkoxy-C1-C2 alkylene, cyano-C 1-C4 alkylene, 6-membered saturated heterocyclyl-C1-C2 alkylene, $R^eR^fN$-C(=O)-, $R^gO$-C(=O)-, $R^hS(=O)_2$-, $R^iR^j$-$S(=O)_2$-,

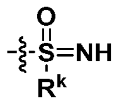, 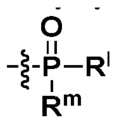,

and 5-to 6-membered heteroaryl;

more preferably, $R^2$ is selected from the group consisting of cyano, halogen, C1-C2 haloalkyl, C1-C2 alkoxy-C1-C2 alkylene, cyano-C1-C4 alkylene, morpholinyl- C1-C2 alkylene, $R^eR^fN$-C(=O)-, $R^gO$-C(=O)-, $R^hS(=O)_2$-, $R^iR^j$-$S(=O)_2$-,

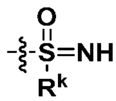, 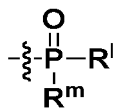,

oxazolyl, thiazolyl, and pyridyl;

$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;

preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, and $R^f$ is selected from the group consisting of hydrogen, methyl, ethyl, isopropyl,

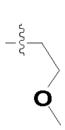, and 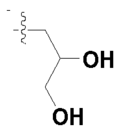;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which they connect form

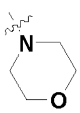,

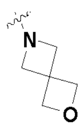;

$R^g$ is selected from the group consisting of hydrogen, and C1-C6 alkyl;
preferably, $R^g$ is selected from the group consisting of hydrogen, and C1-C4 alkyl;
more preferably, $R^g$ is selected from the group consisting of hydrogen, methyl, and ethyl;
$R^h$ is selected from the group consisting of C1-C6 alkyl, and C3-C6 cycloalkyl;
preferably, $R^h$ is selected from the group consisting of C1-C4 alkyl, and C3-C6 cycloalkyl;
more preferably, $R^h$ is selected from the group consisting of methyl, ethyl, and cyclopropyl;
$R^i$, $R^j$ are each independently selected from the group consisting of hydrogen, C1-C6 alkyl, and 5- to 6-membered heteroaryl; or, $R^i$, $R^j$ together with the N atoms to which they connect form a 5- to 6-membered saturated heterocyclic ring or a 7-membered spiro-heterocyclic ring;
preferably, $R^i$ is selected from the group consisting of hydrogen, and C1-C6 alkyl, and $R^j$ is selected from the group consisting of hydrogen, C1-C6 alkyl, and 5- to 6-membered heteroaryl;
more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

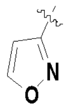;

most preferably, $R^i$ is selected from the group consisting of hydrogen, and methyl, and $R^j$ is selected from the group consisting of hydrogen, methyl, and

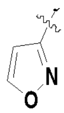;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

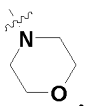,

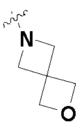;

$R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$, $R^l$, $R^m$ are methyl;
most preferably, $R^2$ is selected from the group consisting of chlorine, cyano, $F_3C$-,

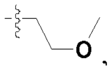,

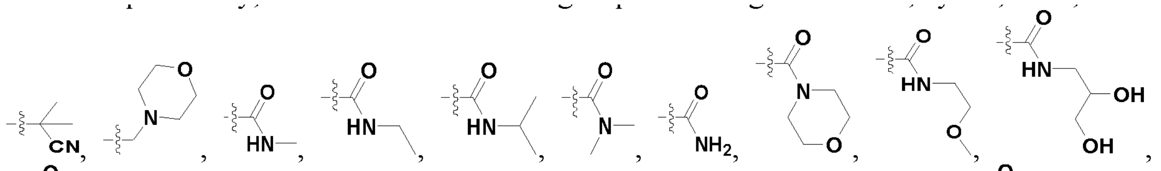

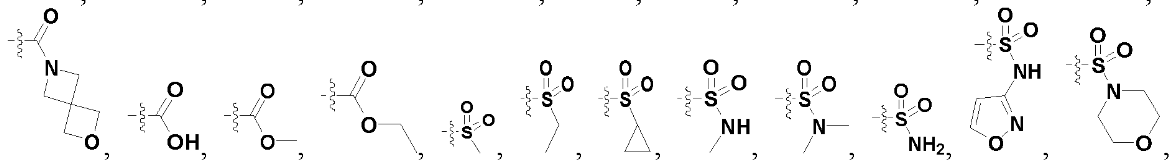

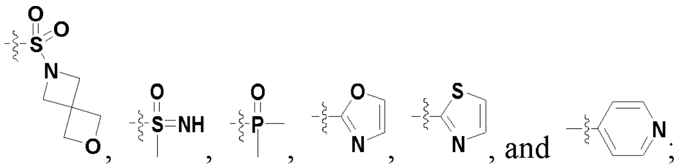 , and ;

$R^3$ is selected from the group consisting of hydrogen, C1-C6 alkyl, C1-C6 haloalkyl, cyano-C1-C6 alkylene, hydroxyl-C1-C6 alkylene, C1-C6 alkoxy-C1-C6 alkylene, and C1-C6 alkyl-C(=O)-;
preferably, $R^3$ is selected from the group consisting of hydrogen, C1-C4 alkyl, C1-C4 haloalkyl, cyano-C1-C2 alkylene, hydroxyl-C1-C2 alkylene, C1-C2 alkoxy-C1-C2 alkylene, and C1-C2 alkyl-C(=0)-;
more preferably, $R^3$ is selected from the group consisting of hydrogen, methyl,

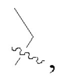,

$F_3C$-,

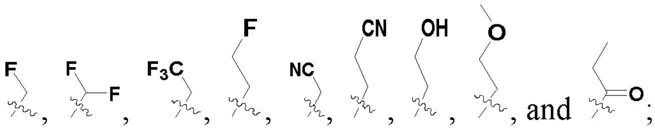, and ;

most preferably, $R^3$ is methyl;
$R^4$ is selected from the group consisting of hydrogen, and halogen;
preferably, $R^4$ is selected from the group consisting of hydrogen, and fluorine;
more preferably, $R^4$ is hydrogen;
$R^5$ is R'-NH-C(=O)-;
R' is C1-C6 alkyl;
preferably, $R^5$ is

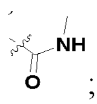 ;

$R^6$ is selected from the group consisting of hydrogen, and halogen;
preferably, $R^6$ is selected from the group consisting of hydrogen, and fluorine;
more preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
further preferably, $R^7$ is selected from the group consisting of methyl,

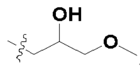,

and

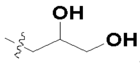;

most preferably, $R^7$ is methyl;

$R^9$ is selected from the group consisting of hydrogen, and hydroxyl;

preferably, $R^9$ is hydrogen;

$R^{10}$ is selected from the group consisting of hydrogen, and halogen;

preferably, $R^{10}$ is selected from the group consisting of hydrogen, and fluorine;

more preferably, $R^{10}$ is hydrogen;

$R^{11}$ is selected from the group consisting of hydrogen, and C1-C6 alkyl;

preferably, $R^{11}$ is selected from the group consisting of hydrogen, and C1-C4 alkyl;

more preferably, $R^{11}$ is selected from the group consisting of hydrogen, and methyl;

most preferably, $R^{11}$ is hydrogen.

In the fifteenth aspect of the present invention, the present invention provides a compound represented by Formula I-6, or a stereoisomer, tautomer, prodrug, crystal form, hydrate, isotope-labeled compound, metabolite, ester, pharmaceutically acceptable salt or pharmaceutically acceptable solvate of the compound,

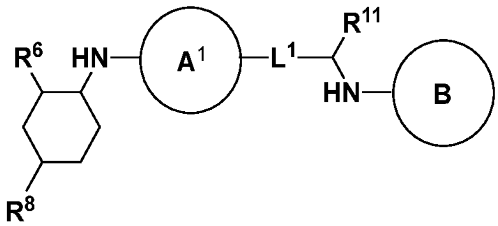

Formula I-6

wherein:

Ring $A^1$ is

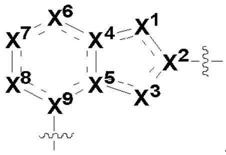,

wherein the site at the lower left end is connected to NH, and the site at the upper right end is connected to $L^1$;

each ------ is independently a single bond or a double bond;

$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,

b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;

preferably, $R^a$ is selected from the group consisting of C1-C4 alkyl, C1-C2 haloalkyl, and cyano-C1-C2 alkylene;

more preferably, $R^a$ is selected from the group consisting of

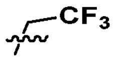, 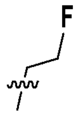, 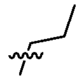,

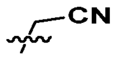, and 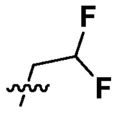;

most preferably, $R^a$ is

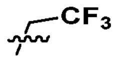;

$R^b$ is selected from the group consisting of hydrogen and hydroxyl;
preferably, $R^b$ is hydrogen;
$R^c$ is selected from the group consisting of hydrogen and halogen;
preferably, $R^c$ is selected from the group consisting of hydrogen and fluorine;
more preferably, $R^c$ is hydrogen;
preferably, Ring $A^1$ is selected from the group consisting of

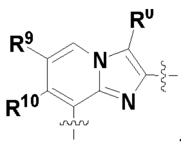, 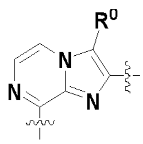,

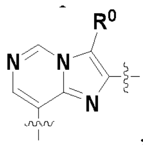, 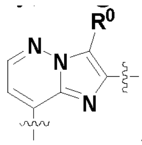, 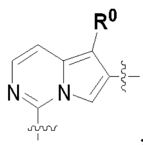, 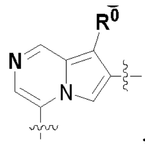, 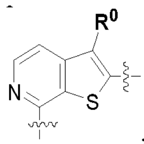, and 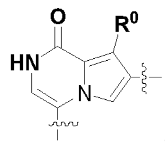,

wherein the site at the lower left end is connected to NH, and the site at the upper right end is connected to $L^1$;
more preferably, Ring $A^1$ is

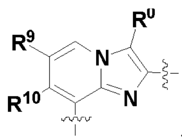,

wherein the site at the lower left end is connected to NH, and the site of the upper right end is connected to $L^1$;
most preferably, Ring $A^1$ is

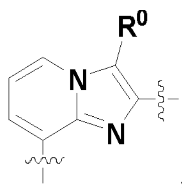,

wherein, the site at the lower left end is connected to NH, and the site at the upper right end is connected to $L^1$;
Ring B is selected from the group consisting of

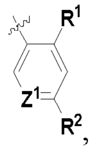, and 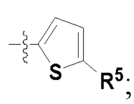;

preferably, Ring B is

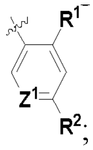;

$L^1$ is selected from the group consisting of

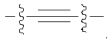,

and 5- to 6-membered heteroarylene;
preferably, $L^1$ is selected from the group consisting of

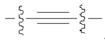,

and 5-membered heteroarylene;
more preferably, $L^1$ is selected from the group consisting of

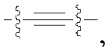,

1,3,4-thiadiazolylene, 1,2,4-oxadiazolylene, 1,3,4-oxadiazolylene, and thiazolylene;
further preferably, $L^1$ is selected from the group consisting of

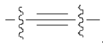, 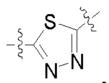, 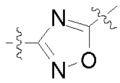,

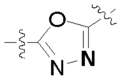, 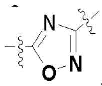, 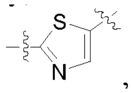, and 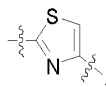,

wherein the site at the left side is connected to Ring $A^1$, the site at the right side is connected to the alkylene/alkylidene;
most preferably, $L^1$ is

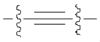;

$Z^1$ is selected from the group consisting of N, and $CR^4$;
preferably, $Z^1$ is $CR^4$;
$R^0$ is selected from the group consisting of C1-C6 alkyl, C1-C6 haloalkyl, and cyano-C1-C6 alkylene;
preferably, $R^0$ is C1-C6 haloalkyl;
preferably, $R^0$ is selected from the group consisting of C1-C4 alkyl, C1-C2 haloalkyl, and cyano-C1-C2 alkylene;
preferably, $R^0$ is C1-C2 haloalkyl alkyl;
more preferably, $R^0$ is selected from the group consisting of

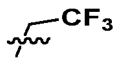 , 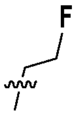 , 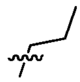 ,

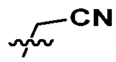 , and 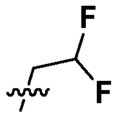 ;

most preferably, $R^0$ is

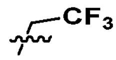 ;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^j$-S$(=O)_2$-,

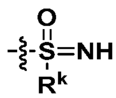 , 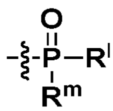 ,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$- C(=O)-, $R^gO$-C(=O)-, $R^hS(=O)_2$-, $R^iR^jN$-S$(=O)_2$-,

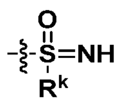 , 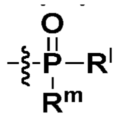 ,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-S$(=O)_2$-,

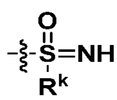 , 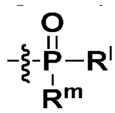 ,

oxazolyl, thiazolyl, and pyridyl;
more preferably, $R^2$ is $R^eR^fN$-C(=O)-;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;
preferably, $R^e$, $R^f$ are each independently selected from the group consisting of hydrogen, and C1-C6 alkyl;
preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

preferably, $R^e$ is hydrogen, and $R^f$ is C1-C6 alkyl;

more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

more preferably, $R^e$ is hydrogen, and $R^f$ is C1-C4 alkyl;

most preferably, $R^e$ is selected from the group consisting of hydrogen, and methyl, and $R^f$ is selected from the group consisting of hydrogen, methyl, ethyl, isopropyl,

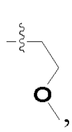, and 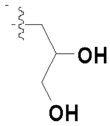 ;

or most preferably, $R^e$ is hydrogen, and $R^f$ is methyl;

or preferably, $R^e$, $R^f$ together with the N atom to which they connect form a 6-membered saturated heterocyclic ring or a 7-membered spiro-heterocyclic ring;

or more preferably, $R^e$, $R^f$ together with the N atom to which they connect form

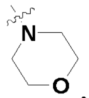,

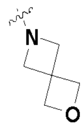;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaromatic alkyl; or, $R^i$, $R^j$ together with the N atom to which they connect form a 5- to 6-membered saturated heterocyclic ring or a 7-membered spiro-heterocyclic ring;

preferably, R’ is selected from the group consisting of hydrogen, and C1-C6 alkyl, and $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, C1-C6 alkyl, and 5-membered heteroaryl;

further preferably, $R^i$ is selected from the group consisting of hydrogen, and C1-C6 alkyl, and $R^j$ is selected from the group consisting of hydrogen, C1-C6 alkyl, and isoxazolyl;

further preferably, $R^i$ is selected from the group consisting of hydrogen, and C1-C4 alkyl, and $R^j$ is selected from the group consisting of hydrogen, C1-C4 alkyl, and

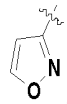;

most preferably, $R^i$ is selected from the group consisting of hydrogen, and methyl, and $R^j$ is selected from the group consisting of hydrogen, methyl, and

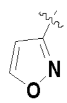;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

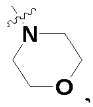,

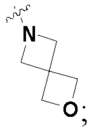;

$R^k$, $R^l$, $R^m$ are each independent selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$ $R^l$, $R^m$ are methyl;
most preferably, $R^2$ is selected from the group consisting of chlorine, cyano, $F_3C$-,

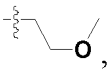,

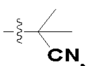, 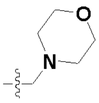, 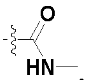, 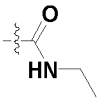, 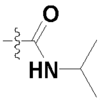, 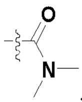, 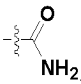, 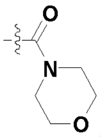, 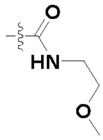, 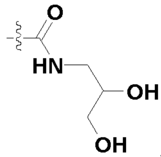, 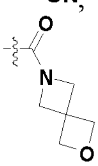, 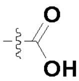, 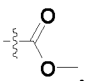, 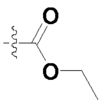, 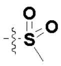, 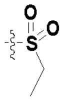, 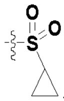, 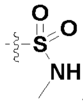, 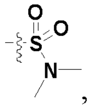, 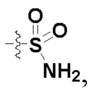, 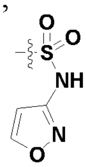, 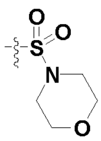,

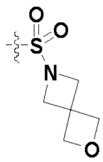, 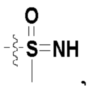, 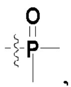, 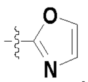, 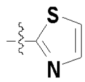, and 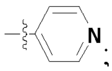;

or most preferably, $R^2$ is

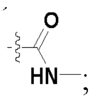;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is C1-C6 alkyl;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;
preferably, $R^3$ is C1-C4 alkyl;
more preferably, $R^3$ is selected from the group consisting of hydrogen, methyl,

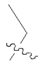,

$F_3C$-,

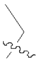, F3C-, 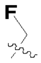, 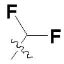, 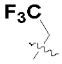, 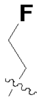, 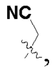, 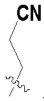, 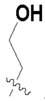, 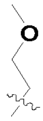, and 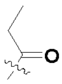;

most preferably, $R^3$ is methyl;

$R^4$ is selected from the group consisting of hydrogen, and halogen;

preferably, $R^4$ is selected from the group consisting of hydrogen, and fluorine;

more preferably, $R^4$ is hydrogen;

$R^5$ is R'-NH-C(=O)-;

R' is C1-C6 alkyl;

preferably, $R^5$ is

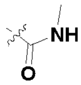;

$R^6$ is selected from the group consisting of hydrogen and halogen;

preferably, $R^6$ is selected from the group consisting of hydrogen and fluorine;

more preferably, $R^6$ is fluorine;

$R^8$ is $R^oR^pN$-;

$R^o$ and $R^p$ are each independently selected from the group consisting of C1-C6 alkyl, and C1-C6 alkoxy-C1-C6 alkylene; or, $R^o$ and $R^p$ together with the N atom to which they connect form a 5- to 6-membered saturated heterocyclic ring, a 7- to 9-membered spiro-heterocyclic ring or a 7-membered bridged heterocyclic ring;

preferably, $R^o$ is C1-C6 alkyl, and $R^p$ is selected from the group consisting of C1-C6 alkyl, and C1-C6 alkoxy-C1-C6 alkylene;

more preferably, $R^o$ is C1-C4 alkyl, and $R^p$ is selected from the group consisting of C1-C4 alkyl, and C1-C2 alkoxy-C1-C2 alkylene;

most preferably, $R^o$ is methyl, and $R^p$ is selected from the group consisting of methyl, and

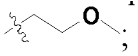;

or preferably, $R^o$ and $R^p$ together with the N atom to which they connect form a 6-membered saturated heterocyclyl, a 7- to 9-membered spiro-heterocyclyl, or a 7-membered bridged heterocyclyl;

or more preferably, $R^o$ and $R^p$ together with the N atom to which they connect form

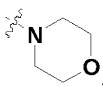,

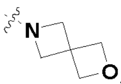, 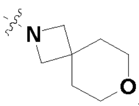, 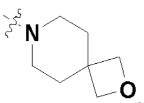, 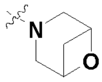;

preferably, $R^8$ is selected from the group consisting of

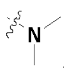, 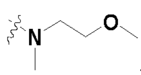, 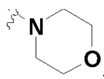, 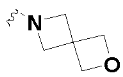,

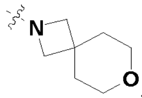, 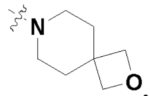, and 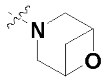;

$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the sixteenth aspect of the present invention, the present invention provides a compound represented by Formula I-7, or a stereoisomer, tautomer, prodrug, crystal form, hydrate, isotope-labeled compound, metabolite, ester, pharmaceutically acceptable salt or pharmaceutically acceptable solvate of the compound,

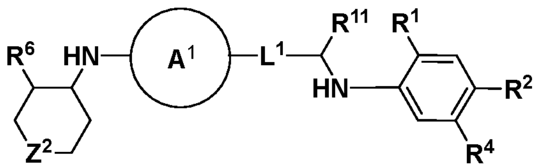

Formula I-7

wherein:

Ring $A^1$ is

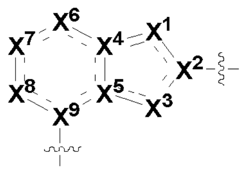

,

wherein the site at the lower left end is connected to NH, and the site at the upper right end is connected to $L^1$;
each ------ is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;
preferably, $R^a$ is selected from the group consisting of C1-C4 alkyl, C1-C2 haloalkyl, and cyano-C1-C2 alkylene;
more preferably, $R^a$ is selected from the group consisting of

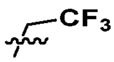, 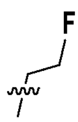, 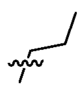,

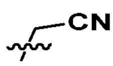, and 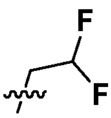;

most preferably, $R^a$ is

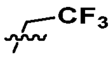;

$R^b$ is selected from the group consisting of hydrogen and hydroxyl;
preferably, $R^b$ is hydrogen;
$R^c$ is selected from the group consisting of hydrogen and halogen;
preferably, $R^c$ is selected from the group consisting of hydrogen and fluorine;
more preferably, $R^c$ is hydrogen;
preferably, Ring $A^1$ is selected from the group consisting of

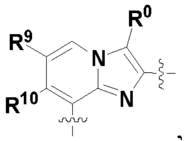, 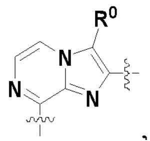,

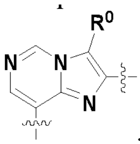, 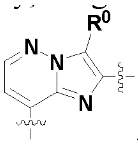, 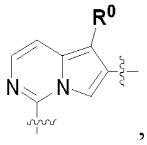, 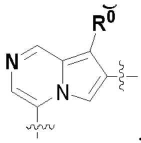, 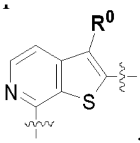, and 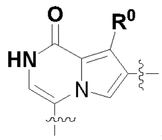,

wherein the site at the lower left end is connected to NH, and the site at the upper right end is connected to $L^1$;
more preferably, Ring $A^1$ is

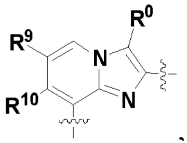,

wherein the site at the lower left end is connected to NH, and the site at the upper right end is connected to $L^1$;
$L^1$ is selected from the group consisting of

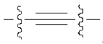,

and 5- to 6-membered heteroarylene;
preferably, $L^1$ is selected from the group consisting of

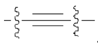,

and 5-membered heteroarylene;
more preferably, $L^1$ is selected from the group consisting of

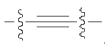,

1,3,4-thiadiazolylene, 1,2,4-oxadiazolylene, 1,3,4-oxadiazolylene, and thiazolylene;
further preferably, $L^1$ is selected from the group consisting of

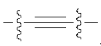, 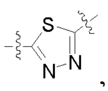, 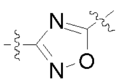,

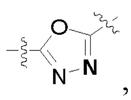, 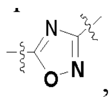, 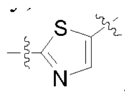, and 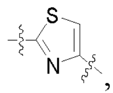,

wherein the site at the left side is connected to Ring $A^1$, and the site at the right side is connected to the alkylene/alkylidene;
most preferably, $L^1$ is

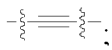;

$Z^2$ is selected from the group consisting of

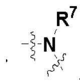, and 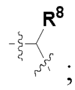;

$R^0$ is selected from the group consisting of C1-C6 alkyl, C1-C6 haloalkyl, and cyano-C1-C6 alkylene;
preferably, $R^0$ is selected from the group consisting of C1-C4 alkyl, C1-C2 haloalkyl, and cyano-C1-C2 alkylene;
more preferably, $R^0$ is selected from the group consisting of

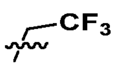, 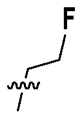, 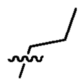,

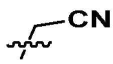, and 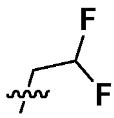;

most preferably, $R^0$ is

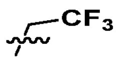;

$R^1$ selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^j$ N-$S(=O)_2$-,

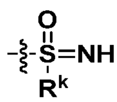, 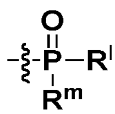,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

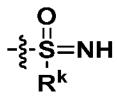 , 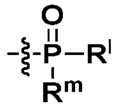 ,

and 5-to 6-membered heteroaryl;

more preferably, $R^2$ is selected from the group consisting of cyano, halogen, C1-C2 haloalkyl, C1-C2 alkoxy C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-$S(=O)_2$-,

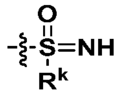 , 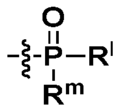 ,

oxazolyl, thiazolyl, and pyridyl;

$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form 5- to 6-membered saturated heterocyclic ring or 7-membered spiro-heterocyclic ring;

preferably, $R^e$ is selected from the group consisting of hydrogen, and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;

more preferably, $R^e$ is selected from the group consisting of hydrogen, and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;

most preferably, $R^e$ is selected from the group consisting of hydrogen, and methyl, and $R^f$ is selected from the group consisting of hydrogen, methyl, ethyl, isopropyl,

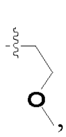 , and 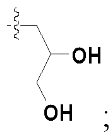 ;

or preferably, $R^e$, $R^f$ together with the N atom to which they commonly connect form a 6-membered saturated heterocyclic ring or a 7-membered spiro-heterocyclic ring;

or more preferably, $R^e$, $R^f$ together with the N atom to which they commonly connect form

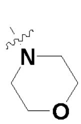 , 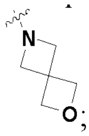 ;

$R^g$ is selected from the group consisting of hydrogen, and C1-C6 alkyl;

preferably, $R^g$ is selected from the group consisting of hydrogen, and C1-C4 alkyl;

more preferably, $R^g$ is selected from the group consisting of hydrogen, methyl, and ethyl;

$R^h$ is selected from the group consisting of C1-C6 alkyl, and C3-C6 cycloalkyl;

preferably, $R^h$ is selected from the group consisting of C1-C4 alkyl, and C3-C6 cycloalkyl;

more preferably, $R^h$ is selected from the group consisting of methyl, ethyl, and cyclopropyl;

$R^i$, $R^j$ are each independently selected from the group consisting of hydrogen, C1-C6 alkyl, and 5- to 6-membered heteroaryl; or, $R^i$, $R^j$ together with the N atom to which they connect form a 5- to 6-membered saturated heterocyclic ring or a 7-membered spiro-heterocyclic ring;

preferably, R’is selected from the group consisting of hydrogen, and C1-C6 alkyl, and $R^j$ is selected from the group consisting of hydrogen, C1-C6 alkyl, and 5- to 6-membered heteroaryl;

more preferably, $R^i$ is selected from the group consisting of hydrogen, and C1-C6 alkyl, and $R^j$ is selected from the group consisting of hydrogen, and C1-C6 alkyl, 5-membered heteroaryl;

further preferably, $R^i$ is selected from the group consisting of hydrogen, and C1-C6 alkyl, and $R^j$ is selected from the group consisting of hydrogen, C1-C6 alkyl, and isoxazolyl;

more further preferably, $R^i$ is selected from the group consisting of hydrogen, and C1-C4 alkyl, and $R^j$ is selected from the group consisting of hydrogen, C1-C4 alkyl, and

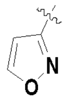 ;

most preferably, $R^i$ is selected from the group consisting of hydrogen, and methyl, and $R^j$ is selected from the group consisting of hydrogen, methyl, and

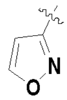 ;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

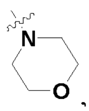 ,

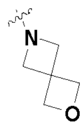 ;

$R^k$, $R^l$, $R^m$ are each independently selected from the group consisting of hydrogen, and C1-C6 alkyl;
preferably, $R^k$, $R^l$, and $R^m$ are each independently C1-C6 alkyl;
more preferably, $R^k$, $R^l$, and $R^m$ are each independently C1-C4 alkyl;
most preferably, $R^k$ $R^l$, and $R^m$ are methyl;
most preferably, $R^2$ is selected from the group consisting of chlorine, cyano, $F_3C$-,

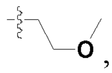 ,

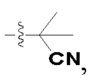 , 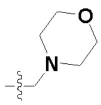 , 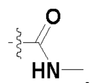 , 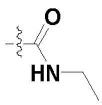 , 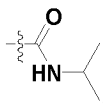 , 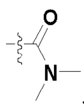 , 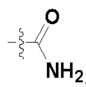 , 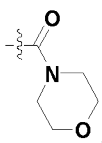 , 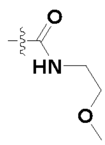 , 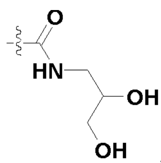 ,
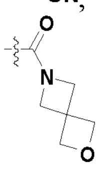 , 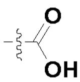 , 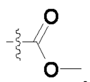 , 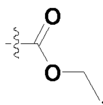 , 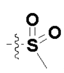 , 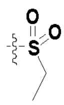 , 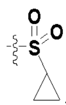 , 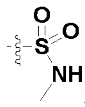 , 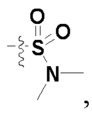 , 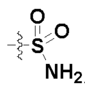 , 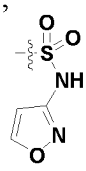 , 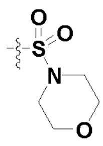 ,

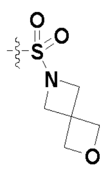 , 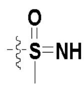 , 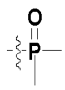 , 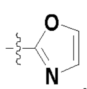 , 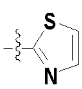 , and 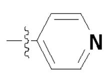 ;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;

preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=O)-;

more preferably, $R^3$ is selected from the group consisting of hydrogen, methyl,

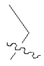,

$F_3C$-,

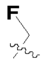, 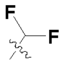, 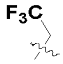, 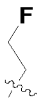, 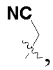, 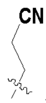, 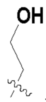, 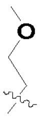, and 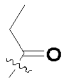;

most preferably, $R^3$ is methyl;

$R^4$ is selected from the group consisting of hydrogen, and halogen;

preferably, $R^4$ is selected from the group consisting of hydrogen, and fluorine;

more preferably, $R^4$ is hydrogen;

$R^6$ is selected from the group consisting of hydrogen and halogen;

preferably, $R^6$ is selected from the group consisting of hydrogen and fluorine;

more preferably, $R^6$ is fluorine;

$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;

more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxv-C1-C4 alkylene is substituted with hydroxyl;

further preferably, $R^7$ is selected from the group consisting of methyl,

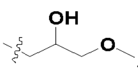,

and

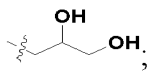;

most preferably, $R^7$ is methyl;

$R^8$ is $R^oR^pN$-;

$R^o$, $R^p$ are each independently selected from the group consisting of C1-C6 alkyl, and C1-C6 alkoxy-C1-C6 alkylene;

or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;

preferably, $R^o$ is C1-C6 alkyl, and $R^p$ is selected from the group consisting of C1-C6 alkyl, and C1-C6 alkoxy-C1-C6 alkylene;

more preferably, $R^o$ is C1-C4 alkyl, and $R^p$ is selected from the group consisting of C1-C4 alkyl, and C1-C2 alkoxy-C1-C2 alkylene;

most preferably, $R^o$ is methyl, and $R^p$ is selected from the group consisting of methyl, and

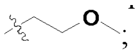;

or preferably, $R^o$ and $R^p$ together with the N atom to which they connect form a 6-membered saturated heterocyclic ring, a 7- to 9-membered spiro-heterocyclic ring, a 7-membered bridged heterocyclyl;

or more preferably, $R^o$ and $R^p$ together with the N atom to which they commonly connect form

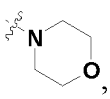, 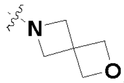, 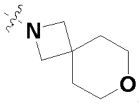, 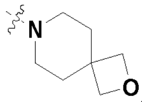, 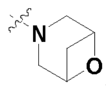,

preferably, $R^8$ is selected from the group consisting of

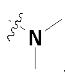, 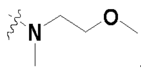, 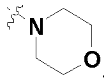, 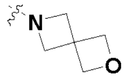,

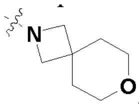, 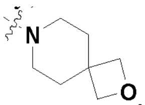, and 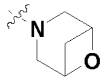;

$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the seventeenth aspect of the present invention, the present invention provides a compound represented by Formula 1-8, or a stereoisomer, tautomer, prodrug, crystal form, hydrate, isotope-labeled compound, metabolite, ester, pharmaceutically acceptable salt or pharmaceutically acceptable solvate of the compound,

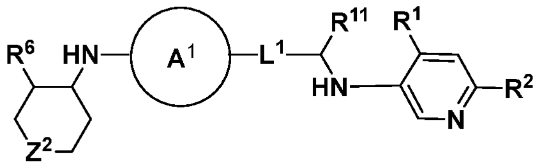

Formula I-8

wherein:

Ring $A^1$ is

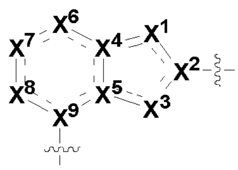,

wherein the site at the lower left end is connected to NH, and the site at the upper right end is connected to $L^1$;
each ------ is independently a single bond or a double bond;
$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C=O, N, NH, and S, and meet the following conditions a) or b):

a) any one of $X^4$ and $X^5$ is N,
b) when neither of $X^4$ and $X^5$ is N, any one of $X^1$ and $X^3$ is S;

$R^a$ is selected from the group consisting of C1-C6 alkyl, C1-C6 haloalkyl, and cyano-C1-C6 alkylene;
preferably, $R^a$ is selected from the group consisting of C1-C4 alkyl, C1-C2 haloalkyl, and cyano-C1-C2 alkylene;
more preferably, $R^a$ is selected from the group consisting of

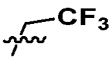, 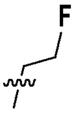, 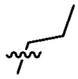,

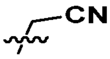, and 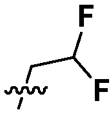;

most preferably, $R^a$ is

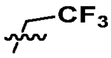;

$R^b$ is selected from the group consisting of hydrogen and hydroxyl;
preferably, $R^b$ is hydrogen;
$R^c$ is selected from the group consisting of hydrogen and halogen;
preferably, $R^c$ is selected from the group consisting of hydrogen and fluorine;
more preferably, $R^c$ is hydrogen;
preferably, Ring $A^1$ is selected from the group consisting of

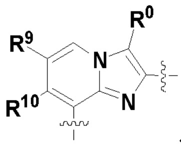, 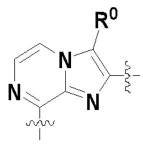,

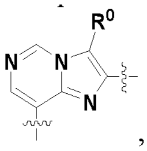, 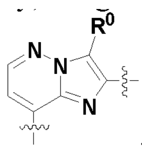, 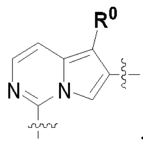, 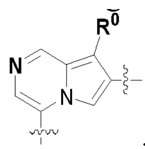, 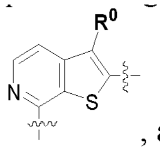, and 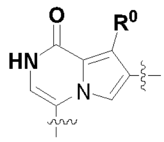,

wherein the site at the lower left end is connected to NH, and the site at the upper right end is connected to $L^1$;
more preferably, Ring $A^1$ is

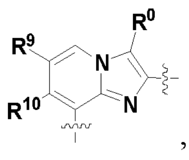,

wherein the site at the lower left end is connected to NH, and the site at the upper right end is connected to $L^1$;
most preferably, Ring $A^1$ is

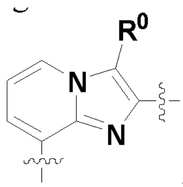,

wherein the site at the lower left end is connected to NH, and the site at the upper right end is connected to $L^1$;
$L^1$ is selected from the group consisting of

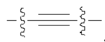,

and 5- to 6-membered heteroarylene;
preferably, $L^1$ is selected from the group consisting of

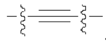,

and 5-membered heteroarylene;
more preferably, $L^1$ is selected from the group consisting of

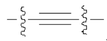,

1,3,4-thiadiazolylene, 1,2,4-oxadiazolylene, 1,3,4-oxadiazolylene, and thiazolylene;
further preferably, $L^1$ is selected from the group consisting of

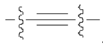, 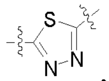, 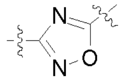,

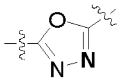, 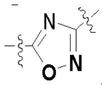, 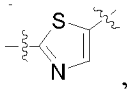, and 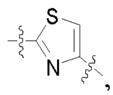,

wherein the site at the left side is connected to Ring $A^1$, and the site at the right side is connected to the alkylene/alkylidene;
most preferably, $L^1$ is

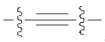;

$Z^2$ is selected from the group consisting of

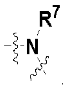, and 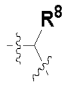;

preferably, $Z^2$ is

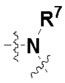;

$R^0$ is selected from the group consisting of C1-C6 alkyl, C1-C6 haloalkyl, and cyano-C1-C6 alkylene;
preferably, $R^0$ is C1-C6 haloalkyl;
preferably, $R^0$ is selected from the group consisting of C1-C4 alkyl, C1-C2 haloalkyl, and cyano-C1-C2 alkylene;
preferably, $R^0$ is C1-C2 haloalkyl;
more preferably, $R^0$ is selected from the group consisting of

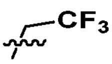, 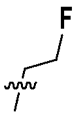, 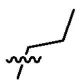,

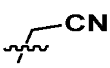, and 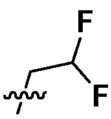;

most preferably, $R^0$ is

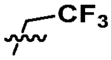 ;

$R^1$ is selected from the group consisting of C1-C6 haloalkyl, and $R^3O$-;
preferably, $R^1$ is selected from the group consisting of C1-C2 haloalkyl, and $R^3O$-;
more preferably, $R^1$ is selected from the group consisting of $F_3C$-, and $R^3O$-;
most preferably, $R^1$ is $R^3O$-;
$R^2$ is selected from the group consisting of cyano, halogen, C1-C6 haloalkyl, C1-C6 alkoxy-C1-C6 alkylene, cyano-C1-C6 alkylene, 5- to 6-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

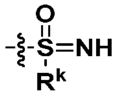 , 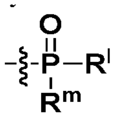 ,

and 5-to 6-membered heteroaryl;
preferably, $R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

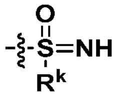 , 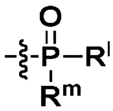 ,

and 5-to 6-membered heteroaryl;
more preferably, $R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^jN$-$S(=O)_2$-,

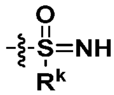 , 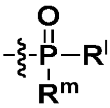 ,

oxazolyl, thiazolyl, and pyridyl;
more preferably, $R^2$ is selected from the group consisting of cyano, and $R^eR^fN$-C(=O)-;
$R^e$, $R^f$ are each independently selected from the group consisting of hydrogen, C1-C6 alkyl, C1-C6 alkoxy-C1-C6 alkylene, and C1-C6 alkyl substituted with 2 hydroxyl groups; or, $R^e$, $R^f$ together with the N atom to which they connect form a 5- to 6-membered saturated heterocyclic ring or a 7-membered spiro-heterocyclic ring;
preferably, $R^e$ and $R^f$ are each independently selected from the group consisting of hydrogen and C1-C6 alkyl;
preferably, $R^e$ is selected from the group consisting of hydrogen and C1-C6 alkyl, and $R^f$ is selected from the group consisting of hydrogen, C1-C6 alkyl, C1-C6 alkoxy-C1-C6 alkylene, and C1-C6 alkyl substituted with 2 hydroxyl groups;
preferably, $R^e$ is hydrogen, and $R^f$ is C1-C6 alkyl;
more preferably, $R^e$ is selected from the group consisting of hydrogen and C1-C4 alkyl, and $R^f$ is selected from the group consisting of hydrogen, C1-C4 alkyl, C1-C2 alkoxy-C1-C2 alkylene, and C1-C4 alkyl substituted with 2 hydroxyl groups;
more preferably, $R^e$ is hydrogen, and $R^f$ is C1-C4 alkyl;
most preferably, $R^e$ is selected from the group consisting of hydrogen, and methyl, and $R^f$ is selected from the group consisting of hydrogen, methyl, ethyl, isopropyl,

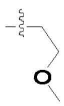, and 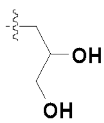 ;

or most preferably, $R^e$ is hydrogen, and $R^f$ is selected from the group consisting of ethyl, and isopropyl;
or preferably, $R^e$, $R^f$ together with the N atom to which they connect form a 6-membered saturated heterocyclic ring or a 7-membered spiro-heterocyclic ring;
or more preferably, $R^e$, $R^f$ together with the N atom to which they connect form

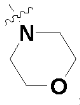 ,

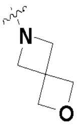 ;

$R^g$ is selected from the group consisting of hydrogen, and C1-C6 alkyl;
preferably, $R^g$ is selected from the group consisting of hydrogen, and C1-C4 alkyl;
more preferably, $R^g$ is selected from the group consisting of hydrogen, methyl, and ethyl;
$R^h$ is selected from the group consisting of C1-C6 alkyl, and C3-C6 cycloalkyl;
preferably, $R^h$ is selected from the group consisting of C1-C4 alkyl, and C3-C6 cycloalkyl;
more preferably, $R^h$ is selected from the group consisting of methyl, ethyl, and cyclopropyl;
$R^i$, $R^j$ are each independently selected from the group consisting of hydrogen, C1-C6-membered alkyl, and 5- to 6-membered heteroaryl; or, $R^i$, $R^j$ together with the N atom to which they connect form a 5- to 6-membered saturated heterocyclic ring or a 7-membered spiro-heterocyclic ring;
preferably, $R^i$ is selected from the group consisting of hydrogen, and C1-C6 alkyl, and $R^j$ is selected from the group consisting of hydrogen, C1-C6 alkyl, and 5- to 6-membered heteroaryl;
more preferably, $R^i$ is selected from the group consisting of hydrogen, and C1-C6 alkyl, and $R^j$ is selected from the group consisting of hydrogen, C1-C6 alkyl, and 5-membered heteroaryl;
further preferably, $R^i$ is selected from the group consisting of hydrogen, and C1-C6 alkyl, and $R^j$ is selected from the group consisting of hydrogen, C1-C6 alkyl, and isoxazolyl;
further preferably, $R^i$ is selected from the group consisting of hydrogen, and C1-C4 alkyl, and $R^j$ is selected from the group consisting of hydrogen, C1-C4 alkyl, and

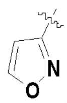 ;

most preferably, $R^i$ is selected from the group consisting of hydrogen, and methyl, $R^j$ is selected from the group consisting of hydrogen, methyl, and

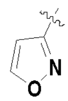 ;

or preferably, $R^i$, $R^j$ together with the N atom to which they connect form a 6-membered saturated heterocyclic ring or a 7-membered spiro-heterocyclic ring;
or more preferably, $R^i$, $R^j$ together with the N atom to which they connect form

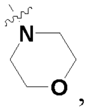 ,

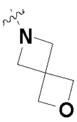 ;

$R^k$, $R^l$, $R^m$ are each independently selected from the group consisting of hydrogen, and C1-C6 alkyl;
preferably, $R^k$, $R^l$, $R^m$ are each independently C1-C6 alkyl;
more preferably, $R^k$, $R^l$, $R^m$ are each independently C1-C4 alkyl;
most preferably, $R^k$, $R^l$, $R^m$ are methyl;
most preferably, $R^2$ is selected from the group consisting of chlorine, cyano, $F_3C$-,

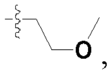,

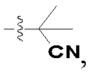, 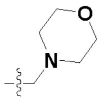, 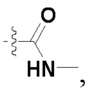, 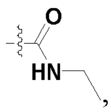, 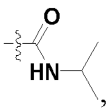, 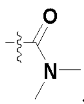, 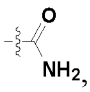, 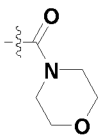, 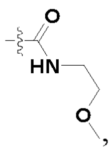, 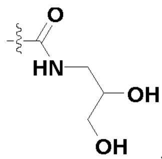,

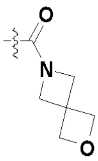, 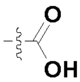, 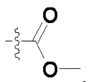, 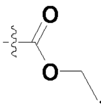, 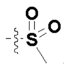, 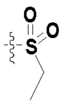, 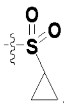, 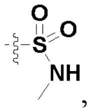, 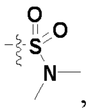, 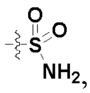, 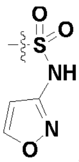, 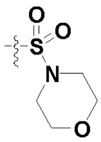,

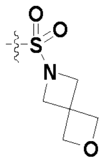, 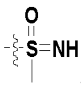, 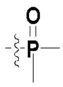, 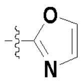, 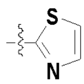, and 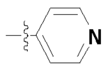;

or most preferably, $R^2$ is selected from the group consisting of

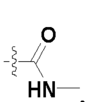, and 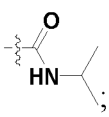;

$R^3$ is selected from the group consisting of hydrogen, C1-C6 alkyl, C1-C6 haloalkyl, cyano-C1-C6 alkylene, hydroxy-C1-C6 alkylene, C1-C6 alkoxy-C1-C6 alkylene, and C1-C6 alkyl-C(=O)-;
preferably, $R^3$ is C1-C6 alkyl;
preferably, $R^3$ is selected from the group consisting of hydrogen, C1-C4 alkyl, C1-C4 haloalkyl, cyano-C1-C2 alkylene, hydroxy-C1-C2 alkylene, C1-C2 alkoxy-C1-C2 alkylene, and C1-C2 alkyl-C(=0)-;
preferably, $R^3$ is C1-C4 alkyl;
more preferably, $R^3$ is selected from the group consisting of hydrogen, methyl,

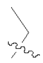, $F_3C$-,

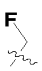, 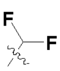, 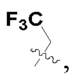, 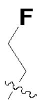, 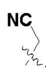, 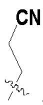, 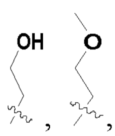, and 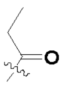;

most preferably, $R^3$ is methyl;
$R^5$ is R'-NH-C(=O)-;
R' is C1-C6 alkyl;
preferably, $R^5$ is

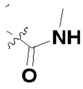;

$R^6$ is selected from the group consisting of hydrogen, and halogen;
preferably, $R^6$ is halogen;
preferably, $R^6$ is selected from the group consisting of hydrogen and fluorine;
more preferably, $R^6$ is fluorine;
$R^7$ is selected from the group consisting of hydrogen, C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is selected from the group consisting of C1-C6 alkyl, C1-C6 alkyl substituted with 2 hydroxyl groups, and C1-C6 alkoxy-C1-C6 alkylene, wherein the C1-C6 alkoxy-C1-C6 alkylene is substituted with hydroxyl;
preferably, $R^7$ is C1-C6 alkyl;
more preferably, $R^7$ is selected from the group consisting of C1-C4 alkyl, C1-C4 alkyl substituted with 2 hydroxyl groups, and C1-C2 alkoxy-C1-C4 alkylene, wherein the C1-C2 alkoxy-C1-C4 alkylene is substituted with hydroxyl;
more preferably, $R^7$ is C1-C4 alkyl;
further preferably, $R^7$ is selected from the group consisting of methyl,

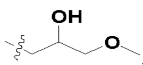,

and

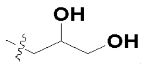;

most preferably, $R^7$ is methyl;
$R^8$ is $R^oR^pN$-;
$R^o$, $R^p$ are each independently selected from the group consisting of C1-C6 alkyl, and C1-C6 alkoxy-C1-C6 alkylene;
or, $R^o$ and $R^p$ together with the N atom to which they connect form a 5-to 6-membered saturated heterocyclic ring, a 7- to 9-membered spiro-heterocyclic ring or a 7-membered bridged heterocyclic ring;
preferably, $R^o$ is C1-C6 alkyl, and $R^p$ is selected from the group consisting of C1-C6 alkyl, and C1-C6 alkoxy-C1-C6 alkylene;
more preferably, $R^o$ is C1-C4 alkyl, and $R^p$ is selected from the group consisting of C1-C4 alkyl, and C1-C2 alkyl Oxy-C1-C2 alkylene;
most preferably, $R^o$ is methyl, and $R^p$ is selected from the group consisting of methyl, and

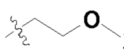;

or preferably, $R^o$ and $R^p$ together wtih the N atom to which they commonly connect form a 6-membered saturated heterocyclyl, a 7- to 9-membered spiro-heterocyclyl, a 7-membered bridged heterocyclyl;
or more preferably, $R^o$ and $R^p$ together with the N atom to which they commonly connect form

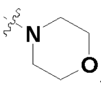, 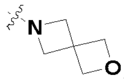, 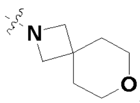, 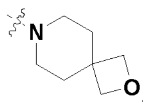, 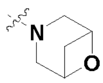;

preferably, $R^8$ is selected from the group consisting of

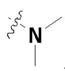, 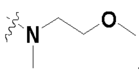, 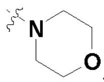, 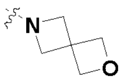,

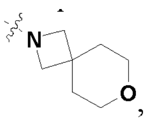, 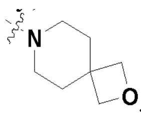, and 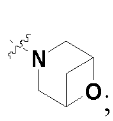;

$R^9$ is selected from the group consisting of hydrogen, and hydroxyl;
preferably, $R^9$ is hydrogen;
$R^{10}$ is selected from the group consisting of hydrogen, and halogen;
preferably, $R^{10}$ is selected from the group consisting of hydrogen, and fluorine;
more preferably, $R^{10}$ is hydrogen;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In the eighteenth aspect of the present invention, the present invention provides a compound of Formula II, or a stereoisomer, tautomer, prodrug, crystal form, hydrate, isotope-labeled compound, metabolite, ester, pharmaceutically acceptable salt or pharmaceutically acceptable solvate of the compound,

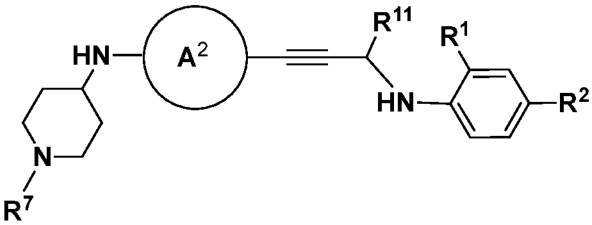

Formula II

wherein:

Ring $A^2$ is selected from the group consisting of

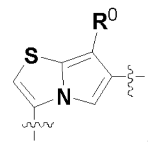, 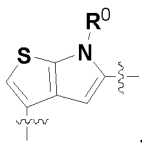, 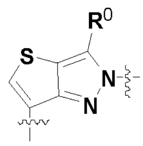,

and

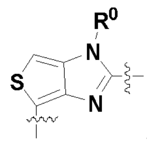,

wherein the site at the lower left end is connected to NH, and the site at the upper right end is connected to the alkynyl;
$R^0$ is C1-C6 haloalkyl;
preferably, $R^0$ is C1-C2 haloalkyl;
more preferably, $R^0$ is

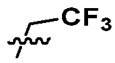;

$R^1$ is $R^3O$-;
$R^2$ is $R^eR^fN$-C(=O)-;
$R^e$, $R^f$ are each independently selected from the group consisting of hydrogen, and C1-C6 alkyl;
preferably, $R^e$ is hydrogen, and $R^f$ is C1-C6 alkyl;
more preferably, $R^e$ is hydrogen, and $R^f$ is C1-C4 alkyl;

most preferably, $R^e$ is hydrogen, and $R^f$ is methyl;
preferably, $R^2$ is

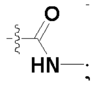

$R^3$ is C1-C6 alkyl;
preferably, $R^3$ is C1-C4 alkyl;
more preferably, $R^3$ is methyl;
$R^7$ is C1-C6 alkyl;
preferably, $R^7$ is C1-C4 alkyl;
more preferably, $R^7$ is methyl;
$R^{11}$ is selected from the group consisting of hydrogen, and C1-C6 alkyl;
preferably, $R^{11}$ is selected from the group consisting of hydrogen, and C1-C4 alkyl;
more preferably, $R^{11}$ is selected from the group consisting of hydrogen, and methyl;
most preferably, $R^{11}$ is hydrogen.

In some embodiments, the compound is selected from:

| Structural formula | Structural formula |
|---|---|
| 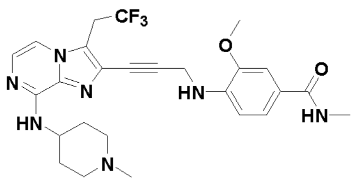 | 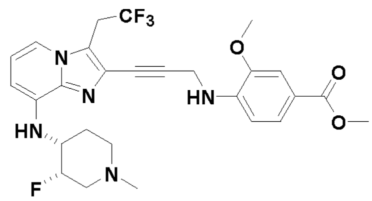 |
| 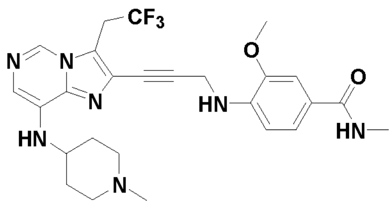 | 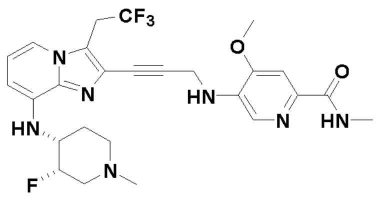 |
| 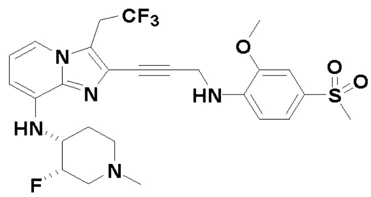 | 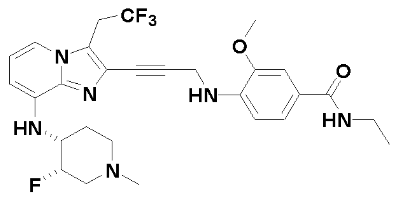 |
| 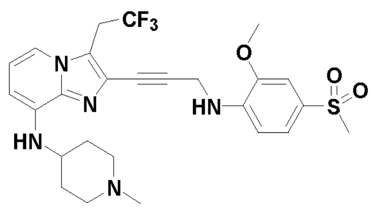 | 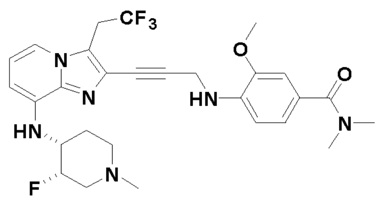 |
| 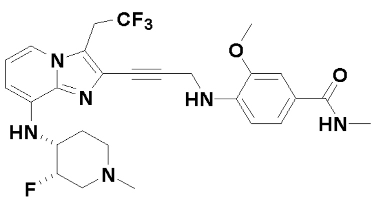 | 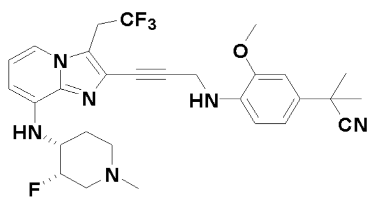 |

(continued)

| Structural formula | Structural formula |
|---|---|
| 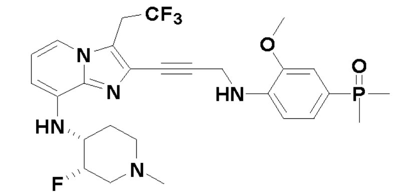 | 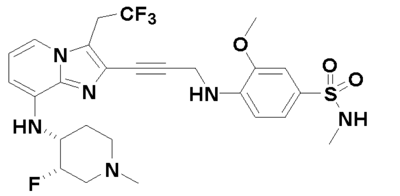 |
| 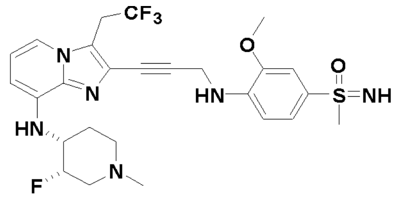 | 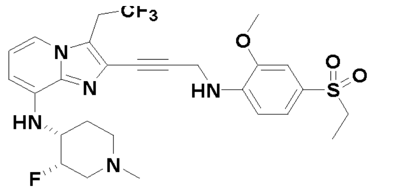 |
| 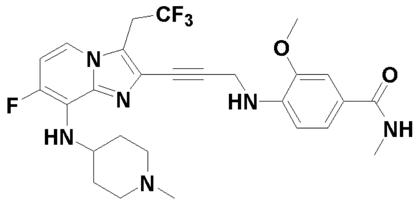 | 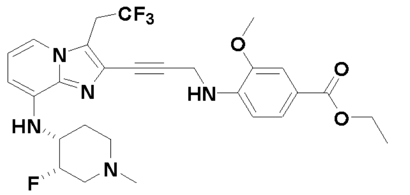 |
| 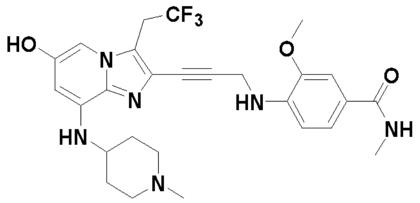 | 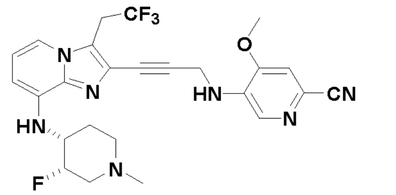 |
| 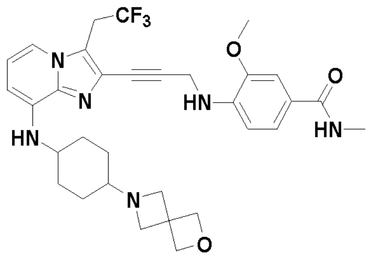 | 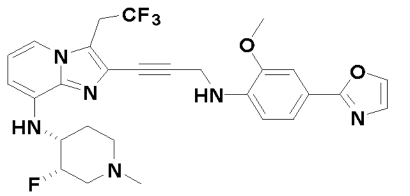 |
| 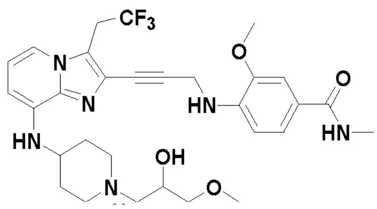 | 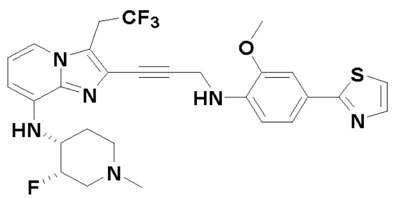 |
| 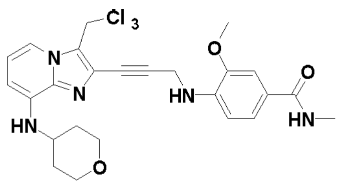 | 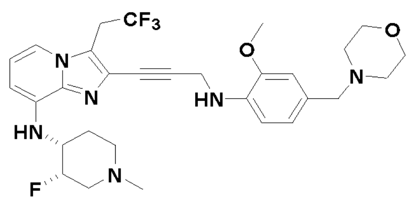 |

(continued)

| Structural formula | Structural formula |
|---|---|
| 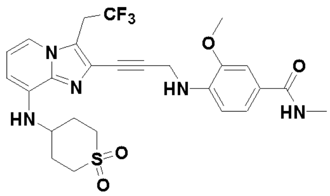 | 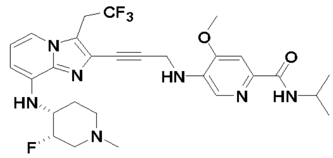 |
| 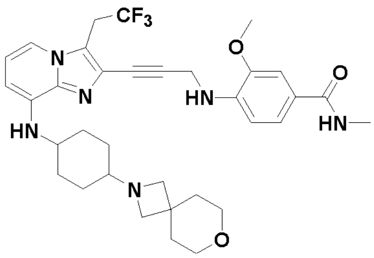 | 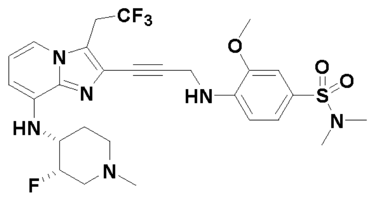 |
| 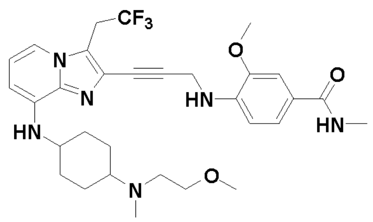 | 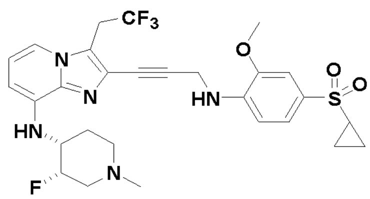 |
| 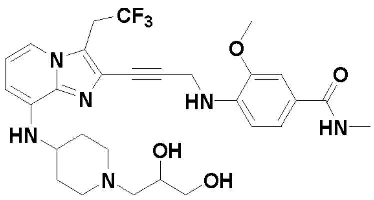 | 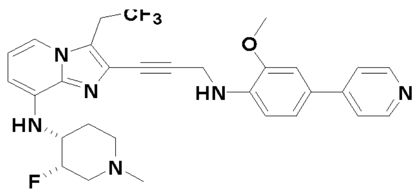 |
| 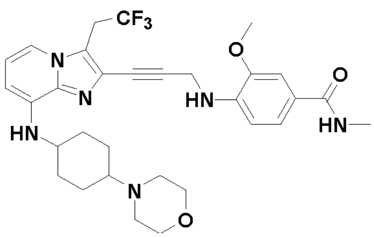 | 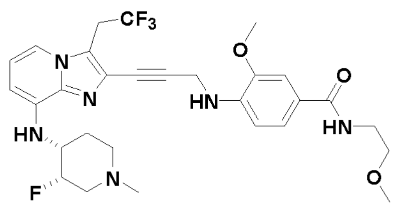 |
| 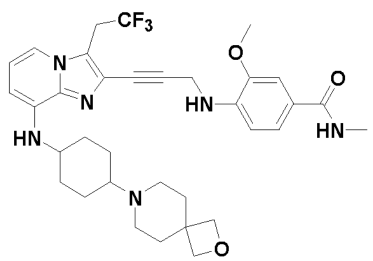 | 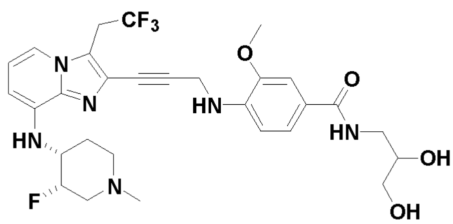 |
| 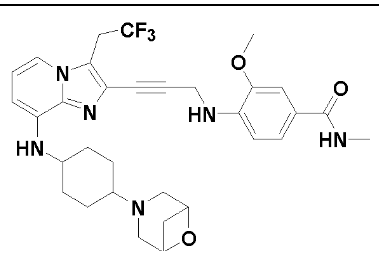 | 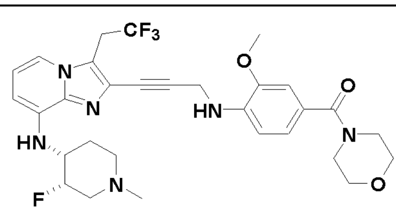 |

(continued)

| Structural formula | Structural formula |
|---|---|
| 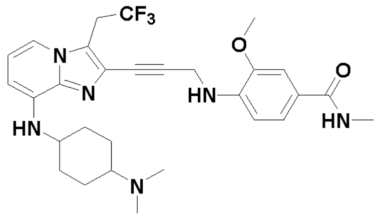 | 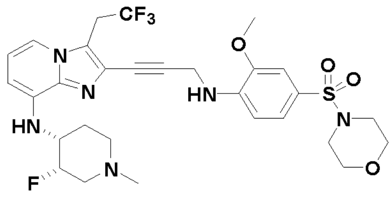 |
| 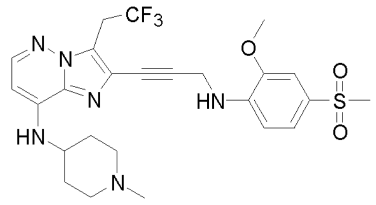 | 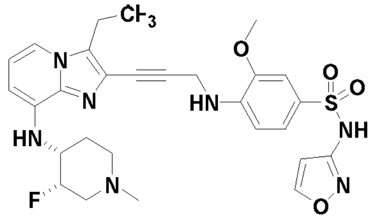 |
| 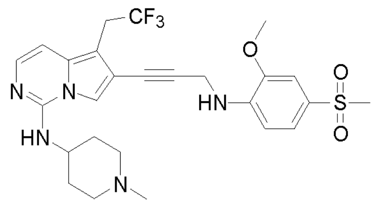 | 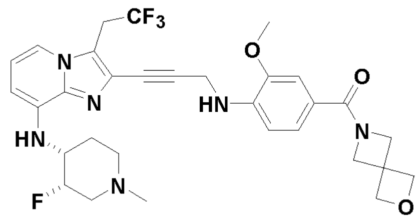 |
| 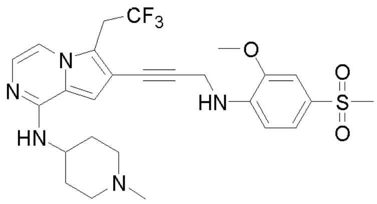 | 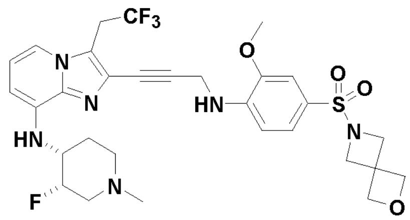 |
| 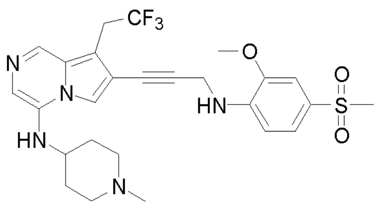 | 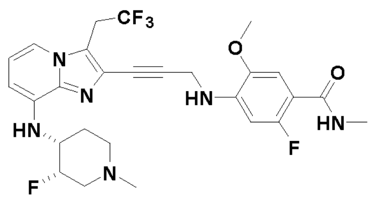 |
| 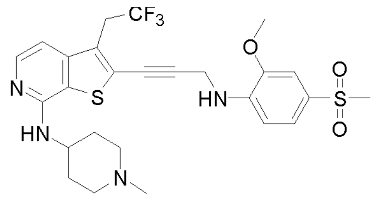 | 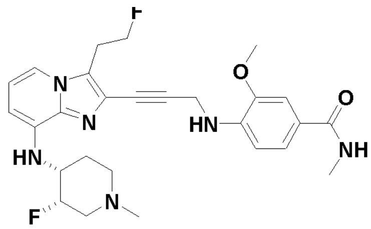 |
| 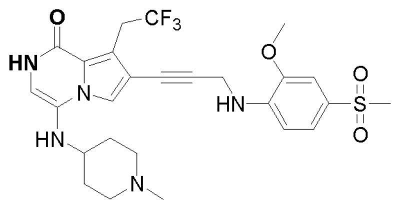 | 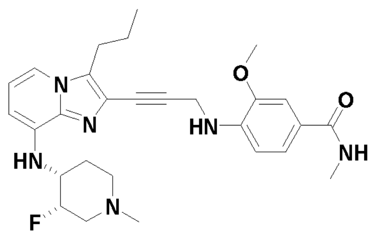 |

(continued)

| Structural formula | Structural formula |
|---|---|
| 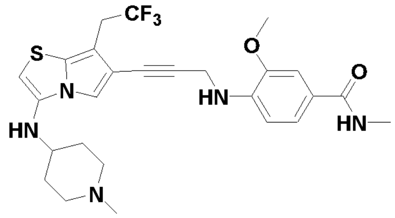 | 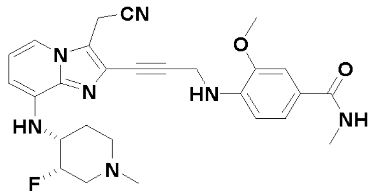 |
| 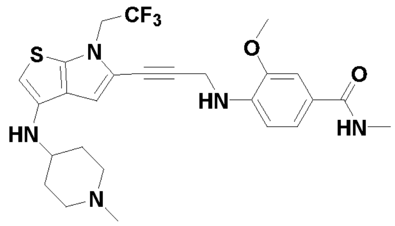 | 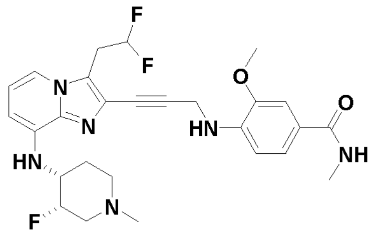 |
| 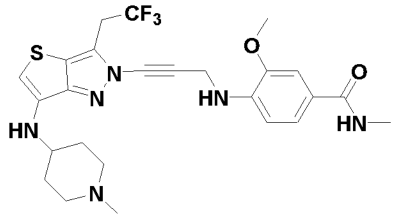 | 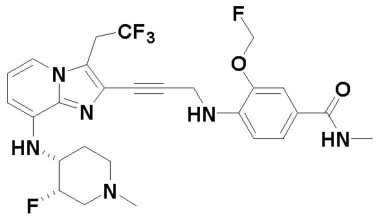 |
| 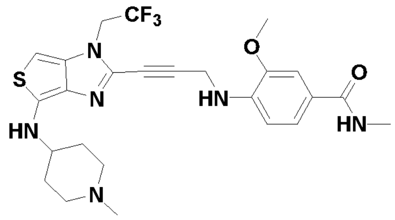 | 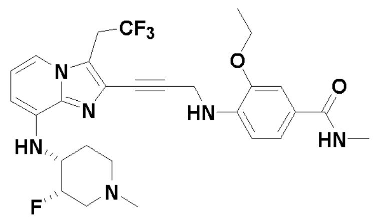 |
| 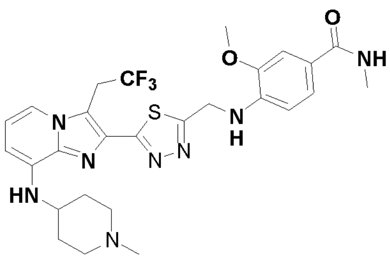 | 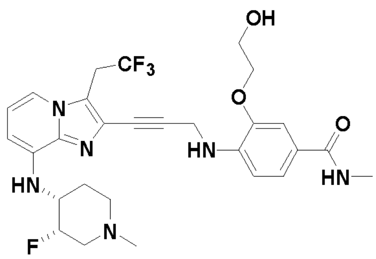 |
| 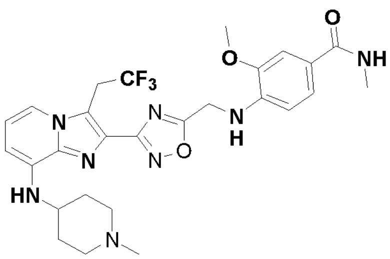 | 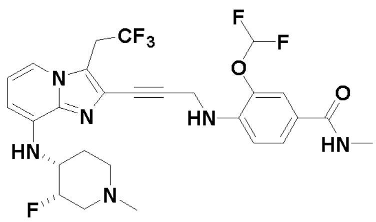 |

(continued)

| Structural formula | Structural formula |
|---|---|
| 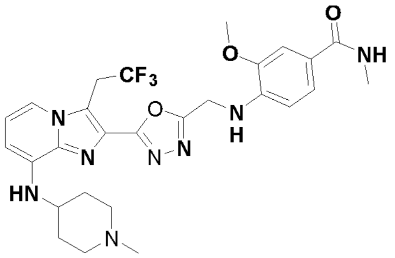 | 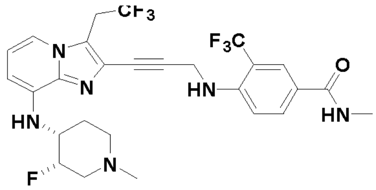 |
| 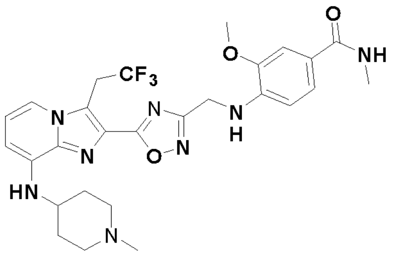 | 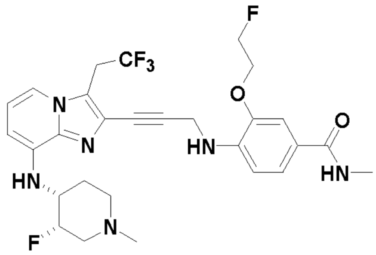 |
| 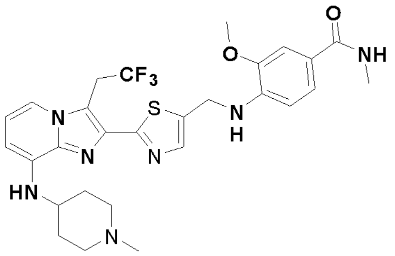 | 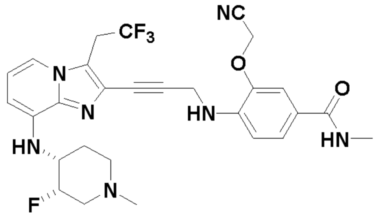 |
| 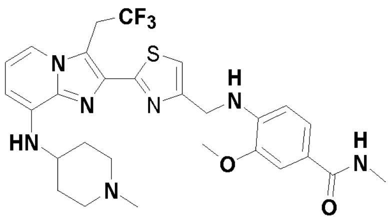 | 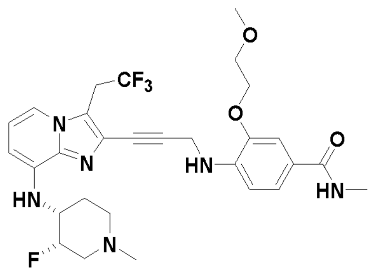 |
| 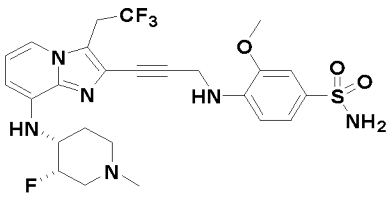 | 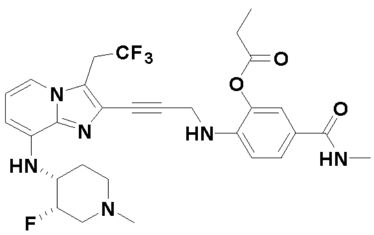 |
| 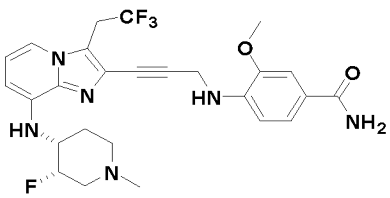 | 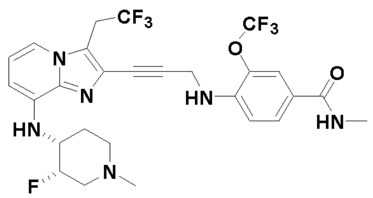 |

(continued)

| Structural formula | Structural formula |
|---|---|
| 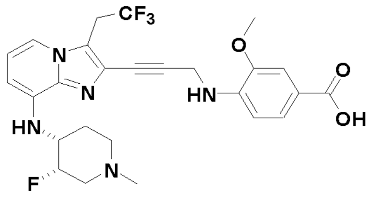 | 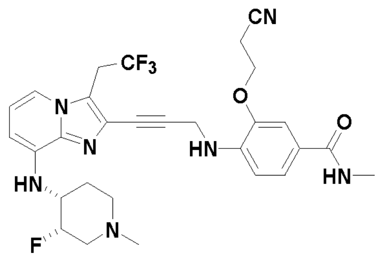 |
| 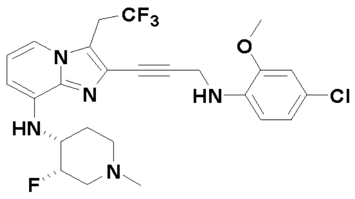 | 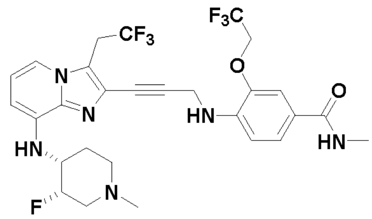 |
| 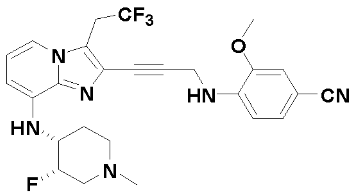 | 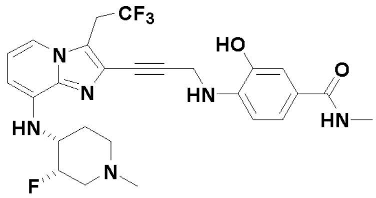 |
| 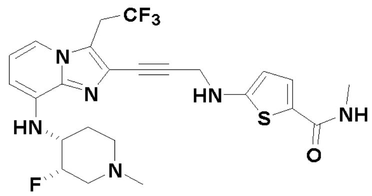 | 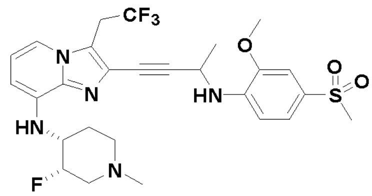 |
| 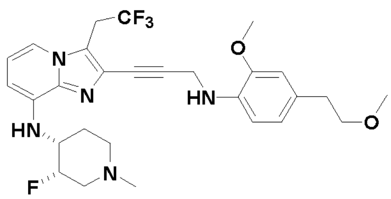 | 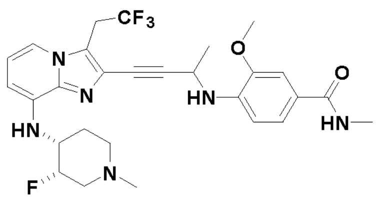 |
| 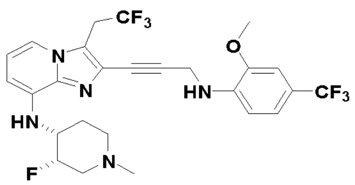 | |

In some embodiments, the compound is selected from the group consisting of

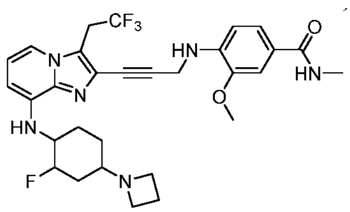

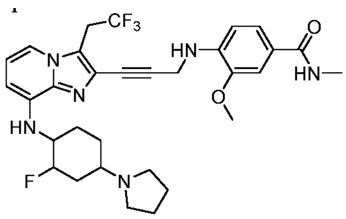

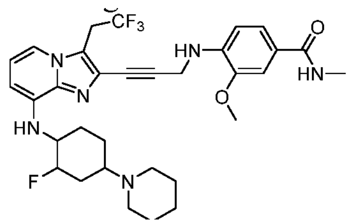

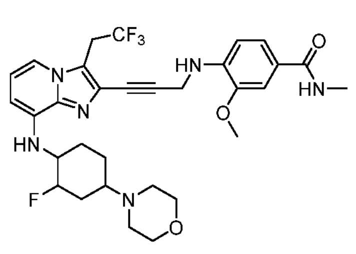
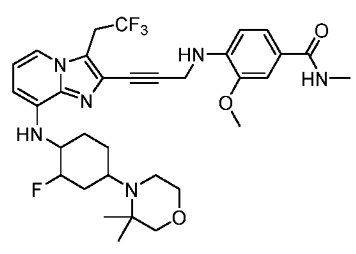
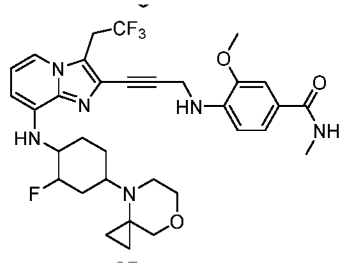

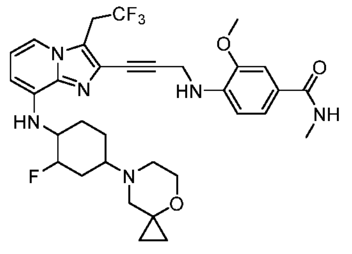
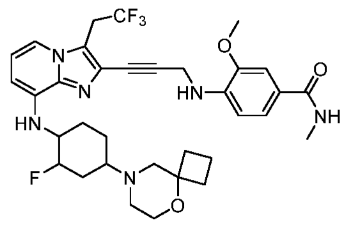
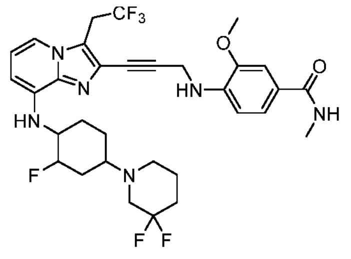

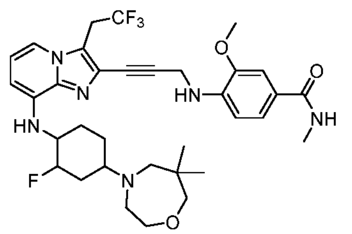
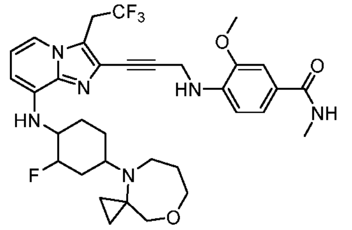
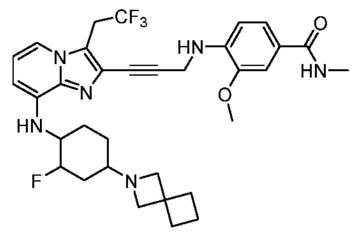

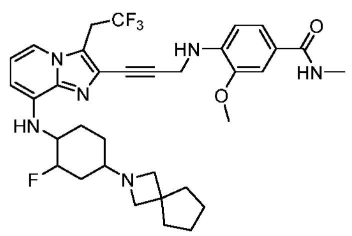
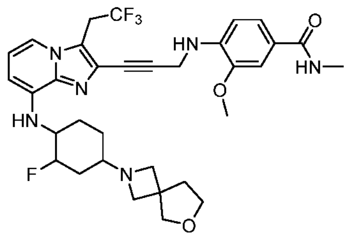
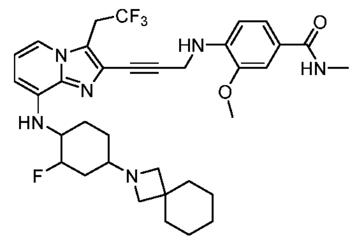

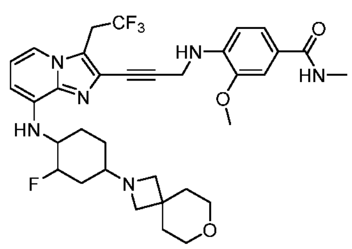
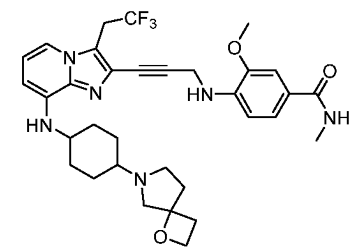
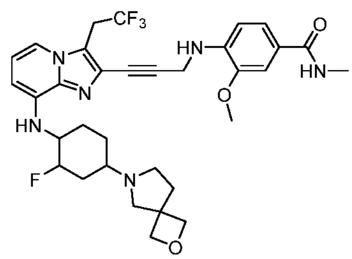

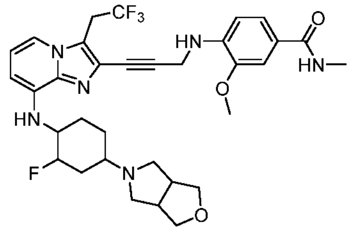
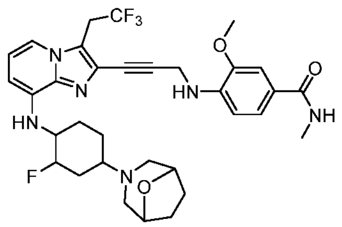
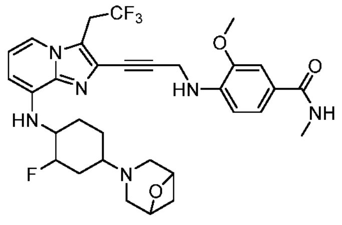

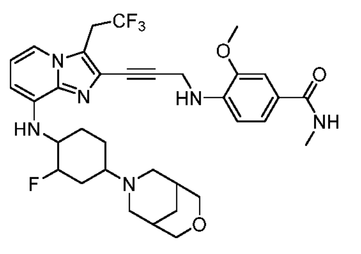
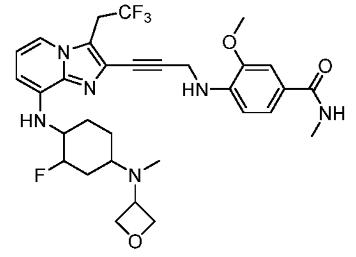
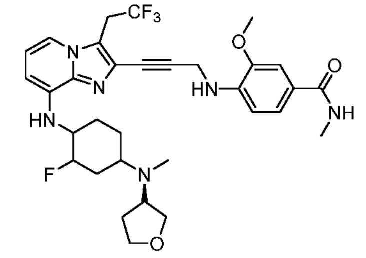
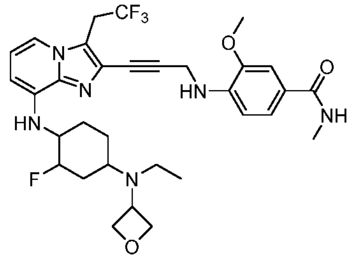
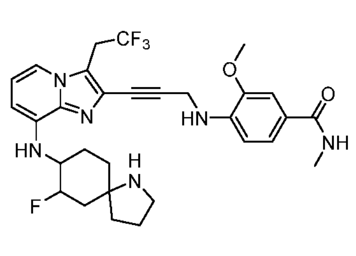
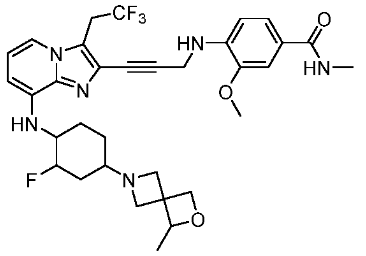
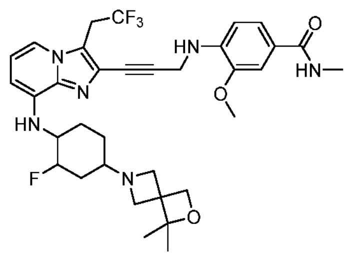
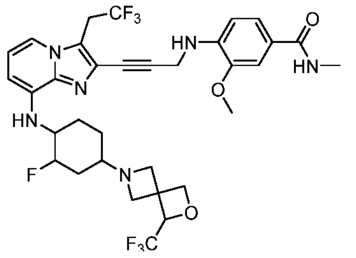
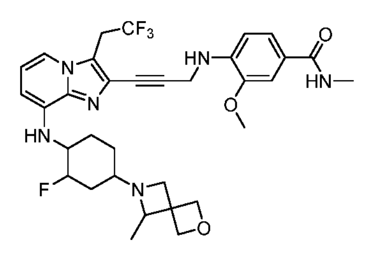
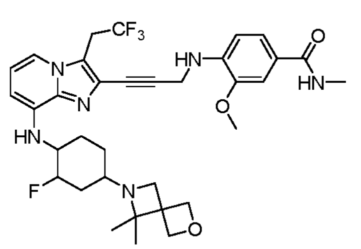
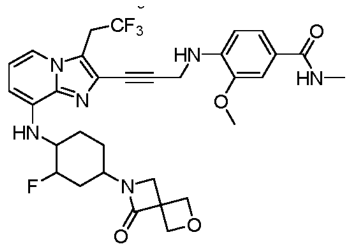
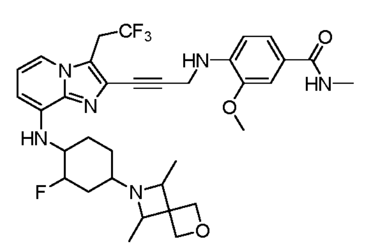
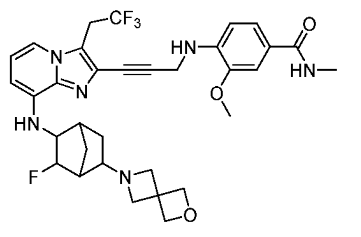
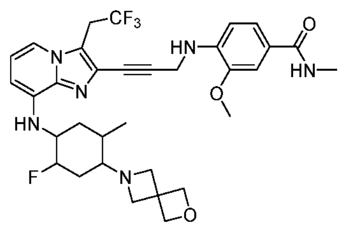
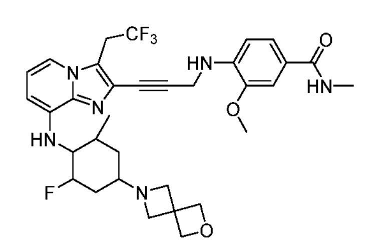
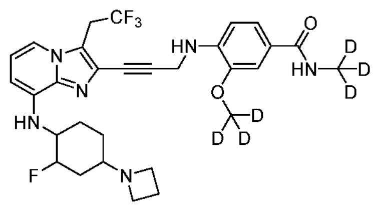
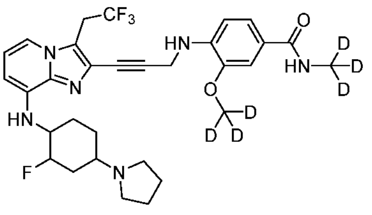
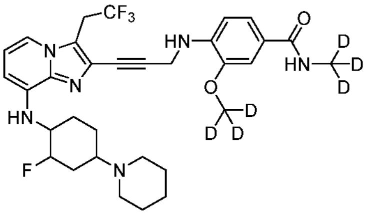

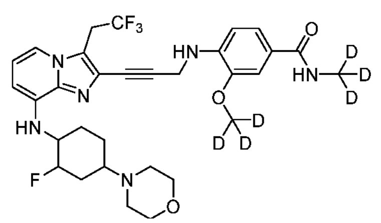
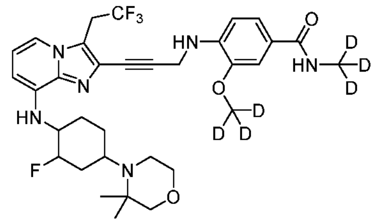
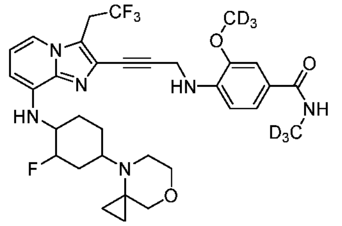

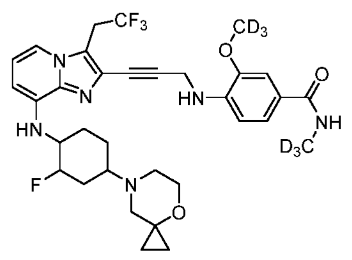
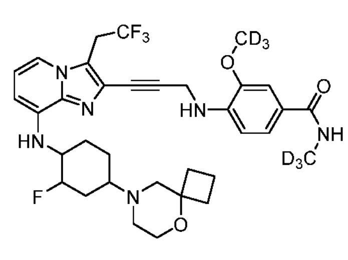
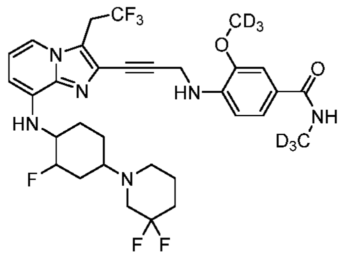

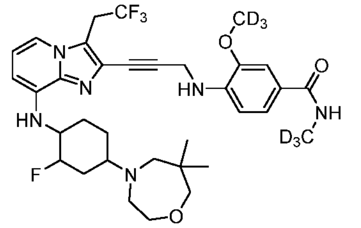
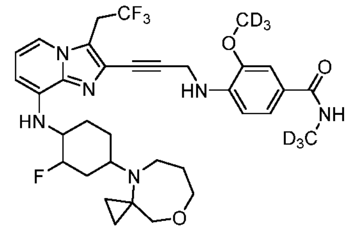
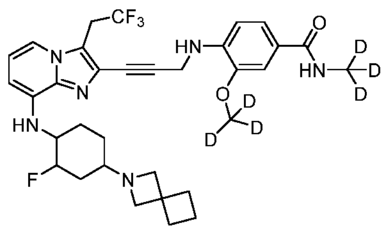

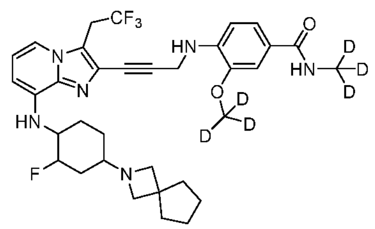
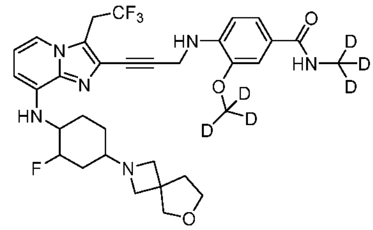
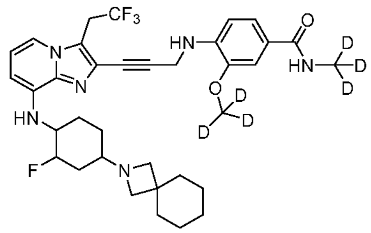

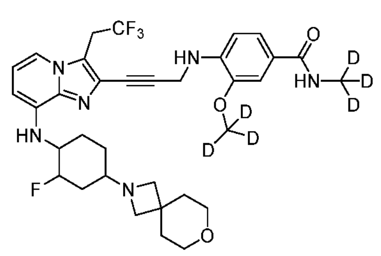
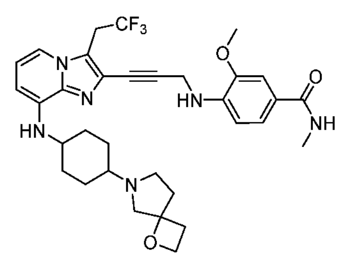
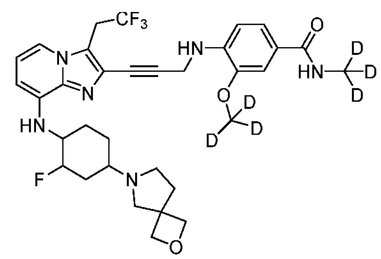

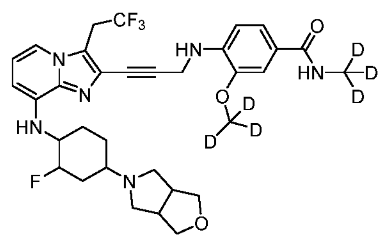
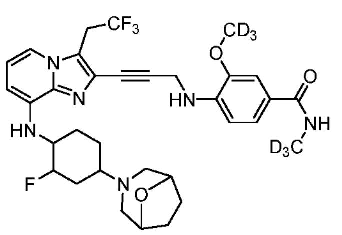
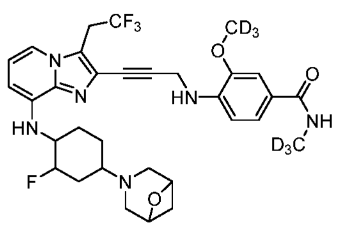

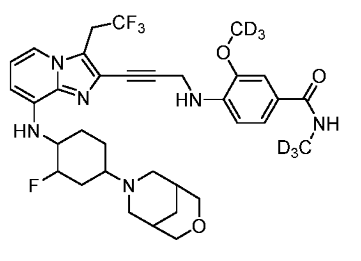
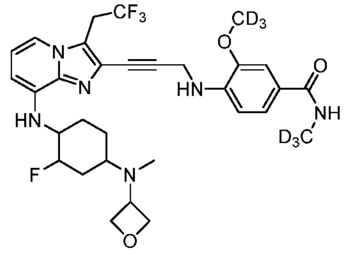
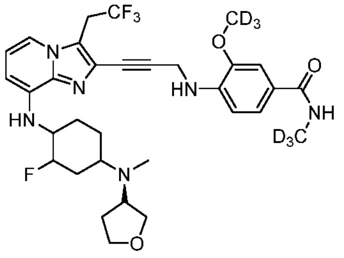

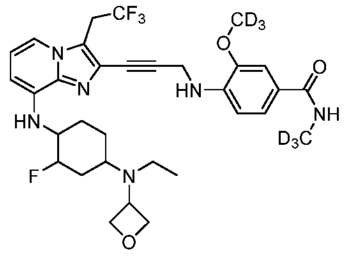
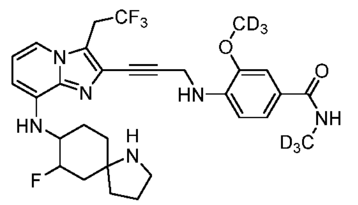
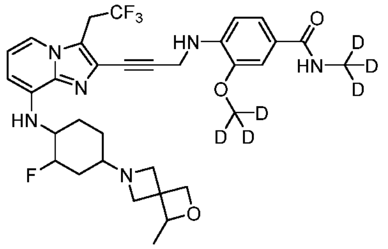

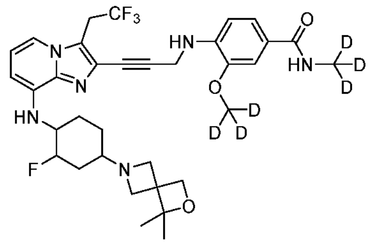
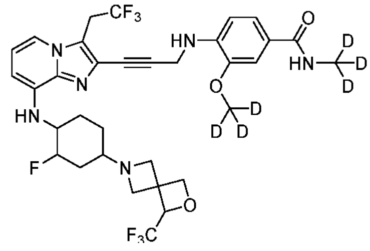
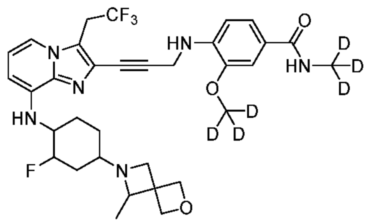

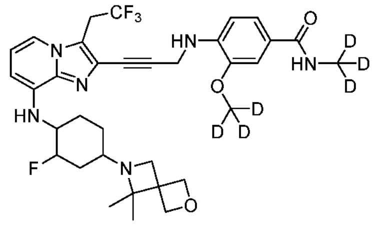
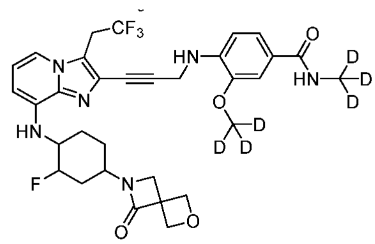
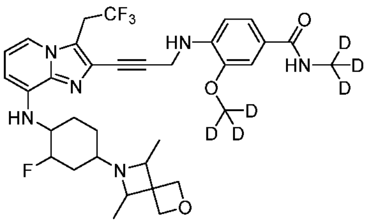

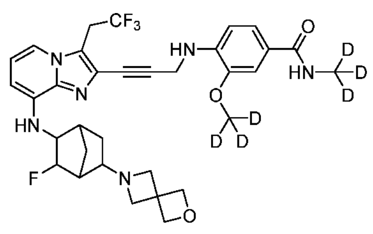
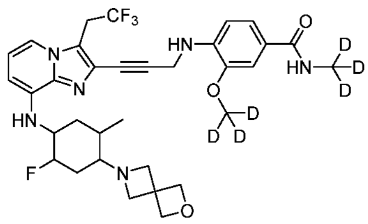
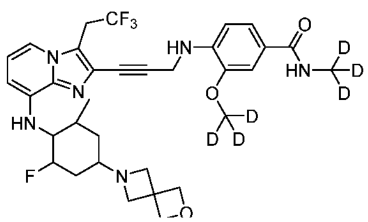

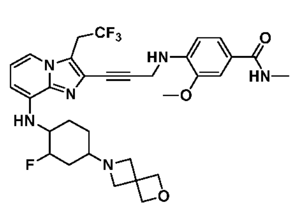
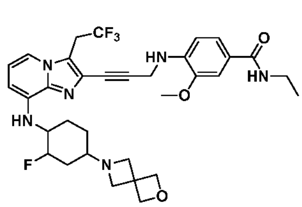
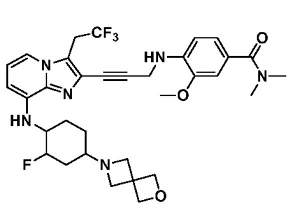
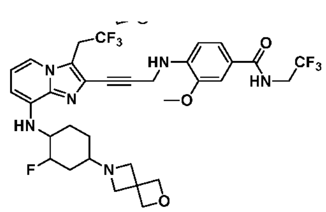

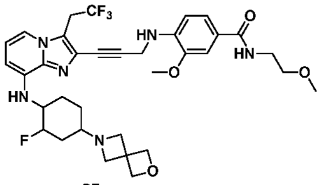
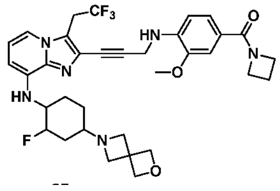
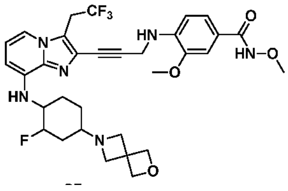
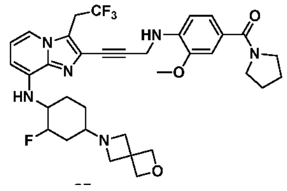

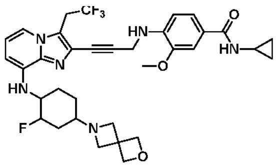
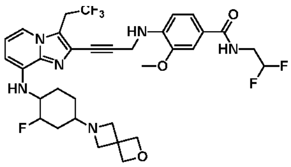
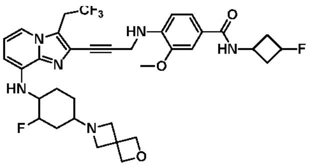
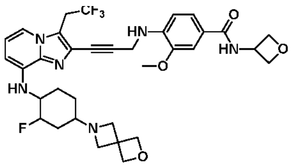

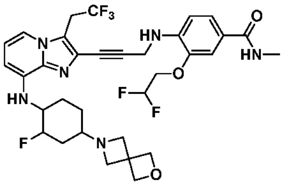
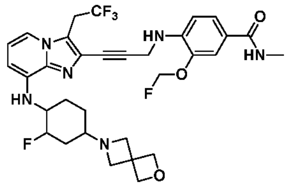
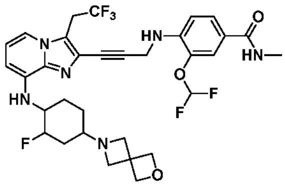
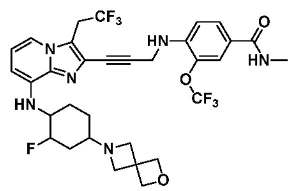

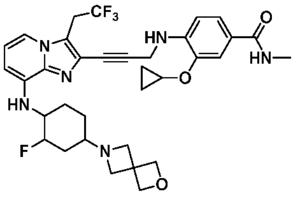
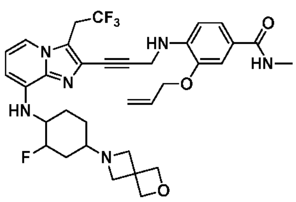
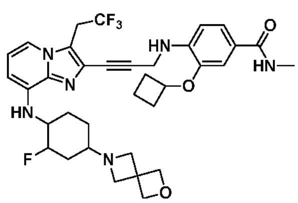
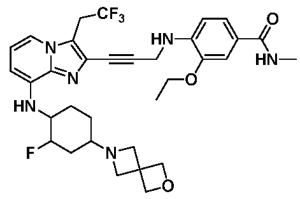

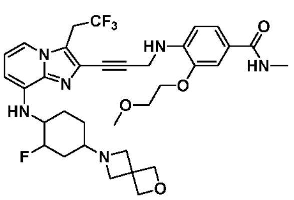
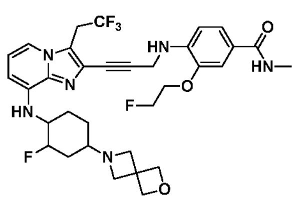
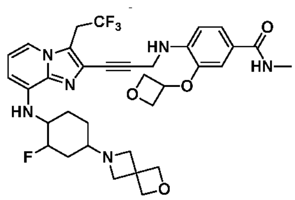
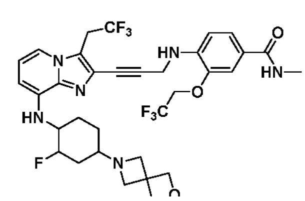

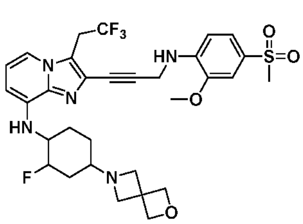
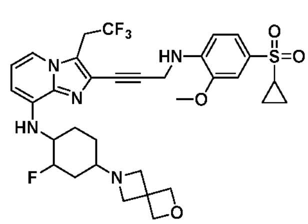
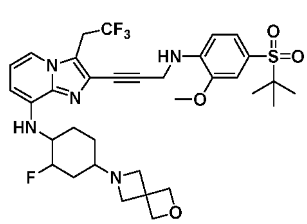

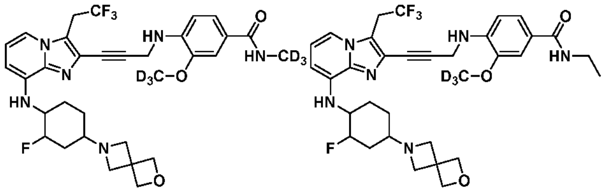
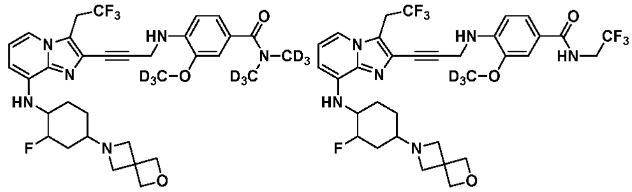

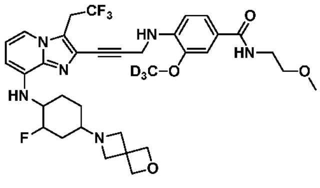
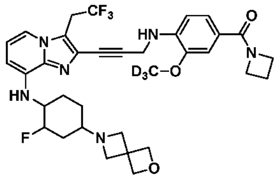
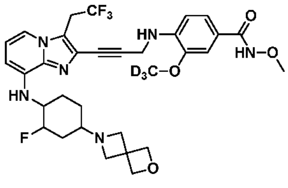
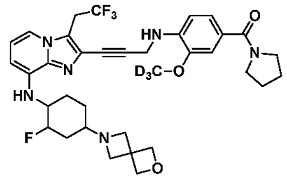

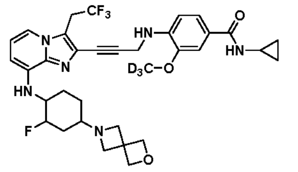
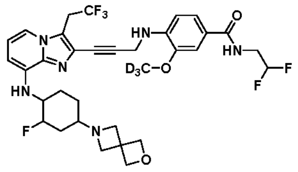
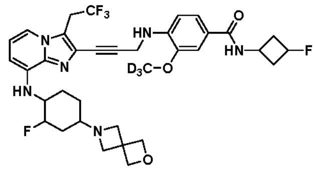
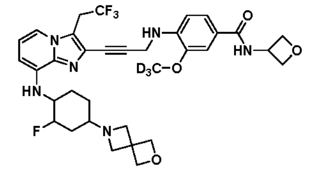

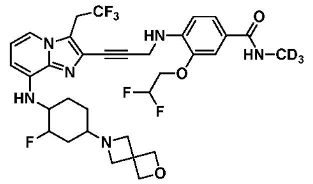
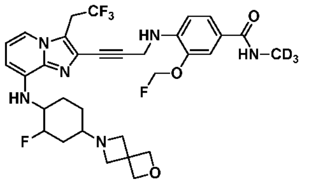
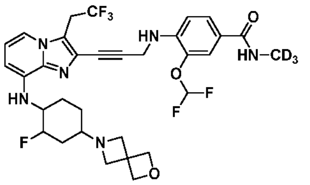
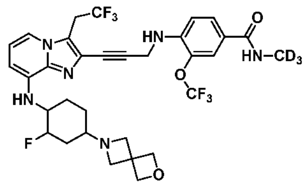

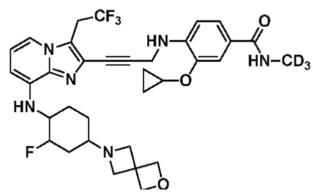
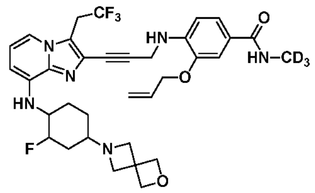
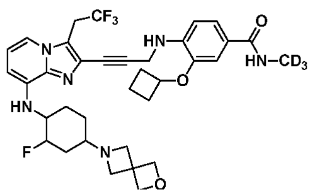
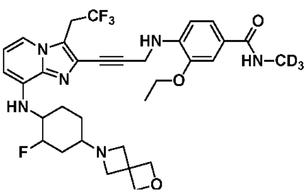

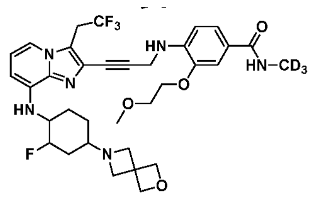
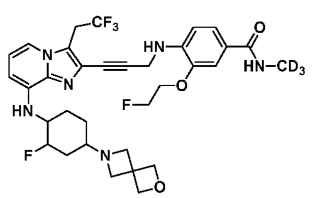
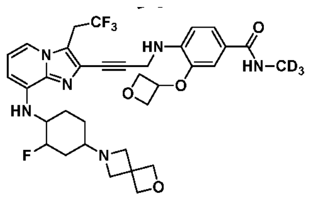
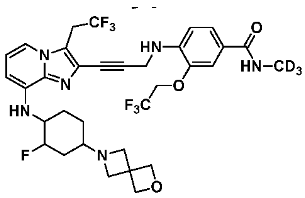

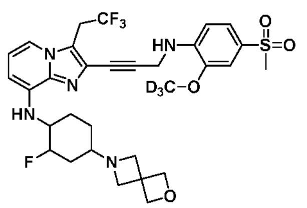
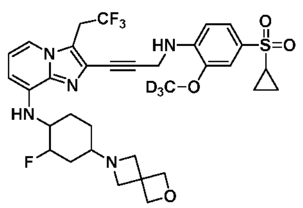
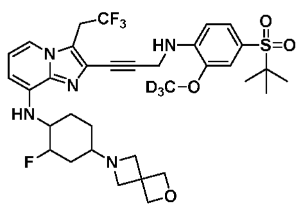

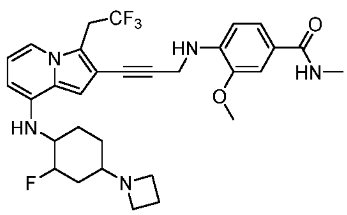
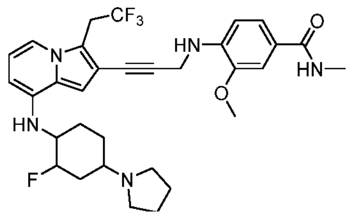
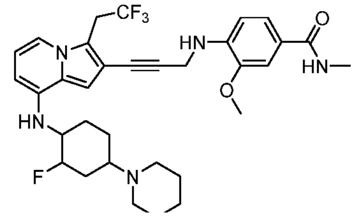

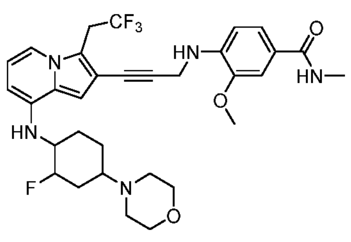
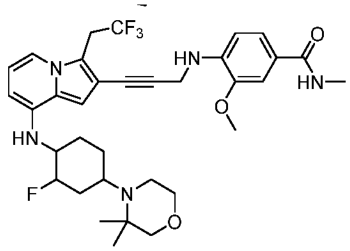
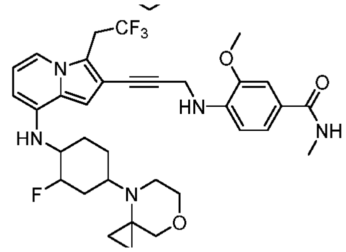

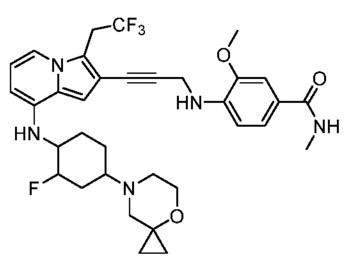
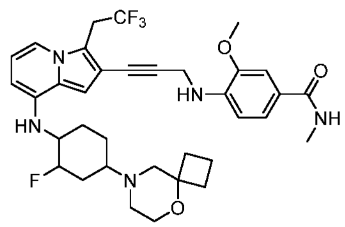
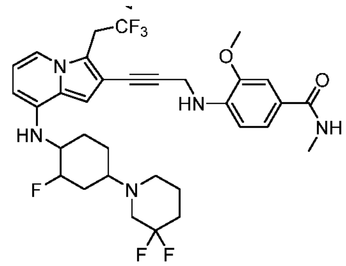

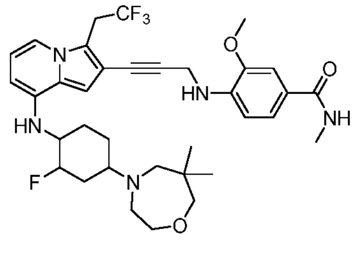
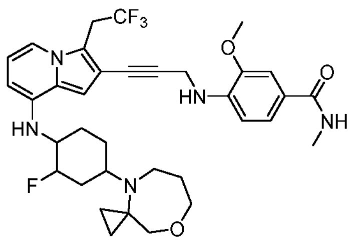
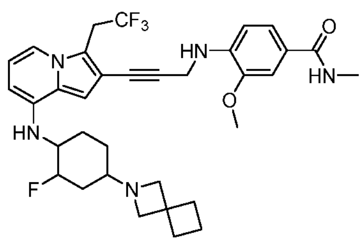

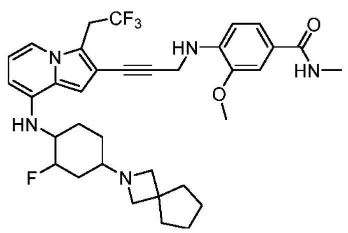
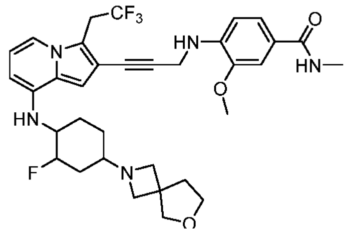
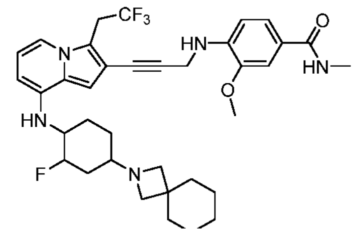

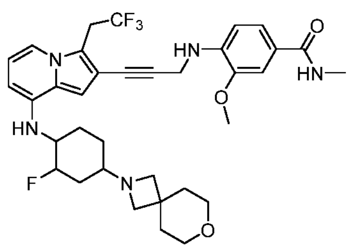
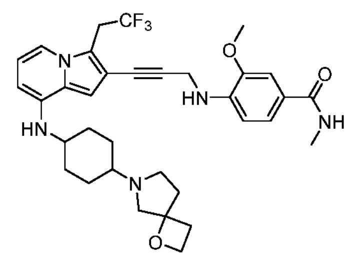
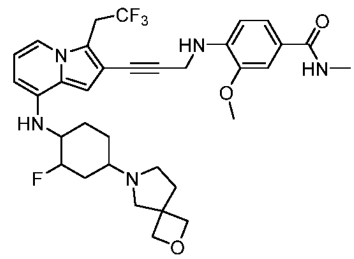

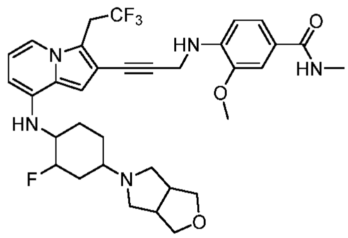
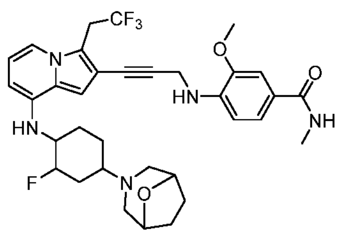
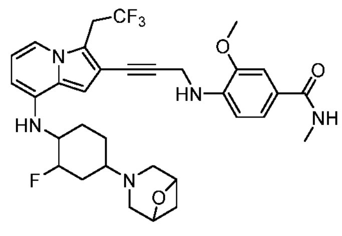

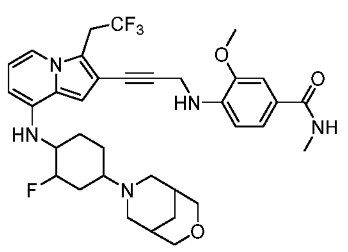
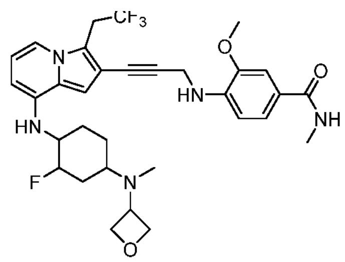
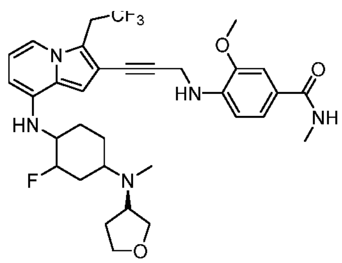

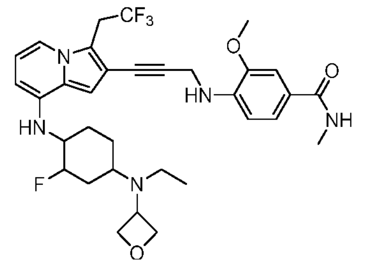
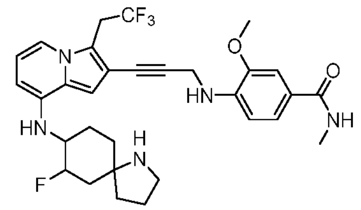
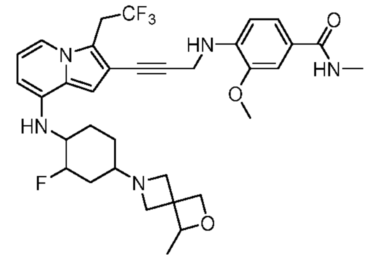

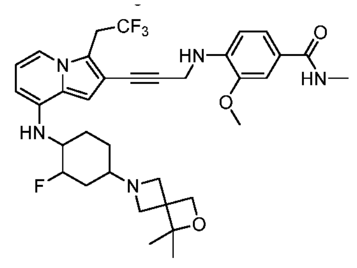
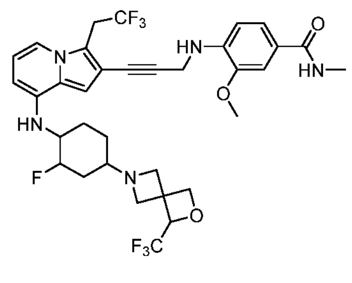
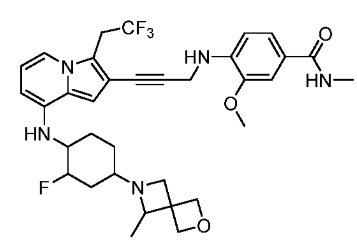

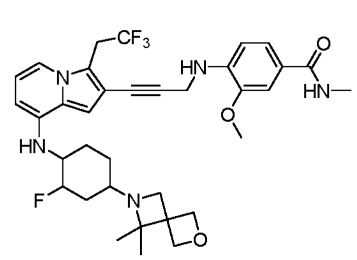
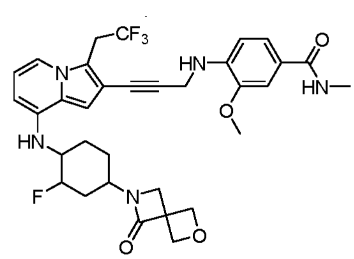
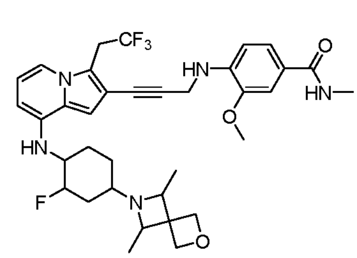

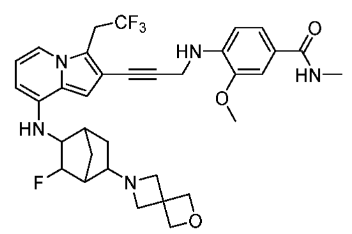
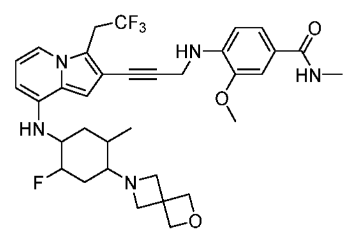
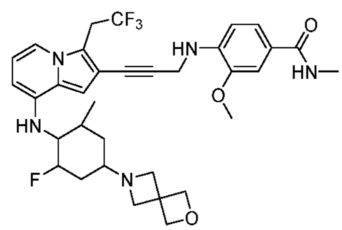

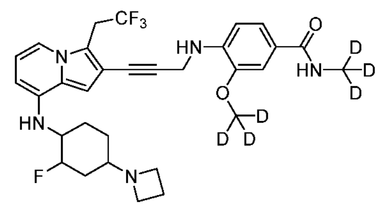
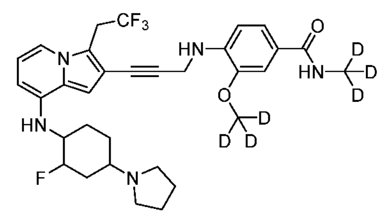
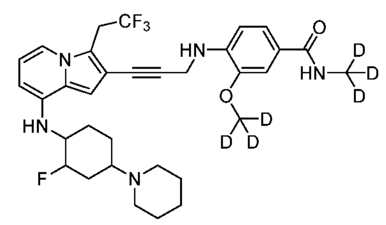

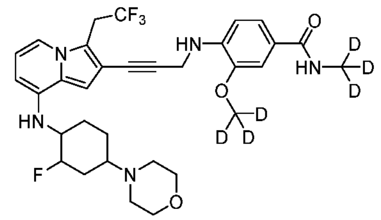
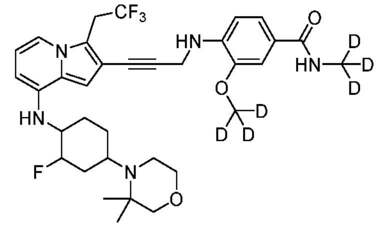
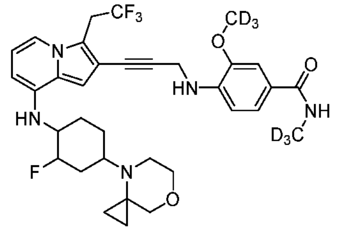

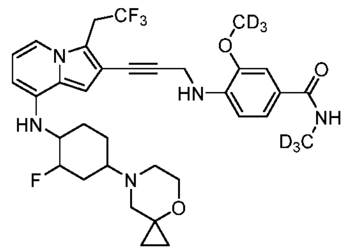
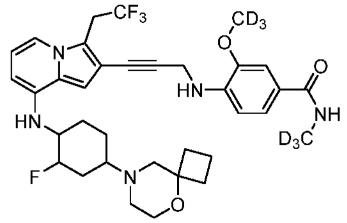
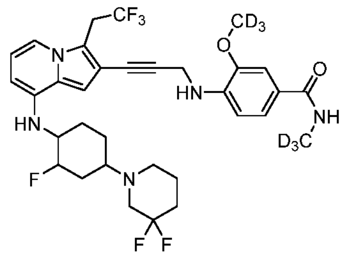

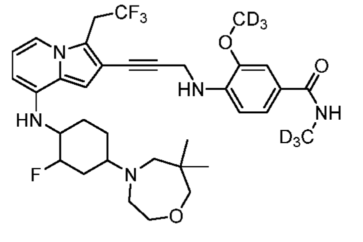
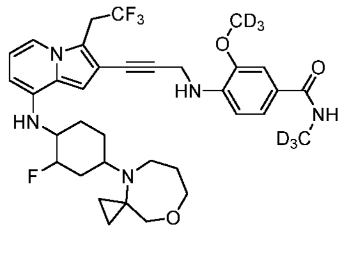
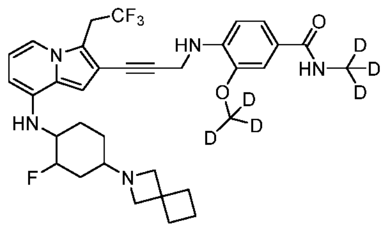

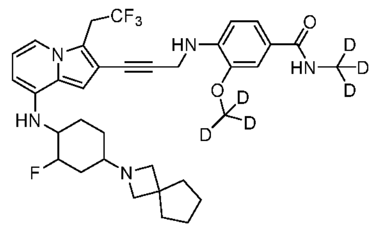
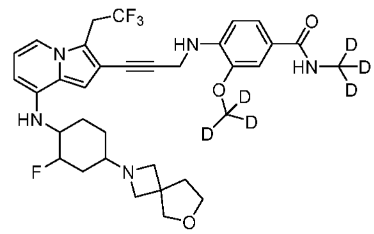
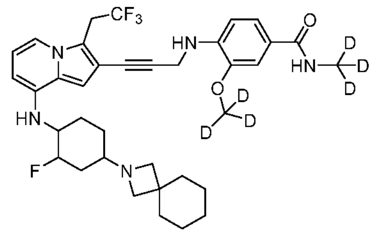

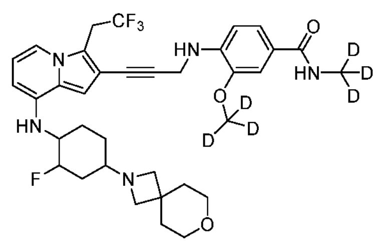
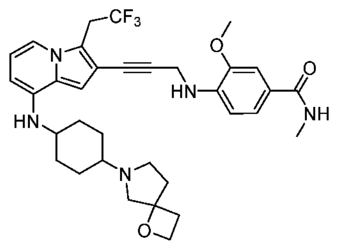
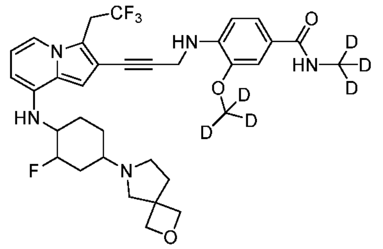

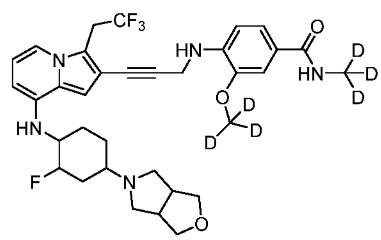
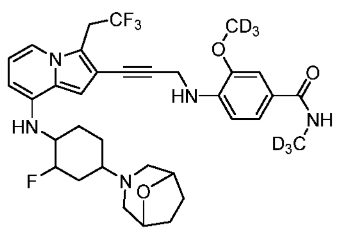
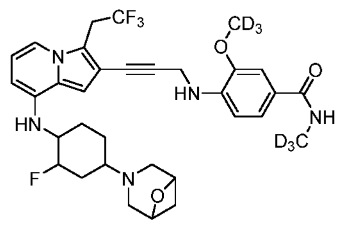

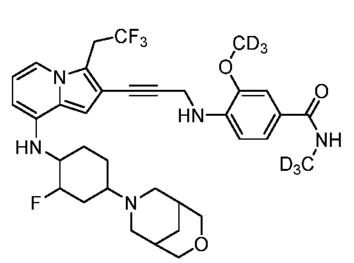
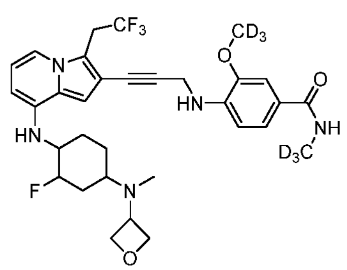
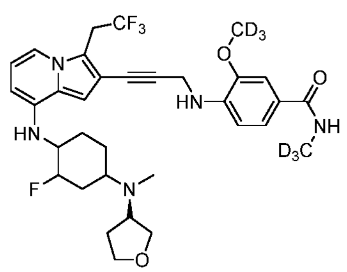

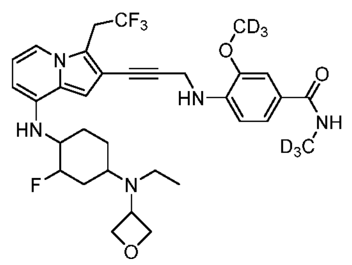
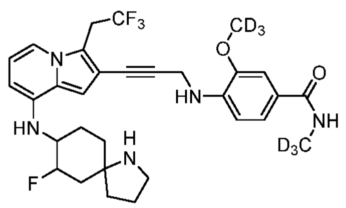
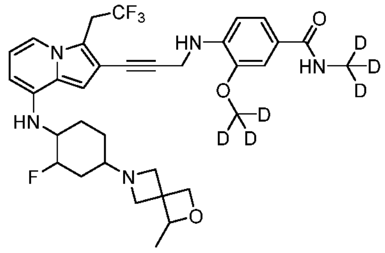

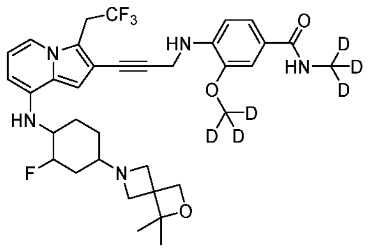
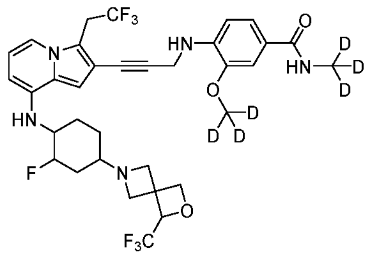
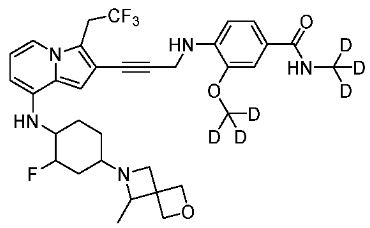

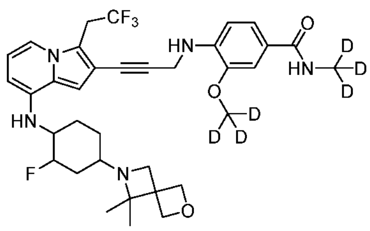
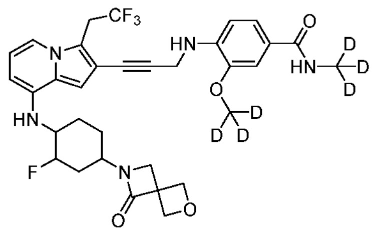
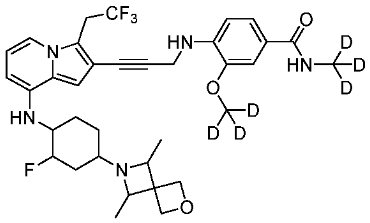

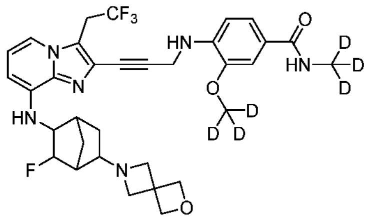
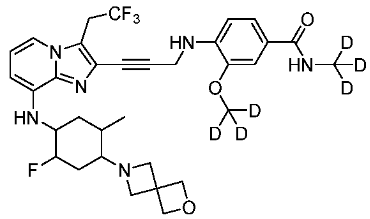
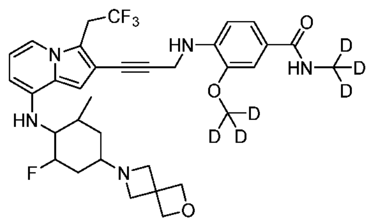

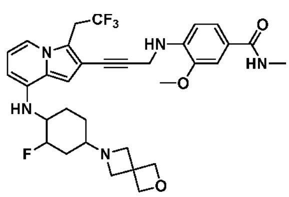
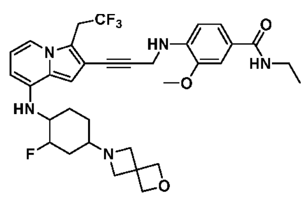
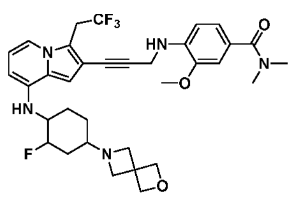
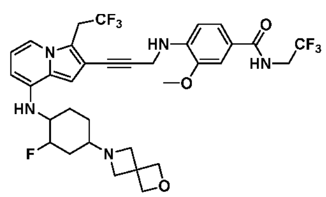

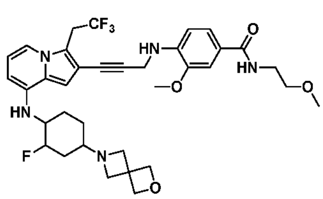
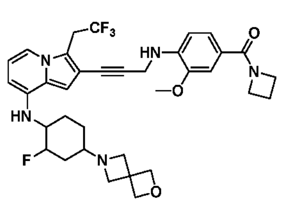
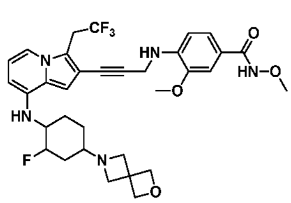
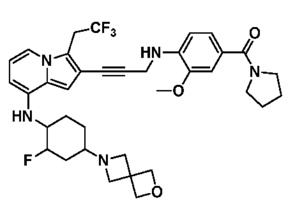

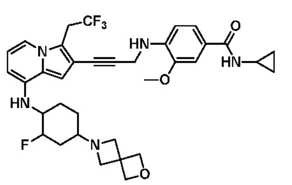
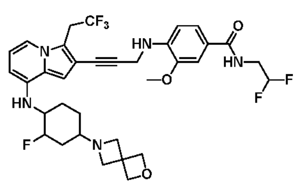
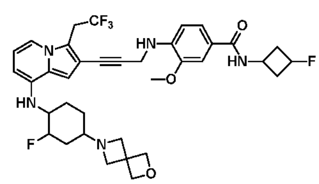
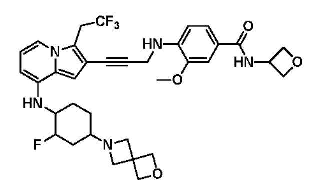

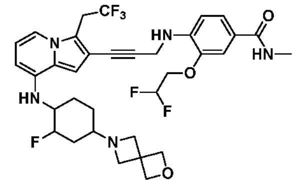
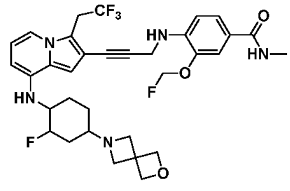
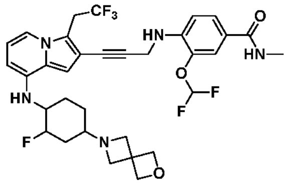
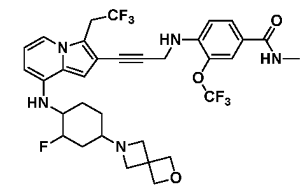

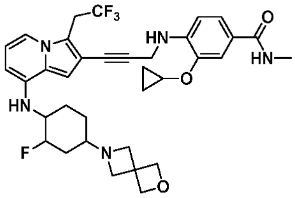
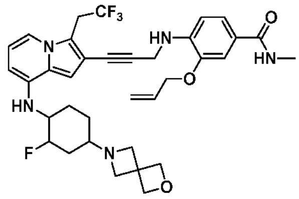
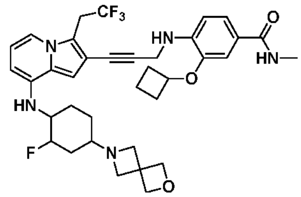
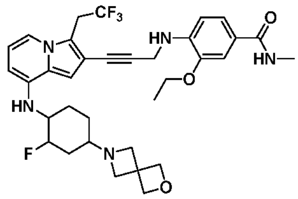

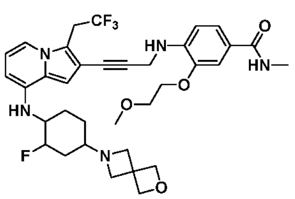
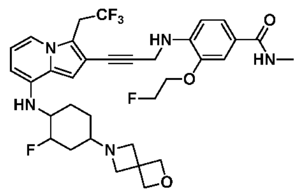
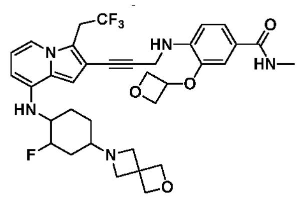
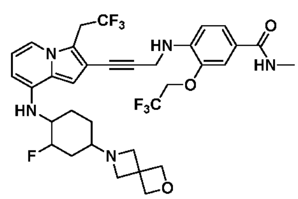

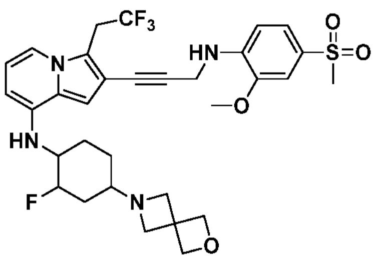
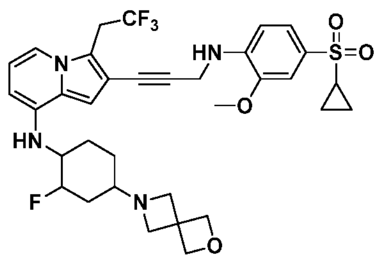
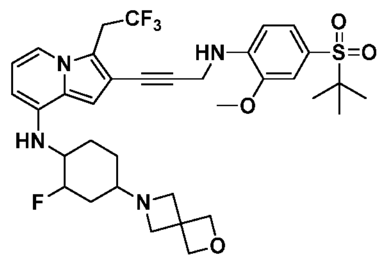

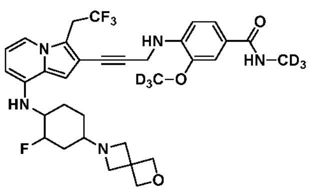
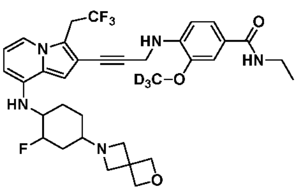
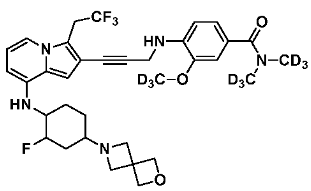
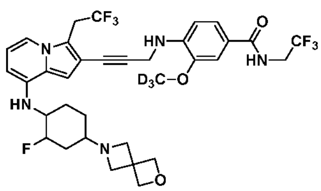

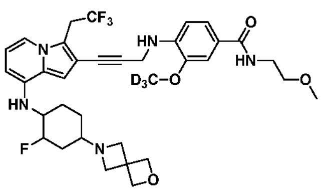
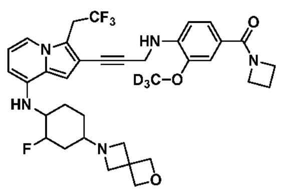
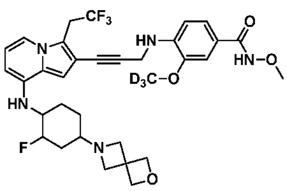
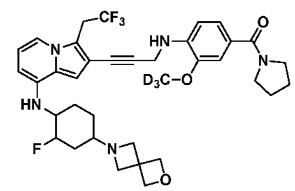

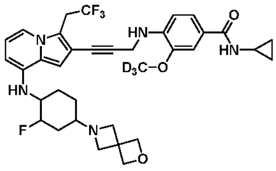
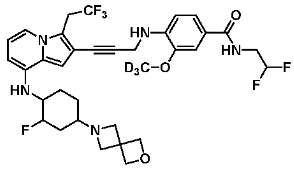
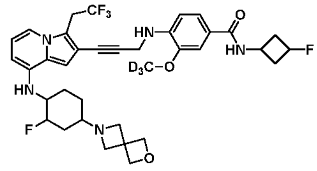
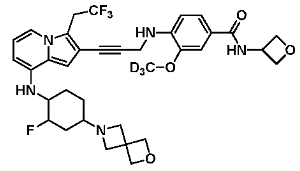

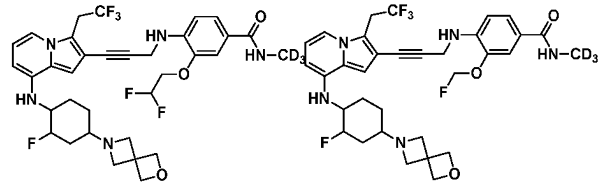
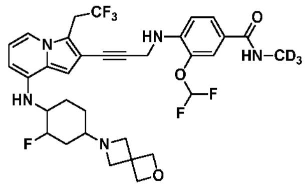
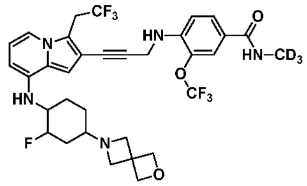

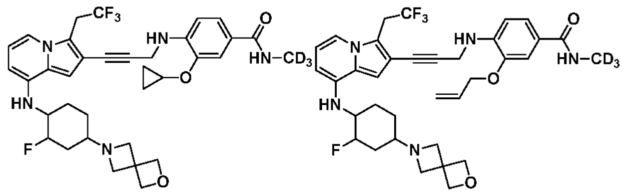
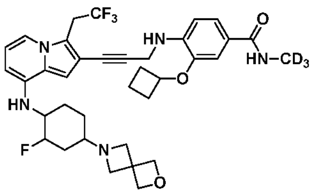
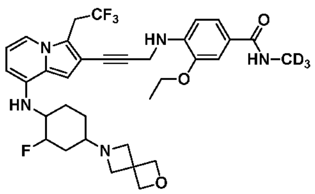

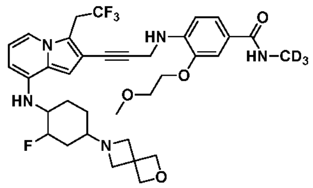
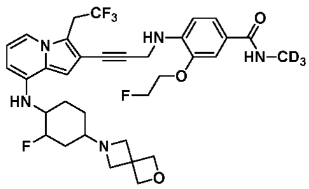
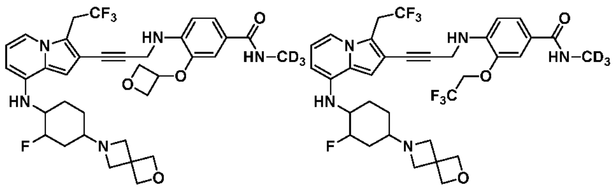

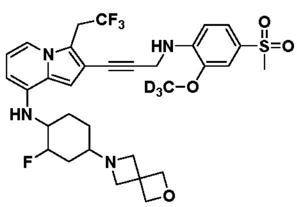
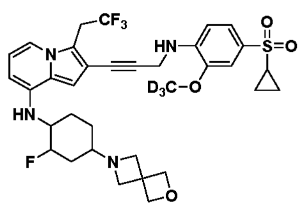
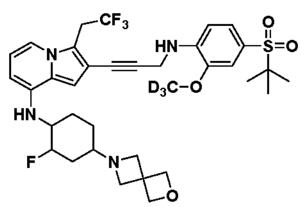

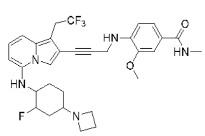
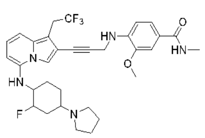
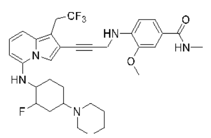
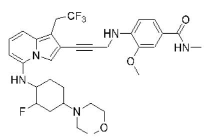
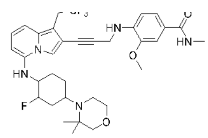

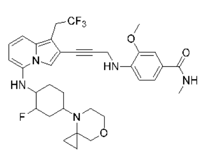
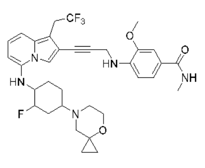
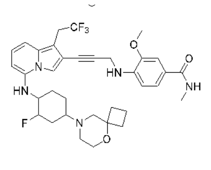
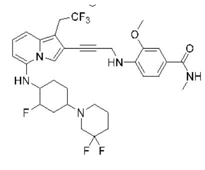
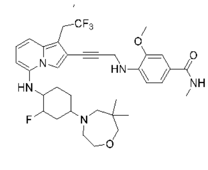

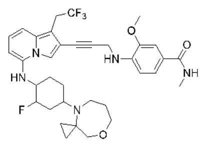
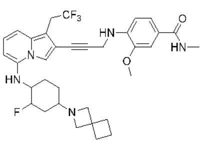
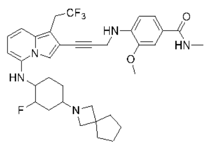
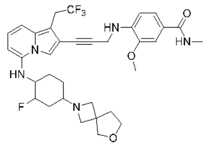
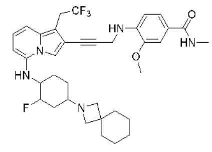

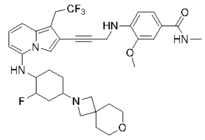
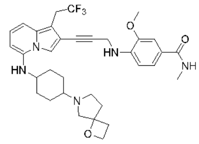
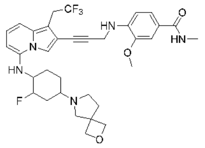
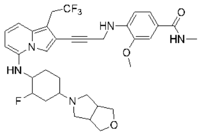
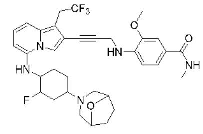

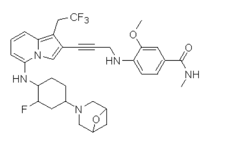
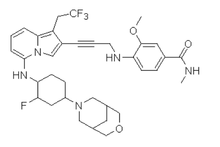
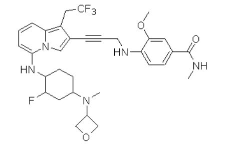
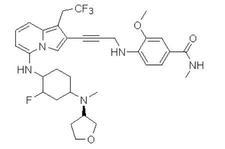
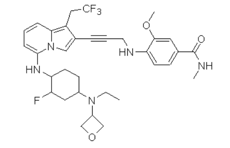

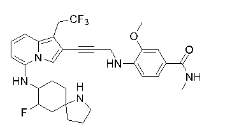
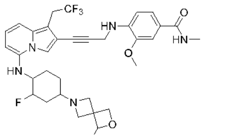
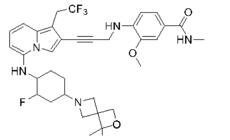
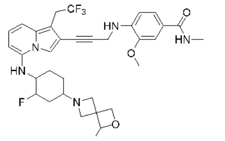
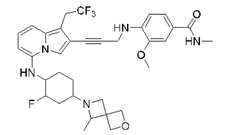

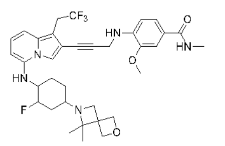
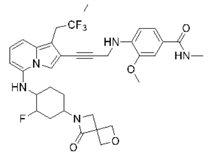
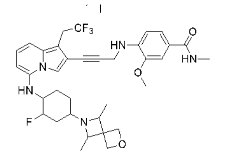
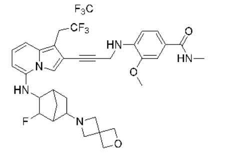
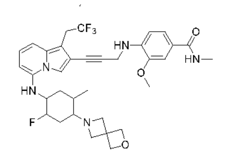

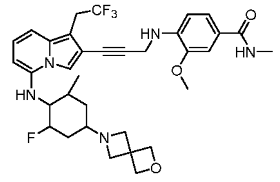

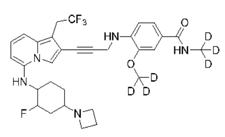
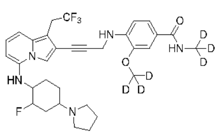
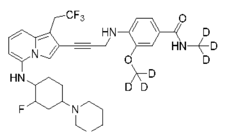
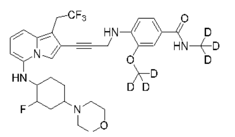
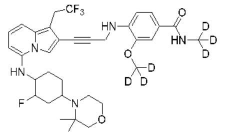

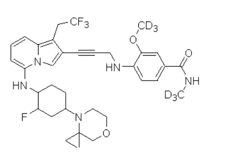
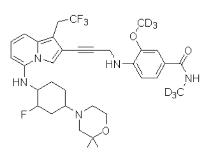
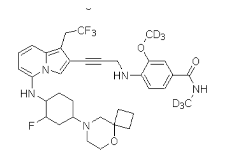
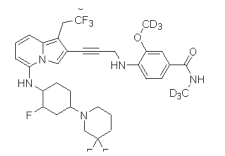
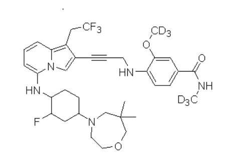

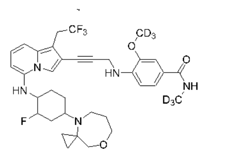
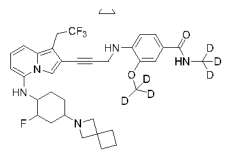
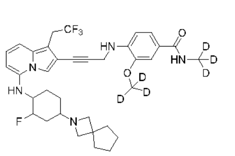
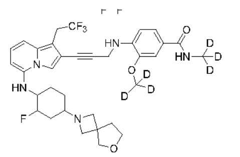
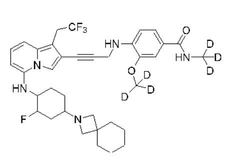

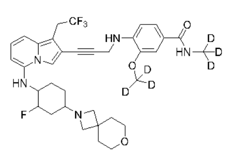
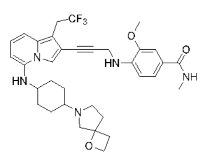
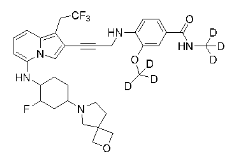
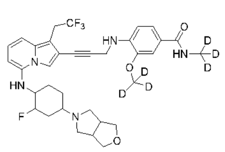
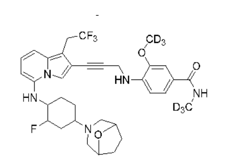

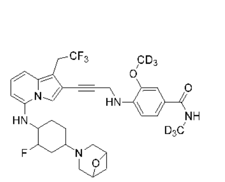
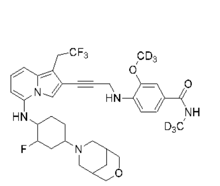
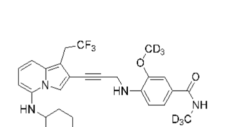
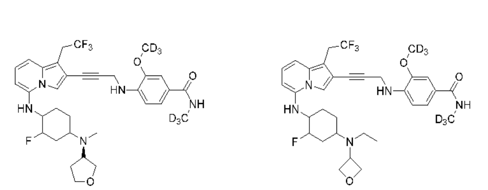

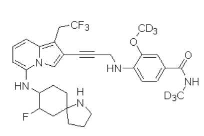 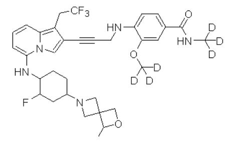 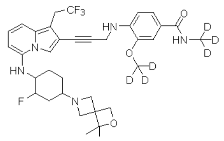 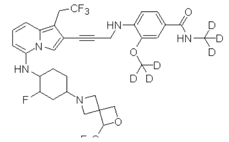 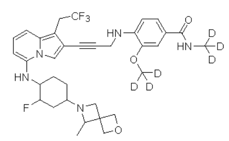

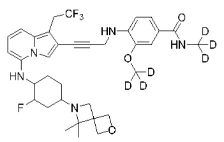 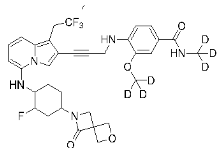 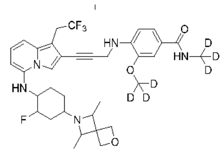 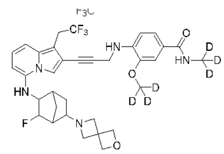 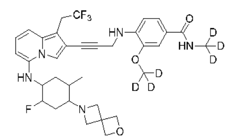

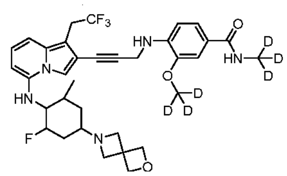

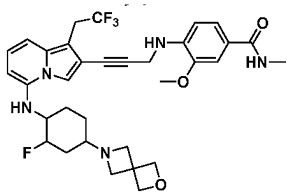 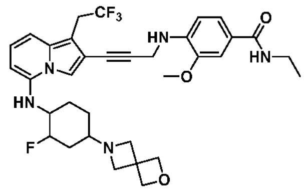 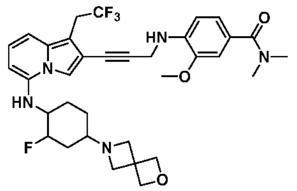 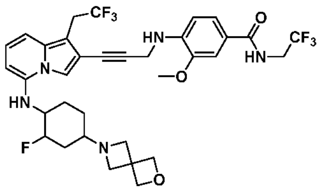

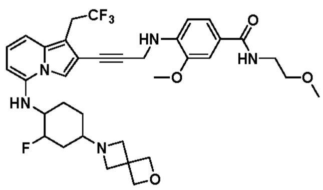 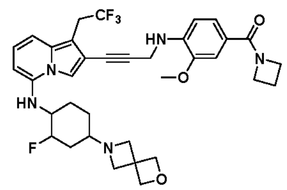 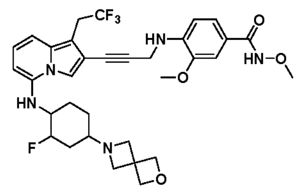 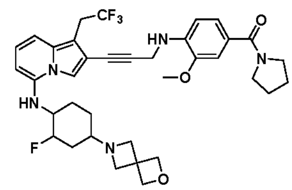

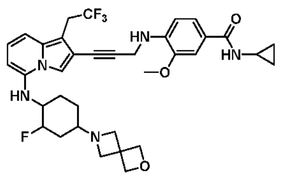 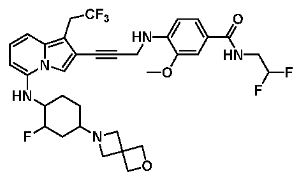 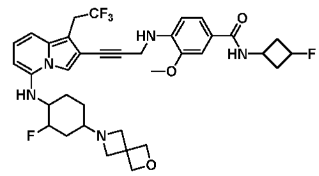 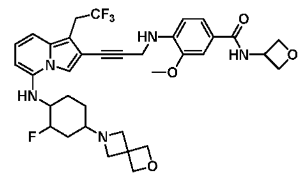

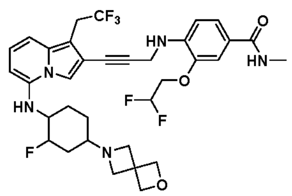 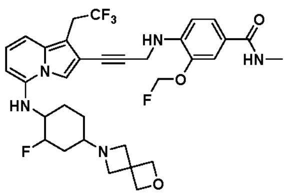 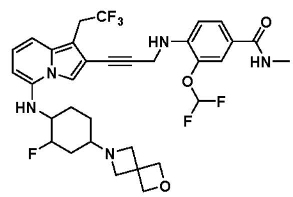 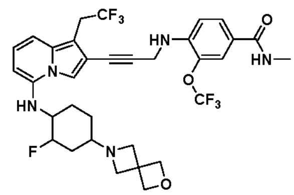

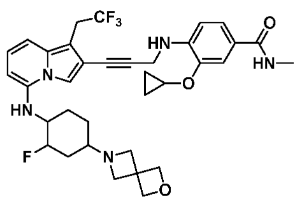 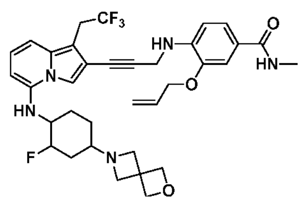 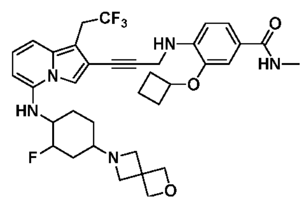 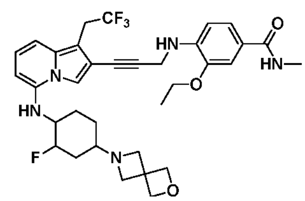

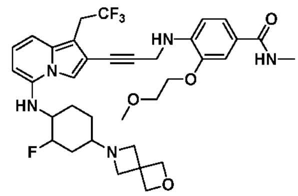
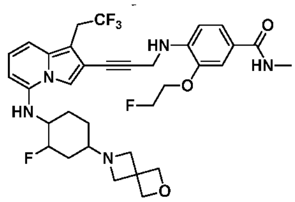
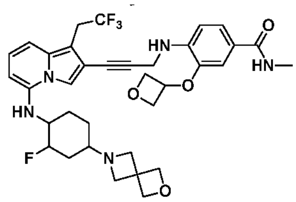
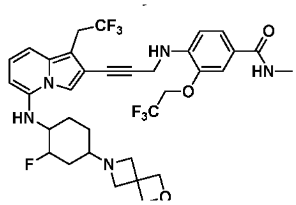

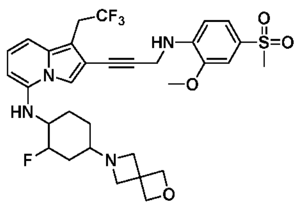
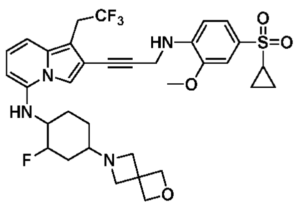
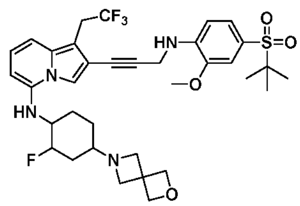

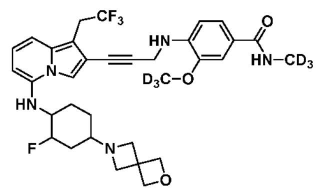
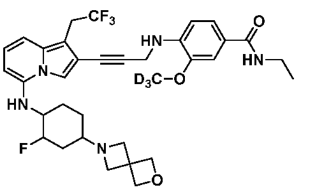
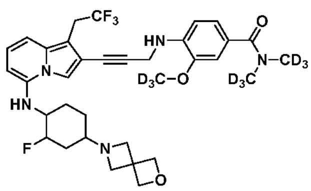
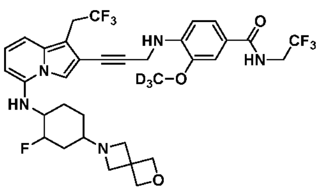

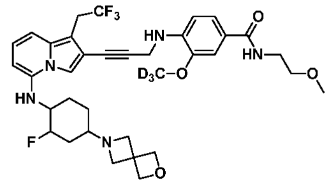
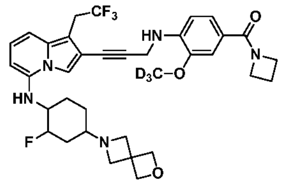
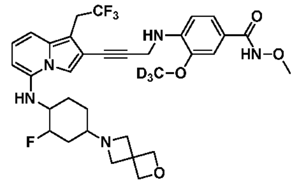
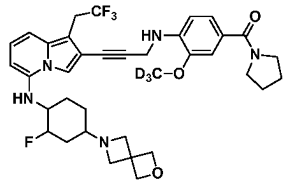

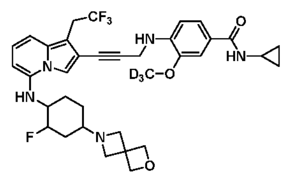
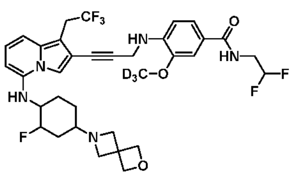
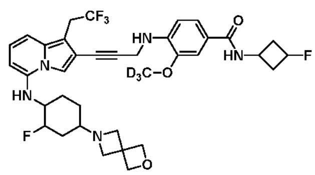
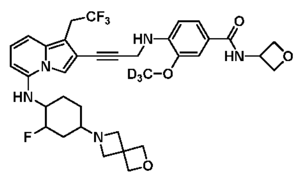

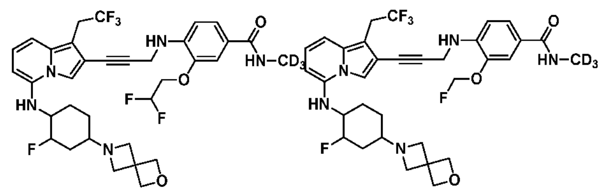
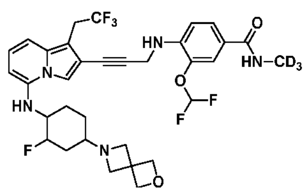
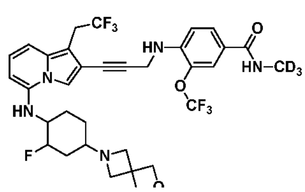

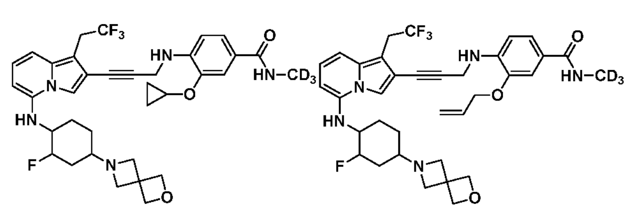
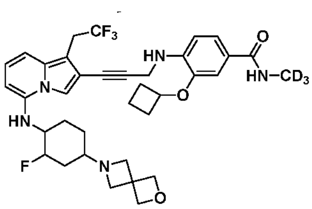
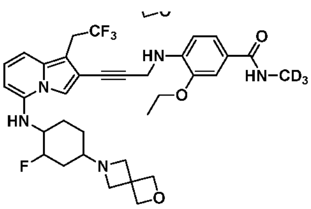

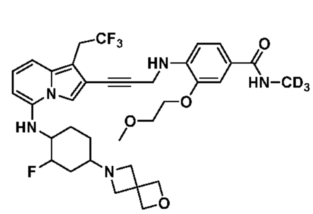
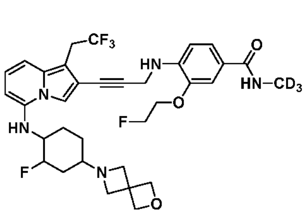
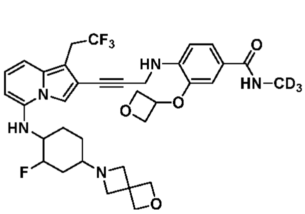
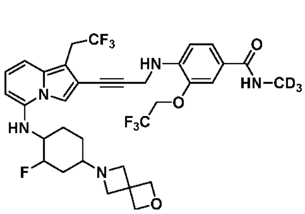

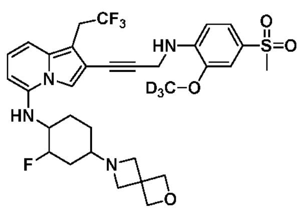

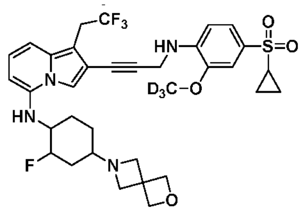

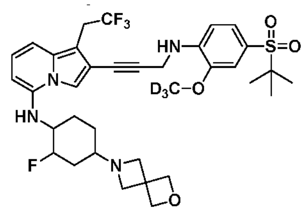

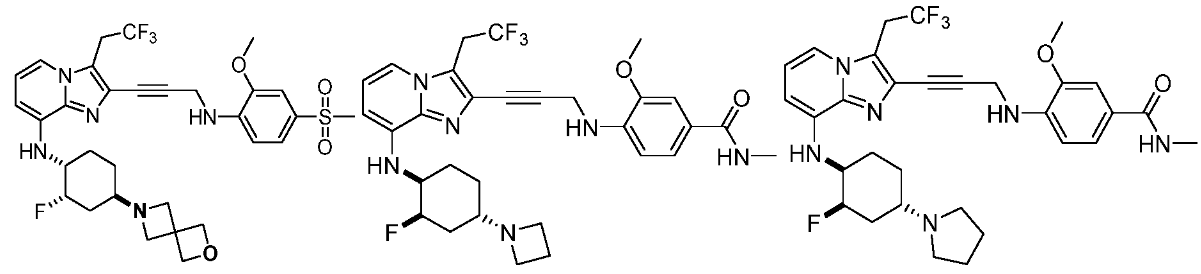

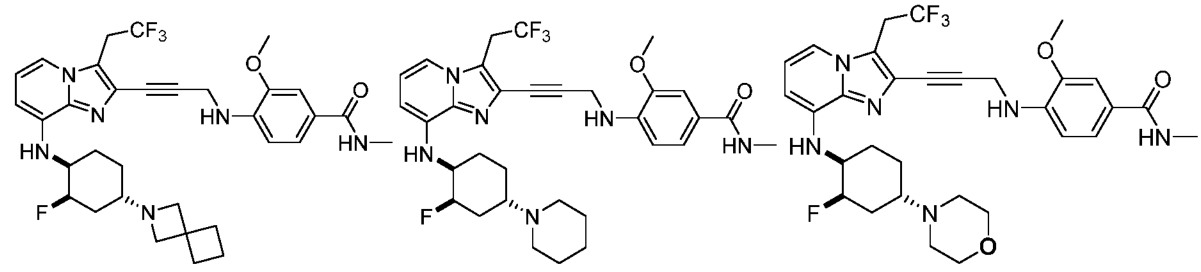

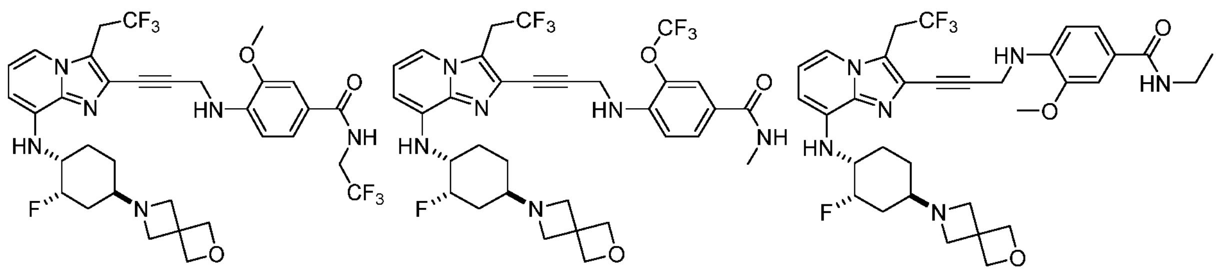

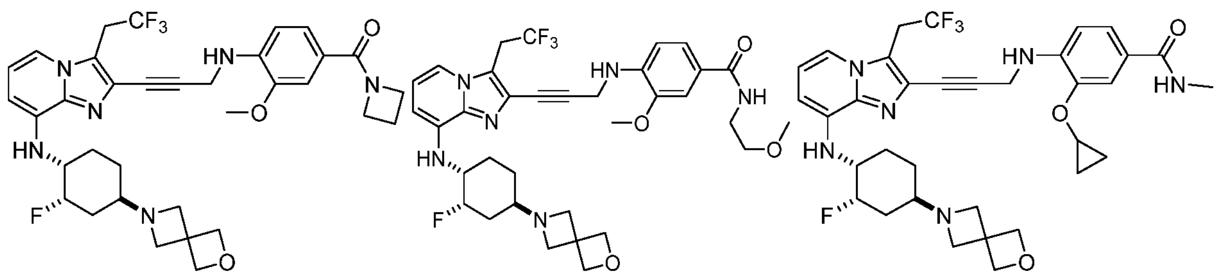

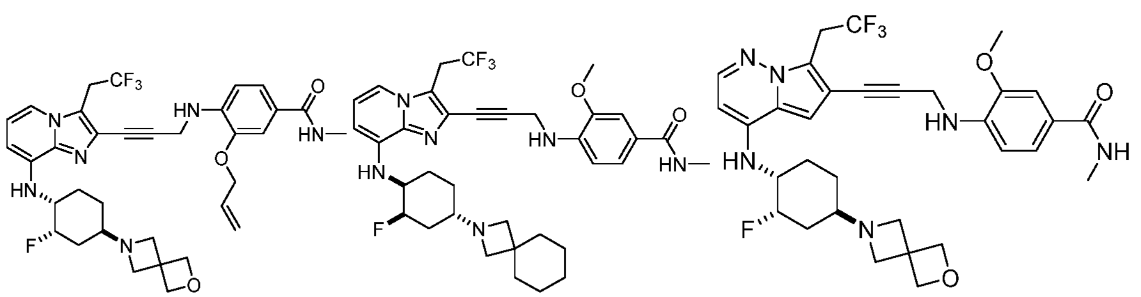

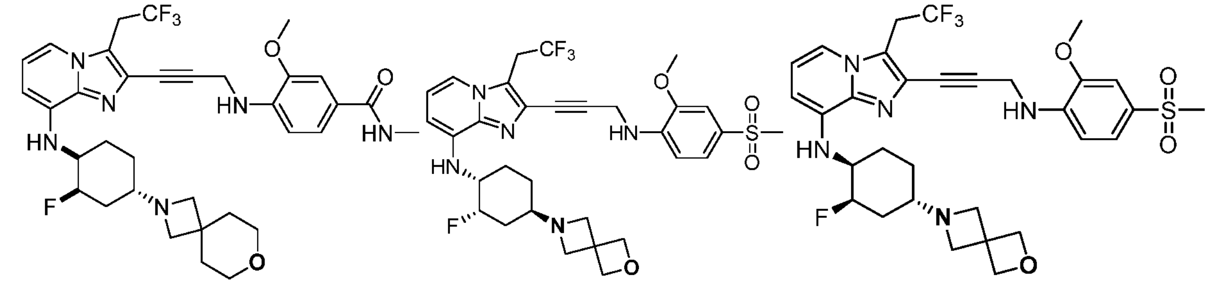

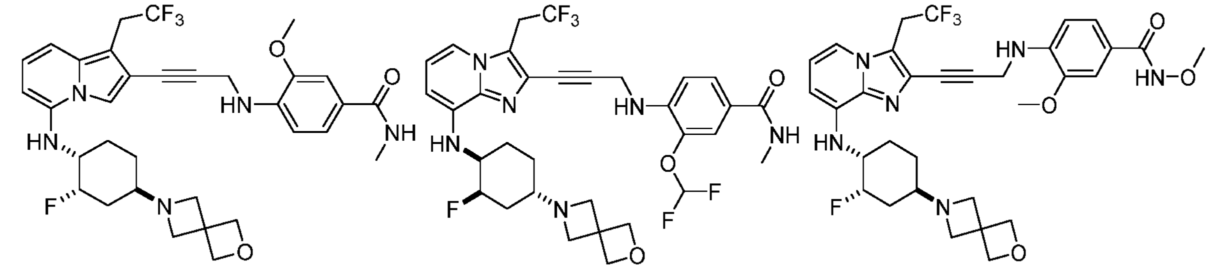

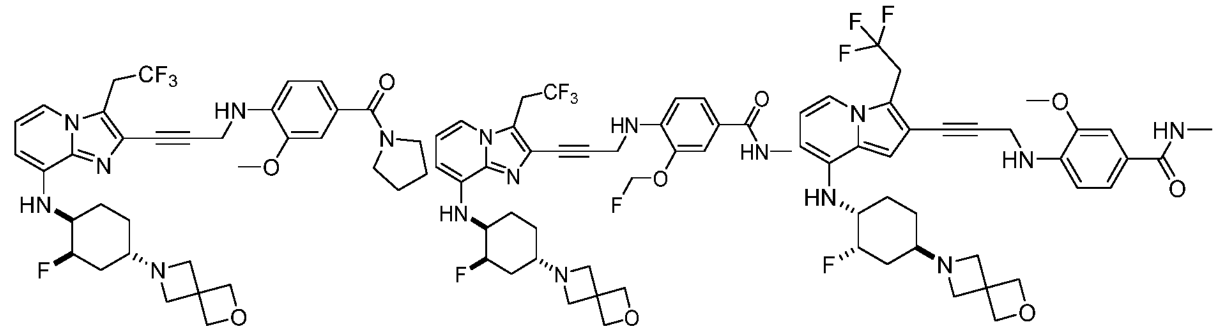

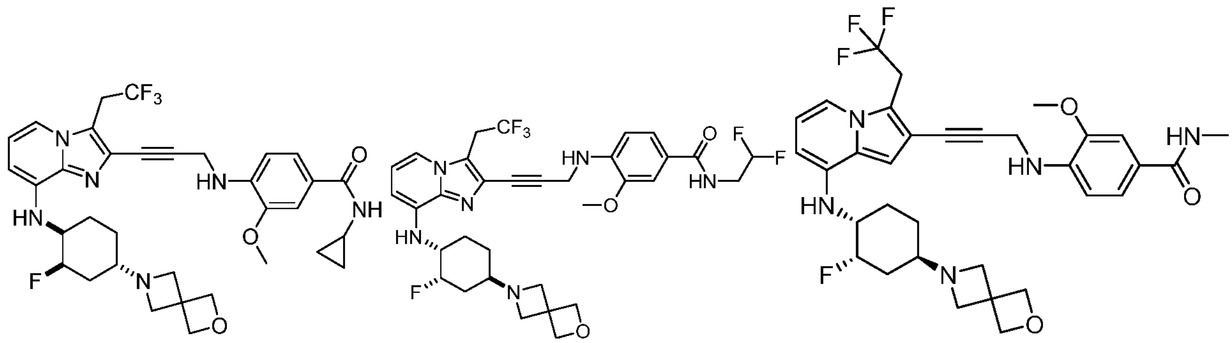

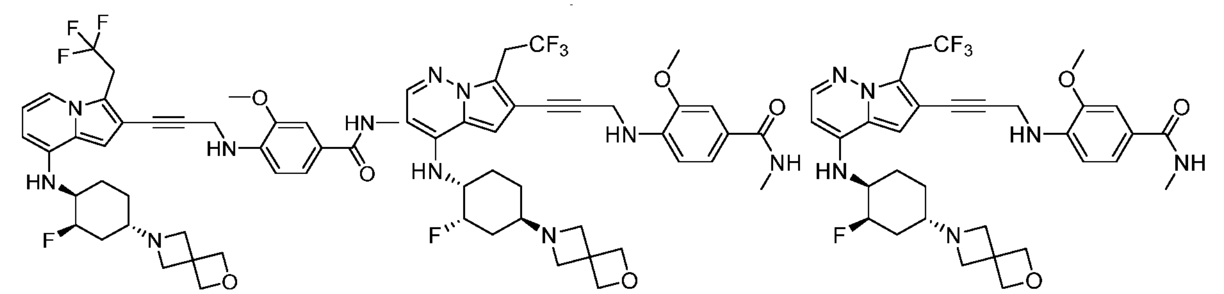

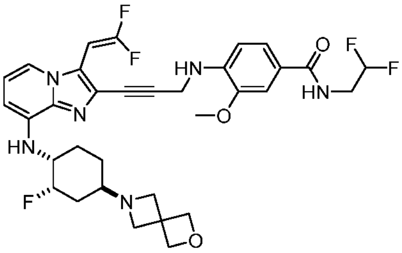
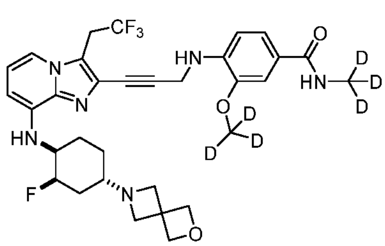
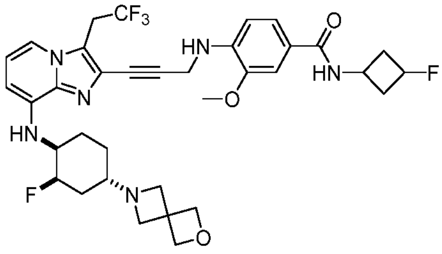

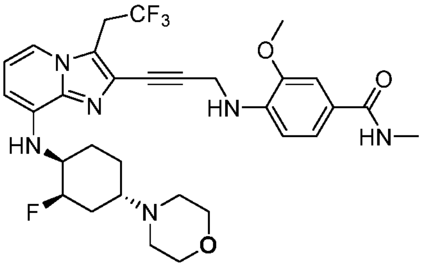
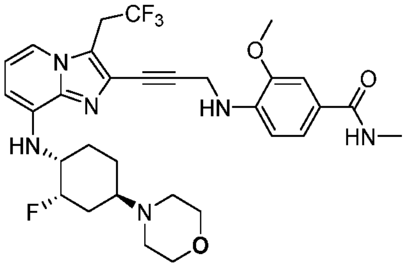
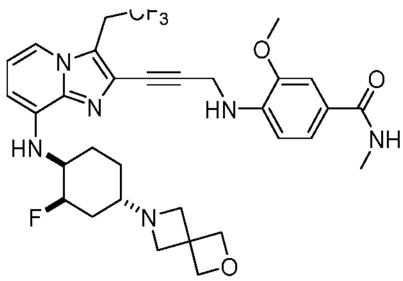

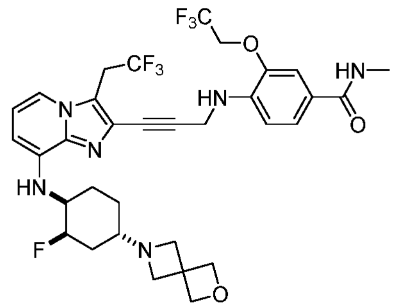
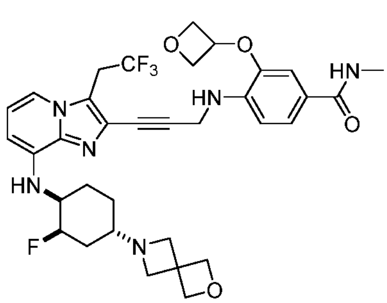
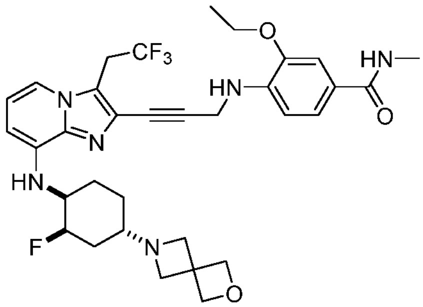

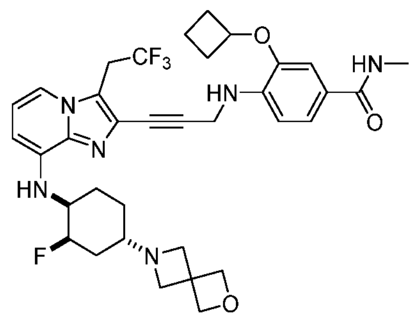
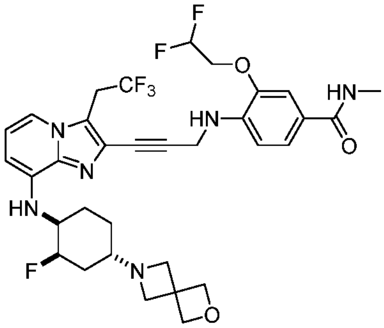
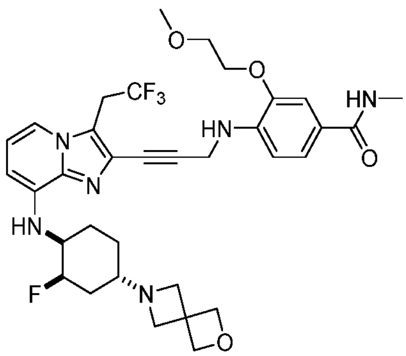

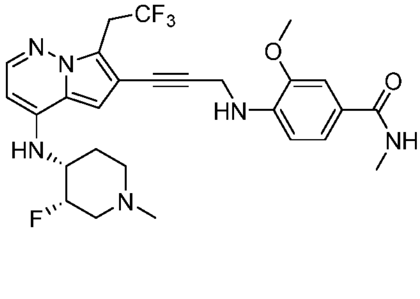
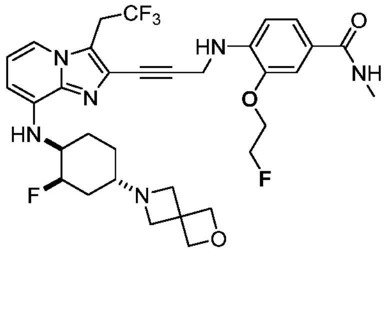
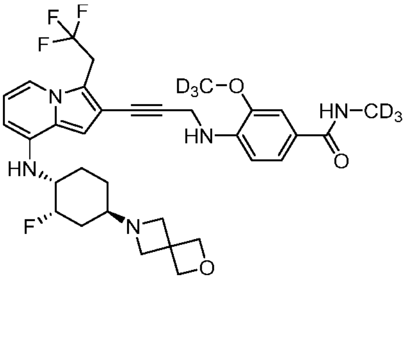

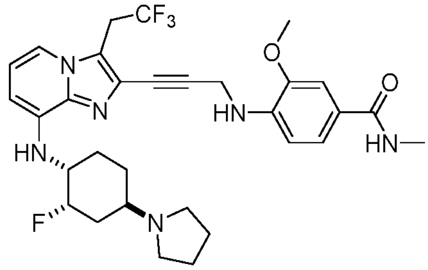
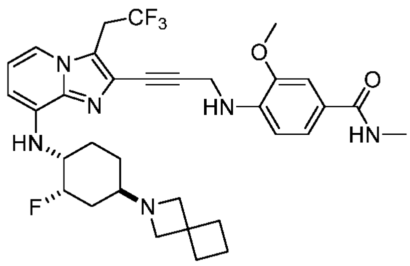
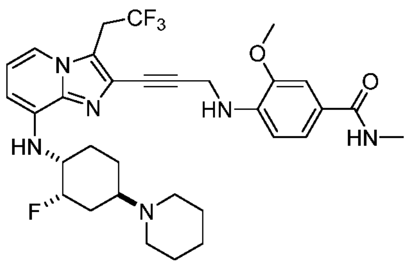
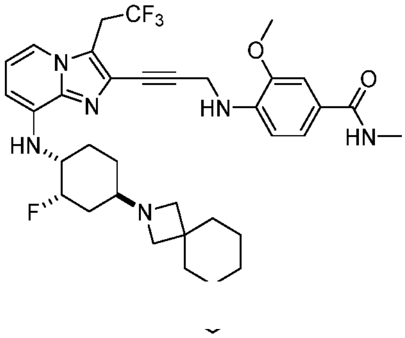
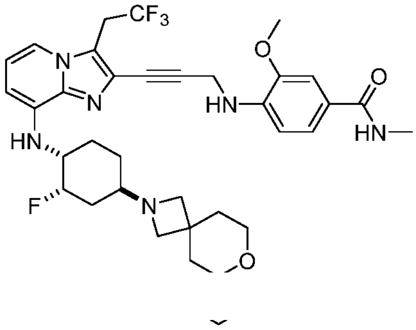
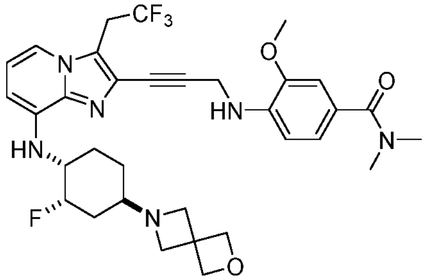
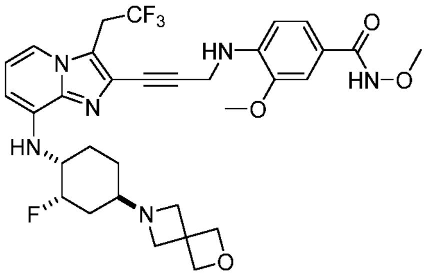
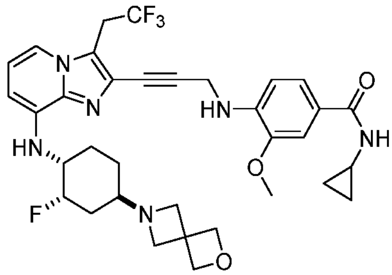
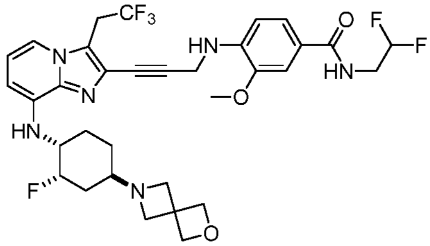
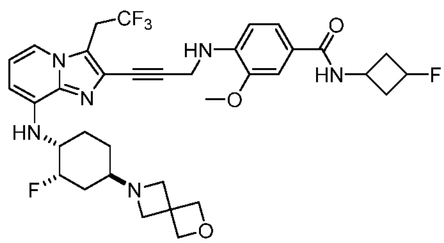
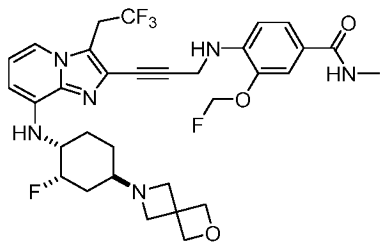
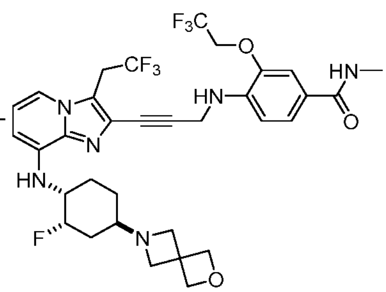
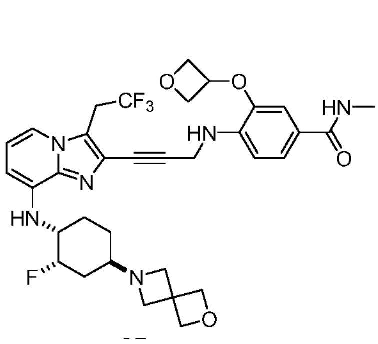
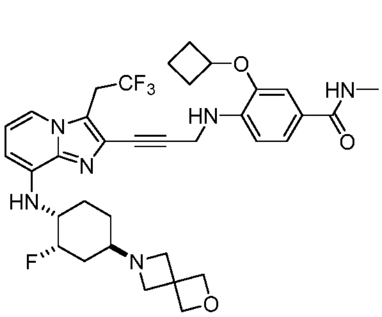
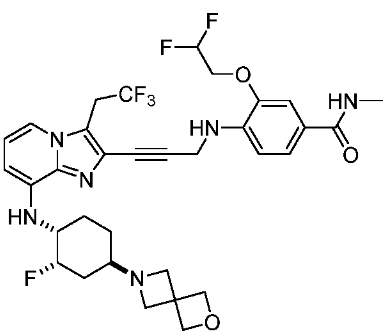

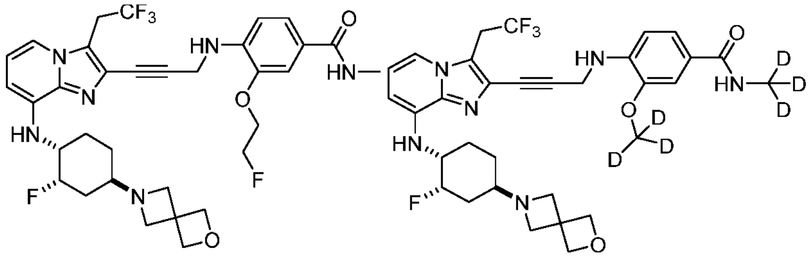
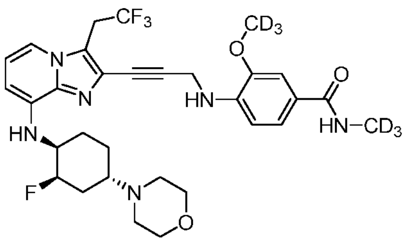

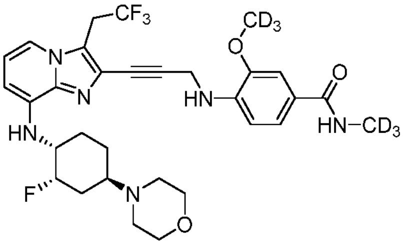
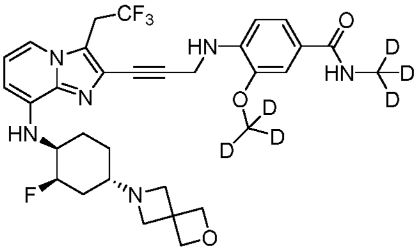
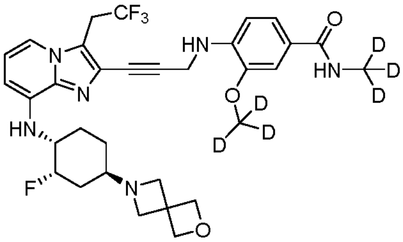

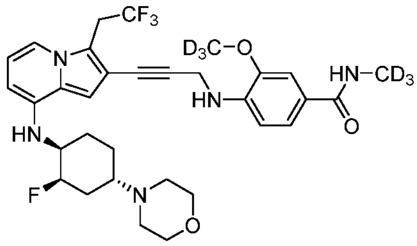
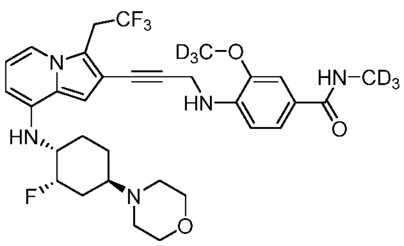
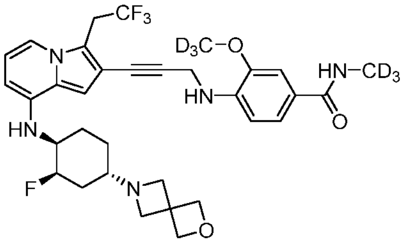

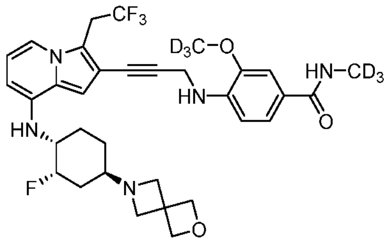
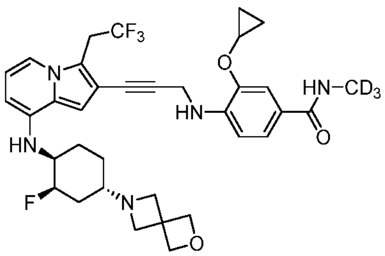
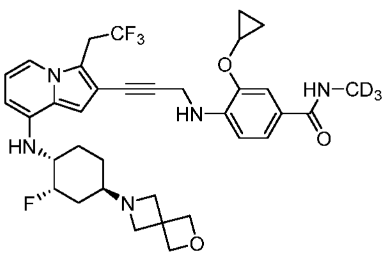

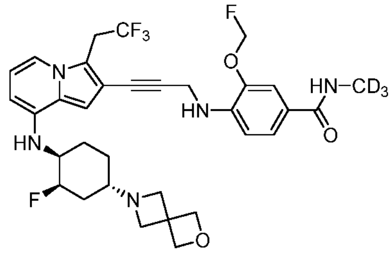
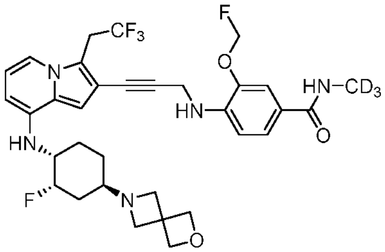
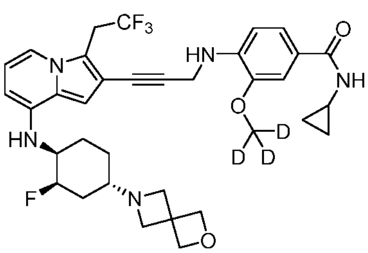

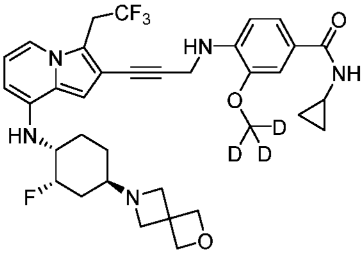
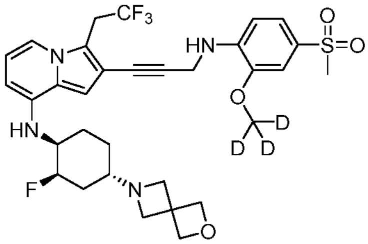
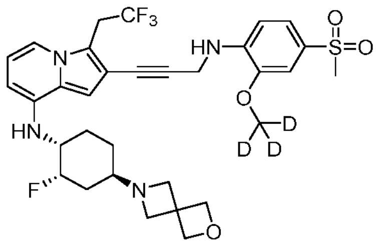

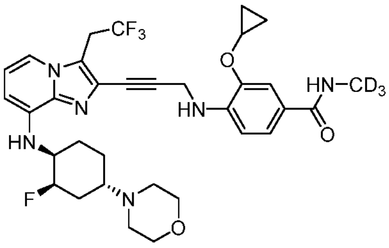 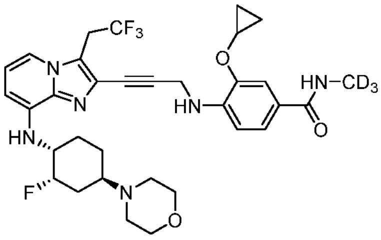 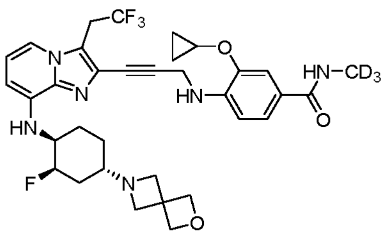

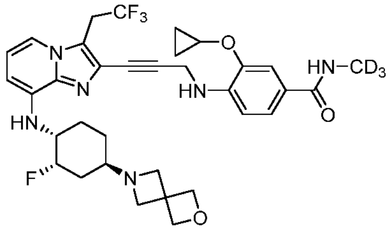.

**In** the nineteenth aspect of the present invention, the present invention provides a pharmaceutical composition, which comprises the aforementioned compound, or an enantiomer, diastereomer, racemate, tautomer, stereoisomer, geometric isomer, crystal form, N-oxide, metabolite of the compound, or a pharmaceutically acceptable salt, ester, solvate, hydrate, isotope-labeled (preferably deuterated) compound or prodrug thereof, and optionally a pharmaceutically acceptable excipient.

**In** the twentieth aspect of the present invention, the present invention provides a combination drug, which comprises:

1) a first drug, wherein the first drug is the aforementioned compound, or an enantiomer, diastereomer, racemate, tautomer, stereoisomer, geometric isomer, crystal form, N-oxide, metabolite of the compound, or a pharmaceutically acceptable salt, ester, solvate, hydrate, isotope-labeled (preferably deuterated) compound or prodrug thereof;
2) a second drug, wherein the second drug is a PD-1 inhibitor or a PD-L1 inhibitor.

In some embodiments, the PD-1 inhibitor is selected from the group consisting of nivolumab, pembrolizumab, cemiplimab, tislelizumab, camrelizumab, sintilimab, toripalimab, penpulimab, zimberelimab, and pucotenlimab.

In some embodiments, the PD-L1 inhibitor is selected from the group consisting of atezolizumab, avelumab, and durvalumab.

In the twenty-first aspect of the present invention, the present invention provides a use of the aforementioned compound, or an enantiomer, diastereomer, racemate, tautomer, stereoisomer, geometric isomer, crystal form, N-oxide, metabolite of the compound, or a pharmaceutically acceptable salt, ester, solvate, hydrate, isotope-labeled (preferably deuterated) compound or prodrug thereof, or the aforementioned pharmaceutical composition, or the aforementioned combination drug in the manufacture of a medicament.

In some embodiments, the medicament is used for the treatment and/or prevention of a disease.

In some embodiments, the disease is a disease caused by p53 Y220C mutation.

In some embodiments, the disease caused by p53 Y220C mutation is a cancer.

In some embodiments, the cancer is selected from the group consisting of gastric adenocarcinoma, pancreatic cancer, breast cancer, liver cancer, prostate cancer, cervical cancer, ovarian cancer, oral cancer, esophageal cancer, gastric cancer, colorectal cancer, nasopharyngeal cancer, lung cancer (e.g., non-small cell lung cancer, small cell lung cancer), bladder cancer, soft tissue sarcoma, brain tumor, lymphocyte tumor, osteogenic sarcoma, endometrial cancer, and head and neck cancer.

In some embodiments, the disease is selected from the group consisting of gastric adenocarcinoma, pancreatic cancer, prostate cancer, ovarian cancer, breast cancer, endometrial cancer, head and neck cancer, and small cell lung cancer.

In the twenty-second aspect of the present invention, the present invention provides the aforementioned compound, or an enantiomer, diastereomer, racemate, tautomer, stereoisomer, geometric isomer, crystal form, N-oxide, metabolite of the compound or a pharmaceutically acceptable salt, ester, solvate, hydrate, isotope-labeled (preferably deuterated) compound or prodrug thereof, or the aforementioned pharmaceutical composition, or the aforementioned combination drug, for use in the treatment and/or prevention of a disease.

In some embodiments, the disease is a disease caused by p53 Y220C mutation.

In some embodiments, the disease caused by p53 Y220C mutation is a cancer.

In some embodiments, the cancer is selected from the group consisting of gastric adenocarcinoma, pancreatic cancer, breast cancer, liver cancer, prostate cancer, cervical cancer, ovarian cancer, oral cancer, esophageal cancer, gastric cancer, colorectal cancer, nasopharyngeal cancer, lung cancer (e.g., non-small cell lung cancer, small cell lung cancer), bladder cancer, soft tissue sarcoma, brain tumor, lymphocyte tumor, osteogenic sarcoma, endometrial cancer, and head and neck cancer.
In some embodiments, the disease is selected from the group consisting of gastric adenocarcinoma, pancreatic cancer, prostate cancer, ovarian cancer, breast cancer, endometrial cancer, head and neck cancer, and small cell lung cancer.
In some embodiments, the first drug and the second drug in the combination drug are administered simultaneously, or are administered sequentially.
In the twenty-third aspect of the present invention, the present invention provides a method for treating and/or preventing a disease, comprising: administering to a subject an effective amount of the aforementioned compound, or an enantiomer, diastereomer, racemate, tautomer, stereoisomer, geometric isomer, crystal form, N-oxide, metabolite of the compound or a pharmaceutically acceptable salt, ester, solvate, hydrate, isotope-labeled (preferably deuterated) compound or prodrug thereof, or the aforementioned pharmaceutical composition, or the aforementioned combination drug, wherein the disease is a disease caused by p53 Y220C mutation.
In some embodiments, the disease is selected from the group consisting of gastric adenocarcinoma, pancreatic cancer, breast cancer, liver cancer, prostate cancer, cervical cancer, ovarian cancer, oral cancer, esophageal cancer, gastric cancer, colorectal cancer, nasopharyngeal carcinoma, lung cancer (e.g., non-small cell lung cancer, small cell lung cancer), bladder cancer, soft tissue sarcoma, brain tumor, lymphocytic tumor, osteosarcoma, endometrial cancer, and head and neck cancer.
In some embodiments, the disease is selected from the group consisting of gastric adenocarcinoma, pancreatic cancer, prostate cancer, ovarian cancer, breast cancer, endometrial cancer, head and neck cancer, and small cell lung cancer.
In some embodiments, when the aforementioned combination drug is administered, the first drug and the second drug are administered simultaneously, or are administered sequentially.
In the twenty-fourth aspect of the present invention, the present invention provides a method for inducing apoptosis of a cell, the method comprising contacting the cell with a therapeutically effective amount of a compound capable of binding to a mutant p53 mutant, wherein the compound is the aforementioned compound, or an enantiomer, diastereomer, racemate, tautomer, stereoisomer, geometric isomer, crystalline form, N-oxide, metabolite of the compound, or a pharmaceutically acceptable salt, ester, solvate, hydrate, isotope-labeled (preferably deuterated) compound or prodrug thereof, or the aforementioned pharmaceutical composition, or the aforementioned combination drug.
In some embodiments, the compound increases the ability of the p53 mutant to bind to DNA.
In some embodiments, the p53 mutant is p53 Y220C.
In some embodiments, the compound induces a conformational change in the p53 mutant.
In some embodiments, the compound increases the stability of the biologically active conformation of the p53 mutant relative to the stability of the biologically active conformation of the p53 mutant in the absence of the compound.

Beneficial effects

1. The compounds of the present invention have excellent in vitro activity and can increase the ability of the Y220C mutant P53 protein to bind to DNA.
2. The compounds of the present invention can significantly inhibit the proliferation of NUGC-3 gastric adenocarcinoma cells containing the p53 Y220C mutation.
3. The compounds of the present invention can significantly inhibit the proliferation of BxPC-3 pancreatic cancer cells containing the p53 Y220C mutation.

## Specific Models for Carrying Out the Invention

It should be understood that the terms used herein are intended to describe specific embodiments and are not intended to be limiting. In addition, although any methods, devices and materials similar or equivalent to those described herein can be used to implement or test the present invention, the preferred methods, devices and materials are now described.
In the present invention, unless otherwise explicitly stated, the description method used throughout herein "... independently selected from" can mean that the specific options expressed by the same or different symbols in different groups do not affect each other, and can also mean that the specific options expressed by the same or different symbols in

the same group do not affect each other.

The term "optional", "optionally" or "arbitrarily" means that the event or situation described subsequently may but need not occur, and the description includes instances where the event or situation occurs and instances where it does not occur. For example, "optionally substituted with ..." means that the substitution may or may not be present.

When the term "each independently" is used in combination with "optionally", for example, "each independently optionally substituted with ..." means that the specific options are either substituted with or not substituted with ... independently of each other.

Unless otherwise indicated, the following definitions used herein shall apply. For the purposes of the present invention, the chemical elements are consistent with the Periodic Table of the Elements, CAS version, and Handbook of Chemistry and Physics, 75th edition, 1994. In addition, general principles of organic chemistry can be found in "Organic Chemistry", Thomas Sorrell, University Science Books, Sausalito: 1999, and "March's Advanced Organic Chemistry" by Michael B. Smith and Jerry March, John Wiley & Sons, New York:2007, and the entire contents of which are incorporated herein by reference.

The substituents of the compounds of the present invention are disclosed in terms of group types or ranges. It is particularly noted that the present invention comprises each independent secondary combination of the individual members of these group types and ranges. For example, the term "C1-C6 alkyl" specifically refers to independently disclosed methyl, ethyl, C3 alkyl, C4 alkyl, C5 alkyl and C6 alkyl.

The term "alkyl" refers to a branched and straight-chain saturated aliphatic hydrocarbon group with a specified number of carbon atoms. For example, "C1-C6 alkyl" refers to an alkyl with 1 to 6 carbon atoms, preferably "C1-C4 alkyl", more preferably "C1-C3 alkyl", and most preferably "C1-C2 alkyl". Examples of "C1-C6 alkyl" include, but are not limited to, methyl, ethyl, propyl (e.g., n-propyl, isopropyl), butyl (e.g., n-butyl, isobutyl, tert-butyl), pentyl (e.g., n-pentyl, isopentyl, neopentyl), etc. Examples of "C1-C4 alkyl" include, but are not limited to methyl, ethyl, propyl (e.g., n-propyl, isopropyl), butyl (e.g., n-butyl, isobutyl, tert-butyl), etc. Examples of "C1-C3 alkyl" include methyl, ethyl, propyl (e.g., n-propyl, isopropyl), etc. Examples of "C1-C2 alkyl" include methyl, ethyl.

The term "alkoxy" refers to any of the above alkyl groups (e.g., C1-C6 alkyl, C1-C4 alkyl, C1-C3 alkyl, C1-C2 alkyl, etc.) that is connected to the rest moiety of molecule via an oxygen atom -O-, and its examples include C1-C6 alkoxy, C1-C4 alkoxy, C1-C3 alkoxy, specifically methoxy, ethoxy, etc.

The term "cycloalkyl" refers to a monovalent ring of saturated hydrocarbon containing 3-15 ring carbon atoms or 3-10 ring carbon atoms, for example, it can be "C3-C7 cycloalkyl", "C4-C9 cycloalkyl", "C3-C6 cycloalkyl", and the cycloalkyl can be in the form of a single ring, a condensed ring, a bridged ring, or a spiro ring. Exemplary cycloalkyl examples include, but are not limited to, the following moieties:

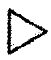 , 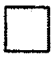 , 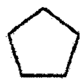 , 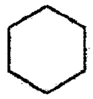 , 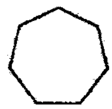 , 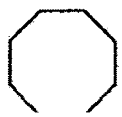 , 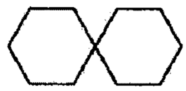 ,

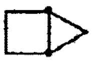 , 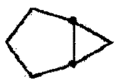 , 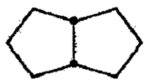 , 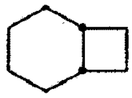 , 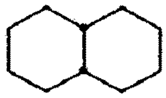 , 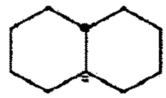 ,

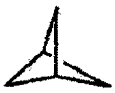 , 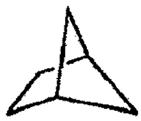 , 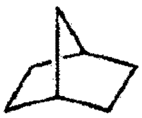 , 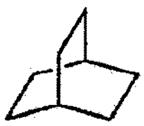 , 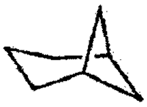 , 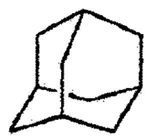 ,

Examples of the term "C3-C6 cycloalkyl" include,

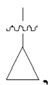 , 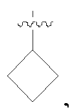 , 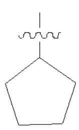 or 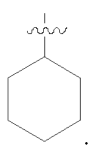 .

Halogen refers to fluorine, chlorine, bromine or iodine.

The term "C1-C6 haloalkyl" refers to a group obtained by replacing one or more hydrogen atoms in any of the

above alkyl groups (e.g., C1-C6 alkyl, C1-C4 alkyl, C1-C3 alkyl, C1-C2 alkyl, etc.) with a halogen (preferably fluorine), for example, monofluoromethyl, difluoromethyl, difluoroethyl, trifluoromethyl,

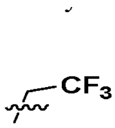 , 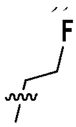 , 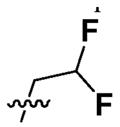 ,

etc.

The term "halogenated cycloalkyl" refers to a group obtained by replacing one or more hydrogen atoms in any of the above cycloalkyl groups (e.g., C3-C7 cycloalkyl, C4-C9 cycloalkyl, C3-C6 cycloalkyl, etc.) with a halogen (preferably fluorine), for example, it can be a C3-C6 halocycloalkyl.

Heteroatom refers to N, O, S or P.

The terms "heteroaryl" and "heteroaromatic ring" are used interchangeably and refer to a substituted and unsubstituted aromatic 5-membered or 6-membered monocyclic group, 8-membered, 9-membered or 10-membered bicyclic group, or 11-membered to 14-membered tricyclic group, which has at least one heteroatom (N, O, S, or P) in at least one ring and optionally further has 1, 2 or 3 heteroatoms selected from the group consisting of N, O, S and P in the heteroatom-containing ring. Among them, the substituted and unsubstituted aromatic 8-membered, 9-membered or 10-membered bicyclic group and 11-membered to 14-membered tricyclic group having at least one heteroatom (N, O, S or P) in at least one ring are "fused heteroaryl" or "fused heteroaromatic ring". Bicyclic or tricyclic heteroaryl or heteroaromatic ring requires a bicyclic or tricyclic overall structure to form an aromatic system. The heteroaryl or heteroaromatic ring may be attached at any available nitrogen or carbon atom of any ring. And those skilled in the art can understand that two adjacent atoms (preferably carbon atoms) are shared between every two rings in the fused ring.

Exemplary monocyclic heteroaryl or monocyclic heteroaromatic ring includes, but is not limited to: pyrrolyl/pyrrole ring, pyrazolyl/pyrazole ring, imidazolyl/imidazole ring, oxazolyl/oxazole ring, isoxazolyl/isoxazole ring, thiazolyl/thiazole ring, thiadiazolyl/thiadiazole ring, isothiazolyl/isothiazole ring, furanyl/furane ring, thienyl/thiophene ring, oxadiazolyl/oxadiazole ring, pyridyl/pyridine ring, pyrazinyl/pyrazine ring, pyrimidinyl/pyrimidine ring, pyridazinyl/pyridazine ring, triazinyl/triazine ring, triazolyl/triazole ring, pyridazinyl/pyridazine ring, 2-pyridone, etc.

Exemplary bicyclic heteroaryl includes, but is not limited to, indolyl, 5-azaindolyl, pyrrolo[2,3-d]pyrimidinyl, 5,6-diazaindolyl, 6-azaindolyl, 7-azaindolyl, pyrazolo[3,4-b]pyridinyl, pyrrolo[2,3-c]pyridazinyl, thieno[2,3-d]imidazolyl, thieno [2,3-d]imidazolyl, pyrazolo[3,4-c]pyridinyl, benzothiazolyl, benzimidazolyl, benzoxazolyl, benzothienyl, quinolyl, isoquinolyl, benzofuranyl, indolizinyl, quinoxalinyl, indazolyl, pyrrolopyrimidinyl, furopyridinyl, isoindolyl, and the like.

The terms "heteroarylene" and "heteroarylene ring" are used interchangeably and refer to a divalent group obtained by losing one hydrogen atom of any of the above heteroaryl or losing two hydrogen atoms of the above heteroaromatic ring. For example, 5- to 6-membered heteroarylene can be 1,3,4-thiadiazolylene, 1,2,4-oxadiazolylene, 1,3,4-oxadiazolylene or thiazolylene, etc., and specifically can be

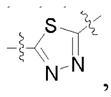 , 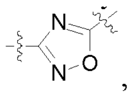 , 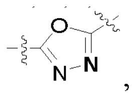 , 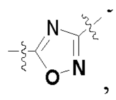 , 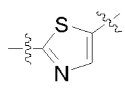 or

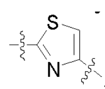 ,

etc.

The terms "heterocyclic ring", "heterocyclic" or "heterocyclyl" are used interchangeably and refer to a substituted and unsubstituted 3- to 7-membered (preferably 4- to 7-membered, more preferably 5- to 6-membered or 4- to 6-membered) monocyclic group, 7- to 11-membered bicyclic group or a 10- to 15-membered tricyclic group, which may contain one or more double bonds but do not constitute an aromatic ring; wherein at least one ring has at least one heteroatom (N, O, S or P). The fused, bridged or spiro rings that constitute bicyclic and tricyclic groups may contain only carbon atoms and may be saturated or partially saturated and do not constitute an aromatic ring. The heterocyclyl may be attached at any available nitrogen or carbon atom. Exemplary heterocyclic rings include, but are not limited to, the following moieties:

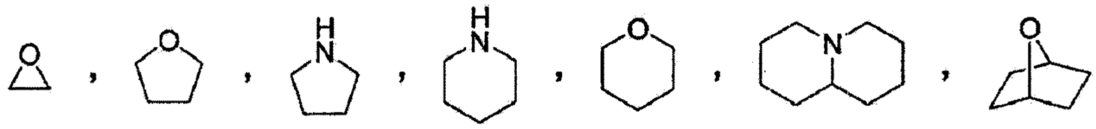
, , , , , ,

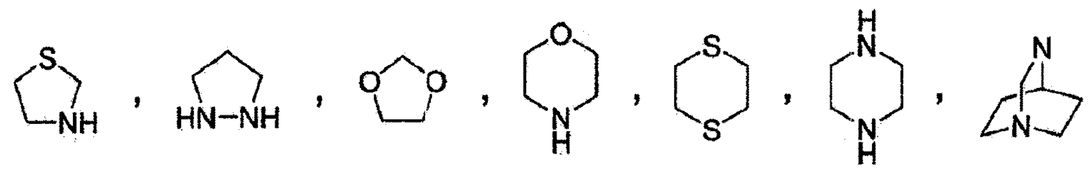
, , , , , ,

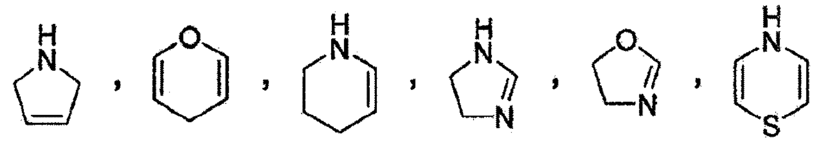
, , , , ,

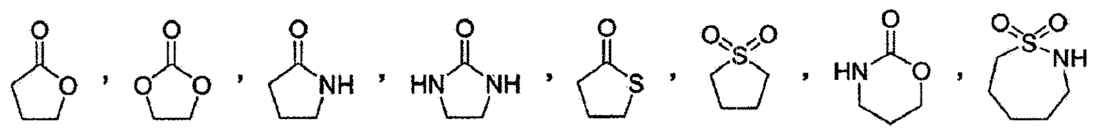
, , , , , , ,

Exemplary monocyclic heterocyclyl groups include azetidinyl, oxetanyl, pyrrolidinyl, imidazolinyl, oxazolidinyl, isoxazolinyl, thiazolidinyl, tetrahydrofuranyl, piperidinyl, piperazinyl, 2-oxopiperazinyl, 2-oxopiperidinyl, 2-oxopyrrolidinyl, 2-oxazacycloheptatrienyl, 1-pyridonyl, 4-piperidonyl, tetrahydropyranyl, morpholinyl, 1,3-dioxolanyl, and the like.

The terms "saturated heterocyclyl" and "saturated heterocyclic ring" are used interchangeably and refer to the heterocyclyl defined above that does not contain unsaturated bonds, such as double bonds. For example, examples of "4- to 8-membered saturated heterocyclic rings" include, but are not limited to, hexahydropyrimidine, hexahydropyrazine,

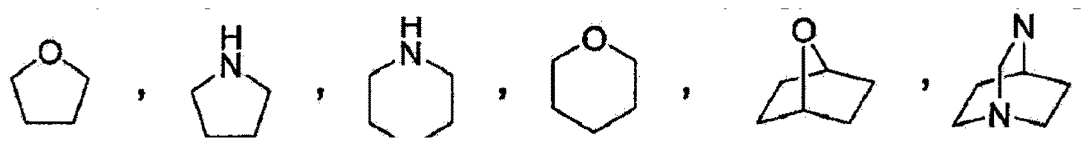
, , , , ,

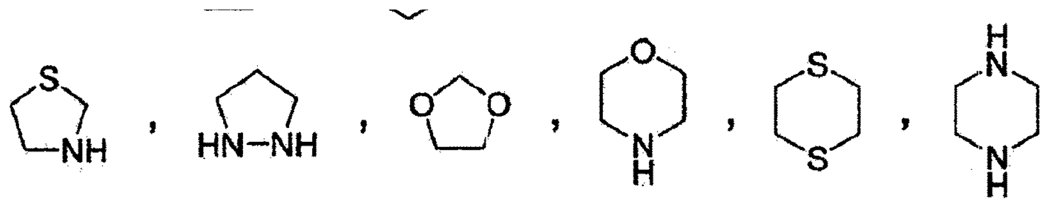
, , , , , .

For example, examples of "6-membered saturated heterocyclyl" includes, but is not limited to, morpholinyl, piperazinyl, piperidinyl, tetrahydropyranyl, hexahydropyrimidinyl, hexahydropyrazinyl, specifically,

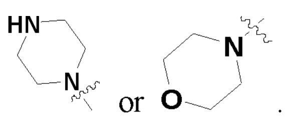
or .

The terms "bridged heterocyclyl" and "bridged heterocyclic ring" are used interchangeably and refer to a polycyclic hydrocarbon in which two or more carbon atoms are shared between several 3- to 7-membered monocyclic rings, the ring atoms contain at least one heteroatom, and one or more double bonds may be contained, but none of the rings has a conjugated $\pi$ electron system; and specific examples may include "7-membered bridged heterocyclyl". Therein, "member" refers to the total number of ring atoms of the bridged heterocyclyl. In addition, according to the number of rings, it can be classified into diheterocyclic hydrocarbons, triheterocyclic hydrocarbons, tetraheterocyclic hydrocarbons, and the like. Preferred heteroatoms include N, O, S or P, more preferably N. Therein, "7-membered bridged heterocyclyl" can be, for example,

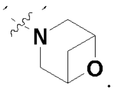

The terms "spiro-heterocyclyl" and "spiro-heterocyclic ring" are used interchangeably and refer to a polycyclic hydrocarbon in which two 3- to 7-membered monocyclic rings share one carbon atom, the ring atoms contain at least one heteroatom, and one or more double bonds may be contained, but none of the rings has a conjugated π electron system; and specific examples may include "7- to 11-membered spiro-heterocyclic ring" and "7- to 9-membered spiro-heterocyclic ring". Preferred heteroatoms include N, O, S or P.

Therein, "7- to 11-membered spiro-heterocyclic ring" can be, for example:

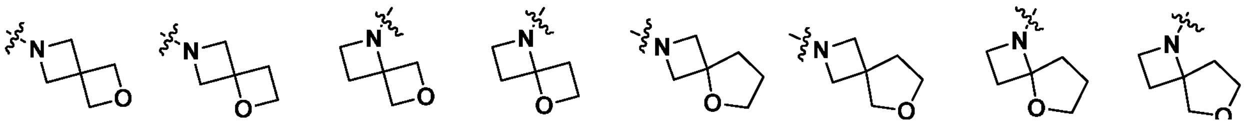

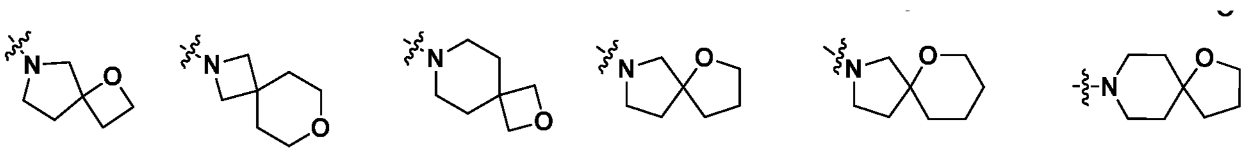

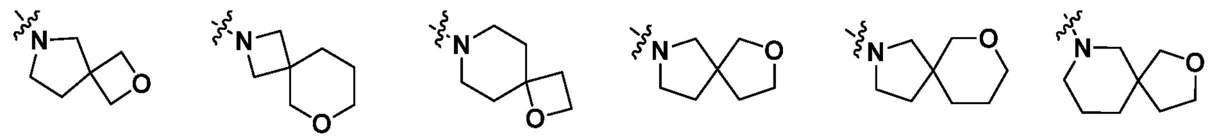

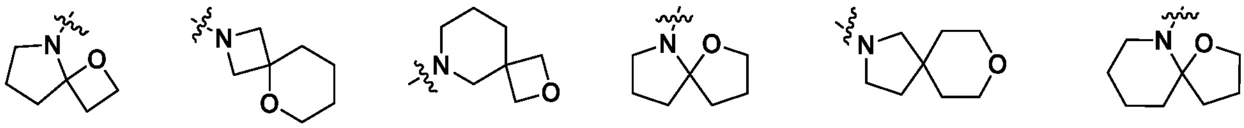

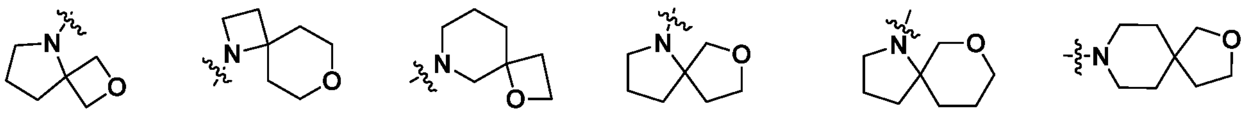

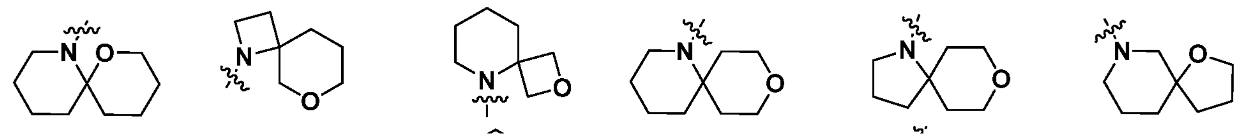

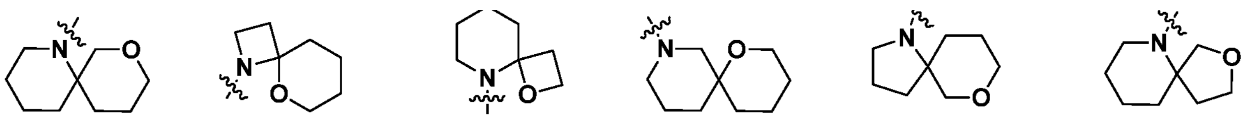

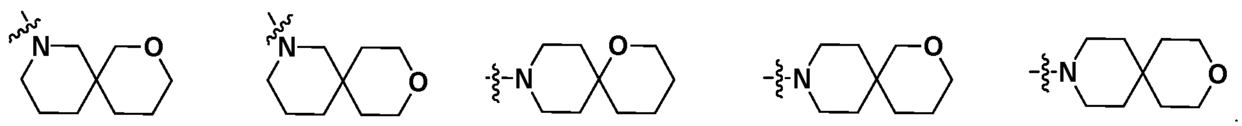

Specifically, "7- to 9-membered spiro-heterocyclic ring" can preferably be

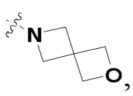

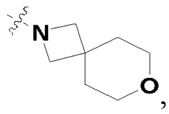, 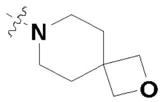.

From all the above descriptions, it is obvious to those skilled in the art that any group whose name is a composite name, such as "cyano-C1-C6 alkylene", should refer to that it is constituted conventionally with moieties derived therefrom from left to right, for example, from "C1-C6 alkylene" substituted with cyano, wherein "C1-C6 alkylene" is as defined above. Specifically, "cyano-C1-C6 alkylene" can be, for example

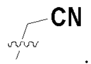.

Similarly, "hydroxy-C1-C6 alkylene", "C1-C6 alkoxy-C1-C6 alkylene", "5- to 6-membered saturated heterocyclyl-C1-C6 alkylene" should refer to that it is constituted conventionally with moieties derived therefrom from left to right, for example, from "C1-C6 alkylene" substituted with hydroxyl, C1-C6 alkoxy or 5- to 6-membered saturated heterocyclyl, wherein "C1-C6 alkylene" is as defined above. Specifically, "hvdroxv-C1-C6 alkylene" can be, for example

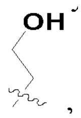,

"C1-C6 alkoxy-C1-C6 alkylene" can be, for example

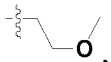,

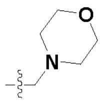.

"5- to 6-membered saturated heterocyclyl-C1-C6 alkylene" can be, for example

Other similar composite groups can be understood with reference to the above content.

The term "substituted" or "substituted with a substituent ..." means that any one or more hydrogen atoms on a specified atom or group are replaced by a specified group, provided that the normal valence of the specified atom is not exceeded. For example, "hydroxyl-substituted C1-C6 alkyl" can be hydroxymethyl. For another example, "C1-C6 alkyl substituted with 2 hydroxyl groups" can be

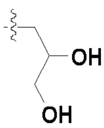.

For another example, "$R^7$ is selected from the group consisting of C1-C6 alkoxv-C1-C6 alkylene, wherein the C1-C6 alkoxv-C1-C6 alkylene is substituted with hydroxyl", then $R^7$ can be

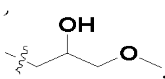,

for example.

In the present invention, when $R^2$ is $R^eR^fN$-C(=O)-, and $R^e$, $R^f$ together with the N atom to which they connect form a 5- to 6-membered saturated heterocyclic ring or a 7-membered spiro-heterocyclic ring, if the 5- to 6-membered saturated heterocyclic ring is

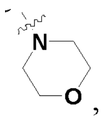

and the 7-membered spiro-heterocyclic ring is

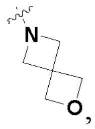

then it means that $R^2$ can be

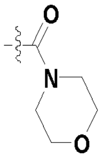 or 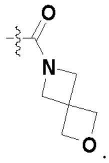

Other similar definitions can be understood with reference to the above content.

In the present invention, "treatment" generally refers to obtaining the desired pharmacological and/or physiological effect. The effect can be preventive based on the complete or partial prevention of a disease or symptoms thereof; and/or can be therapeutic based on partial or complete stabilization or cure of a disease and/or side effects caused by the disease. "Treatment" as used herein covers any treatment on patient's disease, including: (a) preventing a disease or symptom in a patient who is susceptible to the disease or symptoms but has not yet been diagnosed with the disease; (b) inhibiting a symptom of a disease, i.e., preventing its development; or (c) alleviating a symptom of a disease, i.e., causing the disease or symptom to degenerate.

In the present invention, "subject" refers to a vertebrate. In certain embodiments, the vertebrate refers to a mammal. The mammal includes, but is not limited to, livestock (e.g., cattle), pet (e.g., cat, dog, and horse), primate, mouse, and rat. In certain embodiments, the mammal refers to a human.

In the present invention, "effective amount" refers to an amount that is effective at the required dose and time to achieve the desired therapeutic or preventive effect. The "therapeutically effective amount" of the substance/molecule of the present invention may vary according to factors such as the disease state, age, sex, and weight of individual and the ability of the substance/molecule to elicit the desired response in the individual. The therapeutically effective amount also encompasses an amount in which the therapeutically beneficial effect of the substance/molecule outweighs any toxic or harmful consequence. "Prophylactically effective amount" refers to an amount that is effective at the required dose and time to achieve the desired preventive effect. Usually, but not necessarily, since the preventive dose is administered to the subject before the onset of the disease or in the early stages of the disease, the preventive effective amount will be lower than the therapeutically effective amount. In the case of cancer, a therapeutically effective amount of the drug can reduce the number of cancer cells; reduce the size of tumor; inhibit (i.e., slow down to a certain extent, preferably stop) the infiltration of cancer cells into surrounding organs; inhibit (i.e., slow down to a certain extent, preferably stop) tumor metastasis; inhibit tumor growth to a certain extent; and/or alleviate one or more symptoms associated with cancer to a certain extent.

The pharmaceutical composition involved in the present invention may contain a pharmaceutically acceptable excipient, the excipient includes but is not limited to: ion exchanger, aluminum oxide, aluminum stearate, lecithin, serum protein such as human albumin, buffer substance such as phosphate, glycerol, sorbic acid, potassium sorbate, mixture of partial glycerides of saturated vegetable fatty acids, water, salt or electrolyte, such as protamine sulfate, disodium hydride phosphate, potassium hydrogen phosphate, sodium chloride, zinc salt, colloidal silica, magnesium trisilicate, polyvinylpyrrolidone, cellulose substance, polyethylene glycol, sodium carboxymethyl cellulose, polyacrylate, beeswax, lanolin, etc.

The pharmaceutical composition of the present invention can be prepared in various forms according to different administration routes. For example, the pharmaceutical composition can be administered by any of the following ways: oral, spray inhalation, rectal, nasal, buccal, vaginal, topical, parenteral administration, such as subcutaneous, intravenous, intramuscular, intraperitoneal, intrathecal, intraventricular, intrasternal and intracranial injection or infusion, or by means of an explanted reservoir. Oral or intravenous administration is preferred.

The compound of the present invention may optionally be used in combination with one or more other active ingredients, and their respective dosages and proportions may be adjusted by those skilled in the art according to specific

symptoms and patient conditions and clinical needs.

As used herein, unless otherwise specified, the term "prodrug" refers to a derivative that can be hydrolyzed, oxidized or otherwise reacted under biological conditions (in vitro or in vivo) to provide the compound of the present invention. Prodrugs become active compounds only through this reaction under biological conditions, or they have no or only low activity in their unreactive forms. Prodrugs can generally be prepared using well-known methods, such as those described in Burger's Medicinal Chemistry and Drug Discovery (1995) 172-178, 949-982 (Manfred E. Wolff, ed., 5th ed.).

The term "enantiomers" refers to two non-superimposable isomers of a compound that are mirror images of each other.

The term "diastereomers" refers to stereoisomers that have two or more chiral centers and whose molecules are not mirror images of each other. Diastereomers have different physical properties, such as melting points, boiling points, spectral properties and reactivity. Diastereomeric mixtures can be separated by high-resolution analytical procedures such as electrophoresis and chromatography, such as HPLC.

The term "racemate", "raceme" or "racemic mixture" refers to an equimolar mixture of two enantiomers that lacks optical activity.

The term "tautomers" or "tautomeric forms" refers to structural isomers with different energies that can be converted into each other through a low energy barrier. If tautomerism is possible (e.g., in solution), a chemical equilibrium of tautomers can be achieved. For example, proton tautomers (also called prototropic tautomers) involve in interconversion that occurs by proton migration, such as keto-enol isomerization and imine-enamine isomerization. Valence tautomers involve in interconversion that occurs by reconfiguration of some of the bonding electrons. A specific example of keto-enol tautomerism is the interconversion of pentane-2,4-dione and 4-hydroxypent-3-en-2-one tautomers. Another example of tautomerism is phenol-keto tautomerism. A specific example of phenol-keto tautomerism is the interconversion of pyridin-4-ol and pyridin-4(1H)-one tautomers. Unless otherwise indicated, all tautomeric forms of the compound of the present invention are within the scope of the present invention.

The term "stereoisomers" refers to compounds that have the same chemical constitution, but differ in the way the atoms or groups are arranged in space. Stereoisomers include enantiomers, diastereomers, conformers (rotamers), geometric isomers (cis/trans isomers), atropisomers, and the like. The term "geometric isomer" is also called "cis-trans isomer", which is an isomer caused by double bond (including olefin double bond, C=N double bond, and N=N double bond) or a single bond of ring carbon atom that cannot rotate freely, wherein, taking

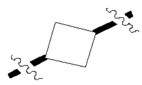

as an example, it represents the cis configuration, that is, the two substituents on the cyclobutyl ring are located on the same side.

As used herein, from the perspective of a single chiral carbon atom, the connecting chains " 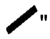 " and " 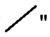 " on the carbon atom have the same meaning, that is, " 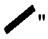 " and " 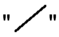 " both indicate that "R" configuration and "S" configuration are included.

The stereochemical definitions and rules used in the present invention generally follow those described by S. P. Parker, Ed., McGraw-Hill Dictionary of Chemical Terms (1984) McGraw-Hill Book Company, New York; and Eliel, E. and Wilen, S, "Stereochemistry of Organic Compounds", John Wiley & Sons, Inc, New York, 1994. Many organic compounds exist in optically active forms, that is, they have the ability to rotate the plane of plane polarized light. When describing optically active compounds, the prefixes D and L or R and S are used to indicate the absolute configuration of the molecule about its one or more chiral centers. The prefixes d and l or (+) and (-) are the symbols used to specify the rotation of plane polarized light caused by the specific compound, wherein (-) or l indicates that the compound is left-handed, and the prefix (+) or d indicates that the compound is right-handed. A specific stereoisomer is an enantiomer, and a mixture of such isomers is called an enantiomeric mixture. A mixture of enantiomers at ratio of 50:50 is called a racemic mixture or racemate, which can occur when there is no stereoselectivity or stereospecificity in a chemical reaction or process.

Any asymmetric atom (e.g., carbon, etc.) of the compound disclosed herein can exist in a racemic or enantiomerically enriched form, such as in form of (R)-, (S)-, or (R,S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess, in terms of (R)- or (S)-configuration.

Depending on the choice of starting materials and process, the compound of the present invention can exist in the form of one of its possible isomers or a mixture thereof, such as a racemate and a diastereomeric mixture (depending on the number of asymmetric carbon atoms). Optically active (R)- or (S)-isomer may be prepared using a chiral synthon or chiral reagent, or resolved using conventional techniques. If the compound contains a double bond, the substituents may

be in the E or Z configuration; if the compound contains a disubstituted cycloalkyl, the cycloalkyl substituents may be in the cis or trans configuration.

Any mixture of stereoisomers obtained may be separated into pure or substantially pure geometric isomers, enantiomers, diastereomers, on the basis of differences in the physicochemical properties of components, for example, by chromatography and/or fractional crystallization.

Any racemate of the resulting final product or intermediate may be resolved into the optical enantiomers by methods familiar to those skilled in the art, for example, by separation of the diastereomeric salts thereof as obtained. The racemic product may also be separated by chiral chromatography, for example, by high performance liquid chromatography (HPLC) using a chiral adsorbent. In particular, enantiomers can be prepared by asymmetric synthesis, for example, by referring to Jacques, et al., Enantiomers, Racemates and Resolutions (Wiley Interscience, New York, 1981); Principles of Asymmetric Synthesis (2nd Ed. Robert E. Gawley, Jeffrey Aube, Elsevier, Oxford, UK, 2012); Eliel, E. L. Stereochemistry of Carbon Compounds (McGraw-Hill, NY, 1962); Wilen, S. H. Tables of Resolving Agents and Optical Resolutions p. 268 (E. L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The compounds described herein include all possible isotopically labeled compounds of all compounds described herein. The term "isotopically labeled compound" refers to a compound obtained by replacing any atom in the compound with its isotope atom.

"Solvant" or "solvate" can be used interchangeably and refers to a compound that exists in combination with a certain solvent molecule. The combination may contain a stoichiometric amount of a certain solvent, for example, when the solvent is water, a "hydrate" such as monohydrate or dihydrate is formed, or any amount of water may be contained; for example, when the solvent is an alcohol such as methanol or ethanol, an "alcoholate" may be formed, which may also be stoichiometric or non-stoichiometric. The term "solvate" used herein refers to a solid form, that is, a compound in a solution of a solvent is not a solvate as described by the term used herein although it may be solvated.

The term "hydrate" refers to an associated matter formed by one or more water molecules and the compound of the present invention.

The term "N-oxide" refers to that when a compound contains several amino groups, one or more nitrogen atoms may be oxidized to form an N-oxide. Specific examples of N-oxides are N-oxides of tertiary amines or N-oxides of nitrogen atoms of nitrogen-containing heterocyclic rings. The corresponding amines can be treated with oxidants such as hydrogen peroxide or peracids (e.g., peroxycarboxylic acids) to form N-oxides (see, Advanced Organic Chemistry, Wiley Interscience, 4th edition, Jerry March, pages). In particular, N-oxides can be prepared by the method of L.W. Deady (Syn. Comm. 1977, 7, 509-514), wherein, for example, the amine compound is reacted with meta-chloroperbenzoic acid (MCPBA) in an inert solvent such as dichloromethane.

As used herein, the term "metabolite" refers to a derivative of a compound formed when the compound is metabolized. The term "metabolism" refers to the sum of processes (including, but not limited to, hydrolysis reactions and enzyme-catalyzed reactions) by which a particular substance is changed by an organism.

As used herein, the term "ester" refers to an ester formed by -COOH present in the compound provided by the present invention and a suitable alcohol, or an ester formed by -OH present in the compound provided by the present invention and a suitable acid (e.g., a carboxylic acid or an oxygen-containing inorganic acid). Suitable ester groups include, but are not limited to, formate, acetate, propionate, butyrate, acrylate, ethylsuccinate, stearate or palmitate. Esters can undergo hydrolysis reactions in the presence of acids or bases to generate the corresponding acids or alcohols.

The term "pharmaceutically acceptable" means that a substance or composition must be chemically and/or toxicologically compatible with other ingredients of a formulation containing the same and/or a mammal treated therewith. Preferably, the "pharmaceutically acceptable" described in the present invention refers to that it is approved by federal regulatory agencies or national governments or listed in the United States Pharmacopoeia or other generally recognized pharmacopoeias for use in animals, especially humans.

As used herein, the term "pharmaceutically acceptable salt" refers to: (i) a salt formed by an acidic group present in the compound provided by the present invention and an appropriate inorganic or organic cation (base), which includes, but is not limited to, alkali metal salt, such as sodium salt, potassium salt, lithium salt, etc.; alkaline earth metal salt, such as calcium salt, magnesium salt, etc.; other metal salt, such as aluminum salt, iron salt, zinc salt, copper salt, nickel salt, cobalt salt, etc.; inorganic base salt, such as ammonium salt; organic base salt, such as tert-octylamine salt, dibenzylamine salt, morpholine salt, glucosamine salt, phenylglycine alkyl ester salt, ethylenediamine salt, N-methylglucosamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylamine salt, tris(hydroxymethyl)aminomethane salt; and, (ii) a salt formed by a basic group present in the compound provided by the present invention and an appropriate inorganic or organic anion (acid), which includes, but is not limited to, hydrohalide, such as hydrofluoride, hydrochloride, hydrobromide, hydroiodide, etc.; inorganic acid salt, such as nitrate, perchlorate, sulfate, phosphate, etc.; lower-alkanesulfonate, such as methanesulfonate, trifluoromethanesulfonate, ethanesulfonate, etc.; arylsulfonate, such as benzenesulfonate, p-toluene sulfonate, etc.; organic acid salt, such as acetate, malate,

fumarate, succinate, citrate, tartrate, oxalate, maleate, etc.; amino acid salt, such as glycinate, trimethylglycinate, arginine salt, ornithine salt, glutamate, aspartate, etc.

As used herein, the term "crystal form" refers to the crystal structure of a substance. When a substance crystallizes, it is affected by various factors, which changes the intramolecular or intermolecular bonding mode, resulting in different arrangements of molecules or atoms in the lattice space, and forming different crystal structures. The compound of the present invention may exist in one crystal structure or in multiple crystal structures, i.e., they have "polymorphism". The compound of the present invention may exist in different crystal forms.

The present invention will be further described below in conjunction with specific examples. It should be understood that these examples are only used to illustrate the present invention and are not used to limit the scope of the present invention. In the following examples, if the experimental methods for specific conditions are not specified, they are usually carried out according to the conventional conditions of such reactions or according to the conditions recommended by the manufacturer. Unless otherwise specified, percentages and parts are weight percentages and weight parts. Unless otherwise specified, the ratio of liquids is volume ratio. The experimental materials and reagents used in the following examples were obtained from commercial channels unless otherwise specified.

Preparation Examples: Synthesis of Compounds

Preparation Example 1: Preparation of Compound 1

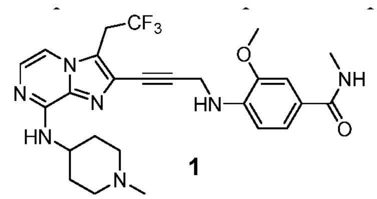

**1**

Synthesis scheme:

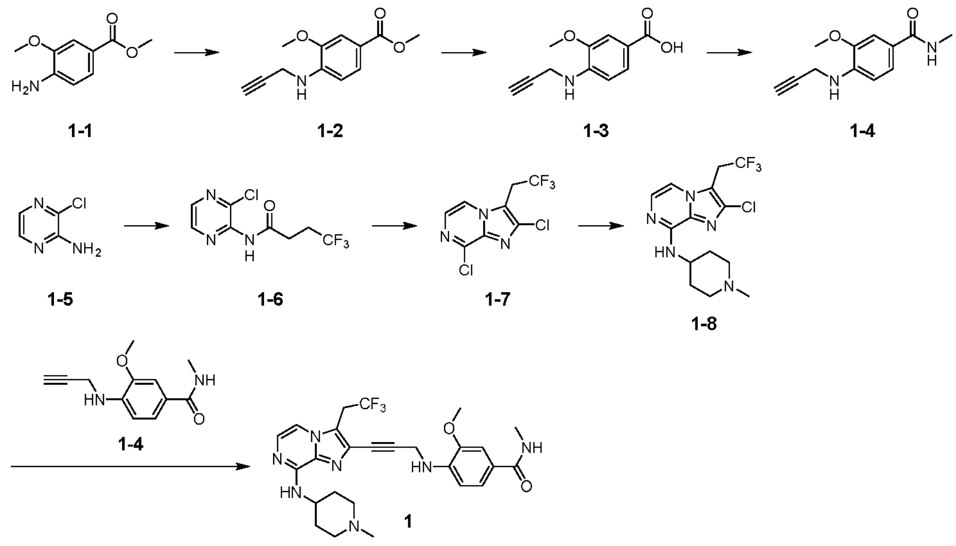

1-1 1-2 1-3 1-4

1-5 1-6 1-7 1-8

1-4

1

Step 1: Preparation of Compound **1-2**

Potassium carbonate (22.8 g, 165.6 mmol) and propargyl bromide (5.5 mL, 66.2 mmol) were added to a solution of Compound **1-1** (10.0 g, 55.2 mmol) in N,N-dimethylformamide (40 mL), heated to 70 °C and stirred for 4 hours under nitrogen protection. The reaction solution was poured into water (150 mL), extracted with ethyl acetate (80 mL x3), the organic phases were combined, washed with saturated brine (100 mL x2), and the resultant organic phase was concentrated under reduced pressure. The crude product was purified by rapid silica gel column chromatography to

obtain Compound **1-2. MS m/z (ESI):** 219.9 [M+H]$^+$. **$^1$H NMR** (400 MHz, $CDCl_3$) δ 7.66 (dd, *J* = 1.6, 8.4 Hz, 1H), 7.43 (d, *J* = 1.6 Hz, 1H), 6.65 (d, *J* = 8.4 Hz, 1H), 4.02 (d, *J* = 2.4 Hz, 2H), 3.89 (s, 3H), 3.86 (s, 3H), 2.24 (t, *J* = 2.4 Hz, 1H).

Step 2: Synthesis of Compound **1-3**

Compound **1-2** (7.5 g, 34.2 mmol) was dissolved in a mixture of methanol (90 mL), water (30 mL) and tetrahydrofuran (30 mL), added with lithium hydroxide (4.1 g, 171.2 mmol), heated to 40 °C and stirred for 4 hours under nitrogen protection. The reaction solution was concentrated under reduced pressure, the mixture was dissolved in water (100 mL), the pH was adjusted to 2 with dilute hydrochloric acid, the mixture was extracted with ethyl acetate (80 mL x3), the organic phases were combined and washed with saturated brine (100 mL x2), and the resultant organic phase was concentrated under reduced pressure to obtain Compound **1-3. MS m/z (ESI):** 205.9 [M+H]$^+$. **$^1$H NMR** (400 MHz, DMSO-$d_6$) δ 12.24 (s, 1H), 7.50 (dd, *J* = 1.6, 8.4 Hz, 1H), 7.32 (d, *J* = 1.6 Hz, 1H), 6.66 (d, *J= 8.4* Hz, 1H), 6.12 (t, *J* = 6.0 Hz, 1H), 3.98 (dd, *J*= 2.4, 6.0 Hz, 2H), 3.83 (s, 3H), 3.08 **(t,** *J* = 2.4 Hz, 1H).

Step 3: Synthesis of Compound **1-4:**

HATU (20.0 g, 52.7 mmol), methylamine hydrochloride (4.7 g, 70.2 mmol) and N,N-diisopropylethylamine (24.5 mL, 140.4 mmol) were added in sequence to a solution of Compound **1-3** (7.2 g, 35.1 mmol) in N,N-dimethylformamide (100 mL), and the reaction solution was stirred at room temperature for 4 hours under nitrogen protection. The reaction solution was poured into water (150 mL), extracted with ethyl acetate (80 mL x3), the organic phases were combined and washed with saturated brine (200 mL x2), the resultant organic phase was concentrated under reduced pressure, and the crude product was purified by rapid silica gel column chromatography to obtain Compound **1-4. MS m/z (ESI):** 219.0 [M+H]$^+$. **$^1$H NMR** (400 MHz, DMSO-$d_6$) δ 8.22 - 8.05 (m, 1H), 7.38 (dd, *J* = 2.0, 8.0 Hz, 1H), 7.32 (s, 1H), 6.62 (d, *J* = 8.4 Hz, 1H), 5.81 (t, *J* = 6.0 Hz, 1H), 3.95 (dd, *J* = 2.4, 6.4 Hz, 2H), 3.82 (s, 3H), 3.05 (t, *J* = 2.4 Hz, 1H), 2.75 (d, *J* = 4.4 Hz, 3H).

Step 4: Synthesis of Compound **1-6**

Under nitrogen protection, N-methylimidazole (4.3 mL, 54.3 mmol) and N,N,N',N'-tetramethylchloroformamidine hexafluorophosphate (4.0 g, 14.3 mmol) were added to a solution of Compound **1-5** (2.0 g, 15.4 mmol) and 4,4,4-trifluorobutyric acid (1.7 g, 11.9 mmol) in acetonitrile (31 mL). The mixture was heated to 85°C and stirred for 12 hours under nitrogen protection. The mixture was poured into water (150 mL), extracted with ethyl acetate (50 mL x3), and the organic phases were combined, washed with saturated brine (100 mL x 2), and concentrated under reduced pressure. The crude product was purified by flash silica gel column chromatography to obtain Compound **1-6. MS m/z (ESI):** 253.8 [M+H]$^+$. **$^1$H NMR** (400 MHz, $CDCl_3$) δ 8.30 (d, *J* = 2.4 Hz, 1H), 8.12 (d, *J* = 2.4 Hz, 1H), 8.07 (s, 1H), 3.14 - 3.08 (m, 2H), 2.66 - 2.54 (m, 2H).

Step 5: Synthesis of Compound **1-7**

Under nitrogen protection, triethylamine (1.6 mL, 11.6 mmol) and thionyl chloride (0.8 mL, 11.6 mmol) were added dropwise in sequence to a solution of Compound **1-6** (1.4 g, 5.5 mmol) in chloroform (10 mL). The mixture was heated to 90 °C with microwave under nitrogen protection and stirred for 15 hours. After the reaction solution was cooled to room temperature, saturated sodium bicarbonate solution (100 mL) was added, and extracted with dichloromethane (50 mL x3), the organic phases were combined, washed with saturated brine (100 mL x2) and concentrated under reduced pressure. The crude product was purified by rapid silica gel column chromatography to obtain Compound **1-7. MS m/z (ESI):** 269.9 [M+H]$^+$. **$^1$H NMR** (400 MHz, $CDCl_3$) δ 7.93 (d, *J* = 4.8 Hz, 1H), 7.84 (d, *J* = 4.8 Hz, 1H), 3.81 (q, *J* = 9.6 Hz, 2H).

Step 6: Synthesis of Compound **1-8**

Under nitrogen protection, 4-amino-1-methylpiperidine (0.09 mL, 0.74 mmol) was added to a solution of Compound **1-7** (200 mg, 0.67 mmol) in pyridine (2.0 mL). The mixture was heated to 110°C and stirred for 8 hours. The reaction solution was cooled to room temperature and concentrated under reduced pressure. The crude product was purified by rapid silica gel column chromatography to obtain Compound **1-8. MS m/z (ESI):** 348.0 [M+H]$^+$. **$^1$H NMR** (400 MHz, $CDCl_3$) δ 7.45 (d, *J* = 4.8 Hz, 1H), 7.25 (d, *J* = 4.8 Hz, 1H), 5.86 (d, *J* = 8.0 Hz, 1H), 4.16 - 4.01 (m, 1H), 3.68 (q, *J* = 9.6 Hz, 2H), 2.89 - 2.85 (m, 2H), 2.32 (s, 3H), 2.26 - 2.16 (m, 2H), 2.15 - 2.07 (m, 2H), 1.76 - 1.68 (m, 2H).

Step 7: Synthesis of Compound **1**

Under nitrogen protection, Compound **1-4** (8.0 mg, 0.04 mmol) was added to a solution of Compound **1-8** (10 mg,

0.03 mmol), 2-dicyclohexylphosphino-2',4',6'-triisopropylbiphenyl (4.0 mg, 0.01 mmol), bis(acetonitrile)palladium chloride (1.0 mg, 0.01 mmol) and $Cs_2CO_3$ (25 mg, 0.08 mmol) in acetonitrile (1.0 mL). The mixture was heated to 90°C and stirred for 2 hours under nitrogen protection. The reaction solution was cooled to room temperature and filtered, the filter cake was washed with acetonitrile (5.0 mL x3), and the filtrate was concentrated under reduced pressure. The crude product was purified by reverse phase flash chromatography to obtain Compound 1. **MS m/z (ESI): =** 530.1 $[M+H]^+$. **$^1$H NMR** (400 MHz, DMSO-$d_6$) δ 8.13 (d, *J*= 4.4 Hz, 1H), 7.71 (d, *J* = 4.8 Hz, 1H), 7.45 (d, *J* = 8.0 Hz, 1H), 7.40 (dd, *J* = 2.0, 8.0 Hz, 1H), 7.35 - 7.33 (m, 2H), 6.72 (d, *J* = 8.4 Hz, 1H), 5.99 (t, *J* = 6.4 Hz, 1H), 4.26 (d, *J* = 6.4 Hz, 2H), 4.01 (q, *J* = 10.4 Hz, 2H), 3.95 - 3.87 (m, 1H), 3.83 (s, 3H), 2.81 - 2.71 (m, 2H), 2.74 (d, *J* = 4.4 Hz, 3H), 2.16 (s, 3H), 1.96 (t, *J* = 12.0 Hz, 2H), 1.85 - 1.73 (m, 2H), 1.70 - 1.57 (m, 2H).

Preparation Example 2: Preparation of Compound 2

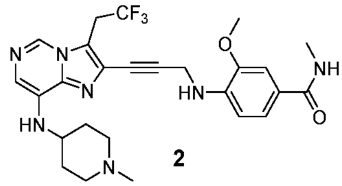

2

Synthesis scheme:

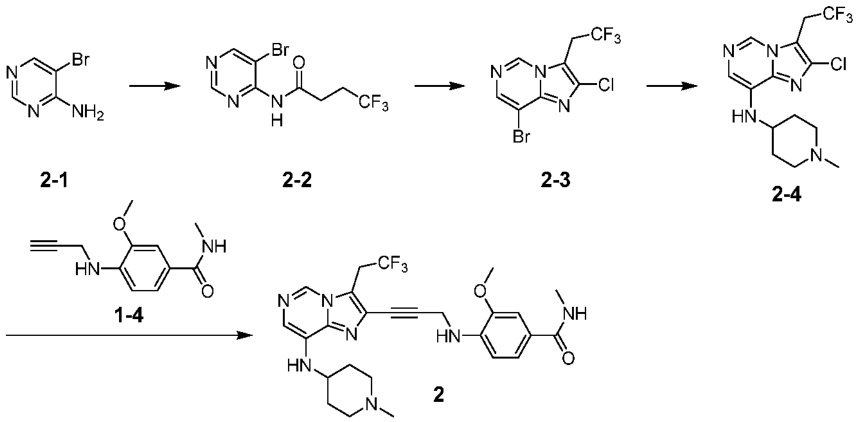

2-1 2-2 2-3 2-4 1-4 2

Step 1: Synthesis of Compound **2-2**

Under nitrogen protection, N-methylimidazole (3.2 mL, 40.4 mmol) and N,N,N',N'-tetramethylchloroformamidine hexafluorophosphate (3.0 g, 10.6 mmol) were added to a solution of Compound **2-1** (2.0 g, 11.5 mmol) and 4,4,4-trifluorobutyric acid (1.3 g, 8.8 mmol) in acetonitrile (22 mL), and the mixture was heated to 85°C and stirred for 12 hours under nitrogen protection. After the reaction solution was cooled to room temperature, it was poured into water (150 mL), extracted with ethyl acetate (50 mL x3), and the organic phases were combined and washed with saturated brine (100 mL x2), and the resultant organic phase was concentrated under reduced pressure. The crude product was purified by rapid silica gel column chromatography to obtain Compound **2-2. MS m/z (ESI):** 297.8/299.8 $[M+H]^+$. **$^1$H NMR** (400 MHz, $CDCl_3$) δ 8.76 (d, *J* = 2.4 Hz, 1H), 8.63 (d, *J* = 2.0 Hz, 1H), 7.99 (s, 1H), 3.27 - 3.20 (m, 2H), 2.61 - 2.44 (m, 2H).

Step 2: Synthesis of Compound **2-3**

Under nitrogen protection, triethylamine (1.0 mL, 7.2 mmol) and thionyl chloride (0.5 mL, 7.2 mmol) were added dropwise in sequence to a solution of Compound **2-2** (1.0 g, 3.4 mmol) in chloroform (10 mL). The mixture was heated to 90°C with microwave under nitrogen protection and stirred for 15 hours. After the reaction solution was cooled to room temperature, saturated sodium bicarbonate aqueous solution (100 mL) was added, and the mixture was extracted with dichloromethane (50 mL x3), and the organic phases were combined and washed with saturated brine (100 mL x2), and

the resultant organic phase was concentrated under reduced pressure. The crude product was purified by rapid silica gel column chromatography to obtain Compound **2-3. MS m/z (ESI):** 313.8/315.8 [M+H]$^+$. **$^1$H NMR** (400 MHz, $CDCl_3$) δ 8.86 (s, 1H), 8.25 (s, 1H), 3.86 (q, *J* = 9.6 Hz, 2H).

Step 3: Synthesis of Compound **2-4**

Under nitrogen protection, palladium acetate (251 mg, 1.12 mmol) and 2-dicyclohexylphosphino-2',4',6'-triisopropylbiphenyl (1.07 g, 2.24 mmol) were added to a solution of Compound **2-3** (280 mg, 0.89 mmol), 4-amino-1-methylpiperidine (510 mg, 4.47 mmol) and sodium tert-butoxide (644 mg, 6.71 mmol) in toluene (28 mL). The mixture was heated to 110°C and stirred for 2 hours under nitrogen protection. After the reaction solution was cooled to room temperature, it was filtered, the filter cake was washed with methanol (5.0 mL x3), and the filtrate was concentrated under reduced pressure. The crude product was purified by rapid silica gel column chromatography to obtain Compound **2-4. MS m/z (ESI):** = 348.0 [M+H]$^+$.

Step 4: Synthesis of Compound **2**

Under nitrogen protection, Compound **1-4** (38 mg, 0.17 mmol) was added to a solution of Compound **2-4** (30 mg, 0.08 mmol), 2-dicyclohexylphosphino-2',4',6'-triisopropylbiphenyl (12 mg, 0.03 mmol), bis(acetonitrile)palladium chloride (3.0 mg, 0.01 mmol) and cesium carbonate (73 mg, 0.22 mmol) in acetonitrile (1.0 mL). The mixture was heated to 90°C and stirred for 2 hours under nitrogen protection. After the reaction solution was cooled to room temperature, it was filtered, the filter cake was washed with acetonitrile (5.0 mL x3), and the filtrate was concentrated under reduced pressure. The crude product was purified by reverse phase flash chromatography to obtain Compound **2. MS m/z (ESI):** 530.3 [M+H]$^+$. **$^1$H NMR** (400 MHz, DMSO-$d_6$) δ 8.67 (s, 1H), 8.10 (d, *J* = 4.8 Hz, 1H), 7.41 (dd, *J = 2.0,* 8.4 Hz, 1H), 7.35 (d, *J* = 2.0 Hz, 1H), 7.18 (s, 1H), 6.73 (d, *J* = 8.4 Hz, 1H), 6.02 - 5.91 (m, 2H), 4.28 (d, *J* = 6.4 Hz, 2H), 4.10 (q, *J* = 10.4 Hz, 2H), 3.84 (s, 3H), 3.83 - 3.80 (m, 1H), 2.81 - 2.68 (m, 5H), 2.15 (s, 3H), 2.04 - 1.93 (m, 2H), 1.89 - 1.80 (m, 2H), 1.63 - 1.48 (m, 2H).

Preparation Example 3: Preparation of Compound 3

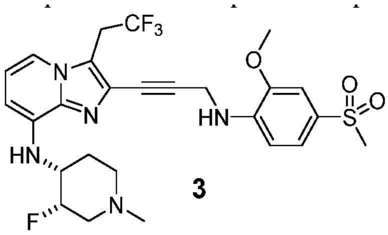

**3**

Synthesis scheme:

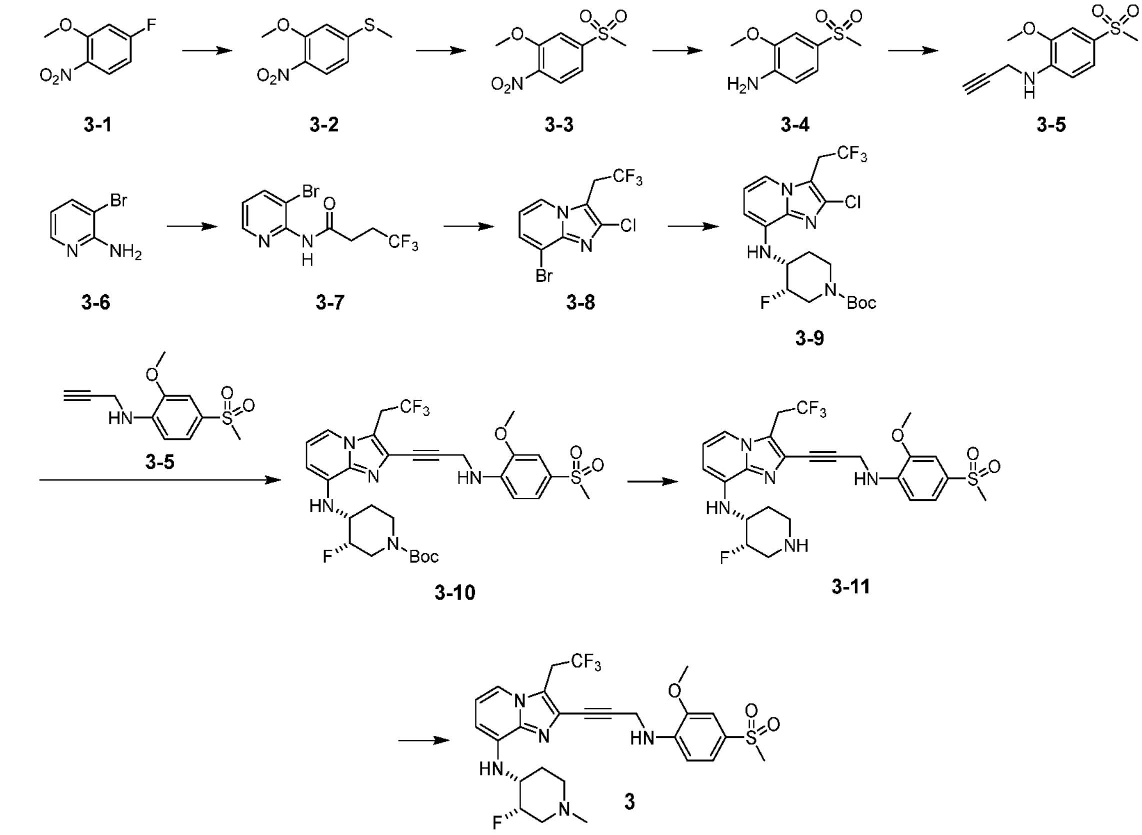

3-1 3-2 3-3 3-4 3-5

3-6 3-7 3-8 3-9

3-5

3-10 3-11

3

Step 1: Synthesis of Compound **3-2**

Compound **3-1** (6.0 g, 35.1 mmol) was added to a mixed solution of 20% sodium thiomethoxide aqueous solution (16 mL) and N,N-dimethylformamide (50 mL) at 0°C, and the reaction solution was stirred at room temperature for 2 hours under nitrogen protection. The reaction solution was poured into water (300 mL), extracted with ethyl acetate (100 mL x3), the organic phases were combined and washed with saturated brine (300 mL x2), and the resultant organic phase was concentrated under reduced pressure to obtain Compound **3-2. MS m/z (ESI):** 199.9 $[M+H]^+$. **$^1$H NMR** (400 MHz, $CDCl_3$) δ 7.82 (d, *J* = 8.4 Hz, 1H), 6.79 (d, *J* = 2.0 Hz, 1H), 6.74 (dd, *J* = 2.0, 8.4 Hz, 1H), 3.89 (s, 3H), 2.47 (s, 3H).

Step 2: Synthesis of Compound **3-3**

Oxone (27.0 g, 43.9 mmol) was added to a mixed solution of Compound **3-2** (3.5 g, 17.6 mmol) in acetone (30 mL), water (30 mL) and methanol (3 mL) at 0 °C, and the reaction solution was stirred in an open state at room temperature for 2 hours. The reaction solution was poured into a saturated sodium thiosulfate aqueous solution (200 mL), extracted with ethyl acetate (100 mL x3), the organic phases were combined, washed with saturated brine (300 mL x2), and the resultant organic phase was concentrated under reduced pressure to obtain Compound **3-3. $^1$H NMR (400** MHz, $CDCl_3$) δ 7.88 (d, *J* = 8.4 Hz, 1H), 7.60 (d, *J* = 1.6 Hz, 1H), 7.55 (dd, *J* = 1.6, 8.4 Hz, 1H), 3.99 (s, 3H), 3.04 (s, 3H).

Step 3: Synthesis of Compound **3-4**

Reduced iron powder (3.4 g, 60.55 mmol) and ammonium chloride (3.24 g, 60.55 mmol) were added to a mixed solution of Compound **3-3** (3.5 g, 15.14 mmol) in ethanol (30 mL) and water (10 mL), and the mixture was heated to 80 °C and stirred for 2 hours under nitrogen protection. The reaction solution was filtered through diatomaceous earth while hot, the diatomaceous earth was washed with methanol, the reaction solution was concentrated under reduced pressure, and then poured into water (50 mL), extracted with ethyl acetate (50 mL x3), the organic phases were combined and washed with saturated brine (100 mL x2), and the resultant organic phase was concentrated under reduced pressure to obtain Compound **3-4. MS m/z (ESI):** 201.8 $[M+H]^+$. **$^1$H NMR** (400 MHz, $CDCl_3$) δ 7.39 (dd, *J* = 2.0, 8.0 Hz, 1H), 7.27 (d, *J* = 0.8 Hz, 1H), 6.75 (d, *J* = 8.0 Hz, 1H), 4.44 (br s, 2H), 3.92 (s, 3H), 3.02 (s, 3H).

Step 4: Synthesis of Compound **3-5**

Potassium carbonate (6.2 g, 14.91 mmol) and propargyl bromide (1.5 mL, 17.9 mmol) were added to a solution of Compound **3-4** (3.0 g, 14.9 mmol) in N,N-dimethylformamide (10 mL), and the mixture was heated to 70 °C and stirred for 12 hours under nitrogen protection. The reaction solution was poured into 100 mL of water, extracted with ethyl acetate (50 mL x3), the organic phases were combined, washed with saturated brine (100 mL x2), the resultant organic phase was concentrated under reduced pressure, and the crude product was purified by rapid silica gel column chromatography to obtain Compound **3-5. MS m/z (ESI):** 239.8 $[M+H]^+$. **$^1$H NMR** (400 MHz, $CDCl_3$) δ 7.51 (dd, *J* = 2.0, 8.4 Hz, 1H), 7.26 (s, 1H), 6.74 (d, *J* = 8.4 Hz, 1H), 4.04 (d, *J* = 2.4 Hz, 2H), 3.92 (s, 3H), 3.03 (s, 3H), 2.26 (t, *J* = 2.4 Hz, 1H).

Step 5: Synthesis of Compound 3-7

Under nitrogen protection, N-methylimidazole (3.2 mL, 40.4 mmol) and N,N,N',N'-tetramethylchloroformamidine hexafluorophosphate (3.0 g, 10.6 mmol) were added to a solution of Compound **3-6** (2.0 g, 11.5 mmol) and 4,4,4-trifluorobutyric acid (1.3 g, 8.8 mmol) in acetonitrile (22 mL). The mixture was stirred at 25°C for 12 hours under nitrogen protection. The reaction solution was dissolved in water (150 mL), extracted with ethyl acetate (50 mL x3), the organic phases were combined, washed with saturated brine (100 mL x2), and the resultant organic phase was concentrated under reduced pressure. The crude product was purified by flash silica gel column chromatography to obtain Compound **3-7. MS m/z (ESI):** 296.8/298.8 $[M+H]^+$. **$^1$H NMR** (400 MHz, $CDCl_3$) δ 8.76 (d, *J* = 3.2 Hz, 1H), 8.63 (d, *J* = 2.0 Hz, 1H), 7.99 (s, 1H), 3.25 - 3.22 (m, 2H), 2.56 - 2.50 (m, 2H).

Step 6: Synthesis of Compound **3-8**

Under nitrogen protection, triethylamine (1.0 mL, 7.2 mmol) and thionyl chloride (0.5 mL, 7.2 mmol) were added dropwise in sequence to a solution of Compound **3-7** (1.0 g, 3.4 mmol) in chloroform (10.0 mL). The mixture was heated to 90 °C with microwave under nitrogen protection and stirred for 15 hours. After the reaction solution was cooled to room temperature, saturated sodium bicarbonate aqueous solution (100 mL) was added, the mixture was extracted with dichloromethane (50 mL x3), the organic phases were combined and washed with saturated brine (100 mL x2), and the resultant organic phase was concentrated under reduced pressure. The crude product was purified by rapid silica gel column chromatography to obtain Compound 3-8. **MS m/z (ESI):** 312.8/314.8 $[M+H]^+$. **$^1$H NMR** (400 MHz, DMSO-$d_6$) δ 8.69 (d, *J* = 6.8 Hz, 1H), 7.79 (dd, *J* = 0.8, 7.6 Hz, 1H), 7.07 (d, *J* = 7.2 Hz, 1H), 4.25 (q, *J* = 10.8 Hz, 2H).

Step 7: Synthesis of Compound **3-9**

Under nitrogen protection, palladium acetate (34 mg, 0.15 mmol) and 2-dicyclohexylphosphino-2',4',6'-triisopropylbiphenyl (143 mg, 0.3 mmol) were added to a solution of Compound **3-8** (470 mg, 1.5 mmol), cis-4-amino-3-fluoropiperidine-1-carboxylic acid tert-butyl ester (390 mg, 1.8 mmol) and cesium carbonate (980 mg, 3.0 mmol) in 1,4-dioxane (5 mL). mmol). The mixture was heated to 110 °C and stirred for 12 hours under nitrogen protection. The reaction solution was cooled to room temperature and filtered, the filter cake was washed with methanol (5.0 mL x3), and the filtrate was concentrated under reduced pressure. The crude product was purified by rapid silica gel column chromatography to obtain Compound **3-9. MS m/z (ESI):** 451.0 $[M+H]^+$. **$^1$H NMR** (400 MHz, $CDCl_3$) δ 7.41 (d, *J* = 6.8 Hz, 1H), 6.83 (t, *J* = 7.2 Hz, 1H), 6.26 (d, *J* = 7.2 Hz, 1H), 4.88 - 4.76 (m, 1H), 4.45 - 4.28 (m, 2H), 3.72 (q, *J* = 10.0 Hz, 2H), 3.68 - 3.62 (m, 1H), 3.22 - 2.84 (m, 2H), 1.95 - 1.92 (m, 2H), 1.48 (s, 9H).

Step 8: Synthesis of Compound **3-10**

Under nitrogen protection, Compound **3-5** (109 mg, 0.46 mmol) was added to a solution of Compound **3-9** (160 mg, 0.35 mmol), 2-dicyclohexylphosphino-2',4',6'-triisopropylbiphenyl (51 mg, 0.11 mmol), bis(acetonitrile)palladium chloride (9 mg, 0.03 mmol) and cesium carbonate (297 mg, 0.91 mmol) in acetonitrile (1.0 mL). The mixture was heated to 90 °C and stirred for 1.5 hours under nitrogen protection. After the reaction solution was cooled to room temperature, it was filtered, the filter cake was washed with acetonitrile (5.0 mL x3), and the filtrate was concentrated under reduced pressure. The crude product was purified by rapid silica gel column chromatography to obtain Compound **3-10. MS m/z (ESI):** 654.2 $[M+H]^+$. **$^1$H NMR** (400 MHz, $CDCl_3$) δ 7.52 (dd, *J= 2.0,* 8.4 Hz, 1H), 7.36 (d, *J* = 6.8 Hz, 1H), 7.27 - 7.25 (m, 2H), 6.84 - 6.80 (m, 2H), 6.17 (br s, 1H), 5.16 (t, *J*= 6.0 Hz, 1H), 4.34 (d, *J* = 6.0 Hz, 2H), 3.94 - 3.91 (m, 5H), 3.71 - 3.64 (m, 3H), 3.07 - 3.02 (m, 6H), 1.92 (br s, 2H), 1.48 (s, 9H).

Step 9: Synthesis of Compound **3-11**

Under nitrogen protection, Compound **3-10** (70 mg, 0.11 mmol) was dissolved in hydrochloric acid-dioxane solution (5 mL, 4M). The mixture was stirred at room temperature for 1 hour. The reaction solution was concentrated under reduced pressure, saturated sodium bicarbonate aqueous solution (30 mL) was added, the mixture was extracted with ethyl acetate (10 mL x 3), the organic phases were combined and washed with saturated brine (30 mL x2), and the resultant organic phase was concentrated under reduced pressure to obtain crude Compound **3-11. MS m/z (ESI):** 554.2 $[M+H]^+$.

Step 10: Synthesis of Compound **3**

Under nitrogen protection, paraformaldehyde (13.5 mg, 0.45 mmol), sodium cyanoborohydride (27 mg, 0.45 mmol) and acetic acid (20 μL) were added to a solution of Compound **3-11** (50 mg, 0.09 mmol) in methanol (5 mL). The mixture was heated to 50 °C and stirred for 2 hours. The reaction solution was poured into a saturated sodium bicarbonate aqueous solution (30 mL), extracted with ethyl acetate (10 mL x3), the organic phases were combined, washed with saturated brine (30 mL x2), and the resultant organic phase was concentrated under reduced pressure. The crude product was purified by reverse phase flash chromatography to obtain Compound 3. **MS m/z (ESI):** 568.2 $[M+H]^+$. **$^1$H NMR** (400 MHz, DMSO-$d_6$) δ 7.80 (d, *J* = 6.8 Hz, 1H), 7.39 (dd, *J* = 2.0, 8.4 Hz, 1H), 7.25 (d, *J* = 2.0 Hz, 1H), 6.87 (d, *J* = 8.4 Hz, 1H), 6.83 *(t, J* = 7.2 Hz, 1H), 6.50 (t, *J* = 6.0 Hz, 1H), 6.40 (d, *J* = 7.6 Hz, 1H), 5.38 (d, *J* = 9.2 Hz, 1H), 4.88 (d, *J* = 52.0 Hz, 1H), 4.31 (d, *J* = 6.0 Hz, 2H), 4.03 (q, *J* = 10.4 Hz, 2H), 3.89 (s, 3H), 3.73 (m, 1H), 3.10 (s, 3H), 3.03 - 3.00 (m, 1H), 2.78 - 2.63 (m, 1H), 2.30 - 2.26 (m, 1H), 2.18 (s, 3H), 2.11-2.06 (m, 1H), 1.85 - 1.74 (m, 2H).

Preparation Example 4: Preparation of Compound **4**

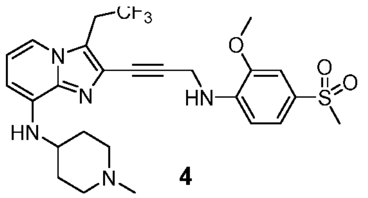

**4**

Synthesis scheme:

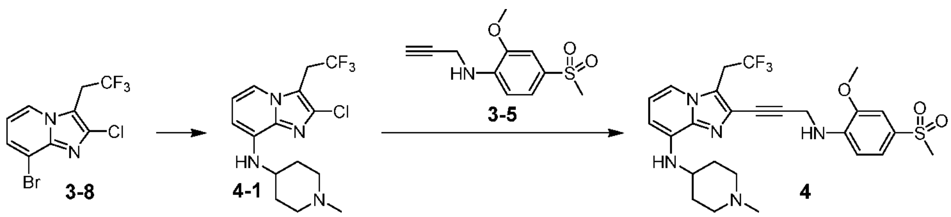

Step 1: Synthesis of Compound **4-1**

Under nitrogen protection, palladium acetate (17 mg, 0.08 mmol) and 2-dicyclohexylphosphino-2',4',6'-triisopropylbiphenyl (72 mg, 0.15 mmol) were added to a solution of Compound **3-8** (240 mg, 0.77 mmol), 4-amino-1-methylpiperidine (100 mg, 0.9 mmol) and cesium carbonate (490 mg, 1.5 mmol) in 1,4-dioxane (5 mL). The mixture was heated to 110 °C and stirred for 12 hours under nitrogen protection. The reaction solution was cooled to room temperature and filtered, the filter cake was washed with methanol (5.0 mL x3), and the filtrate was concentrated under reduced pressure. The crude product was purified by rapid silica gel column chromatography to obtain Compound **4-1. MS m/z (ESI):** 347.0 $[M+H]^+$.

Step 2: Synthesis of Compound **4**

Under nitrogen protection, Compound **3-5** (43 mg, 0.18 mmol) was added to a solution of Compound 4-1 (50 mg,

0.14 mmol), 2-dicyclohexylphosphino-2',4',6'-triisopropylbiphenyl (20 mg, 0.04 mmol), bis(acetonitrile)palladium chloride (3.6 mg, 0.014 mmol) and cesium carbonate (119 mg, 0.36 mmol) in acetonitrile (1.0 mL). The mixture was heated to 90 °C and stirred for 2 hours. The reaction solution was cooled to room temperature and filtered, the filter cake was washed with acetonitrile (5.0 mL x3), and the filtrate was concentrated under reduced pressure. The crude product was purified by reverse phase flash chromatography to obtain Compound 4. **MS m/z (ESI):** 550.2 $[M+H]^+$. **$^1$H NMR** (400 MHz, DMSO-$d_6$) δ 8.29 (s, 1H), 7.72 (d, *J* = 6.8 Hz, 1H), 7.39 (dd, *J* = 2.0, 8.4 Hz, 1H), 7.25 (d, *J = 2.0* Hz, 1H), 6.86 (d, *J* = 8.4 Hz, 1H), 6.80 (t, *J = 7.2* Hz, 1H), 6.50 (t, *J* = 6.4 Hz, 1H), 6.22 (d, *J* = 7.6 Hz, 1H), 5.72 (d, *J* = 8.4 Hz, 1H), 4.31 (d, *J* = 6.4 Hz, 2H), 4.03 (q, *J* = 10.8 Hz, 2H), 3.89 (s, 3H), 3.49-3.45 (m, 1H), 3.10 (s, 3H), 2.75 - 2.67 (m, 2H), 2.17 (s, 3H), 2.05 - 2.00 (m, 2H), 1.89 - 1.84 (m, 2H), 1.60 - 1.51 (m, 2H).

Preparation Example 5: Preparation of Compound **5**

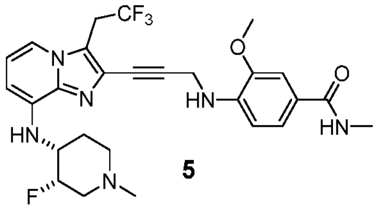

**5**

Synthesis scheme:

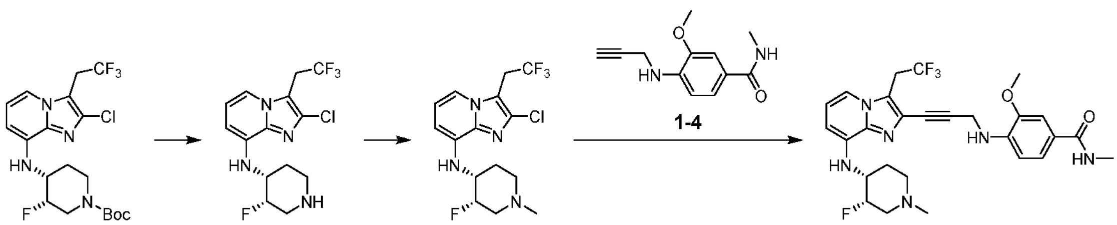


**3-9** **5-1** **5-2** **5**

Step 1: Synthesis of Compound **5-1**

Under nitrogen protection, trifluoroacetic acid (1.0 mL, 13.42 mmol) was added to a solution of Compound **3-9** (600 mg, 0.64 mmol) in dichloromethane (5 mL). Under nitrogen protection, the reaction solution was stirred at 25 °C for 0.5 hours. The reaction solution was poured into a saturated sodium bicarbonate aqueous solution (60 mL) and extracted with dichloromethane (60 mL x3). The organic phases were combined, dried over anhydrous sodium sulfate, and filtered. The filtrate was concentrated under reduced pressure to obtain Compound **5-1. MS m/z (ESI):** 351.0 $[M+H]^+$. **$^1$H NMR** (400 MHz, $CDCl_3$) δ 7.39 (d, *J* = 6.8 Hz, 1H), 6.78 (t, *J*=7.2 Hz, 1H), 6.21 (d, *J = 7.6* Hz, 1H), 5.38 (d, *J* = 9.2 Hz, 1H), 4.84 - 4.81 (m, 1H), 3.71 (q, *J* = 10.0 Hz, 2H), 3.66 - 3.57 (m, 1H), 3.51 - 3.40 (m, 1H), 3.29 - 3.21 (m, 1H), 3.01 - 2.84 (m, 1H), 2.84 - 2.74 (m, 1H), 2.03 - 1.94 (m, 1H), 1.88 - 1.75 (m, 1H).

Step 2: Synthesis of Compound **5-2**

Under nitrogen protection, sodium cyanoborohydride (48 mg, 0.76 mmol) was added to a solution of Compound **5-1** (267 mg, 0.61 mmol), paraformaldehyde (114 mg, 3.80 mmol) and acetic acid (13 mg, 0.21 mmol) in methanol (6.0 mL). Under nitrogen protection, the reaction solution was stirred at 50°C for 2 hours. The reaction solution was poured into a saturated sodium bicarbonate aqueous solution (60 mL) and extracted with ethyl acetate (60 mL x3). The organic phases were combined and dried over anhydrous sodium sulfate and then filtered. The filtrate was concentrated under reduced pressure. The crude product was purified by rapid silica gel column chromatography to obtain Compound **5-2. MS m/z (ESI):** 365.0 $[M+H]^+$. **$^1$H NMR** (400 MHz, $CDCl_3$) δ 7.38 (d, *J*= 6.8 Hz, 1H), 6.81 - 6.75 (m, 1H), 6.19 (d, *J*= 7.6 Hz, 1H), 5.37 (d, *J*= 9.2 Hz, 1H), 4.96 - 4.76 (m, 1H), 3.71 (q, *J* = 10.0 Hz, 2H), 3.63 - 3.45 (m, 1H), 3.29 - 3.14 (m, 1H), 3.02 - 2.88 (m, 1H), 2.35 (s, 3H), 2.32 - 2.12 (m, 2H), 2.08 - 1.92 (m, 2H).

Step 3: Synthesis of Compound **5**

Under nitrogen protection, Compound **1-4** (120 mg, 0.55 mmol) was added to a solution of Compound **5-2** (100 mg, 0.25 mmol), 2-dicyclohexylphosphino-2',4',6'-triisopropylbiphenyl (40 mg, 0.08 mmol), cesium carbonate (224 mg, 0.69 mmol) and bis(acetonitrile)palladium chloride (8.0 mg, 0.03 mmol) in acetonitrile (4.0 mL). Under nitrogen protection, the reaction solution was stirred at 90 °C for 2 hours. After the reaction solution was cooled to room temperature, it was filtered and the filter cake was washed with acetonitrile (10 mL x3). The filtrate was concentrated and purified by SFC preparative and reverse phase flash chromatography to obtain Compound **5. MS m/z (ESI):** 547.3 $[M+H]^+$. **$^1$H NMR** (400 MHz, DMSO-$d_6$) δ 8.09 (d, *J* = 4.8 Hz, 1H), 7.78 (d, *J* = 6.8 Hz, 1H), 7.41 (dd, *J* = 2.0, 8.4 Hz, 1H), 7.34 (d, *J = 2.0* Hz, 1H), 6.82 (t, *J* = 7.2 Hz, 1H), 6.74 (d, *J* = 8.4 Hz, 1H), 6.39 (d, *J* = 7.6 Hz, 1H), 5.95 (t, *J* = 6.4 Hz, 1H), 5.37 (d, *J* = 9.2 Hz, 1H), 4.81 (d, *J* = 49.2 Hz, 1H), 4.26 (d, *J* = 6.4 Hz, 2H), 4.01 (q, *J* = 10.8 Hz, 2H), 3.84 (s, 3H), 3.78 - 3.60 (m, 1H), 3.08 - 2.96 (m, 1H), 2.81 - 2.70 (m, 4H), 2.31 - 2.22 (m, 1H), 2.19 (s, 3H), 2.14 - 2.04 (m, 1H), 1.87 - 1.67 (m, 2H).

Preparation Example 6: Preparation of Compound 6

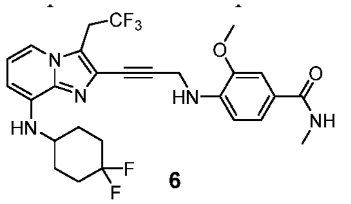

6

Synthesis scheme:

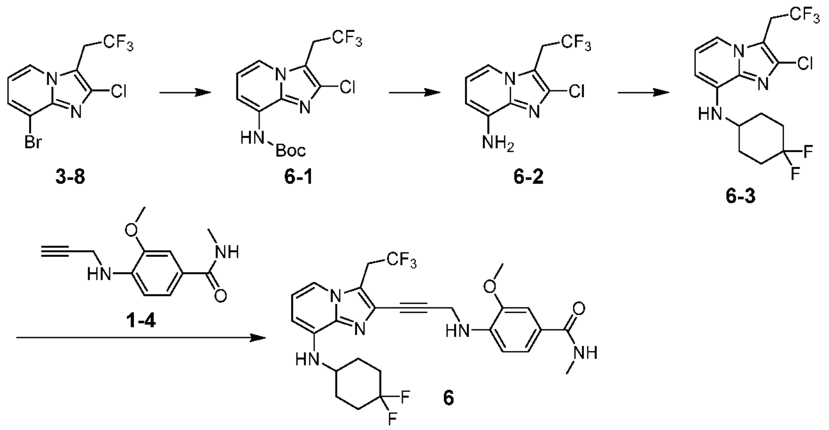

Step 1: Synthesis of Compound **6-1**

Under nitrogen protection, palladium acetate (0.144 g, 0.64 mmol) and 2-dicyclohexylphosphino-2',4',6'-triisopropylbiphenyl (0.611 g, 1.28 mmol) were added to a solution of Compound **3-8** (2.0 g, 3.06 mmol), tert-butyl carbamate (1.13 g, 9.65 mmol) and cesium carbonate (2.09 g, 6.41 mmol) in 1,4-dioxane (15 mL). Under nitrogen protection, the reaction solution was stirred at 110°C for 16 hours. After the reaction solution was cooled to room temperature, it was filtered, the filter cake was washed with acetonitrile (20 mL x3), and the filtrate was concentrated under reduced pressure. The crude product was purified by rapid silica gel column chromatography to obtain Compound **6-1.** $^1$H NMR (400 MHz, $CDCl_3$) δ 7.91 (d, *J* = 7.6 Hz, 1H), 7.77 (s, 1H), 7.64 (d, *J* = 6.8 Hz, 1H), 6.92 (dd, *J* = 6.8, 7.6 Hz, 1H), 3.74 (q, *J= 10.0* Hz, 2H), 1.53 (s, 9H).

Step 2: Synthesis of Compound **6-2**

Under nitrogen protection, trifluoroacetic acid (4.0 mL, 53.67 mmol) was added to a solution of Compound **6-1**

(1.55 g, 3.99 mmol) in dichloromethane (20 mL). Under nitrogen protection, the mixture was stirred at 25°C for 2 hours. The reaction solution was poured into a saturated sodium bicarbonate aqueous solution (100 mL) and extracted with ethyl acetate (100 mL x3). The organic phases were combined and dried over anhydrous sodium sulfate, filtered, and the filtrate was concentrated under reduced pressure. The crude product was purified by rapid silica gel column chromatography to obtain Compound **6-2.** **$^1$H NMR** (400 MHz, $CDCl_3$) δ 7.44 (d, *J* = 6.8 Hz, 1H), 6.76 (t, *J* = 7.2 Hz, 1H), 6.43 (dd, *J* = 0.8, 7.6 Hz, 1H), 4.52 (s, 2H), 3.71 (q, *J* = 10.0 Hz, 2H).

Step 3: Synthesis of Compound **6-3**

Trimethylsilyl chloride (0.17 mL, 1.35 mmol) was added to a solution of Compound **6-2** (150 mg, 0.54 mmol) and 4,4-difluorocyclohexanone (190.92 mg, 1.35 mmol) in N,N-dimethylformamide (3 mL). The reaction solution was stirred at 25°C for 2 hours under nitrogen protection. Borane tetrahydrofuran (2.70 mL, 1M) was added dropwise to the above reaction solution, and the reaction solution was stirred at room temperature for 2 hours at 25°C. The reaction solution was quenched with saturated sodium carbonate (20 mL) solution, extracted with ethyl acetate (30 mL x3), the organic phases were combined and washed with saturated brine (50 mL), dried over anhydrous sodium sulfate, filtered, and the filtrate was concentrated under reduced pressure. The crude product was purified by rapid silica gel column chromatography to obtain Compound **6-3. MS m/z (ESI):** 368.0 $[M+H]^+$.

Step 4: Synthesis of Compound **6**

Under nitrogen protection, Compound **1-4** (60 mg, 0.23 mmol) was added to a solution of Compound **6-3** (50 mg, 0.13 mmol), 2-dicyclohexylphosphino-2',4',6'-triisopropylbiphenyl (40 mg, 0.08 mmol), cesium carbonate (112 mg, 0.34 mmol) and bis(acetonitrile)palladium chloride (8.0 mg, 0.03 mmol) in acetonitrile (2.0 mL). The reaction solution was stirred at 90 °C for 2 hours under nitrogen protection. After the reaction solution was cooled to room temperature, it was filtered and the filter cake was washed with acetonitrile (10 mL x3). The filtrate was concentrated and purified by reverse phase flash chromatography to obtain Compound **6. MS m/z (ESI):** 550.2 $[M+H]^+$. **$^1$H NMR** (400 MHz, $CDCl_3$) δ 7.36 (d, *J* = 2.0 Hz, 1H), 7.31 (d, *J* = 6.8 Hz, 1H), 7.22 (dd, *J* = 2.0, 8.4 Hz, 1H), 6.81 - 6.70 (m, 2H), 6.14 (d, *J* = 7.6 Hz, 1H), 6.06 (brs, 1H), 5.19 - 5.16 (m, 1H), 4.90 (brs, 1H), 4.29 (s, 2H), 3.90 (s, 3H), 3.63 (q, *J* = 10.0 Hz, 2H), 3.55 - 3.50 (m, 1H), 2.99 (d, *J* = 4.8 Hz, 3H), 2.22 - 2.08 (m, 4H), 2.04 - 1.74 (m, 4H).

Preparation Example 7: Preparation of Compound 7

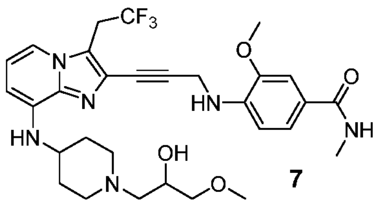

7

Synthesis scheme:

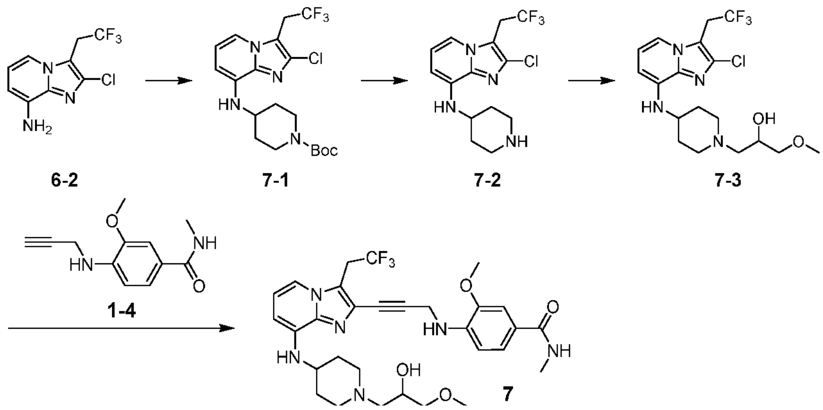

6-2 7-1 7-2 7-3

1-4

7

Step 1: Synthesis of Compound 7-1

Under nitrogen protection, trimethylsilyl chloride (0.61 mL, 4.76 mmol) was added to a solution of Compound 6-2 (500 mg, 1.90 mmol) and N-tert-butyloxycarbonyl-4-piperidone (134 mg, 0.95 mmol) in N,N-dimethylformamide (5 mL), and the reaction solution was stirred at room temperature for 2 hours. Then, borane tetrahydrofuran (4.76 mL, 4.76 mmol) was added dropwise to the reaction solution, and stirring was continued at room temperature for 2 hours. The reaction solution was quenched with saturated sodium carbonate solution (20 mL), extracted with ethyl acetate (30 mL x2), the organic phases were combined and washed with saturated brine (50 mL), dried over anhydrous sodium sulfate and filtered, and the filtrate was concentrated under reduced pressure. The crude product was purified by rapid silica gel column chromatography to obtain Compound **7-1. MS m/z (ESI):** 433.2 $[M+H]^+$.

Step 2: Synthesis of Compound **7-2**

Trifluoroacetic acid (2.5 mL, 33.55 mmol) was added to a solution of Compound **7-1** (620 mg, 1.29 mmol) in dichloromethane (8 mL), and the reaction solution was stirred at room temperature for 2 hours under nitrogen protection. It was quenched with saturated sodium bicarbonate aqueous solution (30 mL) and extracted with dichloromethane (40 mL x3). The organic phases were combined and dried over anhydrous sodium sulfate and filtered, and the filtrate was concentrated under reduced pressure to obtain Compound **7-2. MS m/z (ESI):** 333.2 $[M+H]^+$.

Step 3: Synthesis of Compound **7-3**

In an ice bath, potassium hydroxide (18 mg, 0.31 mmol) was added to a solution of Compound **7-2** (0.03 mL, 0.27 mmol) in methanol (2 mL), and the reaction solution was stirred in an ice bath for 15 minutes. 1-Chloro-3-methoxy-2-propanol (33 mg, 0.09 mmol) was added to the reaction solution, and the reaction solution was stirred at 50 °C for 3 hours. The reaction solution was diluted with water (40 mL), extracted with ethyl acetate (40 mL x3), and the organic phases were combined and washed with saturated brine (40 mL), dried over anhydrous sodium sulfate, and filtered. The filtrate was concentrated under reduced pressure. The crude product was purified by rapid silica gel column chromatography to obtain Compound **7-3. MS m/z (ESI):** 421.0 $[M+H]^+$.

Step 4: Synthesis of Compound **7**

Under nitrogen protection, bis(acetonitrile)palladium chloride (1.6 mg, 0.01 mmol) was added to a mixture of Compound **7-3** (30 mg, 0.06 mmol), cesium carbonate (52 mg, 0.16 mmol) and 2-dicyclohexylphosphino-2',4',6'-triisopropylbiphenyl (6 mg, 0.01 mmol) in acetonitrile (2 mL), and the reaction solution was stirred at 100 °C for 10 minutes. Compound 1-4 (28 mg, 0.13 mmol) was added to the reaction solution, and the reaction solution was stirred at 100 °C for 2 hours under nitrogen protection. After the reaction solution was cooled to room temperature, it was filtered, and the filter cake was washed with acetonitrile (4 mL x3). The filtrate was concentrated and purified by reverse phase flash chromatography to obtain Compound **7. MS m/z (ESI):** 603.3 $[M+H]^+$. **$^1$H NMR** (400 MHz, DMSO-$d_6$) δ 8.12 (d, *J* = 4.8 Hz, 1H), 7.77 (d, *J* = 6.4 Hz, 1H), 7.41 (d, *J* = 8.0 Hz, 1H), 7.35 (s, 1H), 6.83 (t, *J* = 7.2 Hz, 1H), 6.73 (d, *J* = 8.0 Hz, 1H), 6.29 (d, *J* = 7.6 Hz, 1H), 6.09 (brs, 1H), 5.98 (brs, 1H), 5.77 - 5.66 (m, 1H), 4.27 (d, *J* = 6.4 Hz, 2H), 4.11 - 3.98 (m, 3H), 3.85 (s, 3H), 3.68 - 3.53 (m, 2H). 3.30 (s, 3H), 3.21 - 2.98 (m, 7H), 2.75 (d, *J* = 4.4 Hz, 3H), 2.03 - 1.77 (m, 4H).

Preparation Example 8: Preparation of Compound **8**

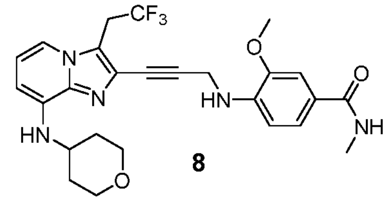

8

Synthesis scheme:

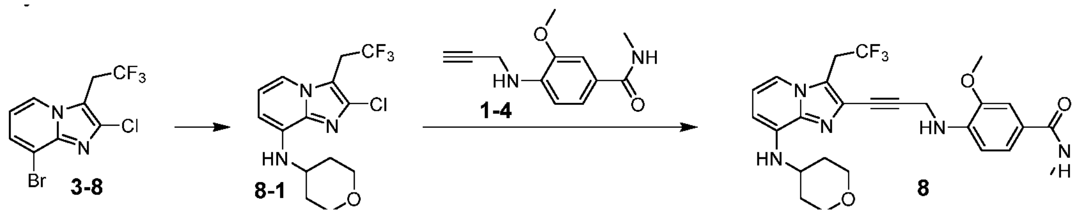


3-8 8-1 8

Step 1: Synthesis of Compound **8-1**

Under nitrogen protection, methanesulfonic acid (2-di-tert-butylphosphino-2',4',6'-triisopropyl-1,1'-biphenyl) (2'-amino-1,1'-biphenyl-2-yl) palladium (58 mg, 0.07 mmol) was added to a mixed solution of Compound **3-8** (230 mg, 0.73 mmol), 4-aminotetrahydropyran (0.11 mL, 1.10 mmol), sodium tert-butoxide (211 mg, 2.20 mmol) and 2-di-tert-butylphosphino-2',4',6'-triisopropylbiphenyl (30 mg, 0.07 mmol) in toluene (5 mL). The reaction solution was stirred at 80 °C for 2 hours under nitrogen protection. After the reaction solution was cooled to room temperature, it was concentrated under reduced pressure. The crude product was purified by rapid silica gel column chromatography to obtain Compound **8-1.** $^1$**H NMR** (400 MHz, $CDCl_3$) δ 7.29 (d, *J* = 6.8 Hz, 1H), 6.76 - 6.63 (m, 1H), 6.16 (d, *J* = 7.6 Hz, 1H), 5.03 - 4.92 (m, 1H), 3.98 (dt, *J* = 3.6, 11.8 Hz, 2H), 3.76 - 3.42 (m, 5H), 2.04 - 1.97 (m, 2H), 1.63 - 1.55 (m, 2H).

Step 2: Synthesis of Compound **8**

Under nitrogen protection, bis(acetonitrile)palladium chloride (4 mg, 0.02 mmol) was added to a solution of Compound **8-1** (40 mg, 0.12 mmol), 2-dicyclohexylphosphino-2',4',6'-triisopropylbiphenyl (12 mg, 0.03 mmol) and cesium carbonate (102 mg, 0.31 mmol) in acetonitrile (1 mL), and the reaction solution was stirred at 90 °C for 20 minutes. Compound **1-4** (53 mg, 0.24 mmol) was added to the reaction solution. The reaction solution was stirred at 90 °C for 2 hours under nitrogen protection. After the reaction solution was cooled to room temperature, it was concentrated under reduced pressure. The crude product was purified by reverse phase flash chromatography to obtain Compound **8. MS m/z (ESI):** 516.2 $[M+H]^+$. $^1$**H NMR** (400 MHz, DMSO-$d_6$) δ 8.12 (d, *J* = 4.8 Hz, 1H), 7.72 (d, *J* = 6.8 Hz, 1H), 7.41 (dd, *J* = 2.0, 8.4 Hz, 1H), 7.35 (d, *J*= *2.0* Hz, 1H), 6.80 (t, *J* = 7.2 Hz, 1H), 6.74 (d, *J* = 8.4 Hz, 1H), 6.28 (d, *J* = 7.6 Hz, 1H), 5.98 (t, *J* = 6.4 Hz, 1H), 5.86 (d, *J* = 8.4 Hz, 1H), 4.27 (d, *J* = 6.4 Hz, 2H), 4.02 (d, *J* = 10.4 Hz, 2H), 3.91 - 3.82 (m, 5H), 3.63 - 3.58 (m, 1H), 3.51 - 3.46 (m, 2H), 2.75 (d, *J* = 4.4 Hz, 3H), 1.89 - 1.84 (m, 2H), 1.60 - 1.55 (m, 2H).

Preparation Example 9: Preparation of Compound **9**

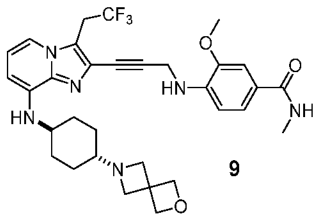

9

Synthesis scheme:

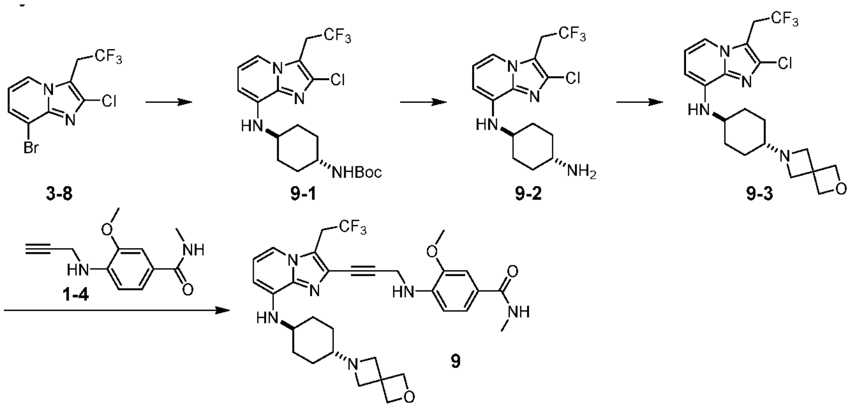

Step 1: Synthesis of Compound **9-1**

Cesium carbonate (936 mg, 2.87 mmol), 2-dicyclohexylphosphino-2',4',6'-triisopropylbiphenyl (457 mg, 0.96 mmol) and palladium acetate (108 mg, 0.48 mmol) were added to a solution of Compound **3-8** (300 mg, 0.96 mmol) and tert-butyl trans-(4-aminocyclohexyl)carbamate (310 mg, 1.45 mmol) in 1,4-dioxane (10 mL). The mixture was stirred at 100 °C for 16 hours under nitrogen protection. After the reaction solution was cooled to room temperature, it was concentrated under reduced pressure. The crude product was purified by rapid silica gel column chromatography to obtain Compound **9-1. MS m/z (ESI):** 447.2 $[M+H]^+$.

Step 2: Synthesis of Compound **9-2**

Trifluoroacetic acid (0.8 mL) was added to a solution of Compound **9-1** (230 mg, 0.51 mmol) in dichloromethane (4 mL), and the reaction solution was stirred at 25 °C for 0.5 hours. The reaction solution was poured into a saturated sodium bicarbonate aqueous solution for quenching, extracted with ethyl acetate (20 mL x2), and the organic phases were combined and washed with saturated brine (40 mL), dried over anhydrous sodium sulfate, and filtered. The filtrate was concentrated under reduced pressure to obtain Compound **9-2. MS m/z (ESI):** 347.0 $[M+H]^+$.

Step 3: Synthesis of Compound **9-3**

Sodium carbonate (375 mg, 3.54 mmol) was added to a solution of Compound **9-2** (175 mg, 0.50 mmol) and 3,3-bis(bromomethyl)oxetane (310 mg, 1.27 mmol) in acetonitrile (4 mL), and the reaction solution was stirred at 80 °C for 16 hours. After the reaction solution was cooled to room temperature, it was filtered, the filter cake was washed with ethyl acetate (5 mL x3), and the filtrate was concentrated under reduced pressure. The crude product was purified by rapid silica gel column chromatography to obtain Compound **9-3. MS m/z (ESI):** 429.2 $[M+H]^+$.

Step 4: Synthesis of Compound **9**

Under nitrogen protection, bis(acetonitrile)palladium chloride (3 mg, 0.01 mmol) was added to a mixed solution of Compound **9-3** (20 mg, 0.05 mmol), cesium carbonate (37 mg, 0.11mmol), 2-dicyclohexylphosphino-2',4',6'-triisopropylbiphenyl (9 mg, 0.02 mmol) in acetonitrile (2 mL), and the reaction solution was stirred at 90 °C for 10 minutes. Compound **1-4** (41 mg, 0.19 mmol) was added to the reaction solution, and the reaction solution was stirred at 90 °C for 3

hours under nitrogen protection. After the reaction solution was cooled to room temperature, it was concentrated under reduced pressure. The crude product was purified by reverse phase flash chromatography to obtain Compound **9. MS m/z (ESI):** 611.3 [M+H]$^+$. **$^1$H NMR** (400 MHz, $CDCl_3$) δ 7.37 (d, *J* = 2.0 Hz, 1H), 7.29 (s, 1H), 7.22 (dd, *J* = 2.0, 8.0 Hz, 1H), 6.78 - 6.69 (m, 2H), 6.12 (d, *J* = 7.6 Hz, 1H), 6.01 (s, 1H), 5.05 (d, *J* = 8.0 Hz, 1H), 4.89 (t, *J* = 6.4 Hz, 1H), 4.75 (s, 4H), 4.29 (d, *J* = 6.4 Hz, 2H), 3.91 (s, 3H), 3.63 (d, *J* = 10.0 Hz, 2H), 3.39 (s, 6H), 3.00 (d, *J* = 4.8 Hz, 3H), 2.19 - 2.11 (m, 2H), 1.91 - 1.83 (m, 2H), 1.31 - 1.08 (m, 4H).

Preparation Example 10: Preparation of Compound **10**

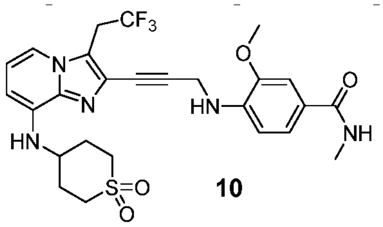

**10**

Synthesis scheme:

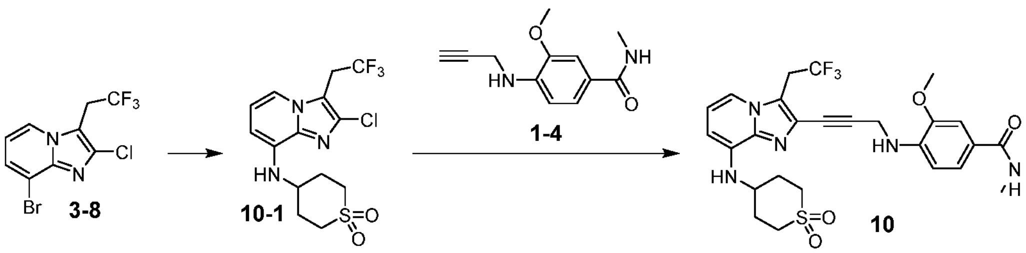

Step 1: Synthesis of Compound **10-1**

Under nitrogen protection, palladium acetate (15 mg, 0.07 mmol) was added to a mixed solution of Compound **3-8** (200 mg, 0.64 mmol), 4-aminotetrahydro-2H-thiopyran 1,1-dioxide (170 mg, 0.92 mmol), cesium carbonate (624 mg, 1.92 mmol) and 1,1'-binaphthyl-2,2'-bisdiphenylphosphine (40 mg, 0.06 mmol) in toluene (5 mL), and the reaction solution was stirred at 90 °C for 2 hours. After the reaction solution was cooled to room temperature, it was concentrated under reduced pressure. The crude product was purified by rapid silica gel column chromatography to obtain Compound **10-1. MS m/z (ESI):** 381.1 [M+H]$^+$.

Step 2: Synthesis of Compound **10:**

Under the protection of nitrogen, bis(acetonitrile)palladium chloride (13 mg, 0.05 mmol) was added to a mixed solution of Compound **10-1** (40 mg, 0.10 mmol), 2-dicyclohexylphosphino-2',4',6'-triisopropylbiphenyl (24 mg, 0.05 mmol) and cesium carbonate (88 mg, 0.27 mmol) in acetonitrile (2 mL), and the reaction solution was stirred at 90 °C for 20 minutes. Compound **1-4** (100 mg, 0.46 mmol) was added to the reaction solution. The reaction solution was stirred at 90 °C for 2 hours under the protection of nitrogen. After the reaction solution was cooled to room temperature, it was filtered, the filter cake was washed with acetonitrile (3 mL x3), and the filtrate was concentrated under reduced pressure. The crude product was purified by reverse phase flash chromatography to obtain Compound **10. MS m/z (ESI):** 564.2 [M+H]$^+$. **$^1$H NMR** (400 MHz, DMSO-$d_6$) δ 8.11 (d, *J* = 4.8 Hz, 1H), 7.75 (d, *J* = 6.8 Hz, 1H), 7.41 (d, *J* = 8.4 Hz, 1H), 7.34 (d, *J* = 2.0 Hz, 1H), 6.83 (t, *J* = 7.2 Hz, 1H), 6.73 (d, *J* = 8.4 Hz, 1H), 6.28 - 6.21 (m, 2H), 5.97 (t, *J* = 6.4 Hz, 1H), 4.26 (d, *J* = 6.4 Hz, 2H), 4.03 (d, *J* = 10.4 Hz, 2H), 3.84 (s, 3H), 3.83 - 3.79 (m, 1H), 3.29 - 3.23 (m, 2H), 3.16 - 3.11 (m, 2H), 2.75 (d, *J* = 4.4 Hz, 3H), 2.17 - 2.13 (m, 2H), 2.08 - 2.03 (m, 2H).

Preparation Example 11: Preparation of Compound **11**

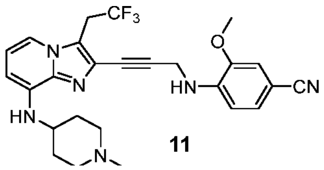

**11**

Synthesis scheme:

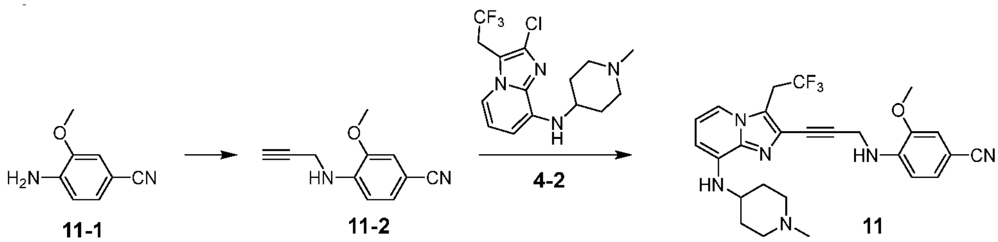


**11-1** **11-2** **11**

Step 1: Synthesis of Compound **11-2**

3-Bromopropyne (0.17 mL, 2.02 mmol) and potassium carbonate (560 mg, 4.05 mmol) were added to a solution of Compound **11-1** (200 mg, 1.35 mmol) in N,N-dimethylformamide (2 mL). The reaction mixture was stirred at 70 °C for 4 hours. The reaction mixture was poured into water (20 mL), extracted with ethyl acetate (10 mL x2), and the organic layers were combined and washed with saturated brine (10 mL), dried over anhydrous sodium sulfate, and filtered. The filtrate was concentrated under reduced pressure. The crude product was purified by rapid silica gel column chromatography to obtain Compound **11-1. MS m/z (ESI):** 187.3 $[M+H]^+$.

Step 2: Synthesis of Compound **11**

Compound **4-2** (54 mg, 0.29 mmol), bis(acetonitrile)palladium chloride (3.7 mg, 0.01 mmol), 2-dicyclohexylphosphino-2',4',6'-triisopropylbiphenyl (21 mg, 0.04 mmol) and cesium carbonate (61 mg, 0.19 mmol) were added to a solution of Compound **11-2** (25 mg, 0.07 mmol) in acetonitrile (1 mL). The reaction solution was stirred at 90 °C for 2 hours under nitrogen protection. After the reaction solution was cooled to room temperature, it was filtered, the filter cake was washed with acetonitrile (3 mL x3), and the filtrate was concentrated under reduced pressure. The crude product was purified by reverse phase flash chromatography to obtain Compound **11. MS m/z (ESI):** 497.2 $[M+H]^+$. **$^1$H NMR** (400 MHz, $CDCl_3$) δ 8.27 (brs, 1H), 7.27 (d, *J* = 6.8 Hz, 1H), 7.18 (d, *J* = 2.0 Hz, 1H), 6.89 (d, *J* = 2.0 Hz, 1H), 6.73 - 6.65 (m, 2H), 6.08 (d, *J* = 7.6 Hz, 1H), 5.12 (d, *J* = 7.2 Hz, 1H), 5.05 (t, *J* = 6.0 Hz, 1H), 4.24 (d, *J* = 6.0 Hz, 2H), 3.81 (s, 3H), 3.59 (q, *J* = 10.0 Hz, 2H), 3.52 - 3.48 (m, 1H), 3.03 - 2.97 (m, 2H), 2.73 - 2.42 (m, 5H), 2.20 - 1.86 (m, 2H), 1.80 - 1.77 (m, 2H).

Preparation Example 12: Preparation of Compound **12**

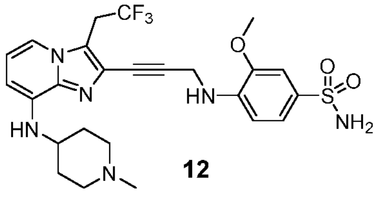

**12**

Synthesis scheme:

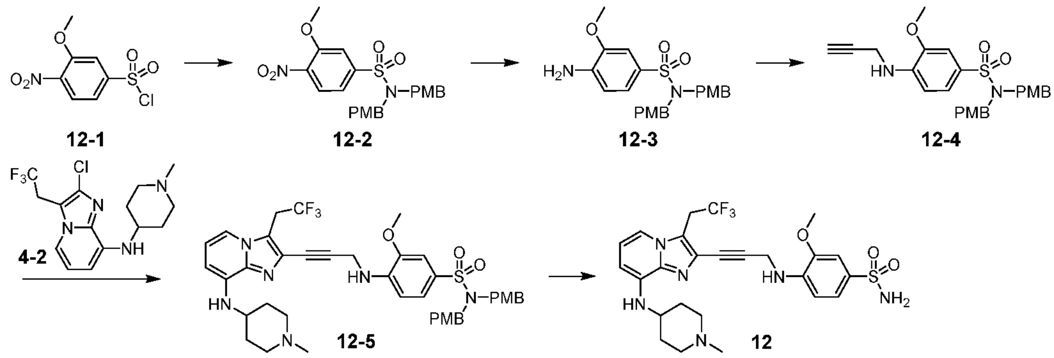

Step 1: Synthesis of Compound **12-2**

Compound **12-1** (900 mg, 3.58 **mmol) and** bis[(4-methoxyphenyl)methyl]amine (0.93 mL, 3.89 mmol) were dissolved in dichloromethane (10 mL), and N,N-diisopropylethylamine (0.7 mL, 4.22 mmol) was added. The mixture was stirred at 25°C for 2 hours. The mixture was diluted with dichloromethane (20 mL), washed with dilute hydrochloric acid (20 mL, 2M) and saturated brine (20 mL), dried over anhydrous sodium sulfate and filtered. The filtrate was concentrated under reduced pressure to obtain Compound **12-2.** $^1$**H NMR** (400 MHz, $CDCl_3$) δ 7.78 (d, *J* = 8.4 Hz, 1H), 7.36 - 7.30 (m, 1H), 7.28 (d, *J*= 1.8 Hz, 1H), 6.96 (d, *J* = 8.4 Hz, 4H), 6.72 (d, *J* = 8.4 Hz, 4H), 4.24 (s, 4H), 3.83 (s, 3H), 3.72 (s, 6H).

Step: Synthesis of Compound **12-3**

Iron powder (1.0 g, 17.91 mmol) was added to a mixed solution of Compound **12-2** (2.0 g, 4.23 mmol) and ammonium chloride (496 mg, 9.35 mmol) in water (10 mL) and ethanol (40 mL). The mixture was stirred at 75°C for 2 hours. After the reaction solution was cooled to room temperature, it was filtered, the filter cake was washed with ethanol (20 mL x3), and the filtrate was concentrated under reduced pressure. The crude product was purified by rapid silica gel column chromatography to obtain Compound **12-3. MS m/z (ESI):** 443.0 $[M+H]^+$.

Step 3: Synthesis of Compound **12-4**

3-Bromopropyne (0.54 mL, 6.32 mmol) was added to a mixed solution of Compound **12-3** (1.4 g, 3.16 mmol) and potassium carbonate (1.3 g, 9.41 mmol) in N,N-dimethylformamide (10 mL), and the reaction mixture was stirred at 75°C for 4 hours. The reaction mixture was diluted with water (20 mL), extracted with ethyl acetate (20 mL x 3), and the organic layers were combined and washed with saturated brine (50 mL), dried over anhydrous sodium sulfate, and filtered. The filtrate was concentrated under reduced pressure. The crude product was purified by rapid silica gel column chromatography to obtain Compound **12-4.** $^1$**H NMR** (400 MHz, $CDCl_3$) δ 7.38 (dd, *J = 2.0,* 8.4 Hz, 1H), 7.04 (d, *J* = 2.0 Hz, 1H), 6.98 - 6.87 (m, 4H), 6.75 - 6.66 (m, 4H), 6.62 (d, *J* = 8.4 Hz, 1H), 4.14 (s, 4H), 4.06 (d, *J* = 6.8 Hz, 2H), 3.96 (s, 1H), 3.74 (s, 3H), 3.71 (s, 6H).

Step 4: Synthesis of Compound **12-5**

Under nitrogen protection, a mixture of Compound **12-4** (40 mg, 0.12 mmol), 2-dicyclohexylphosphino-2',4',6'-triisopropylbiphenyl (22 mg, 0.05 mmol), bis(acetonitrile)palladium chloride (6 mg, 0.02 mmol), cesium carbonate (80 mg, 0.25 mmol) and acetonitrile (1 mL) was stirred at 90°C for 20 minutes. Compound **4-2** (220 mg, 0.46 mmol) was added to the reaction solution, and the reaction solution was stirred at 90°C for 2 hours. After the reaction solution was cooled to room temperature, it was filtered, the filter cake was washed with acetonitrile (5 mL x3), and the filtrate was concentrated under reduced pressure. The crude product was purified by rapid silica gel column chromatography to obtain Compound **12-5. MS m/z (ESI):** 791.2 $[M+H]^+$.

Step 5: Synthesis of Compound **12**

Compound **12-5** (25 mg, 0.03 mmol) was dissolved in trifluoroacetic acid (0.5 mL), and the reaction solution was

stirred at 25°C for 2 hours. The mixture was concentrated under reduced pressure and purified by reverse phase flash chromatography to obtain Compound **12. MS m/z (ESI):** 551.2 [M+H]$^+$. **$^1$H NMR** (400 MHz, $CD_3OD$) δ 7.54 (d, *J* = 6.8 Hz, 1H), 7.36 (dd, *J* = 2.0, 8.4 Hz, 1H), 7.23 (d, *J* = 2.0 Hz, 1H), 6.82 - 6.73 (m, 2H), 6.27 (d, *J* = 7.6 Hz, 1H), 4.25 (s, 2H), 3.85 - 3.72 (m, 5H), 3.04 - 3.00 (m, 1H), 2.60 - 2.55 (m, 2H), 2.45 (s, 3H), 2.12 - 2.07 (m, 2H), 1.70 - 1.62 (m, 2H), 1.53 - 1.49 (m, 2H).

Preparation Example 13: Preparation of Compound **13**

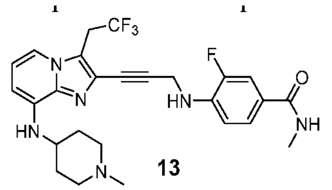

**13**

Synthesis scheme:

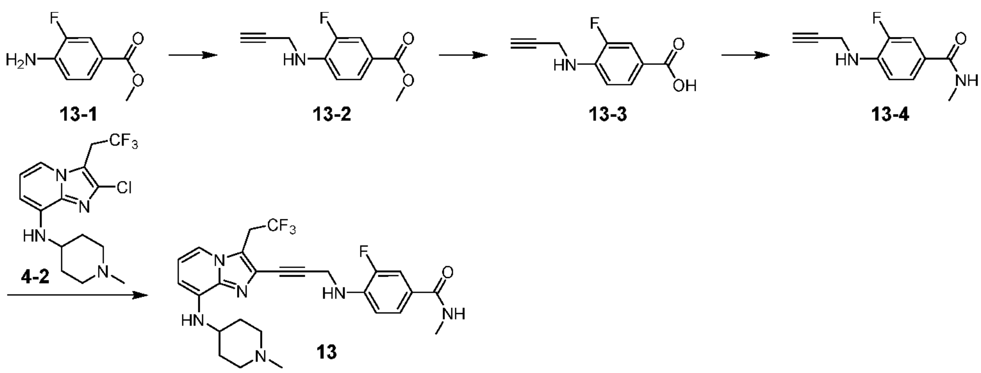

**13-1** **13-2** **13-3** **13-4** **4-2** **13**

Step 1: Synthesis of Compound **13-2**

3-Bromopropyne (1.22 mL, 14.19 mmol) and potassium carbonate (4.90 g, 35.47 mmol) were added to a solution of Compound **13-1** (1.59 mL, 11.82 mmol) in N,N-dimethylformamide (10 mL). The reaction solution was stirred at 70°C for 18 hours. After the reaction solution was cooled to room temperature, it was diluted with water (50 mL), extracted with ethyl acetate (30 mL x3), and the organic layers were combined and washed with saturated brine (100 mL), dried over anhydrous sodium sulfate, and filtered. The filtrate was concentrated under reduced pressure. The crude product was purified by rapid silica gel column chromatography to obtain Compound **13-2. MS m/z (ESI):** 208.0 [M+H]$^+$.

Step 2: Synthesis of Compound **13-3**

Lithium hydroxide (920 mg, 21.93 mmol) was added to a mixed solution of Compound **13-2** (900 mg, 4.34 mmol) in methanol (13.5 mL), water (4.5 mL) and tetrahydrofuran (4.5 mL), and the reaction solution was stirred at 45°C for 18 hours. The reaction solution was quenched with dilute hydrochloric acid (40 mL, 1M), extracted with ethyl acetate (30 mL x3), and the organic layers were combined and washed with saturated brine (100 mL), dried over anhydrous sodium sulfate and filtered. The filtrate was concentrated under reduced pressure to obtain Compound **13-3. MS m/z (ESI):** 194.0 [M+H]$^+$.

Step 3: Synthesis of Compound **13-4**

Methylamine (0.31 g, 4.56 mmol), N,N-diisopropylethylamine (3.5 mL, 21.12 mmol) and 2-(7-azabenzotriazole)-N,N,N',N'-tetramethyluronium hexafluorophosphate (1.89 g, 4.97 mmol) were added to a solution of Compound **13-3** (0.80 g, 4.14 mmol) in N,N-dimethylformamide (16 mL), and the reaction solution was stirred at 25°C for 2 hours. The

reaction solution was diluted with water (5 mL), extracted with ethyl acetate (20 mL x3), and the organic layers were combined and dried over anhydrous sodium sulfate and filtered, and the filtrate was concentrated under reduced pressure. The crude product was purified by rapid silica gel column chromatography to obtain Compound **13-4. MS m/z (ESI):** = 207.2 $[M+H]^+$.

Step 4: Synthesis of Compound **13**

Cesium carbonate (70 mg, 0.22mmol), 2-dicyclohexylphosphino-2',4',6'-triisopropylbiphenyl (16 mg, 0.03 mol) and bis(acetonitrile)palladium chloride (5 mg, 0.02mmol) were added to a solution of Compound **4-2** (30 mg, 0.09 mmol) in acetonitrile (1 mL), and the reaction solution was stirred at 90°C for 10 minutes under nitrogen protection. Compound **13-4** (75 mg, 0.36mmol) was added to the reaction solution, and the reaction solution was stirred at 90°C for 4 hours under nitrogen protection. After the reaction solution was cooled to room temperature, it was filtered, the filter cake was washed with acetonitrile (5 mL x3), and the filtrate was concentrated under reduced pressure. The crude product was purified by reverse phase flash chromatography to obtain Compound **13. MS m/z (ESI):** 517.6 $[M+H]^+$. **$^1$H NMR** (400 MHz, $CDCl_3$) δ 7.48 - 7.35 (m, 2H), 7.23 (d, *J* = 6.8 Hz, 1H), 6.80 (t, *J* = 8.4 Hz, 1H), 6.68 (t, *J* = 7.2 Hz, 1H), 6.07 (d, *J* = 7.6 Hz, 1H), 5.91 (brs, 1H), 5.09 (d, *J* = 7.6 Hz, 1H), 4.53 (brs, 1H), 4.25 (d, *J* = 6.4 Hz, 2H), 3.58 (q, *J* = 10.0 Hz, 2H), 3.38 - 3.32 (m, 1H), 2.96 (d, *J* = 4.8 Hz, 3H), 2.84 - 2.80 (m, 2H), 2.28 (s, 3H), 2.21 - 1.95 (m, 4H), 1.26 - 1.22 (m, 2H).

Preparation Example 14: Preparation of Compound **14**

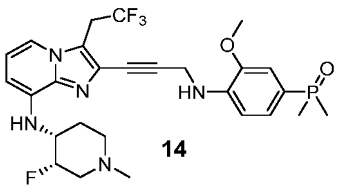

**14**

Synthesis scheme:

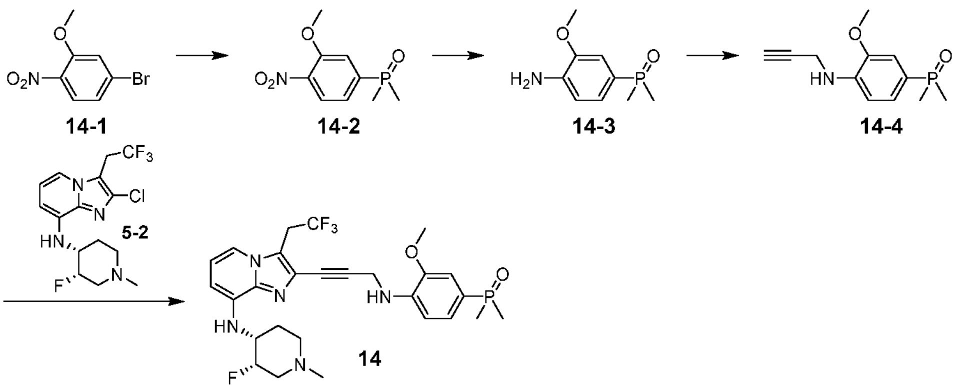

**14-1** **14-2** **14-3** **14-4** **5-2** **14**

Step 1: Synthesis of Compound **14-2**

Under nitrogen protection, potassium phosphate (2.01 mg, 9.48 mmol), 4,5-bis(diphenylphosphino)-9,9-dimethyloxaanthracene (499 mg, 0.86 mmol) and palladium acetate (194 mg, 0.86 mmol) were added in sequence to a solution of Compound **14-1** (2.0 g, 8.62 mmol) and dimethylphosphine oxide (740 mg, 9.48 mmol) in N,N-dimethylformamide (20 mL). The mixture was stirred at 130°C for 12 hours under nitrogen protection. After the reaction solution was cooled to room temperature, it was filtered, the filter cake was washed with dichloromethane (30 mL x2), the filtrate was diluted with water (100 mL), extracted with dichloromethane (100 mL x3), the organic phases were combined and washed with saturated brine (100 mL), and the solution was concentrated under reduced pressure. The crude product was purified by rapid silica gel column chromatography to obtain Compound **14-2. MS m/z (ESI):** 229.8 $[M+H]^+$. **$^1$H NMR** (400 MHz, $CDCl_3$) δ 7.89 (d, *J* = 8.0 Hz, 1H), 7.68 (dd, *J* = 1.6 Hz, 1H), 7.19 (dd, *J* = 1.6, 8.0 Hz, 1H), 4.05 (s, 3H), 1.81 (s, 3H), 1.78 (s,

3H).

Step 2: Synthesis of Compound **14-3**

Iron powder (1660 mg, 29.67 mmol) and ammonium chloride (1590 mg, 29.67 mmol) were added to a solution of Compound **14-2** (1700 mg, 7.42 mmol) in ethanol (30 mL). The mixture was stirred at 80°C for 2 hours under nitrogen protection. After the reaction solution was cooled to room temperature, it was filtered, the filter cake was washed with methanol (30 mL x2), and the filtrate was concentrated under reduced pressure. The crude product was dissolved in dichloromethane (300 ml) and filtered, and the filtrate was concentrated under reduced pressure to obtain Compound **14-3. MS m/z (ESI):** 199.8 $[M+H]^+$. **$^1$H NMR** (400 MHz, DMSO-$d_6$) δ 7.12 - 7.00 (m, 2H), 6.69 (dd, *J* = 3.2, 8.0 Hz, 1H), 5.26 (brs, 2H), 3.80 (s, 3H), 1.57 (s, 3H), 1.53 (s, 3H).

Step 3: Synthesis of Compound **14-4**

Propargyl bromide (0.65 mL, 7.53 mmol) and potassium carbonate (2.08 g, 15.06 mmol) were added to a solution of Compound **14-3** (1.0 g, 5.02 mmol) in N,N-dimethylformamide (15 mL). The mixture was stirred at 80 °C for 2 hours under nitrogen protection. After the reaction solution was cooled to room temperature, it was filtered, the filter cake was washed with dichloromethane (30 mL x2), the filtrate was diluted with water (50 mL), extracted with dichloromethane (30 mL x3), the organic phases were combined and washed with saturated brine (100 mL), and the solution was concentrated under reduced pressure. The crude product was purified by rapid silica gel column chromatography to obtain Compound **14-4. $^1$H NMR** (400 MHz, $CDCl_3$) δ 7.15 (d, *J* = 1.6 Hz, 1H), 7.05 (dd, *J* = 1.6, 8.0 Hz, 1H), 6.67 (d, *J* = 8.0 Hz, 1H), 4.83 - 4.65 (m, 1H), 3.94 (dd, *J* = 2.4, 6.4 Hz, 2H), 3.84 (s, 3H), 2.17 (t, *J* = 2.4 Hz, 1H), 1.65 (s, 3H), 1.62 (s, 3H).

Step 4: Synthesis of Compound **14**

Under nitrogen protection, a mixed solution of Compound **5-2** (100 mg, 0.26 mmol), 2-dicyclohexylphosphino-2',4',6'-triisopropylbiphenyl (32 mg, 0.07 mmol), cesium carbonate (72 mg, 0.22 mmol) and bis(acetonitrile)palladium chloride (6.0 mg, 0.02 mmol) in acetonitrile (3.0 mL) was stirred at 25 °C for 10 minutes. Compound **14-4** (120 mg, 0.55 mmol) was added to the reaction solution under nitrogen protection, and the mixture was stirred at 90 °C for 2 hours. After the reaction solution was cooled to room temperature, it was filtered, the filter cake was washed with acetonitrile (5 mL x3), and the filtrate was concentrated under reduced pressure, and purified by rapid silica gel column chromatography to obtain Compound **14. MS m/z (ESI):** 566.3 $[M+H]^+$. **$^1$H NMR** (400 MHz, DMSO-$d_6$) δ 7.78 (d, *J* = 6.8 Hz, 1H), 7.25 - 7.18 (m, 1H), 7.13 (d, *J* = 8.0 Hz, 1H), 6.86 - 6.79 (m, 2H), 6.39 (d, *J* = 7.6 Hz, 1H), 6.02 (brs, 1H), 5.38 (d, *J* = 9.2 Hz, 1H), 4.82 (d, *J* = 49.2 Hz, 1H), 4.27 (d, *J* = 6.4 Hz, 2H), 4.00 (q, *J* = 10.8 Hz, 2H), 3.85 (s, 3H), 3.78 - 3.60 (m, 1H), 3.03 (t, *J* = 11.2 Hz, 1H), 2.79 - 2.75 (m, 1H), 2.32 - 2.28 (m, 1H), 2.19 (s, 3H), 2.14 - 2.04 (m, 1H), 1.88 - 1.73 (m, 2H), 1.59 (s, 3H), 1.55 (s, 3H).

Preparation Example 15: Preparation of Compound **15**

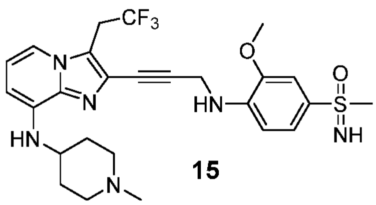

**15**

Synthesis scheme:

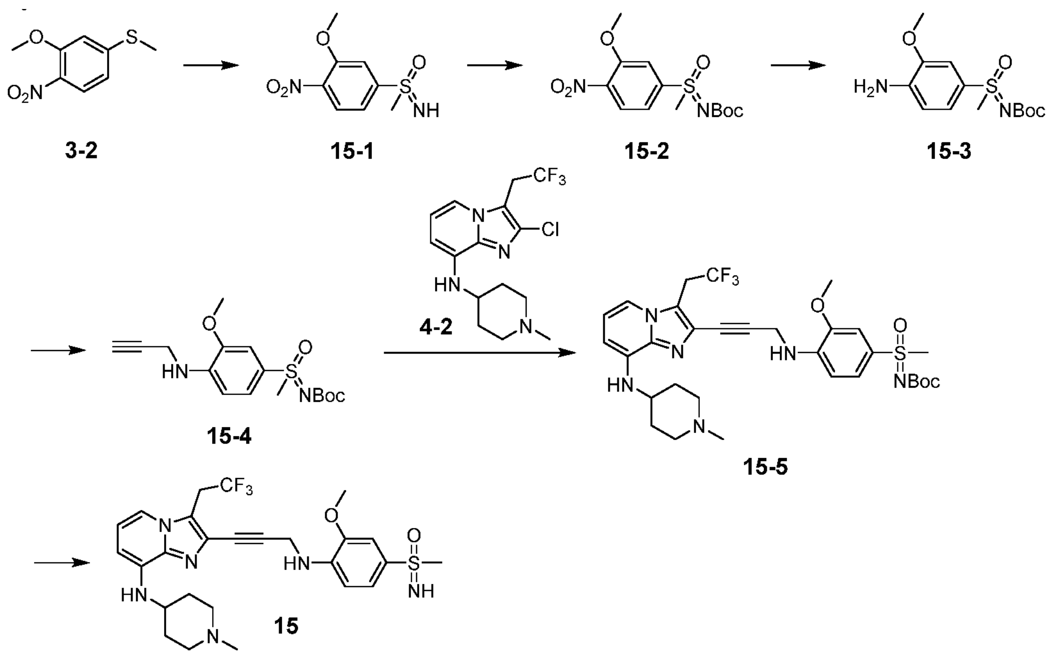

3-2 15-1 15-2 15-3

4-2

15-4 15-5

15

Step 1: Syntnesis or Compound **15-1**

Iodosobenzene diacetate (10.99 g, 34.13 mmol) was added to a solution of Compound **3-2** (3.4 g, 17.07 mmol) and ammonium formate (4.17 g, 68.27 mmol) in methanol (50 mL), and the reaction solution was stirred in an open state at 25 °C for 1 hour. The reaction solution was concentrated under reduced pressure and then diluted with water (40 mL), extracted with ethyl acetate (30 mL x3), the organic layers were combined and washed with saturated brine (80 mL), dried over anhydrous sodium sulfate and filtered, and the filtrate was concentrated under reduced pressure to obtain Compound 15-1. **$^1$H NMR** (400 MHz, $CDCl_3$) δ 7.90 (d, *J*= 8.4 Hz, 1H), 7.64 (d, *J* = 1.6 Hz, 1H), 7.54 (dd, *J* = 1.6, 8.4 Hz, 1H), 3.98 (s, 3H), 3.19 (s, 3H).

Step 2: Synthesis of Compound **15-2**

Potassium tert-butoxide (2.63 g, 23.45 mmol) was added to a solution of Compound **15-1** (2.70 g, 11.73 mmol) and di-tert-butyl dicarbonate (5.02 mL, 23.45 mmol) in tetrahydrofuran (30 mL), and the reaction solution was stirred at 25°C for 2 hours. The reaction solution was diluted with water (100 mL), extracted with ethyl acetate (50 mL x 3), and the organic layers were combined and washed with saturated brine (80 mL), dried over anhydrous sodium sulfate, and filtered. The filtrate was concentrated under reduced pressure. The crude product was purified by rapid silica gel column chromatography to obtain Compound **15-2. $^1$H NMR** (400 MHz, $CDCl_3$) δ 7.96 (d, *J* = 8.4 Hz, 1H), 7.70 (d, *J* = 1.6 Hz, 1H), 7.61 (dd, *J* = 1.6, 8.4 Hz, 1H), 4.04 (s, 3H), 3.26 (s, 3H), 1.41 (s, 9H).

Step 3: Synthesis of Compound **15-3**

Iron powder (1.15 g, 20.58 mmol) and ammonium chloride (1.10 g, 20.58 mmol) were added to a mixed solution of Compound **15-2** (1.7 g, 5.15 mmol) in ethanol (30 mL) and water (10 mL). The mixture was stirred at 80°C for 2 hours. After the reaction solution was cooled to room temperature, it was filtered, the filter cake was washed with methanol (50 mL x2), and the filtrate was concentrated under reduced pressure. The crude product was dissolved in dichloromethane (200 ml) and filtered, and the filtrate was concentrated under reduced pressure to obtain Compound **15-3. MS m/z (ESI):** 301.2 $[M+H]^+$.

Step 4: Synthesis of Compound **15-4**

Compound **15-3** (1500 mg, 4.99 mmol), 3-bromopropyne (2 mL, 23.20 mmol) and potassium carbonate (2100 mg, 15.20 mmol) were dissolved in N,N-dimethylformamide (15 mL), and the reaction solution was stirred at 75°C for 2 hours. After the reaction solution was cooled to room temperature, it was poured into water (100 mL), extracted with ethyl acetate (50 mL x3), and the organic layers were combined and washed with saturated brine (100 mL), dried over

anhydrous sodium sulfate, filtered, and the filtrate was concentrated under reduced pressure. The crude product was purified by rapid silica gel column chromatography to obtain Compound **15-4.** **$^1$H NMR** (400 MHz, $CDCl_3$) δ 7.46 (dd, *J* = 2.0, 8.4 Hz, 1H), 7.20 (d, *J* = 2.4 Hz, 1H), 6.68 (d, *J* = 8.4 Hz, 1H), 4.97 (brs, 1H), 3.96 (s, 2H), 3.84 (s, 3H), 3.14 (s, 3H), 2.27 (s, 1H), 1.35 (s, 9H).

Step 5: Synthesis of Compound **15-5**

Under nitrogen protection, acetonitrile (2 mL) was added to a mixture of Compound **4-2** (90 mg, 0.26 mmol), bis(acetonitrile)palladium chloride (14 mg, 0.05 mmol), 2-dicyclohexylphosphino-2',4',6'-triisopropylbiphenyl (50 mg, 0.10 mmol) and cesium carbonate (170 mg, 0.52 mmol), and the reaction solution was stirred at 90°C for 20 minutes. Compound **15-4** (180 mg, 0.53 mmol) was added to the reaction solution. The reaction solution was stirred at 90°C for 2 hours. After the reaction solution was cooled to room temperature, it was concentrated under reduced pressure, and the crude product was purified by rapid silica gel column chromatography to obtain Compound **15-5. MS m/z (ESI):** 649.2 $[M+H]^+$.

Step 6: Synthesis of Compound **15**

Trifluoroacetic acid (0.5 mL) was added to a solution of Compound **15-5** (10 mg, 0.02 mmol) in dichloromethane (2 mL), and the reaction solution was stirred at 25°C for 2 hours. The reaction solution was quenched with saturated sodium bicarbonate solution, extracted with ethyl acetate (20 mL x 3), and the combined organic phase was concentrated under reduced pressure. The crude product was purified by reverse phase flash chromatography to obtain Compound **15. MS m/z (ESI):** 549.2 $[M+H]^+$. **$^1$H NMR** (400 MHz, $CD_3OD$) δ 7.64 (d, *J* = 6.8 Hz, 1H), 7.57 (dd, *J* = 2.0, 8.4 Hz, 1H), 7.40 (d, *J* = 2.0 Hz, 1H), 6.98 (d, *J* = 8.4 Hz, 1H), 6.87 (t, *J* = 7.2 Hz, 1H), 6.38 (d, *J* = 7.6 Hz, 1H), 4.40 (s, 2H), 3.97 (s, 3H), 3.95 - 3.84 (m, 2H), 3.62 - 3.59 (m, 1H), 3.13 (s, 3H), 3.02 - 2.95 (m, 2H), 2.55 - 2.50 (m, 2H), 2.45 (s, 3H), 2.18 - 2.14 (m, 2H), 1.74 - 1.70 (m, 2H).

Preparation Example 16: Preparation of Compound **16**

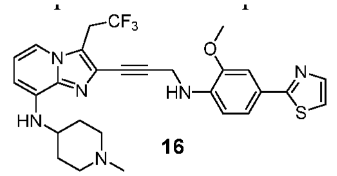

**16**

Synthesis scheme:

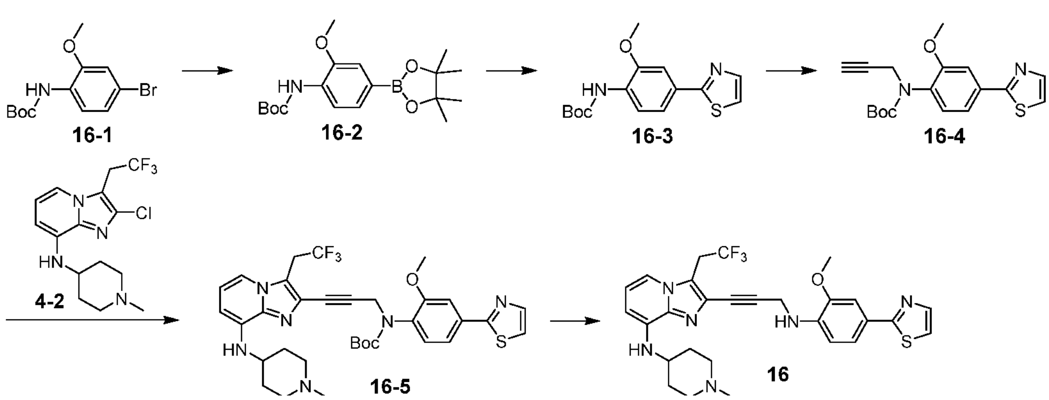

**16-1** **16-2** **16-3** **16-4** **4-2** **16-5** **16**

Step 1: Synthesis of Compound **16-2**

Bis(pinacolato)diboron (2.52 g, 9.93 mmol), (1,1'-bis(diphenylphosphino) ferrocene)palladium dichloride (0.48 g, 0.66 mmol) and potassium acetate (1.95 g, 19.86 mmol) were added to a solution of Compound **16-1** (2.00 g, 6.62 mmol)

in N,N-dimethylformamide (24 mL), and the reaction solution was stirred at 100°C for 18 hours under nitrogen protection. After the reaction solution was cooled to room temperature, water (150 mL) was added and the mixture was extracted with ethyl acetate (80 mL x2). The organic layers were combined and washed with saturated brine (100 mL), dried over anhydrous sodium sulfate and filtered. The filtrate was concentrated under reduced pressure. The crude product was purified by rapid silica gel column chromatography to obtain Compound **16-2.** **$^1$H NMR** (400 MHz, DMSO-*$d_6$*) δ 7.94 (s, 1H), 7.82 (d, *J* = 8.0 Hz, 1H), 7.24 (dd, *J* = 1.2, 8.0 Hz, 1H), 7.16 (d, *J* = 1.2 Hz, 1H), 3.82 (s, 3H), 1.46 (s, 9H), 1.28 (s, 12H).

Step 2: Synthesis of Compound **16-3**

Compound **16-2** (1.96 g, 5.61 mmol) was dissolved in water (6 mL) and 1,4-dioxane (24 mL), and 2-bromo-1,3-thiazole (1.01 mL, 11.22 mmol), tetrakis(triphenylphosphine)palladium (0.65 g, 0.56 mmol) and potassium phosphate (3.57 g, 16.84 mmol) were added. The reaction solution was stirred at 100°C for 18 hours under nitrogen protection. After the reaction solution was cooled to room temperature, water (50 mL) was added and the mixture was extracted with ethyl acetate (30 mL x2). The organic layers were combined and washed with saturated brine (50 mL), dried over anhydrous sodium sulfate, and filtered. The filtrate was concentrated under reduced pressure. The crude product was purified by rapid silica gel column chromatography to obtain Compound **16-3. MS m/z (ESI):** 307.0 $[M+H]^+$.

Step 3: Synthesis of Compound **16-4**

In an ice bath, sodium hydride (0.34 g, 8.49 mmol, 60%) was added to a solution of Compound **16-3** (1.3 g, 4.24 mmol) in N,N-dimethylformamide (20 mL), and the reaction solution was stirred at 0°C for 0.5 hours under nitrogen protection. 3-Bromoprop-1-yne (0.73 mL, 8.49 mmol) was added to the reaction solution and the stirring was continued at 0°C for 1 hour. The reaction solution was quenched with saturated ammonium chloride aqueous solution (20 mL), diluted with water (50 mL) and extracted with ethyl acetate (30 mL x2). The organic layers were combined and washed with saturated brine (50 mL), dried over anhydrous sodium sulfate and filtered. The filtrate was concentrated under reduced pressure. The crude product was purified by rapid silica gel column chromatography to obtain Compound **16-4. MS m/z (ESI):** 345.0 $[M+H]^+$.

Step 4: Synthesis of Compound **16-5**

Compound **4-2** (28 mg, 0.08 mmol) was dissolved in acetonitrile (1.5 mL), and cesium carbonate (65 mg, 0.20 mmol), bis(acetonitrile)palladium chloride (5 mg, 0.02 mmol) and 2-dicyclohexylphosphino-2',4',6'-triisopropylbiphenyl (16 mg, 0.03 mmol) were added. The reaction solution was stirred at 90°C for 10 minutes under nitrogen protection. Compound **16-4** (110 mg, 0.32 mmol) was added to the reaction solution and the stirring was continued at 90°C for 2 hours. After the reaction solution was cooled to room temperature, it was filtered, the filter cake was washed with acetonitrile (10 mL x2), and the filtrate was concentrated under reduced pressure to obtain Compound **16-5. MS m/z (ESI):** 655.2 $[M+H]^+$.

Step 5: Synthesis of Compound **16**

Trifluoroacetic acid (2 mL, 26.84 mmol) was added to a solution of Compound **16-5** (40 mg, 0.06 mmol) in dichloromethane (4 mL). The reaction solution was stirred at 25°C for 0.5 h under nitrogen protection. The mixture was concentrated under reduced pressure and purified by reverse phase flash chromatography to obtain Compound **16. MS m/z (ESI):** 555.3 $[M+H]^+$. **$^1$H NMR** (400 MHz, $CDCl_3$) δ 7.70 (d, *J* = 3.2 Hz, 1H), 7.45 - 7.37 (m, 2H), 7.22 (d, *J* = 6.8 Hz, 1H), 7.13 (d, *J* = 3.2 Hz, 1H), 6.76 - 6.64 (m, 2H), 6.06 (d, *J* = 7.6 Hz, 1H), 5.10 (d, *J* = 7.6 Hz, 1H), 4.80 (t, *J* = 6.4 Hz, 1H), 4.24 (d, *J* = 6.4 Hz, 2H), 3.88 (s, 3H), 3.66 - 3.54 (m, 2H), 3.41 - 3.37 (m, 1H), 2.84 - 2.81 (m, 2H), 2.28 (s, 3H), 2.17 - 2.02 (m, 4H), 1.70 - 1.58 (m, 2H).

Preparation Example 17: Preparation of Compound **17**

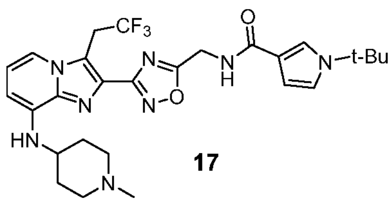

**17**

Synthesis scheme:

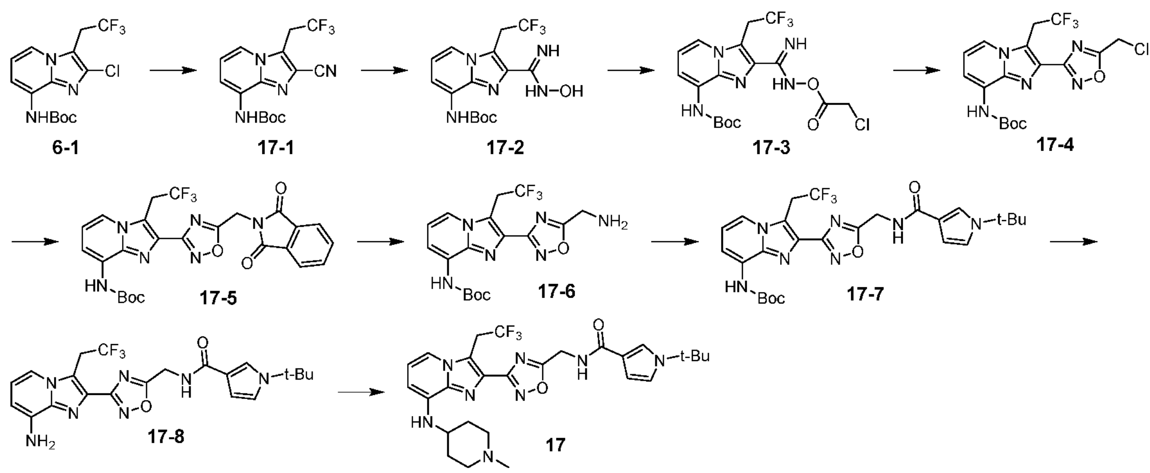

Step 1: Synthesis of Compound **17-1**

(2-Dicyclohexylphosphino-2',4',6'-triisopropyl-1,1'-biphenyl)(2'-amino-1,1'-biphenyl-2-yl) palladium(II) methanesulfonate (0.54 g, 0.64 mmol) and 2-dicyclohexylphosphino-2',4',6'-triisopropylbiphenyl (0.29 g, 0.60 mmol) were added to a solution of Compound 6-1 (3.0 g, 8.58 mmol), potassium ferrocyanide (3.16 g, 8.58 mmol) and potassium acetate (1.26 g, 12.87 mmol) in 1,4-dioxane (30 mL) and water (30 mL). The reaction solution was stirred at 100°C for 4 hours under nitrogen protection. After the reaction solution was cooled to room temperature, it was poured into water (50 mL) and extracted with ethyl acetate (50 mL x2). The organic layers were combined and washed with saturated brine (50 mL) and dried over anhydrous sodium sulfate, then filtered, and the filtrate was concentrated under reduced pressure. The crude product was purified by rapid silica gel column chromatography to obtain Compound **17-1. MS m/z (ESI):** 341.1 $[M+H]^+$. **$^1$H NMR** (400 MHz, $CDCl_3$) δ 7.92 (d, *J* = 7.6 Hz, 1H), 7.74 (s, 1H), 7.62 (d, *J* = 6.8 Hz, 1H), 6.95 (t, *J* = 7.2 Hz, 1H), 3.83 (q, *J* = 9.6 Hz, 2H), 1.48 (s, 9H).

Step 2: Synthesis of Compound **17-2**

Sodium carbonate (156 mg, 1.47 mmol) was added to a mixture of Compound **17-1** (500 mg, 1.47 mmol) and hydroxylamine hydrochloride (102 mg, 1.47 mmol) in ethanol (5 mL). The reaction solution was stirred at 90°C for 16 hours. After the reaction solution was cooled to room temperature, it was filtered, the filter cake was washed with ethanol (10 mL x2), and the filtrate was concentrated under reduced pressure to obtain Compound **17-2. MS m/z (ESI):** 374.2 $[M+H]^+$.

Step 3: Synthesis of Compound **17-3**

Chloroacetyl chloride (1.33 g, 11.80 mmol) was added to a solution of Compound **17-2** (2.20 g, 5.9 mmol) and sodium carbonate (0.63 g, 5.90 mmol) in acetone (20 mL) at room temperature. The reaction solution was stirred at room temperature for 30 minutes. The reaction mixture was quenched with water (50 mL), extracted with ethyl acetate (20 mL x3), the organic layers were combined and washed with saturated brine (50 mL), dried over anhydrous sodium sulfate, and filtered. The filtrate was concentrated under reduced pressure to obtain Compound **17-3. MS m/z (ESI):** 450.1 $[M+H]^+$.

Step 4: Synthesis of Compound **17-4**

Compound **17-3** (2.65 g, 5.9 mmol) was dissolved in toluene (30 mL). The reaction mixture was stirred at 110°C for 1 hour. After the reaction mixture was cooled to room temperature, it was filtered, the filter cake was washed with toluene (10 mL x2), and the filter cake was evaporated under reduced pressure to obtain Compound **17-4. MS m/z (ESI):** 432.0 $[M+H]^+$.

Step 5: Synthesis of Compound **17-5**

Potassium phthalimide (0.90 g, 4.86 mmol) was added to a solution of Compound **17-4** (2.0 g, 4.63 mmol) in N,N-dimethylformamide (20 mL). The reaction solution was stirred at room temperature for 1 hour. The reaction solution was filtered, the filter cake was washed with ethyl acetate (10 mL x2), and the filtrate was evaporated to dryness under reduced pressure to obtain Compound **17-5. MS m/z (ESI):** 543.2 $[M+H]^+$.

Step 6: Synthesis of Compound **17-6**

Hydrazine hydrate (180 mg, 3.69 mmol) was added to a solution of Compound **17-5** (2.0 g, 3.69 mmol) in ethanol (20 mL), and the reaction solution was stirred at 90°C for 1 hour. After the reaction solution was cooled to room temperature, it was filtered, and the filtrate was concentrated under reduced pressure. The crude product was purified by rapid silica gel column chromatography to obtain Compound **17-6. MS m/z (ESI):** 413.1 $[M+H]^+$.

Step 7: Synthesis of Compound **17-7**

1-Tert-butyl-1H-pyrrole-3-carboxylic acid (81 mg, 0.49 mmol), 2-(7-azabenzotriazole)-N,N,N',N'-tetramethyluronium hexafluorophosphate (277 mg, 0.73 mmol) and N,N-diisopropylethylamine (0.24 mL, 1.46 mmol) were added to a solution of Compound **17-6** (200 mg, 0.49 mmol) in tetrahydrofuran (2 mL), and the reaction solution was stirred at room temperature for 3 hours. The reaction solution was diluted with water (10 mL), extracted with ethyl acetate (10 mL x3), and the organic layers were combined and washed with saturated brine (20 mL), dried over anhydrous sodium sulfate, and filtered. The filtrate was concentrated under reduced pressure. The crude product was purified by rapid silica gel column chromatography to obtain Compound **17-7. MS m/z (ESI):** 562.2 $[M+H]^+$.

Step 8: Synthesis of Compound **17-8**

Trifluoroacetic acid (0.4 mL) was added to a solution of Compound **17-7** (110 mg, 0.20 mmol) in dichloromethane (2 mL), and the reaction solution was stirred at 25°C for 10 hours. The reaction solution was concentrated under reduced pressure to obtain Compound **17-8. MS m/z (ESI):** 461.8 $[M+H]^+$.

Step 9: Synthesis of Compound **17**

Under nitrogen protection, trimethylsilyl chloride (0.20 mL, 1.56 mmol) was added to a solution of Compound **17-8** (90 mg, 0.20 mmol) and N-methyl-4-piperidone (176 mg, 1.56 mmol) in N,N-dimethylformamide (3 mL), and the reaction solution was stirred at 45 °C for 2 hours. After the reaction solution was cooled to room temperature, borane tetrahydrofuran (1.5 mL, 1.5 mmol) was added dropwise to the reaction solution and stirred at room temperature for 2 hours. The reaction solution was quenched with water (15 mL), extracted with ethyl acetate (15 mL x2), the organic phases were combined and washed with saturated brine (20 mL), dried over anhydrous sodium sulfate, filtered, and the filtrate was concentrated under reduced pressure. The crude product was purified by reverse phase flash chromatography to obtain Compound **17. MS m/z (ESI):** 559.1 $[M+H]^+$. **$^1$H NMR** (400 MHz, DMSO-$d_6$) δ 8.67 (t, *J* = 5.6 Hz, 1H), 7.88 (d, *J* = 6.8 Hz, 1H), 7.55 (t, *J* = 2.0 Hz, 1H), 7.00 (t, *J* = 2.8 Hz, 1H), 6.90 (t, *J* = 7.2 Hz, 1H), 6.50 (dd, *J* = 1.6, 3.0 Hz, 1H), 6.32 (d, *J* = 7.6 Hz, 1H), 5.77 (d, *J* = 8.4 Hz, 1H), 4.75 (d, *J* = 5.6 Hz, 2H), 4.53 (q, *J* = 10.4 Hz, 2H), 3.47 - 3.42 (m, 1H), 2.81 - 2.76 (m, 2H), 2.21 (s, 3H), 2.12 - 2.05 (m, 2H), 1.95 - 1.88 (m, 2H), 1.67 - 1.58 (m, 2H), 1.50 (s, 9H).

Preparation Example 18: Preparation of Compound **18**

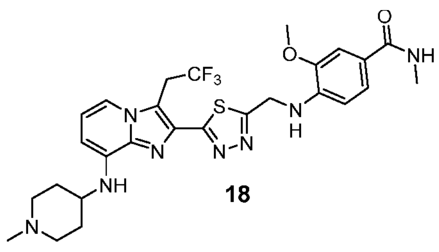

**18**

Synthesis scheme:

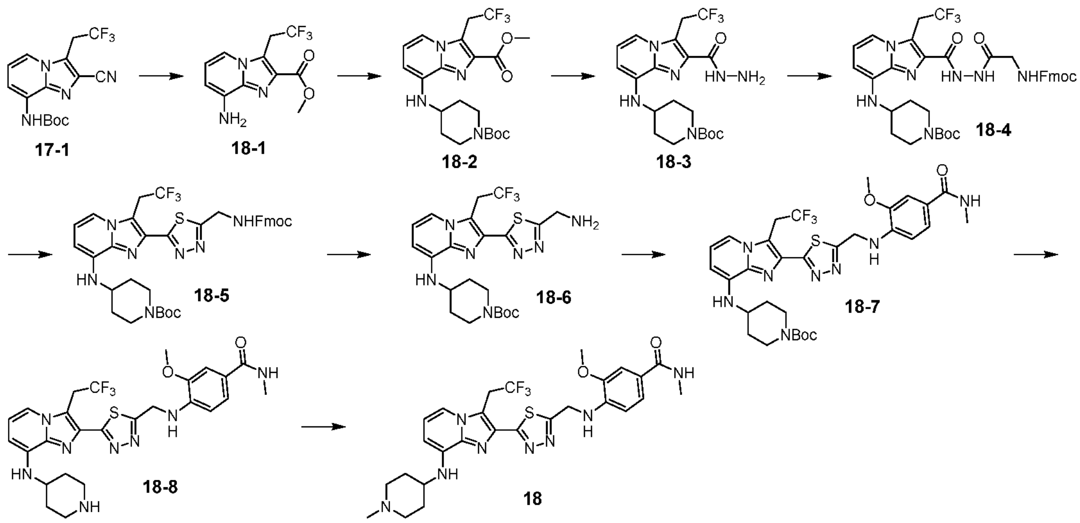

17-1, 18-1, 18-2, 18-3, 18-4, 18-5, 18-6, 18-7, 18-8, 18

Step 1: Synthesis of Compound **18-1**

Thionyl chloride (1.60 mL, 22.04 mmol) was added to a solution of Compound **17-1** (1.5 g, 4.41 mmol) in methanol (20 mL). The reaction solution was stirred at 80°C for 12 hours under nitrogen protection. After the reaction solution was cooled to room temperature, it was poured into a saturated sodium bicarbonate aqueous solution and extracted with ethyl acetate (30 mL x2). The organic layers were combined and washed with saturated brine (50 mL), dried over anhydrous sodium sulfate and filtered. The filtrate was concentrated under reduced pressure to obtain Compound **18-1. MS m/z (ESI):** 274.4 $[M+H]^+$.

Step 2: Synthesis of Compound **18-2**

Under nitrogen protection at room temperature, trimethylsilyl chloride (1.49 mL, 11.90 mmol) was added to a solution of Compound **18-1** (1.3 g, 4.76 mmol) and 2-methylpropan-2-yl 4-oxohexahydropyridine-1-carboxylate (0.66 g, 3.33 mmol) in N,N-dimethylformamide (8 mL), and the mixture was stirred at 25°C for 1 hour. Borane tetrahydrofuran (7.14 mL, 7.14 mmol) was added to the reaction solution at 0°C and the stirring was continued for 1 hour. The reaction mixture was poured into water. The aqueous layer was extracted with ethyl acetate (20 mL x2), and the organic layers were combined and washed with saturated brine (50 mL), dried over anhydrous sodium sulfate, and filtered. The filtrate was concentrated under reduced pressure. The crude product was purified by rapid silica gel column chromatography to obtain Compound **18-2. MS m/z (ESI):** 457.2 $[M+H]^+$.

Step 3: Synthesis of Compound **18-3**

At room temperature, hydrazine hydrate (5 mL, 2.00 mmol) was added to a solution of Compound **18-2** (1.2 g, 2.63 mmol) in ethanol (5 mL), and the reaction solution was stirred at 80°C for 1 hour. After the reaction solution was cooled to room temperature, it was concentrated under reduced pressure. The crude product was purified by rapid silica gel column chromatography to obtain Compound **18-3. MS m/z (ESI):** 457.2 $[M+H]^+$.

Step 4: Synthesis of Compound **18-4**

Tetramethylchlorouronium hexafluorophosphate (1.23 g, 4.38 mmol) and 1-methylimidazole (0.70 mL, 8.76 mmol) were added to a solution of Compound **18-3** (1.0 g, 2.19 mmol) and Fmoc-glycine (1.1 g, 3.7 mmol) in acetonitrile (10 mL). The reaction mixture was stirred at 20°C for 2 hours. The reaction mixture was poured into water and extracted twice with ethyl acetate (20 mL x2). The organic layers were combined and washed with saturated brine (20 mL), dried over anhydrous sodium sulfate and filtered. The filtrate was concentrated under reduced pressure. The crude product was purified by rapid silica gel column chromatography to obtain Compound **18-4. MS m/z (ESI):** 736.3 $[M+H]^+$.

Step 5: Synthesis of Compound **18-5**

Lawesson reagent (1.32 g, 3.26 mmol) was added to a solution of Compound **18-4** (1.2 g, 1.63 mmol) in toluene (20 mL). The reaction mixture was stirred at 100°C for 2 hours. After the reaction solution was cooled to room temperature,

it was poured into water and extracted with ethyl acetate (20 mL x3). The organic layers were combined and washed with saturated brine (60 mL), dried over anhydrous sodium sulfate and filtered. The filtrate was concentrated under reduced pressure. The crude product was purified by rapid silica gel column chromatography to obtain Compound **18-5. MS m/z (ESI):** 734.2 $[M+H]^+$.

Step 6: Synthesis of Compound **18-6**

Diethylamine (0.33 mL, 2.04 mmol) was added to a solution of Compound **18-5** (500 mg, 0.68 mmol) in acetonitrile (5 mL) at room temperature. The reaction mixture was stirred at 20°C for 2 hours. After the reaction solution was concentrated, it was purified by rapid silica gel column chromatography to obtain Compound **18-6. MS m/z (ESI):** 512.2 $[M+H]^+$.

Step 7: Synthesis of Compound **18-7**

(2-Di-tert-butylphosphino-2',4',6'-triisopropyl-1,1'-biphenyl)(2'-amino-1,1'-biphenyl-2-yl) palladium methanesulfonate (16 mg, 0.02 mmol), 2-di-tert-butylphosphino- 2',4',6'-triisopropylbiphenyl (9 mg, 0.02 mmol) and sodium tert-butoxide (56 mg, 0.58 mmol) were added to a solution of Compound **18-6** (100 mg, 0.20 mmol) and 4-bromo-3-methoxy-N-methylbenzamide (72 mg, 0.29 mmol) in toluene (1 mL), and the reaction solution was stirred at 100°C for 1 hour under nitrogen protection. After the reaction solution was cooled to room temperature, it was poured into water (5 mL), the aqueous layer was extracted with ethyl acetate (5 mL x2), the organic layers were combined and washed with saturated brine (10 mL), dried over anhydrous sodium sulfate and filtered. The filtrate was concentrated under reduced pressure. The crude product was purified by rapid silica gel column chromatography to obtain Compound **18-7. MS m/z (ESI):** 675.4 $[M+H]^+$.

Step 8: Synthesis of Compound **18-8**

Trifluoroacetic acid (0.2 mL) was added to a solution of Compound **18-7** (80 mg, 0.12 mmol) in dichloromethane (0.5 mL), and the reaction mixture was stirred at 20°C for 1 hour. The reaction mixture was poured into a saturated sodium bicarbonate aqueous solution, extracted with ethyl acetate (15 mL x3), the organic layers were combined and washed with saturated brine (30 mL), dried over anhydrous sodium sulfate and filtered. The filtrate was concentrated under reduced pressure to obtain Compound **18-8. MS m/z (ESI):** 574.8 $[M+H]^+$.

Step 9: Synthesis of Compound **18**

Sodium cyanoborohydride (23 mg, 0.37 mmol) and acetic acid (0.01 mL, 0.01 mmol) were added to a solution of Compound **18-8** (70 mg, 0.12 mmol) and paraformaldehyde (33 mg, 0.37 mmol) in methanol (0.5 mL), and the reaction mixture was stirred at 60°C for 20 minutes. After the reaction solution was cooled to room temperature, it was concentrated under reduced pressure, and the crude product was purified by reverse phase flash chromatography to obtain Compound **18. MS m/z (ESI):** 589.2 $[M+H]^+$. **$^1$H NMR** (400 MHz, $CD_3OD$) δ 7.70 (d, *J* = 6.8 Hz, 1H), 7.36 (d, *J* = 2.0 Hz, 1H), 7.32 (dd, *J* = 2.0, 8.4 Hz, 1H), 6.87 (t, *J* = 7.2 Hz, 1H), 6.64 (d, *J* = 8.4 Hz, 1H), 6.33 (d, *J* = 7.6 Hz, 1H), 4.90 (s, 2H), 4.51 (q, *J* = 10.4 Hz, 2H), 3.94 (s, 3H), 3.50 (d, *J* = 11.6 Hz, 1H), 2.89 - 2.82 (m, 5H), 2.33 - 2.27 (m, 5H), 2.13 - 2.04 (m, 2H), 1.66 - 1.60 (m, 2H).

Preparation Example 19: Preparation of Compound **19**

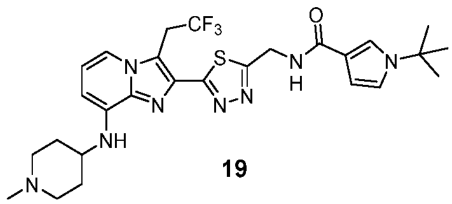

**19**

Synthesis scheme:

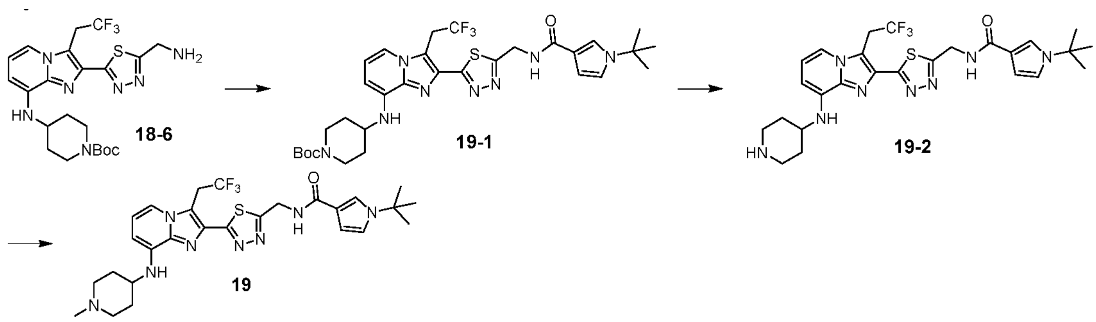

18-6

19-1

19-2

19

Step 1: Synthesis of Compound **19-1**

Tetramethylchlorouronium hexafluorophosphate (33 mg, 0.12 mmol) and 1-methylimidazole (0.02 mL, 0.24 mmol) were added to a solution of Compound **18-6** (30 mg, 0.06 mmol) and 1-(2-methylpropyl-2-yl)pyrrole-3-carboxylic acid (20 mg, 0.12 mmol) in acetonitrile (0.5 mL). The reaction mixture was stirred at 20°C for 2 hours. The reaction mixture was poured into water and extracted with ethyl acetate (10 mL x2). The organic layers were combined and washed with saturated brine (15 mL), dried over anhydrous sodium sulfate and filtered. The filtrate was concentrated under reduced pressure. The crude product was purified by rapid silica gel column chromatography to obtain Compound **19-1. MS m/z (ESI):** 661.2 $[M+H]^+$.

Step 2: Synthesis of Compound **19-2**

Trifluoroacetic acid (0.2 mL) was added to a solution of Compound **19-1** (15 mg, 0.02 mmol) in dichloromethane (0.5 mL), and the reaction mixture was stirred at 20°C for 1 hour. The reaction mixture was poured into a saturated sodium bicarbonate aqueous solution, extracted with ethyl acetate (15 mL x3), and the organic layers were combined and washed with saturated brine (30 mL), dried over anhydrous sodium sulfate, and filtered. The filtrate was concentrated under reduced pressure to obtain Compound **19-2. MS m/z (ESI):** 561.3 $[M+H]^+$.

Step 3: Synthesis of Compound **19**

Acetic acid (0.01 mL) and sodium cyanoborohydride (4 mg, 0.06 mmol) were added to a solution of Compound **19-2** (9 mg, 0.02 mmol) and paraformaldehyde (5 mg, 0.06 mmol) in methanol (0.5 mL), and the reaction mixture was stirred at 60 °C for 10 minutes. After the reaction solution was cooled to room temperature, it was concentrated under reduced pressure, and the crude product was purified by reverse phase flash chromatography to obtain Compound **19. MS m/z (ESI):** 575.2 $[M+H]^+$. **$^1$H NMR** (400 MHz, $CD_3OD$) δ 7.66 (d, *J* = 6.8 Hz, 1H), 7.48 (t, *J* = 2.0 Hz, 1H), 6.86 (dd, *J* = 2.4, 3.2 Hz, 1H), 6.84 - 6.78 (m, 1H), 6.46 (dd, *J* = 2.0, 3.2 Hz, 1H), 6.30 (d, *J* = 7.6 Hz, 1H), 4.82 (s, 2H), 4.44 (q, *J* = 10.4 Hz, 2H), 3.56 - 3.51 (m, 1H), 3.08 - 3.00 (m, 2H), 2.57 - 2.46 (m, 2H), 2.44 (s, 3H), 2.13 - 2.05 (m, 2H), 1.74 - 1.60 (m, 2H), 1.46 (s, 9H).

Preparation Example 20: Preparation of Compound **20**

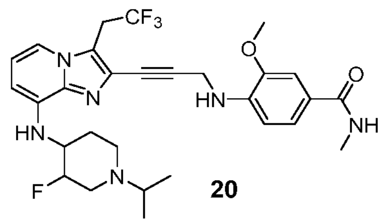

**20**

For the synthesis of Compound **20,** Preparation Example 5 could be referred to.

Compound **20: MS m/z (ESI):** 575.3 $[M+H]^+$. **$^1$H NMR** (400 MHz, DMSO-$d_6$) δ 8.09 (d, *J* = 4.8 Hz, 1H), 7.80 - 7.61 (m, 1H), 7.41 (d, *J* = 8.4 Hz, 1H), 7.34 (s, 1H), 6.84 - 6.81 (m, 1H), 6.75 - 6.70 (m, 1H), 6.41 - 6.32 (m, 1H), 6.07 - 5.91 (m, 1H), 5.38 - 5.36 (m, 1H), 4.89 - 4.77 (m, 1H), 4.27 (s, 2H), 4.03 - 4.00 (m, 1H), 3.84 (s, 3H), 3.82 - 3.64 (m, 2H), 3.27 - 3.24 (m, 2H), 3.14 - 3.03 (m, 1H), 2.78 - 2.71 (m, 5H), 1.84 - 1.76 (m, 2H), 0.97 (s, 6H).

Preparation Example 21: Preparation of Compound **21**

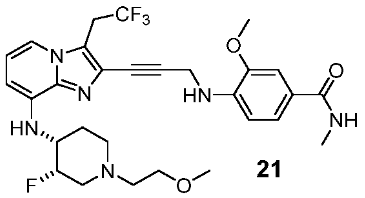

**21**

For the synthesis of Compound **21,** Preparation Example 5 could be referred to.

Compound **21: MS m/z (ESI):** 591.3 $[M+H]^+$. **$^1$H NMR** (400 MHz, DMSO-$d_6$) δ 8.10 (d, *J* = 4.8 Hz, 1H), 7.79 (d, *J* = 6.8 Hz, 1H), 7.42 (dd, *J* = 1.6, 8.0 Hz, 1H), 7.35 (d, *J* = 2.0 Hz, 1H), 6.83 (t, *J* = 7.2 Hz, 1H), 6.75 (d, *J* = 8.4 Hz, 1H), 6.39 (d, *J* = 7.6 Hz, 1H), 5.96 (t, *J* = 6.4 Hz, 1H), 5.39 (d, *J* = 9.1 Hz, 1H), 4.81 (d, *J* = 49.6 Hz, 1H), 4.27 (d, *J* = 6.4 Hz, 2H), 4.02 (q, *J* = 10.6 Hz, 2H), 3.85 (s, 3H), 3.82 - 3.66 (m, 1H), 3.44 (t, *J* = 5.6 Hz, 2H), 3.24 (s, 3H), 3.17 - 3.11 (m, 1H), 2.90 - 2.86 (m, 1H), 2.75 (d, *J* = 4.4 Hz, 3H), 2.48 - 2.35 (m, 3H), 2.26 - 2.22 (m, 1H), 1.81 - 1.76 (m, 2H).

Preparation Example 22: Preparation of Compound **22**

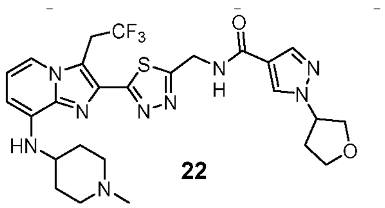

**22**

For the synthesis of Compound **22,** Preparation Example 19 could be referred to.

Compound **22: MS m/z (ESI):** 590.2 $[M+H]^+$. **$^1$H NMR** (400 MHz, $CD_3OD$) δ 8.21 (s, 1H), 7.95 (s, 1H), 7.72 (d, *J* = 7.2 Hz, 1H), 6.89 (t, *J* = 7.2 Hz, 1H), 6.36 (d, *J* = 7.6 Hz, 1H), 5.09 - 5.06 (m, 1H), 4.93 (s, 2H), 4.52 (q, *J* = 10.4 Hz, 2H), 4.11 (q, *J* = 7.8 Hz, 1H), 4.05 - 3.99 (m, 2H), 3.93 - 3.88 (m, 1H), 3.56 - 3.50 (m, 1H), 2.91 - 2.86 (m, 2H), 2.53 - 2.46 (m, 1H), 2.37 - 2.22 (m, 6H), 2.14 - 2.10 (m, 2H), 1.70 - 1.66 (m, 2H).

Preparation Example 23: Preparation of Compound **23**

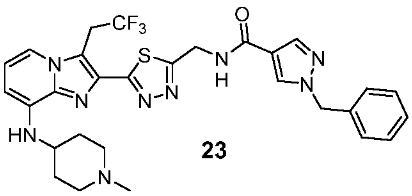

**23**

For the synthesis of Compound **23,** Preparation Example 19 could be referred to.

Compound **23: MS m/z (ESI):** 610.3 $[M+H]^+$. **$^1$H NMR** (400 MHz, $CD_3OD$) δ 8.18 (s, 1H), 7.97 (s, 1H), 7.72 (d, *J* = 6.8 Hz, 1H), 7.41 - 7.25 (m, 5H), 6.89 (t, *J* = 7.2 Hz, 1H), 6.36 (d, *J* = 7.6 Hz, 1H), 5.38 (s, 2H), 4.93 (s, 2H), 4.52 (q, *J* = 10.4 Hz, 2H), 3.55 - 3.50 (m, 1H), 2.91 - 2.86 (m, 2H), 2.33 (s, 3H), 2.30 - 2.26 (m, 2H), 2.17 - 2.08 (m, 2H), 1.72 - 1.63 (m, 2H).

Preparation Example 24: Preparation of Compound **24**

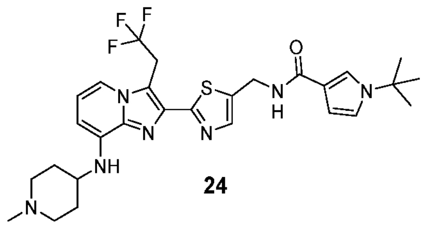

24

Synthesis scheme:

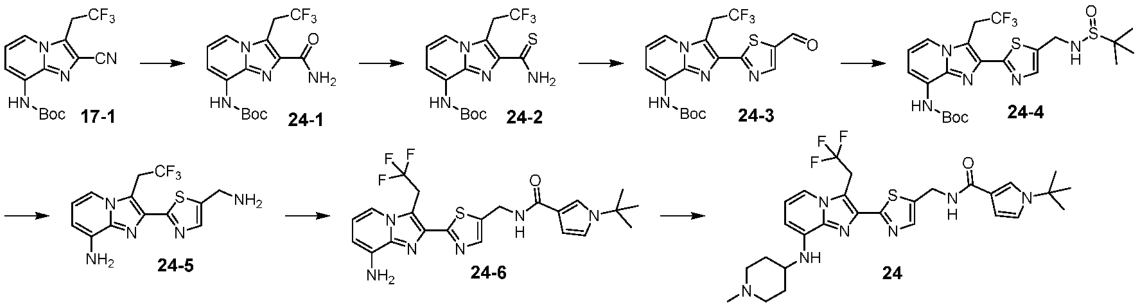

Step 1: Synthesis of Compound **24-1**

Hydrogen peroxide (2.36 mL, 23.51 mmol, 30%) was added dropwise to a solution of Compound **17-1** (2.0 g, 5.88 mmol) and potassium carbonate (2.44 g, 17.63 mmol) in dimethyl sulfoxide (20 mL) at 0 °C. The reaction mixture was stirred at room temperature for 3 hours. The reaction mixture was quenched with saturated sodium sulfite aqueous solution (10 mL), extracted with ethyl acetate (40 mL x2), and the organic layers were combined and washed with saturated brine (50 mL), dried over anhydrous sodium sulfate, and filtered. The filtrate was concentrated under reduced pressure to obtain Compound **24-1. MS m/z (ESI):** 359.2 $[M+H]^+$.

Step 2: Synthesis of Compound **24-2**

Under nitrogen protection, Lawesson reagent (2.48 g, 6.14 mmol) was added to a solution of Compound **24-1** (2.2 g, 6.14 mmol) in tetrahydrofuran (50 mL). The reaction mixture was stirred at 70 °C for 16 hours. After the reaction solution was cooled to room temperature, it was filtered, the filtrate was concentrated under reduced pressure, and the crude product was purified by rapid silica gel column chromatography to obtain Compound **24-2. MS m/z (ESI):** 375.2 $[M+H]^+$.

Step 3: Synthesis of Compound **24-3**

A solution of Compound **24-2** (880 mg, 2.35 mmol) and 2-bromomalonaldehyde (355 mg, 2.35 mmol) in tetrahydrofuran (10 mL) was stirred at 55 °C for 10 hours. After the reaction solution was cooled to room temperature, it was concentrated under reduced pressure, and the crude product was purified by rapid silica gel column chromatography to obtain Compound **24-3. MS m/z (ESI):** 427.0 $[M+H]^+$.

Step 4: Synthesis of Compound **24-4**

A solution of Compound **24-3** (400 mg, 0.94 mmol), tert-butylsulfenamide (227 mg, 1.88 mmol) and tetraethyl titanate (428 mg, 1.88 mmol) in tetrahydrofuran (1 mL) was stirred at 60 °C for 2 hours. After the reaction solution was cooled to room temperature, methanol (1 mL) and sodium borohydride (106 mg, 2.81 mmol) were added in sequence, and the reaction solution was stirred at room temperature for 1 hour. The reaction solution was concentrated under reduced pressure, and the crude product was purified by rapid silica gel column chromatography to obtain Compound **24-4. MS m/z (ESI):** 532.2 $[M+H]^+$.

Step 5: Synthesis of Compound **24-5**

At room temperature, concentrated hydrochloric acid (1 mL, 12.00 mmol) was added to a solution of Compound **24-4** (200 mg, 0.38 mmol) in methanol (0.5 mL), and the reaction solution was stirred at room temperature for 10 hours. The reaction solution was diluted with dichloromethane (5 mL), dried over anhydrous sodium sulfate and filtered. The filtrate was concentrated under reduced pressure to obtain Compound **24-5. MS m/z (ESI):** 328.2 $[M+H]^+$.

Step 6: Synthesis of Compound **24-6**

At room temperature, 1-tert-butyl-1H-pyrrole-3-carboxylic acid (61.30 mg, 0.37 mmol) and 2-(7-azabenzotriazole)-N,N,N',N'-tetramethyluronium hexafluorophosphate (174 mg, 0.46 mmol) were added to a solution of Compound **24-5** (100 mg, 0.31 mmol) and N,N-diisopropylethylamine (118 mg, 0.92 mmol) in tetrahydrofuran (2 mL), and the reaction solution was stirred at room temperature for 3 hours. The reaction solution was diluted with water (5 mL), extracted with ethyl acetate (1 mL x3), and the organic layers were combined and washed with saturated brine (5 mL), dried over anhydrous sodium sulfate, filtered, and the filtrate was concentrated under reduced pressure. The crude product was purified by reverse phase preparative chromatography to obtain Compound **24-6. MS m/z (ESI):** 477.0 $[M+H]^+$.

Step 7: Synthesis of Compound **24**

At room temperature, trimethylsilyl chloride (36 mg, 0.34 mmol) was added to a solution of Compound **24-6** (20 mg, 0.04 mmol) and N-methyl-4-piperidone (38 mg, 0.34 mmol) in N,N-dimethylformamide (0.3 mL), and the reaction solution was stirred at 45 °C for 2 hours under nitrogen protection. After the reaction solution was cooled to room temperature, borane tetrahydrofuran (0.67 mL, 0.67 mmol, 1M) was added, and the reaction solution was stirred at room temperature for 2 hours. The reaction solution was quenched by adding methanol (1 mL) and concentrated under reduced pressure. The crude product was purified by reverse phase preparative chromatography to obtain Compound **24. MS m/z (ESI):** 574.2 $[M+H]^+$. **$^1$H NMR** (400 MHz, DMSO-$d_6$) δ 8.48 (t, *J* = 6.0 Hz, 1H), 7.82 (d, *J* = 6.6 Hz, 1H), 7.78 (s, 1H), 7.51 (s, 1H), 6.96 (s, 1H), 6.85 (t, *J* = 7.2 Hz, 1H), 6.48 (t, *J* = 2.4 Hz, 1H), 6.30 (d, *J* = 7.6 Hz, 1H), 5.57 (d, *J* = 8.4 Hz, 1H), 4.68 - 4.55 (m, 4H), 3.35 - 3.32 (m, 1H), 2.79 - 2.74 (m, 2H), 2.19 (s, 3H), 2.05 (t, *J* = 11.2 Hz, 2H), 1.95 - 1.92 (m, 2H), 1.67 - 1.59 (m, 2H), 1.48 (s, 9H).

Preparation Example 25: Preparation of Compound **25**

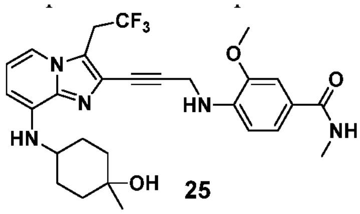

**25**

For the synthesis of Compound **25,** Preparation Example 6 could be referred to.

Compound **25: MS m/z (ESI):** 544.3 $[M+H]^+$. **$^1$H NMR** (400 MHz, $CD_3OD$) δ 7.56 (d, *J* = 6.8 Hz, 1H), 7.43 (dd, *J* = 2.0, 8.4 Hz, 1H), 7.35 (d, *J* = 2.0 Hz, 1H), 6.89 - 6.81 (m, 2H), 6.31 (d, *J* = 7.6 Hz, 1H), 4.33 (s, 2H), 3.91 (s, 3H), 3.83 (q, *J* = 10.4 Hz, 2H), 3.41 - 3.37 (m, 1H), 2.89 (s, 3H), 1.94 - 1.89 (m, 2H), 1.79 - 1.67 (m, 4H), 1.58 - 1.53 (m, 2H), 1.23 (s, 3H).

Preparation Example 26: Preparation of Compound **26**

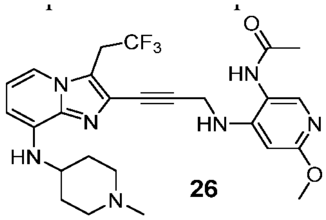

**26**

Synthesis scheme:

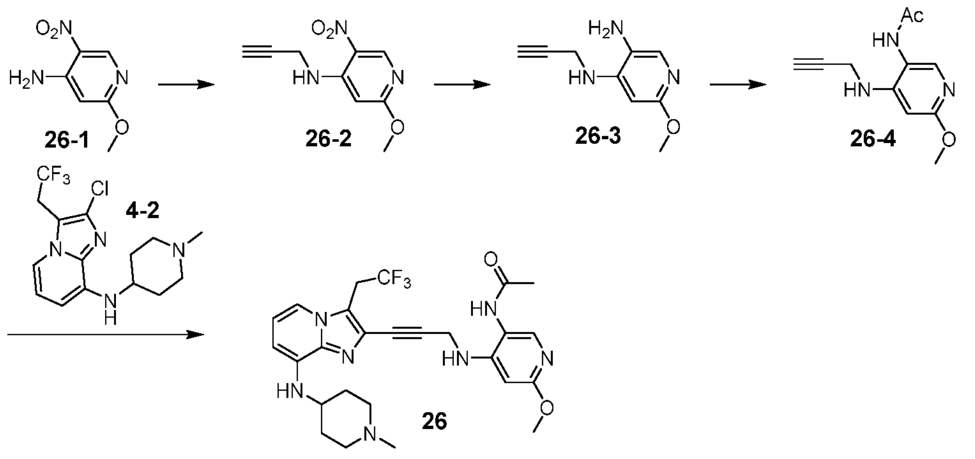

26-1 26-2 26-3 26-4 4-2 26

Step 1: Synthesis of Compound **26-2**

At 0 °C, sodium hydride (301.5 mg, 7.54 mmol) was added to a solution of Compound **26-1** (850 mg, 5.03 mmol) in DMF (8 mL). Under nitrogen protection, the reaction solution was stirred at 0 °C for 0.5 hours. The reaction mixture was poured into water (20 mL), the aqueous layer was extracted with ethyl acetate (20 mL x2), the organic layers were combined and washed with saturated sodium chloride aqueous solution (10 mL), dried over anhydrous sodium sulfate, and filtered through filter paper, and the filtrate was concentrated under reduced pressure. The crude product was purified by silica gel column chromatography to obtain Compound **26-2. MS m/z (ESI):** 208.2 $[M+H]^+$. **$^1$H NMR** (400 MHz, $CDCl_3$) δ 9.02 (s, 1H),8.08 (s, 1H), 6.08 (s, 1H), 4.07 (dd, *J* = 2.4, 5.6 Hz, 2H), 4.00 (s, 3H), 2.33 (t, *J* = 2.4 Hz, 1H).

Step 2: Synthesis of Compound **26-3**

Iron powder (202 mg, 3.62 mmol) and ammonium chloride (323 mg, 6.03 mmol) were added to a solution of Compound **26-2** (250 mg, 1.21 mmol) in ethanol (15 mL) and water (3 mL). The reaction solution was stirred at 50 °C for 2 hours under nitrogen protection. After the reaction solution was cooled to room temperature, it was filtered through filter paper, and the filter cake was washed with methanol (15 mL x3). The filtrate was dried over anhydrous sodium sulfate, filtered through filter paper, and the filtrate was concentrated under reduced pressure to obtain Compound **26-3. MS m/z (ESI):** 178.1 $[M+H]^+$.

Step 3: Synthesis of Compound **26-4**

Triethylamine (0.59 mL, 4.23 mmol) and acetic acid (0.20 mL, 2.12 mmol) were added to a solution of Compound **26-3** (250 mg, 1.41 mmol) in dichloromethane (5 mL). The reaction mixture was stirred at room temperature for 2 hours under nitrogen protection. The reaction mixture was poured into water (20 mL) and extracted with ethyl acetate (20 mL x2). The organic layers were combined and washed with saturated brine (20 mL) and dried over anhydrous sodium sulfate. After filtering through filter paper, the filtrate was concentrated under reduced pressure. The crude product was purified by silica gel column chromatography to obtain Compound **26-4. MS m/z (ESI):** 220.2 $[M+H]^+$. **$^1$H NMR** (400 MHz, DMSO-$d_6$) δ 8.98 (s, 1H), 7.59 (s, 1H), 6.26 (t, *J* = 6.0 Hz, 1H), 5.98 (s, 1H), 3.93 (dd, *J* = 2.4, 6.0 Hz, 2H), 3.76 (s, 3H), 3.16 - 3.12 (m, 1H), 2.01 (s, 3H).

Step 4: Synthesis of Compound **26:**

Compound **26-4** (95 mg, 0.43 mmol), bis(acetonitrile)palladium chloride (7.5 mg, 0.03 mmol), 2-dicyclohexylphosphino-2',4',6'-triisopropylbiphenyl (41 mg, 0.09 mmol) and cesium carbonate (94 mg, 0.29 mmol) were added to a solution of Compound **4-2** (10 mg, 0.13 mmol) in acetonitrile (1 mL). The reaction solution was stirred at 90 °C for 2 hours under nitrogen protection. After the reaction mixture was cooled to room temperature, it was poured into water (5 mL) and extracted with ethyl acetate (5 mL x2). The organic layers were combined and washed with saturated brine (10 mL), and dried over anhydrous sodium sulfate. After filtering through filter paper, the filtrate was concentrated under reduced

pressure. The crude product was purified by reverse phase flash chromatography to obtain Compound **26. MS m/z (ESI):** 530.3 [M+H]+. **1H NMR** (400 MHz, DMSO-$d_6$) δ 9.04 (s, 1H), 7.72 (d, *J* = 6.8 Hz, 1H), 7.61 (s, 1H), 6.80 (t, *J* = 7.2 Hz, 1H), 6.42 (t, *J* = 6.0 Hz, 1H), 6.22 (d, *J* = 7.6 Hz, 1H), 6.08 (s, 1H), 5.77 (d, *J* = 8.4 Hz, 1H), 4.23 (d, *J* = 6.0 Hz, 2H), 4.06 (q, *J* = 10.8 Hz, 2H), 3.76 (s, 3H), 3.55 - 3.51 (m, 1H), 2.76 - 2.70 (m, 2H), 2.17 (s, 3H), 2.02 - 1.96 (m, 5H), 1.90 - 1.85 (m, 2H), 1.61 - 1.55 (m, 2H).

Preparation Example 27: Preparation of Compound **27**

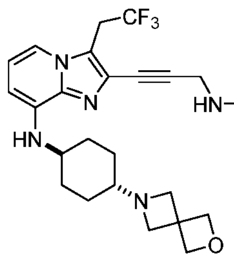

**27**

Synthesis scheme:

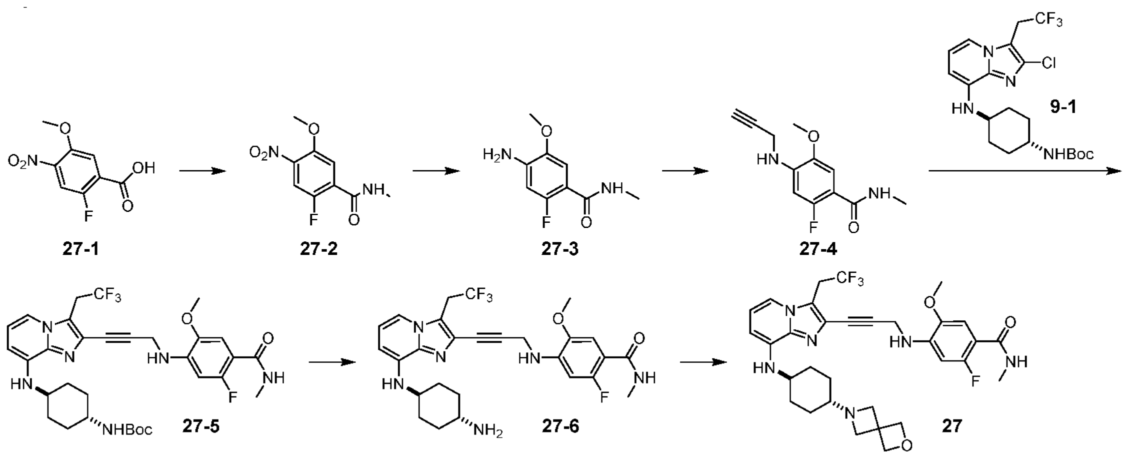

27-1 27-2 27-3 27-4 9-1 27-5 27-6 27

Step 1: Synthesis of Compound **27-2**

Under nitrogen protection, N-methylimidazole (0.67 mL, 40.4 mmol) and N,N,N',N'tetramethylchloroformamidine hexafluorophosphate (1.17 g, 10.6 mmol) were added to a solution of Compound **27-1** (600 mg, 2.79 mmol) and methylamine hydrochloride (280 mg, 4.18 mmol) in acetonitrile (10 mL). The mixture was stirred at room temperature for 12 hours under nitrogen protection. The reaction solution was dissolved in water (60 mL), extracted with ethyl acetate (30 mL x3), the organic phases were combined, washed with saturated brine (50 mL x2), and the resultant organic phase was concentrated under reduced pressure. The crude product was purified by rapid silica gel column chromatography to obtain Compound **27-2. MS m/z (ESI):** 229.1 [M+H]+.

Step 2: Synthesis of Compound **27-3**

Iron powder (11.01 g, 197.21 mmol) and ammonium chloride (17.58 g, 328.69 mmol) were added to a solution of Compound **27-2** (15.0 g, 65.74 mmol) in ethanol (150 mL) and water (10 mL). The reaction solution was stirred at 50 °C for 2 hours under nitrogen protection. After the reaction solution was cooled to room temperature, it was filtered through filter paper and the filter cake was washed with methanol (20 mL x3). The filtrate was dried over anhydrous sodium sulfate, filtered through filter paper, and concentrated under reduced pressure to obtain Compound **27-3. MS m/z (ESI):** 199.0 [M+H]+.

Step 3: Synthesis of Compound **27-4**

3-Bromopropyne (0.48 mL, 5.55 mmol) was added to a mixture of Compound **27-3** (1.0 g, 5.05 mmol) and potassium carbonate (2.09 g, 15.14 mmol) in N,N-dimethylformamide (10 mL), and the reaction solution was stirred at 80 °C for 2 hours. The reaction solution was diluted with water (40 mL), extracted with ethyl acetate (10 mL x3), and the organic layers were combined and washed with saturated brine (50 mL), dried over anhydrous sodium sulfate, and filtered. The filtrate was concentrated under reduced pressure. The crude product was purified by rapid silica gel column chromatography to obtain Compound **27-4. MS m/z (ESI):** 237.0 $[M+H]^+$.

Step 4: Synthesis of Compound **27-5**

Under nitrogen protection, a mixture of Compound **9-1** (300 mg, 0.67 mmol), 2-dicyclohexylphosphino-2',4',6'-triisopropylbiphenyl (128 mg, 0.27 mmol), bis(acetonitrile)palladium chloride (35 mg, 0.13 mmol), cesium carbonate (437 mg, 1.34 mmol) and acetonitrile (1 mL) was stirred at 90 °C for 20 minutes. Compound **27-4** (317 mg, 1.34 mmol) was added to the reaction solution, and the reaction solution was stirred at 90 °C for 2 hours. After the reaction solution was cooled to room temperature, it was filtered, the filter cake was washed with acetonitrile (15 mL x3), and the filtrate was concentrated under reduced pressure. The crude product was purified by rapid silica gel column chromatography to obtain Compound **27-5. MS m/z (ESI):** 647.3 $[M+H]^+$.

Step 5: Synthesis of Compound **27-6**

Compound **27-5** (52 mg, 0.07 mmol) was dissolved in trifluoroacetic acid (1 mL), and the reaction solution was stirred at 25 °C for 2 hours. The mixture was concentrated under reduced pressure to obtain Compound **27-6. MS m/z (ESI):** 547.2 $[M+H]^+$.

Step 6: Synthesis of Compound **27**

Sodium carbonate (434 mg, 4.10 mmol) was added to a solution of Compound **27-6** (280 mg, 0.51 mmol) and 3,3-bis(bromomethyl)oxetane (250 mg, 1.02 mmol) in acetonitrile (6 mL), and the reaction solution was stirred at 80 °C for 16 hours. After the reaction solution was cooled to room temperature, it was filtered, the filter cake was washed with ethyl acetate (5 mL x3), and the filtrate was concentrated under reduced pressure. The crude product was purified by reverse phase flash chromatography to obtain Compound **27. MS m/z (ESI):** 629.3 $[M+H]^+$. **$^1$H NMR** (400 MHz, $CDCl_3$) δ 7.49 (d, *J* = 7.6 Hz, 1H), 7.39 (br s, 1H), 7.28 - 7.26 (m, 1H), 6.76 - 6.66 (m, 1H), 6.45 - 6.41 (m 1H), 5.07 - 5.03 (m, 3H), 4.71 (s, 2H), 4.60 - 4.48 (m, 2H), 4.30 (d, *J* = 6.0 Hz, 2H), 3.89 - 3.82 (m, 4H), 3.68 (q, *J* = 9.6 Hz, 2H), 3.52 - 3.49 (m, 1H), 3.01 (dd, *J* = 1.2, 4.8 Hz, 3H), 2.92 - 2.90 (m, 1H), 2.33 - 2.28 (m, 2H), 2.05 - 2.00 (m, 2H), 1.93 - 1.75 (m, 2H), 1.61 - 1.58 (m, 2H).

Preparation Example 28: Preparation of Compound **28**

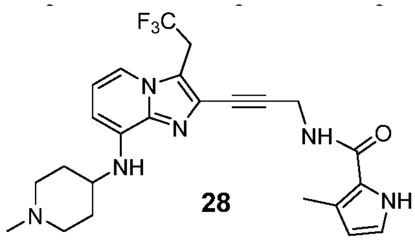

**28**

Synthesis scheme:

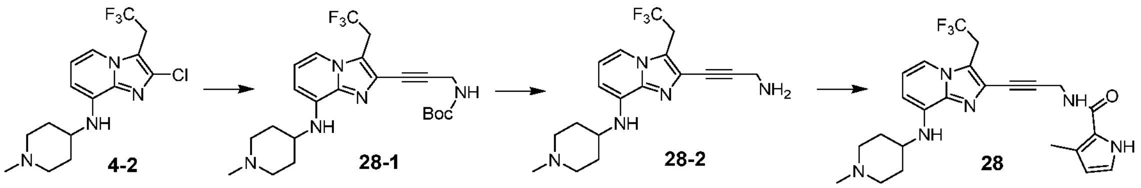

Step 1: Synthesis of Compound **28-1**

To a solution of Compound **4-2** (60 mg, 0.17 mmol) in acetonitrile (2 mL), 2-dicyclohexylphosphino-2',4',6'-triisopropylbiphenyl (33 mg, 0.07 mmol), bis(acetonitrile)palladium chloride (9 mg, 0.03 mmol) and cesium carbonate (140 mg, 0.43 mmol) were added. The mixture was purged with nitrogen three times, and N-Boc-aminopropyne (108 mg, 0.70 mmol) was added under nitrogen atmosphere. The reaction mixture was stirred at 90 °C for 2 hours under nitrogen protection. After the reaction solution was cooled to room temperature, it was filtered, the filter cake was washed with ethyl acetate (10 mL x3), and the filtrate was concentrated to dryness under reduced pressure. The crude product was purified by rapid silica gel column chromatography to obtain Compound **28-1. MS m/z (ESI):** 466.2 $[M+H]^+$.

Step 2: Synthesis of Compound **28-2**

Trifluoroacetic acid (0.3 mL, 4.03 mmol) was added to a solution of Compound **28-1** (20 mg, 0.04 mmol) in dichloromethane (2 mL), and the reaction mixture was stirred at room temperature for 10 minutes. The mixture was concentrated under reduced pressure to obtain Compound **28-2. MS m/z (ESI):** 366.3 $[M+H]^+$.

Step 3: Synthesis of Compound **28**

To a solution of Compound **28-2** (15 mg, 0.04 mmol) in acetonitrile (1 mL), 3-methyl-1H-pyrrole-2-carboxylic acid (8 mg, 0.06 mmol), N,N,N',N'-tetramethylchloroformamidine hexafluorophosphate (18 mg, 0.06 mmol) and 1-methylimidazole (20 μL, 0.25 mmol) were added. The reaction mixture was stirred at room temperature for 1 hour. The reaction solution was filtered and concentrated under reduced pressure, and the crude product was purified by reverse phase flash chromatography to obtain Compound **28. MS m/z (ESI):** 473.2 $[M+H]^+$. **$^1$H NMR** (400 MHz, $CD_3OD$) δ 7.69 (d, *J* = 6.8 Hz, 1H), 6.88 (dd, *J* = 6.8, 7.6 Hz, 1H), 6.80 (d, *J* = 2.4 Hz, 1H), 6.41 (d, *J* = 7.6 Hz, 1H), 6.02 (d, *J* = 2.4 Hz, 1H), 4.43 (s, 2H), 4.08 - 3.97 (m, 2H), 3.74 (br s, 1H), 3.30 - 3.23 (m, 2H), 2.94 - 2.90 (m, 2H), 2.69 (s, 3H), 2.36 (s, 3H), 2.27 - 2.23 (m, 2H), 1.83 - 1.79 (m, 2H).

Preparation Example 29: Preparation of Compound **29**

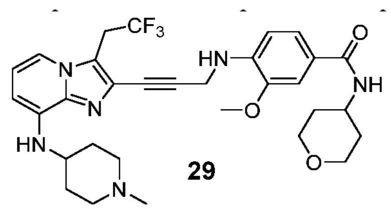

**29**

For the synthesis of Compound **29,** Preparation Example 13 could be referred to.

Compound **29: MS m/z (ESI):** 599.3 $[M+H]^+$. **$^1$H NMR** (400 MHz, DMSO-$d_6$) δ 7.94 (d, *J* = 7.6 Hz, 1H), 7.78 (d, *J* = 6.8 Hz, 1H), 7.45 (dd, *J* = 2.0, 8.4 Hz, 1H), 7.35 (d, *J* = 2.0 Hz, 1H), 6.83 (t, *J* = 6.0, 8.4 Hz, 1H), 6.73 (d, *J* = 8.4 Hz, 1H), 6.28 (d, *J* = 7.6 Hz, 1H), 6.19 - 6.12 (m, 1H), 6.08 - 5.88 (m, 1H), 4.27 (s, 2H), 4.10 - 3.99 (m, 2H), 4.00 - 3.96 (m, 2H), 3.91 - 3.88 (m, 1H), 3.85 (s, 3H), 3.48 (d, *J* = 12.0 Hz, 2H), 3.42 - 3.32 (m, 2H), 3.31 - 3.24 (m, 1H), 3.12 - 3.00 (m, 2H), 2.79 (d, *J* = 4.8 Hz, 3H), 2.17 - 2.09 (m, 2H), 1.80 - 1.68 (m, 4H), 1.63 - 1.51 (m, 2H).

Preparation Example 30: Preparation of Compound **30**

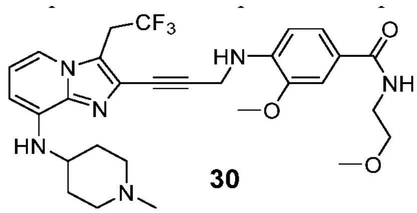

30

For the synthesis of Compound **30,** Preparation Example 13 could be referred to.

Compound **30: MS m/z (ESI):** 573.3 $[M+H]^+$. **$^1$H NMR** (400 MHz, DMSO-$d_6$) δ 8.21 (d, *J* = 5.6 Hz, 1H), 7.78 (dd, *J* = 6.8, 11.2 Hz, 1H), 7.44 (dd, *J* = 2.0, 8.0 Hz, 1H), 7.37 (d, *J* = *2.0* Hz, 1H), 6.83 (t, *J* = 7.2 Hz, 1H), 6.73 (d, *J* = 8.4 Hz, 1H), 6.29 - 6.25 (m, 1H), 6.20 - 6.11 (m, 1H), 6.10 - 5.93 (m, 1H), 4.27 (s, 2H), 4.08 - 4.00 (m, 2H), 3.85 (s, 3H), 3.51 - 3.46 (m, 2H), 3.44 - 3.37 (m, 4H), 3.27 - 3.25 (m, 4H), 3.14 - 3.00 (m, 2H), 2.83 - 2.74 (m, 3H), 2.16 - 2.08 (m, 2H), 1.80 - 1.67 (m, 2H).

Preparation Example 31: Preparation of Compounds **31** and **32**

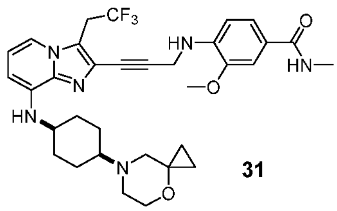

31

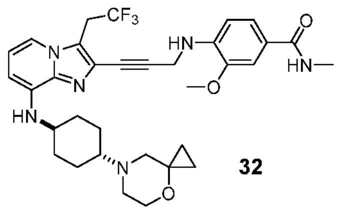

32

Synthesis scheme:

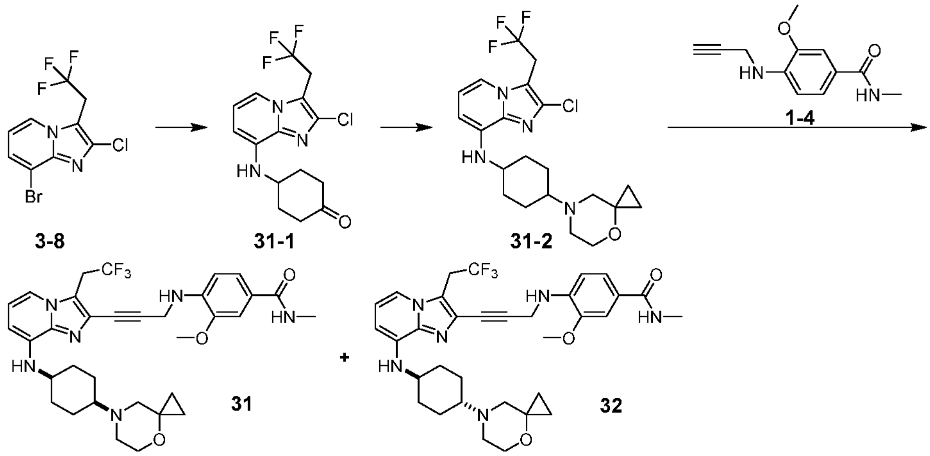

3-8 31-1 31-2 1-4

31 + 32

Step 1: Synthesis of Compound **31-1**

Under nitrogen protection, tris(dibenzylideneacetone)dipalladium (290 mg, 0.32 mmol) was added to a mixture of Compound **3-8** (500 mg, 1.59 mmol), 4-aminocyclohexanone hydrochloride (170 mg, 0.92 mmol), cesium carbonate (1.20 g, 3.68 mmol) and 1,1'-binaphthyl-2,2'-bisdiphenylphosphine (200 mg, 0.32 mmol) in 1,4-dioxane (10 mL), and the reaction solution was stirred at 110 °C for 1 hour. After the reaction solution was cooled to room temperature, it was concentrated under reduced pressure. The crude product was purified by rapid silica gel column chromatography to obtain Compound **31-1. MS m/z (ESI):** 346.2 $[M+H]^+$.

Step 2: Synthesis of Compound **31-2**

Compound **31-1** (160 mg, 0.46 mmol) was dissolved in dichloromethane (8 mL), and 4-oxa-7-azaspiro[2.5]octane hydrochloride (138 mg, 0.93 mmol), triethylamine (0.13 mL, 0.94 mmol) and sodium triacetoxyborohydride (295 mg, 1.40 mmol) were added. The reaction mixture was stirred at room temperature for 2 hours. The reaction solution was added with water (10 mL) and extracted with ethyl acetate (10 mL x2). The organic layers were combined and washed with saturated brine (10 mL), dried over anhydrous sodium sulfate, filtered, and the filtrate was concentrated under reduced pressure. The crude product was purified by silica gel column chromatography to obtain Compound **31-2. MS m/z (ESI):** 443.2 $[M+H]^+$. **$^1$H NMR** (400 MHz, DMSO-$d_6$) δ 7.82 (d, *J* = 6.8 Hz, 1H), 6.88 (t, *J* = 7.2 Hz, 1H), 6.33 (d, *J* = 7.6 Hz, 1H), 5.46 (d, *J* = 7.6 Hz, 1H), 4.12 (q, *J* = 10.8 Hz, 2H), 3.66 - 3.62 (m, 3H), 2.50 - 2.48 (m, 2H), 2.38 (s, 2H), 2.19 - 2.17 (m, 1H), 1.85 - 1.47 (m, 8H), 0.66 - 0.59 (m, 2H), 0.53 - 0.43 (m, 2H).

Step 3: Synthesis of Compounds **31a** and **32a**

Bis(acetonitrile)palladium chloride (2 mg, 0.01 mmol), 2-dicyclohexylphosphino- 2',4',6'-triisopropylbiphenyl (6 mg, 0.01 mmol) and cesium carbonate (23 mg, 0.07 mmol) were added to a solution of Compound **31-2** (13 mg, 0.03 mmol) in acetonitrile (1 mL). The mixture was stirred at 90 °C for 10 minutes under nitrogen protection, and Compound **1-4** (26 mg, 0.12 mmol) was added. The mixture was stirred at 90 °C for 2 hours under nitrogen protection. After the reaction solution was cooled to room temperature, it was filtered, the filter cake was washed with ethyl acetate (4 mL x3), and the filtrate was concentrated under reduced pressure. The crude product was purified by reverse phase flash chromatography to obtain Compounds **31a** and **32a** as isomers. Compounds **31a** and **32a** were selected from the group consisting of Compounds **31** and **32,** respectively, and Compounds **31a** and **32a** were different.

Compound **31a:** Reverse phase flash chromatography column type: Waters-Xbridge-C18-10μm-19*250mm; mobile phase: A: 10mM ammonium bicarbonate/water B: acetonitrile; flow rate: 25; retention time: 8.05 min. **MS m/z (ESI):** 625.3 $[M+H]^+$. **$^1$H NMR** (400 MHz, DMSO-$d_6$) δ 8.11 (d, *J* = 4.8 Hz, 1H), 7.72 (d, *J* = 6.8 Hz, 1H), 7.41 (dd, *J* = 1.6, 8.4 Hz, 1H), 7.34 (d, *J* = 2.0 Hz, 1H), 6.80 (t, *J* = 7.2 Hz, 1H), 6.74 (d, *J* = 8.4 Hz, 1H), 6.23 (d, *J* = 7.6 Hz, 1H), 5.97 (t, *J* = 6.4 Hz, 1H), 5.43 (d, *J* = 6.4 Hz, 1H), 4.8 Hz, 2H), 4.01 (q, *J* = 10.4 Hz, 2H), 3.84 (s, 3H), 3.64 - 3.60 (m, 3H), 2.75 (d, *J* = 4.4 Hz, 3H), 2.50 - 2.48 (m, 2H), 2.37 (s, 2H), 2.14 (br s, 1H), 1.86 - 1.73 (m, 2H), 1.70 - 1.49 (m, 6H), 0.67 - 0.58 (m, 2H), 0.48 (t, *J* = 3.6 Hz, 2H).

Compound **32a:** Reverse phase flash chromatography column type: Waters-Xbridge-C18-10μm-19*250mm; mobile phase: A: 10mM ammonium bicarbonate/water B: acetonitrile; flow rate: 25; retention time: 6.87 min. **MS m/z (ESI):** 625.3 $[M+H]^+$. **$^1$H NMR** (400 MHz, DMSO-$d_6$) δ 8.11 (d, *J* = 4.8 Hz, 1H), 7.69 (d, *J* = 6.8 Hz, 1H), 7.40 (dd, *J* = 2.0, 8.4 Hz, 1H), 7.34 (d, *J* = 2.0 Hz, 1H), 6.79 (t, *J* = 7.2 Hz, 1H), 6.72 (d, *J* = 8.4 Hz, 1H), 6.19 (d, *J* = 7.6 Hz, 1H), 5.96 (t, *J* = 6.4 Hz, 1H), 5.66 (d, *J* = 8.4 Hz, 1H), 4.23 (d, *J* = 6.4 Hz, 2H), 4.00 (q, *J* = 10.4 Hz, 2H), 3.85 - 3.82 (m, 4H), 3.61 - 3.59 (m, 2H), 2.75 (d, *J* = 4.4 Hz, 3H), 2.43 (s, 2H), 2.17 (br s, 1H), 2.02 - 1.98 (m, 2H), 1.88 - 1.84 (m, 2H), 1.38 - 1.20 (m, 6H), 0.62 - 0.59 (m, 2H), 0.50 - 0.41 (m, 2H).

Preparation Example 33: Preparation of Compound **33**

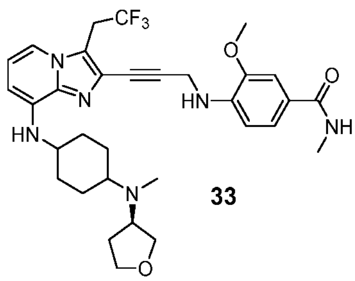

**33**

Synthesis scheme:

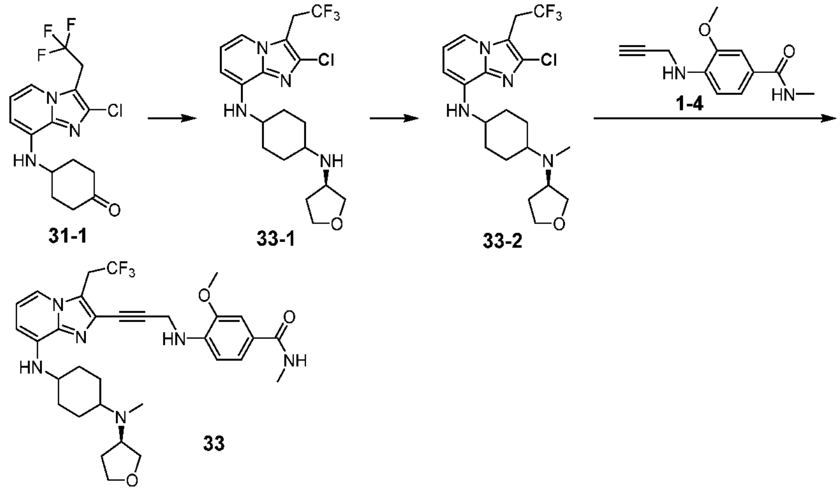


31-1 33-1 33-2 33

Step 1: Synthesis of Compound 33-1

Sodium triacetoxyborohydride (183 mg, 0.87 mmol) was added to a solution of Compound 31-1 (100 mg, 0.29 mmol), (R)-3-aminotetrahydrofuran hydrochloride (72 mg, 0.58 mmol) and triethylamine (0.08 mL, 0.58 mmol) in dichloromethane (10 mL), and the reaction mixture was stirred at room temperature for 2 hours. The reaction solution was diluted with water (10 mL), extracted with ethyl acetate (10 mL x3), and the organic layers were combined and washed with saturated brine (10 mL), dried over anhydrous sodium sulfate, filtered, and the filtrate was concentrated under reduced pressure. The crude product was purified by silica gel column chromatography to obtain Compound **33-1. MS m/z (ESI):** 417.2 $[M+H]^+$. **$^1$H NMR** (400 MHz, DMSO-*$d_6$*) δ 7.88 - 7.86 (m, 1H), 6.92 - 6.90 (m, 1H), 6.37 - 6.35 (m, 1H), 5.46 (d, *J* = 6.8 Hz, 1H), 4.18 - 4.11 (m, 2H), 3.98 - 3.86 (m, 2H), 3.84 - 3.71 (m, 3H), 3.69 - 3.61 (m, 1H), 3.21 - 2.99 (m, 1H), 2.28 - 2.17 (m, 1H), 2.15 - 2.02 (m, 1H), 1.99 - 1.81 (m, 4H), 1.74 - 1.60 (m, 3H), 1.52 - 1.33 (m, 2H).

Step 2: Synthesis of Compound **33-2**

Sodium cyanoborohydride (24 mg, 0.38 mmol) was added to a solution of Compound **33-1** (31 mg, 0.07 mmol), paraformaldehyde (4 mg, 0.13 mmol) and acetic acid (0.01 mL, 0.01 mmol) in methanol (2 mL), and the reaction mixture was stirred at 50 °C for 20 minutes. The reaction solution was cooled to room temperature and concentrated. The crude product was purified by silica gel plate chromatography to obtain Compound **33-2. MS m/z (ESI):** 431.3 $[M+H]^+$.

Step 3: Synthesis of Compound **33**

Bis(acetonitrile)palladium chloride (3 mg, 0.01 mmol), 2-dicyclohexylphosphino- 2',4',6'-triisopropylbiphenyl (9 mg, 0.02 mmol) and cesium carbonate (31 mg, 0.10 mmol) were added to a solution of Compound **33-2** (20 mg, 0.05 mmol) in acetonitrile (1 mL). After the mixture was stirred at 90 °C for 10 minutes under nitrogen protection, Compound **1-4** (45 mg, 0.21 mmol) was added. The mixture was stirred at 90 °C for 2 hours under nitrogen protection. After the reaction solution was cooled to room temperature, it was filtered, the filter cake was washed with ethyl acetate (4 mL x3), and the filtrate was concentrated under reduced pressure. The crude product was purified by reverse phase flash chromatography to obtain Compound **33. MS m/z (ESI):** 613.3 $[M+H]^+$. **$^1$H NMR** (400 MHz, DMSO-*$d_6$*) δ 8.12 (d, *J* = 4.8 Hz, 1H), 7.76 (d, *J* = 6.8 Hz, 1H), 7.41 (dd, *J* = 2.0, 8.4 Hz, 1H), 7.35 (d, *J* = 2.0 Hz, 1H), 6.82 (t, *J* = 7.2 Hz, 1H), 6.74 (d, *J* = 8.4 Hz, 1H), 6.22 (d, *J* = 7.6 Hz, 1H), 5.99 (t, *J* = 6.0 Hz, 1H), 5.71 - 5.50 (m, 1H), 4.27 (d, *J* = 6.4 Hz, 2H), 4.07 - 4.00 (m, 2H), 3.84 (s, 3H), 3.81 - 3.67 (m, 2H), 3.67 - 3.57 (m, 1H), 3.49 - 3.40 (m, 1H), 2.97 - 2.87 (m, 1H), 2.75 (d, *J* = 4.4 Hz, 3H), 2.47 (s, 3H), 2.06 - 1.41 (m, 12H).

Preparation Example 34: Preparation of Compound **34**

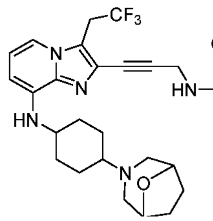

**34**

Synthesis scheme:

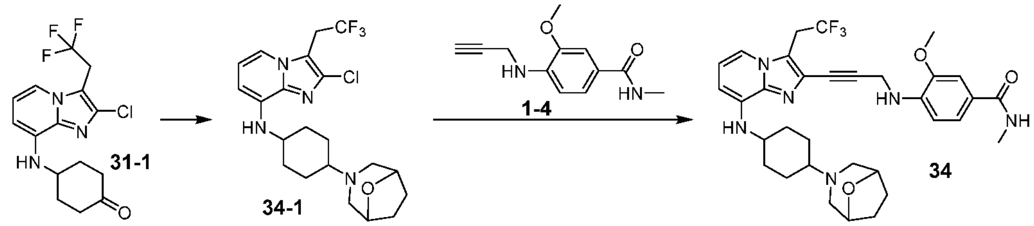

**31-1** **34-1** **1-4** **34**

Step 1: Synthesis of Compound **34-1**

Sodium triacetoxyborohydride (183 mg, 0.87 mmol) was added to a solution of Compound **31-1** (100 mg, 0.29 mmol), 8-oxo-3-azabicyclo[3,2,1]octane hydrochloride (87 mg, 0.58 mmol) and triethylamine (0.08 mL, 0.58 mmol) in dichloromethane (10 mL), and the reaction mixture was stirred at room temperature for 2 hours. The reaction solution was diluted with water (10 mL), extracted with ethyl acetate (10 mL x3), and the organic layers were combined and washed with saturated brine (10 mL), dried over anhydrous sodium sulfate, filtered, and the filtrate was concentrated under reduced pressure. The crude product was purified by silica gel column chromatography to obtain Compound **34-1. MS m/z (ESI):** 443.2 $[M+H]^+$.

Step 2: Synthesis of Compound **34**

To a solution of Compound **34-1** (90 mg, 0.20 mmol) in acetonitrile (6 mL), bis(acetonitrile)palladium chloride (11 mg, 0.04 mmol), 2-dicyclohexylphosphino- 2',4',6'-triisopropylbiphenyl (40 mg, 0.08 mmol) and cesium carbonate (130 mg, 0.40 mmol) were added. The mixture was stirred at 90 °C for 10 minutes under nitrogen protection, and Compound **1-4** (45 mg, 0.21 mmol) was added. The mixture was stirred at 90 °C for 2 hours under nitrogen protection. After the reaction solution was cooled to room temperature, it was filtered, the filter cake was washed with ethyl acetate (4 mL x3), and the filtrate was concentrated under reduced pressure. The crude product was purified by reverse phase flash chromatography to obtain Compound **34. MS m/z (ESI):** 625.4 $[M+H]^+$. **$^1$H NMR** (400 MHz, DMSO-$d_6$) δ 8.16 - 8.03 (m, 1H), 7.79 - 7.70 (m, 1H), 7.41 (d, $J$ = 8.0 Hz, 1H), 7.35 (s, 1H), 6.86 - 6.77 (m, 1H), 6.73 (d, $J$ = 8.0 Hz, 1H), 6.25 - 6.20 (m, 1H), 6.02 - 5.97 (m, 1H), 5.67 - 5.41 (m, 1H), 4.27 - 4.21 (m, 2H), 4.12 - 3.97 (m, 2H), 3.84 (s, 3H), 3.18 - 3.00 (m, 2H), 2.96 - 2.88 (m, 1H), 2.75 (d, $J$ = 4.4 Hz, 3H), 2.70 - 2.58 (m, 1H), 2.35 - 2.24 (m, 2H), 2.14 - 2.06 (m, 2H), 2.02 - 1.87 (m, 4H), 1.73 - 1.41 (m, 8H).

Preparation Example 35: Preparation of Compounds **35-6aa, 35-6ab, 35-6ba, 35-6bb, 35-6ca, 35-6cb, 35-6da** and **35db**

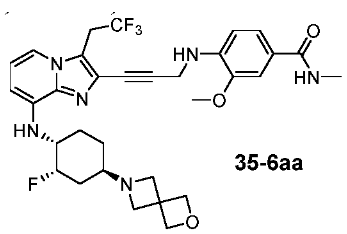

**35-6aa**

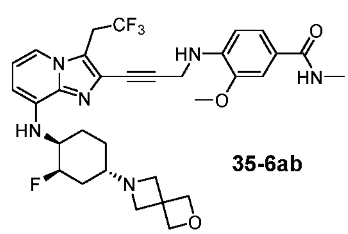

**35-6ab**

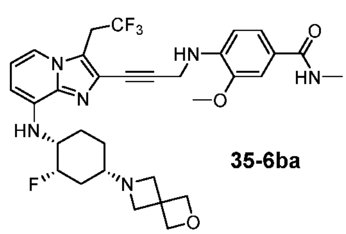

**35-6ba**

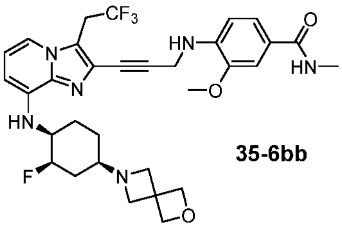

35-6bb

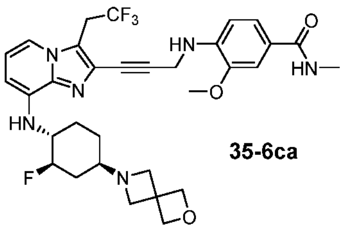

35-6ca

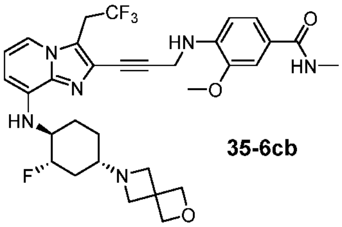

35-6cb

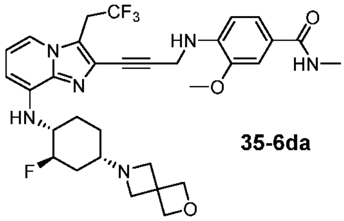

35-6da

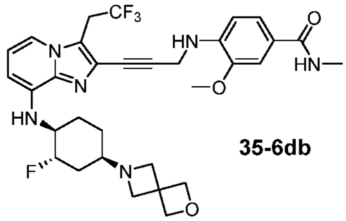

35-6db

Synthesis scheme:

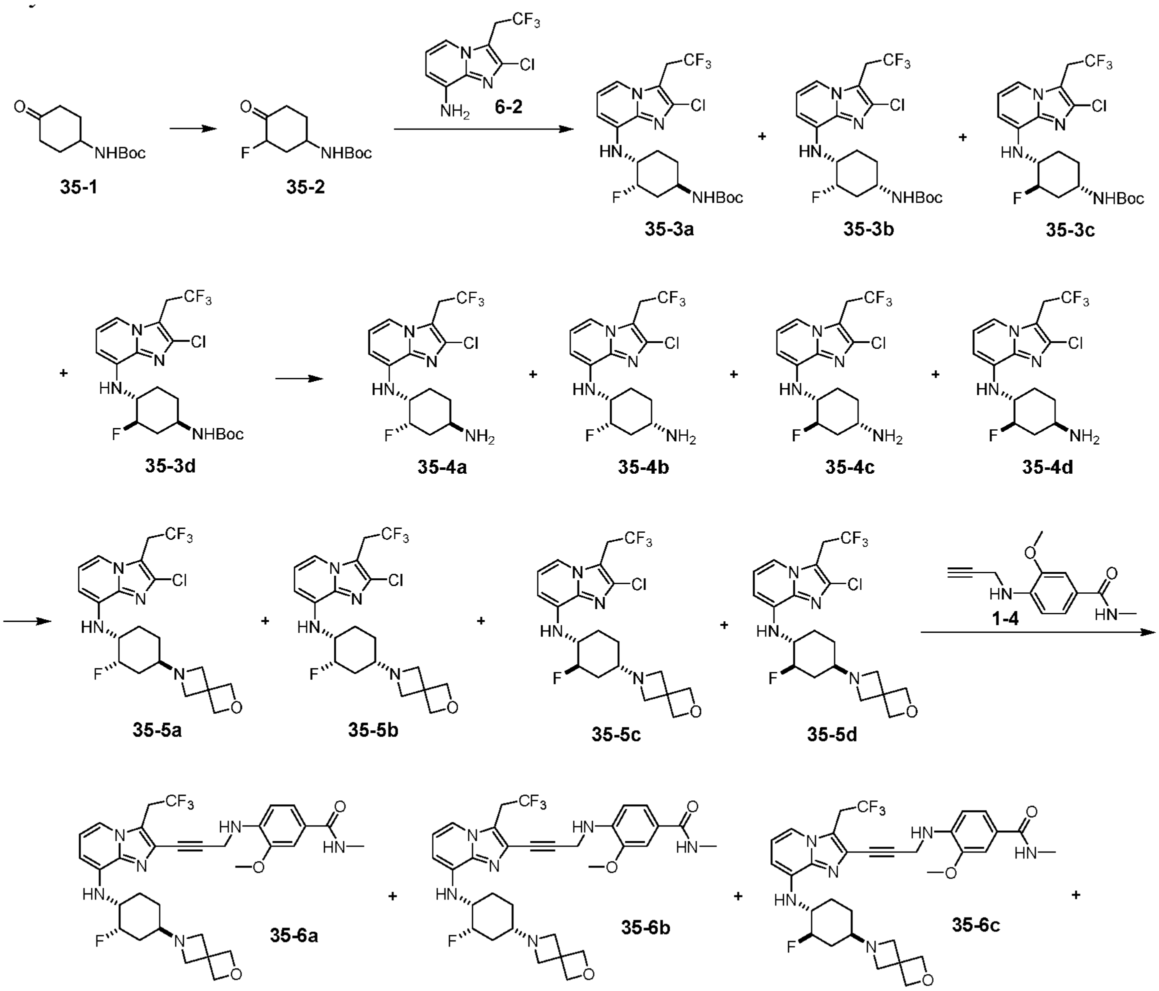

6-2

35-1 35-2 35-3a + 35-3b + 35-3c

+ 35-3d 35-4a + 35-4b + 35-4c + 35-4d

35-5a + 35-5b + 35-5c + 35-5d

1-4

35-6a + 35-6b + 35-6c +

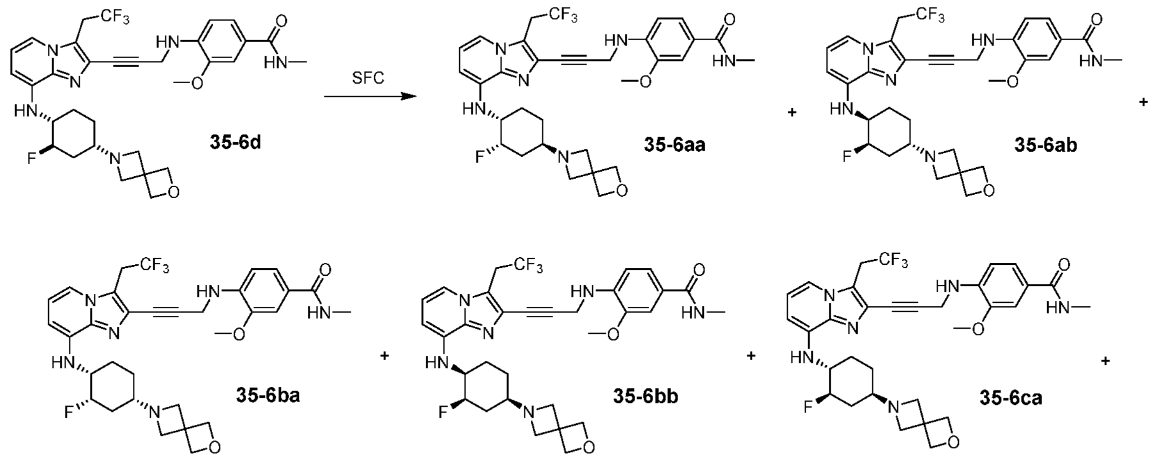


35-6d 35-6aa + 35-6ab + 35-6ba + 35-6bb + 35-6ca +

Step 1: Synthesis of Compound **35-2**

Under nitrogen protection and in an ice bath, lithium bis(trimethylsilyl)amide (2.21 mL, 2.21 mmol, 1 M tetrahydrofuran solution) was added to a solution of Compound **35-1** (200 mg, 0.94 mmol) in tetrahydrofuran (20 mL). The reaction solution was stirred at 0 °C for 0.5 hours, and trimethylsilyl chloride (0.13 mL, 1.02 mmol) was added to the reaction solution. After the addition was completed, the reaction solution was stirred at room temperature for 1 hour under nitrogen protection. A solution of 1-chloromethyl-4-fluoro-1,4-diazabicyclo[2.2.2]octane di(tetrafluoroborate) (1.17 g, 3.29 mmol) in acetonitrile (27 mL) was added to the reaction solution. After the addition was completed, the reaction solution was stirred at room temperature under nitrogen protection for 8 hours. The reaction solution was quenched with water (50 mL), extracted with ethyl acetate (50 mL x 3), and the organic layers were combined and washed with saturated brine (200 mL), dried over anhydrous sodium sulfate, and filtered. The filtrate was concentrated under reduced pressure. The crude product was purified by silica gel column chromatography to obtain Compound **35-2.** **$^1$H NMR** (400 MHz, $CDCl_3$) δ 5.07 - 4.79 (m, 1H), 4.59 (br s, 1H), 4.26 - 3.97 (m, 1H), 2.78 - 2.65 (m, 1H), 2.60 - 2.10 (m, 3H), 1.99 - 1.75 (m, 1H), 1.68 - 1.58 (m, 1H), 1.46 (s, 9H).

Step 2: Synthesis of Compounds **35-3e, 35-3f** and **35-3g**

To a solution of Compound **6-2** (485 mg, 1.85 mmol) and Compound **35-2** (540 mg, 2.22 mmol) in N,N-dimethylformamide (25 mL), trimethylsilyl chloride (0.62 mL, 4.89 mmol) was added. The reaction solution was stirred at 0 °C for 2 hours under nitrogen protection. Borane tetrahydrofuran (10 mL, 10.00 mmol, 1 M tetrahydrofuran solution) was added dropwise to the above reaction solution, and the reaction solution was stirred at room temperature for 1 hour. The reaction solution was quenched with saturated sodium carbonate aqueous solution (50 mL), extracted with ethyl acetate (100 mL x3), the organic phases were combined and washed with saturated brine (300 mL), dried over anhydrous sodium sulfate, filtered, and the filtrate was concentrated under reduced pressure. The crude product was purified by rapid silica gel column chromatography to obtain isomers of Compounds **35-3e, 35-3f** and **35-3g.** Among them, **35-3e** and **35-3f** were single isomers selected from the group consisting of Compounds **35-3a, 35-3b, 35-3c** and **35-3d; 35-3g** was composed of two isomers of Compounds **35-3a, 35-3b, 35-3c,** and **35-3d** except **35-3e** and **35-3f.**

Compound **35-3e: MS m/z (ESI):** 465.2 $[M+H]^+$. **$^1$H NMR** (400 MHz, $CDCl_3$) δ 7.39 (d, *J* = 6.8 Hz, 1H), 6.80 (t, *J* = 7.2 Hz, 1H), 6.31 (d, *J* = 7.6 Hz, 1H), 5.10 (d, *J* = 7.2 Hz, 1H), 4.91 - 4.69 (m, 1H), 4.45 (br s, 1H), 3.92 (br s, 1H), 3.77 - 3.62 (m, 3H), 2.22 - 2.07 (m, 2H), 1.94 - 1.86 (m, 1H), 1.82 - 1.73 (m, 1H), 1.70 - 1.55 (m, 2H), 1.46 (s, 9H).

Compound **35-3f: MS m/z (ESI):** 465.2 $[M+H]^+$. **$^1$H NMR** (400 MHz, $CDCl_3$) δ 7.36 (d, *J* = 6.8 Hz, 1H), 6.78 (t, *J* = 7.2 Hz, 1H), 6.34 (d, *J* = 7.6 Hz, 1H), 5.13 (d, *J* = 7.2 Hz, 1H), 4.66 - 4.35 (m, 2H), 3.78 - 3.62 (m, 3H), 3.59 - 3.42 (m, 1H), 2.53 - 2.44 (m, 1H), 2.26 - 2.13 (m, 1H), 1.65 - 1.53 (m, 2H), 1.46 (s, 9H), 1.40 - 1.24 (m, 2H).

Compound **35-3g: MS m/z (ESI):** 465.2 $[M+H]^+$. **$^1$H NMR** (400 MHz, $CDCl_3$) δ 7.36 (d, *J* = 6.8 Hz, 1H), 6.77 (t, *J* = 7.2 Hz, 1H), 6.15 (d, *J* = 7.6 Hz, 1H), 5.48 - 5.33 (m, 1H), 4.99 (d, *J* = 49.2 Hz, 1H), 4.62 - 4.29 (m, 1H), 4.00 - 3.78 (m, 1H), 3.70 (q, *J* = 10.0 Hz, 2H), 3.59 - 3.33 (m, 1H), 2.59 - 2.35 (m, 1H), 2.32 - 2.07 (m, 1H), 2.03 - 1.95 (m, 1H), 1.91 - 1.75 (m, 1H), 1.45 (s, 9H), 1.40 - 1.22 (m, 2H).

Step 3: Synthesis of **35-6e, 35-6f, 35-6g, 35-6h, 35-6i** and **35-6j**

Compounds **35-6e, 35-6f, 35-6g, 35-6h, 35-6i** and **35-6j** were isomers of each other; wherein **35-6g, 35-6h, 35-6i** and **35-6j** were single isomers different from each other, selected from the group consisting of **35-6aa, 35-6ab, 35-6ba, 35**

**-6bb, 35-6ca, 35-6cb, 35-6da,** and **35-6db;** among **35-6aa, 35-6ab, 35-6bb, 35-6ca, 35-6cb, 35-6da, 35-6db,** except **35-6g, 35-6h, 35-6i** and 35-6j, the other 4 isomers were divided into 2 groups, each group had 2 isomers, namely **35-6e** and **35-6f.**

Compound **35-6e:** synthesized from Compound **35-3e** by referring to the method of Preparation Example 9. **MS m/z (ESI):** 629.3 [M+H]$^+$. **$^1$H NMR** (400 MHz, DMSO-$d_6$) δ 8.09 (q, *J* = 4.8 Hz, 1H), 7.71 (d, *J* = 6.8 Hz, 1H), 7.41 (dd, *J* = 2.0, 8.4 Hz, 1H), 7.34 (d, *J* = 2.0 Hz, 1H), 6.78 (t, *J* = 7.2 Hz, 1H), 6.74 (d, *J* = 8.4 Hz, 1H), 6.28 (d, *J* = 7.6 Hz, 1H), 5.95 (t, *J* = 6.4 Hz, 1H), 5.85 (d, *J* = 8.4 Hz, 1H), 4.90 - 4.66 (m, 1H), 4.59 (s, 4H), 4.27 (d, *J* = 6.4 Hz, 2H), 4.00 (q, *J* = 10.4 Hz, 2H), 3.84 (s, 3H), 3.61 (br s, 1H), 3.22 (br s, 4H), 2.75 (d, *J* = 4.4 Hz, 3H), 2.41 - 2.37 (m, 1H), 2.00 - 1.84 (m, 1H), 1.79 - 1.51 (m, 3H), 1.51 - 1.38 (m, 2H).

Compound **35-6f:** synthesized from Compound **35-3f** by referring to the method of Preparation Example 9. **MS m/z (ESI):** 629.3 [M+H]$^+$. **$^1$H NMR** (400 MHz, DMSO-$d_6$) δ 8.09 (d, *J* = 4.8 Hz, 1H), 7.69 (d, *J* = 6.4 Hz, 1H), 7.40 (dd, *J* = 2.0, 8.4 Hz, 1H), 7.34 (d, *J* = 2.0 Hz, 1H), 6.77 (t, *J* = 7.2 Hz, 1H), 6.73 (d, *J* = 8.4 Hz, 1H), 6.27 (d, *J* = 7.6 Hz, 1H), 6.04 (d, *J* = 9.2 Hz, 1H), 5.95 (t, *J* = 6.4 Hz, 1H), 4.70 - 4.52 (m, 5H), 4.26 (d, *J* = 6.4 Hz, 2H), 4.01 (q, *J* = 10.8 Hz, 2H), 3.84 (s, 3H), 3.62 - 3.48 (m, 1H), 3.26 (s, 4H), 2.75 (d, *J* = 4.4 Hz, 3H), 2.17 - 2.07 (m, 1H), 2.06 - 1.96 (m, 1H), 1.94 - 1.83 (m, 1H), 1.68 - 1.54 (m, 1H), 1.39 - 1.12 (m, 2H), 1.09 - 0.94 (m, 1H).

Compounds **35-6g, 35-6h, 35-6i** and **35-6j** were synthesized from Compound **35-3g** by referring to the method of Preparation Example 9, and were prepared and purified by SFC.

Compound **35-6g:** SFC method: Instrument: Waters UPC2 analytical SFC (SFC-H); Column: Whelk O1(S, S), 100×4.6mm I.D., 3μm; Mobile phase: A for $CO_2$ and B for Ethanol (0.05%DEA); Gradient: B 50%; Flow rate: 2.0 mL/min; Back pressure: 100 bar; Column temperature: 35°C; Wavelength: 220nm. Retention time: 6.860 min. **MS m/z (ESI):** 629.3 [M+H]$^+$. **$^1$H NMR** (400 MHz, DMSO-$d_6$) δ 8.10 (d, *J* = 4.8 Hz, 1H), 7.77 (d, *J* = 6.8 Hz, 1H), 7.41 (dd, *J* = 2.0, 8.0 Hz, 1H), 7.35 (d, *J* = 2.0 Hz, 1H), 6.82 (t, *J* = 7.2 Hz, 1H), 6.75 (d, *J* = 8.4 Hz, 1H), 6.35 (d, *J* = 7.6 Hz, 1H), 5.95 (t, *J* = 6.4 Hz, 1H), 5.24 (d, *J* = 8.4 Hz, 1H), 4.81 (d, *J* = 47.2 Hz, 1H), 4.59 (s, 4H), 4.27 (d, *J* = 6.4 Hz, 2H), 4.03 - 3.91 (m, 3H), 3.84 (s, 3H), 3.28 - 3.06 (m, 4H), 2.75 (d, *J* = 4.4 Hz, 3H), 2.20 - 2.02 (m, 1H), 1.95 - 1.76 (m, 2H), 1.67 - 1.39 (m, 3H), 1.21 - 1.08 (m, 1H).

Compound **35-6h:** SFC method: Instrument: Waters UPC2 analytical SFC (SFC-H); Column: Whelk O1(S, S), 100×4.6mm I.D., 3μm; Mobile phase: A for $CO_2$ and B for Ethanol (0.05%DEA); Gradient: B 50%; Flow rate: 2.0 mL/min; Back pressure: 100 bar; Column temperature: 35°C; Wavelength: 220nm. Retention time: 8.153 min. **MS m/z (ESI):** 629.3 [M+H]$^+$. **$^1$H NMR** (400 MHz, DMSO-$d_6$) δ 8.10 (d, *J* = 4.4 Hz, 1H), 7.78 (d, *J* = 6.8 Hz, 1H), 7.41 (dd, *J* = 2.0, 8.0 Hz, 1H), 7.35 (d, *J* = 2.0 Hz, 1H), 6.82 (t, *J* = 7.2 Hz, 1H), 6.75 (d, *J* = 8.4 Hz, 1H), 6.36 (d, *J* = 7.6 Hz, 1H), 5.95 (t, *J* = 6.4 Hz, 1H), 5.25 (br s, 1H), 4.81 (d, *J* = 48.0 Hz, 1H), 4.59 (s, 4H), 4.27 (d, *J* = 6.4 Hz, 2H), 4.03 - 3.88 (m, 3H), 3.84 (s, 3H), 3.28 - 3.05 (m, 4H), 2.75 (d, *J* = 4.4 Hz, 3H), 2.19 - 2.02 (m, 1H), 1.97 - 1.77 (m, 2H), 1.71 - 1.39 (m, 3H), 1.21 - 1.02 (m, 1H).

Compound **35-6i:** SFC method: Instrument: Waters UPC2 analytical SFC (SFC-H); Column: ChiralPak IC, 100×4.6mm I.D., 3μm; Mobile phase: A for $CO_2$ and B for Ethanol (0.05%DEA); Gradient: B 50%; Flow rate: 2.0 mL/min; Back pressure: 100 bar; Column temperature: 35°C; Wavelength: 220nm. Retention time: 7.008 min. **MS m/z (ESI):** 629.5 [M+H]$^+$. **$^1$H NMR** (400 MHz, DMSO-$d_6$) δ 8.09 (d, *J* = 4.4 Hz, 1H), 7.77 (d, *J* = 6.8 Hz, 1H), 7.41 (dd, *J* = 2.0, 8.0 Hz, 1H), 7.34 (d, *J* = 2.0 Hz, 1H), 6.82 (t, *J* = 7.2 Hz, 1H), 6.73 (d, *J* = 8.4 Hz, 1H), 6.35 (d, *J* = *7.6* Hz, 1H), 5.95 (t, *J* = 6.4 Hz, 1H), 5.35 (d, *J* = 9.2 Hz, 1H), 4.93 (d, *J* = 50.0 Hz, 1H), 4.58 (s, 4H), 4.26 (d, *J* = 6.4 Hz, 2H), 4.01 (q, *J* = 10.8 Hz, 2H), 3.84 (s, 3H), 3.77 - 3.54 (m, 1H), 3.23 (br s, 4H), 2.75 (d, *J* = 4.4 Hz, 3H), 2.28 - 2.12 (m, 1H), 2.12 - 1.95 (m, 1H), 1.87 - 1.66 (m, 2H), 1.62 - 1.46 (m, 1H), 1.44 - 1.19 (m, 1H), 1.17 - 0.99 (m, 1H).

Compound **35-6j:** SFC method: Instrument: Waters UPC2 analytical SFC (SFC-H); Column: ChiralPak IC, 100×4.6mm I.D., 3μm; Mobile phase: A for $CO_2$ and B for Ethanol (0.05%DEA); Gradient: B 50%; Flow rate: 2.0 mL/min; Back pressure: 100 bar; Column temperature: 35°C; Wavelength: 220nm. Retention time: 8.955 min. **MS m/z (ESI):** 629.4 [M+H]$^+$. **$^1$H NMR** (400 MHz, DMSO-$d_6$) δ 8.09 (d, *J* = 4.4 Hz, 1H), 7.77 (d, *J* = 6.8 Hz, 1H), 7.41 (dd, *J* = 2.0, 8.0 Hz, 1H), 7.34 (d, *J* = 2.0 Hz, 1H), 6.82 (t, *J* = 7.2 Hz, 1H), 6.73 (d, *J* = 8.4 Hz, 1H), 6.35 (d, *J* = *7.6* Hz, 1H), 5.95 (t, *J* = 6.4 Hz, 1H), 5.36 (d, *J* = 9.2 Hz, 1H), 4.93 (d, *J* = 50.0 Hz, 1H), 4.58 (s, 4H), 4.26 (d, *J* = 6.4 Hz, 2H), 4.01 (q, *J* = 10.8 Hz, 2H), 3.84 (s, 3H), 3.76 - 3.59 (m, 1H), 3.29 - 3.12 (m, 4H), 2.75 (d, *J* = 4.4 Hz, 3H), 2.29 - 2.13 (m, 1H), 2.11 - 1.94 (m, 1H), 1.86 - 1.65 (m, 2H), 1.63 - 1.48 (m, 1H), 1.44 - 1.20 (m, 1H), 1.17 - 0.99 (m, 1H).

Preparation Example 36: Preparation of Compound **36**

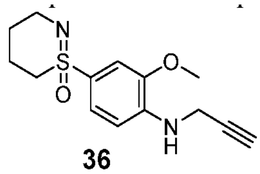

**36**

Synthesis scheme:

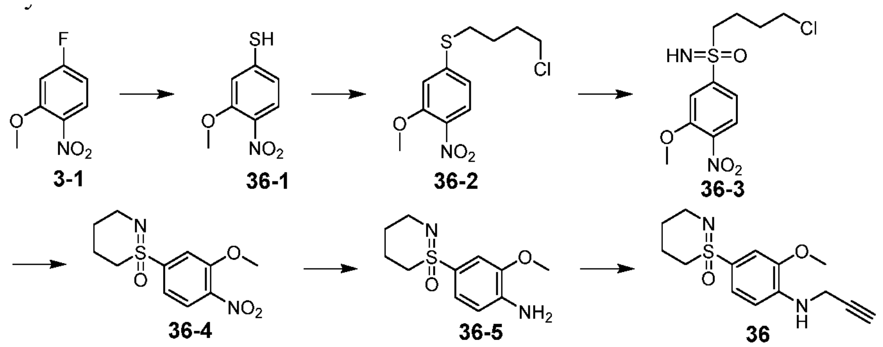

Step 1: Synthesis of Compound **36-1**

Under nitrogen protection, sublimed sulfur (1.4 g, 43.75 mmol), sodium sulfide nonahydrate (10.1 g, 105.15 mmol) and sodium hydroxide (2.3 g, 58.50 mmol) were added to a solution of Compound **3-1** (10.0 g, 58.44 mmol) in ethanol (200 mL). Under nitrogen protection, the mixture was stirred at 80 °C for 2 hours. After the reaction solution was cooled to room temperature, it was poured into dilute hydrochloric acid (300 mL, 1.0 M) and extracted with ethyl acetate (300 mL x3). The combined organic phase was washed with saturated brine (500 mL), dried over anhydrous sodium sulfate, filtered, and the filtrate was concentrated under reduced pressure to obtain Compound **36-1.** The crude product was directly used in the next reaction. **MS m/z (ESI):** 186.0 $[M+H]^+$. **$^1$H NMR** (400 MHz, $CDCl_3$) $\delta$ 7.80 (d, *J* = 8.4 Hz, 1H), 6.91 (d, *J* = 2.0 Hz, 1H), 6.86 (dd, *J* = 2.0, 8.4 Hz, 1H), 3.95 (s, 3H), 3.72 (s, 1H).

Step 2: Synthesis of Compound **36-2**

Under nitrogen protection, potassium carbonate (8.9 g, 64.76 mmol) and 1-bromo-3-chloropropane (5.0 mL, 43.28 mmol) were added to a solution of Compound **36-1** (8.0 g, 43.20 mmol) in N,N-dimethylformamide (200 mL). Under nitrogen protection, the mixture was stirred at room temperature for 2 hours. The reaction solution was poured into water (800 mL) and extracted with ethyl acetate (200 mL x3). The organic phases were combined and washed with saturated brine (300 mL), dried over anhydrous sodium sulfate, filtered, and the filtrate was concentrated under reduced pressure to obtain Compound **36-2.** The crude product was used directly in the next reaction. **MS m/z (ESI):** 276.0 $[M+H]^+$.

Step 3: Synthesis of Compound **36-3**

Under nitrogen protection, iodosophenyl diacetate (29.0 g, 89.48 mmol) and ammonium carbamate (7.1 g, 90.94 mmol) were added to a solution of Compound **36-2** (5.0 g, 18.13 mmol) in methanol (50 mL). Under nitrogen protection, the mixture was stirred at room temperature for 2 hours. The reaction solution was poured into a saturated sodium bicarbonate solution (100 mL), extracted with ethyl acetate (100 mL x3), and the organic phases were combined and washed with saturated brine (300 mL), dried over anhydrous sodium sulfate, filtered, and the filtrate was concentrated under reduced pressure. The crude product was purified by silica gel column chromatography to obtain Compound **36-3. MS m/z (ESI):** 307.2 $[M+H]^+$.

Step 4: Synthesis of Compound **36-4**

Under nitrogen protection, sodium hydride (753 mg, 18.83 mol) was added in batches to a solution of Compound **36-3** (3.6 g, 11.74 mol) in N,N-dimethylformamide (72 mL). The mixture was stirred at room temperature for 2 hours under nitrogen protection. The reaction solution was poured into water (200 mL) and extracted with ethyl acetate (200 mL x3). The combined organic phase was washed with saturated brine (500 mL), dried over anhydrous sodium sulfate, filtered, and the filtrate was concentrated under reduced pressure. The crude product was purified by silica gel column chromatography to obtain Compound **36-4. MS m/z (ESI):** 271.0 $[M+H]^+$. **$^1$H NMR** (400 MHz, $CDCl_3$) $\delta$ 7.91 (d, *J* = 8.4 Hz, 1H), 7.81 (d, *J* = 1.6 Hz, 1H), 7.72 (dd, *J* = 1.6, 8.4 Hz, 1H), 4.05 (s, 3H), 3.75 - 3.64 (m, 1H), 3.59 - 3.51 (m, 1H), 3.26 - 3.18 (m, 1H), 2.99 - 2.90 (m, 1H), 2.63 - 2.48 (m, 1H), 2.31 - 2.20 (m, 1H), 1.85 - 1.75 (m, 2H).

Steps 5 and 6: Referring to the synthesis method of Compound **15-4,** Compounds **36-5** and **36** were prepared in sequence.

Compound **36: MS m/z (ESI):** 279.2 [M+H]+. **¹H NMR** (400 MHz, $CDCl_3$) δ 7.62 (dd, *J* = 2.0, 8.4 Hz, 1H), 7.39 (d, *J* = 2.0 Hz, 1H), 6.72 (d, *J* = 8.4 Hz, 1H), 5.00 - 4.96 (m, 1H), 4.02 (dd, *J* = 2.4, 6.0 Hz, 2H), 3.93 (s, 3H), 3.77 - 3.68 (m, 1H), 3.56 - 3.46 (m, 1H), 3.18 - 3.11 (m, 1H), 2.95 - 2.84 (m, 1H), 2.61 - 2.45 (m, 1H), 2.27 - 2.15 (m, 2H), 1.80 - 1.70 (m, 2H).

Preparation Example 37: Preparation of Compounds **37a, 37b, 37c** and **37d**

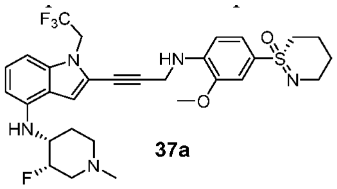

**37a**

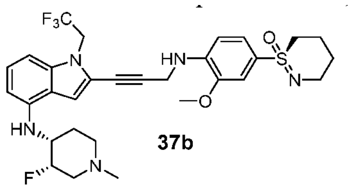

**37b**

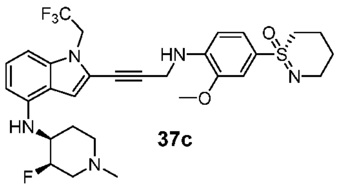

**37c**

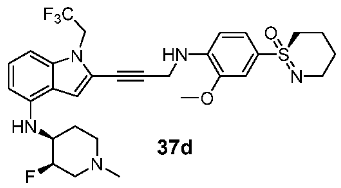

**37d**

Synthesis scheme:

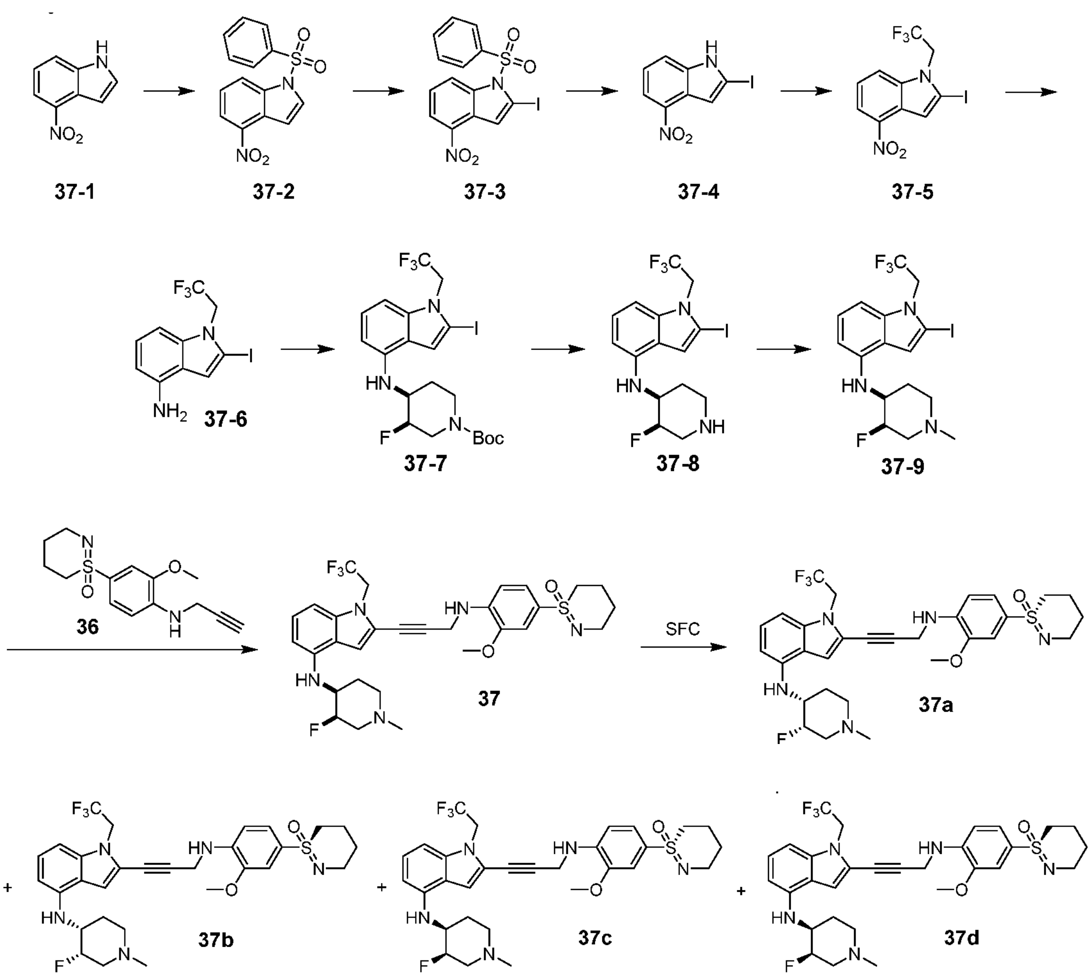


**37-1** **37-2** **37-3** **37-4** **37-5** **37-6** **37-7** **37-8** **37-9** **36** **37** **37a** **37b** **37c** **37d**

Step 1: Synthesis of Compound **37-2**

At 0 °C, sodium hydride (14.8 g, 370.03 mmol) was added in batches to a solution of Compound **37-1** (40.0 g, 246.69 mmol) in N,N-dimethylformamide (800 mL), and the reaction solution was stirred at 0 °C for 30 minutes. Benzenesulfonyl chloride (40 mL, 312.55 mmol) was added dropwise to the reaction solution, and the reaction solution was stirred at room temperature for 2 hours. The reaction solution was quenched with saturated ammonium chloride aqueous solution (100 mL), diluted with water (2.0 L), and extracted with ethyl acetate (300 ml x3). The combined organic phase was washed with saturated brine (600 mL), dried over anhydrous sodium sulfate, and filtered. The filtrate was concentrated under reduced pressure to obtain Compound **37-2.** The crude product was directly used in the next step. **$^1$H NMR** (400 MHz, $CDCl_3$) δ 8.35 (d, *J* = 8.4 Hz, 1H), 8.20 (d, *J* = 8.0 Hz, 1H), 7.93 - 7.87 (m, 2H), 7.83 (d, *J* = 3.6 Hz, 1H), 7.64 - 7.56 (m, 1H), 7.52 - 7.41 (m, 4H).

Step 2: Synthesis of Compound **37-3**

Compound **37-2** (69.0 g, 228.25 mmol) was dissolved in tetrahydrofuran (800 mL), and lithium diisopropylamide (230 mL, 460.00 mmol) was added at -78 °C. The reaction solution was stirred at -78 °C for 1 hour. A solution of iodine (116 g, 457.03 mmol) in tetrahydrofuran (200 mL) was added dropwise to the reaction solution. The reaction solution was stirred at -78 °C for 1 hour. The reaction solution was quenched with saturated ammonium chloride solution (300 mL) and diluted with water (500 mL), and extracted with ethyl acetate (300 mL x3). The organic layers were combined and washed with sodium thiosulfate aqueous solution (500 mL) and saturated brine (500 mL), dried over anhydrous sodium sulfate and filtered, and the filtrate was concentrated under reduced pressure to obtain Compound **37-3.** The crude product was used directly in the next step. **MS m/z (ESI):** 426.8 $[M-H]^-$.

Step 3: Synthesis of Compound **37-4**

Potassium carbonate (106.5 g, 770.7 mmol) was added to a solution of Compound **37-3** (110.0 g, 256.89 mmol) in methanol (300 mL), and the reaction solution was stirred at 80 °C for 2 hours. After the reaction solution was cooled to room temperature, it was filtered, the filtrate was concentrated under reduced pressure, the crude product was diluted with water (300 mL), extracted with ethyl acetate (100 mL x2), the combined organic phase was washed with saturated brine (100 mL), dried over anhydrous sodium sulfate, and filtered, and the filtrate was concentrated under reduced pressure. The crude product was purified by silica gel chromatography to obtain Compound **37-4. MS m/z (ESI):** 288.8 $[M+H]^+$.

Step 4: Synthesis of Compound **37-5**

At 0 °C, sodium hydride (3.06 g, 76.38 mmol) was added in batches to a solution of Compound **37-4** (11.0 g, 38.19 mmol) in tetrahydrofuran (100 mL). The reaction mixture was stirred at room temperature for 10 minutes under nitrogen protection. Then 2,2,2-trifluoroethyl trifluoromethanesulfonate (11.0 mL, 76.38 mmol) was added. The reaction mixture was stirred at room temperature for 3 hours. Saturated ammonium chloride aqueous solution (100 mL) was slowly added to the reaction solution, and the resulting solution was extracted with ethyl acetate (50 ml x3). The combined organic phase was washed with saturated brine, dried over anhydrous sodium sulfate, and filtered. The filtrate was concentrated under reduced pressure. The crude product was purified by column chromatography to obtain Compound **37-5. MS m/z (ESI):** 370.8 $[M+H]^+$.

Step 5: Synthesis of Compound **37-6**

Iron powder (10.6 g, 189.15 mmol) and ammonium chloride (10.1 g, 189.15 mmol) were added to a solution of Compound **37-5** (14.0 g, 37.83 mmol) in ethanol (100 mL) and water (50 mL). The mixture was heated to 80 °C and stirred for 2 hours under nitrogen protection. The reaction mixture was filtered through diatomaceous earth while hot, and washed with methanol (60 mL). The filtrate was concentrated under reduced pressure, diluted with water (30 mL), and extracted with ethyl acetate (30 mL x3). The combined organic phase was washed with saturated brine (60 mL), dried over anhydrous sodium sulfate and filtered, and the filtrate was concentrated under reduced pressure to obtain Compound **37-6.** The crude product was directly used for the next reaction. **MS m/z (ESI):** 340.9 $[M+H]^+$.

Step 6: Synthesis of Compound **37-7**

Under nitrogen protection, trimethylsilyl chloride (3.57 mL, 27.93 mmol) was added to a solution of Compound **37-6** (2.0 g, 5.59 mmol) and tert-butyl 3-fluoro-4-oxopiperidine-1-carboxylate (2.0 g, 9.21 mmol) in N,N-dimethylformamide (60 mL). Under nitrogen protection, the mixture was stirred at room temperature for 1 hour, and then a borane

tetrahydrofuran solution (11.17 mL, 11.17 mmol) was added. Under nitrogen protection, the mixture was stirred at room temperature for 1 hour. The reaction solution was poured into water (200 mL) and extracted with ethyl acetate (150 mL x2). The combined organic phase was washed with brine (200 mL), dried over anhydrous sodium sulfate, filtered, and the filtrate was concentrated under reduced pressure. The crude product was purified by silica gel column chromatography to obtain Compound **37-7. MS m/z (ESI):** 542.0 $[M+H]^+$.

Step 7: Synthesis of Compound **37-8**

Under nitrogen protection, trifluoroacetic acid (5.0 mL, 67.09 mmol) was added to a solution of Compound **37-7** (600 mg, 0.89 mmol) in dichloromethane (25 mL). Under nitrogen protection, the mixture was stirred at 0 °C for 1 hour. The reaction solution was poured into a saturated sodium carbonate solution (30 mL), diluted with water (30 mL), and extracted with dichloromethane (60 mL x3). The combined organic phase was washed with saturated brine (100 mL), dried over anhydrous sodium sulfate, filtered, and the filtrate was concentrated under reduced pressure. The crude product was purified by silica gel column chromatography to obtain Compound **37-8. MS m/z (ESI):** 442.0 $[M+H]^+$.

Step 8: Synthesis of Compound **37-9**

Under nitrogen protection, sodium cyanoborohydride (22 mg, 0.35 mmol) was added to a solution of Compound **37-8** (155 mg, 0.32 mmol), paraformaldehyde (53 mg, 1.77 mmol) and acetic acid (19 mg, 0.31 mmol) in methanol (6.0 mL). Under nitrogen protection, the mixture was stirred at 50 °C for 2 hours. The reaction solution was poured into a saturated sodium bicarbonate solution (40 mL) and extracted with dichloromethane (40 mL x3). The combined organic phase was washed with saturated brine (100 mL), dried over anhydrous sodium sulfate and filtered, and the filtrate was concentrated under reduced pressure to obtain Compound **37-9.** The crude product was used directly in the next step. **MS m/z (ESI):** 456.2 $[M+H]^+$.

Step 9: Synthesis of Compound **37**

Under nitrogen protection, ferrocene palladium dichloride (8.0 mg, 10.93 μmol) and cuprous iodide (21 mg, 110.27 μmol) were added to a solution of Compound **37-9** (45 mg, 0.09 mmol), Compound **36** (50 mg, 0.15 mmol) and diisopropylamine (0.16 mL, 1.12 mmol) in dimethyl sulfoxide (1.7 mL). Under nitrogen protection, the mixture was stirred at 45 °C for 2 hours. After the reaction solution was cooled to room temperature, it was filtered, the filter cake was washed with acetonitrile (5.0 mL x3), and the combined filtrate was concentrated. The crude product was purified by silica gel column chromatography to obtain Compound **37. MS m/z (ESI):** 606.3 $[M+H]^+$.

Compound **37** was separated by SFC to obtain Compounds **37e, 37f, 37g** and **37h** that were isomers between each other. Compounds **37e, 37f, 37g** and **37h** were selected from the group consisting of Compounds **37a, 37b, 37c** and **37d,** respectively, and they were different from each other.

Compound **37e:** SFC method: Instrument: Waters UPC2 analytical SFC (SFC-H); Column: ChiralPak AD, 50×4.6mm I.D., 3μm; Mobile phase: A for $CO_2$ and B for Ethanol (0.05%DEA); Gradient: B 40%; Flow rate: 3 mL/min; Back pressure: 100 bar; Column temperature: 35 °C; Wavelength: 220nm. Retention time: 2.743 min. **MS m/z (ESI):** 606.1 $[M+H]^+$. **$^1$H NMR** (400 MHz, DMSO-$d_6$) δ 7.46 (dd, *J* = 2.0, 8.4 Hz, 1H), 7.28 (d, *J* = 2.0 Hz, 1H), 7.19 (s, 1H), 7.01 (t, *J* = 8.0 Hz, 1H), 6.88 (d, *J* = 8.4 Hz, 1H), 6.74 (d, *J* = 8.0 Hz, 1H), 6.45 (t, *J* = 6.0 Hz, 1H), 6.24 (d, *J* = 7.6 Hz, 1H), 5.52 (d, *J* = 8.4 Hz, 1H), 4.94 (q, *J* = 9.2 Hz, 2H), 4.81 (d, *J* = 49.6 Hz, 1H), 4.35 (d, *J* = 6.0 Hz, 2H), 3.87 (s, 3H), 3.64 - 3.48 (m, 1H), 3.46 - 3.36 (m, 1H), 3.32 - 3.24 (m, 1H), 3.10 - 3.00 (m, 2H), 2.97 - 2.88 (m, 1H), 2.85 - 2.74 (m, 1H), 2.30 - 2.13 (m, 2H), 2.18 (s, 3H), 2.13 - 2.03 (m, 2H), 1.97 - 1.84 (m, 1H), 1.74 - 1.53 (m, 3H).

Compound **37f:** SFC method: Instrument: Waters UPC2 analytical SFC (SFC-H); Column: ChiralPak AD, 50×4.6mm I.D., 3μm; Mobile phase: A for $CO_2$ and B for Ethanol (0.05%DEA); Gradient: B 40%; Flow rate: 3 mL/min; Back pressure: 100 bar; Column temperature: 35 °C; Wavelength: 220nm. Retention time: 3.291 min. **MS m/z (ESI):** 606.1 $[M+H]^+$. **$^1$H NMR** (400 MHz, DMSO-$d_6$) δ 7.46 (dd, *J* = 2.0, 8.4 Hz, 1H), 7.28 (d, *J* = 2.0 Hz, 1H), 7.19 (s, 1H), 7.01 (t, *J* = 8.0 Hz, 1H), 6.88 (d, *J* = 8.4 Hz, 1H), 6.74 (d, *J* = 8.0 Hz, 1H), 6.45 (t, *J* = 6.4 Hz, 1H), 6.23 (d, *J* = 7.6 Hz, 1H), 5.53 (d, *J* = 8.4 Hz, 1H), 4.94 (q, *J* = 9.2 Hz, 2H), 4.81 (d, *J* = 49.6 Hz, 1H), 4.35 (d, *J* = 6.0 Hz, 2H), 3.87 (s, 3H), 3.66 - 3.47 (m, 1H), 3.45 - 3.36 (m, 1H), 3.30 - 3.23 (m, 1H), 3.10 - 2.99 (m, 2H), 2.98 - 2.88 (m, 1H), 2.86 - 2.74 (m, 1H), 2.30 - 2.15 (m, 2H), 2.19 (s, 3H), 2.15 - 2.01 (m, 2H), 2.00 - 1.86 (m, 1H), 1.74 - 1.48 (m, 3H).

Compound **37g:** SFC method: Instrument: Waters UPC2 analytical SFC (SFC-H); Column: ChiralPak AD, 50×4.6mm I.D., 3μm; Mobile phase: A for $CO_2$ and B for Ethanol (0.05%DEA); Gradient: B 40%; Flow rate: 3 mL/min; Back pressure: 100 bar; Column temperature: 35 °C; Wavelength: 220nm. Retention time: 6.081 min. **MS m/z (ESI):** 606.1 $[M+H]^+$. **$^1$H NMR** (400 MHz, DMSO-$d_6$) δ 7.46 (dd, *J* = 2.0, 8.4 Hz, 1H), 7.28 (d, *J* = 2.0 Hz, 1H), 7.19 (s, 1H), 7.00 (t, *J* = 8.0 Hz, 1H), 6.88 (d, *J* = 8.4 Hz, 1H), 6.74 (d, *J* = 8.0 Hz, 1H), 6.45 (t, *J* = 6.4 Hz, 1H), 6.23 (d, *J* = 7.6 Hz, 1H), 5.52 (d, *J* = 8.4 Hz, 1H), 4.94 (q, *J* = 9.2 Hz, 2H), 4.80 (d, *J* = 49.6 Hz, 1H), 4.35 (d, *J* = 6.0 Hz, 2H), 3.87 (s, 3H), 3.65 - 3.46 (m, 1H), 3.44 - 3.36

(m, 1H), 3.31 -3.22 (m, 1H), 3.11 - 2.98 (m, 2H), 2.98 - 2.86 (m, 1H), 2.86 - 2.72 (m, 1H), 2.30 - 2.14 (m, 2H), 2.18 (s, 3H), 2.14 - 2.01 (m, 2H), 2.00 - 1.85 (m, 1H), 1.77 - 1.50 (m, 3H).

Compound **37h:** SFC method: Instrument: Waters UPC2 analytical SFC (SFC-H); Column: ChiralPak AD, 50×4.6mm I.D., 3μm; Mobile phase: A for $CO_2$ and B for Ethanol (0.05%DEA); Gradient: B 40%; Flow rate: 3 mL/min; Back pressure: 100 bar; Column temperature: 35 °C; Wavelength: 220nm. Retention time: 6.657 min. **MS m/z (ESI):** 606.1 $[M+H]^+$. **$^1$H NMR** (400 MHz, DMSO-$d_6$) δ 7.46 (dd, *J* = 2.0, 8.4 Hz, 1H), 7.28 (d, *J* = 2.0 Hz, 1H), 7.19 (s, 1H), 7.00 (t, *J* = 8.0 Hz, 1H), 6.88 (d, *J* = 8.4 Hz, 1H), 6.74 (d, *J* = 8.0 Hz, 1H), 6.45 (t, *J* = 6.4 Hz, 1H), 6.23 (d, *J* = 7.6 Hz, 1H), 5.52 (d, *J* = 8.4 Hz, 1H), 4.94 (q, *J* = 9.2 Hz, 2H), 4.80 (d, *J* = 49.6 Hz, 1H), 4.35 (d, *J* = 6.0 Hz, 2H), 3.87 (s, 3H), 3.66 - 3.47 (m, 1H), 3.46 - 3.38 (m, 1H), 3.31 - 3.22 (m, 1H), 3.10 - 2.98 (m, 2H), 2.98 - 2.87 (m, 1H), 2.86 - 2.75 (m, 1H), 2.31 - 2.14 (m, 2H), 2.18 (s, 3H), 2.12 - 2.01 (m, 2H), 1.99 - 1.84 (m, 1H), 1.73 - 1.53 (m, 3H).

Preparation Example 38: Preparation of Compound **38**

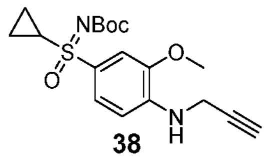

**38**

Synthesis scheme:

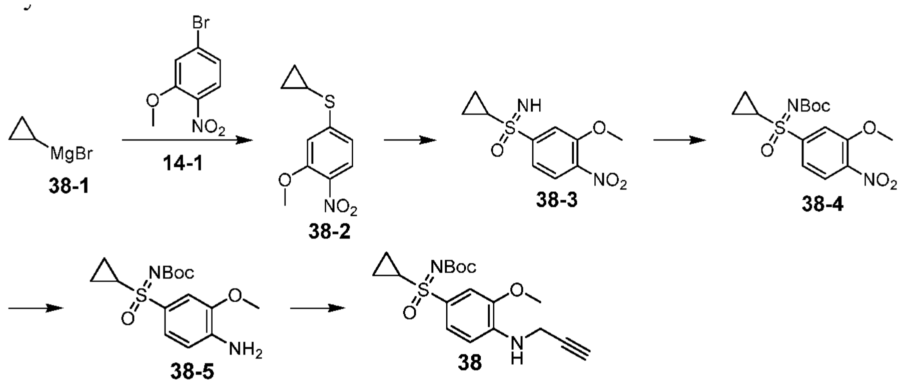

Step 1: Synthesis of Compound **38-2**

Under nitrogen protection, sulfur powder (4.41 g, 17.21 mmol) was added to a tetrahydrofuran solution of Compound **38-1** (68.8 mL, 68.83 mmol, 1 M) at 0 °C, and the reaction solution was stirred in a 50°C oil bath for 2 hours. After the reaction solution was cooled in an ice bath, a tetrahydrofuran solution of lithium aluminum tetrahydride (68.8 mL, 68.83 mmol, 1 M) was added. The reaction solution was stirred at room temperature for 2 hours. The reaction mixture was poured into dilute hydrochloric acid (60 mL, 0.5 M), the aqueous layer was extracted with ethyl acetate (50 mL x2), the organic layers were combined and washed with saturated brine (30 mL), dried over anhydrous sodium sulfate and filtered, and the filtrate was concentrated under reduced pressure and dissolved in dioxane (15 mL). Compound **14-1** (1.5 g, 6.46 mmol), ethyldiisopropylamine (3.21 mL, 19.39 mmol), tris(dibenzylideneacetone)dipalladium (0.59 g, 0.65 mmol) and 4,5-bis(diphenylphosphino)-9,9-dimethylxanthene (0.37 g, 0.65 mmol) were added to the above solution. The mixture was stirred at 100 °C for 18 hours under nitrogen protection. After the reaction mixture was cooled to room temperature, it was poured into water (20 mL), the aqueous layer was extracted with ethyl acetate (20 mL x2), the organic layers were combined and washed with saturated brine (20 mL), dried over anhydrous sodium sulfate and filtered, and the filtrate was concentrated under reduced pressure. The crude product was purified by silica gel column chromatography to obtain Compound **38-2. MS m/z (ESI):** 226.2 $[M+H]^+$.

Step 2: Synthesis of Compound **38**

For the synthesis of Compound **38** from Compound **38-2,** synthesis method of Compound **15-4** could be referred to.

Compound **38: MS m/z (ESI):** 365.2 $[M+H]^+$. **$^1$H NMR** (400 MHz, DMSO-$d_6$) δ 7.32 (dd, *J* = 2.0, 8.4 Hz, 1H), 7.14 (d, *J* = 2.0 Hz, 1H), 6.78 (d, *J* = 8.4 Hz, 1H), 6.37 (t, *J* = 6.4 Hz, 1H), 4.03 - 3.98 (m, 2H), 3.85 (s, 3H), 3.09 (s, 1H), 2.96 - 2.91 (m, 1H), 1.25 (s, 9H), 1.13 - 1.05 (m, 2H), 1.01 - 0.85 (m, 2H).

Preparation Example 39: Preparation of Compounds **39a** and **39b**

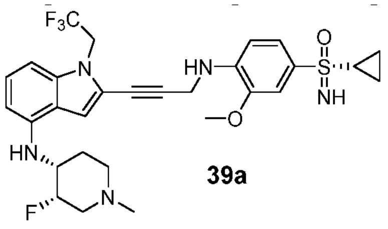

**39a**

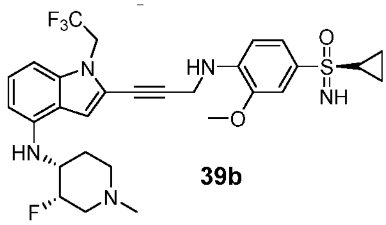

**39b**

Synthesis scheme:

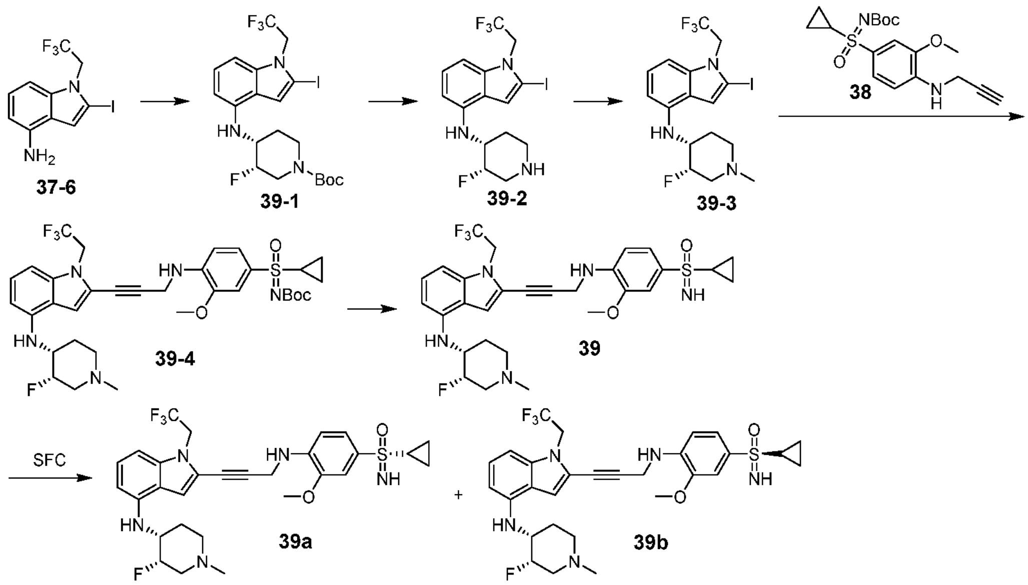


**37-6**, **39-1**, **39-2**, **39-3**, **38**, **39-4**, **39**, **39a**, **39b**

Step 1: Synthesis of Compound **39-4**

For the synthesis of Compound **39-4,** Preparation Example 37 could be referred to. Compound **39-4: MS m/z (ESI):** 692.2 $[M+H]^+$.

Step 2: Synthesis of Compound 39

Trifluoroacetic acid (1 mL) was added to a solution of Compound **39-4** (500 mg, 0.72 mmol) in dichloromethane (3 mL). The reaction mixture was stirred at room temperature for 30 minutes. The reaction mixture was poured into saturated sodium bicarbonate (50 mL). The aqueous layer was extracted with ethyl acetate (50 mL x2), and the organic layers were combined and washed with saturated brine (50 mL), dried over anhydrous sodium sulfate, filtered, and the filtrate was concentrated under reduced pressure. The crude product was purified by silica gel column chromatography to obtain Compound **39. MS m/z (ESI):** 592.2 $[M+H]^+$.

Compound **39** was separated by SFC to obtain Compounds **39c** and **39d** that were isomers between each other. Compound **39c** or **39d** was selected from Compounds **39a** and **39b,** respectively, and they were different from each other.

Compound **39c:** SFC method: Instrument: Waters UPC2 analytical SFC (SFC-H); Column: ChiralPak AS, 150×4.6mm I.D., 3μm; Mobile phase: A for $CO_2$ and B for Ethanol (0.05%DEA); Gradient: B 40%; Flow rate: 2.5 mL/min; Back pressure: 100 bar; Column temperature: 35 °C; Wavelength: 220nm. Retention time: 2.476 min. **MS m/z (ESI):** 592.2 $[M+H]^+$. **$^1$H NMR** (400 MHz, $CD_3OD$) δ 7.49 (dd, *J* = 2.0, 8.4 Hz, 1H), 7.32 (d, *J* = 2.0 Hz, 1H), 7.09 (t, *J* = 8.0 Hz, 1H), 6.94 - 6.90 (m, 2H), 6.75 (d, *J* = 8.4 Hz, 1H), 6.35 (d, *J* = 7.6 Hz, 1H), 5.03 - 4.80 (m, 1H), 4.77 (q, *J* = 8.8 Hz, 2H), 4.38 (s, 2H), 3.93 (s, 3H), 3.80 - 3.76 (m, 1H), 3.47 (t, *J* = 11.6 Hz, 1H), 3.25 - 3.16 (m, 1H), 2.84 - 2.77 (m, 1H), 2.73 - 2.59 (m, 2H), 2.57 (s, 3H), 2.14 - 1.98 (m, 2H), 1.27 - 1.18 (m, 1H), 1.09 - 1.02 (m, 2H), 0.94 - 0.89 (m, 1H).

Compound **39d:** SFC method: Instrument: Waters UPC2 analytical SFC (SFC-H); Column: ChiralPak AS, 150×4.6mm I.D., 3μm; Mobile phase: A for $CO_2$ and B for Ethanol (0.05%DEA); Gradient: B 40%; Flow rate: 2.5 mL/min; Back pressure: 100 bar; Column temperature: 35 °C; Wavelength: 220nm. Retention time: 4.04 min. **MS m/z (ESI):** 592.2 $[M+H]^+$. **$^1$H NMR** (400 MHz, $CD_3OD$) δ 7.49 (dd, *J* = 2.0, 8.4 Hz, 1H), 7.32 (d, *J* = 2.0 Hz, 1H), 7.08 (t, *J* = 8.0 Hz, 1H), 6.94 - 6.90 (m, 2H), 6.74 (d, *J* = 8.4 Hz, 1H), 6.34 (d, *J* = 7.6 Hz, 1H), 4.96 - 4.78 (m, 1H), 4.77 (q, *J* = 8.8 Hz, 2H), 4.38 (s, 2H), 3.94 (s, 3H), 3.77 - 3.64 (m, 1H), 3.35 - 3.29 (m, 1H), 3.06 - 3.03 (m, 1H), 2.72 - 2.31 (m, 6H), 2.14 - 1.98 (m, 2H), 1.27 - 1.18 (m, 1H), 1.09 - 1.02 (m, 2H), 0.94 - 0.89 (m, 1H).

Preparation Example 40: Preparation of Compound **40**

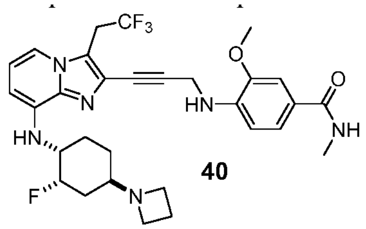

**40**

Synthesis scheme:

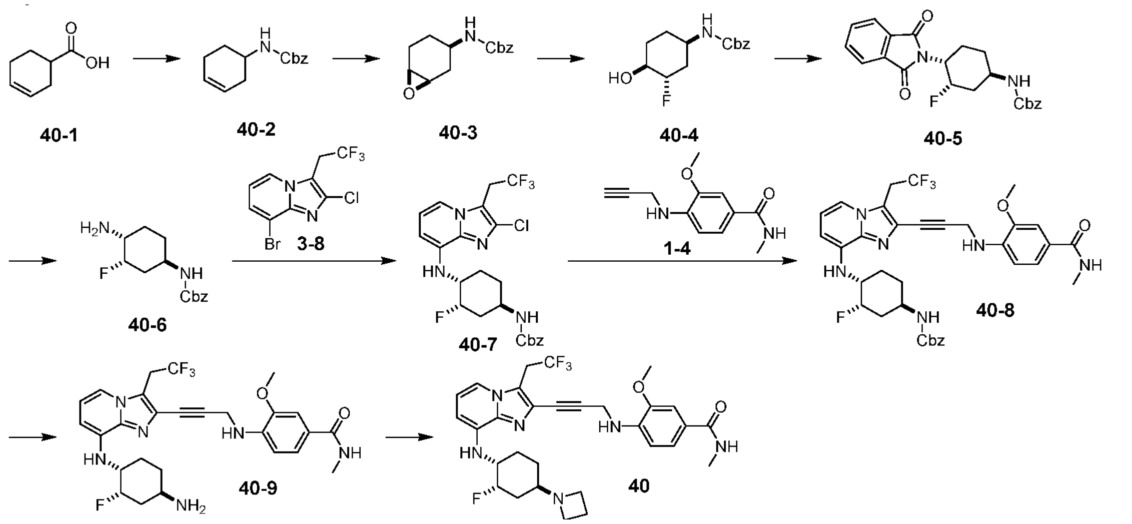

**40-1** **40-2** **40-3** **40-4** **40-5**

**3-8** **40-6** **40-7** **1-4** **40-8**

**40-9** **40**

Step 1: Synthesis of Compound **40-2**

Compound **40-1** (34.72 mL, 297.24 mmol) was added to toluene (500 mL), and triethylamine (45 mL, 324.64 mmol) and diphenylphosphoryl azide (68 mL, 313.81 mmol) were added. The reaction solution was stirred at 25 °C for 1.5 hours under nitrogen protection. Then it was heated to 110 °C and stirred for another 2.5 hours. Benzyl alcohol (34.00 mL, 326.97 mmol) was added to the reaction solution, and the reaction solution was stirred at 110 °C for 12 hours. The reaction solution was concentrated and then diluted with N,N-dimethylformamide (100 mL) and water (1000 mL). The mixture was

filtered and the filter cake was washed with water (200 mL x 2). The filter cake was dried under vacuum to obtain crude Compound **40-2.** **$^1$H NMR** (400 MHz, $CDCl_3$) δ 7.34 - 7.22 (m, 5H), 5.64 - 5.56 (m, 1H), 5.55 - 5.48 (m, 1H), 5.02 (s, 2H), 3.81 (d, *J* = 9.2 Hz, 1H), 2.36 - 2.30 (m, 1H), 2.12 - 1.99 (m, 2H), 1.85 - 1.78 (m, 1H), 1.59 - 1.43 (m, 1H).

Step 2: Synthesis of Compound **40-3**

To a solution of Compound **40-2** (83.0 g, 358.86 mmol) in dichloromethane (1000 mL), m-chloroperbenzoic acid (169.64 mL, 467.95 mmol) was added. The mixture was stirred at 25 °C for 3 hours. The mixture was quenched with saturated sodium thiosulfate (500 mL) and washed with saturated sodium bicarbonate (300 mL x2). The organic phase was concentrated to obtain crude Compound **40-3.**

Step 3: Synthesis of Compound **40-4**

A solution of triethylamine hydrofluoride (622 mL, 3821.42 mmol) containing Compound **40-3** (189 g, 764.28 mmol) was stirred at 100 °C for 2 hours. The mixture was quenched with an aqueous solution (1000 mL) of potassium carbonate (528.12 g) and extracted with ethyl acetate (500 mL x3). The combined organic phase was concentrated. The crude product was purified by silica gel column chromatography (petroleum ether/ethyl acetate = 1/1) to obtain Compound **40-4.** **$^1$H NMR** (400 MHz, $CDCl_3$) δ 7.38 - 7.23 (m, 5H), 5.02 (s, 2H), 4.67 (br s, 1H), 4.61 - 4.37 (m, 1H), 3.93 - 3.86 (m, 1H), 3.81 - 3.69 (m, 1H), 1.94 - 1.76 (m, 3H), 1.67 - 1.58 (m, 2H), 1.58 - 1.47 (m, 1H).

Step 4: Synthesis of Compound **40-5**

A solution of Compound **40-4** (80.0 g, 299.29 mmol), phthalimide (46.96 g, 319.45 mmol), triphenylphosphine (100 g, 381.26 mmol) and diisopropyl azodicarboxylate (77 mL, 388.41 mmol) in tetrahydrofuran (500 mL) was stirred at 45 °C for 12 hours. The resulting mixture was concentrated, diluted with acetonitrile (300 mL) and filtered. The filter cake was washed with acetonitrile (100 mL x3) and dried in vacuum to obtain Compound **40-5.** **MS m/z (ESI):** 397.1 $[M+H]^+$. **$^1$H NMR** (400 MHz, DMSO-$d_6$) δ 7.91 - 7.82 (m, 4H), 7.43 - 7.28 (m, 5H), 5.13 - 4.98 (m, 2H), 4.95 - 4.75 (m, 1H), 4.26 - 4.00 (m, 1H), 3.74 - 3.62 (m, 1H), 2.99 - 2.85 (m, 1H), 2.23 - 2.13 (m, 1H), 2.04 - 1.94 (m, 1H), 1.84 - 1.76 (m, 1H), 1.74 - 1.32 (m, 2H).

Step 5: Synthesis of Compound **40-6**

Compound **40-5** (42.0 g, 105.95 mmol) was added to hydrazine hydrate (100 mL, 1646.02 mmol, 85%) and ethanol (420 mL), and the reaction solution was stirred at 80 °C for 2 hours. The reaction solution was concentrated and then diluted with dichloromethane (500 mL). The mixture was filtered and the filter cake was washed with dichloromethane (100 mL). The organic phase was concentrated to obtain crude Compound **40-6.** **MS m/z (ESI):** 267.2 $[M+H]^+$.

Step 6: Synthesis of Compound **40-7**

Compound **3-8** (5.0 g, 15.95 mmol), Compound **40-6** (5.0 g, 18.77 mmol), tri(dibenzylideneacetone)dipalladium (1.5 g, 1.64 mmol), 1,1'-binaphthyl-2,2'-bisdiphenylphosphine (2 g, 3.21 mmol) and cesium carbonate (11 g, 33.76 mmol) were added to 1,4-dioxane (100 mL), and the reaction solution was stirred at 110 °C under nitrogen protection for 1 hour. The mixture was filtered, diluted with water (50 mL), and extracted with dichloromethane (50 mL x 3). The combined organic phase was concentrated. It was purified by silica gel column chromatography (dichloromethane/methanol = 20/1) to obtain Compound **40-7.** **$^1$H NMR** (400 MHz, DMSO-$d_6$) δ 7.88 (d, *J* = 6.8 Hz, 1H), 6.90 (t, *J* = 7.2 Hz, 5H), 6.51 (d, *J* = 7.6 Hz, 1H), 5.41 (d, *J* = 9.2 Hz, 1H), 5.02 (s, 2H), 4.23 - 4.10 (m, 2H), 4.06 - 3.98 (m, 1H), δ 3.84 - 3.52 (m, 2H), 1.93 - 1.81 (m, 2H), 1.75 - 1.53 (m, 2H), 1.5 1.29 (m, 2H).

Step 7: Synthesis of Compound **40-8**

Compound **40-7** (1.0 g, 2.00 mmol), Compound **1-4** (1.31 g, 6.00 mmol), 2-dicyclohexylphosphino-2',4',6'-triisopropylbiphenyl (0.38 g, 0.80 mmol), cesium carbonate (1.31 g, 4.01 mmol) and bis(acetonitrile)palladium chloride (0.10 g, 0.40 mmol) were added to acetonitrile (10 mL), and the reaction solution was stirred at 90 °C for 2 hours. The reaction solution was filtered and the organic layer was concentrated. The crude product was purified by silica gel column chromatography (petroleum ether/ethyl acetate = 1/30) to obtain Compound **40-8.** **$^1$H NMR** (400 MHz, DMSO-$d_6$) δ 8.10 (d, *J* = 4.6 Hz, 1H), 7.77 (d, *J* = 6.8 Hz, 1H), 7.43 - 7.28 (m, 6H), 6.82 (dd, *J* = 6.0, 8.4 Hz, 1H), 6.73 (dd, *J* =8.4, 11.2 Hz, 1H), 6.41 (dd, *J* = 4.4, 7.6 Hz, 1H), 5.93 (q, *J* = 6.0 Hz, 1H), 5.40 (d, *J* = 9.3 Hz, 1H), 5.09 - 4.83 (m, 3H), 4.27 (d, *J* = 6.3 Hz, 2H), 4.06 - 3.96 (m, 2H), 3.85 (s, 3H), 3.68 - 3.59 (m, 1H), 2.76 (d, *J* = 4.5 Hz, 3H), 2.22 - 2.13 (m, 1H), 1.91 - 1.81 (m, 2H), 1.76 - 1.56 (m, 2H), 1.52 - 1.22 (m, 2H).

Step 8: Synthesis of Compound **40-9**

A solution of Compound **40-8** (620 mg, 0.91 mmol) in trifluoroacetic acid (10 mL) was stirred at 50 °C for 2 hours. The mixture was quenched with saturated sodium bicarbonate solution (50 mL), the mixture was filtered, and the filter cake was dried to obtain Compound **40-9. MS m/z (ESI):** 547.4 $[M+H]^+$.

Step 8: Synthesis of Compound **40**

A solution of Compound **40-9** (50 mg, 0.09 mmol), 1,3-dibromopropane (0.04 mL, 0.36 mmol), and sodium carbonate (38.2 mg, 0.36 mmol) in acetonitrile (1 mL) was stirred at 80 °C for 18 hours under nitrogen protection. The reaction solution was filtered and concentrated under reduced pressure. The crude product was purified by reverse phase flash chromatography (Waters-Xbridge-C18-10μm-19*250mm; mobile phase: A: 10mM ammonium bicarbonate/water B: acetonitrile; flow rate: 25; retention time: 6.32 min) to obtain Compound **40. MS m/z (ESI):** 587.3 $[M+H]^+$. **$^1$H NMR** (400 MHz, DMSO-$d_6$) δ 8.07 (d, *J* = 4.8 Hz, 1H), 7.76 (d, *J* = 6.8 Hz, 1H), 7.41 (dd, *J* =2.0, 8.4 Hz, 1H), 7.34 (d, *J* = 2.0 Hz, 1H), 6.81 (t, *J* = 7.2 Hz, 1H), 6.74 (d, *J* = 8.4 Hz, 1H), 6.36 (d, *J* = 7.8 Hz, 1H), 5.92 (t, *J* = 6.0 Hz, 1H), 5.34 (d, *J* = 9.2 Hz, 1H), 4.93 (d, *J* = 50.0 Hz, 1H), 4.26 (d, *J* = 6.4 Hz, 2H), 4.01 (q, *J* = 10.8 Hz, 2H), 3.84 (s, 3H), 3.76 - 3.59 (m, 1H), 3.07 (t, *J* = 8.4 Hz, 4H), 2.75 (d, *J* = 4.4 Hz, 3H), 2.29 - 2.21 (m, 1H), 2.09 - 1.98 (m, 1H), 1.94 - 1.86 (m, 2H), 1.85 - 1.77 (m, 1H), 1.77 - 1.70 (m, 1H), 1.67 - 1.48 (m, 1H), 1.46 - 1.32 (m, 1H), 1.16 - 0.99 (m, 1H).

Preparation Example 41: Preparation of Compound **41**

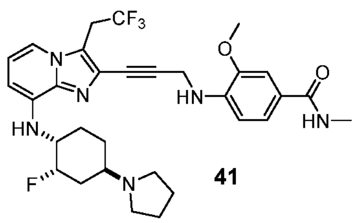

**41**

For the synthesis of Compound **41,** Preparation Example 40 could be referred to.

Compound **41: MS m/z (ESI):** 601.3 $[M+H]^+$. **$^1$H NMR** (400 MHz, DMSO-$d_6$) δ 8.08 (d, *J* = 5.2 Hz, 1H), 7.77 (d, *J* = 6.8 Hz, 1H), 7.41 (d, *J* = 8.4 Hz, 1H), 7.34 (s, 1H), 6.82 (t, *J* = 7.2 Hz, 1H), 6.74 (d, *J* = 8.4 Hz, 1H), 6.37 (d, *J* = 7.6 Hz, 1H), 5.93 (t, *J* = 6.4 Hz, 1H), 5.36 (d, *J* = 9.6 Hz, 1H), 4.96 (d, *J* = 50.4 Hz, 1H), 4.26 (d, *J* = 6.4 Hz, 2H), 4.07 - 3.95 (m, 2H), 3.84 (s, 3H), 3.76 - 3.59 (m, 1H), 2.75 (d, *J* = 4.4 Hz, 3H), 2.57 - 2.50 (m, 4H), 2.39 - 2.22 (m, 4H), 2.01 - 1.92 (m, 1H), 1.90 - 1.81 (m, 1H), 1.76 - 1.62 (m, 6H), 1.60 - 1.51 (m, 2H), 1.48 - 1.29 (m, 2H).

Preparation Example 42: Preparation of Compound **42**

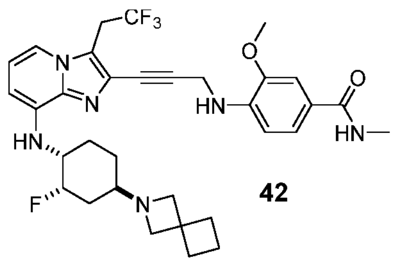

**42**

For the synthesis of Compound **42,** Preparation Example 40 could be referred to.

Compound **42: MS m/z (ESI):** 627.3 $[M+H]^+$. **$^1$H NMR** (400 MHz, DMSO-$d_6$) δ 8.08 (d, *J* = 4.8 Hz, 1H), 7.76 (d, *J* = 6.8 Hz, 1H), 7.41 (dd, *J* = 2.0, 8.0 Hz, 1H), 7.34 (d, *J* = 2.0 Hz, 1H), 6.81 (t, *J* = 7.2 Hz, 1H), 6.74 (d, *J* = 8.4 Hz, 1H), 6.34 (d, *J* = 7.6 Hz, 1H), 5.93 (t, *J* = 6.4 Hz, 1H), 5.34 (d, *J* = 9.2 Hz, 1H), 4.92 (d, *J* = 50.0 Hz, 1H), 4.26 (d, *J* = 6.4 Hz, 2H), 4.09 - 3.94 (m, 2H), 3.84 (s, 3H), 3.76 - 3.59 (m, 1H), 3.03 (d, *J* = 3.2 Hz, 4H), 2.75 (d, *J* = 4.4 Hz, 3H), 2.23 - 2.10 (m, 1H), 2.07 - 1.94 (m, 5H), 1.86 - 1.66 (m, 4H), 1.60 - 1.49 (m, 1H), 1.30 - 1.18 (m, 1H), 1.16 - 0.99 (m, 1H).

Preparation Example 43: Preparation of Compound **43**

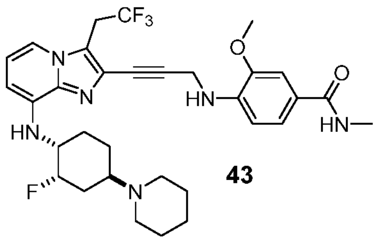

**43**

For the synthesis of Compound **43,** Preparation Example 40 could be referred to.

Compound **43: MS m/z (ESI):** 615.3 [M+H]+. **1H NMR** (400 MHz, DMSO-$d_6$) δ 8.11 (d, *J* = 4.8 Hz, 1H), 7.78 (d, *J* = 6.8 Hz, 1H), 7.40 (dd, *J* = 2.0, 8.0 Hz, 1H), 7.34 (d, *J* = 2.0 Hz, 1H), 6.83 (t, *J* = 7.2 Hz, 1H), 6.73 (d, *J* = 8.4 Hz, 1H), 6.35 (d, *J* = 7.6 Hz, 1H), 5.97 (t, *J* = 6.0 Hz, 1H), 5.43 - 5.33 (m, 1H), 5.01 (d, *J* = 47.8 Hz, 1H), 4.26 (d, *J* = 6.4 Hz, 2H), 4.07 - 3.93 (m, 2H), 3.83 (s, 3H), 3.75 - 3.63 (m, 1H), 2.74 (d, *J* = 4.4 Hz, 3H), 2.14 - 2.04 (m, 1H), 1.96 - 1.61 (m, 4H), 1.64 - 1.46 (m, 3H), 1.40 - 1.32 (m, 3H), 1.25 - 1.20 (m, 6H).

Preparation Example 44: Preparation of Compounds **44, 44a** and **44b**

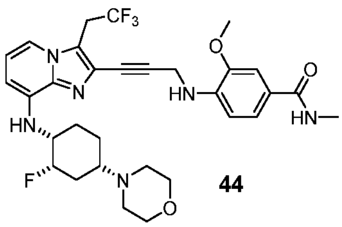

**44**

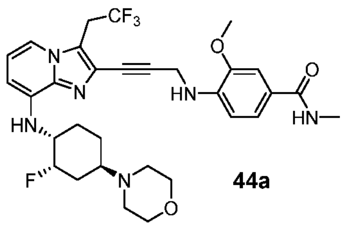

**44a**

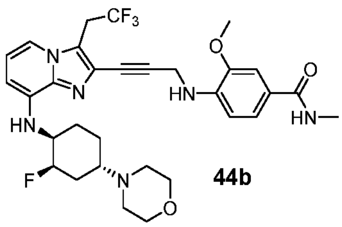

**44b**

For the synthesis of Compound **44,** Preparation Example 40 could be referred to.

Compound **44: MS m/z (ESI):** 617.3 [M+H]+. **1H NMR** (400 MHz, DMSO-$d_6$) δ 8.08 (d, *J* = 4.8 Hz, 1H), 7.76 (d, *J* = 6.8 Hz, 1H), 7.40 (dd, *J* = 2.0, 8.0 Hz, 1H), 7.34 (d, *J* = 2.0 Hz, 1H), 6.82 (t, *J* = 7.2 Hz, 1H), 6.73 (d, *J* = 8.4 Hz, 1H), 6.35 (d, *J* = 7.6 Hz, 1H), 5.93 (t, *J* = 6.0 Hz, 1H), 5.37 (d, *J* = 9.2 Hz, 1H), 5.00 (d, *J* = 50.8 Hz, 1H), 4.26 (d, *J* = 6.4 Hz, 2H), 4.01 (d, *J* = 10.8 Hz, 2H), 3.83 (s, 3H), 3.73 - 3.63 (m, 1H), 3.56 (t, *J* = 4.8 Hz, 4H), 2.74 (d, *J* = 4.4 Hz, 3H), 2.49 - 2.44 (m, 4H), 2.19 - 2.08 (m, 1H), 1.93 - 1.82 (m, 2H), 1.80 - 1.70 (m, 1H), 1.68 - 1.54 (m, 2H), 1.50 - 1.33 (m, 1H).

Compound **44** was subjected to SFC resolution to give Compounds **44c** and **44d** that were isomers between each other. Compounds **44c** and **44d** were selected from Compounds **44a** and **44b,** respectively, and they were different from each other.

Compound **44c:** SFC method: Instrument: Shimadzu LC-A HPLC, Column: Chiralpak IC, 250×30mm, 10 μm, Mobile phase: A for Heptane and B for Ethanol (0.2%$NH_3H_2O$), Isocratic: B: 20%, Flow rate: 100 mL /min, Column temperature: R.T., Wavelength: 220 nm, 254 nm, Sample preparation: The sample was dissolved in ~1000 mL Heptane / Ethanol, Injection: 10 ml, Cycle time: 9 min. Retention time: 1.027 min. **MS m/z (ESI):** 617.0 [M+H]+. **1H NMR** (400 MHz, DMSO-$d_6$) δ 8.11 (d, *J* = 4.8 Hz, 1H), 7.78 (d, *J* = 6.8 Hz, 1H), 7.41 (dd, *J* = 2.0, 8.4 Hz, 1H), 7.35 (d, *J* = 2.0 Hz, 1H), 6.83 (t, *J* = 7.2 Hz, 1H), 6.74 (d, *J* = 8.4 Hz, 1H), 6.36 (d, *J* = 7.6 Hz, 1H), 5.96 (t, *J* = 6.4 Hz, 1H), 5.39 (d, *J* = 9.2 Hz, 1H), 5.01 (d, *J* = 50.8 Hz, 1H), 4.27 (d, *J* = 6.4 Hz, 2H), 4.02 (q, *J* = 10.8 Hz, 2H), 3.84 (s, 3H), 3.70 (d, *J* = 29.2 Hz, 1H), 3.57 (t, *J* = 4.4 Hz, 4H), 2.75 (d, *J* = 4.4 Hz, 3H), 2.51 - 2.44 (m, 4H), 2.19 - 2.08 (m, 1H), 1.94 - 1.82 (m, 2H), 1.80 - 1.71 (m, 1H), 1.69 - 1.57 (m, 2H), 1.50 - 1.34 (m, 1H).

Compound **44d:** SFC method: Instrument: Shimadzu LC-A HPLC, Column: Chiralpak IC, 250×30mm, 10 μm, Mobile phase: A for Heptane and B for Ethanol (0.2%$NH_3H_2O$), Isocratic: B: 20%, Flow rate: 100 mL /min, Column temperature: R.T., Wavelength: 220 nm, 254 nm, Sample preparation: The sample was dissolved in ~1000 mL Heptane / Ethanol, Injection: 10 ml, Cycle time: 9 min. Retention time: 1.324 min. **MS m/z (ESI):** 617.0 [M+H]+. **1H NMR** (400 MHz, DMSO-$d_6$) δ 8.11 (d, *J* = 4.8 Hz, 1H), 7.78 (d, *J* = 6.8 Hz, 1H), 7.41 (dd, *J* = 2.0, 8.4 Hz, 1H), 7.35 (d, *J* = 2.0 Hz, 1H), 6.83 (t, *J* = 7.2 Hz, 1H), 6.74 (d, *J* = 8.4 Hz, 1H), 6.36 (d, *J* = 7.6 Hz, 1H), 5.96 (t, *J* = 6.4 Hz, 1H), 5.39 (d, *J* = 9.2 Hz, 1H), 5.01 (d, *J* = 50.8 Hz, 1H), 4.27 (d, *J* = 6.4 Hz, 2H), 4.02 (q, *J* = 10.8 Hz, 2H), 3.84 (s, 3H), 3.70 (d, *J* = 29.2 Hz, 1H), 3.57 (t, *J* = 4.4 Hz, 4H), 2.75 (d, *J* = 4.4 Hz, 3H), 2.51 - 2.44 (m, 4H), 2.19 - 2.08 (m, 1H), 1.93 - 1.81 (m, 2H), 1.80 - 1.71 (m, 1H), 1.69 - 1.55 (m, 2H), 1.50 -

1.35 (m, 1H).

Preparation Example 45: Preparation of Compound **45**

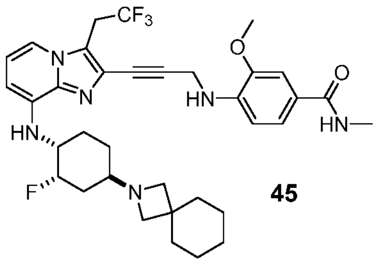

**45**

For the synthesis of Compound **45,** Preparation Example 40 could be referred to.

Compound **45: MS m/z (ESI):** 655.3 $[M+H]^+$. **$^1$H NMR** (400 MHz, $CD_3OD$) δ 7.64 (t, *J* = 6.4 Hz, 1H), 7.45 (dd, *J* =2.0, 8.4 Hz, 1H), 7.38 (d, *J* = 2.0 Hz, 1H), 6.90 - 6.84 (m, 2H), 6.42 (d, *J* = 7.6 Hz, 1H), 5.09 - 4.93 (m, 1H), 4.35 (s, 2H), 3.94 (s, 3H), 3.87 (q, *J* = 10.4 Hz, 2H), 3.62 (t, *J* = 10.4 Hz, 1H), 3.12 (br s, 4H), 2.92 (s, 3H), 2.65 - 2.55 (m, 1H), 2.35 - 1.93 (m, 6H), 1.74 - 1.22 (m, 10H).

Preparation Example 46: Preparation of Compound **46**

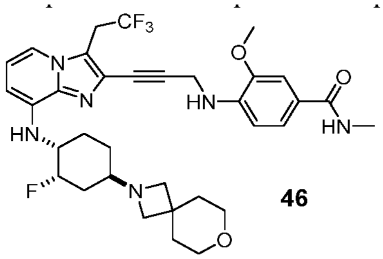

**46**

For the synthesis of Compound **46,** Preparation Example 40 could be referred to.

Compound **46: MS m/z (ESI):** 657.3 $[M+H]^+$. **$^1$H NMR** (400 MHz, DMSO-$d_6$) δ 8.12 (d, *J* = 4.8 Hz, 1H), 7.78 (d, *J* = 6.8 Hz, 1H), 7.41 (d, *J* = 8.2 Hz, 1H), 7.35 (d, *J* = 1.6 Hz, 1H), 6.83 (t, *J* = 7.2 Hz, 1H), 6.74 (d, *J* = 8.4 Hz, 1H), 6.36 (d, *J* = 7.6 Hz, 1H), 5.97 (t, *J* = 6.4 Hz, 1H), 5.38 (d, *J* = 9.2 Hz, 1H), 4.96 (d, *J* = 50.0 Hz, 1H), 4.27 (d, *J* = 6.4 Hz, 9 (m, 1H), 2.77 - 2.81 (m, 1H), 2.66 - 2.73 (m, 1H), 2.89 - 2.91 (m, 1H), 2.35 - 2.42 (m, 1H), 2.44 - 2.53 (m, 1H), 2.39 - 2.40 (m, 1H), 2.54 - 2.62 (m, 1H), 2.70 - 2.72 (m, 1H), 2.82 - 2.82 (m, 1H), 2.98 - 2.99 (m, 1H), 2.12 - 2.19 (m, 1H), 2.20 - 2.31 (m, 1H), 2.34 - 2.36 (m, 1H), 2.38 - 2.30 (m, 1H), 2. 6.9 Hz, 2H), 1.66 - 1.60 (m, 1H), 1.59 - 1.49 (m, 1H), 1.42 - 1.32 (m, 1H), 1.24 (s,1H).

Preparation Example 47: Preparation of Compound **47**

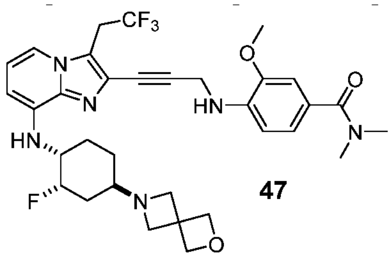

**47**

Synthesis scheme:

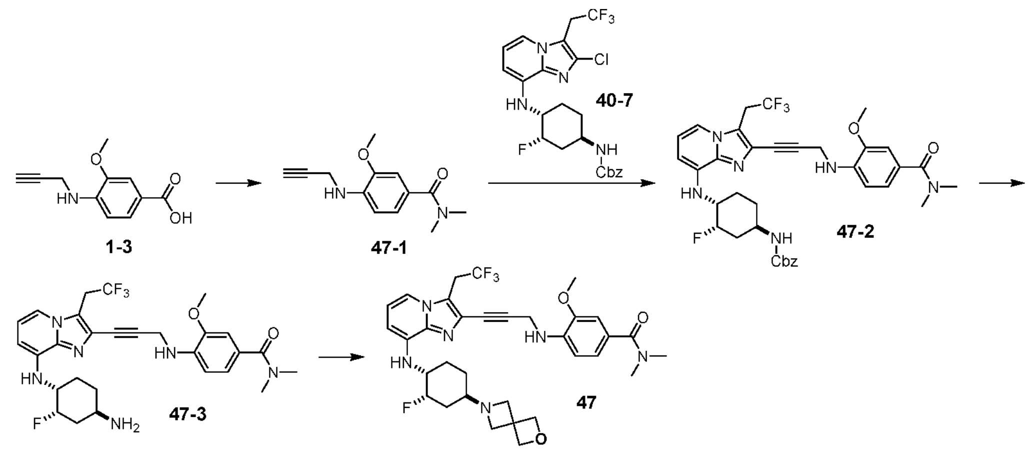


Step 1: Synthesis of Compound **47-1**

Compound **1-3** (1.65 g, 8.04 mmol) was dissolved in acetonitrile (20 mL), and dimethylamine (16 mL, 32.16 mmol), tetramethylchlorouronium hexafluorophosphate (3.38 g, 12.06 mmol) and 1-methylimidazole (4.0 mL, 24.12 mmol) were added. The reaction solution was stirred at 25 °C for 2 hours. It was added with water (5 mL) and extracted with ethyl acetate (20 mL x2). The organic layers were combined and washed with saturated brine (10 mL), dried over anhydrous sodium sulfate, filtered, and the filtrate was concentrated. The crude product was purified by silica gel column chromatography (petroleum ether: ethyl acetate = 5:1) to obtain Compound **47-1. MS m/z (ESI):** 233.0 $[M+H]^+$.

Step 2: Synthesis of Compound **47-2**

Compound **40-7** (200 mg, 0.40 mmol) was dissolved in acetonitrile (6 mL), and 2-dicyclohexylphosphino-2',4',6'-triisopropylbiphenyl (78 mg, 0.16 mmol), bis(acetonitrile)palladium chloride (21 mg, 0.08 mmol) and cesium carbonate (325 mg, 1.00 mmol) were added. The mixture was purged with nitrogen three times, stirred at 90 °C for 10 minutes under a nitrogen atmosphere, and then Compound **47-1** (375 mg, 1.61 mmol) was added. The reaction solution was stirred at 90 °C for 2 hours under nitrogen atmosphere. The mixture was filtered and the filter cake was washed with ethyl acetate (10 mL x3), and the filtrate was concentrated to dryness under reduced pressure. The crude product was purified by silica gel column chromatography (petroleum ether: ethyl acetate = 1:20) to obtain Compound **47-2. MS m/z (ESI):** 695.3 $[M+H]^+$.

Step 3: Synthesis of Compound **47-3**

Compound **47-2** (90 mg, 0.13 mmol) was dissolved in trifluoroacetic acid (2 mL), and the reaction solution was stirred at 50 °C for 1 hour. After the reaction solution was cooled to room temperature, the reaction solution was slowly added dropwise to a saturated sodium bicarbonate aqueous solution (20 mL), and extracted with ethyl acetate (30 mL x2). The organic layers were combined and washed with saturated brine (10 mL), dried over anhydrous sodium sulfate, filtered, and the filtrate was concentrated. The obtained Compound **47-3** was directly used in the next step. **MS m/z (ESI):** 561.3 $[M+H]^+$.

Step 4: Synthesis of Compound **47**

Compound **47-3** (75 mg, 0.13 mmol) was dissolved in acetonitrile solution (4 mL), 3,3-bis(bromomethyl)oxetane (98 mg, 0.40 mmol) and sodium carbonate (115 mg, 1.08 mmol) were added, and the reaction solution was stirred at 80 °C for 48 hours. After the reaction solution was cooled to room temperature, water (20 mL) was added and the mixture was extracted with ethyl acetate (20 mL x2). The organic layers were combined and washed with saturated brine (10 mL), dried over anhydrous sodium sulfate, filtered, and the filtrate was concentrated. The crude product was purified by reverse phase flash chromatography (Waters-Xbridge-C18-10μm-19*250mm; mobile phase: A: 10mM ammonium bicarbonate/-water B: acetonitrile; flow rate: 25; retention time: 6.32 min) to obtain Compound **47. MS m/z (ESI):** 643.3 $[M+H]^+$. **$^1$H NMR** (400 MHz, DMSO-$d_6$) δ 7.77 (d, *J* = 6.8 Hz, 1H), 6.97 - 6.90 (m, 2H), 6.82 (t, *J* = 7.2 Hz, 1H), 6.73 (d, *J* = 8.0 Hz, 1H), 6.35 (d, *J*

= 7.6 Hz, 1H), 5.87 (t, *J* = 6.4 Hz, 1H), 5.36 (d, *J* = 9.2 Hz, 1H), 4.92 (d, *J* = 50.0 Hz, 1H), 4.58 (s, 4H), 4.25 (d, *J* = 6.4 Hz, 2H), 4.02 (q, *J* = 10.4 Hz, 2H), 3.81 (s, 3H), 3.72 - 3.64 (m, 1H), 3.22 (d, *J* = 3.6 Hz, 4H), 2.96 (s, 6H), 2.24 - 2.13 (m, 1H), 2.02 (s, 1H), 1.76 (dd, *J* = 11.2, 39.2 Hz, 2H), 1.54 (t, *J* = 12.4 Hz, 1H), 1.30 (m, 1H), 1.14 - 1.00 (m, 1H).

Preparation Example 48: Preparation of Compounds **48, 48a** and **48b**

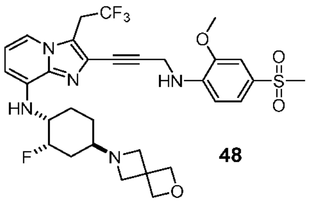
48

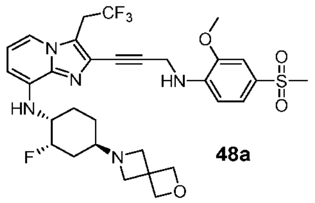
48a

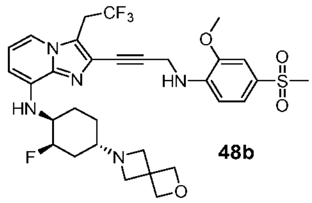
48b

For the synthesis of Compound **48,** Preparation Example 40 could be referred to.

Compound **48: MS m/z (ESI):** 650.3 $[M+H]^+$. **$^1$H NMR** (400 MHz, $CD_3OD$) δ 7.67 (d, *J* = 6.8 Hz, 1H), 7.48 (dd, *J* = 2.0, 8.4 Hz, 1H), 7.29 (d, *J= 2.0* Hz, 1H), 6.96 (d, *J* = 8.4 Hz, 1H), 6.86 (t, *J* = 7.2 Hz, 1H), 6.43 (d, *J* = 7.6 Hz, 1H), 5.07 (d, *J* = 48.9 Hz, 1H), 4.75 (s, 4H), 4.37 (s, 2H), 3.95 (s, 3H), 3.88 (q, *J* = 10.4 Hz, 2H), 3.79 - 3.73 (m, 1H), 3.31 (s, 4H), 3.06 (s, 3H), 2.49 - 2.43 (m, 1H), 2.18 - 2.02 (m, 2H), 1.79 - 1.40 (m, 4H).

Compound **48** was subjected to SFC resolution to obtain Compounds **48c** and **48d** that were isomers between each other. Compounds **48c** and **48d** were selected from Compounds **48a** and **48b,** respectively, and they were different from each other.

Compound **48c:** SFC method: Instrument: WATERS 150 preparative SFC(SFC-26); Column: ChiralCel OD, 250×30mm I.D., 10μm; Mobile phase: A for $CO_2$ and B for Ethanol(0.1%$NH_3H_2O$), Gradient: B 30%; Flow rate: 150 mL /min; Back pressure: 100 bar; Column temperature: 38°C; Wavelength: 220nm; Cycle time: ~18min. Retention time: 5.829 min. **MS m/z (ESI):** 650.2 $[M+H]^+$. **$^1$H NMR** (400 MHz, $CD_3OD$) δ 7.57 (d, *J* = 6.8 Hz, 1H), 7.41 (dd, *J* = 2.0, 8.4 Hz, 1H), 7.22 (d, *J* = 2.0 Hz, 1H), 6.89 (d, *J* = 8.4 Hz, 1H), 6.78 (t, *J* = 7.2 Hz, 1H), 6.33 (d, *J* = 7.6 Hz, 1H), 4.98 - 4.81 (m, 1H), 4.68 (s, 4H), 4.30 (s, 2H), 3.87 (s, 3H), 3.80 (q, *J* = 10.4 Hz, 2H), 3.58 - 3.54 (m, 1H), 3.24 (s, 4H), 2.98 (s, 3H), 2.22 - 2.13 (m, 1H), 2.00 - 1.53 (m, 6H).

Compound **48d:** SFC method: Instrument: WATERS 150 preparative SFC(SFC-26); Column: ChiralCel OD, 250×30mm I.D., 10μm; Mobile phase: A for $CO_2$ and B for Ethanol(0.1%$NH_3H_2O$), Gradient: B 30%; Flow rate: 150 mL /min; Back pressure: 100 bar; Column temperature: 38°C; Wavelength: 220nm; Cycle time: ~18min. Retention time: 7.027 min. **MS m/z (ESI):** 650.2 $[M+H]^+$. **$^1$H NMR** (400 MHz, $CD_3OD$) δ 7.67 (d, *J* = 6.8 Hz, 1H), 7.48 (dd, *J* = 2.0, 8.4 Hz, 1H), 7.29 (d, *J* = 2.0 Hz, 1H), 6.96 (d, *J* = 8.4 Hz, 1H), 6.86 (t, *J* = 7.2 Hz, 1H), 6.43 (d, *J* = 7.6 Hz, 1H), 5.07 (d, *J* = 48.9 Hz, 1H), 4.75 (s, 4H), 4.37 (s, 2H), 3.95 (s, 3H), 3.88 (q, *J* = 10.4 Hz, 2H), 3.79 - 3.73 (m, 1H), 3.31 (s, 4H), 3.06 (s, 3H), 2.49 - 2.43 (m, 1H), 2.18 - 2.02 (m, 2H), 1.79 - 1.40 (m, 4H).

Preparation Example 49: Preparation of Compound **49**

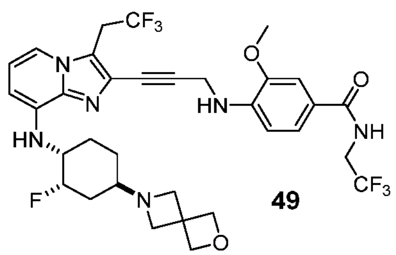
49

For the synthesis of Compound **49,** Preparation Example 47 could be referred to.

Compound **49: MS m/z (ESI):** 617.0 $[M+H]^+$. **$^1$H NMR** (400 MHz, DMSO-$d_6$) δ 8.11 (d, *J* = 4.8 Hz, 1H), 7.78 (d, *J* = 6.8 Hz, 1H), 7.41 (dd, *J* = 2.0, 8.4 Hz, 1H), 7.35 (d, *J* = 2.0 Hz, 1H), 6.83 (t, *J* = 7.2 Hz, 1H), 6.74 (d, *J* = 8.4 Hz, 1H), 6.36 (d, *J* = 7.6 Hz, 1H), 5.96 (t, *J* = 6.4 Hz, 1H), 5.39 (d, *J* = 9.2 Hz, 1H), 5.01 (d, *J* = 50.8 Hz, 1H), 4.27 (d, *J* = 6.4 Hz, 2H), 4.02 (q, *J* = 10.8 Hz, 2H), 3.84 (s, 3H), 3.74 - 3.69 (m, 1H), 3.57 (t, *J* = 4.4 Hz, 4H), 2.75 (d, *J* = 4.4 Hz, 3H), 2.49 - 2.44 (m, 4H), 2.17 - 2.11 (m, 1H), 1.93 - 1.81 (m, 2H), 1.80 - 1.71 (m, 1H), 1.69 - 1.55 (m, 2H), 1.50 - 1.35 (m, 1H).

Preparation Example 50: Preparation of Compound **50**

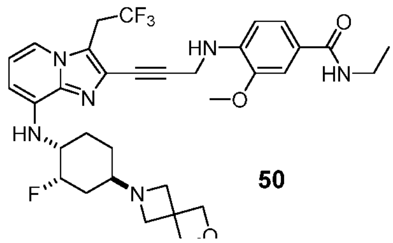

**50**

For the synthesis of Compound **50,** Preparation Example 47 could be referred to.

Compound **50: MS m/z (ESI):** 643.3 $[M+H]^+$. **$^1$H NMR** (400 MHz, DMSO-$d_6$) δ 8.11 (t, *J* = 5.6 Hz, 1H), 7.76 (d, *J* = 6.8 Hz, 1H), 7.42 (dd, *J* = 2.0, 8.4 Hz, 1H), 7.35 (d, *J* = 2.0 Hz, 1H), 6.81 (t, *J* = 7.2 Hz, 1H), 6.73 (d, *J* = 8.4 Hz, 1H), 6.35 (d, *J* = 7.6 Hz, 1H), 5.92 (t, *J* = 6.4 Hz, 1H), 5.34 (d, *J* = 9.2 Hz, 1H), 5.04 - 4.80 (m, 1H), 4.58 (s, 4H), 4.26 (d, *J* = 6.4 Hz, 2H), 4.01 (q, *J* = 10.8 Hz, 2H), 3.84 (s, 3H), 3.68 (m, 1H), 3.66 - 3.19 (m, 6H), 2.24 - 2.14 (m, 1H), 2.03 (br s, 1H), 1.85 - 1.67 (m, 2H), 1.55 (q, *J* = 12.4 Hz, 1H), 1.41 - 1.22 (m, 1H), 1.10 (m, 4H).

Preparation Example 51: Preparation of Compound **51**

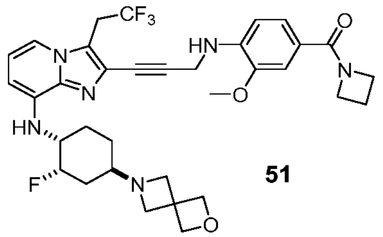

**51**

For the synthesis of Compound **51,** Preparation Example 47 could be referred to.

Compound **51: MS m/z (ESI):** 655.3 $[M+H]^+$. **$^1$H NMR** (400 MHz, $CD_3OD$) δ 7.64 (d, *J* = 6.8 Hz, 1H), 7.26 (dd, *J* = 2.0, 8.4 Hz, 1H), 7.21 (d, *J* = 2.0 Hz, 1H), 6.89 - 6.82 (m, 2H), 6.41 (d, *J* = 7.6 Hz, 1H), 5.05 - 4.93 (m, 1H), 4.76 (s, 4H), 4.54 - 4.42 (m, 2H), 4.35 (s, 2H), 4.20 (brs, 2H), 3.92 (s, 3H), 3.86 (t, *J* = 10.4 Hz, 2H), 3.72 - 3.60 (m, 1H), 3.45 (s, 4H), 2.54 - 2.34 (m, 2H), 2.23 (t, *J* = 11.8 Hz, 3H). 11.2 Hz, 1H), 2.02 - 1.90 (m, 2H), 1.68 (q, *J* = 12.4 Hz, 1H), 1.55 - 1.07 (m, 3H).

Preparation Example 52: Preparation of Compound **52**

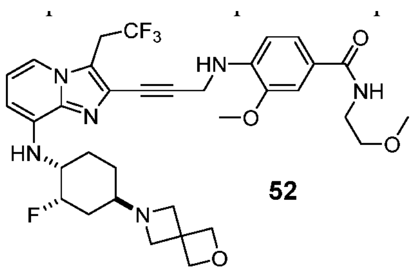

**52**

For the synthesis of Compound **52,** Preparation Example 47 could be referred to.

Compound **52: MS m/z (ESI):** 673.3 $[M+H]^+$. **$^1$H NMR** (400 MHz, $CD_3OD$) δ 7.64 (d, *J* = 6.8 Hz, 1H), 7.53 - 7.43 (m, 1H), 7.39 (dd, *J* = 2.0, 8.4 Hz, 1H), 6.91 - 6.82 (m, 2H), 6.41 (d, *J* = 7.6 Hz, 1H), 5.05 - 4.93 (m, 1H), 4.76 (s, 4H), 4.35 (s, 2H), 3.94 (s, 3H), 3.86 (q, *J* = 10.4 Hz, 2H), 3.70 - 3.64 (m, 1H), 3.59 - 3.54 (m, 4H), 3.40 (s, 3H), 2.53 - 2.40 (m, 1H), 2.25 (t, *J* = 9.6 Hz, 1H), 2.07 - 1.86 (m, 2H), 1.68 (q, *J* = 12.4, 13.2 Hz, 1H), 1.45 - 1.17 (m, 2H).

Preparation Example 53: Preparation of Compound **53**

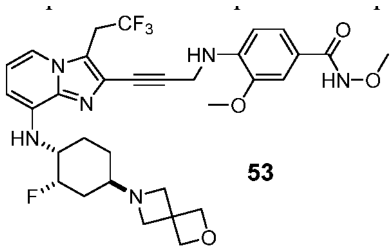

**53**

For the synthesis of Compound **53,** Preparation Example 47 could be referred to.

Compound **53: MS m/z (ESI):** 645.3 [M+H]$^+$. **$^1$H NMR** (400 MHz, DMSO-$d_6$) δ 8.29 (s, 1H), 7.77 (d, *J* = 6.8 Hz, 1H), 7.33 (dd, *J* = 2.0, 8.4 Hz, 1H), 7.25 (d, *J* = 2.0 Hz, 1H), 6.82 (t, *J* = 7.2 Hz, 1H), 6.74 (d, *J* = 8.4 Hz, 1H), 6.35 (d, *J* = 7.6 Hz, 1H), 6.08 (t, *J* = 6.4 Hz, 1H), 5.36 (d, *J* = 9.2 Hz, 1H), 4.92 (d, *J* = 50.0 Hz, 1H), 4.58 (s, 4H), 4.26 (d, *J* = 6.4 Hz, 2H), 4.02 (q, *J* = 10.8 Hz, 2H), 3.84 (s, 3H), 3.74 - 3.60 (m, 4H), 3.28 - 3.19 (m, 4H), 2.23 - 2.16 (m, 1H), 2.07 - 1.99 (m, 1H), 1.85 - 1.76 (m, 1H), 1.74 - 1.67 (m, 1H), 1.61 - 1.49 (m, 1H), 1.41 - 1.21 (m, 1H), 1.14 - 0.99 (m, 1H).

Preparation Example 54: Preparation of Compound **54**

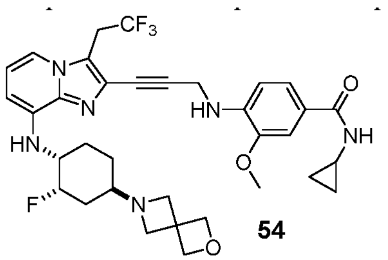

**54**

Synthesis scheme:

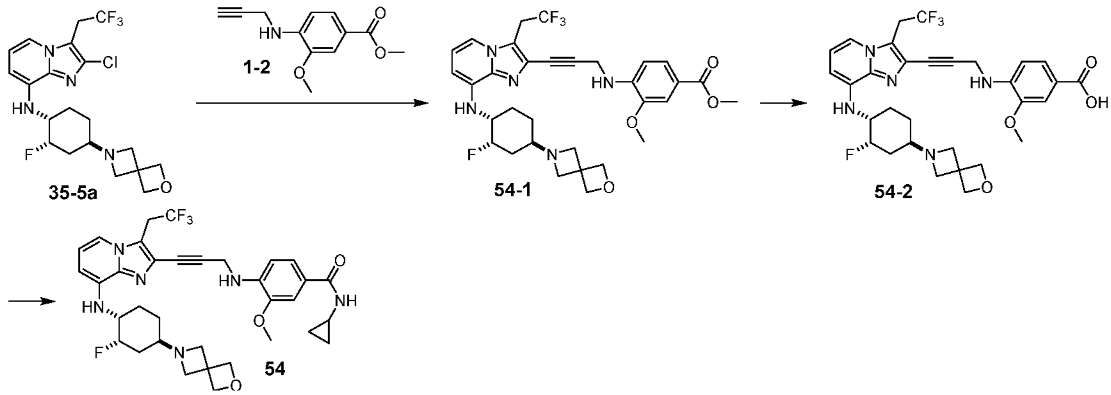

**35-5a** **1-2** **54-1** **54-2** **54**

Step 1: Synthesis of Compound **54-1**

2-Dicyclohexylphosphino-2',4',6'-triisopropylbiphenyl (85 mg, 0.18 mmol), bis(acetonitrile)palladium chloride (4 mg, 0.02 mmol) and cesium carbonate (335 mg, 1.03 mmol) were added to a solution of Compound **35-5a** (200 mg, 0.45 mmol) in acetonitrile (6 mL). The mixture was purged with nitrogen three times and stirred at 90 °C for 10 min. Then, a solution of Compound **1-2** (390 mg, 1.78 mmol) in acetonitrile (2 mL) was added, and the reaction solution was stirred at 90 °C for 2 hours under nitrogen atmosphere. The mixture was filtered, the filter cake was washed with ethyl acetate (10 mL

x3), and the filtrate was concentrated to dryness under reduced pressure. The crude product was purified by silica gel column chromatography (dichloromethane: methanol = 15: 1) to obtain Compound **54-1. MS m/z (ESI):** 630.3 [M+H]$^+$

Step 2: Synthesis of Compound **54-2**

Lithium hydroxide (0.03 mL, 1.75 mmol) was added to a solution of Compound **54-1** (220 mg, 0.35 mmol) in methanol (2 mL) and water (0.6 mL). The reaction solution was stirred at 60 °C for 2 hours. The reaction solution was adjusted to pH 5 with 1M dilute hydrochloric acid, and extracted with dichloromethane/isopropanol (30 mL X2, 3: 1). The mixed organic layer was washed with saturated brine (20 mL), dried over anhydrous sodium sulfate and filtered, and the filtrate was concentrated to obtain Compound **54-2,** which was directly used in the next step. **MS m/z (ESI):** 616.3 [M+H]$^+$.

Step 3: Synthesis of Compound **54**

Compound **54-2** (50 mg, 0.08 mmol) was dissolved in dichloromethane (1 mL), and 2-(7-azobenzotriazole)-N,N,N',N'-tetramethyluronium hexafluorophosphate (46 mg, 0.0.12 mmol), N,N-diisopropylethylamine (0.04 mL, 0.25 mmol) and cyclopropylamine (0.01 mL, 0.12 mmol) were added. The reaction solution was stirred at 20 °C for 30 minutes. The reaction mixture was poured into water (10 mL). The aqueous layer was extracted with dichloromethane (10 mL x2). The organic layers were combined and washed with saturated brine (10 mL), dried over anhydrous sodium sulfate, filtered, and the filtrate was concentrated. The crude product was purified by reverse phase flash chromatography (Waters-SunFire-C18-10μm-19*250mm, A: 0.1% FA/$H_2O$ B:ACN, flow rate: 25mL/min) to obtain Compound **54. MS m/z (ESI):** 655.3 [M+H]$^+$. **$^1$H NMR** (400 MHz, $CD_3OD$) δ 7.65 (d, *J* = 6.8 Hz, 1H), 7.45 (dd, *J* = 2.0, 8.4 Hz, 1H), 7.37 (d, *J* = 2.0 Hz, 1H), 6.89 - 6.83 (m, 2H), 6.42 (d, *J* = 7.6 Hz, 1H), 5.06 (brs, 1H), 4.77 (s, 4H), 4.35 (s, 2H), 3.93 (s, 3H), 3.85 (q, *J* = 10.4 Hz, 2H), 3.73 - 3.68 (m, 1H), 3.57 (s, 4H), 2.87 - 2.80 (m, 1H), 2.58 (br s, 1H), 2.30 - 2.26 (m, 1H), 2.04 - 1.93 (m, 2H), 1.74 - 1.62 (m, 1H), 1.52 - 1.19 (m, 2H), 0.81 (td, *J* = 4.8, 7.2 Hz, 2H), 0.64 (dt, *J* = 4.8, 7.2 Hz, 2H).

Preparation Example 55: Preparation of Compound **55**

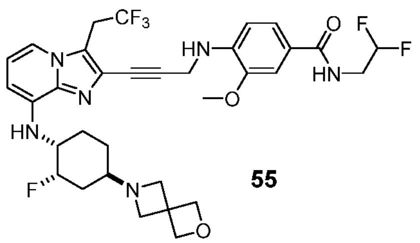

**55**

For the synthesis of Compound **55,** Preparation Example 54 could be referred to.

Compound **55: MS m/z (ESI):** 679.3 [M+H]$^+$. **$^1$H NMR** (400 MHz, $CD_3OD$) δ 7.61 (d, *J* = 6.8 Hz, 1H), 7.50 (dd, *J* = 2.0, 8.4 Hz, 1H), 7.40 (d, *J* = 2.0 Hz, 1H), 7.01 - 6.90 (m, 1H), 6.86 (d, *J* = 8.3 Hz, 1H), 6.55 (d, *J* = 7.4 Hz, 1H), 6.00 (tt, *J* = 4.4, 56.4 Hz, 1H), 5.72 (d, *J* = 25.1 Hz, 1H), 5.16 (s, 1H), 4.43 (s, 4H), 4.37 (s, 2H), 3.94 (s, 3H), 3.87 - 3.77 (m, 1H), 3.76 - 3.67 (m, 2H), 3.4 (s, 4H), 2.51 (dd, *J* = 12.2, 7.8 Hz, 1H), 2.17 (q, *J* = 8.8, 7.2 Hz, 2H), 1.90 - 1.26 (m, 4H).

Preparation Example 56: Preparation of Compound **56**

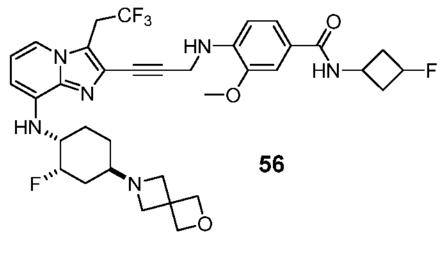

**56**

For the synthesis of Compound **56,** Preparation Example 54 could be referred to.

Compound **56: MS m/z (ESI):** 687.3 [M+H]$^+$. **$^1$H NMR** (400 MHz, DMSO-*d*$_6$) δ 7.66 (d, *J* = 6.8 Hz, 1H), 7.50 (dt, *J* =

2.4, 8.0 Hz, 1H), 7.39 (d, *J* = 2.0 Hz, 1H), 6.90 - 6.84 (m, 2H), 6.43 (d, *J* = 7.6 Hz, 1H), 5.25 - 5.07 (m, 1H), 4.77 (s, 4H), 4.68 - 4.60 (m, 1H), 4.36 (s, 2H), 4.11 - 4.02 (m, 1H), 3.94 (s, 3H), 3.87 (q, *J* = 10.4 Hz, 2H), 3.63 (s, 4H), 2.90 - 2.85 (m, 1H), 2.65 - 2.55 (m, 2H), 2.52 - 2.48 (m, 1H), 2.36 - 2.21 (m, 2H), 2.09 - 1.90 (m, 2H), 1.73 - 1.67 (m, 1H), 1.57 - 1.13 (m, 2H).

Preparation Examole 57: Preparation of Compound **57**

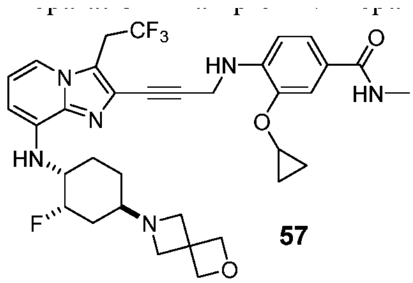

**57**

Synthesis scheme:

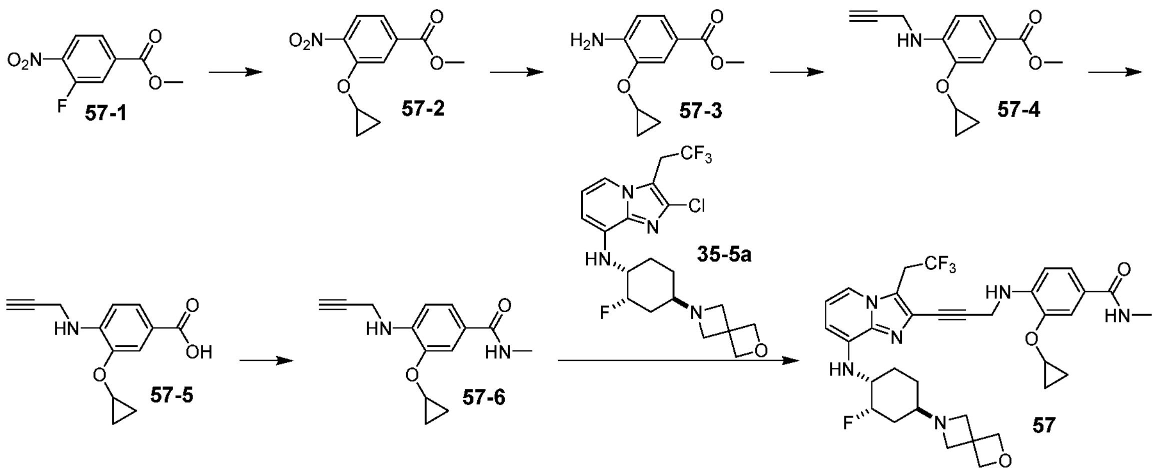

**57-1** **57-2** **57-3** **57-4** **35-5a** **57-5** **57-6** **57**

Step 1: Synthesis of Compound **57-2**

A solution of cyclopropanol (4.37 g, 75.32 mmol) in tetrahydrofuran (150 mL) was cooled to 0 °C. A solution of lithium bis(trimethylsilyl)amide in tetrahydrofuran (45.19 mL, 45.19 mmol) was added dropwise. The reaction solution was stirred at 0 °C for 30 minutes, and then a solution of Compound **57-1** (7.5 g, 37.66 mmol) in tetrahydrofuran (10 mL) was added dropwise to the reaction solution. The mixture was stirred at room temperature for 4 hours. The reaction solution was quenched with saturated ammonium chloride solution (30 mL), diluted with water (5 mL), and extracted with ethyl acetate (40 mL x2). The organic layers were combined and washed with saturated brine (40 mL), dried over anhydrous sodium sulfate, filtered, and the filtrate was concentrated. The crude product was purified by silica gel column chromatography (ethyl acetate/petroleum ether = 5/95) to obtain Compound **57-2.** **$^1$H NMR** (400 MHz, DMSO-*$d_6$*) δ 8.07 (d, *J* = 1.6 Hz, 1H), 7.99 (d, *J* = 8.4 Hz, 1H), 7.69 (dd, *J* = 1.6, 8.4 Hz, 1H), 4.26 - 4.18 (m, 1H), 3.92 (s, 3H), 0.93 - 0.85 (m, 2H), 0.79 - 0.70 (m, 2H).

Step 2: Synthesis of Compound **57-3**

To a solution of Compound **57-2** (5.1 g, 21.50 mmol) in ethanol (60 mL) and water (30 mL), iron powder (6.00 g, 107.50 mmol) and ammonium chloride (5.75 g, 107.50 mmol) were added, and the reaction solution was stirred at 80 °C for 2 hours. The reaction mixture was filtered through diatomaceous earth, washed with ethanol (20 mL x2), and ethanol was removed from the filtrate under reduced pressure. The mixture was diluted with water (100 mL) and extracted with ethyl acetate (100 mL x3). The combined organic phase was washed with saturated brine, dried over anhydrous sodium sulfate,

and filtered, and the filtrate was concentrated to obtain Compound **57-3. MS m/z (ESI):** 208.0 [M+H]+.

Step 3: Synthesis of Compound **57-4**

To a solution of Compound **57-3** (4.4 g, 21.23 mmol) in N,N-dimethylformamide (90 mL), 3-bromopropyne (2.75 mL, 31.85 mmol) and potassium carbonate (8.80 g, 63.70 mmol) were added, and the reaction solution was stirred at 70 °C for 18 hours. After the reaction solution was cooled to room temperature, it was diluted with water (40 mL) and extracted with ethyl acetate (80 mL x2). The organic layers were combined and washed with saturated brine (40 mL), dried over anhydrous sodium sulfate, filtered, and the filtrate was concentrated. The crude product was purified by silica gel column chromatography (ethyl acetate/petroleum ether = 1/6) to obtain Compound **57-4. MS m/z (ESI):** 246.0 [M+H]+.

Step 4: Synthesis of Compound **57-5**

Lithium hydroxide (1.71 g, 3.10 mmol) was added to a mixed solution of Compound **57-4** (2.0 g, 8.15 mmol) in methanol (20 mL), water (7 mL) and tetrahydrofuran (7 mL), and the mixture was stirred at 40 °C for 18 hours. The reaction solution was adjusted to pH 6 with 2M dilute hydrochloric acid and extracted with ethyl acetate (100 mL x2). The organic layers were combined and washed with saturated brine (10 mL), dried over anhydrous sodium sulfate and filtered, and the filtrate was concentrated to obtain Compound **57-5. MS m/z (ESI):** 232.0 [M+H]+.

Step 5: Synthesis of Compound **57-6**

Methylammonium hydrochloride (1.06 g, 15.74 mmol), N,N,N',N'-tetramethylchloroformamidine hexafluorophosphate (3.31 g, 11.81 mmol) and 1-methylimidazole (3.76 mL, 47.22 mmol) were added to a solution of Compound **57-5** (1.82 g, 7.87 mmol) in acetonitrile (36 mL), and the mixture was stirred at 25 °C for 2 hours. The reaction solution was diluted with water (100 mL) and extracted with ethyl acetate (80 mL x2). The organic layers were combined and washed with saturated brine (40 mL), dried over anhydrous sodium sulfate, filtered, and the filtrate was concentrated. The crude product was purified by silica gel column chromatography (ethyl acetate/petroleum ether = 1/1) to obtain Compound **57-6. MS m/z (ESI):** 245.0 [M+H]+.

Step 6: Synthesis of Compound **57**

To a solution of Compound **35-5a** (40 mg, 0.09 mmol) in acetonitrile (4 mL), 2-dicyclohexylphosphino-2',4',6'-triisopropylbiphenyl (90 mg, 0.37 mmol), bis(acetonitrile)palladium chloride (5 mg, 0.02 mmol) and cesium carbonate (70 mg, 0.21 mmol) were added. The mixture was purged with nitrogen three times and stirred at 90 °C for 10 minutes under nitrogen atmosphere. A solution of Compound **57-6** (90 mg, 0.37 mmol) in acetonitrile (1 mL) was added to the reaction solution, and the reaction solution was stirred at 90 °C for 2 hours under nitrogen atmosphere. After the reaction solution was cooled to room temperature, it was filtered, the filter cake was washed with ethyl acetate (10 mL x3), and the filtrate was concentrated to dryness under reduced pressure. The crude product was purified by reverse phase flash chromatography (Waters-SunFire-C18-10μm-19*250mm, A: 0.1% FA/$H_2O$ B:ACN, flow rate: 25mL/min) to obtain Compound **57. MS m/z (ESI):** 655.3 [M+H]+. **$^1$H NMR** (400 MHz, DMSO-$d_6$) δ 8.10 (d, *J* = 4.8 Hz, 1H), 7.77 (d, *J* = 6.8 Hz, 1H), 7.62 (d, *J* = 2.0 Hz, 1H), 7.41 (dd, *J* = 2.0, 8.4 Hz, 1H), 6.82 (d, *J* = 7.2 Hz, 1H), 6.72 (d, *J* = 8.4 Hz, 1H), 6.35 (d, *J* = 7.6 Hz, 1H), 5.84 (t, *J* = 6.4 Hz, 1H), 5.35 (d, *J* = 9.2 Hz, 1H), 4.92 (d, *J* = 50.0 Hz, 1H), 4.58 (s, 4H), 4.24 (d, *J* = 6.4 Hz, 2H), 4.01 (q, *J* = 10.8 Hz, 2H), 3.88 (td, *J* = 2.8, 6.0 Hz, 1H), 3.65 - 3.25 (m, 1H), 3.23 (t, *J* = 5.6 Hz, 4H), 2.74 (d, *J* = 4.4 Hz, 3H), 2.19 (t, *J* = 11.2 Hz, 1H), 2.03 - 2.01 (m, 1H), 1.81 (d, *J* = 9.6 Hz, 1H), 1.71 (d, *J* = 12.8 Hz, 1H), 1.55 (q, *J* = 11.8, - Hz, 1H), 1.31 (dt, *J* = 12.4, 44.4 Hz, 1H), 1.09 - 1.06 (m, 1H), 0.86 - 0.76 (m, 2H), 0.73 - 0.68 (m, 2H).

Preparation Example 58: Preparation of Compound **58**

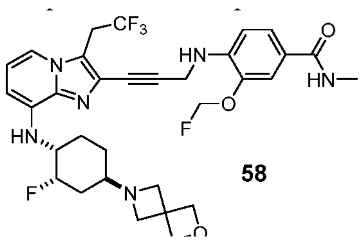

**58**

Synthesis scheme:

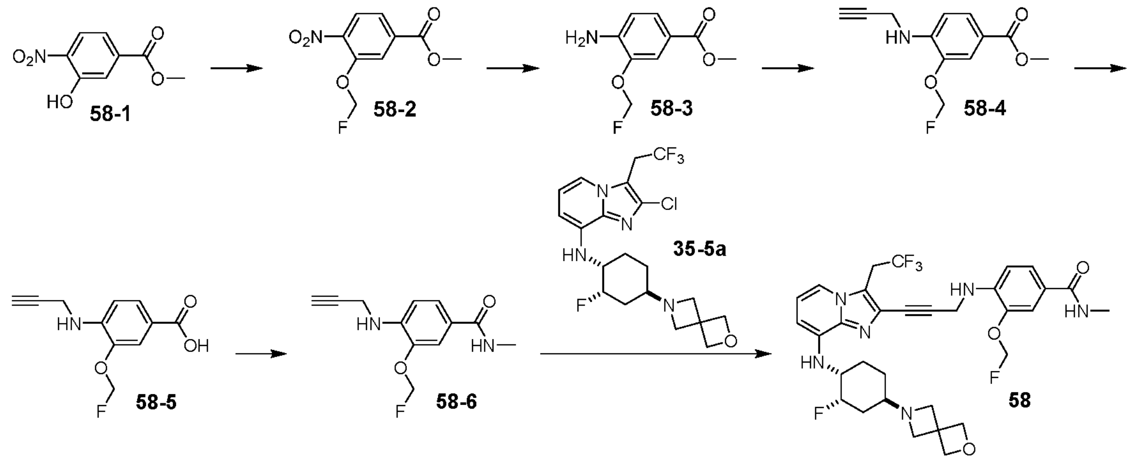

**58-1** **58-2** **58-3** **58-4** **35-5a** **58-5** **58-6** **58**

Step 1: Synthesis of Compound **58-2**

At 0 °C, sodium hydride (0.91 g, 22.83 mmol) was added in batches to a solution of Compound **58-1** (3.0 g, 15.22 mmol) in N,N-dimethylformamide (50 mL). The mixture was purged with nitrogen three times and stirred at 20 °C for 30 minutes, then chlorofluoromethane (3.79 mL, 60.87 mmol) was added, and the mixture was sealed and stirred at 80 °C for 4 hours under nitrogen atmosphere. After the reaction solution was cooled to room temperature, it was diluted with water (20 mL) and extracted with ethyl acetate (80 mL x2). The organic layers were combined and washed with saturated brine (40 mL), dried over anhydrous sodium sulfate, filtered, and the filtrate was concentrated. The crude product was purified by silica gel column chromatography (petroleum ether/ethyl acetate = 10/1) to obtain Compound **58-2.** $^1$H **NMR** (400 MHz, DMSO-$d_6$) δ 8.10 (d, *J* = 8.4 Hz, 1H), 7.94 (t, *J* = 1.6 Hz, 1H), 7.86 (dd, *J* =1.6, 8.4 Hz, 1H), 6.06 (d, *J* = 52.8 Hz, 2H), 3.92 (s, 3H).

Step 2: Synthesis of Compound **58**

For the synthesis of Compound **58,** Preparation Example 57 could be referred to.

Compound **58: MS m/z (ESI):** 647.3 [M+H]$^+$. **$^1$H NMR** (400 MHz, DMSO-$d_6$) δ 8.16 (d, *J* = 4.4 Hz, 1H), 7.79 - 7.75 (m, 1H), 7.54 (d, *J* = 6.2 Hz, 2H), 6.86 - 6.78 (m, 2H), 6.35 (d, *J* = 7.6 Hz, 1H), 6.25 (t, *J* = 6.4 Hz, 1H), 5.83 (d, *J* = 54.8 Hz, 2H), 5.36 (d, *J* = 9.2 Hz, 1H), 4.92 (d, *J* = 50.4 Hz, 1H), 4.58 (s, 4H), 4.28 (d, *J* = 6.0 Hz, 2H), 4.02 (d, *J* = 10.8 Hz, 2H), 3.76 - 3.60 (m, 1H), 3.22 (d, *J* = 3.6 Hz, 4H), 2.74 (d, *J* = 4.4 Hz, 3H), 2.20 - 2.16 (m, 1H), 2.05 - 2.01 (m, 1H), 1.85 - 1.66 (m, 2H), 1.55 (d, *J* = 12.0 Hz, 1H), 1.31 (d, *J* = 44.8 Hz, 1H), 1.07 (d, *J* = 12.4 Hz, 1H).

Preparation Example 59: Preparation of Compound **59**

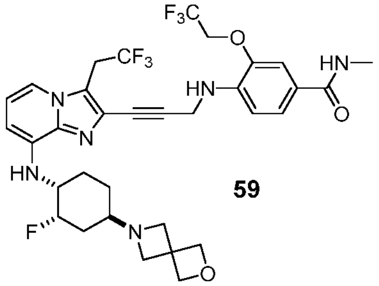

**59**

Synthesis scheme:

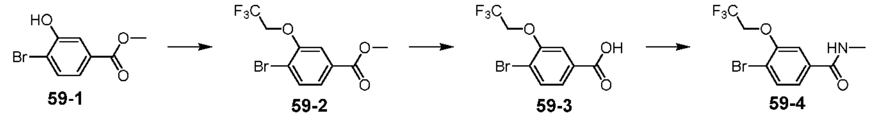

59-1 59-2 59-3 59-4

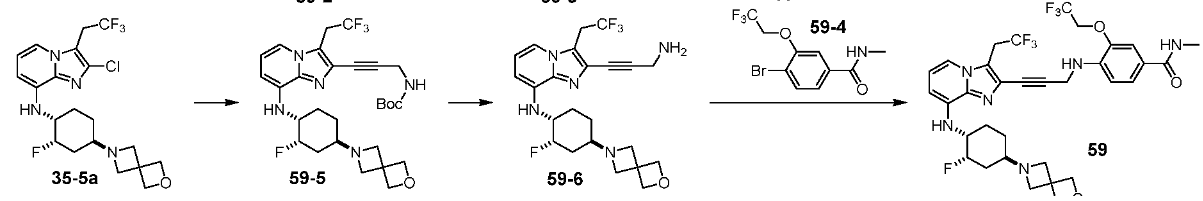

35-5a 59-5 59-6 59-4 59

Step 1: Synthesis of Compound **59-2**

Cesium carbonate (11.44 g, 82.79 mmol) and trifluoroethyl trifluoromethanesulfonate (0.94 mL, 6.49 mmol) were added to a solution of Compound **59-1** (1.0 g, 4.33 mmol) in N,N-dimethylformamide (10 mL). The reaction solution was stirred at 80 °C for 2 hours. The reaction mixture was poured into water (30 mL) and extracted with ethyl acetate (20 mL x2). The organic layers were combined and washed with saturated brine (10 mL), dried over anhydrous sodium sulfate and filtered, and the filtrate was concentrated under reduced pressure. The crude product was purified by silica gel column chromatography (petroleum ether/ethyl acetate = 5/1) to obtain Compound **59-2. MS m/z (ESI):** 313.1&315.1 $[M+H]^+$.

Step 2: Synthesis of Compound **59-3**

Lithium hydroxide (737 mg, 17.57 mmol) was added to a solution of Compound **59-2** (1.1 g, 3.51 mmol) in methanol (10 mL), tetrahydrofuran (3 mL) and water (3 mL). The reaction solution was stirred at 40 °C for 3 hours. The reaction mixture was adjusted to pH ~6 with 1M hydrochloric acid and extracted with ethyl acetate (50 mL x2). The organic layers were combined and washed with saturated sodium chloride aqueous solution (50 mL), dried over anhydrous sodium sulfate and filtered, and the filtrate was concentrated under reduced pressure to obtain Compound **59-3,** and the crude product was directly used in the next step.

Step 3: Synthesis of Compound **59-4**

To a solution of Compound **59-3** (1.0 g, 3.34 mmol) in dichloromethane (20 mL), 2-(7-azobenzotriazole)-N,N,N',N'-tetramethyluronium hexafluorophosphate (1.91 g, 5.02 mmol), N,N-diisopropylethylamine (1.66 mL, 10.03 mmol) and methylamine hydrochloride (268 mg, 4.01 mmol) were added. The reaction mixture was stirred at room temperature for 2 hours. The reaction mixture was poured into water (40 mL). The aqueous layer was extracted with ethyl acetate (40 mL x2). The organic layers were combined and washed with saturated sodium chloride aqueous solution (20 mL), dried over anhydrous sodium sulfate, filtered, and the filtrate was concentrated under reduced pressure. The crude product was purified by silica gel column chromatography (petroleum ether/ethyl acetate = 3/1) to obtain Compound **59-4. MS m/z (ESI):** 312.2, 314.2 $[M+H]^+$.

Step 4: Synthesis of Compound **59-5**

To a solution of Compound **35-5a** (40 mg, 0.09 mmol) in acetonitrile (4 mL), 2-dicyclohexylphosphino-2',4',6'-triisopropylbiphenyl (90 mg, 0.37 mmol), bis(acetonitrile)palladium chloride (5 mg, 0.02 mmol) and cesium carbonate (70 mg, 0.21 mmol) were added. The mixture was purged with nitrogen three times and stirred at 90 °C for 10 minutes under nitrogen atmosphere. A solution of N-Boc-aminopropyne (57 mg, 0.37 mmol) in acetonitrile (0.2 mL) was added to the reaction solution, and the reaction solution was stirred at 90 °C for 2 hours under nitrogen atmosphere. After the reaction solution was cooled to room temperature, it was filtered, and the filter cake was washed with ethyl acetate (10 mL x3). The filtrate was concentrated to dryness under reduced pressure. The crude product was purified by silica gel column chromatography (petroleum ether/ethyl acetate = 1/5) to obtain Compound **59-5. MS m/z (ESI):** 566.3 $[M+H]^+$.

Step 5: Synthesis of Compound **59-6**

Trifluoroacetic acid (1 mL) was added to a solution of Compound **59-5** (90 mg, 0.16 mmol) in dichloromethane (5 mL), and the reaction solution was stirred at room temperature for 2 hours. The reaction solution was slowly added dropwise to a saturated sodium bicarbonate aqueous solution (10 mL) and extracted with ethyl acetate (5 mL x2). The organic layers were combined and washed with saturated brine (5 mL), dried over anhydrous sodium sulfate, filtered, and the filtrate was concentrated. The obtained Compound **59-6** was directly used in the next step. **MS m/z (ESI):** 466.2

[M+H]+.

Step 6: Synthesis of Compound **59**

(2-Di-tert-butylphosphino-2',4',6'-triisopropyl-1,1'-biphenyl)(2'-amino-1,1'-biphenyl-2-yl) palladium(II) methanesulfonate (7 mg, 0.01 mmol), 2-di-tert-butylphosphino- 2',4',6'-triisopropylbiphenyl (3.6 mg, 0.01 mmol), sodium tert-butoxide (16 mg, 0.17 mmol) and Compound **59-4** (95 mg, 0.43 mmol) were added to a solution of Compound **59-6** (40 mg, 0.09 mmol) in toluene (2 mL). The reaction solution was stirred at 90 °C for 2 hours under nitrogen protection. The reaction mixture was poured into water (20 mL) and extracted with ethyl acetate (20 mL x2). The organic layers were combined and washed with saturated brine (20 mL), dried over anhydrous sodium sulfate, filtered, and the filtrate was concentrated. The crude product was purified by reverse phase flash chromatography (Waters-SunFire-C18-10μm-19*250mm, A: 0.1% FA/$H_2O$ B:ACN, flow rate: 25mL/min) to obtain Compound **59. MS m/z (ESI):** 697.3 $[M+H]^+$. **$^1$H NMR** (400 MHz, $CD_3OD$) δ 7.65 (d, *J* = 6.8 Hz, 1H), 7.55 (dd, *J* = 8.4, 2.0 Hz, 1H), 7.46 (d, *J* = 2.0 Hz, 1H), 6.96 (d, *J* = 8.4 Hz, 1H), 6.90 - 6.83 (m, 1H), 6.42 (d, *J* = 7.6 Hz, 1H), 5.06 - 4.90 (m, 1H), 4.77 (s, 4H), 4.63 (q, *J* = 8.4 Hz, 2H), 4.39 (s, 2H), 3.88 (q, *J* = 10.2 Hz, 2H), 3.50 (s, 4H), 2.96 (s, 5H). 3H), 2.53 - 2.48 (m, 1H), 2.28 - 2.24 (m, 1H), 2.04 - 1.92 (m, 2H), 1.69 (d, *J* = 14.0 Hz, 1H), 1.44 - 1.20 (m, 2H).

Preparation Example 60: Preparation of Compound **60**

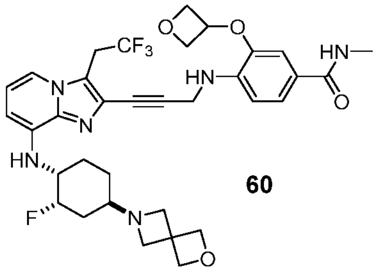

**60**

For the synthesis of Compound **60,** Preparation Example 59 could be referred to.

Compound **60: MS m/z (ESI):** 671.3 $[M+H]^+$. **$^1$H NMR** (400 MHz, $CD_3OD$) δ 7.66 (d, *J* = 6.8 Hz, 1H), 7.48 (dd, *J* = 2.0, 8.4 Hz, 1H), 6.97 (d, *J* = 2.0 Hz, 1H), 6.94 - 6.84 (m, 2H), 6.43 (d, *J* = 7.6 Hz, 1H), 5.38 - 5.32 (m, 1H), 5.08 (t, *J* = 6.4 Hz, 2H), 5.07 - 4.90 (m, 1H), 4.84 - 4.79 (m, 2H), 4.78 (s, 4H), 4.39 (s, 2H), 3.88 (q, *J* = 10.6 Hz, 2H), 3.84 - 3.71 (m, 1H), 3.70 (d, *J* = 3.6 Hz, 4H), 2.91 (s, 3H), 2.74 - 2.70 (m, 1H), 2.33 - 2.30 (m, 1H), 2.06 - 1.97 (m, 2H), 1.71 (d, *J* = 13.2 Hz, 1H), 1.56 - 1.31 (m, 2H).

Preparation Example 61: Preparation of Compound **61**

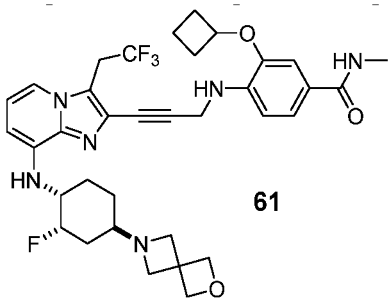

**61**

For the synthesis of Compound **61,** Preparation Example 59 could be referred to.

Compound **61: MS m/z (ESI):** 669.3 $[M+H]^+$. **$^1$H NMR** (400 MHz, $CD_3OD$) δ 7.65 (d, *J* = 6.8 Hz, 1H), 7.42 (dd, *J* = 2.0, 8.0 Hz, 1H), 7.22 (d, *J* = 2.0 Hz, 1H), 6.92 - 6.82 (m, 2H), 6.42 (d, *J* = 7.6 Hz, 1H), 5.06 - 4.90 (m, 1H), 4.82 - 4.78 (m, 1H), 4.77 (s, 4H), 4.36 (s, 2H), 3.87 (q, *J* = 10.4 Hz, 2H), 3.73 - 3.63 (m, 1H), 3.53 (s, 4H), 2.91 (s, 3H), 2.59 - 2.50 (m, 3H), 2.25 - 2.20 (m, 3H), 2.10 - 1.61 (m, 6H), 1.45 - 1.40 (m, 1H).

Preparation Example 62: Preparation of Compound **62**

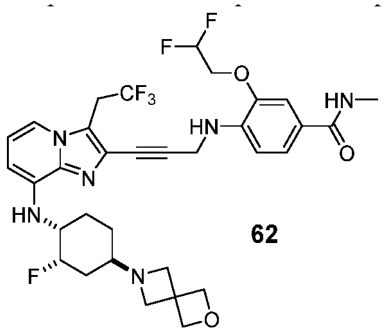

**62**

For the synthesis of Compound **62,** Preparation Example 59 could be referred to.

Compound **62: MS m/z (ESI):** 679.3 $[M+H]^+$. **$^1$H NMR** (400 MHz, $CD_3OD$) δ 7.65 (d, *J* = 6.4 Hz, 1H), 7.52 (dd, *J* = 8.4, 2.0 Hz, 1H), 7.42 (d, *J* = 2.0 Hz, 1H), 6.93 (d, *J* = 8.4 Hz, 1H), 6.87 (t, *J* = 7.2 Hz, 1H), 6.56 - 6.10 (m, 2H), 5.07 - 4.90 (m, 1H), 4.77 (s, 4H), 4.37 (s, 2H), 4.37 - 4.27 (m, 2H), 3.87 (q, *J* = 10.0 Hz, 2H), 3.70 (d, *J* = 29.6 Hz, 1H), 3.57 (s, 4H), 2.92 (s, 3H), 2.60 - 2.56 (m, 1H), 2.30 - 2.27 (m, 1H), 2.04 - 1.93 (m, 2H), 1.69 (d, *J* = 14.0 Hz, 1H), 1.46 - 1.32 (m, 2H).

Preparation Example 63: Preparation of Compound **63**

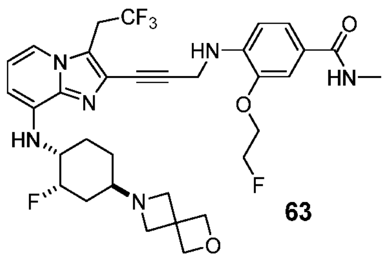

**63**

For the synthesis of Compound **63,** Preparation Example 59 could be referred to.

Compound **63: MS m/z (ESI):** 661.2 $[M+H]^+$. **$^1$H NMR** (400 MHz, $CD_3OD$) δ 7.66 (d, *J* = 6.8 Hz, 1H), 7.49 (dd, *J* = 2.0, 8.0 Hz, 1H), 7.40 (d, *J* = 2.0 Hz, 1H), 6.91 (d, *J* = 8.4 Hz, 1H), 6.88 (t, *J* = 7.2 Hz, 1H), 6.43 (d, *J* = 7.6 Hz, 1H), 5.15 (d, *J* = 57.2 Hz, 1H), 4.81 - 4.64 (m, 6H), 4.44 - 4.26 (m, 4H), 3.88 (q, *J* = 10.0 Hz, 2H), 3.78 - 3.65 (m, 1H), 3.59 (s, 4H), 2.92 (s, 3H), 2.66 - 2.54 (m, 1H), 2.34 - 2.24 (m, 1H), 2.00 (dd, *J* = 13.2, 30.0 Hz, 2H), 1.70 (d, *J* = 12.6 Hz, 1H), 1.56 - 1.37 (m, 1H), 1.31 - 1.15 (m, 1H).

Preparation Example 64: Preparation of Compound **64**

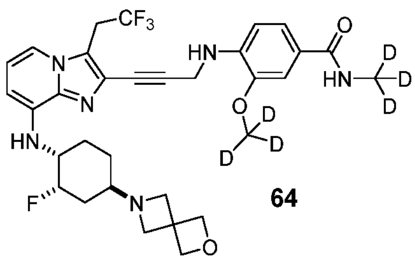

**64**

For the synthesis of Compound **64,** Preparation Example 58 could be referred to.

Compound **64: MS m/z (ESI):** 635.3 $[M+H]^+$. **$^1$H NMR** (400 MHz, DMSO-$d_6$) δ 8.09 (s, 1H), 7.77 (d, *J* = 6.8 Hz, 1H), 7.44 - 7.37 (m, 1H), 7.34 (d, *J= 2.0* Hz, 1H), 6.82 (t, *J* = 7.2 Hz, 1H), 6.74 (d, *J* = 8.4 Hz, 1H), 6.36 (d, *J* = 7.6 Hz, 1H), 5.97

(t, $J$ = 6.4 Hz, 1H), 5.36 (d, $J$ = 9.2 Hz, 1H), 4.93 (d, $J$ = 50.0 Hz, 1H), 4.58 (s, 4H), 4.26 (d, $J$ = 6.4 Hz, 2H), 4.02 (q, $J$ = 10.4 Hz, 2H), 3.72 - 3.65 (m, 1H), 3.26 - 3.20 (m, 4H), 2.21 - 2.17 (m, 1H), 2.05 - 2.01 (m, 1H), 1.83 - 1.70 (m, 2H), 1.58 - 1.54 (m, 1H), 1.37 - 1.26 (m, 1H), 1.10 - 1.06 (m, 1H).

Preparation Example 65: Preparation of Compounds **65, 65a** and **65b**

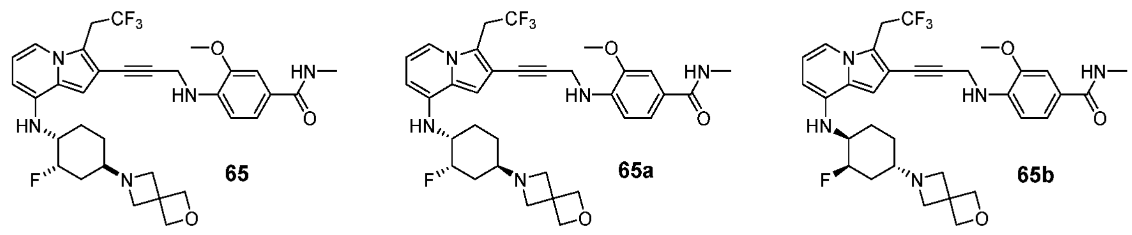

65 65a 65b

Synthesis scheme:

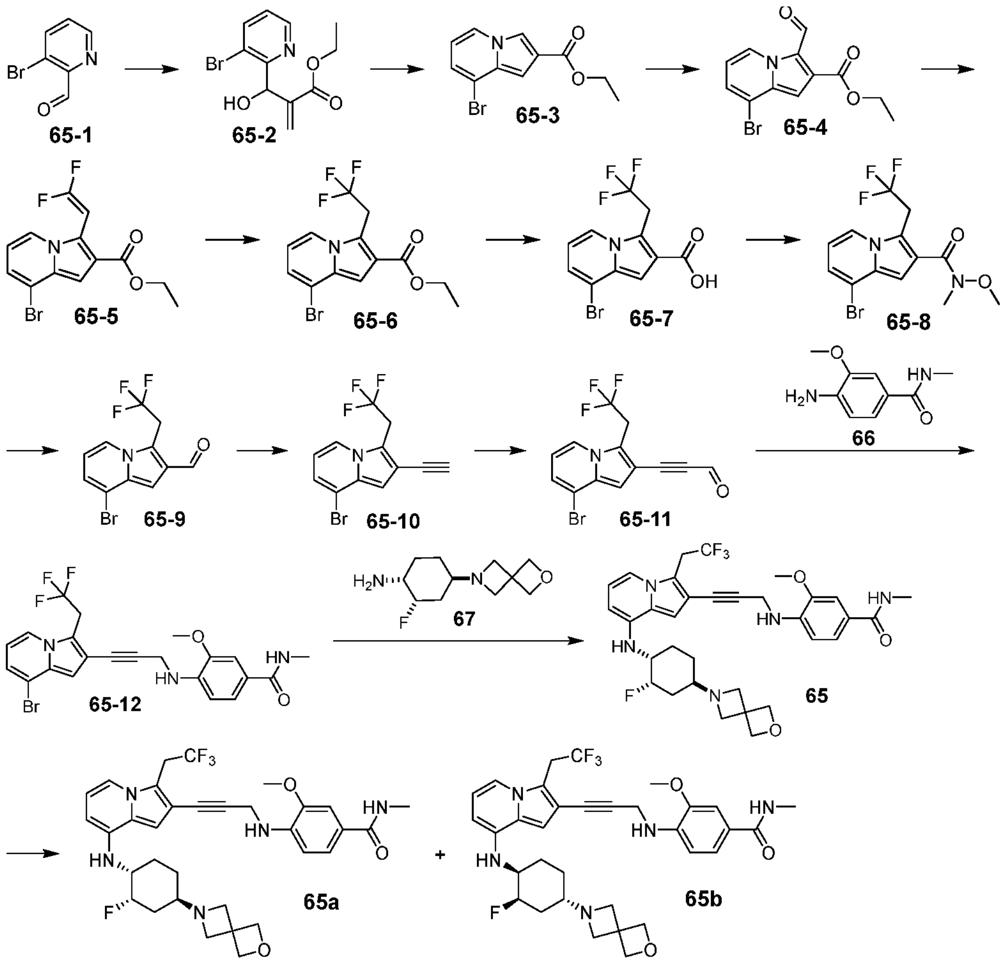

65-1 65-2 65-3 65-4 65-5 65-6 65-7 65-8 65-9 65-10 65-11 66 65-12 67 65 65a + 65b

Step 1: Synthesis of Compound **65-2**

DABCO (18.06 g, 161.28 mmol) was added to a solution of Compound 65-1 (50 g, 268.80 mmol) and ethyl acrylate (43.88 mL, 403.20 mmol) in water (500 mL) and dioxane (500 mL). The mixture was stirred at 20 °C for 18 hours. The reaction mixture was poured into water (500 mL). The aqueous layer was extracted with ethyl acetate (500 mL x2). The organic layers were combined and washed with saturated brine (500 mL) and dried over anhydrous sodium sulfate. After

filtering with filter paper, the filtrate was concentrated under reduced pressure. The crude product was purified by silica gel column chromatography (petroleum ether/ethyl acetate = 5/1) to obtain Compound **65-2. MS m/z (ESI):** 286.0, 288.0 $[M+H]^+$.

Step 2: Synthesis of Compound **65-3**

Compound **65-2** (35 g, 122.32 mmol) was dissolved in acetic anhydride (500 mL, 5338.43 mmol) and stirred at 120 °C under nitrogen protection for 18 hours. The reaction mixture was poured into a saturated sodium hydroxide aqueous solution (500 mL). The aqueous layer was extracted with ethyl acetate (500 mL x2). The organic layers were combined and washed with saturated brine (500 mL) and dried over anhydrous sodium sulfate. After filtering with filter paper, the organic layer was concentrated under reduced pressure. The crude product was purified by silica gel column chromatography (petroleum ether/ethyl acetate = 5/1) to obtain Compound **65-3.** $^1$**H NMR** (400 MHz, $CDCl_3$) δ 7.92 - 7.83 (m, 2H), 7.03 (d, *J* = 1.6 Hz, 1H), 6.96 (d, *J* = 7.2 Hz, 1H), 6.44 (t, *J* = 7.2 Hz, 1H), 4.39 (q, *J* = 7.2 Hz, 2H), 1.42 (t, *J* = 7.2 Hz, 3H).

Step 3: Synthesis of Compound **65-4**

At 0 °C and nitrogen atmosphere, phosphorus oxychloride (9.0 mL, 96.98 mmol) was added to N,N-dimethylformamide (60 mL) and they were stirred for 30 minutes. The above solution was added to a solution of Compound **65-3** (20 g, 74.60 mmol) in dichloromethane (200 mL). The mixture was stirred at 0 °C for 1 hour under nitrogen atmosphere. The reaction mixture was poured into water (300 mL) and extracted with ethyl acetate (300 mL x2). The organic layers were combined and washed with a saturated sodium chloride aqueous solution (300 mL) and dried over anhydrous sodium sulfate. After filtering with filter paper, it was concentrated under reduced pressure. The crude product was purified by silica gel column chromatography (petroleum ether/ethyl acetate = 5/1) to obtain Compound **65-4.** $^1$**H NMR** (400 MHz, $CDCl_3$) δ 10.57 (s, 1H), 9.84 (dd, *J* = 0.8, 7.2 Hz, 1H), 7.46 (d, *J* = 7.2 Hz, 1H), 7.24 (s, 1H), 6.89 (t, *J* = 7.2 Hz, 1H), 4.47 (q, *J* = 7.2 Hz, 2H), 1.47 (t, *J* = 7.2 Hz, 3H).

Step 4: Synthesis of Compound **65-5**

To a solution of Compound **65-4** (20 g, 60.79 mmol) in methylpyrrolidone (300 mL), (triphenylphosphonium) difluoroacetate (inner salt) (48.09 g, 135.08 mmol) was added. The mixture was stirred at 100 °C for 2 hours. The reaction mixture was poured into water (900 mL) and extracted with ethyl acetate (500 mL x 2). The organic layers were combined and washed with saturated sodium chloride aqueous solution (500 mL) and dried over anhydrous sodium sulfate. After filtering with filter paper, the filtrate was concentrated under reduced pressure. The crude product was purified by silica gel column chromatography (petroleum ether/ethyl acetate = 5/1) to obtain Compound **65-5.** $^1$**H NMR** (400 MHz, $CDCl_3$) δ 7.73 (dd, *J* = 2.8, 7.2 Hz, 1H), 7.13 (s, 1H), 7.04 (d, *J* = 7.2 Hz, 1H), 6.56 (t, *J* = 7.2 Hz, 1H), 5.81 (d, *J* = 26.7 Hz, 1H), 4.39 (q, *J* = 7.2 Hz, 2H), 1.43 (t, *J* = 7.2 Hz, 3H).

Step 5: Synthesis of Compound **65-6**

To a solution of Compound **65-5** (4.0 g, 12.12 mmol) in tetrahydrofuran (60 mL), 1M tetrabutylammonium fluoride tetrahydrofuran solution (24.23 mL, 24.23 mmol) was added at room temperature. The mixture was stirred at 60 °C for 2 hours. The reaction mixture was poured into water (20 mL) and extracted with ethyl acetate (20 mL x2). The organic layers were combined and washed with saturated brine (10 mL) and dried over anhydrous sodium sulfate. After filtering with filter paper, the filtrate was concentrated under reduced pressure. The crude product was purified by silica gel column chromatography (petroleum ether/ethyl acetate = 5/1) to obtain Compound **65-6.** $^1$**H NMR** (400 MHz, $CDCl_3$) δ 7.90 (d, *J* = 7.2 Hz, 1H), 7.13 (d, *J* = 0.8 Hz, 1H), 7.06 (d, *J* = 7.2 Hz, 1H), 6.57 (t, *J* = 7.2 Hz, 1H), 4.41 (q, *J* = 7.2 Hz, 2H), 4.26 (q, *J* = 10.0 Hz, 2H), 1.44 (t, *J* = 7.2 Hz, 3H).

Step 6: Synthesis of Compound **65-7**

To a solution of Compound **65-6** (2 g, 5.71 mmol) in ethanol (15 mL), tetrahydrofuran (15 mL) and water (10 mL), potassium hydroxide (0.96 g, 17.14 mmol) was added. The reaction mixture was stirred at 80 °C for 2 hours. The pH of the reaction mixture was adjusted to 5 with 1M dilute hydrochloric acid. The aqueous layer was extracted with ethyl acetate (50 mL x2). The combined organic layers were washed with saturated brine (50 mL) and dried over sodium sulfate. After filtering through filter paper, the filtrate was concentrated under reduced pressure to obtain Compound **65-7.** The crude product was used directly in the next step. **MS m/z (ESI):** 321.9, 323.9 $[M+H]^+$.

Step 7: Synthesis of Compound **65-8**

To a solution of Compound **65-7** (13 g, 40.36 mmol) in dichloromethane (130 mL), 2-(7-azobenzotriazole)-N,N,N',N'-Tetramethyluronium hexafluorophosphate (23.02 g, 60.54 mmol), N,N-diisopropylethylamine (33.4 mL, 201.81 mmol) and N,O-dimethylhydroxylamine hydrochloride (8.41 g, 121.09 mmol) were added. The reaction mixture was stirred at 20 °C for 2 hours. The reaction mixture was poured into water (300 mL). The aqueous layer was extracted with dichloromethane (100 mL x2). The combined organic layers were washed with saturated brine (50 mL) and dried over sodium sulfate. After filtering through filter paper, the organic layer was concentrated under reduced pressure. The crude product was purified by silica gel column chromatography (petroleum ether/ethyl acetate = 3/1) to obtain Compound **65-8. MS m/z (ESI):** 364.8, 366.8 $[M+H]^+$. **$^1$H NMR** (400 MHz, $CDCl_3$) δ 7.84 - 7.78 (m, 1H), 6.98 - 6.92 (m, 2H), 6.46 (t, *J* = 7.2 Hz, 1H), 4.15 (q, *J* = 10.3 Hz, 2H), 3.56 (s, 3H), 3.33 (s, 3H).

Step 8: Synthesis of Compound **65-9**

Diisobutylaluminum hydride (5.32 mL, 5.32 mmol, 1M n-hexane solution) was added to a solution of Compound **65-8** (1.8 g, 4.44 mmol) in tetrahydrofuran (20 mL) at 0 °C. The mixture was stirred at 0 °C for 1 hour. The reaction mixture was poured into water (100 mL) and extracted with ethyl acetate (100 mL x2). The organic layers were combined and washed with saturated brine (100 mL) and dried over sodium sulfate. After filtering through filter paper, the filtrate was concentrated under reduced pressure. The crude product was purified by silica gel column chromatography (petroleum ether/ethyl acetate = 5/1) to obtain Compound **65-9. $^1$H NMR** (400 MHz, $CDCl_3$) δ 10.12 (s, 1H), 7.84 (d, *J* = 7.2 Hz, 1H), 7.05 - 6.98 (m, 2H), 6.55 (t, *J* = 7.2 Hz, 1H), 4.11 (q, *J* = 10.0 Hz, 2H).

Step 9: Synthesis of Compound **65-10**

To a solution of Compound **65-9** (6.0 g, 19.60 mmol) in methanol (60 mL), (1Z)-1-(diazine-1-diamino)-1-λ5-phosphate (5.65 g, 29.40 mmol) and potassium carbonate (8.13 g, 58.81 mmol) were added. The mixture was stirred at 20 °C for 2 hours. The reaction mixture was poured into water (10 mL). The aqueous layer was extracted with ethyl acetate (30 mL x2). The organic layers were combined and washed with saturated brine (30 mL) and dried over sodium sulfate. After filtering through filter paper, the filtrate was concentrated under reduced pressure. The crude product was purified by silica gel column chromatography (petroleum ether/ethyl acetate = 10/1) to obtain Compound **65-10. MS m/z (ESI):** 301.9, 303.9 $[M+H]^+$. **$^1$H NMR** (400 MHz, $CDCl_3$) δ 7.72 (d, *J* = 7.2 Hz, 1H), 6.94 (d, *J* = 7.2 Hz, 1H), 6.71 (d, *J* = 0.8 Hz, 1H), 6.44 (t, *J* = 7.2 Hz, 1H), 3.75 (q, *J* = 10.0 Hz, 2H), 3.17 (s, 1H).

Step 10: Synthesis of Compound **65-11**

Compound **65-10** (300 mg, 0.99 mmol) was dissolved in N,N-dimethylformamide dimethyl acetal (6 mL), and the mixture was stirred at 100 °C for 18 hours. The reaction mixture was poured into water (30 mL), and extracted with ethyl acetate (50 mL x2). The organic layers were combined and washed with saturated brine (50 mL) and dried over sodium sulfate. After filtering through filter paper, the filtrate was concentrated under reduced pressure. The crude product was purified by silica gel column chromatography (petroleum ether/ethyl acetate = 10/1) to obtain Compound **65-11. MS m/z (ESI):** 330.0, 332.0 $[M+H]^+$. **$^1$H NMR** (400 MHz, $CDCl_3$) δ 9.39 (s, 1H), 7.76 (d, *J* = 7.2 Hz, 1H), 7.01 (d, *J* = 7.2 Hz, 1H), 6.85 (d, *J* = 0.8 Hz, 1H), 6.52 (t, *J* = 7.2 Hz, 1H), 3.80 (q, *J* = 10.0 Hz, 2H).

Step 11: Synthesis of Compound **65-12**

Acetic acid (0.1 mL) and sodium triacetoxyborohydride (124 mg, 0.59 mmol) were added to a solution of Compound **66** (122 mg, 0.68 mmol) and Compound **65-11** (130 mg, 0.39 mmol) in dichloromethane (3 mL). The mixture was stirred at 20 °C for 1 hour. The reaction mixture was poured into water (10 mL) and extracted with dichloromethane (10 mL x2). The organic layers were combined and washed with saturated brine (10 mL) and dried over sodium sulfate. After filtering through filter paper, the filtrate was concentrated under reduced pressure. The crude product was purified by silica gel column chromatography (petroleum ether/ethyl acetate = 1/1) to obtain Compound **65-12. MS m/z (ESI):** 494.1, 496.1 $[M+H]^+$.

Step 12: Synthesis of Compound **65**

To a solution of Compound **65-12** (60 mg, 0.12 mmol) and Compound **67** (40 mg, 0.19 mmol) in 1,4-dioxane (1 mL), cesium carbonate (107 mg, 0.33 mmol), tris(dibenzylideneacetone)dipalladium (17 mg, 0.02 mmol) and 1,1'-binaphthyl-2,2'-bisdiphenylphosphine (12 mg, 0.02 mmol) were added. The mixture was stirred at 100 °C for 3 hours

under nitrogen atmosphere. After the reaction solution was cooled to room temperature, it was poured into water (10 mL) and extracted with ethyl acetate (10 mL x2). The organic layers were combined and washed with saturated brine (10 mL) and dried over sodium sulfate. After filtering through filter paper, the filtrate was concentrated under reduced pressure. The crude product was purified by reverse phase flash chromatography (Waters-SunFire-C18-10μm-19*250mm, A: 0.1% FA/$H_2O$ B:ACN, flow rate: 25mL/min) to give Compound **65. MS m/z (ESI):** 628.3 $[M+H]^+$. **$^1$H NMR** (400 MHz, $CD_3OD$) δ 7.50 - 7.41 (m, 2H), 7.37 (d, *J* = 2.0 Hz, 1H), 6.86 (d, *J* = 8.4 Hz, 1H), 6.62 (s, 1H), 6.53 (t, *J* = 7.2 Hz, 1H), 5.89 (d, *J* = 7.2 Hz, 1H), 5.12 - 5.03 (m, 1H), 4.79 (s, 4H), 4.29 (s, 2H), 3.94 (s, 3H), 3.87 (s, 4H), 3.78 (t, *J*= 10.4 Hz, 2H), 3.65 - 3.62 (m, 1H), 2.92 (s, 3H), 2.35 - 2.31 (m, 1H), 2.05 - 2.00 (m, 2H), 1.85 - 1.74 (m, 1H), 1.56 - 1.15 (m, 3H).

Compound **65** was subjected to SFC resolution to obtain Compounds **65c** and **65d** that were isomers between each other. Compounds **65c** and **65d** were selected from Compounds **65a** and **65b,** respectively, and they were different from each other.

Compound **65c:** SFC method: Instrument: Waters 150 SFC (SFC-26), Column: AD, 250×30mm, Mobile phase: A for $CO_2$ and B for Ethanol (0.1%$NH_3H_2O$), Isocratic: B: 35%, Flow rate: 150 mL/min, Column temperature: R.T., Wavelength: 220 nm, Back pressure: 100 bar. Retention time: 1.91 min. **MS m/z (ESI):** 628.3 $[M+H]^+$. **$^1$H NMR** (400 MHz, $CD_3OD$) δ 7.48 - 7.41 (m, 2H), 7.37 (d, *J* = 2.0 Hz, 1H), 6.86 (d, *J* = 8.4 Hz, 1H), 6.61 (s, 1H), 6.53 (t, *J* = 7.2 Hz, 1H), 5.88 (d, *J* = 7.2 Hz, 1H), 5.05 - 5.01 (m, 1H), 4.76 (s, 4H), 4.29 (s, 2H), 3.93 (s, 3H), 3.79 (q, *J* = 10.4 Hz, 2H), 3.63 - 3.60 (m, 1H), 3.46 (s, 4H), 2.92 (s, 3H), 2.35 - 2.31 (m, 1H), 2.05 - 2.00 (m, 2H), 1.85 - 1.74 (m, 1H), 1.49 - 1.16 (m, 3H).

Compound **65d:** SFC method: Instrument: Waters 150 SFC (SFC-26), Column: AD, 250×30mm, Mobile phase: A for $CO_2$ and B for Ethanol (0.1%$NH_3H_2O$), Isocratic: B: 35%, Flow rate: 150 mL/min, Column temperature: R.T., Wavelength: 220 nm, Back pressure: 100 bar. Retention time: 2.61 min. **MS m/z (ESI):** 628.3 $[M+H]^+$. **$^1$H NMR** (400 MHz, $CD_3OD$) δ 7.48 - 7.41 (m, 2H), 7.37 (d, *J* = 2.0 Hz, 1H), 6.86 (d, *J* = 8.4 Hz, 1H), 6.61 (s, 1H), 6.53 (t, *J* = 7.2 Hz, 1H), 5.88 (d, *J*= *7.2* Hz, 1H), 5.05 - 5.01 (m, 1H), 4.76 (s, 4H), 4.29 (s, 2H), 3.94 (s, 3H), 3.79 (q, *J* = 10.4 Hz, 2H), 3.63 - 3.60 (m, 1H), 3.47 (s, 4H), 2.92 (s, 3H), 2.35 - 2.31 (m, 1H), 2.05 - 2.00 (m, 2H), 1.85 - 1.74 (m, 1H), 1.47 - 1.16 (m, 3H).

Preparation Example 66: Preparation of Compound **66**

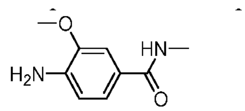

**66**

For the synthesis of Compound **66,** Preparation Example 27-3 could be referred to.

Compound **66: MS m/z (ESI):** 181.1 $[M+H]^+$. **$^1$H NMR** (400 MHz, DMSO-$d_6$) δ 8.01 (q, *J* = 4.8 Hz, 1H), 7.30 (d, *J* = 2.0 Hz, 1H), 7.26 (dd, *J* = 2.0, 8.4 Hz, 1H), 6.61 (d, *J* = 8.0 Hz, 1H), 5.19 (s, 2H), 3.80 (s, 3H), 2.74 (d, *J* = 4.4 Hz, 3H).

Preparation Example 67: Preparation of Compound **67**

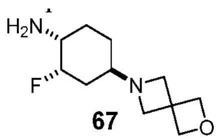

**67**

Synthesis scheme:

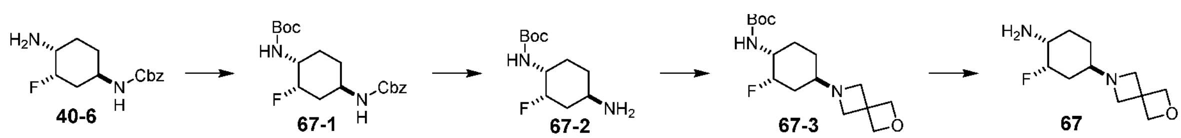

**40-6** **67-1** **67-2** **67-3** **67**

Step 1: Synthesis of Compound **67-1**

Compound **40-6** (3.0 g, 11.26 mmol), di-tert-butyl dicarbonate (5.0 mL, 23.37 mmol) and triethylamine (5.0 mL, 36.07 mmol) were added to dichloromethane (30 mL), and the mixture was stirred at 25 °C for 18 hours. The reaction solution was concentrated, and the crude product was purified by silica gel column chromatography (petroleum ether/ethyl acetate = 3/1) to obtain Compound **67-1.** $^1$**H NMR** (400 MHz, $CD_3OD$) δ 7.26 - 7.16 (m, 5H), 4.96 (s, 2H), 4.83 - 4.61 (m, 1H), 3.66 - 3.58 (m, 1H), 3.44 - 3.32 (m, 1H), 2.26 - 2.06 (m, 1H), 1.89 - 1.84 (m, 1H), 1.68 - 1.55 (m, 2H), 1.57 - 1.21 (m, 11H).

Step 2: Synthesis of Compound **67-2**

Palladium hydroxide (3.0 g, 4.27 mmol) was added to a mixed solution of methanol (100 mL) and tetrahydrofuran (100 mL) containing Compound **67-1** (4.0 g, 10.92 mmol). The mixture was stirred at 25 °C for 4 hours under the hydrogen protection at normal pressure. The reaction solution was filtered through diatomaceous earth, and the filter cake was washed with methanol (20 mL x3). The filtrate was concentrated to obtain Compound **67-2.** $^1$**H NMR** (400 MHz,DMSO-$d_6$) δ 6.81 (d, *J* = 7.6 Hz, 1H), 4.72 (dd, *J* = 4.0, 52.4 Hz, 1H), 3.50 - 3.31 (m, 1H), 2.81 - 2.69 (m, 1H), 2.10 - 1.99 (m, 1H), 1.79 - 1.70 (m, 1H), 1.58 - 1.49 (m, 2H), 1.39 (s, 9H), 1.36 - 1.18 (m, 1H), 1.18 - 1.03 (m, 1H).

Step 3: Synthesis of Compound **67-3**

Acetonitrile (10 mL) was added to Compound **67-2 (6.0** g, 24.60 mmol) and sodium carbonate (2.74 g, 25.83 mmol), and the mixture was stirred at 80 °C for 18 hours. The reaction solution was filtered and concentrated under reduced pressure. The crude product was purified by silica gel column chromatography (dichloromethane/methanol = 10/1) to obtain Compound **67-3.** $^1$**H NMR** (400 MHz, DMSO-$d_6$) δ 6.90 (d, *J* = 7.6 Hz, 1H), 4.82 - 4.63 (m, 1H), 4.57 (s, 4H), 3.49 - 3.41 (m, 1H), 3.23 - 3.15 (m, 4H), 2.16 - 2.04 (m, 1H), 1.99 - 1.93 (m, 1H), 1.73 - 1.62 (m, 1H), 1.55 - 1.45 (m, 2H), 1.38 (s, 9H), 1.26 - 1.05 (m, 1H), 1.03 - 0.90 (m, 1H).

Step 4: Synthesis of Compound **67**

Compound **67-3** (2.5 g, 7.95 mmol) was added to a mixed solution of trifluoroacetic acid (4 mL) and dichloromethane (20 mL), and the reaction solution was stirred at 25 °C for 2 hours. The reaction solution was concentrated and diluted with dichloromethane (20 mL), and washed with saturated sodium bicarbonate aqueous solution (10 mL). The organic phase was dried over anhydrous sodium sulfate and filtered, and the filtrate was concentrated under reduced pressure. The crude product was purified by silica gel chromatography (dichloromethane/methanol = 10/1) to obtain Compound 67. $^1$**H NMR** (400 MHz, DMSO-$d_6$) δ 4.73 - 4.53 (m, 5H), 3.20 (s, 4H), 2.67 - 2.52 (m, 1H), 2.14 - 2.04 (m, 1H), 1.98 - 1.90 (m, 1H), 1.65 - 1.42 (m, 4H), 1.36 - 1.03 (m, 2H), 0.99 - 0.86 (m, 1H).

Preparation Example 68: Preparation of Compounds **68, 68a** and **68b**

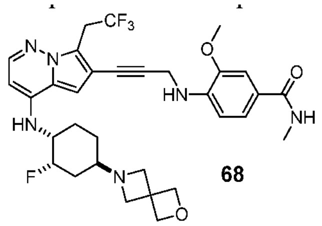
**68**

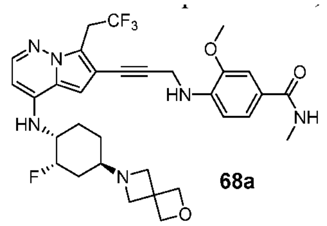
**68a**

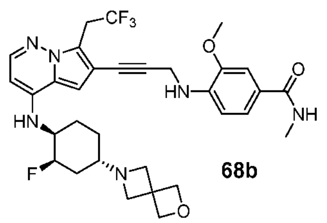
**68b**

Synthesis scheme:

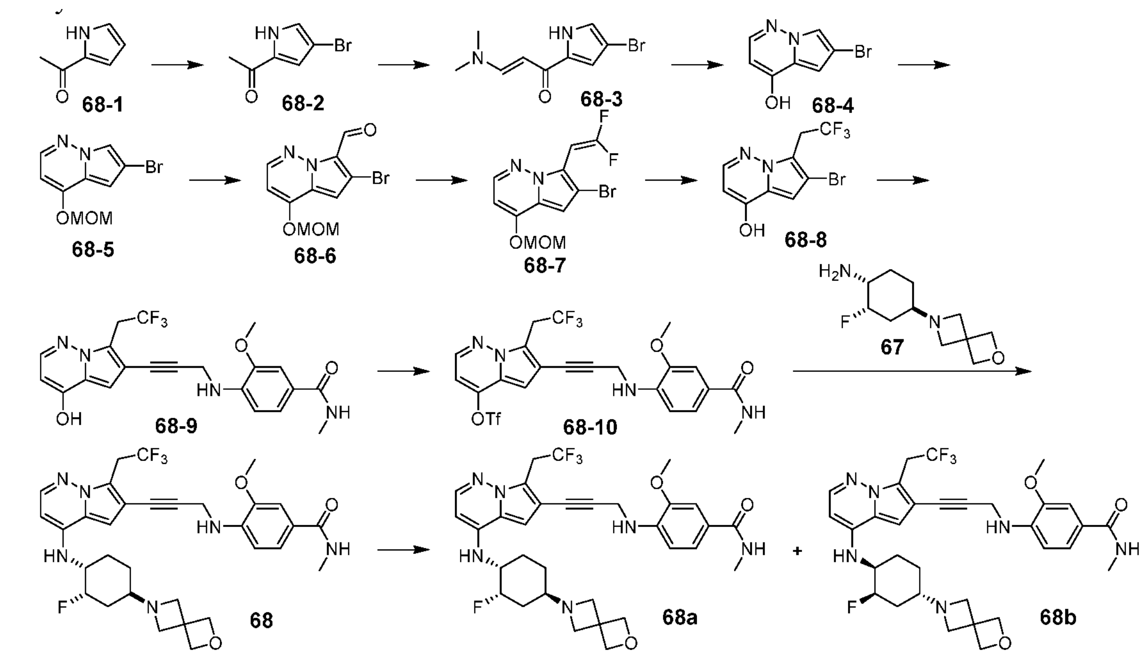

68-1 68-2 68-3 68-4 68-5 68-6 68-7 68-8 67 68-9 68-10 68 68a + 68b

Step 1: Synthesis of Compound **68-2**

Under nitrogen protection at -60 °C, a solution of bromine (2.83 mL, 55.13 mmol) in dichloromethane (115 mL) was added dropwise to a solution of Compound **68-1** (5.0 g, 45.82 mmol) in dichloromethane (230 mL). Under nitrogen protection, the mixture was stirred at -60 °C for 2 hours. The reaction solution was poured into water (500 mL) and extracted with dichloromethane (500 mL x3). The organic phases were combined and washed with saturated brine (1000 mL). The organic phase was dried over anhydrous sodium sulfate and filtered, and the filtrate was concentrated to obtain Compound **68-2.** The crude product was directly used in the next reaction. **MS m/z (ESI):** 188.0, 190.0 $[M+H]^+$. **$^1$H NMR** (400 MHz, $CDCl_3$) δ 9.40 (s, 1H), 6.94 (dd, *J* = 1.6, 2.8 Hz, 1H), 6.82 (dd, *J* = 1.6, 2.8 Hz, 1H), 2.34 (s, 3H).

Step 2: Synthesis of Compound **68-3**

Under nitrogen protection, Compound **68-2** (5.0 g, 45.82 mmol) was dissolved in N,N-dimethylformamide dimethyl acetal (42 mL). Under nitrogen protection, the mixture was stirred at 85 °C for 18 hours. The reaction solution was concentrated under reduced pressure, slurried with methyl tert-butyl ether (15 mL), and filtered to obtain Compound **68-3. MS m/z (ESI):** 243.0, 245.0 $[M+H]^+$. **$^1$H NMR** (400 MHz, $CDCl_3$) δ 9.80 (s, 1H), 7.75 (d, *J* = 12.4 Hz, 1H), 6.91 (dd, *J* = 1.6, 2.8 Hz, 1H), 6.73 (dd, *J* = 1.6, 2.8 Hz, 1H), 5.47 (d, *J* = 12.4 Hz, 1H), 3.11 (s, 3H), 2.92 (s, 3H).

Step 3: Synthesis of Compound **68-4**

Under nitrogen protection, potassium tert-butoxide (3.7 g, 33.04 mmol) was added to a solution of Compound **68-3** (5.34 g, 19.77 mmol) in N-methylpyrrolidone (160 mL). Under nitrogen protection, the mixture was stirred at 25 °C for 0.5 hours. O-p-nitrobenzoylhydroxylamine (6.0 g, 32.94 mmol) was added to the reaction solution under nitrogen protection, and the mixture was stirred at 25 °C for 2.5 hours. The reaction solution was poured into water (500 mL) and extracted with methyl tert-butyl ether (500 mL x3). The organic phases were combined and washed with saturated brine (1000 mL), dried over anhydrous sodium sulfate, filtered, and the filtrate was concentrated. The crude product was purified by silica gel column chromatography (ethyl acetate: petroleum ether = 1:4) to obtain Compound **68-4. MS m/z (ESI):** 213.0, 245.0 $[M+H]^+$.

Step 4: Synthesis of Compound **68-5**

Under nitrogen protection, chloromethyl methyl ether (0.76 g, 9.38 mmol) was added to a solution of Compound **68** (1.0 g, 2.16 mmol) and N,N-diisopropylethylamine (1.55 mL, 9.36 mmol) in dichloromethane (10 mL). The mixture was stirred at 25 °C for 16 hours under nitrogen protection. The reaction solution was poured into water (50 mL) and extracted with dichloromethane (50 mL x3). The organic phases were combined and washed with saturated brine (100 mL). The resultant organic phase was dried over anhydrous sodium sulfate and filtered, and the filtrate was concentrated. The crude

product was purified by silica gel column chromatography (ethyl acetate: petroleum ether = 1:9) to obtain Compound **68-5. MS m/z (ESI):** 257.0, 259.0 [M+H]+. **$^1$H NMR** (400 MHz, $CDCl_3$) δ 7.88 (d, *J* = 5.6 Hz, 1H), 7.67 (d, *J* = 2.0 Hz, 1H), 6.66 (d, *J = 2.0* Hz, 1H), 6.17 (d, *J* = 5.6 Hz, 1H), 5.35 (s, 2H), 3.52 (s, 3H).

Step 5: Synthesis of Compound **68-6**

Under nitrogen protection at 0 °C, phosphorus oxychloride (0.17 mL, 1.88 mmol) was dissolved in N,N-dimethylformamide (10 mL). Under nitrogen protection at 0 °C, the above solution of phosphorus oxychloride in N,N-dimethylformamide was added to a solution of Compound **68-5** (960 mg, 1.57 mmol) in dichloromethane (10 mL). Under nitrogen protection, the mixture was stirred at 0 °C for 1 hour. The reaction solution was poured into water (50 mL) and extracted with dichloromethane (50 mL x3). The organic phases were combined and washed with saturated brine (100 mL). The resultant organic phase was dried over anhydrous sodium sulfate and filtered, and the filtrate was concentrated. The crude product was purified by silica gel column chromatography (ethyl acetate: petroleum ether = 1:4) to obtain Compound **68-6. MS m/z (ESI):** 285.0, 287.0 [M+H]+. **$^1$H NMR** (400 MHz, $CDCl_3$) δ 10.28 (s, 1H), 8.20 (d, *J* = 5.6 Hz, 1H), 6.83 (s, 1H), 6.55 (d, *J* = 5.6 Hz, 1H), 5.40 (s, 2H), 3.54 (s, 3H).

Step 6: Synthesis of Compound **68-7**

Under nitrogen protection, ethyl 2,2-difluoro-2-(triphenylphosphonyl)acetate (6.10 g, 17.13 mmol) was added to a solution of Compound **68-6** (3.97 g, 13.41 mmol) in N-methylpyrrolidone (40 mL). The mixture was stirred at 100 °C for 2 hours under nitrogen protection. The reaction solution was poured into water (200 mL) and extracted with ethyl acetate (200 mL x3). The organic phases were combined and washed with saturated brine (500 mL). The organic phase was dried over anhydrous sodium sulfate and filtered, and the filtrate was concentrated. The crude product was purified by silica gel column chromatography (ethyl acetate: petroleum ether = 1:9) to obtain Compound **68-7. MS m/z (ESI):** 319.0, 321.0 [M+H]+. **$^1$H NMR** (400 MHz, $CDCl_3$) δ 7.96 (d, *J* = 5.6 Hz, 1H), 6.77 (s, 1H), 6.22 (d, *J* = 5.6 Hz, 1H), 5.58 (dd, *J* = 1.6, 26.0 Hz, 1H), 5.36 (s, 2H), 3.52 (s, 3H).

Step 7: Synthesis of Compound **68-8**

Under nitrogen protection, 1M tetrabutylammonium fluoride tetrahydrofuran solution (4.0 mL, 4.00 mmol) was added to a solution of Compound **68-7** (335 mg, 1.05 mmol) in tetrahydrofuran (4.0 mL). Under nitrogen protection, the mixture was stirred at 70 °C for 16 hours. The reaction solution was poured into water (50 mL) and extracted with ethyl acetate (200 mL x3). The organic phases were combined and washed with saturated brine (500 mL). The resultant organic phase was dried over anhydrous sodium sulfate and filtered, and the filtrate was concentrated. The crude product was purified by silica gel column chromatography (ethyl acetate: petroleum ether = 1:1) to obtain Compound **68-8. MS m/z (ESI):** 295.0, 297.0 [M+H]+. **$^1$H NMR** (400 MHz, DMSO-*$d_6$*) δ 11.79 (s, 1H), 8.06 (d, *J* = 5.6 Hz, 1H), 6.78 (s, 1H), 6.15 (d, *J* = 5.6 Hz, 1H), 4.06 - 3.94 (m, 2H).

Step 8: Synthesis of Compound **68-9**

Under nitrogen protection, bis(acetonitrile)palladium dichloride (70 mg, 0.27 mmol) and 2-dicyclohexylphosphino-2',4',6'-triisopropylbiphenyl (388 mg, 0.81 mmol) were added to a solution of Compound **68-8** (335 mg, 1.05 mmol), Compound **1-4** (1180 mg, 4.87 mmol), and cesium carbonate (2650 mg, 8.13 mmol) in acetonitrile (40 mL). Under nitrogen protection, the mixture was stirred at 70 °C for 20 hours. The reaction solution was poured into water (50 mL), adjusted to pH 2 with 1M dilute hydrochloric acid, and extracted with ethyl acetate (100 mL x3). The organic phases were combined and washed with saturated brine (200 mL). The resultant organic phase was dried over anhydrous sodium sulfate and filtered, and the filtrate was concentrated. The crude product was purified by silica gel column chromatography (methanol: dichloromethane = 1:19) to obtain Compound **68-9. MS m/z (ESI):** 433.2 [M+H]+. **$^1$H NMR** (400 MHz, DMSO-*$d_6$*) δ 11.75 (s, 1H), 8.14 - 8.07 (m, 1H), 8.05 (d, *J* = 5.6 Hz, 1H), 7.41 (dd, *J* = 2.0, 8.4 Hz, 1H), 7.35 - 7.31 (m, 1H), 6.74 (d, *J* = 8.4 Hz, 1H), 6.63 (s, 1H), 6.10 (d, *J* = 5.6 Hz, 1H), 5.93 (t, *J* = 6.0 Hz, 1H), 4.23 (d, *J* = 6.0 Hz, 2H), 3.95 - 3.85 (m, 2H), 3.83 (s, 3H), 2.74 (d, *J* = 4.4 Hz, 3H).

Step 9: Synthesis of Compound **68-10**

Under nitrogen protection, N-phenylbis(trifluoromethanesulfonimide) (265 mg, 0.74 mmol) was added to a solution of Compound **68-9** (107 mg, 0.22 mmol) and triethylamine (0.27 mL, 0.20 mmol) in tetrahydrofuran (10 mL). Under nitrogen protection, the mixture was stirred at 25 °C for 4 hours. The reaction solution was poured into water (50 mL) and extracted with ethyl acetate (50 mL x3). The organic phases were combined and washed with saturated brine (100 mL).

The resultant organic phase was dried over anhydrous sodium sulfate and filtered, and the filtrate was concentrated. The crude product was purified by silica gel column chromatography (ethyl acetate: petroleum ether = 1: 1) to obtain Compound **68-10. MS m/z (ESI):** 565.2 $[M+H]^+$.

Step 10: Synthesis of Compound **68**

Under nitrogen protection, $Pd_2(dba)_3$ (6.0 mg, 0.01 mmol) and BINAP (8.0 mg, 0.01 mmol) were added to a solution of Compound **68-10** (35 mg, 0.06 mmol), Compound 67 (21 mg, 0.09 mmol), and cesium carbonate (40 mg, 0.12 mmol) in dioxane (2.0 mL). Under nitrogen protection, the mixture was stirred at 100 °C for 3 hours. The reaction solution was directly concentrated, and the crude product was purified by silica gel column chromatography (methanol: dichloromethane = 1: 19) to obtain Compound **68. MS m/z (ESI):** 629.4 $[M+H]^+$. **$^1$H NMR** (400 MHz, DMSO-$d_6$) δ 8.10 (q, *J* = 4.8 Hz, 1H), 7.86 (d, *J* = 5.6 Hz, 1H), 7.41 (dd, *J* = 2.0, 8.0 Hz, 1H), 7.33 (d, *J* = 2.0 Hz, 1H), 7.01 (s, 1H), 6.86 (d, *J* = 8.0 Hz, 1H), 6.74 (d, *J* = 8.4 Hz, 1H), 5.92 (t, *J* = 6.0 Hz, 1H), 5.86 (d, *J* = 5.6 Hz, 1H), 4.90 (d, *J* = 50.4 Hz, 1H), 4.58 (s, 4H), 4.22 (d, *J* = 6.0 Hz, 2H), 3.88 (q, *J* = 11.2 Hz, 2H), 3.83 (s, 3H), 3.75 - 3.62 (m, 1H), 3.25 - 3.19 (m, 4H), 2.75 (d, *J* = 4.4 Hz, 3H), 2.21 - 2.13 (m, 1H), 2.05 - 1.97 (m, 1H), 1.74 - 1.69 (m, 2H), 1.56 - 1.50 (m, 1H), 1.47 - 1.42 (m, 1H), 1.10 - 1.04 (m, 1H).

Compound **68** was subjected to SFC resolution to obtain Compounds **68c** and **68d** that were isomers between each other. Compounds **68c** and **68d** were selected from Compounds **68a** and **68b,** respectively, and they were different from each other.

Compound **68c:** SFC method: Instrument: WATERS 150 preparative SFC(SFC-26); Column: ChiralPak AD, 250×30mm I.D., 10μm; Mobile phase: A for $CO_2$ and B for Ethanol(0.1%$NH_3H_2O$); Gradient: B 35 %; Flow rate:150 mL /min; Back pressure: 100 bar; Column temperature: 38°C; Wavelength: 220nm; Cycle time: ~9 min. Retention time: 1.82 min. **MS m/z (ESI):** 629.3 $[M+H]^+$. **$^1$H NMR** (400 MHz, DMSO-$d_6$) δ 8.11 (q, *J* = 4.8 Hz, 1H), 7.86 (d, *J = 5.6* Hz, 1H), 7.40 (dd, *J = 2.0,* 8.4 Hz, 1H), 7.33 (d, *J= 2.0* Hz, 1H), 7.00 (s, 1H), 6.87 (d, *J* = 8.0 Hz, 1H), 6.73 (d, *J* = 8.4 Hz, 1H), 5.93 (t, *J* = 6.0 Hz, 1H), 5.85 (d, *J* = 5.6 Hz, 1H), 4.90 (d, *J* = 50.4 Hz, 1H), 4.58 (s, 4H), 4.22 (d, *J* = 6.0 Hz, 2H), 3.87 (q, *J* = 11.2 Hz, 2H), 3.83 (s, 3H), 3.77 - 3.75 (m, 1H), 3.25 - 3.19 (m, 4H), 2.74 (d, *J* = 4.4 Hz, 3H), 2.24 - 2.13 (m, 1H), 2.06 - 1.95 (m, 1H), 1.81 - 1.66 (m, 3H), 1.43 - 1.25 (m, 1H), 1.12 - 1.02 (m, 1H).

Compound **68d:** SFC method: Instrument: WATERS 150 preparative SFC(SFC-26); Column: ChiralPak AD, 250×30mm I.D., 10μm; Mobile phase: A for $CO_2$ and B for Ethanol(0.1%$NH_3H_2O$); Gradient: B 35 %; Flow rate:150 mL /min; Back pressure: 100 bar; Column temperature: 38°C; Wavelength: 220nm; Cycle time: ~9 min. Retention time: 2.14 min. **MS m/z (ESI):** 629.3 $[M+H]^+$. **$^1$H NMR** (400 MHz, DMSO-$d_6$) δ 8.11 (q, *J* = 4.8 Hz, 1H), 7.86 (d, *J = 5.6* Hz, 1H), 7.40 (dd, *J= 2.0,* 8.4 Hz, 1H), 7.33 (d, *J= 2.0* Hz, 1H), 7.00 (s, 1H), 6.87 (d, *J* = 8.0 Hz, 1H), 6.73 (d, *J* = 8.4 Hz, 1H), 5.93 (t, *J* = 6.0 Hz, 1H), 5.85 (d, *J* = 5.6 Hz, 1H), 4.87 (d, *J* = 50.4 Hz, 1H), 4.58 (s, 4H), 4.22 (d, *J* = 6.0 Hz, 2H), 3.87 (q, *J* = 11.2 Hz, 2H), 3.83 (s, 3H), 3.74 - 3.69 (m, 1H), 3.25 - 3.20 (m, 4H), 2.74 (d, *J* = 4.4 Hz, 3H), 2.22 - 2.13 (m, 1H), 2.06 - 1.96 (m, 1H), 1.80 - 1.66 (m,3H), 1.44 - 1.22 (m, 1H), 1.13 - 1.00 (m, 1H).

Preparation Example 69: Preparation of Compound **69**

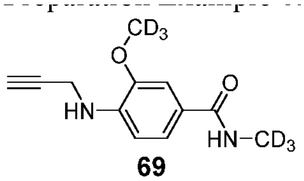

**69**

Synthesis scheme:

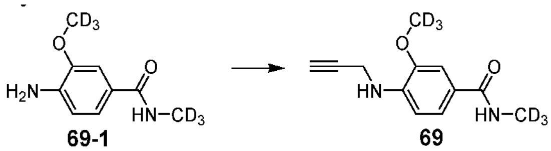

**69-1** **69**

For the synthesis of Compound 69-1, Preparation Example 27-3 could be referred to. Compound **69-1: MS m/z (ESI):** 187.1 $[M+H]^+$.

Step 1: Synthesis of Compound **69**

To a solution of Compound **69-1** (14.0 g, 50.37 mmol) in N,N-dimethylformamide (160 mL), 3-bromopropyne (4.56 mL, 52.88 mmol) and potassium carbonate (20.88 g, 151.1 mmol) were added, and the reaction solution was stirred at 80 °C for 18 hours. After the reaction solution was cooled to room temperature, it was diluted with water (500 mL) and extracted with ethyl acetate (150 mL x2). The organic layers were combined and washed with saturated brine (100 mL), dried over anhydrous sodium sulfate, filtered, and the filtrate was concentrated. The crude product was purified by silica gel column chromatography (ethyl acetate/petroleum ether = 1/9) to obtain Compound **69. MS m/z (ESI):** 225.0 $[M+H]^+$. **$^1$H NMR** (400 MHz, DMSO-$d_6$) δ 8.08 (s, 1H), 7.39 (dd, *J* = 2.0, 8.4 Hz, 1H), 7.31 (d, *J* = 2.0 Hz, 1H), 6.63 (d, *J* = 8.4 Hz, 1H), 5.81 (t, *J* = 6.4 Hz, 1H), 3.96 (dd, *J* = 2.4, 6.4 Hz, 2H), 3.05 (t, *J* = 2.4 Hz, 1H).

Preparation Example 70: Preparation of Compound 70d

Synthesis scheme:

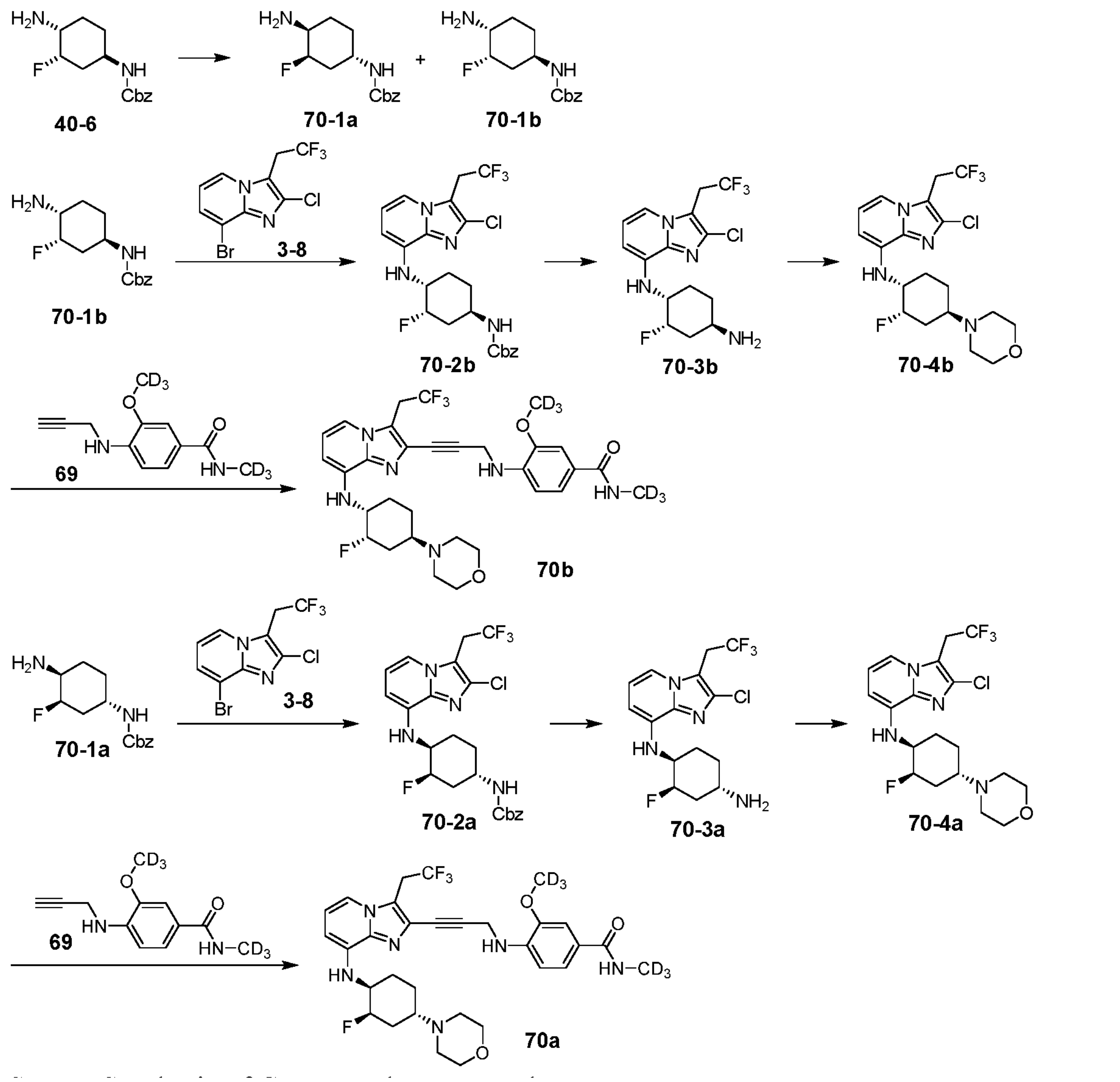


Step 1: Synthesis of Compounds **70-1a** and **70-1b**

Compound **40-6** was subjected to SFC resolution to obtain Compounds **70-1c** and **70-1d** that were isomers between each other. Compounds **70-1c** and **70-1d** were selected from Compounds **70-1a** and **70-1b,** respectively, and

they were different from each other.

Compound 70-1c: SFC method: Instrument: Shimadzu LC-A HPLC, Column: Chiralpak IC, 250×30mm, 10 μm, Mobile phase: A for Heptane and B for Ethanol (0.2%$NH_3H_2O$), Isocratic: B: 20%, Flow rate: 100 mL /min, Column temperature: R.T. Wavelength: 220 nm, 254 nm, Injection: 10 ml, Cycle time: 9 min. Retention time: 1.932 min. **MS m/z (ESI):** 267.2 $[M+H]^+$. **$^1$H NMR** (400 MHz, DMSO-$d_6$) δ 7.41 - 7.27 (m, 5H), 7.23 (d, *J* = 8.0 Hz, 1H), 5.00 (s, 2H), 4.78 - 4.58 (m, 1H), 3.59 - 3.45 (m, 2H), 2.65 - 2.52 (m, 1H), 2.14 - 2.02 (m, 1H), 1.80 - 1.73 (m, 1H), 1.65 - 1.57 (m, 1H), 1.55 - 1.47 (m, 1H), 1.44 - 1.32 (m, 2H), 1.30 - 1.18 (m, 1H).

Compound **70-1d:** SFC method: Instrument: Shimadzu LC-A HPLC, Column: Chiralpak IC, 250×30mm, 10 μm, Mobile phase: A for Heptane and B for Ethanol (0.2%$NH_3H_2O$), Isocratic: B: 20%, Flow rate: 100 mL /min, Column temperature: R.T. Wavelength: 220 nm, 254 nm, Injection: 10 ml, Cycle time: 9 min. Retention time: 2.559 min. **MS m/z (ESI):** 267.2 $[M+H]^+$. **$^1$H NMR** (400 MHz, DMSO-$d_6$) δ 7.41 - 7.27 (m, 5H), 7.23 (d, *J* = 8.0 Hz, 1H), 5.00 (s, 2H), 4.78 - 4.58 (m, 1H), 3.59 - 3.45 (m, 2H), 2.65 - 2.52 (m, 1H), 2.14 - 2.02 (m, 1H), 1.80 - 1.73 (m, 1H), 1.65 - 1.57 (m, 1H), 1.55 - 1.47 (m, 1H), 1.44 - 1.32 (m, 2H), 1.30 - 1.18 (m, 1H).

Step 2: Synthesis of Compound **70-2d**

Under nitrogen protection, tri(dibenzylideneacetone)dipalladium (0.66 g, 0.72 mmol) and 1,1'-binaphthyl-2,2'-bisdiphenylphosphine (0.45 g, 0.72 mmol) were added to a mixture of Compound **3-8** (1.5 g, 4.78 mmol), Compound **70-1d** (1.2 g, 4.51 mmol) and cesium carbonate (3.90 g, 11.96 mmol) in dioxane (40 mL). Under nitrogen protection, the mixture was stirred at 100 °C for 18 hours. After the reaction solution was cooled to room temperature, it was filtered, and the filter cake was washed with acetonitrile (20 mL x3), and the filtrate was concentrated. The crude product was purified by silica gel column chromatography (ethyl acetate: petroleum ether = 1:1) to obtain Compound **70-2d. MS m/z (ESI):** 499.2 $[M+H]^+$. **$^1$H NMR** (400 MHz, $CDCl_3$) δ 7.40 - 7.29 (m, 6H), 6.77 (t, *J* = 7.6 Hz, 1H), 6.15 (d, *J* = 7.6 Hz, 1H), 5.35 (d, *J* = 9.2 Hz, 1H), 5.10 (s, 2H), 4.99 (d, *J* = 49.2 Hz, 1H), 4.62 (s, 1H), 3.99 - 3.85 (m, 1H), 3.70 (q, *J* = 10.0 Hz, 2H), 3.58 - 3.38 (m, 1H), 2.58 - 2.45 (m, 1H), 2.22 - 2.12 (m, 1H), 2.08 - 1.99 (m, 1H), 1.90 - 1.76 (m, 1H), 1.55 -1.31 (m, 2H).

Step 3: Synthesis of Compound **70-3d**

Compound **70-2d** (1.47 g, 2.65 mmol) was dissolved in trifluoroacetic acid (20 mL). The mixture was stirred at 50 °C for 2 hours under nitrogen protection. The reaction solution was concentrated and diluted with ethyl acetate (200 mL), washed with saturated sodium bicarbonate aqueous solution (200 mL), and extracted with ethyl acetate (200 mL x2). The organic phases were combined and washed with saturated brine (500 mL). The resultant organic phase was dried over anhydrous sodium sulfate and filtered, and the filtrate was concentrated under reduced pressure. The crude product was purified by silica gel column chromatography (methanol: dichloromethane = 3:17) to obtain Compound **70-3d. MS m/z (ESI):** 365.0 $[M+H]^+$. **$^1$H NMR** (400 MHz, DMSO-$d_6$) δ 7.88 (d, *J* = 6.8 Hz, 1H), 6.90 (t, *J* = 7.2 Hz, 1H), 6.51 (d, *J* = 7.6 Hz, 1H), 5.41 (d, *J* = 9.2 Hz, 1H), 5.03 (d, *J* = 49.6 Hz, 1H), 4.13 (q, *J* = 10.8 Hz, 2H), 3.82 - 3.69 (m, 1H), 3.17 - 3.06 (m, 1H), 2.35 - 2.21 (m, 1H), 1.98 - 1.85 (m, 2H), 1.81 - 1.63 (m, 2H), 1.53 - 1.40 (m, 1H).

Step 4: Synthesis of Compound **70-4d**

Under nitrogen protection, 2,2'-dibromodiethyl ether (1.69 g, 7.29 mmol) was added to a mixed solution of Compound **70-3d** (663 mg, 1.64 mmol) and sodium carbonate (772 mg, 7.28 mmol) in acetonitrile (14 mL). Under nitrogen protection, the mixture was stirred at 80 °C for 16 hours. The reaction solution was directly filtered, the filter cake was washed with acetonitrile (10 mL x3), and the filtrate was concentrated under reduced pressure. The crude product was purified by silica gel column chromatography (methanol: dichloromethane = 1:19) to obtain Compound **70-4d. $^1$H NMR** (400 MHz, DMSO-$d_6$) δ 7.87 (d, *J* = 6.8 Hz, 1H), 6.90 (t, *J*= 7.6 Hz, 1H), 6.45 (d, *J* = 7.6 Hz, 1H), 5.37 (d, *J* = 9.2 Hz, 1H), 5.02 (d, *J* = 50.4 Hz, 1H), 4.12 (q, *J* = 10.8 Hz, 2H), 3.80 - 3.65 (m, 1H), 3.60 - 3.54 (m, 4H), 3.51 - 3.44 (m, 1H), 2.62 - 2.53 (m, 1H), 2.49 - 2.53 (m, 3H), 2.21 - 2.11 (m, 1H), 1.95 - 1.81 (m, 2H), 1.80 - 1.58 (m, 2H), 1.51 - 1.38 (m, 1H).

Step 5: Synthesis of Compound **70d**

Under nitrogen protection, bis(acetonitrile)palladium dichloride (10 mg, 0.04 mmol) and 2-dicyclohexylphosphino-2',4',6'-triisopropylbiphenyl (30 mg, 0.06 mmol) were added to a mixed solution of Compound **70-4** (100 mg, 0.21 mmol), Compound **69** (100 mg, 0.40 mmol) and cesium carbonate (190 mg, 0.58 mmol) in acetonitrile (10 mL). Under nitrogen protection, the mixture was stirred at 90 °C for 2 hours. After the reaction solution was cooled to room temperature, it was filtered, the filter cake was washed with acetonitrile (20 mL x3), and the filtrate was concentrated under reduced pressure. The crude product was purified by reverse phase flash chromatography (Waters-Xbridge-C18-10μm-19*250mm; mobile phase ACN in water (10 mmol/L NH4HCO3), 32% to 62% gradient in 8 min; 25 mL/min,

detected, UV 220nm) to obtain Compound **70d. MS m/z (ESI):** 623.0 $[M+H]^+$. **$^1$H NMR** (400 MHz, DMSO-$d_6$) δ *8.07* (s, 1H), 7.77 (d, *J* = 6.8 Hz, 1H), 7.41 (dd, *J* = 2.0, 8.4 Hz, 1H), 7.34 (d, *J* = 2.0 Hz, 1H), 6.83 (t, *J* = 7.2 Hz, 1H), 6.74 (d, *J* = 8.4 Hz, 1H), 6.35 (d, *J* = 7.6 Hz, 1H), 5.94 (t, *J* = 6.4 Hz, 1H), 5.38 (d, *J* = 9.2 Hz, 1H), 5.00 (d, *J* = 50.8 Hz, 1H), 4.26 (d, *J* = 6.4 Hz, 2H), 4.01 (q, *J* = 10.8 Hz, 2H), 3.77 - 3.62 (m, 1H), 3.56 (t, *J* = 4.8 Hz, 4H), 2.61 - 2.53 (m, 1H), 2.47 (t, *J* = 4.8 Hz, 4H), 2.19 - 2.09 (m, 1H), 1.92 - 1.81 (m, 2H), 1.79 - 1.57 (m, 2H), 1.49 - 1.35 (m, 1H).

It should be noted that:

As shown in the reaction scheme,
when **70-1a** was used as the starting material (i.e., **70-1d** was **70-1a), 70-2d** was **70-2a, 70-3d** was **70-3a, 70-4d** was **70-4a,** and **70d** was **70a;**
when **70-1b** was used as the starting material (i.e. **70-1d** was **70-1b), 70-2d** was **70-2b, 70-3d** was **70-3b, 70-4d** was **70-4b,** and **70d** was **70b.**

Preparation Example 71: Preparation of Compound **71d**

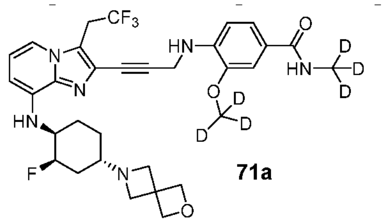

**71a**

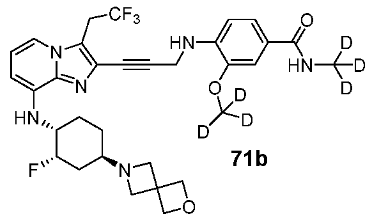

**71b**

Compound **71d** was synthesized using **70-1d** as the starting material. For the synthesis of Compound **71d,** Preparation Example 70d could be referred to.

It should be noted that:

when **70-1a** was used as the starting material (i.e., **70-1d** was **70-1a), 71d** was **71a;**
when **70-1b** was used as the starting material (i.e. **70-1d** was **70-1b), 71d** was **71b.**

Compound **71d: MS m/z (ESI):** 635.4 $[M+H]^+$. **$^1$H NMR** (400 MHz, DMSO-$d_6$) δ 8.08 (s, 1H), 7.75 (d, *J* = 6.8 Hz, 1H), 7.40 (dd, *J* = *2.0,* 8.4 Hz, 1H), 7.33 (d, *J*= *2.0* Hz, 1H), 6.81 (t, *J* = 7.2 Hz, 1H), 6.73 (d, *J* = 8.4 Hz, 1H), 6.35 (d, *J* = 7.6 Hz, 1H), 5.94 (t, *J* = 6.4 Hz, 1H), 5.34 (d, *J* = 9.2 Hz, 1H), 4.92 (d, *J* = 50.4 Hz, 1H), 4.57 (s, 4H), 4.25 (d, *J* = 6.4 Hz, 2H), 3.99 (q, *J* = 10.8 Hz, 2H), 3.86 - 3.75 (m, 1H), 3.26 - 3.20 (m, 4H), 2.26 - 2.13 (m, 1H), 2.09 - 1.96 (m, 1H), 1.84 - 1.77 (m, 1H), 1.76 - 1.68 (m, 1H), 1.60 - 1.47 (m, 1H), 1.41 - 1.22 (m, 1H), 1.13 - 1.01 (m, 1H).

Preparation Example 72: Preparation of Compound **72d**

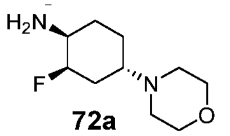

**72a**

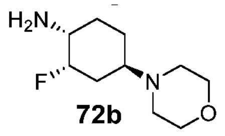

**72b**

Compound **72d** was synthesized using **70-1d** as the starting material, and for the synthesis of Compound **72d,** Preparation Example 67 could be referred to.

It should be noted that:

when **70-1a** was used as the starting material (i.e., **70-1d** was **70-1a), 72d** was **72a;**
When **70-1b** was used as the starting material (i.e., **70-1d** was **70-1b), 72d** was **72b.**

Compound **72: MS m/z (ESI):** 203.2 $[M+H]^+$. **$^1$H NMR** (400 MHz, $CDCl_3$) δ 4.72 (d, *J* = 50.0, 1H), 3.69 - 3.59 (m, 4H), 2.68 - 2.56 (m, 1H), 2.50 - 2.43 (m, 4H), 2.28 - 2.18 (m, 1H), 1.91 - 1.82 (m, 1H), 1.77 - 1.68 (m, 1H), 1.55 - 1.13 (m, 4H).

Preparation Example 73: Preparation of Compound **73d**

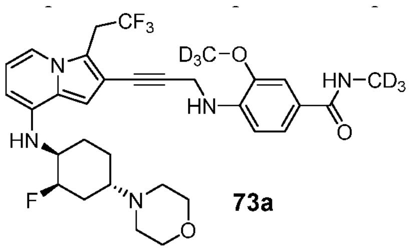

**73a**

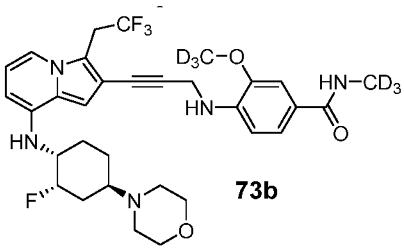

**73b**

Compound **73d** was synthesized using **72-d as** the starting material. For the synthesis of Compound **73d,** Preparation Example 65 could be referred to.

It should be noted that:

when **72a** was used as the starting material (i.e., **72d** was **72a), 73d** was **73a;**
when **72b** was used as the starting material (i.e., **72d** was **72b), 73d** was **73b.**

Compound **73d: MS m/z (ESI):** 622.4 $[M+H]^+$. **$^1$H NMR** (400 MHz, DMSO-$d_6$) δ 8.05 (s, 1H), 7.55 (d, *J* = 6.8 Hz, 1H), 7.40 (dd, *J = 2.0,* 8.4 Hz, 1H), 7.32 (d, *J* = 2.0 Hz, 1H), 6.88 (s, 1H), 6.73 (d, *J* = 8.4 Hz, 1H), 6.50 (t, *J = 7.2* Hz, 1H), 5.88 (t, *J* = 6.4 Hz, 1H), 5.82 (d, *J* = 7.6 Hz, 1H), 5.52 (d, *J* = 8.4 Hz, 1H), 4.99 (d, *J* = 50.4 Hz, 1H), 4.22 (d, *J* = 6.4 Hz, 2H), 3.91 (*q, J* = 10.8 Hz, 2H), 3.59 - 3.52 (m, 5H), 2.58 - 2.55 (m, 1H), 2.45 (s, 4H), 2.13 - 2.07 (m, 1H), 1.91 - 1.58 (m, 4H), 1.47 - 1.32 (m, 1H).

Preparation Example 74: Preparation of Compound **74d**

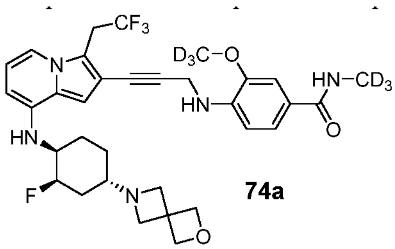

**74a**

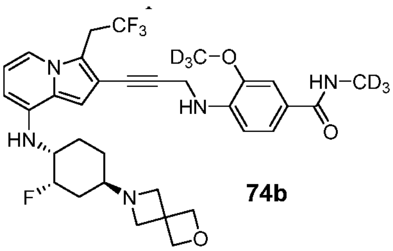

**74b**

Compound **74d** was synthesized using **86d** as the starting material. For the synthesis of Compound **74d,** Preparation Example 65 could be referred to.

It should be noted that:

when **86a** was used as the starting material (i.e., **86d** was **86a), 74d** was **74a;**
when **86b** was used as the starting material (i.e., **86d** was **86b), 74d** was **74b.**

Compound **74d: MS m/z (ESI):** 634.6 $[M+H]^+$. **$^1$H NMR** (400 MHz, DMSO-$d_6$) δ 8.06 (s, 1H), 7.55 (d, *J* = 6.8 Hz, 1H), 7.41 (dd, *J* = 2.0, 8.4 Hz, 1H), 7.33 (d, *J* = 2.0 Hz, 1H), 6.89 (s, 1H), 6.74 (d, *J* = 8.4 Hz, 1H), 6.50 (t, *J = 7.2* Hz, 1H), 5.89 (t, *J* = 6.4 Hz, 1H), 5.82 (d, *J* = 7.6 Hz, 1H), 5.50 (d, *J* = 8.4 Hz, 1H), 4.92 (d, *J* = 50.0 Hz, 1H), 4.58 (s, 4H), 4.22 (d, *J* = 6.4 Hz, 2H), 3.93 (q, *J* = 10.8 Hz, 2H), 3.66 - 3.46 (m, 1H), 3.27 - 3.18 (m, 4H), 2.20 - 2.15 (m, 1H), 2.02 - 1.97 (m, 1H), 1.76 - 1.69 (m, 3H), 1.39 - 1.22 (m, 1H), 1.17 - 0.98 (m, 1H).

Preparation Example 75: Preparation of Compound **75**

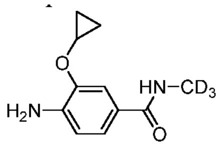

75

Synthesis scheme:

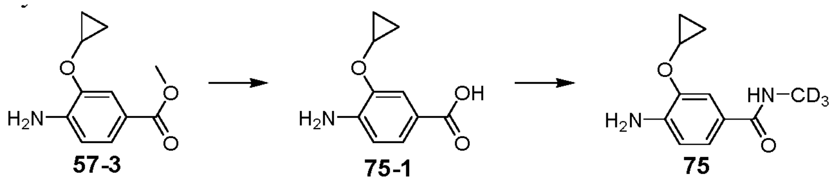

57-3 75-1 75

Step 1: Synthesis of Compound **75-1**

Lithium hydroxide (1.16 g, 48.3 mmol) was added to a solution of Compound **57-3** (2.0 g, 9.65 mmol) in methanol (20 mL) and water (6 mL). The reaction solution was stirred at 60 °C for 2 hours. The reaction solution was adjusted to pH 5 with 1M dilute hydrochloric acid and extracted with dichloromethane/methanol (50 mL x4, 10: 1). The organic layers were combined and dried over anhydrous sodium sulfate and filtered, and the filtrate was concentrated to obtain Compound **75-1,** which was used directly in the next step. **MS m/z (ESI):** 210.3 $[M+H]^+$.

Step 2: Synthesis of Compound **75**

To a solution of Compound **75-1** (1.7 g, 8.80 mmol) in acetonitrile (30 mL), deuterated methylamine hydrochloride (1.24 g, 17.60 mmol), tetramethylchlorouronium hexafluorophosphate (2.71 g, 9.68 mmol) and 1-methylimidazole (6.4 mL, 38.72 mmol) were added. The mixture was stirred at 25 °C for 18 hours. The reaction solution was added with water (100 mL) and extracted with ethyl acetate (100 mL x2). The organic layers were combined and washed with saturated brine (100 mL), dried over anhydrous sodium sulfate, filtered, and the filtrate was concentrated. The crude product was purified by silica gel column chromatography (petroleum ether: ethyl acetate = 5:1) to obtain Compound **75. MS m/z (ESI):** 210.0 $[M+H]^+$. **$^1$H NMR** (400 MHz, DMSO-$d_6$) δ 7.97 (s, 1H), 7.56 (d, *J* = 2.0 Hz, 1H), 7.25 (dd, *J* = 2.0, 8.4 Hz, 1H), 6.58 (d, *J* = 8.4 Hz, 1H), 5.12 (s, 2H), 3.87 - 3.81 (m, 1H), 0.87 - 0.71 (m, 2H), 0.71 - 0.55 (m, 2H).

Preparation Example 76: Preparation of Compound **76d**

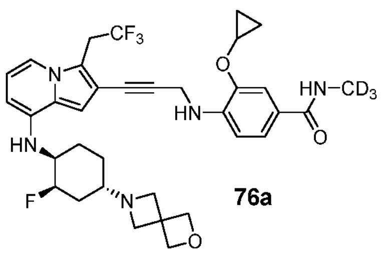

76a

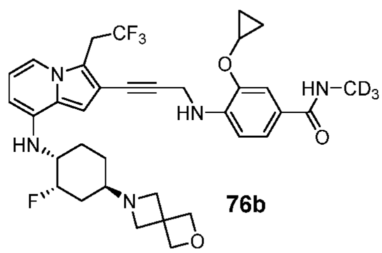

76b

Compound **76d was** synthesized using **86d** as the starting material. For the synthesis of Compound **76d,** Preparation Example 65 could be referred to.

It should be noted that:

when **86a** was used as the starting material (i.e., **86d** was **86a), 76d** was **76a;**
when **86b** was used as the starting material (i.e. **86d** was **86b), 76d** was **76b.**

Compound **76d: MS m/z (ESI):** 657.4 $[M+H]^+$. **$^1$H NMR** (400 MHz, DMSO-$d_6$) δ 8.04 (s, 1H), 7.61 (d, *J* = 2.0 Hz,

1H), 7.55 (d, *J* = 6.8 Hz, 1H), 7.41 (dd, *J* = 2.0, 8.4 Hz, 1H), 6.88 (s, 1H), 6.72 (d, *J* = 8.4 Hz, 1H), 6.49 (t, *J* = 7.2 Hz, 1H), 5.81 (d, *J* = 7.6 Hz, 1H), 5.76 (t, *J* = 6.4 Hz, 1H), 5.50 (d, *J* = 8.4 Hz, 1H), 4.91 (d, *J* = 50.0 Hz, 1H), 4.58 (s, 4H), 4.20 (d, *J* = 6.4 Hz, 2H), 3.99 - 3.80 (m, 3H), 3.60 - 3.46 (m, 1H), 3.25 - 3.18 (m, 4H), 2.20 - 2.14 (m, 1H), 2.03 - 1.97 (m, 1H), 1.75 - 1.67 (m, 3H), 1.36 - 1.25 (m, 1H), 1.09 - 1.03 (m, 1H), 0.85 - 0.77 (m, 2H), 0.71 - 0.67 (m, 2H).

Preparation Example 77: Preparation of Compound **77**

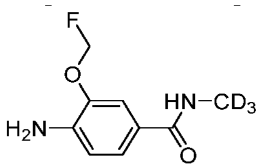

**77**

For the synthesis of Compound 77, Preparation Example 75 could be referred to.

Compound **77: MS m/z (ESI):** 202.0 [M+H]+. **$^1$H NMR** (400 MHz, DMSO-*$d_6$*) δ 8.02 (s, 1H), 7.48 (d, *J* = 2.0 Hz, 1H), 7.38 (dd, *J* = 2.0, 8.4 Hz, 1H), 6.67 (d, *J* = 8.4 Hz, 1H), 5.77 (d, *J* = 54.8 Hz, 2H), 5.44 (s, 2H). Preparation Example 78: Preparation of Compound **78d**

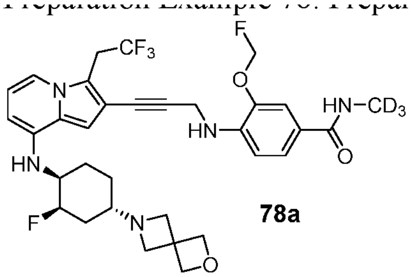

**78a**

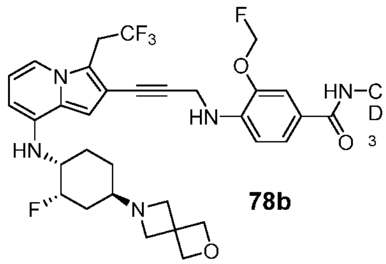

**78b**

Compound **78d** was synthesized using **86d** as the starting material. For the synthesis of Compound **78d,** Preparation Example 65 could be referred to.

It should be noted that:

when **86a** was used as the starting material (i.e., **86d** was **86a), 78d** was **78a;**
when **86b** was used as the starting material (i.e., **86d** was **86b), 78d was 78b.**

Compound **78d: MS m/z (ESI):** 649.2 [M+H]+. **$^1$H NMR** (400 MHz, DMSO-*$d_6$*) δ 8.11 (s, 1H), 7.55 (m, 3H), 6.98 - 6.73 (m, 2H), 6.50 (t, *J* = 7.2 Hz, 1H), 6.18 (t, *J* = 6.4 Hz, 1H), 5.95 - 5.70 (m, 3H), 5.50 (d, *J* = 8.4 Hz, 1H), 4.92 (d, *J* = 50.0 Hz, 1H), 4.58 (s, 4H), 4.24 (d, *J* = 6.4 Hz, 2H), 3.90 (q, *J* = 10.8 Hz, 2H), 3.60 - 3.56 (m, 1H), 3.24 - 3.20 (m, 4H), 2.21 - 2.15 (m, 1H), 2.05 - 1.95 (m, 1H), 1.75 - 1.70 (m, 3H), 1.39 - 1.25 (m, 1H), 1.16 - 0.98 (m, 1H).

Preparation Example 79: Preparation of Compound **79**

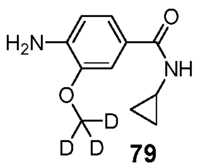

**79**

For the synthesis of Compound **79,** Preparation Example 27-3 could be referred to.

Compound **79: MS m/z (ESI):** 210.0 [M+H]+. **$^1$H NMR** (400 MHz, $CDCl_3$) δ 7.27 (d, *J* = 2.0 Hz, 1H), 6.99 (dd, *J* = 2.0, 8.0 Hz, 1H), 6.56 (d, *J* = 8.0 Hz, 1H), 2.86 - 2.77 (m, 1H), 0.84 - 0.72 (m, 2H), 0.57 - 0.48 (m, 2H).

Preparation Example 80: Preparation of Compound **80d**

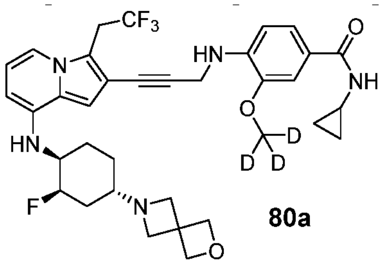

**80a**

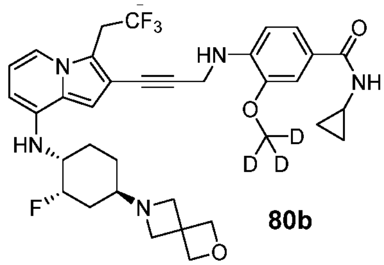

**80b**

Compound **80d** was synthesized using **86d** as the starting material, and for the synthesis of Compound **80d,** Preparation Example 65 could be referred to.

It should be noted that:

when **86a** was used as the starting material (i.e., **86d** was **86a), 80d** was **80a;**
when 86b was used as the starting material (i.e., **86d** was **86b), 80d** was **80b.**

Compound **80d: MS m/z (ESI):** 657.3 $[M+H]^+$. **$^1$H NMR** (400 MHz, $CD_3OD$) δ 7.49 - 7.42 (m, 2H), 7.36 (d, *J = 2.0* Hz, 1H), 6.84 (d, *J* = 8.4 Hz, 1H), 6.60 (s, 1H), 6.53 (t, *J* = 7.2 Hz, 1H), 5.88 *(d, J*= 7.6 Hz, 1H), 5.03 (d, *J* = 50.0 Hz, 1H), 4.76 (s, 4H), 4.28 (s, 2H), 3.79 (q, *J* = 10.4 Hz, 2H), 3.65 - 3.59 (m, 1H), 3.51 (s, 4H), 2.85 - 2.82 (m, 1H), 2.59 - 2.42 (m, 1H), 1.98 - 1.87 (m, 2H), 1.50 - 1.20 (m, 4H), 0.83 - 0.78 (m, 2H), 0.68 - 0.63 (m, 2H).

Preparation Example 81: Preparation of Compound **81**

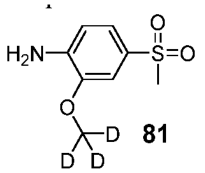

**81**

For the synthesis of Compound **81,** Preparation Example 3-4 could be referred to.

Compound **81: MS m/z (ESI):** 205.0 $[M+H]^+$. **$^1$H NMR** (400 MHz, $CDCl_3$) δ 7.31 (dd, *J* = 2.0, 8.4 Hz, 1H), 7.19 (s, 1H), 6.67 (d, *J* = 8.4 Hz, 1H), 2.95 (s, 3H).

Preparation Example 82: Preparation of Compound **82d**

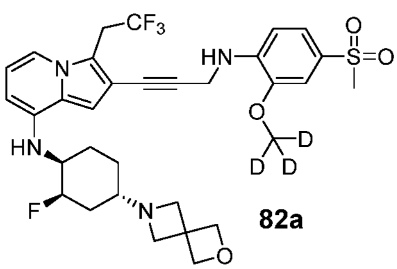

**82a**

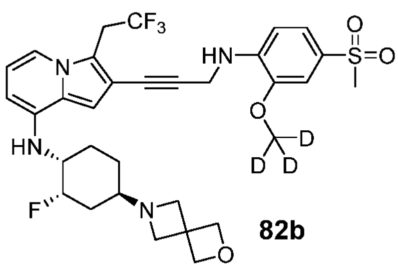

**82b**

Compound **82d** was synthesized using **86d** as the starting material, and for the synthesis of Compound **82d,** Preparation Example 65 could be referred to.

It should be noted that:

when **86a** was used as the starting material (i.e., **86d** was **86a), 82d** was **82a;**
when **86b** was used as the starting material (i.e. **86d** was **86b), 82d** was **82b.**

Compound **82d: MS m/z (ESI):** 652.3 $[M+H]^+$. **$^1$H NMR** (400 MHz, $CD_3OD$) δ 7.53 - 7.43 (m, 2H), 7.30 (d, *J* = 2.0 Hz, 1H), 6.96 (d, *J* = 8.4 Hz, 1H), 6.62 (s, 1H), 6.53 (t, *J* = 7.2 Hz, 1H), 5.88 (d, *J* = 7.2 Hz, 1H), 5.09 - 4.96 (m, 1H), 4.77 (s, 4H), 4.33 (s, 2H), 3.80 (q, *J* = 10.4 Hz, 2H), 3.61 - 3.52 (m, 5H), 3.08 (s, 3H), 2.72 - 2.67 (m, 1H), 2.32 - 1.34 (m, 6H).

Preparation Example 83: Preparation of Compound **83d**

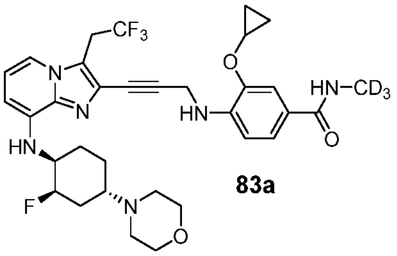
**83a**

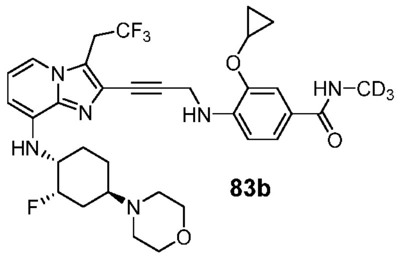
**83b**

Compound **83d** was synthesized using **72d** as the starting material. For the synthesis of Compound **83d,** Preparation Example 70 could be referred to.
It should be noted that:

when **72d** was used as the starting material (i.e., **72d** was **72a), 83d** was **83a;**
when **72d** was used as the starting material (i.e., **72d** was **72b), 83d** was **83b.**

Compound **83d: MS m/z (ESI):** 646.4 $[M+H]^+$. **$^1$H NMR** (400 MHz, DMSO-$d_6$) δ 8.06 (s, 1H), 7.77 (d, *J* = 6.8 Hz, 1H), 7.62 (d, *J* = 2.0 Hz, 1H), 7.41 (dd, *J* = 2.0, 8.4 Hz, 1H), 6.83 (t, *J* = 7.2 Hz, 1H), 6.72 (d, *J* = 8.4 Hz, 1H), 6.35 (d, *J* = 7.6 Hz, 1H), 5.82 (t, *J* = 6.4 Hz, 1H), 5.37 (d, *J* = 9.2 Hz, 1H), 5.00 (d, *J* = 50.8 Hz, 1H), 4.24 (d, *J* = 6.4 Hz, 2H), 4.01 (q, *J* = 10.8 Hz, 2H), 3.92 - 3.85 (m, 1H), 3.74 - 3.65 (m, 1H), 3.60 - 3.52 (m, 4H), 2.61 - 2.54 (m, 1H), 2.51 - 2.44 (m, 4H), 2.20 - 2.09 (m, 1H), 1.93 - 1.81 (m, 2H), 1.79 - 1.56 (m, 2H), 1.49 - 1.35 (m, 1H), 0.83 - 0.77 (m, 2H), 0.73 - 0.67 (m, 2H).

Preparation Example 84: Preparation of Compound **84d**

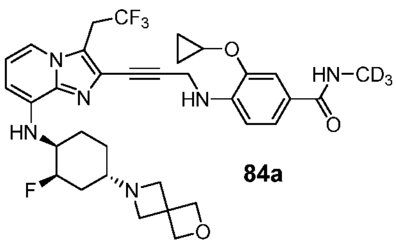
**84a**

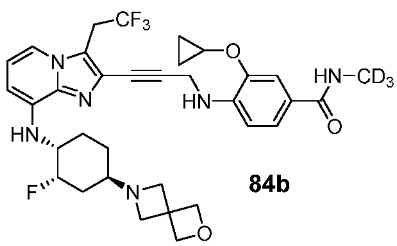
**84b**

Compound **82d** was synthesized using **86d** as the starting material, and for the synthesis of Compound **84d,** Preparation Example 70 could be referred to.
It should be noted that:

when **86a** was used as the starting material (i.e., **86d** was **86a), 84d** was **84a;**
when **86b** was used as the starting material (i.e., **86d** was **86b), 84d** was **84b.**

Compound **84d: MS m/z (ESI):** 658.0 $[M+H]^+$. **$^1$H NMR** (400 MHz, DMSO-$d_6$) δ 8.06 (s, 1H), 7.76 (d, *J* = 6.4 Hz, 1H), 7.62 (d, *J* = 2.0 Hz, 1H), 7.41 (d, *J* = 8.4 Hz, 1H), 6.81 (t, *J* = 7.2 Hz, 1H), 6.72 (d, *J* = 8.4 Hz, 1H), 6.35 (d, *J* = 7.6 Hz, 1H), 5.81 (d, *J* = 6.4 Hz, 1H), 5.35 (d, *J* = 9.2 Hz, 1H), 4.99 - 4.84 (m, 1H), 4.58 (s, 4H), 4.24 (d, *J* = 6.4 Hz, 2H), 4.01 (q, *J* = 10.4 Hz, 2H), 3.91 - 3.86 (m, 1H), 3.72 - 3.63 (m, 1H), 3.24 - 3.21 (m, 4H), 2.23 - 2.14 (m, 1H), 2.08 - 1.97 (m, 1H), 1.83 - 1.68 (m, 2H),

1.58 - 1.50 (m, 1H), 1.36 - 1.18 (m, 1H), 1.14 - 0.98 (m, 1H), 0.82 - 0.78 (m, 2H), 0.72 - 0.68 (m, 2H).

Preparation Example 85: Preparation of Compound **85**

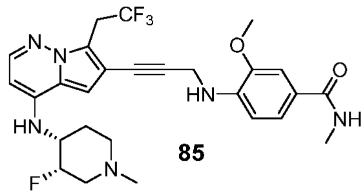

**85**

For the synthesis of Compound **85,** Preparation Example 68 could be referred to.

Compound **85: MS m/z (ESI):** 547.0 $[M+H]^+$. $^1$**H NMR** (400 MHz, DMSO-*$d_6$*) δ 8.10 (q, *J* = 4.8 Hz, 1H), 7.87 (d, *J* = 5.6 Hz, 1H), 7.47.33 (d, *J* = 2.0 Hz, 1H), 7.09 (d, *J* = 2.0 Hz, 1H), 6.70 (d, *J* = 8.0 Hz, 1H), 5.91 - 5.86 (m, 2H), 4.84 (d, *J* = 49.6 Hz, 1H), 4.23 (d, *J* = 6.4 Hz, 2H), 3.91 - 3.83 (m, 2H), 3.83 (s, 3H), 3.67 - 3.62 (m, 1H), 3.08 - 2.91 (m, 1H), 2.89 - 2.94 (m, 1H), 3.80 - 2.81 (m, 1H), 2.75 (d, *J* = 4.4 Hz, 3H), 2.31 - 2.21 (m, 1H), 2.18 (s, 3H), 2.12 - 1.97 (m, 2H), 1.70 - 1.62 (m, 1H).

Preparation Example 86: Preparation of Compound **86d**

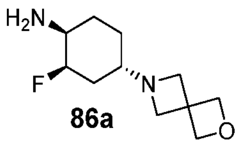

**86a**

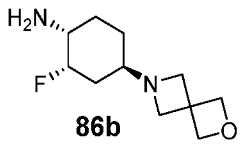

**86b**

Compound **86d** was synthesized using **70-1d** as the starting material. For the synthesis of Compound **86d,** Preparation Example 67 could be referred to.

It should be noted that:

when **70-1a** was used as the starting material (i.e., **70-1d** was **70-1a), 86d** was **86a;**
when **70-1b** was used as the starting material (i.e., **70-1d** was **70-1b), 86d** was **86b.**

Compound **86d: MS m/z (ESI):** 203.2 $[M+H]^+$. $^1$**H NMR** (400 MHz, DMSO-*$d_6$*) δ 4.70 - 4.55 (m, 5H), 3.21 - 3.16 (m, 4H), 2.65 - 2.54 (m, 1H), 2.13 - 2.06 (m, 1H), 1.98 - 1.90 (m, 1H), 1.65 - 1.43 (m, 3H), 1.33 - 1.06 (m, 3H), 0.97 - 0.87 (m, 1H).

Experimental Example 1: DNA binding test

In order to detect the ability of the aforementioned compounds to increase the binding of P53Y220C protein to DNA, the binding activity of p53 Y220C was measured by homogeneous time-resolved fluorescence (HTRF). Recombinant His-tagged p53 Y220C for HTRF measurement was expressed in bacteria *E. coli.* The recombinant protein was a truncated mutant containing only amino acids 94-312 of p53, and its sequence was SSSVPSQKTYQGSYGFRLGFLH SGTAKSVTCTYSPALNKMFCQLAKTCPVQLWVDSTPP PGTRVRAMAIYKQSQHMTEVVRRCPHHERCSDSDGLAPP QHLIRVEGNLRVEYLDDRN TFR$^h$SVVVPCEPPEVGSDCTTIHYNYMCNSSCMGGMNRRPILTIITLEDSSGNLLGRNS FE VHVCACPGRDRRTEEENLRKKGEPHHELPPGSTKRALSNNT. The sense strand of the DNA double strand was 5'-ATTAGGCATGTCTAGGCATGTCTAGG-3', with a biotin tag at the 5' end for activity assay, and the antisense strand was 5'-CCTAGACATGCCTAGACATGCCTAAT-3'.

40 mL of assay buffer (20 mM Tris HCL, pH 7.5, 50 mM NaCl, 2 mM $MgCl_2$, 0.02% Tween 20, 0.01% BSA, 1 mM DTT) was prepared. 10 mM compound was prepared and subjected to 3-fold dilution to obtain 10 dose gradient dilutions of the compound. 20 nL of the compound was added to a 384-well assay plate (PerkinElmer 6007299) using an Echo 655 Liquid Handler. P53Y220C protein was dissolved in assay buffer to reach a final concentration of 150 nM, and 5 μL thereof was added to each well, and incubated at room temperature for 5 min. DNA was dissolved in assay buffer to reach a final concentration of 150 nM, and 5 μL thereof was added to each well, and incubated at room temperature for 15 min. Anti 6HIS-XL665 (Cisbio 61HISXLB) was dissolved in assay buffer to reach a final concentration of 20 nM, and 5 μL thereof was added to each well. Streptavidin-Eu cryptate (Cisbio 610SAKLA) was dissolved in assay buffer to reach a final

concentration of 0.625 nM, and 5 μL thereof was added to each well, and incubated at 27°C for 1 hour. The plate was then read for signal values on an Envision (PerkinElmer 2105-0020) plate reader at 665 nm and 615 nm, respectively. GraphPad 9.0 was used to fit activation rate and compound concentration into a nonlinear regression curve (dose response - variable slope) to calculate $EC_{50}$, and $EC_{50}$ could be defined as the concentration required for the compound to achieve 50% effect. Activation rate = {(A-C)/(C)}*100%, A = (signal value at 665 nm/signal value at 615 nm); C = average signal of negative control (wherein, negative control referred to 0.1% DMSO).

A = 0 nM ≤ $EC_{50}$ < 100 nM; B = 100 nM ≤ $EC_{50}$ < 500 nM; C = 500 nM ≤ $EC_{50}$ < 1000 nM; D = 1000 nM ≤ $EC_{50}$.

The assay results were shown in Table 1.

Table 1. $EC_{50}$ of compounds in DNA binding experiment

| Compound | $EC_{50}$ (nM) | Compound | $EC_{50}$ (nM) |
|---|---|---|---|
| **1** | C | **40** | A |
| **2** | D | **41** | A |
| **3** | B | **42** | A |
| **4** | B | **44** | A |
| **5** | A | **44d** | A |
| **6** | B | **46** | A |
| **7** | B | **48c** | A |
| **8** | C | **50** | A |
| **9** | B | **51** | A |
| **10** | B | **52** | A |
| **11** | B | **53** | A |
| **12** | B | **54** | A |
| **13** | B | **57** | A |
| **14** | B | **58** | A |
| **15** | B | **64** | A |
| **16** | B | **65d** | A |
| **17** | C | **65d** | A |
| **18** | B | **70d** | A |
| **19** | B | **73d** | A |
| **20** | C | **74d** | A |
| **21** | B | **76d** | A |
| **22** | B | **78d** | A |
| **23** | B | **35-6e** | B |
| **24** | B | **35-6f** | A |
| **25** | B | **35-6g** | B |
| **27** | A | **35-6h** | A |
| **28** | C | **35-6i** | A |
| **29** | B | **35-6j** | A |
| **30** | B | **37e** | A |
| **31a** | C | **37f** | A |
| **32a** | B | **37g** | A |

(continued)

| Compound | $EC_{50}$ (nM) | Compound | $EC_{50}$ (nM) |
|---|---|---|---|
| **33** | B | **37h** | A |
| **34** | B | **39c** | A |
| | | **39d** | A |

Table 8 showed the multiple ratio of $EC_{50}$ of the compound to the reference compound.

The structure of the reference compound was as follows:

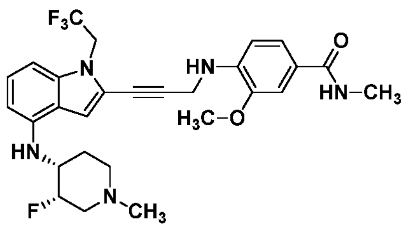

wherein, A: $EC_{50}$ of the compound ≤ $EC_{50}$ of the reference compound; B: $EC_{50}$ of the reference compound < $EC_{50}$ of the compound ≤ 3.0 * $EC_{50}$ of the reference compound; C: $EC_{50}$ of the compound ≥ 3 * $EC_{50}$ of the reference compound;

Table 8

| Compound No. | Compound $EC_{50}$ (nM)/Reference compound $EC_{50}$ (nM) | Compoun d No. | Compound $EC_{50}$ (nM)/Reference compound $EC_{50}$ (nM) | Compoun d No. | Compound $EC_{50}$ (nM)/Reference compound $EC_{50}$ (nM) |
|---|---|---|---|---|---|
| **1** | C | **47** | B | **26** | C |
| **2** | C | **48** | B | **27** | B |
| **3** | A | **40** | B | **28** | C |
| **4** | A | **41** | B | **29** | C |
| **5** | B | **42** | B | **30** | C |
| **6** | C | **43** | C | **31a** | C |
| 7 | C | **44** | B | **32a** | C |
| **8** | C | **49** | B | **33** | C |
| **9** | B | **50** | A | **34** | C |
| **10** | C | **51** | A | **35-6e** | C |
| **11** | B | **52** | A | **35-6f** | B |
| **12** | B | **57** | B | **35-6g** | C |
| **13** | B | **45** | B | **35-6h** | B |
| **14** | B | **68** | C | **35-6i** | B |
| **15** | C | **46** | A | **35-6j** | C |
| **16** | C | **48c** | A | **37e** | B |
| **17** | C | **48d** | B | **37f** | B |
| **18** | C | **53** | A | **37g** | A |
| **19** | B | **58** | B | **37h** | B |
| **20** | C | **65** | B | **39c** | B |
| **21** | C | **54** | B | **39d** | A |

(continued)

| Compound No. | Compound $EC_{50}$ (nM)/Reference compound $EC_{50}$ (nM) | Compoun d No. | Compound $EC_{50}$ (nM)/Reference compound $EC_{50}$ (nM) | Compoun d No. | Compound $EC_{50}$ (nM)/Reference compound $EC_{50}$ (nM) |
|---|---|---|---|---|---|
| **22** | B | **55** | B | **68d** | C |
| **23** | B | **65c** | A | **64** | B |
| **24** | C | **65d** | B | **56** | C |
| **25** | C | **68c** | B | **44c** | C |
| **73d** | B | **85** | C | **44d** | B |
| **76d** | A | **63** | B | **59** | B |
| **78d** | A | **74d** | A | **60** | B |
| **70d** | A | **62** | B | **61** | B |

The experimental results showed that the compounds of the present invention exhibit excellent in vitro activity and could increase the binding ability of the Y220C mutant P53 protein to DNA.

Experimental Example 2: NUGC-3 cell proliferation assay

In order to detect the inhibitory effect of the aforementioned compounds on the proliferation ability of NUGC-3 cells, 250 NUGC-3 cells (Nanjing Cobioer Biosciences Co., Ltd., CBP60492) were seeded into a 384-well plate (Corning 3765), 35 μL per well, and incubated overnight in a constant temperature incubator at 37 °C and 5% $CO_2$. Then, 40 nL of the compound was added to the 384-well plate using an Echo 655 Liquid Handler, with a maximum final concentration of 10 μM, 1:3 serial dilutions, and a total of 10 doses. The culture was performed at 37 °C and 5% $CO_2$ for 6 days. The plate was taken out from the incubator and equilibrated at room temperature for 10 minutes. 20 μL of CTG (Promage G7572) was added to each well of the plate according to the platemap. The plate was centrifuged at 1000 rpm for 1 min. The lysis of cells was induced by mixing on a shaker for 2 min. The plate was centrifuged at 1000 rpm for 1 min. Incubation was performed at room temperature for 10 min to stabilize luminescence signals. Luminescence values were recorded by Envision (PerkinElmer 2105-0020). GraphPad 9.0 was used to fit the inhibition rate and compound concentration into a nonlinear regression curve (dose response-variable slope) to calculate $GI_{50}$, inhibition rate = {1-(A-B)/(C-B)}*100%, A = signal value of compound; B = average signal of blank control; C = average signal of negative control (wherein negative control was 0.1% DMSO).

$$A = 0\ nM \leq GI_{50} < 1000\ nM;\ B = 1000\ nM \leq GI_{50} < 5000\ nM;\ C = 5000\ nM \leq GI_{50}.$$

Table 2. $GI_{50}$ of selected compounds in NUGC-3 cell proliferation experiment

| Compound | $GI_{50}$ (nM) | Compound | $GI_{50}$ (nM) |
|---|---|---|---|
| **1** | B | **35-6e** | C |
| **3** | A | **35-6f** | B |
| **4** | B | **35-6g** | C |
| **5** | B | **35-6h** | B |
| **6** | C | **35-6i** | A |
| **7** | B | **35-6j** | A |
| **8** | B | **40** | A |
| **9** | A | **41** | A |
| **10** | B | **42** | A |
| **12** | C | **44d** | A |

(continued)

| Compound | $GI_{50}$ (nM) | Compound | $GI_{50}$ (nM) |
|---|---|---|---|
| **13** | B | **46** | A |
| **14** | B | **48c** | A |
| **15** | A | **53** | A |
| **16** | C | **54** | A |
| **18** | B | **58** | A |
| **19** | C | **64** | A |
| **20** | B | 65 | A |
| **21** | B | **65c** | A |
| **22** | C | **65d** | A |
| **23** | B | **70d** | A |
| **24** | B | **73d** | A |
| **25** | C | **74d** | A |
| **29** | B | **76d** | A |
| **30** | B | **78d** | A |
| **31a** | C | | |
| **32a** | C | | |

Table 9 showed the multiple ratio of $EC_{50}$ of the compound to the reference compound.

The structure of the reference compound was as follows:

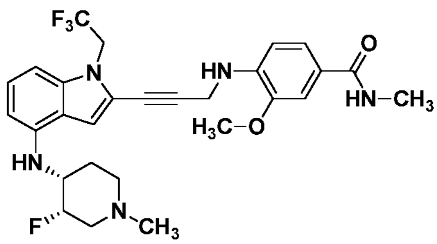

A: $GI_{50}$ of the compound ≤ $GI_{50}$ of the reference compound; B: $GI_{50}$ of the reference compound < $GI_{50}$ of the compound ≤ 3.0 * $GI_{50}$ of the reference compound; C: $GI_{50}$ of the compound ≥ 3 * $GI_{50}$ of the reference compound;

Table 9

| Compound No. | Compound $GI_{50}$ (nM)/Reference compound $GI_{50}$ (nM) | Compound No. | Compound $GI_{50}$ (nM)/Reference compound $GI_{50}$ (nM) |
|---|---|---|---|
| **1** | C | **37e** | B |
| **2** | C | **37f** | C |
| **3** | B | **37g** | A |
| **4** | B | **37h** | C |
| **5** | B | **39c** | A |
| **6** | C | **39d** | A |
| **7** | C | **47** | C |
| **8** | C | **48** | B |
| **9** | B | **40** | B |

(continued)

| Compound No. | Compound $GI_{50}$ (nM)/Reference compound $GI_{50}$ (nM) | Compound No. | Compound $GI_{50}$ (nM)/Reference compound $GI_{50}$ (nM) |
|---|---|---|---|
| **10** | C | **41** | B |
| **11** | C | **42** | B |
| **12** | C | **43** | C |
| **13** | C | **44** | C |
| **14** | B | **49** | C |
| **15** | C | **50** | C |
| **16** | C | **51** | C |
| **17** | C | **52** | C |
| **18** | C | **57** | B |
| **19** | C | **45** | C |
| **20** | C | **68** | C |
| **21** | C | **46** | B |
| **22** | C | **48c** | B |
| **23** | C | **48d** | C |
| **24** | C | **53** | C |
| **25** | C | **58** | B |
| **26** | C | **65** | A |
| **27** | C | **54** | C |
| **28** | C | **55** | C |
| **29** | C | **65c** | A |
| **30** | C | **65d** | B |
| **31a** | C | **68c** | C |
| **32a** | C | **68d** | C |
| **33** | C | **64** | B |
| **34** | C | **56** | C |
| **35-6e** | C | **44c** | C |
| **35-6f** | C | **44d** | B |
| **35-6g** | C | **59** | C |
| **35-6h** | C | **60** | C |
| **35-6i** | A | **61** | C |
| **35-6j** | C | **62** | C |
| **73d** | A | **85** | C |
| **76d** | A | **63** | C |
| **78d** | A | **74d** | A |
| **70d** | A | | |

The experimental results showed that the compounds of the present invention could significantly inhibit the proliferation of NUGC-3 gastric adenocarcinoma cells containing the p53 Y220C mutation.

Experimental Example 3: BxPC-3 cell proliferation test

To detect the inhibitory effects of the aforementioned compounds on the proliferation ability of BxPC-3 cells, 125 BxPC-3 cells (ATCC®CRL-1687) were seeded into a 384-well plate (Corning 3765), 35 μL per well, and placed in a constant temperature incubator at 37°C and 5% $CO_2$ overnight. Then, 40 nL of the compound was added to the 384-well plate using an Echo 655 Liquid Handler, with a maximum final concentration of 10 μM, 1:3 serial dilution, and in a total of 10 doses. Incubation was performed at 37 °C, 5% $CO_2$ for 6 days. The plate was taken out from the incubator and equilibrated at room temperature for 10 minutes. 20 μL of CTG (Promage G7572) was added to each well of the plate according to the platemap. The plate was centrifuged at 1000 rpm for 1 minute. The lysis of cells was induced by mixing on a shaker for 2 minutes. The plate was centrifuged at 1000 rpm for 1 minute. Incubation was performed at room temperature for 10 minutes to stabilize luminescence signals. Luminescence values were recorded by Envision (PerkinElmer 2105-0020). GraphPad 9.0 was used to fit the inhibition rate and compound concentration into a nonlinear regression curve (dose response-variable slope) to calculate $GI_{50}$, inhibition rate = {1-(A-B)/(C-B)}*100%, A = signal value of compound; B = average signal of blank control; C = average signal of negative control (wherein the negative control was 0.1% DMSO).

$$A = 0\ nM \leq GI_{50} < 1000\ nM;\ B = 1000\ nM \leq GI_{50} < 5000\ nM;\ C = 5000\ nM \leq GI_{50}.$$

Table 3. $GI_{50}$ of selected compounds in BXPC-3 cell proliferation experiment

| Compound No. | $GI_{50}$ (nM) |
|---|---|
| **1** | B |
| **3** | A |
| **4** | A |
| **5** | A |
| **8** | B |
| **9** | A |
| **10** | B |
| **35-6i** | A |
| **57** | A |
| **48c** | A |
| **58** | A |
| **65c** | A |
| **65d** | A |
| **68c** | A |
| **64** | A |
| **44d** | A |

The experimental results showed that the compounds of the present invention could significantly inhibit the proliferation of BxPC-3 pancreatic cancer cells containing p53 Y220C mutation.

Example 4: Caco-2 cell permeability test

Caco-2 cells (ATCC, Cat. No. HTB-37) were inoculated to a 96-well plate (Corning HTS Transwell, Cat. No. 3391) at 1.25 x $10^5$/$cm^2$, and the culture medium (DMEM + 10% FBS + 1% Penicillin-Streptomycin Liquid (100X) + 1% NEAA) was replaced every 3 to 4 days. The plate was placed in an incubator at 37 °C with 5% $CO_2$ until a confluent cell monolayer was formed after 21 to 28 days. Before the transport experiment, the culture medium was removed from the cell culture plate, and the plate was washed twice with pre-warmed HBSS buffer (10 mM HEPES, pH 7.4), and 100 μL of HBSS buffer was added to each well. After incubation at 37 °C for 30 minutes, the TEER value was measured using a cell resistance meter (Millicell-ERS2) to confirm the integrity and density of the monolayer cells. The HBSS buffer was discarded, the

upper plate containing the cell monolayer membrane was transferred to the corresponding receiving plate, 10 mM test compound stock solution was diluted to a concentration of 10 μM with HBSS transport buffer containing 0.5% BSA, and it was added to the apical side (A side) and basolateral side (B side), respectively. Incubation was carried out for 120 min at 37°C, 5% $CO_2$, and 95% relative humidity, and the permeation of the test compound from A side to B side or from B side to A side and the efflux rate of the compound were determined. The test compound and the reference compound were quantified by LC-MS/MS analysis based on the peak area ratio of analyte/internal standard. The apparent permeability coefficient Papp (cm/s) was calculated using the following formula:

$$Papp = (dCr/dt) \times Vr/(A \times C0)$$

wherein, dCr/dt represented the functional relation between cumulative concentration of compound in receptor compartment and time; V represented the volume of solution in receptor compartment (0.1 mL at apical side, and 0.25 mL at basolateral side); A represented the transport surface area, i.e., the monolayer cell area was 0.0804 $cm^2$; C0 represented the initial concentration of compound in donor compartment.

The efflux ratio was calculated using the following formula:

$$\text{Efflux Ratio} = Papp(BA) / Papp(AB)$$

The percent recovery was calculated using the following formula:

$$\%\ Recovery = 100 \times [(Vr \times Cr) + (Vd \times Cd)] / (Vd \times C0)$$

$$\%Total\ Recovery = 100 \times [(Vr \times Cr) + (Vd \times Cd) + (Vc \times Cc)] / (Vd \times C0)$$

wherein, Vd represented the volume of donor compartment (0.1 mL on the apical side and 0.25 mL on the basolateral side); C and Cr represented the final concentrations of the transported compound in the donor and receptor compartment, respectively; Cc represented the compound concentration in cell lysate; Vc represented the volume of insert well (which was 0.1 mL in this experiment).

Table 4. Caco-2 cell permeability data of selected compounds

| Compound No. | Papp ($10^{-6}$ cm/s) | | Efflux Ratio |
|---|---|---|---|
| | A to B | B to A | |
| **5** | 2.80 | 23.14 | 8.27 |
| **35-6i** | 2.66 | 11.45 | 4.31 |
| **42** | 2.16 | 3.72 | 1.72 |
| **44** | 3.54 | 6.34 | 1.79 |
| **64** | 4.92 | 5.99 | 1.22 |
| **47** | 3.97 | 6.04 | 1.52 |
| **48** | 2.50 | 6.43 | 2.58 |
| **57** | 3.70 | 6.82 | 1.84 |
| **58** | 2.41 | 7.46 | 3.09 |
| **54** | 2.30 | 5.16 | 2.24 |

The experimental data results showed that the compounds of the present invention have good cell permeability and low efflux properties.

Example 5: Liver microsome stability test

The 10 mM test compound/ketanserin (Energy Chemical, Cat. No. E020711) stock solution was diluted with acetonitrile to 0.5 mM compound/reference compound. The frozen 100 mM NADPH (MCE, Cat. No. HYF003/CS-4998)

was diluted with phosphate buffer solution to a concentration of 6 mM; 20 mg/mL HLM (BioIVT, Cat. No. X008067) or MLM (BioIVT, Cat. No. M00501) was diluted with phosphate buffer solution to a concentration of 0.75 mg/ml, and added to a 96-round well plate, respectively, and shaken evenly on an oscillator at 600 rpm. The above oscillated samples were pipetted 3 times with an electric pipettor and dispensed into a new 96-well plate (containing NADPH group (n = 2), NADPH-free group was added at 0 and 45 min (n = 1)), and there were a total of 5 groups of 0, 5, 15, 30 and 45 min, respectively. After pre-incubation of the 5 groups of samples at 37°C for 5 min, glacial acetonitrile was quickly added to the 0 min group for precipitation, and after rapid vortexing, K-Buffer or NADPH was added with an electric pipettor. The remaining groups were sequentially added with 6 mM NADPH to start the reaction in the order of 45, 30, 15 and 5 min samples, and K-Buffer solution was added to the NADPH-free group. After mixing at 1000 rpm in an oscillating mixer for 10 s, they were continuously incubated at 37°C for the corresponding time. The reaction was terminated by quickly adding glacial acetonitrile internal standard using an electric pipettor; after vortexing at 900 rpm for 10 seconds, heat sealing was performed, oscillation was performed on an oscillator for 10 minutes, centrifugation was performed at 4000 rpm for 50 minutes, the supernatant was collected and diluted with pure water, and the test compound and the reference compound were quantified by LC-MS/MS analysis.

Table 5. Liver microsomal stability data of selected compounds

| Compound No. | $T_{1/2}$ (min) | |
|---|---|---|
| | Human | Mouse |
| Reference compound | 24.9 | 17.1 |
| **35-6i** | - | 19.2 |
| **58** | 37.5 | 33.0 |
| **54** | 32.0 | 78.4 |
| **64** | 44.0 | 55.0 |
| **65c** | 65.3 | 11.2 |
| **65d** | 65.3 | 16.9 |
| **74d** | 55.8 | 22.0 |
| **68c** | 72.2 | 65.9 |
| **68d** | 50.3 | 76.0 |

The experimental data showed that the compounds of the present invention have better metabolic stability in human and mouse liver microsomes than the reference compound.

Example 6: CYP 3A4 time-dependent inhibition test

The test compound and mifepristone (Aladdin, Cat. No. M126999) were formulated with DMSO to form 10 mM stock solutions, which were then diluted to 0, 0.02, 0.06, 0.2, 0.6, 2 and 6 mM, and the final test concentrations of the test compound and mifepristone were 0, 0.1, 0.3, 1, 3, 10 and 30 μM; midazolam (Stanford analytical chemicals Inc., Cat. No. MSS-988112) substrate was formulated with DMSO to form 8 mM stock solution, which was then diluted to 20 μM, the final test concentration of midazolam substrate was 1 μM, and the incubation time was 5 min; 20 mg/mL CYP 3A4 (BioIVT, Cat. No. X008067) was diluted with K-Mg-buffer to a final test concentration of 0.05 mg/mL, and 100 mM NADPH (MCE, Cat. No. HYF003/CS-4998) was diluted to 10 mM in phosphate buffer, and the final test concentration was 1 mM. CYP 3A4 and the test compound or mifepristone of various concentrations were added to a 96-deep well plate for pre-incubation. The plate was placed in a water bath and preheated at 37°C for 5 min. For the 0 min pre-incubation samples, midazolam substrate was added to the incubation plate, then 10 mM NADPH solution was added, and the reaction was carried out in a 37°C water bath for 5 min; for the 30 min pre-incubation samples with NADPH, 10 mM NADPH solution was added to the incubation plate, pre-incubated in a 37°C water bath for 30 min, and then midazolam substrate was added and reacted in a 37°C water bath for 5 min; for the 30 min pre-incubation samples without NADPH, the incubation plate was pre-incubated in a 37°C water bath for 30 min, then midazolam substrate was added, 10 mM NADPH solution was added, and the reaction was carried out in a 37°C water bath for 5 min. After the reaction was completed, the quenching solution (500 nM tolbutamide (Bide Pharmaceuticals, Cat. No. BD154332) and 10 nM terfenadine (Sigma, Cat. No. T96532)) were added to quench the reaction, and the plate was centrifuged at 3,220 g for 40 min at 4°C. The supernatant was pipetted into a new plate, the supernatant was diluted with pure water and mixed well, and the sample was analyzed using UPLC-MS/MS.

Table 6. CYP 3A4 time-dependent inhibition data of selected compounds

| Compound No. | $IC_{50}$ (μM) | | |
|---|---|---|---|
| | 0 min | 30 min, without NADPH | 30 min, with NADPH |
| Reference compound | 3.24 | 2.55 | 0.58 |
| **5** | 8.05 | 8.36 | 1.89 |
| **35-6i** | 7.48 | 7.46 | 3.01 |
| **44d** | 3.87 | 7.06 | 1.99 |
| **70d** | 5.95 | 6.65 | 1.91 |
| **65c** | 4.00 | 4.77 | 1.69 |
| **74d** | 4.55 | 5.91 | 1.56 |

The experimental results showed that the compounds of the present invention could improve the time-dependent inhibition activity of CYP 3A4 in comparison with the reference compound.

Example 7: Pharmacokinetic test in mice

ICR male mice (15 to 35 g, 5 to 10 weeks old, Shanghai Xipuer-Bikai Experimental Animal Co., Ltd.) were selected as test animals in this study. The LC-MS/MS method was used to quantitatively determine drug concentrations in the plasma of mice at different time points after intravenous injection or oral gavage administration of the test compound to evaluate the pharmacokinetic characteristics of the compound in mice. The clear solution of the test compound was injected into ICR mice via the tail vein (without fasting, solvent: 40% hydroxypropyl-β-cyclodextrin) and administered to ICR mice by oral gavage (fasting for 10 to 14 hours, free drinking water, solvent: 0.2% hydroxypropyl cellulose, 0.5% Tween 80). For intravenous administration, blood was collected from the submandibular venous plexus at 0.033, 0.083, 0.25, 0.5, 1, 2, 4, 8, and 24 hours after administration, placed in an anticoagulant tube with EDTA-K2 (Jiangsu Kangjian Medical Supplies Co., Ltd.), mixed, and centrifuged at 2-8°C, 6800 g for 6 minutes to obtain plasma; for oral gavage administration, blood was collected from the submandibular venous plexus at 0.083, 0.25, 0.5, 1, 2, 4, 6, 8, and 24 hours after administration, placed in an anticoagulant tube with EDTA-K2 (Jiangsu Kangjian Medical Supplies Co., Ltd.), mixed, and centrifuged at 2-8°C, 6800 g for 6 minutes to obtain plasma. The blood drug concentration was determined by LC-MS/MS, and the relevant pharmacokinetic parameters were calculated by the non-compartmental linear logarithmic trapezoidal method using Phoenix WinNonlin™ Version 7.0 (Pharsight, USA) pharmacokinetic software.

Table 7. Mouse pharmacokinetic data of selected compounds

| Compound No. | po 25 mpk | | iv 2.5 mpk |
|---|---|---|---|
| | $C_{max}$ (ng/mL) | AUC (ng*h/mL) | CL (mL/min/kg) |
| **5** | 1145 | 4070 | 54 |
| **35-6i** | 6860 | 19705 | 20 |
| **44d** | 11831 | 43085 | 13 |
| **70d** | 13094 | 59732 | 11 |
| **65c** | 3638 | 17194 | 22 |
| **74d** | 4162 | 17363 | 17 |

The experimental results showed that the compounds of the present invention could significantly reduce the clearance rate of the compounds, increase $C_{max}$ and AUC, and improve the PK properties of the compounds.

Example 8: In vivo efficacy test in mice

The use and welfare of experimental animals in this experimental protocol were carried out in accordance with the regulations of the Association for Assessment and Accreditation of Laboratory Animal Care (AAALAC). After Balb/c nude mice (Charles river) arrived at the animal room, they were kept in the experimental environment for 3 days before the test began. The experimental Balb/c nude mice were kept in a specific pathogen-free (SPF) barrier at Shanghai Qishang

Biomedical Technology Co., Ltd., with 5 mice per cage. Human gastric cancer NUGC3 tumor cells were cultured in RPMI1640 complete medium (supplemented with a final concentration of 10% fetal bovine serum and 1% penicillin & streptomycin) in incubator at 37°C and 5% $CO_2$. The tumor cells were passaged 2 to 3 times per week, and the cells in the exponential growth phase were collected and counted for tumor cell inoculation.

$5x10^6$ NUGC3 tumor cells were resuspended in 0.1ml of DPBS (Dulbecco's Phosphate-Buffered Saline) and inoculated subcutaneously on the right flank of each mouse to form a tumor. Appropriate mice were selected for grouping according to the tumor volume and the body weight. The tumor-bearing mice were randomly grouped and administered (the vehicle was 0.2% HPC + 0.5% Tween80). When the body weight of a single animal dropped by more than 15% (BWL ≥ 15%), the corresponding single animal/group of animals was given a drug withdrawal period until its body weight loss recovered to the body weight loss within 10% (BWL ≤ 10%). The tumor size was measured using a vernier caliper, and the calculation formula for tumor volume (TV) was: $V = 1/2 \times a \times b^2$, where a and b represented the long and short diameters of tumor, respectively.

The animals were weighed using an electronic scale, and the relative change in body weight (RCBW) was: RCBW (%) = (BWt - BW0)/BW0×100%; BWt represented the animal weight at the time of measurement; BW0 represented the animal weight at the time of grouping and administration.

TGI (%) or T/C (%) was used to evaluate the efficacy of the test sample. Tumor growth inhibition rate TGI (%): TGI (%) = 100 - TRTV / CRTV * 100%, relative tumor proliferation rate T/C (%): T/C (%) = TRTV / CRTV * 100%, (TRTV: treatment group RTV; CRTV: negative control group RTV). The relative tumor volume (RTV) was calculated based on the measurement results, and the calculation formula was: RTV = Vt / V0, wherein V0 represented the tumor volume measured at the time of grouping and administration (i.e., d0), and Vt represented the tumor volume at each measurement.

## Claims

1. A compound represented by Formula M, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof,

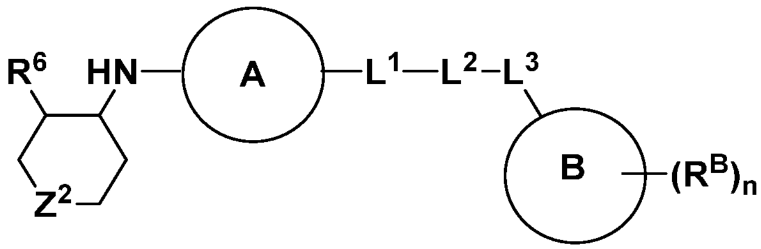

Formula M

wherein:

Ring A is selected from

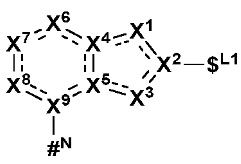

,

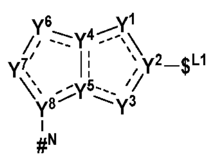

,

**wherein** $\#^N$ represents a connection point between Ring A and NH, $\$^{L1}$ is a connection point between Ring A and $L^1$;

each ------ is independently a single bond or a double bond;

$X^1$, $X^2$, $X^3$, $X^4$, $X^5$, $X^6$, $X^7$, $X^8$, $X^9$ are each independently selected from the group consisting of C, CH, $CR^a$, $CR^b$, $CR^c$, C(=O), N, NH, and S;

$Y^1$, $Y^2$, $Y^3$, $Y^4$, $Y^5$ $Y^6$, $Y^7$, $Y^8$ are each independently selected from the group consisting of S, N, C, CH, $CR^d$, and $NR^d$;

$R^a$, $R^d$ are each independently selected from the group consisting of deuterium, C1-C6 alkyl, C3-C7 cycloalkyl, 4- to 8-membered heterocyclyl, and C5-C6 heteroaryl; wherein the C1-C6 alkyl, C3-C7 cycloalkyl, 4- to 8-membered heterocyclyl, C5-C6 heteroaryl are each independently substituted with deuterium, halogen, hydroxyl, amino, cyano, or C3-C7 cycloalkyl;

each $R^b$ is independently selected from the group consisting of hydrogen, deuterium, hydroxyl, halogen, and cyano;

each $R^c$ is independently selected from the group consisting of hydrogen, deuterium, hydroxyl, halogen, and cyano;

$L^1$ is selected from the group consisting of

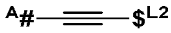,

and 5- to 6-membered heteroarylene; wherein, $\#^A$ represents a connection point between $L^1$ and Ring A, $\$^{L2}$ represents a connection point between $L^1$ and $L^2$;

$L^2$ is

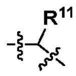;

$L^3$ is selected from the group consisting of NH, -NHC(=O)-, and -NHC(=O)NH-;

Ring B is selected from the group consisting of phenyl, and 5- to 10-membered heteroaryl;

each $R^B$ is independently selected from the group consisting of hydrogen, deuterium, halogen, cyano, amino, C1-C6 alkyl, $R^3O$-, $R^eR^fN$-C(=O)-, $R^gO$-C(=O)-, $R^hS(=O)_2$-, $R^iR^jN$-$S(=O)_2$-,

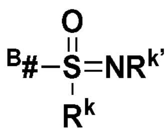, 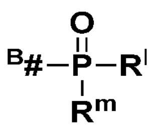,

5- to 6-membered heteroaryl, 4- to 8-membered saturated heterocyclyl, and C3-C7 cycloalkyl; wherein the C1-C6 alkyl, 5- to 6-membered heteroaryl, 4- to 8-membered saturated heterocyclyl, C3-C7 cycloalkyl are optionally substituted with 0, 1, 2, 3 substituents selected from the group consisting of deuterium, halogen, hydroxyl, amino, cyano, C1-C6 alkoxy, and phenyl; $\#^B$ represents a connection point between

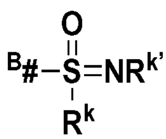

or

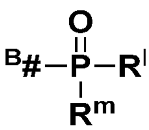

and Ring B;

n is selected from the group consisting of 0, 1, 2, and 3;

each $R^3$ is independently selected from the group consisting of hydrogen, deuterium, C1-C6 alkyl, and C1-C6 alkyl-C(=O)-; wherein the C1-C6 alkyl is optionally substituted with halogen, hydroxyl, cyano, amino, or C1-C6 alkoxy;

$R^e$, $R^f$ are each independently selected from the group consisting of hydrogen, deuterium, and C1-C6 alkyl; wherein the C1-C6 alkyl is optionally substituted with halogen, hydroxyl, cyano, amino, C1-C6 alkyl, C1-C6 alkoxy, or 4- to 8-membered heterocyclyl; or, $R^e$, $R^f$ together with the N atom to which they connect form a 4- to 8-membered saturated heterocyclic ring or a 7- to 11-membered spiro-heterocyclic ring, wherein the 4- to 8-membered saturated heterocyclic ring or 7- to 11-membered spiro-heterocyclic ring is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

each $R^g$ is selected from the group consisting of hydrogen, deuterium, C1-C6 alkyl, and C3-C7 cycloalkyl; wherein the C1-C6 alkyl, C3-C7 cycloalkyl are optionally substituted with deuterium, halogen, hydroxyl, amino, or cyano;
each $R^h$ is selected from the group consisting of hydrogen, deuterium, amino, C1-C6 alkyl, C3-C7 cycloalkyl, and 4- to 8-membered saturated heterocyclyl; or $R^h$ is connected to the ortho position of $R^hS(=O)_2$- to form a 5- to 6-membered heterocyclic ring;
$R^i$, $R^j$ are each independently selected from the group consisting of hydrogen, deuterium, C1-C6 alkyl, C1-C6 haloalkyl, C3-C7 cycloalkyl, C3-C7 halocycloalkyl, and 5- to 6-membered heteroaryl; or, $R^i$, $R^j$ together with the N atom to which they connect form a 4- to 8-membered saturated heterocyclic ring or a 7- to 11-membered spiro-heterocyclic ring;
$R^k$ is selected from the group consisting of hydrogen, deuterium, C1-C6 alkyl, cyano, C3-C7 cycloalkyl, and 4- to 8-membered heterocyclyl; $R^{k'}$ is selected from the group consisting of hydrogen, deuterium, C1-C6 alkyl, cyano, and C3-C7 cycloalkyl; or, $R^k$, $R^{k'}$ together with -S=N-to which they connect form a 4- to 8-membered heterocyclic ring; or $R^k$ is connected to the ortho position of

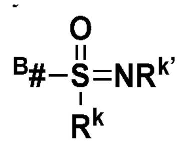

to form a 5- to 6-membered heterocyclic ring;
$R^l$, $R^m$ are each independently selected from the group consisting of hydrogen, deuterium, C1-C6 alkyl, and C3-C7 cycloalkyl; or, $R^l$, $R^m$ together with the P atom to which they connect form a 4- to 8-membered saturated heterocyclic ring; or one of $R^l$, $R^m$ is connected to the ortho position of

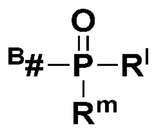

to form a 5- to 6-membered heterocyclic ring;
$R^6$ is selected from the group consisting of hydrogen, deuterium, hydroxyl, halogen, and cyano;
$R^{11}$ is selected from the group consisting of hydrogen, deuterium, and C1-C6 alkyl;
$Z^2$ is selected from the group consisting of $N(R^7)$, $C(R^8)_2$, O, and $S(O)_2$;
$R^7$ is selected from the group consisting of hydrogen, deuterium, and C1-C6 alkyl; wherein the C1-C6 alkyl is optionally substituted with halogen, hydroxyl, amino, cyano, C1-C6 alkoxy, halogen-substituted C1-C6 alkoxy, hydroxyl-substituted C1-C6 alkoxy, or amino-substituted C1-C6 alkoxy;
each $R^8$ is independently selected from the group consisting of hydrogen, deuterium, halogen, hydroxyl, amino, cyano, C1-C6 alkyl, 4- to 8-membered saturated heterocyclyl, 7- to 11-membered spiro-heterocyclyl, 5- to 10-membered bridged heterocyclyl, and $R^oR^pN$-; wherein the C1-C6 alkyl, 4- to 8-membered saturated heterocyclyl, 7- to 11-membered spiro-heterocyclyl, 5- to 10-membered bridged heterocyclyl are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy; provided that: at most one $R^8$ is selected from the group consisting of 4- to 8-membered saturated heterocyclyl, 7- to 11-membered spiro-heterocyclyl, and 5- to 10-membered bridged heterocyclyl; or two $R^8$ groups together with the carbon atom to which they connect form a 4- to 8-membered heterocyclyl; wherein the 4- to 8-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;
$R^o$, $R^p$ are each independently selected from the group consisting of hydrogen, deuterium, C1-C6 alkyl, and 4- to 8-membered saturated heterocyclyl; wherein the C1-C6 alkyl, 4- to 8-membered saturated heterocyclyl are optionally substituted with halogen, hydroxyl, amino, cyano, or C1-C6 alkoxy; or, $R^o$ and $R^p$ together with the N atom to which they connect form a 4-to 8-membered saturated heterocyclic ring, a 7- to 11-membered spiro-heterocyclic ring or a 5-to 10-membered bridged heterocyclic ring; wherein the 4- to 8-membered heterocyclic ring, 7- to 11-membered spiro-heterocyclic ring, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;
the compound of Formula M must simultaneously meet conditions 1) and 2):

1) when Ring A is not

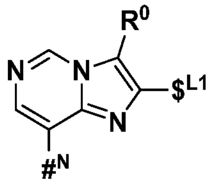 , 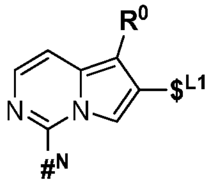 , 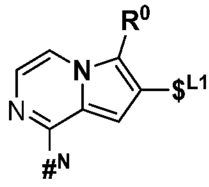 ,

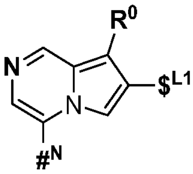 , 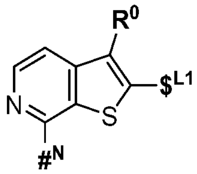 , 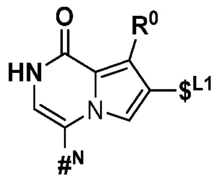 , 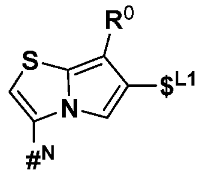 , 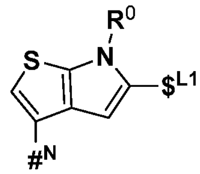 ,

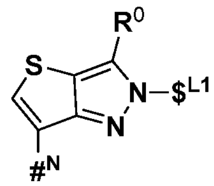 , 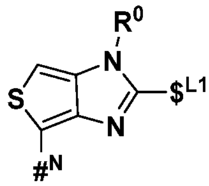 , 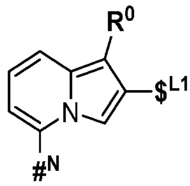 ,

$R^0$ is selected from the group consisting of C1-C6 alkyl, C3-C7 cycloalkyl, 4- to 8-membered heterocyclyl, and C5-C6 heteroaryl, wherein the C1-C6 alkyl, C3-C7 cycloalkyl, 4- to 8-membered heterocyclyl, C5-C6 heteroaryl are each independently optionally substituted with deuterium, halogen, hydroxyl, amino, or cyano, and at least one of the following conditions must be met:

a) $L^1$ is 5- to 6-membered heteroarylene;
b) $L^3$ is selected from the group consisting of -NHC(=O)-, and -NHC(=O)NH-;
c) Ring B is substituted with $R^hS(=O)_2$-, and $R^h$ is connected to the ortho position of $R^hS(=O)_2$- to form a 5- to 6-membered heterocyclic ring; or

Ring B is substituted with

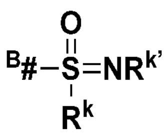 ,

and $R^k$, $R^{k'}$ together with -S=N- to which they connect form a 4- to 8-membered heterocyclic ring, or $R^k$ is connected to the ortho position of

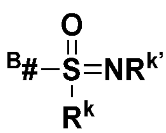

to form a 5- to 6-membered heterocyclic ring; or
Ring B is substituted with

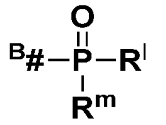

,

and $R^l$, $R^m$ together with the P atom to which they connect form a 4- to 8-membered saturated heterocyclic ring, or one of $R^1$, $R^m$ is connected to the ortho position of

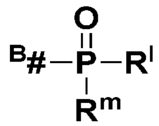

to form a 5- to 6-membered heterocyclic ring;

d) $Z^2$ is $C(R^8)_2$, and only one $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they connect form a 4- to 8-membered saturated heterocyclic ring, a 7-to 11-membered spiro-heterocyclic ring or a 5- to 10-membered bridged heterocyclic ring; wherein the 7- to 11-membered spiro-heterocyclyl, 7- to 11-membered spiro-heterocyclic ring, 5-to 10-membered bridged heterocyclyl, 5- to 10-membered bridged heterocyclic ring, 4- to 8-membered saturated heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;

2) the compound represented by Formula M does not include the following compounds:

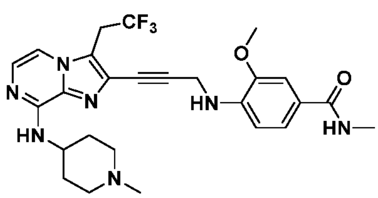
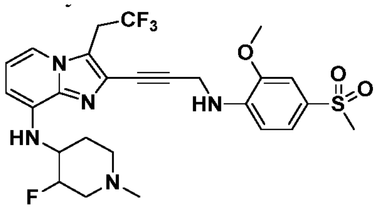
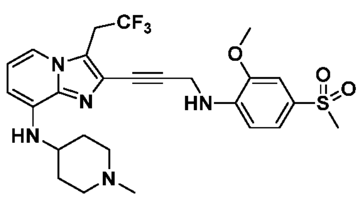

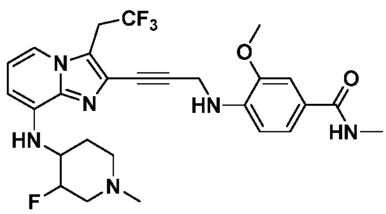
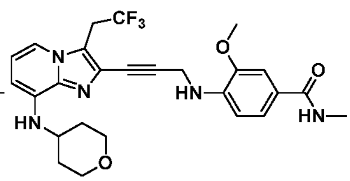
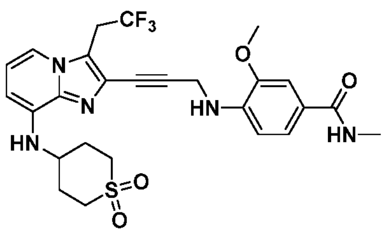

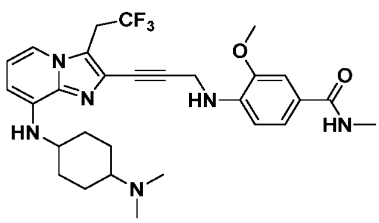
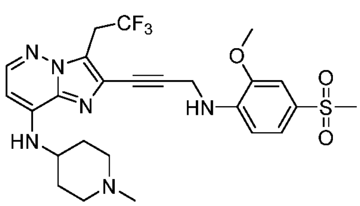
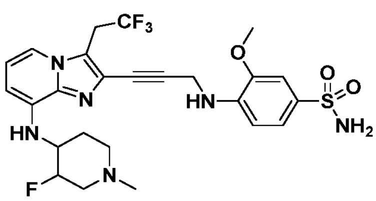

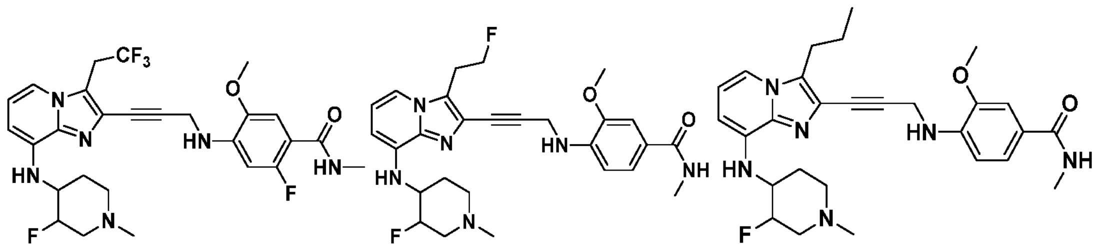

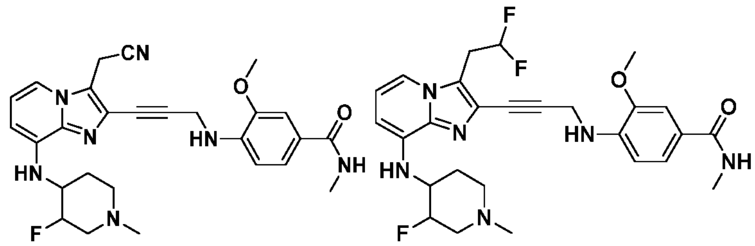

.

2. The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

Ring A is selected from the group consisting of

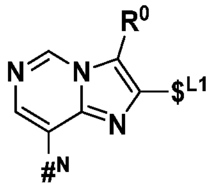 , 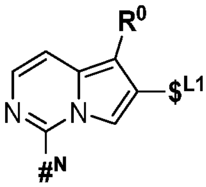 ,

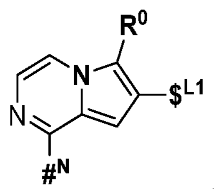 , 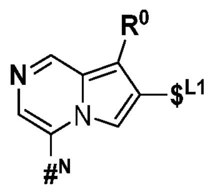 , 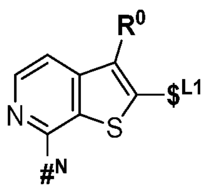 , 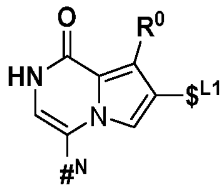 , 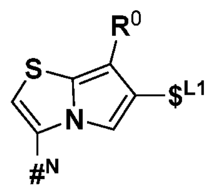 ,

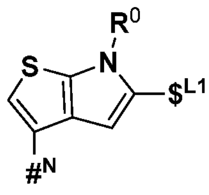 , 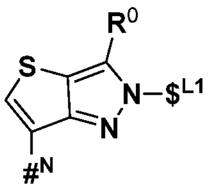 , 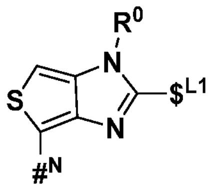 , and 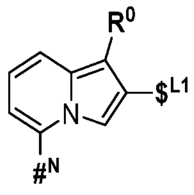 ;

$R^0$ is selected from the group consisting of C1-C6 alkyl, C1-C6 haloalkyl, cyano-C1-C6 alkylene-, C3-C6 cycloalkyl, and C3-C6 halocycloalkyl;
preferably, $R^0$ is selected from the group consisting of C1-C4 alkyl, C1-C2 haloalkyl, and cyano-C1-C2 alkylene-;
more preferably, $R^0$ is selected from the group consisting of $CF_3CH_2$-, $CH_2FCH_2$-, $CH_3CH_2CH_2$-, $CNCH_2$-, and $CHF_2CH_2$-.

3. The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

Ring A is selected from the group consisting of

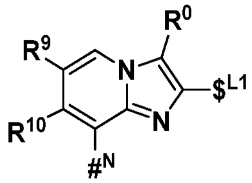,
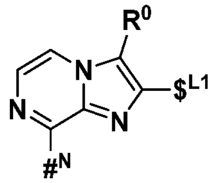,

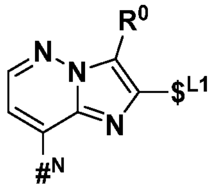,
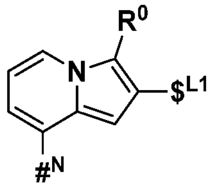, and
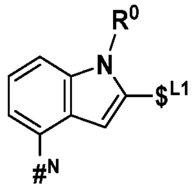;

$R^9$ and $R^{10}$ are each independently selected from the group consisting of hydrogen, deuterium, hydroxyl, amino, halogen, cyano, and C1-C3 alkyl;
preferably, Ring A is selected from the group consisting of

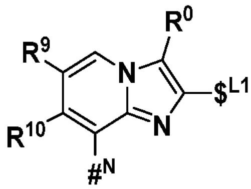,

and

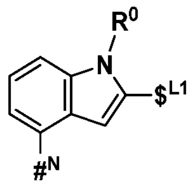;

$R^9$ is selected from the group consisting of hydrogen, and hydroxyl; $R^{10}$ is selected from the group consisting of hydrogen, and fluorine;
$R^0$ is selected from the group consisting of C1-C6 alkyl, C1-C6 haloalkyl, cyano-C1-C6 alkylene-, C3-C6 cycloalkyl, and C3-C6 halocycloalkyl;
more preferably, $R^0$ is selected from the group consisting of C1-C4 alkyl, C1-C2 haloalkyl, and cyano-C1-C2 alkylene-;
most preferably, $R^0$ is selected from the group consisting of $CF_3CH_2$-, $CH_2FCH_2$-, $CH_3CH_2CH_2$-, $CNCH_2$-, and $CHF_2CH_2$-; $R^9$ and $R^{10}$ are hydrogen.

**4.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,
Ring A is selected from the group consisting of

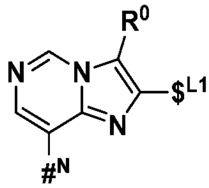
,
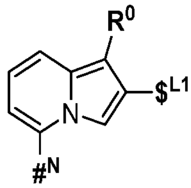
,

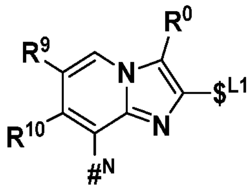
, and
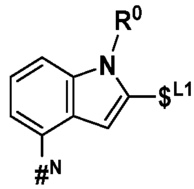
;

$R^9$ and $R^{10}$ are each independently selected from the group consisting of hydrogen, deuterium, hydroxyl, amino, halogen, cyano, and C1-C3 alkyl.

**5.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

$L^1$ is selected from the group consisting of

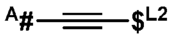
,

and 5-membered heteroarylene;
preferably, $L^1$ is selected from the group consisting of

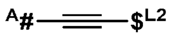
,

1,3,4-thiadiazolylene, 1,2,4-oxadiazolylene, 1,3,4-oxadiazolylene, and thiazolylene;
more preferably, $L^1$ is selected from the group consisting of

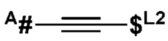
,
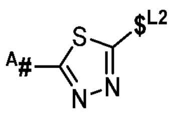
,

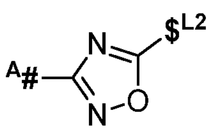
,
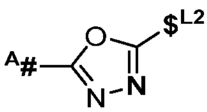
,
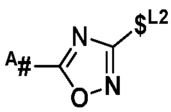
,
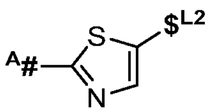
,
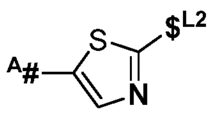
,

and

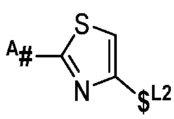
.

**6.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

$R^{11}$ is selected from the group consisting of hydrogen, deuterium, and C1-C4 alkyl;
preferably, $R^{11}$ is selected from the group consisting of hydrogen and methyl;
$L^3$ is selected from the group consisting of NH, $\#^{L2}$-NHC(=O)-$\$^B$, and $\#^{L2}$-NHC(=O)NH-$\$^B$, wherein $\#^{L2}$ represents a connection point between $L^3$ and $L^2$, and $\$^B$ represents a connection point between $L^3$ and Ring B;
preferably, $L^3$ is NH.

**7.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

Ring B is selected from the group consisting of phenyl, pyridyl, 5-membered heteroaromatic ring, and

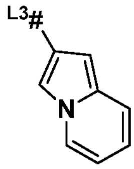;

$\#^{L3}$ represents a connection point between Ring B and $L^3$,
preferably, Ring B is selected from the group consisting of phenyl, pyridyl, thienyl, pyrrolyl, thiazolyl, pyrazolyl, imidazolyl, and furanyl;
more preferably,

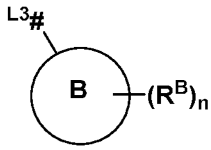

is selected from the group consisting of

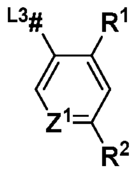,

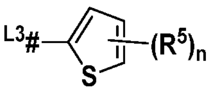, 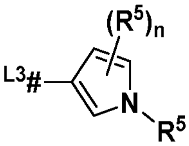, 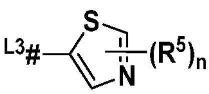, 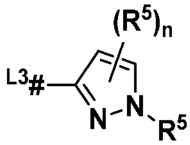, 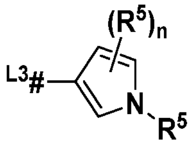,

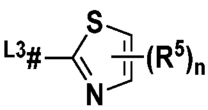, 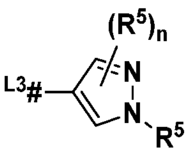, 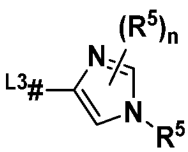, 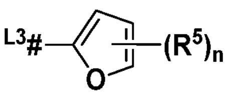, 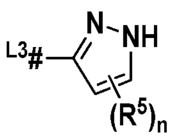,

and

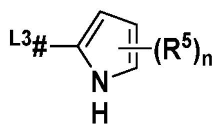

;

n is selected from the group consisting of 0, 1, and 2; preferably n is 0 or 1;

$Z^1$ is selected from the group consisting of N and $CR^4$;

$R^1$ is selected from the group consisting of hydrogen, deuterium, hydroxyl, halogen, amino, C1-C6 alkyl, and $R^3O$-; wherein, the C1-C6 alkyl is optionally substituted with 1, 2 or 3 substituents selected from the group consisting of deuterium, halogen, hydroxyl and amino;

$R^2$ is selected from the group consisting of hydrogen, deuterium, cyano, halogen, C1-C6 alkyl, $R^eR^fN$-C(=O)-, $R^gO$-C(=O)-, $R^hS(=O)_2$-, $R^iR^jN$-$S(=O)_2$-,

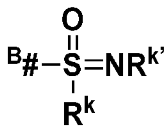 , 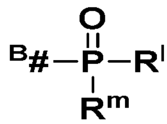 ,

5-to 6-membered heteroaryl, and 4- to 8-membered heterocyclyl; wherein, the C1-C6 alkyl, 5- to 6-membered heteroaryl and 4- to 8-membered heterocyclyl are optionally substituted with 0, 1, 2 and 3 substituents selected from the group consisting of deuterium, halogen, hydroxyl, amino, cyano, C1-C6 alkoxy, and phenyl;

$R^4$ is selected from the group consisting of hydrogen, deuterium, hydroxyl, halogen, and cyano;

$R^5$ is selected from the group consisting of hydrogen, deuterium, R'-NH-C(=O)-, C1-C6 alkyl, 4- to 8-membered saturated heterocyclyl, and C3-C7 cycloalkyl; wherein the C1-C6 alkyl, 4- to 8-membered saturated heterocyclyl, C3-C7 cycloalkyl are optionally substituted with 0, 1, 2, 3 substituents selected from the group consisting of deuterium, halogen, hydroxyl, amino, cyano, C1-C3 alkyl, and phenyl;

R' is selected from the group consisting of hydrogen, deuterium, and C1-C6 alkyl.

**8.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

Ring B is selected from the group consisting of phenyl, and

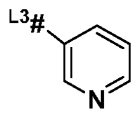

,

n is 2 or 3, each $R^B$ is independently selected from the group consisting of halogen, $R^3O$-, $R^hS(=O)_2$-,

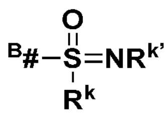

,

and

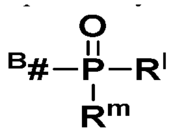

,

and each $R^B$ is different;

$R^3$ is selected from the group consisting of hydrogen, C1-C4 alkyl, C1-C4 haloalkyl, cyano-C1-C2 alkylene-, hydroxy-C1-C2 alkylene-, C1-C2 alkoxy-C1-C2 alkylene-, and C1-C2 alkyl-C(=O)-;

$R^h$ is connected to the ortho position of $R^hS(=O)_2$- to form a 5- to 6-membered heterocyclic ring;
$R^{k'}$ is hydrogen, $R^k$ is connected to the ortho position of

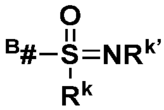

to form a 5- to 6-membered heterocyclic ring;
$R^l$ is C1-C6 alkyl (preferably C1-C3 alkyl), $R^m$ is connected to the ortho position of

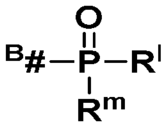

to form a 5- to 6-membered heterocyclic ring;
preferably, Ring B is selected from the group consisting of phenyl, and

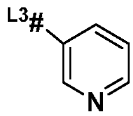

n is 2, one $R^B$ is $R^3O$-, and the other $R^B$ is selected from the group consisting of $R^hS(=O)_2$-,

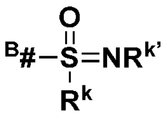 , and 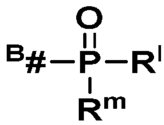 ;

or Ring B is selected from the group consisting of phenyl, and

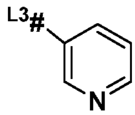 ,

n is 3, one $R^B$ is hydrogen or fluorine, one $R^B$ is $R^3O$-, and one $R^B$ is selected from the group consisting of $R^hS(=O)_2$-,

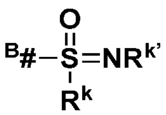 , and 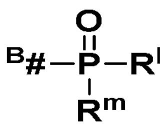 ;

preferably, $R^3$ is selected from the group consisting of hydrogen, methyl, ethyl, trifluoromethyl, monofluoromethyl, difluoromethyl, $CF_3CH_2$-, $CFH_2CH_2$-, $CNCH_2$-, $CNCH_2CH_2$-, $C(OH)H_2CH_2$-, $CH_3$0$CH_2CH_2$-, and $CH_3CH_2C(=O)$-;
more preferably, $R^3$ is methyl.

**9.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

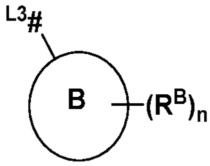

is selected from the group consisting of

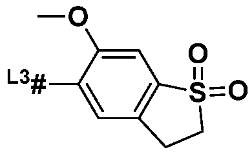

,

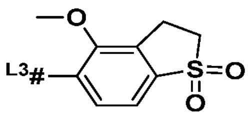 , 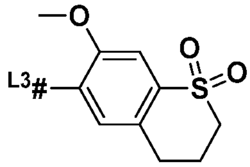 , 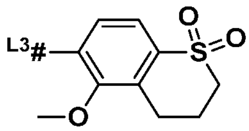 , 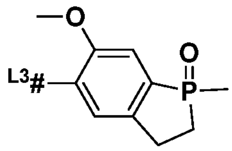 ,

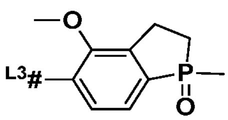 , 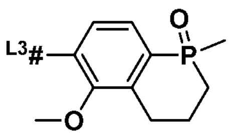 , 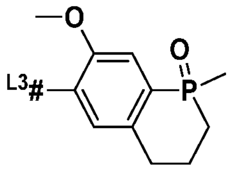 , 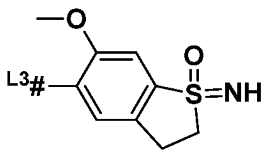 ,

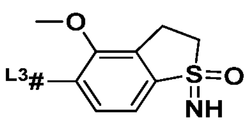 , 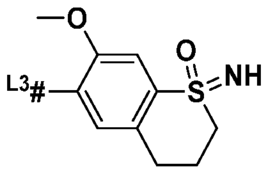 , and 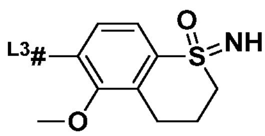 ;

preferably,

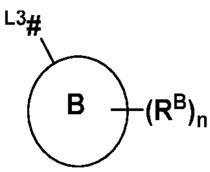

is selected from the group consisting of

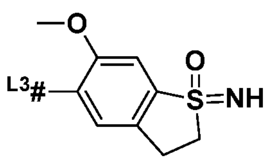

,

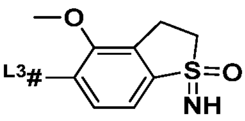, 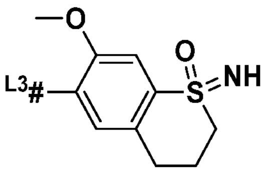, and 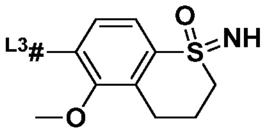.

**10.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 7, wherein,

$R^5$ is selected from the group consisting of hydrogen, R'-NH-C(=O)-, C1-C6 alkyl, 4- to 6-membered saturated heterocyclyl, and C3-C6 cycloalkyl, wherein the C1-C6 alkyl, 4- to 6-membered saturated heterocyclyl, C3-C6 cycloalkyl are optionally substituted with 0, 1, 2, 3 substituents selected from the group consisting of halogen, cyano, C1-C3 alkyl, and phenyl;
R' is C1-C3 alkyl;
preferably,

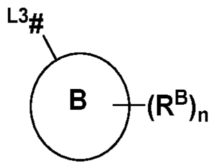

is selected from the group consisting of

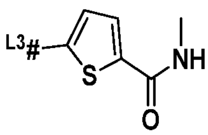,

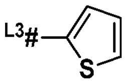, 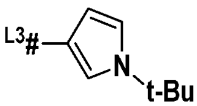, 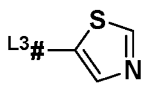, 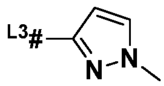, 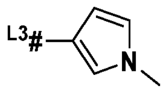, 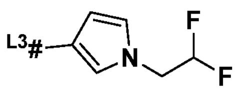,

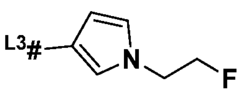, 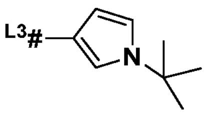, 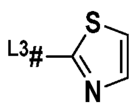, 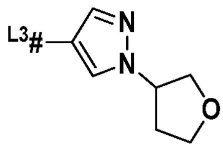, 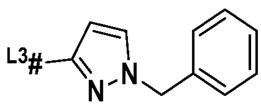,

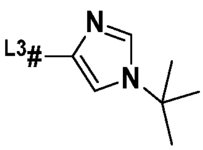, 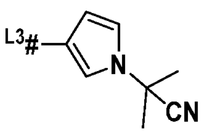, 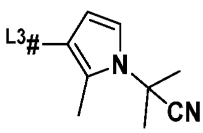, 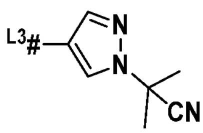, 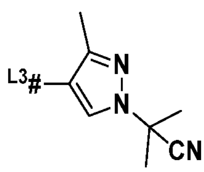,

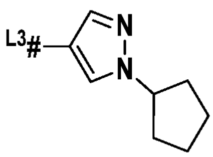, 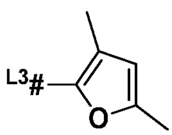, 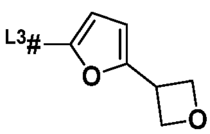, 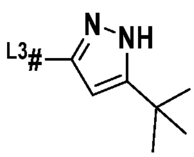, 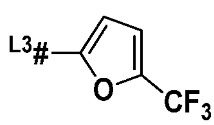,

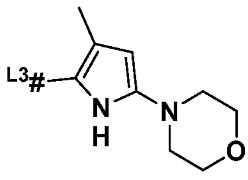, and 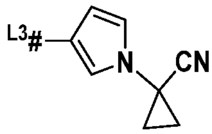 .

**11.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

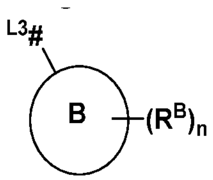 is 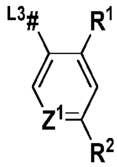;

$R^l$ is selected from the group consisting of hydroxyl, halogen, C1-C2 haloalkyl, and $R^3O$-;
$Z^1$ is selected from the group consisting of N, and $CR^4$;
$R^2$ is selected from the group consisting of cyano, halogen, C1-C6 alkyl, C1-C6 haloalkyl, hydroxyl-substituted C1-C6 alkyl, C1-C6 alkoxy-C1-C6 alkylene, cyano-C1-C6 alkylene, 4- to 8-membered saturated heterocyclyl-C1-C6 alkylene, $R^eR^fN$-C(=O)-, $R^gO$-C(=O)-, $R^hS(=O)_2$-, $R^iR^jN$-$S(=O)_2$-,

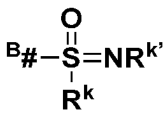, 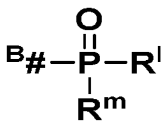,

and 5- to 6-membered heteroaryl;
$R^4$ is selected from the group consisting of hydrogen, deuterium, and fluorine;
preferably, $R^1$ is selected from the group consisting of hydroxyl, fluorine, trifluoromethyl, and $R^3O$-; $R^3$ is selected from the group consisting of hydrogen, methyl, ethyl, trifluoromethyl, difluoromethyl, monofluoromethyl, $CF_3CH_2$-, $CFH_2CH_2$-, $CNCH_2$-, $CNCH_2CH_2$-, $C(OH)H_2CH_2$-, $CH_3OCH_2CH_2$-, and $CH_3CH_2C(=O)$-;
$R^2$ is selected from the group consisting of cyano, halogen, C1-C2 haloalkyl, C1-C2 alkoxy-C1-C2 alkylene, cyano-C1-C4 alkylene, 6-membered saturated heterocyclyl-C1-C2 alkylene, $R^eR^fN$-C(=O)-, $R^gO$-C(=O)-, $R^hS(=O)_2$-, $R^iR^jN$-$S(=O)_2$-,

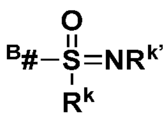, 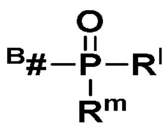,

and 5- to 6-membered heteroaryl;
$R^4$ selected from the group consisting of hydrogen, and fluorine;
more preferably, $R^1$ is methoxy;
$R^2$ is selected from the group consisting of cyano, halogen, C1-C2 haloalkyl, C1-C2 alkoxy-C1-C2 alkylene, cyano-C1-C4 alkylene, morpholinyl-C1-C2 alkylene, $R^eR^fN$-C(=O)-, $R^gO$-C(=O)-, $R^hS(=O)_2$-, $R^iR^iN$-$S(=O)_2$-,

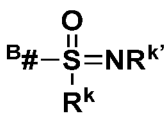, 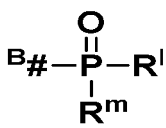,

oxazolyl, thiazolyl, and pyridyl;
most preferably, $R^1$ is methoxy; $R^2$ is selected from the group consisting of $CH_3NHC(=O)$-; $Z^1$ is selected from the

group consisting of CF and CH.

**12.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

$Z^2$ is $C(R^8)_2$;
one $R^8$ is hydrogen, and the other $R^8$ is selected from the group consisting of 7- to 11-membered spiro-heterocyclyl, 5- to 10-membered bridged heterocyclyl, and $R^oR^pN$-; $R^o$ and $R^p$ together with the N atom to which they commonly connect form a 7- to 11-membered spiro-heterocyclic ring or a 5- to 10-membered bridged heterocyclic ring; wherein the 7- to 11-membered spiro-heterocyclyl, 7- to 11-membered spiro-heterocyclic ring, 5- to 10-membered bridged heterocyclyl, 5- to 10-membered bridged heterocyclic ring are optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy; or, two $R^8$ groups together with the carbon atom to which they connect form a 4- to 7-membered heterocyclyl, wherein the 4- to 7-membered heterocyclyl is optionally substituted with halogen, hydroxyl, amino, cyano, C1-C6 alkyl, halogen-substituted C1-C6 alkyl, hydroxyl-substituted C1-C6 alkyl, amino-substituted C1-C6 alkyl, C1-C6 alkoxy, halogen-substituted C1-C6 alkoxy, hydroxyl-substituted C1-C6 alkoxy, or amino-substituted C1-C6 alkoxy;
preferably, the 7- to 11-membered spiro-heterocyclyl is selected from the group consisting of

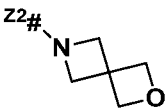, 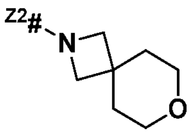, 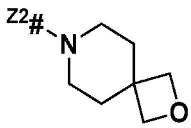, 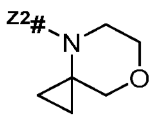, 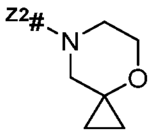, 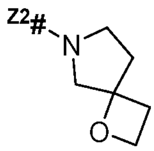,

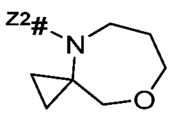, and 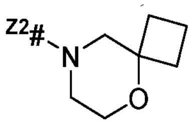;

the 5- to 10-membered bridged heterocyclyl is selected from the group consisting of

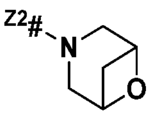, 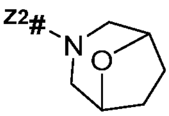, and 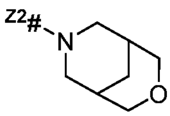;

wherein, $\#^{Z2}$ represents a connection point between $R^8$ and the 6-membered ring where $Z^2$ is located;
more preferably,

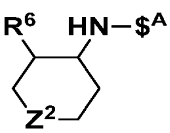

is selected from the group consisting of

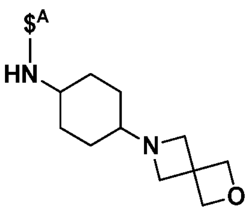, 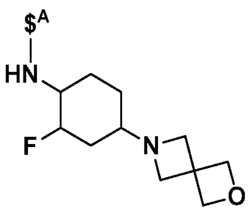, 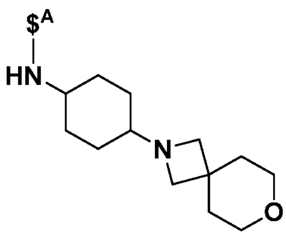, 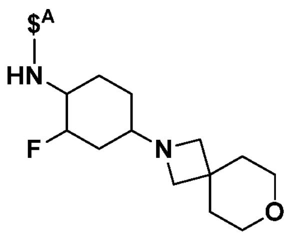,

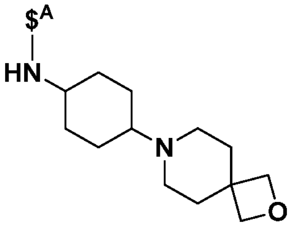, 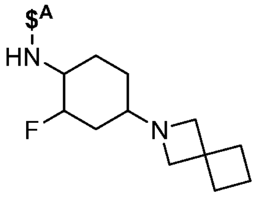, 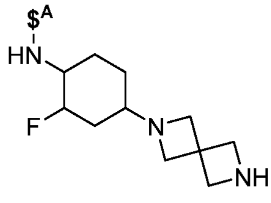, 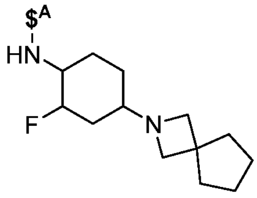,

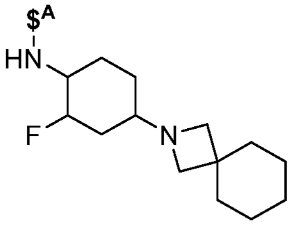, 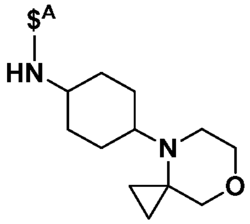, 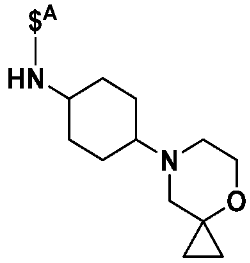, 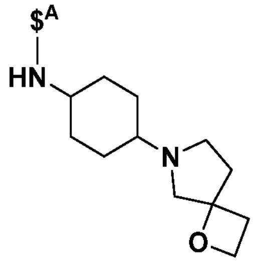,

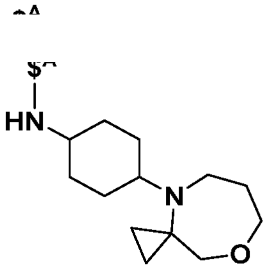, 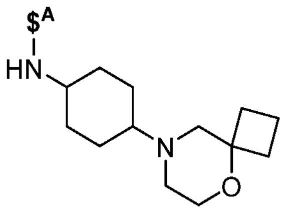, 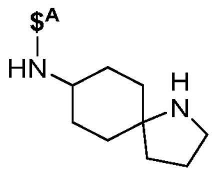, 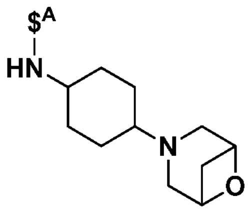,

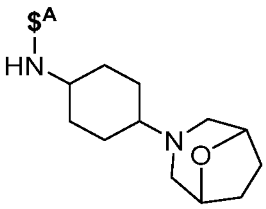, and 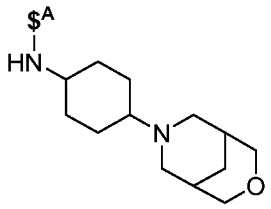;

wherein, $\$^A$ represents a connection point between NH and Ring A.

**13.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

$Z^2$ is $N(R^7)$;
$R^7$ is C1-C6 alkyl; wherein the C1-C6 alkyl is optionally substituted with hydroxyl, C1-C6 alkoxy, or alkoxy-substituted C1-C6 hydroxyl;
preferably, $R^7$ is selected from the group consisting of C1-C4 alkyl, C1-C4 alkyl substituted with 2 hydroxyl groups, and C1-C2 alkoxy-C1-C4 alkyl, and the C1-C2 alkoxy-C1-C4 alkyl is substituted with hydroxyl;
more preferably, $R^7$ is selected from the group consisting of methyl,

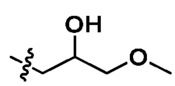,

and

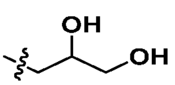.

**14.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein,

$R^6$ is selected from the group consisting of hydrogen and fluorine;
$R^a$ and $R^d$ are each independently selected from the group consisting of C1-C4 alkyl, C1-C2 haloalkyl, and cyano-C1-C2 alkylene;
each $R^b$ is independently selected from the group consisting of hydrogen, hydroxyl, halogen, and cyano;
each $R^c$ is independently selected from the group consisting of hydrogen and halogen;
preferably, $R^a$ and $R^d$ are each independently selected from the group consisting of $CF_3CH_2$-, $CH_2FCH_2$-, $CH_3CH_2CH_2$-, $CNCH_2$-, and $CHF_2CH_2$-;
each $R^b$ is independently selected from the group consisting of hydrogen and hydroxyl;
each $R^c$ is independently selected from the group consisting of hydrogen and fluorine.

**15.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably deuterated) compound or prodrug thereof according to claim 1, wherein, the compound is represented by Formula MI-1 or MI-2:

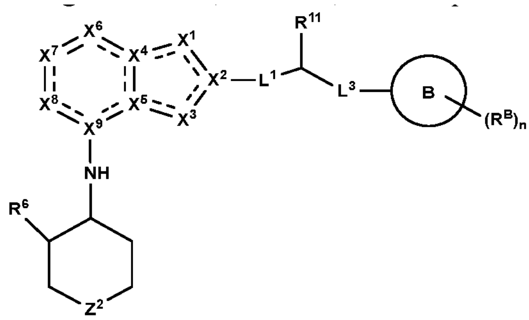

MI-1

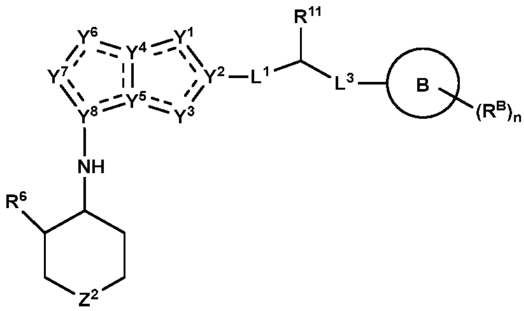

MI-2

$L^1$ is 5-membered heteroarylene;
preferably, $L^1$ is selected from the group consisting of

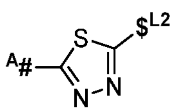 , 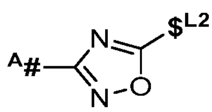 ,

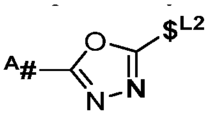 , 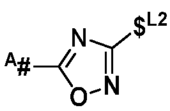 , 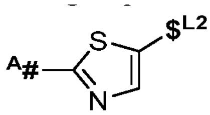 , 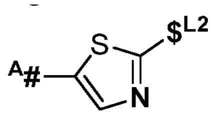 , and 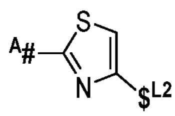 .

**16.** The compound, or enantiomer, diastereomer, racemate, tautomer, stereoisomer, geometric isomer, crystal form, N-oxide, metabolite thereof, or pharmaceutically acceptable salt, ester, solvate, hydrate, isotope-labeled (preferably

deuterated) compound or prodrug thereof according to claim 1, wherein the compound is represented by Formula MI-1-1, MI-1-1A, MI-1-1B, Formula MI-1-1-2, MI-1-1-2A, MI-1-1-2B, MI-1-1-1, MI-1-1-1A, MI-1-1-1B or Formula T:

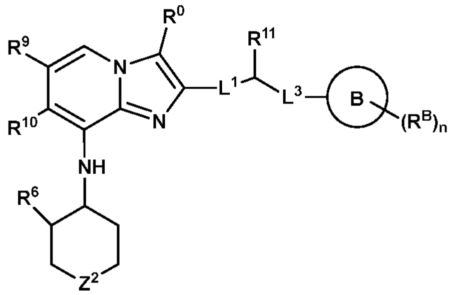

MI-1-1

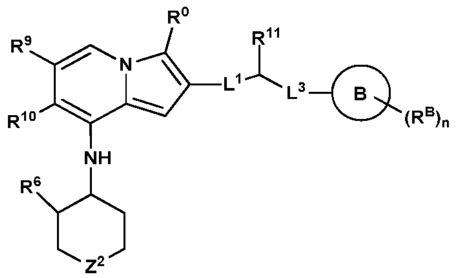

MI-1-1A

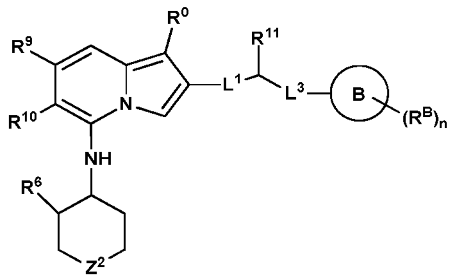

MI-1-1B

preferably, Ring B is selected from the group consisting of phenyl and 5-membered heteroaryl;
preferably, the compound is represented by Formula MI-1-1-2, MI-1-1-2A, MI-1-1-2B:

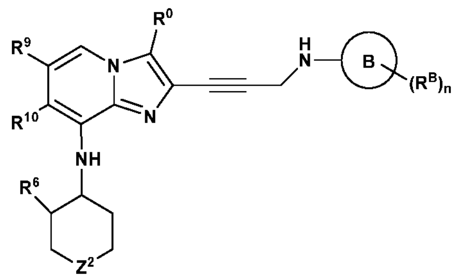

MI- 1-1-2

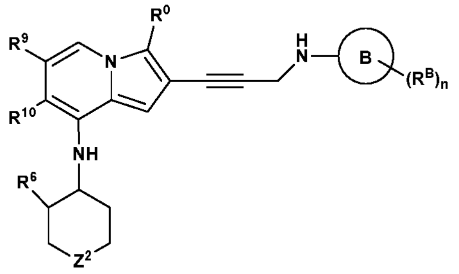

MI-1-1-2A

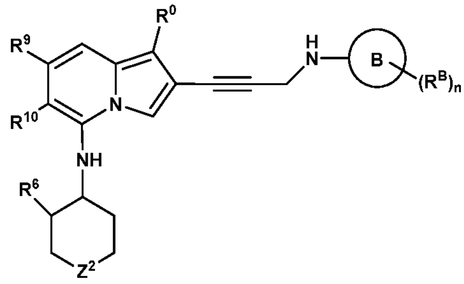

MI-1-1-2B

preferably, Ring B is selected from the group consisting of phenyl and 5-membered heteroaryl; preferably, the compound is represented by Formula MI-1-1-1, MI-1-1-1A, MI-1-1-1B:

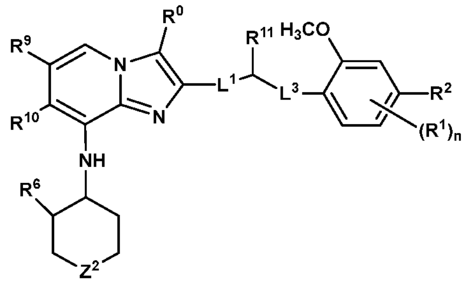

MI-1-1-1

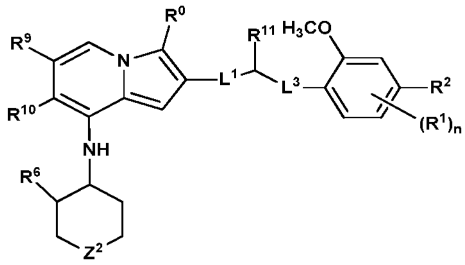

MI-1-1-1A

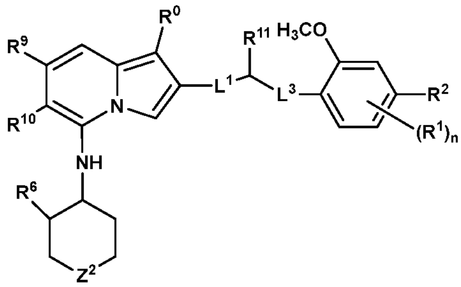

MI-1-1-1B

$R^1$ is halogen, preferably fluorine and chlorine; n is 0 or 1;

$R^2$ is selected from the group consisting of $R^hS(=O)_2$-,

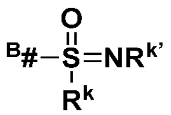

, and

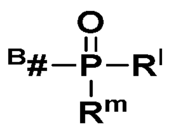

;

$R^h$ is connected to the ortho position of $R^hS(=O)_2$- to form a 5- to 6-membered heterocyclic ring;

$R^k$, $R^{k'}$ together with -S=N- to which they connect form a 5-7-membered heterocyclic ring, or $R^{k'}$ is hydrogen, $R^k$ is connected to the ortho position of

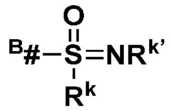

to form a 5- to 6-membered heterocyclic ring;

$R^l$ is C1-C6 alkyl (preferably C1-C3 alkyl), $R^m$ is connected to the ortho position of

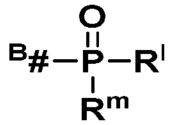

to form a 5- to 6-membered heterocyclic ring;

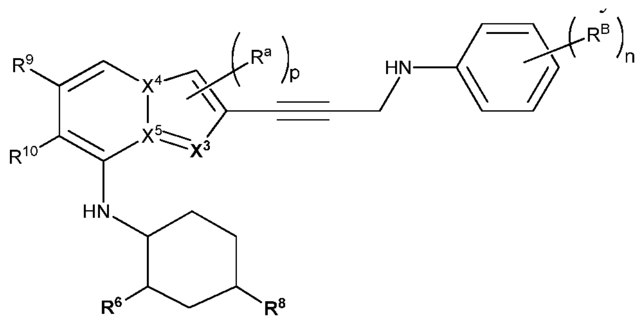

Formula T

in Formula T, $X^3$, $X^4$, $X^5$, $R^6$, $R^8$, $R^9$, $R^{10}$, $R^B$ $R^a$ are as defined in any one of claims 1 to 15, n, p are independently 0, 1, 2 or 3; or

in Formula T, $X^3$ is CH or N, $X^4$ is CH or N, $X^5$ is CH or N, preferably, $X^4$ and/or $X^3$ is N;

$R^B$ is independently selected from the group consisting of $-CONR_{1-1}R_{1-2}$, $-SO_2R_{1-3}$, -O-C1-C6 alkyl, -O-C2-C6 alkenyl, -O-C1-C6 deuterated alkyl, -O-C1-C6 haloalkyl, -O-C1-C6 alkoxy, -O-C3-C6 cycloalkyl, -O-C3-C6 halocycloalkyl, and -O-C3-C6 heterocycloalkyl; preferably, $R^B$ is selected from the group consisting of $-CONR_{1-1}R_{1-2}$, $-SO_2R_{1-3}$, and -O-C1-C6 deuterated alkyl;

$R_{1-1}$, $R_{1-2}$ are independently selected from the group consisting of H, deuterium, C1-C6 alkyl, -O-C1-C6 alkyl, -C1-C3 alkylene-O-C1-C3 alkyl, C1-C6 deuterated alkyl, C1-C6 haloalkyl, C3-C6 cycloalkyl, C3-C6 halocycloalkyl, 3- to 6-membered heterocycloalkyl, and 3- to 6-membered halogenated heterocycloalkyl; preferably H or C1-C6 deuterated alkyl; or $R_{1-1}$, $R_{1-2}$ together with the N atom to which they connect form a 3- to 6-membered heterocycloalkyl, preferably, the 3-to 6-membered heterocycloalkyl is selected from the group consisting of

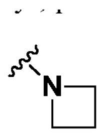, 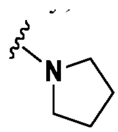,

and

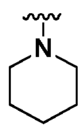;

$R_{1-3}$ is selected from the group consisting of H, C1-C6 alkyl, C1-C6 deuterated alkyl, C1-C6 haloalkyl, C3-C6 cycloalkyl, and C3-C6 halocycloalkyl;

$R^8$ is selected from the group consisting of $-NR_{2-1}R_{2-2}$, 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl; $R_{2-1}$, $R_{2-2}$ are independently C1-C6 alkyl or 4- to 7-membered monoheterocycloalkyl;

$R^6$ is independently selected from the group consisting of H, halogen (preferably fluorine), C1-C6 alkyl, and C1-C6 haloalkyl;

$R^a$ is independently selected from the group consisting of C1-C6 alkyl, C1-C6 haloalkyl, cyano-C1-C6 alkylene-, C3-C6 cycloalkyl, and C3-C6 halocycloalkyl; preferably, $R^a$ is selected from the group consisting of C1-C4 alkyl, C1-C2 haloalkyl, and cyano-C1-C2 alkylene-; more preferably, $R^4$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;

$R^9$ and $R^{10}$ are each independently selected from the group consisting of hydrogen, deuterium, hydroxyl, amino, halogen, cyano, and C1-C3 alkyl;

preferably, in Formula T,

$R^B$ is independently selected from the group consisting of -$CONR_{1-1}R_{1-2}$, -$SO_2R_{1-3}$, -O-C1-C6 alkyl, -O-C1-C6 deuterated alkyl, -O-C1-C6 haloalkyl, -O-C3-C6 cycloalkyl, and -O-C3-C6 heterocycloalkyl;

$R_{1-1}$, $R_{1-2}$ are independently selected from the group consisting of H, C1-C6 alkyl, -C1-C3 alkyl-O-C1-C3 alkyl, -O-C1-C6 alkyl, C1-C6 deuterated alkyl, C1-C6 haloalkyl, C3-C6 cycloalkyl, and C3-C6 halocycloalkyl, or $R_{1-1}$, $R_{1-2}$ together with the N atom to which they connect form a 3- to 6-membered heterocycloalkyl, preferably, the 3- to 6-membered heterocycloalkyl is selected from the group consisting of

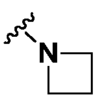, 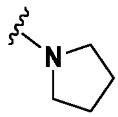, and 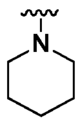;

$R_{1-3}$ is selected from the group consisting of H, and C1-C6 alkyl;

$R^I$ is selected from the group consisting of 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen, C1-C6 alkyl, or C1-C6 haloalkyl;

$R^6$ is independently selected from the group consisting of H, halogen (preferably fluorine), C1-C6 alkyl, and C1-C6 haloalkyl;

$R^a$ is independently selected from the group consisting of C1-C6 alkyl, and C1-C6 haloalkyl; preferably, $R^a$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-;

$R^9$ and $R^{10}$ are each independently selected from the group consisting of hydrogen and deuterium;

preferably, in Formula T,

$R^B$ is independently selected from the group consisting of -$CONR_{1-1}R_{1-2}$, -$SO_2R_{1-3}$, -O-C1-C6 alkyl, -O-C1-C6 deuterated alkyl, -O-C1-C6 haloalkyl, -O-C3-C6 cycloalkyl, and -O-C3-C6 halocycloalkyl; preferably, $R^1$ is selected from the group consisting of -$CONR_{1-1}R_{1-2}$, -$SO_2R_{1-3}$, and O-C1-C6 deuterated alkyl;

$R_{1-1}$ and $R_{1-2}$ are independently selected from the group consisting of H, C1-C6 alkyl, -C1-C3 alkyl-O-C1-C3 alkyl, C1-C6 deuterated alkyl, C1-C6 haloalkyl, C3-C6 cycloalkyl, C3-C6 halocycloalkyl, 3- to 6-membered heterocycloalkyl, and 3- to 6-membered halogenated heterocycloalkyl; preferably H or C1-C6 deuterated alkyl; or $R_{1-1}$, $R_{1-2}$ together with the N atom to which they connect form a 3- to 6-membered heterocycloalkyl, preferably, the 3- to 6-membered heterocycloalkyl is selected from the group consisting of

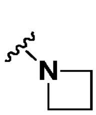, 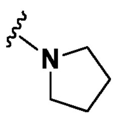,

and

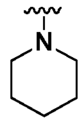;

$R_{1-3}$ is selected from the group consisting of H, C1-C6 alkyl, C1-C6 deuterated alkyl, C1-C6 haloalkyl, C3-C6 cycloalkyl, and C3 -C6 halocycloalkyl;

$R^8$ is selected from the group consisting of 7- to 11-membered spiro-heterocyclyl, 5- to 10-membered bridged heterocycloalkyl, 5- to 10-membered bridged cycloalkyl, 5- to 10-membered fused heterocycloalkyl, and 4- to 7-membered monoheterocycloalkyl, which are optionally substituted with halogen;

preferably, in Formula T,

$R^B$ is independently selected from the group consisting of -$CONR_{1-1}R_{1-2}$, -$SO_2R_{1-3}$, -O-C1-C3 alkyl, -O-C1-C3 deuterated alkyl, -O-C1-C3 haloalkyl, and -O-C3-C6 cycloalkyl; preferably, $R^1$ is selected from the group consisting of -$CONR_{1-1}R_{1-2}$, -$SO_2R_{1-3}$, and O-C1-C3 deuterated alkyl;

$R_{1-1}$, $R_{1-2}$, $R_{1-3}$ are independently selected from the group consisting of H, C1-C3 alkyl, C1-C3 deuterated alkyl, C1-C3 haloalkyl, and C3-C6 cycloalkyl; preferably H or C1-C3 deuterated alkyl;

$R^6$ is independently selected from the group consisting of H, halogen, C1-C3 alkyl, and C1-C3 haloalkyl;

$R^a$ is independently selected from the group consisting of halogen, C1-C3 alkyl, and C1-C3 haloalkyl;

$R^9$ and $R^{10}$ are each independently selected from the group consisting of hydrogen and deuterium; preferably, the Formula T is selected from the group consisting of Formulas T-1 to T-9:

| | |
|---|---|
| 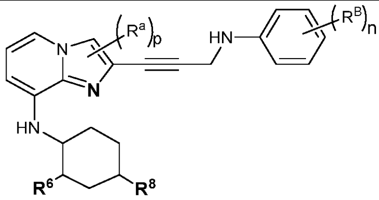 T-1 | 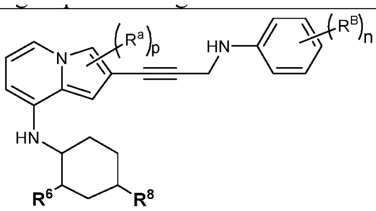 T-2 |

| | |
|---|---|
| 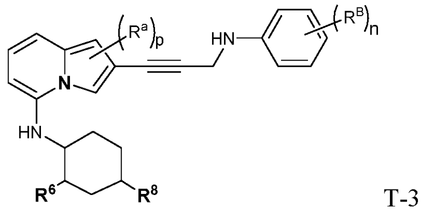 T-3 | 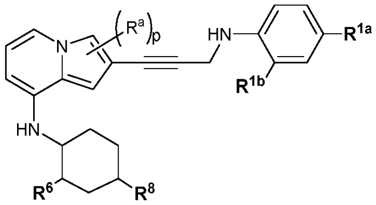 T-4 |
| 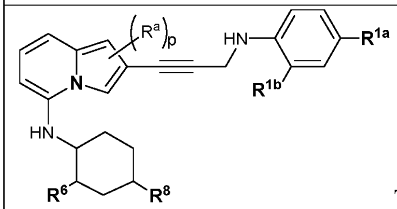 T-5 | 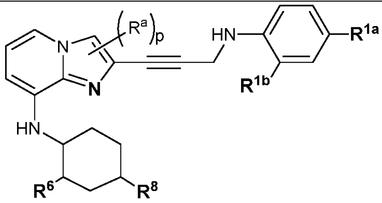 T-6 |
| 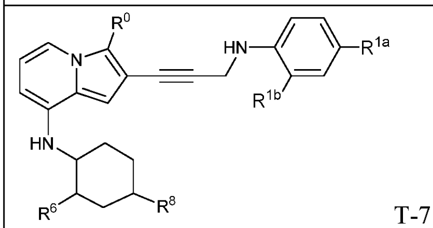 T-7 | 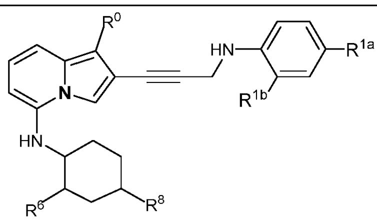 T-8 |
| 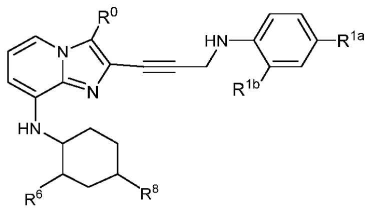 T-9 | |

in Formulas T-1 to T9, the definitions of each symbol are the same as those of Formula T; $R^{1a}$ and $R^{1b}$ are the same as those of $R^B$ in Formula T, and $R^{1a}$ and $R^{1b}$ are different;
preferably, $R^{1a}$ is selected from the group consisting of $-CONR_{1-1}R_{1-2}$, and $-SO_2R_{1-3}$; further preferably, $R^{1a}$ is selected from the group consisting of $-CONHCD_3$, $-CONHCH_3$, $-SO_2CH_3$, and $-SO_2CD_3$;
preferably, $R^{1b}$ is selected from the group consisting of -O-C1-C6 alkyl, -O-C1-C6 haloalkyl, -O-C1-C6 deuterated alkyl, and -O-C3-C6 cycloalkyl; further preferably, $R^{1b}$ is selected from the group consisting of $-OCH_3$, $-O\ CD_3$, $-OCH_2F$, and

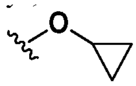 ;

preferably, $R^8$ is selected from the group consisting of

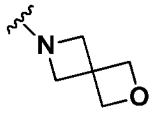 , 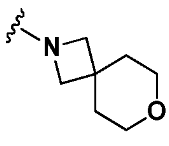 ,

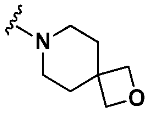 , 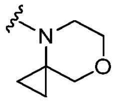 , 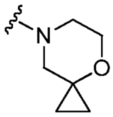 , 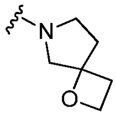 , 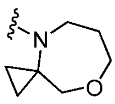 , 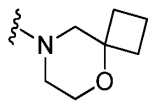 , 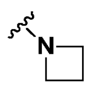 ,

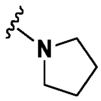, 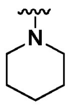, 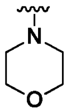, 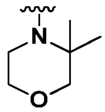, 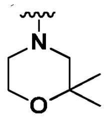, 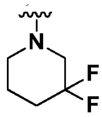, 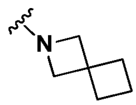, 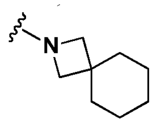,

and

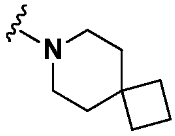;

further preferably, $R^8$ is selected from the group consisting of

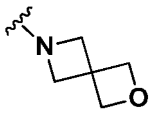, 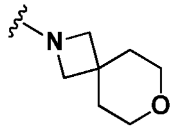,

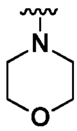, and 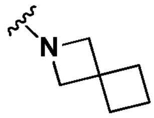;

preferably, $R^0$ is independently selected from the group consisting of C1-C6 alkyl, and C1-C6 haloalkyl; more preferably, $R^0$ is independently selected from the group consisting of halogen, C1-C3 alkyl, and C1-C3 haloalkyl; more preferably, $R^l$ is selected from the group consisting of $CF_3CH_2$-, $CH_2FCH_2$-, $CH_3CH_2CH_2$-, $CNCH_2$-, and $CHF_2CH_2$-; most preferably, $R^a$ is $CF_3CH_2$-; preferably, $R^6$ is halogen, further preferably fluorine.

**17.** The compound, or enantiomer, diastereomer, racemate, tautomer, stereoisomer, geometric isomer, crystalline form, N-oxide, metabolite thereof, or pharmaceutically acceptable salt, ester, solvate, hydrate, isotope-labeled (preferably deuterated) compound or prodrug thereof according to claim 1, wherein the compound is represented by Formula MII:

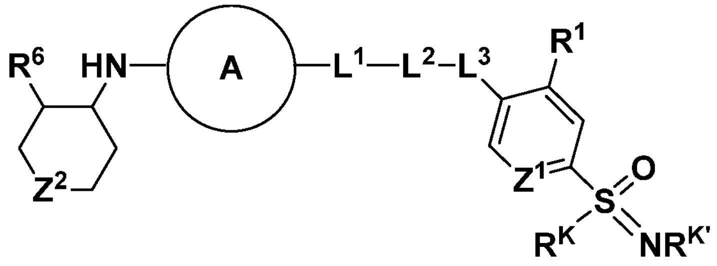
MII

$R^1$ is selected from the group consisting of hydrogen, deuterium, hydroxyl, halogen, amino, C1-C6 alkyl, and $R^3O$-; wherein the C1-C6 alkyl is optionally substituted with 1, 2 or 3 substituents selected from the group consisting of deuterium, halogen, hydroxyl, and amino;

$R^3$ is independently selected from the group consisting of hydrogen, deuterium, C1-C6 alkyl, and C1-C6 alkyl-C(=O)-; wherein the C1-C6 alkyl is optionally substituted with halogen, hydroxyl, cyano, amino, or C1-C6 alkoxy;

$Z^1$ is selected from the group consisting of N, and $CR^4$;

$R^4$ is selected from the group consisting of hydrogen, deuterium, hydroxyl, halogen, and cyano;

preferably, $R^k$, $R^{k'}$ together with -S=N- to which they connect form a 4- to 8-membered heterocyclic ring, or $R^k$ is connected to ring;

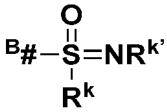

to form a 5- to 6-membered heterocyclic
preferably, the Ring A is selected from the group consisting of

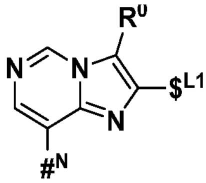

,

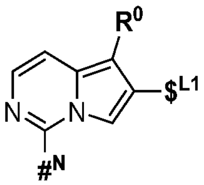 , 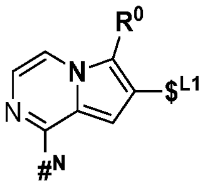 , 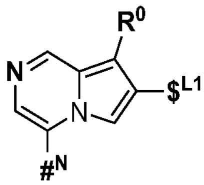 , 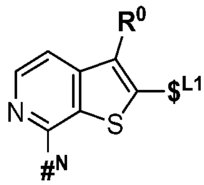 , 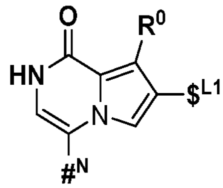 ,

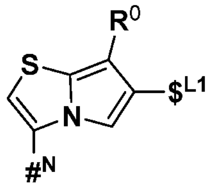 , 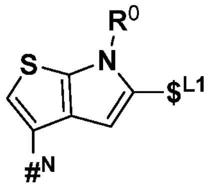 , 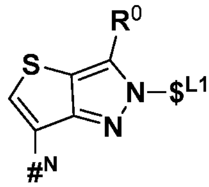 , 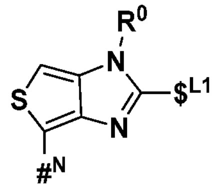 , 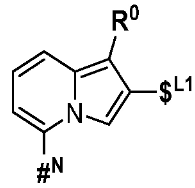 ,

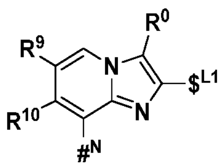 , 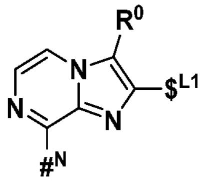 , 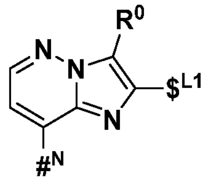 , 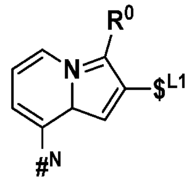 , and 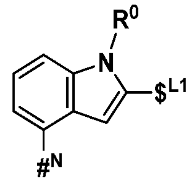 ;

$R^9$ and $R^{10}$ are each independently selected from the group consisting of hydrogen, deuterium, hydroxyl, amino, halogen, cyano, and C1-C3 alkyl;
preferably, $L^1$ is

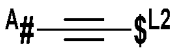

.

preferably, $L^3$ is NH.

**18.** The compound, or enantiomer, diastereomer, racemate, tautomer, stereoisomer, geometric isomer, crystalline form, N-oxide, metabolite thereof, or its pharmaceutically acceptable salt, ester, solvate, hydrate, isotope-labeled (preferably deuterated) compound or prodrug thereof according to claim 17, wherein the compound is represented by Formula MII-1, MII-2 or MII-3:

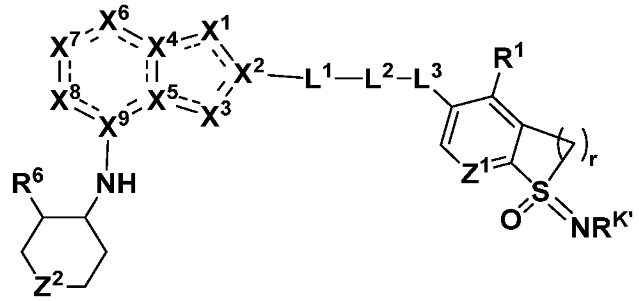

MII-1

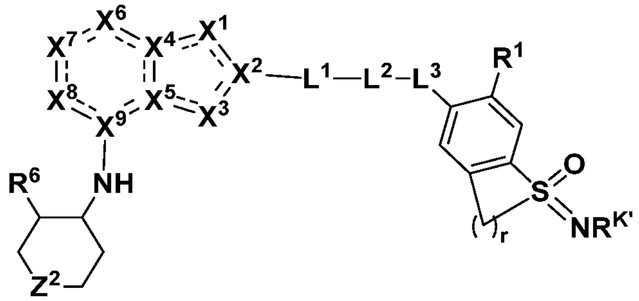

MII-2

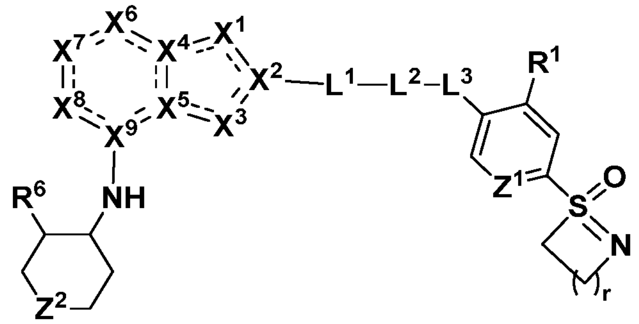

MII-3

r is selected from the group consisting of 1, 2, 3 and 4;
preferably, r is selected from the group consisting of 1, 2 and 3.

**19.** The compound, or enantiomer, diastereomer, racemate, tautomer, stereoisomer, geometric isomer, crystalline form, N-oxide, metabolite thereof, or its pharmaceutically acceptable salt, ester, solvate, hydrate, isotope-labeled (preferably deuterated) compound or prodrug thereof according to claim 18, wherein the compound is represented by Formula MII-1-1, MII-1-2, MII-2-1, MII-2-2, MII-3-1 or MII-3-2:

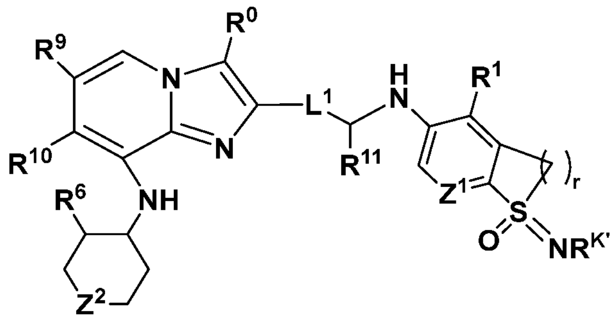

MII-1-1

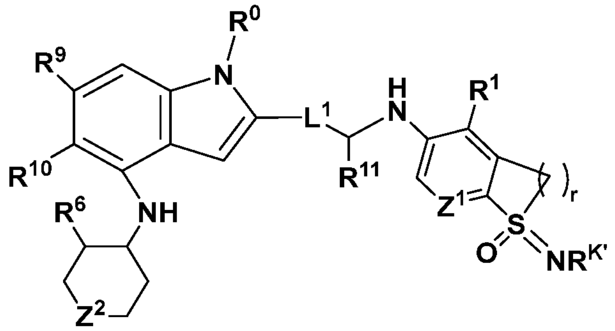
MII-1-2

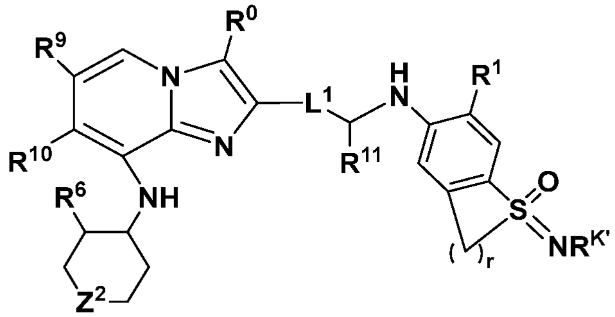
MII-2-1

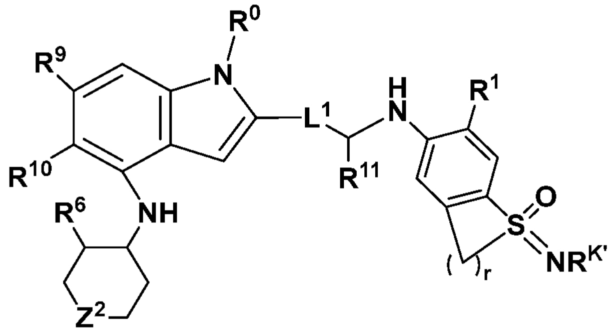
MII-2-2

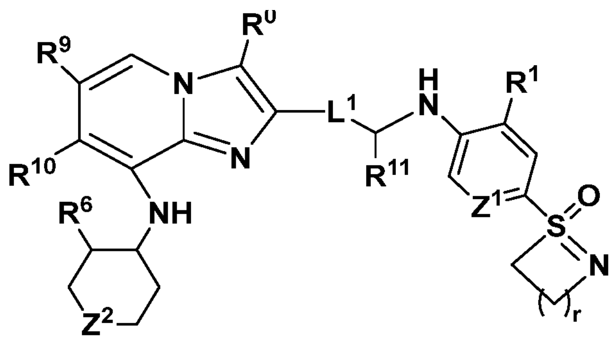
MII-3-1

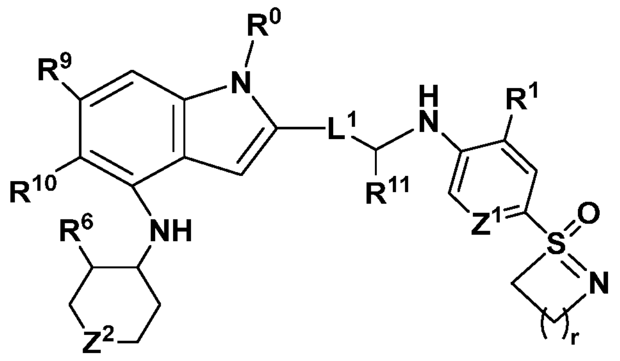

MII-3-2.

**20.** The compound, or enantiomer, diastereomer, racemate, tautomer, stereoisomer, geometric isomer, crystalline form, N-oxide, metabolite thereof, or pharmaceutically acceptable salt, ester, solvate, hydrate, isotope-labeled (preferably deuterated) compound or prodrug thereof according to claim 1, wherein the compound is selected from:

| Structural formula | Structural formula |
|---|---|
| 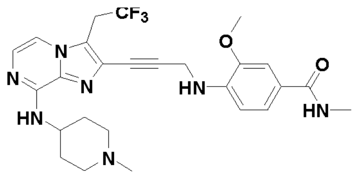 | 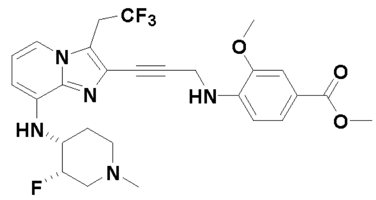 |
| 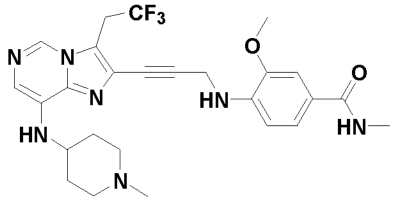 | 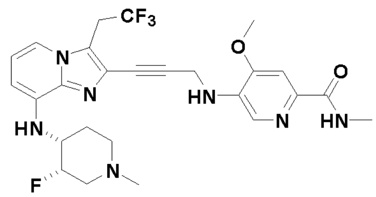 |
| 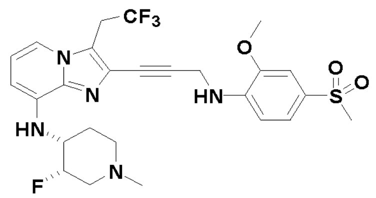 | 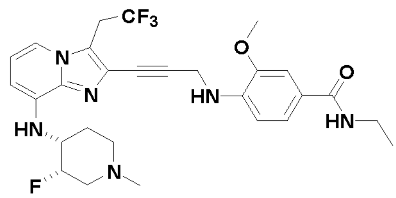 |
| 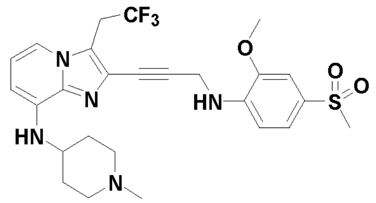 | 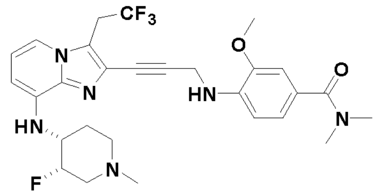 |
| 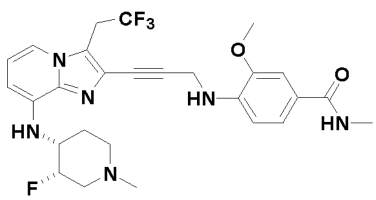 | 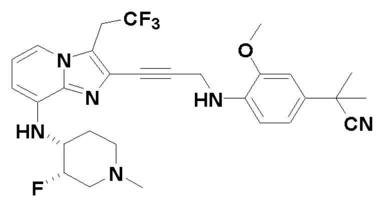 |

(continued)

| Structural formula | Structural formula |
|---|---|
| 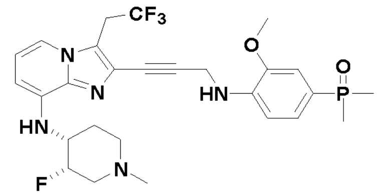 | 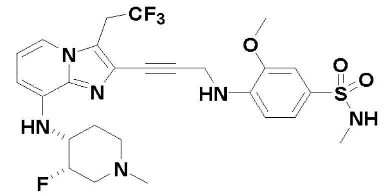 |
| 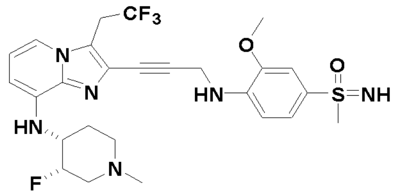 | 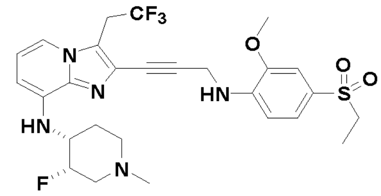 |
| 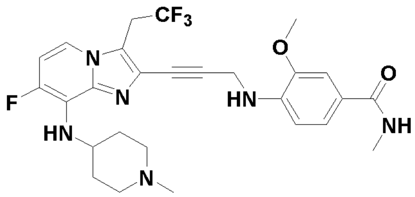 | 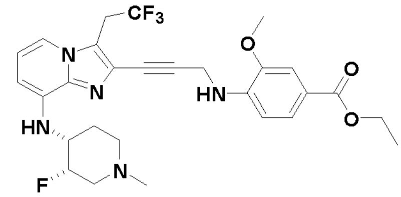 |
| 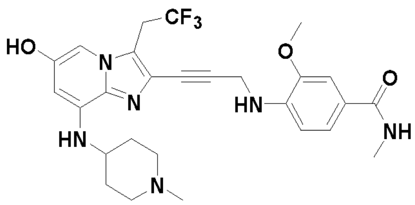 | 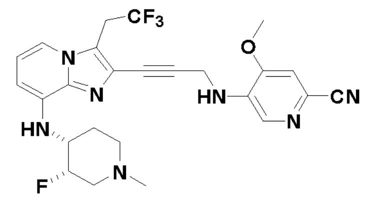 |
| 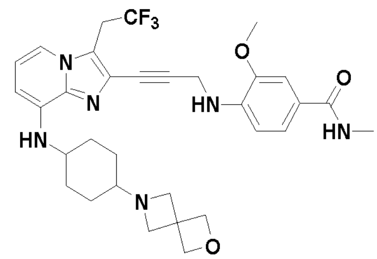 | 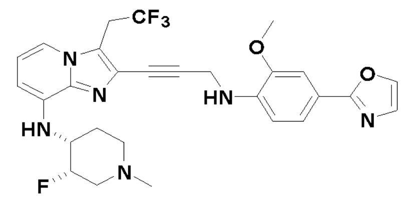 |
| 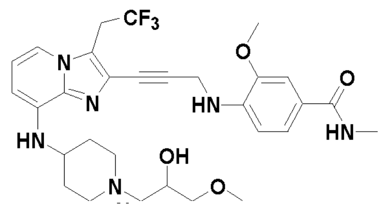 | 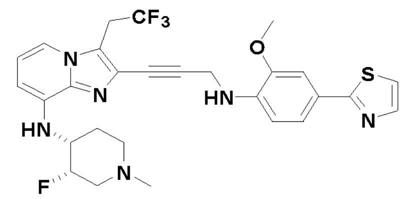 |
| 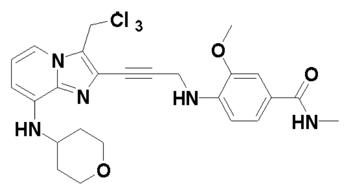 | 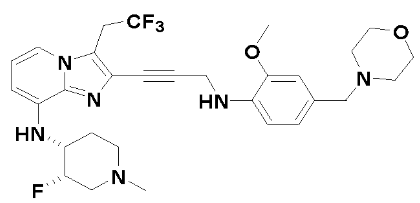 |

(continued)

| Structural formula | Structural formula |
|---|---|
| 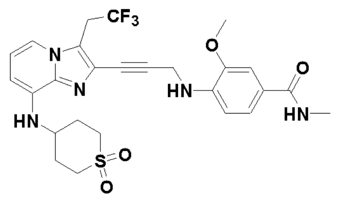 | 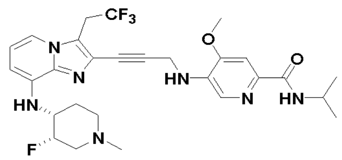 |
| 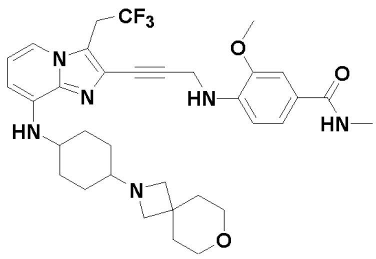 | 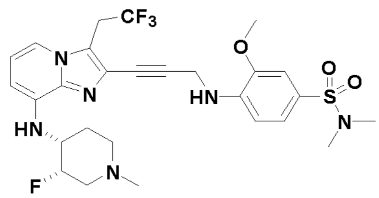 |
| 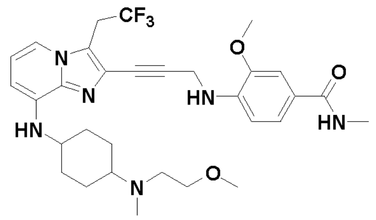 | 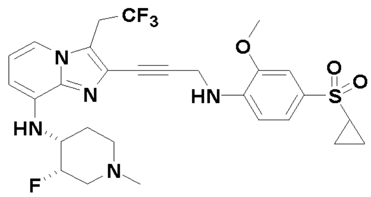 |
| 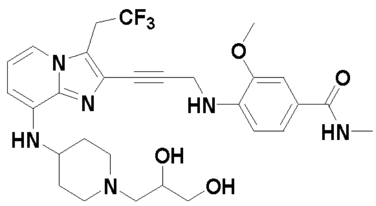 | 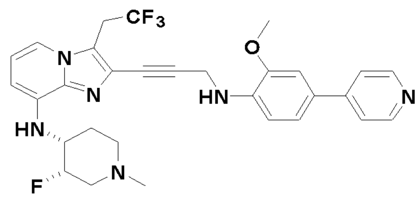 |
| 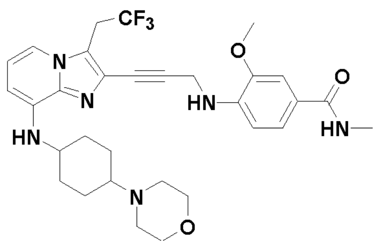 | 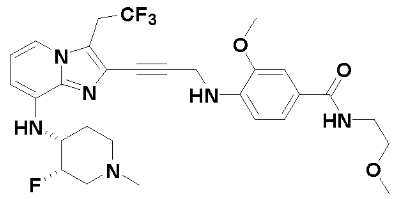 |
| 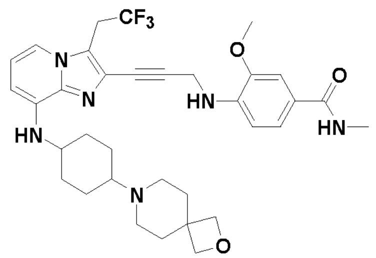 | 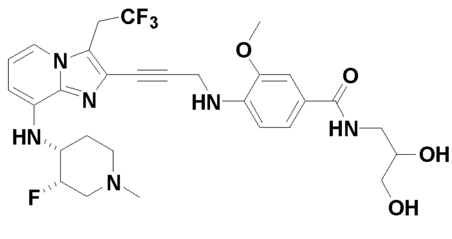 |
| 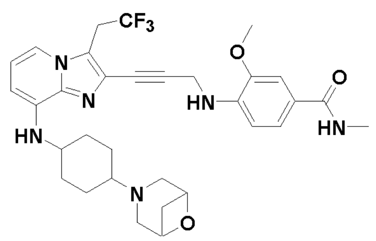 | 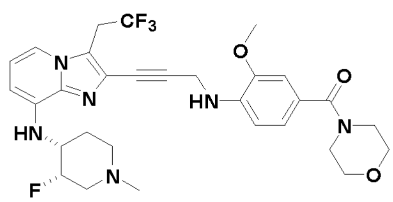 |

(continued)

| Structural formula | Structural formula |
|---|---|
| 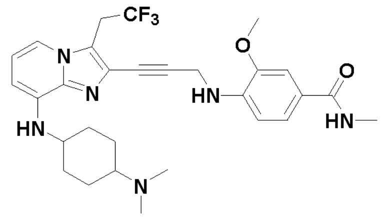 | 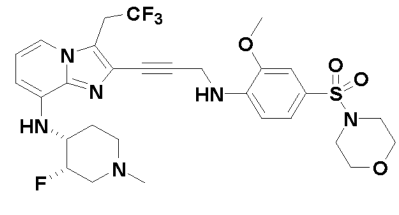 |
| 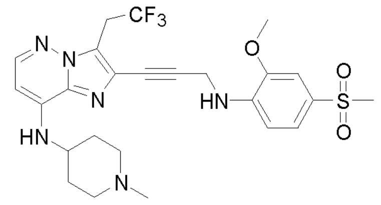 | 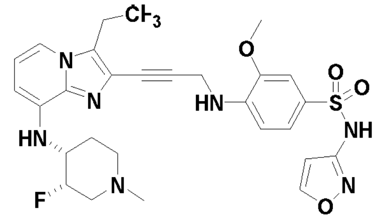 |
| 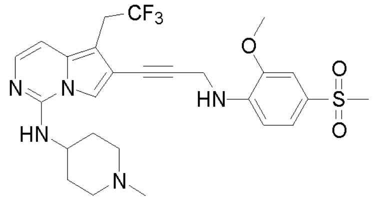 | 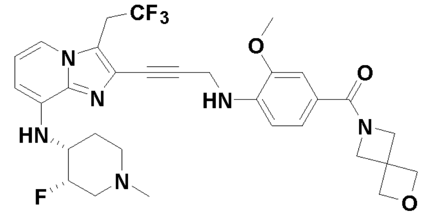 |
| 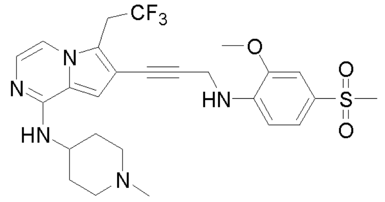 | 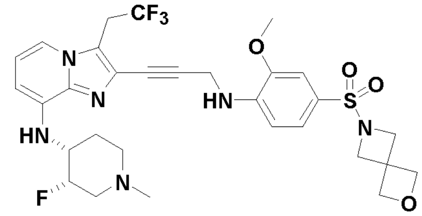 |
| 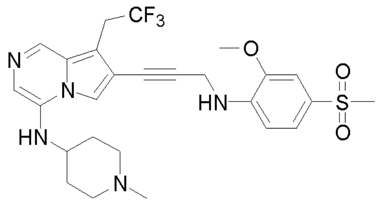 | 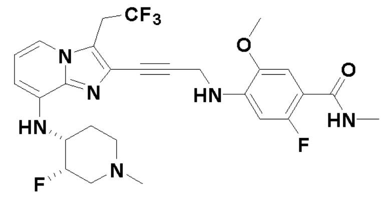 |
| 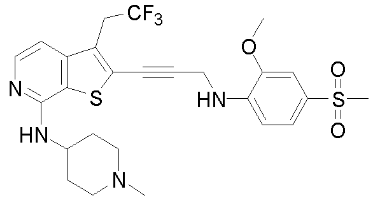 | 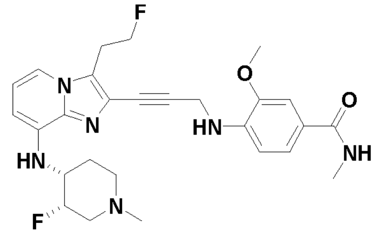 |
| 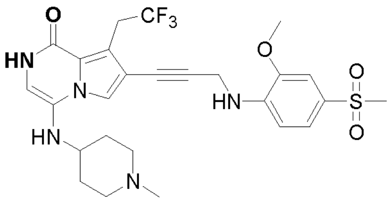 | 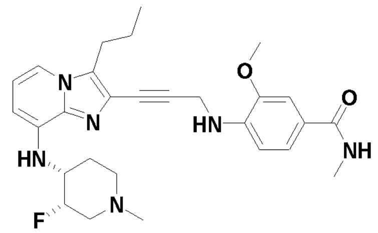 |

(continued)

| Structural formula | Structural formula |
|---|---|
| 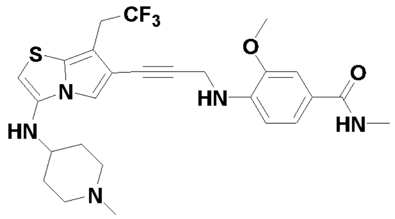 | 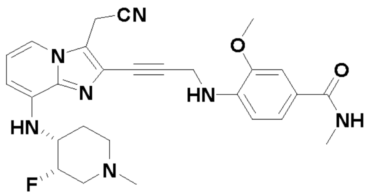 |
| 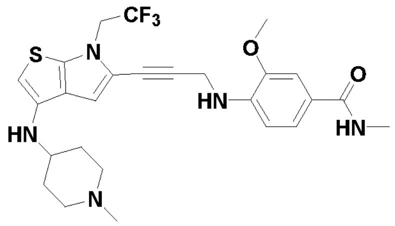 | 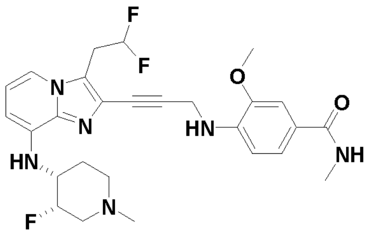 |
| 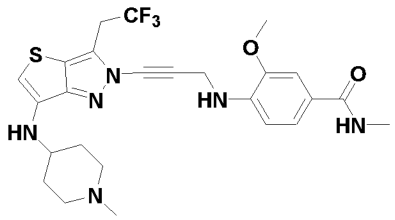 | 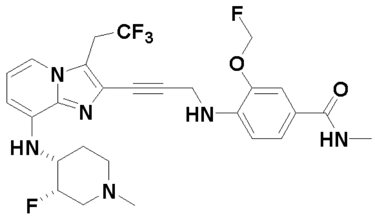 |
| 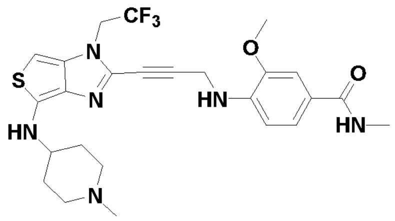 | 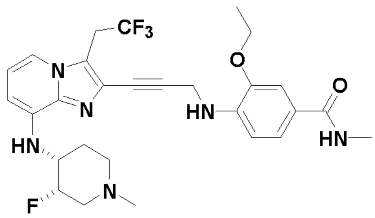 |
| 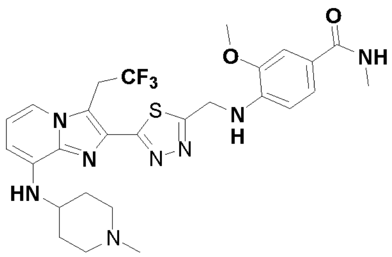 | 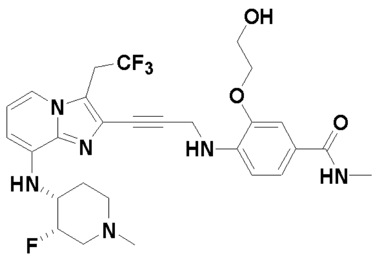 |
| 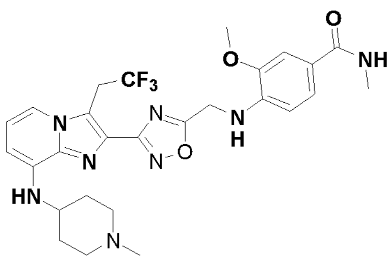 | 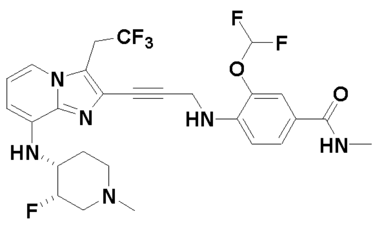 |

(continued)

| Structural formula | Structural formula |
|---|---|
| 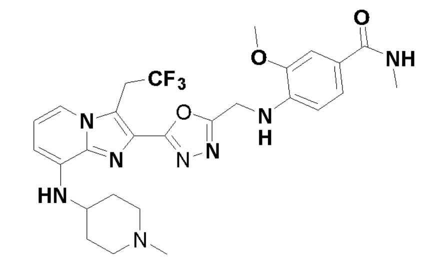 | 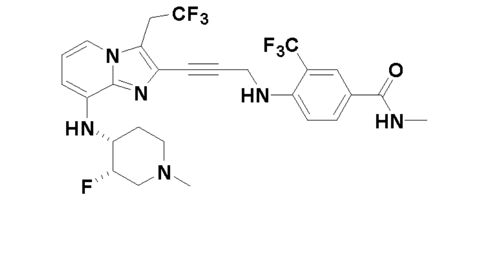 |
| 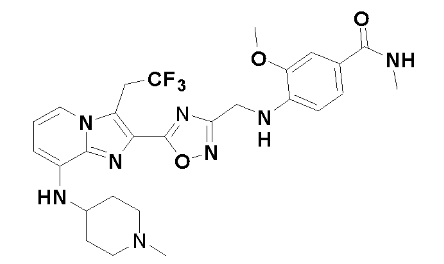 | 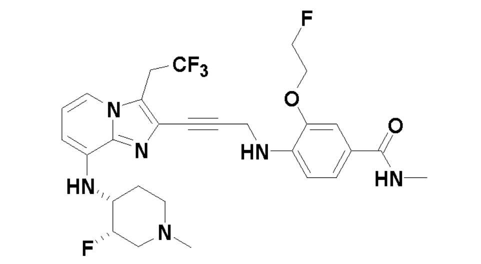 |
| 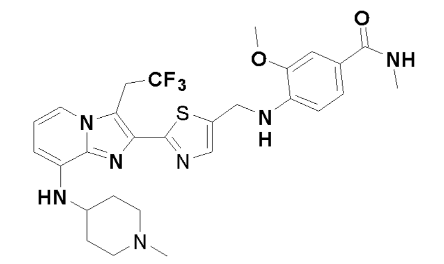 | 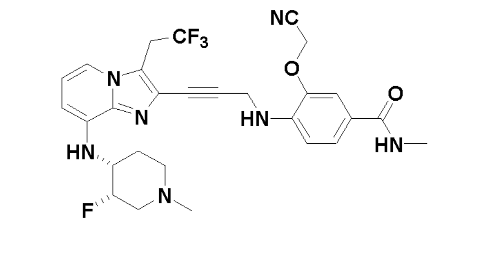 |
| 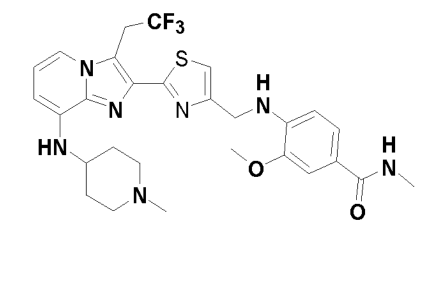 | 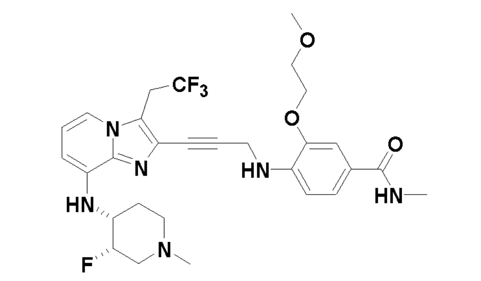 |
| 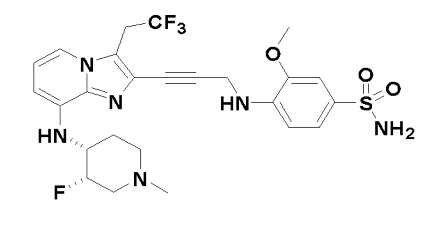 | 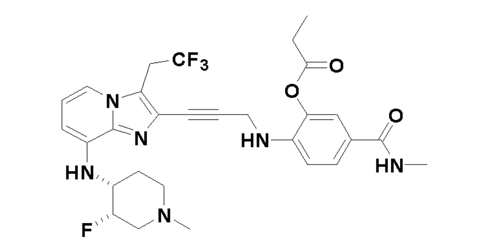 |
| 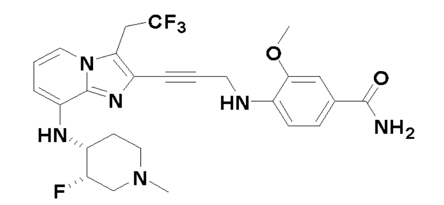 | 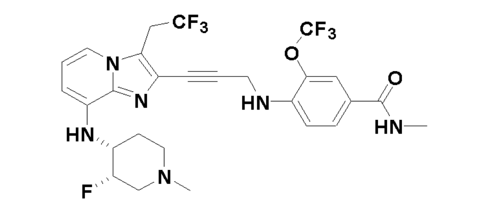 |

(continued)

| Structural formula | Structural formula |
|---|---|
| 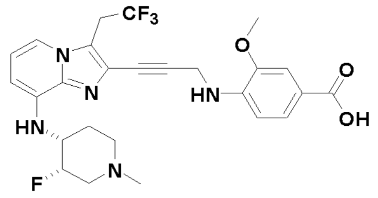 | 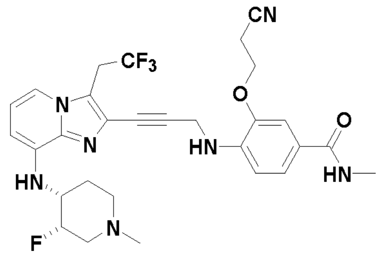 |
| 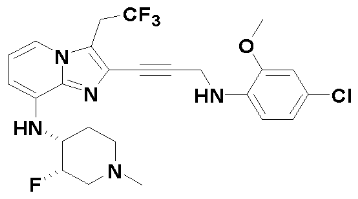 | 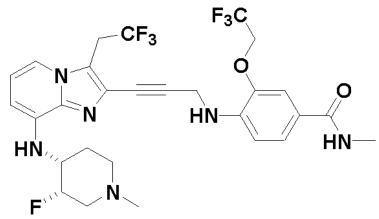 |
| 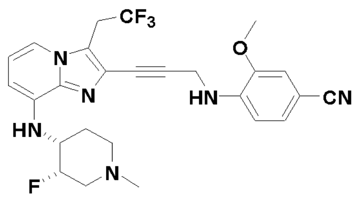 | 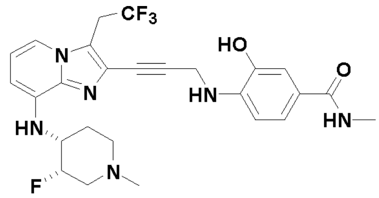 |
| 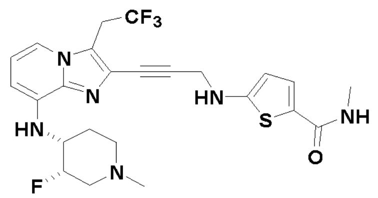 | 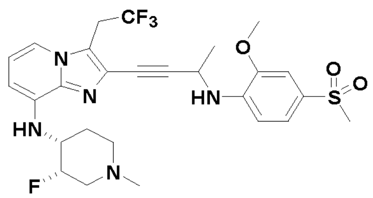 |
| 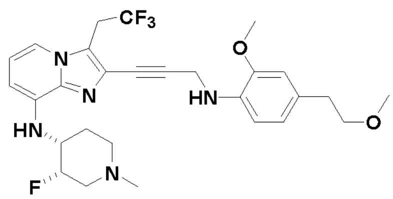 | 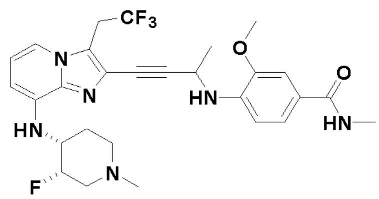 |
| 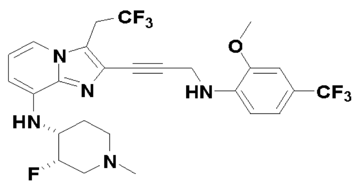 | |

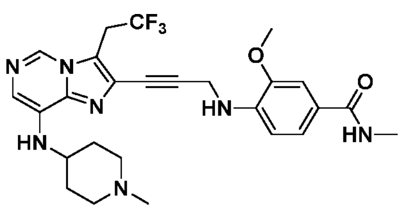 , 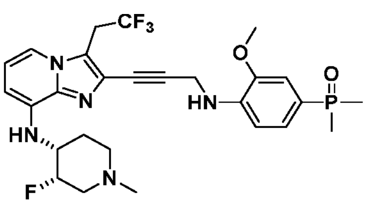 , 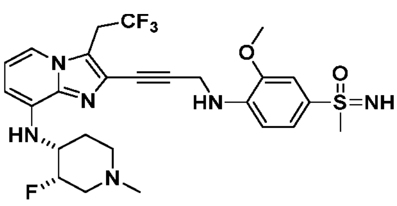 ,

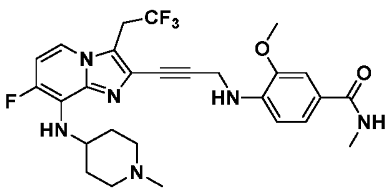,
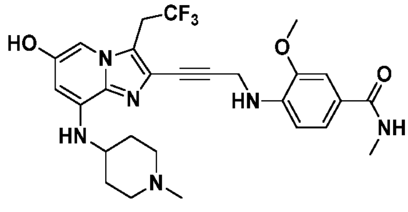,
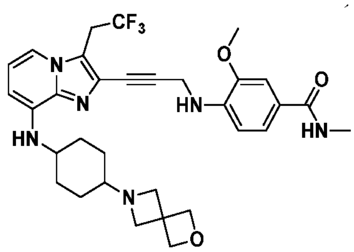,

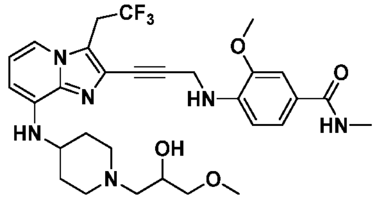,
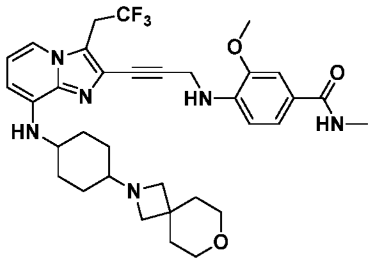,
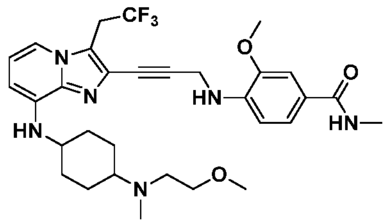,

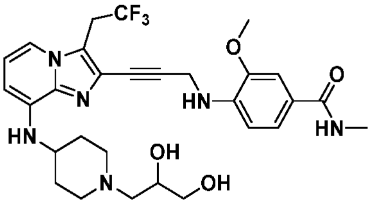,
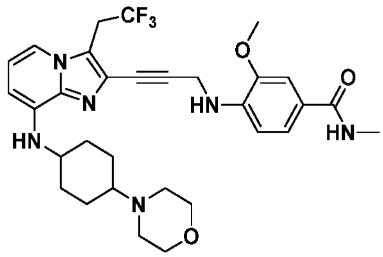,
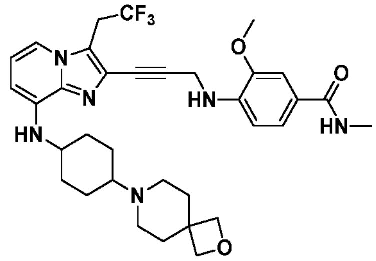,

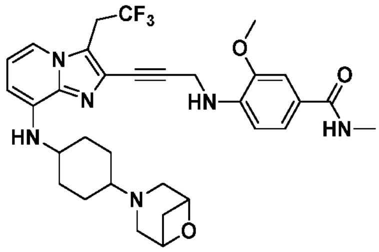,
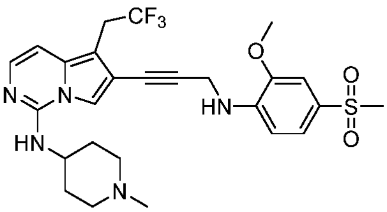,
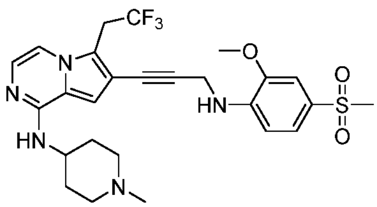,

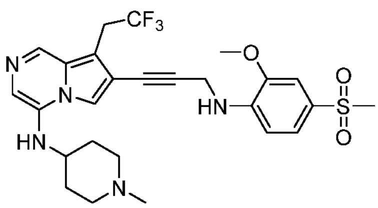,
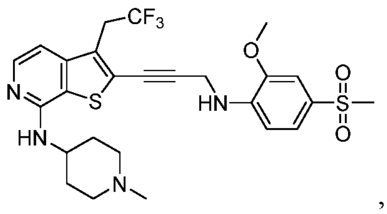,
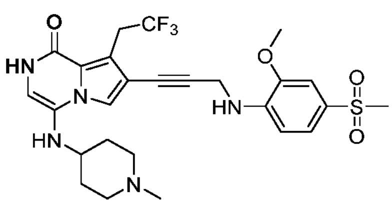,

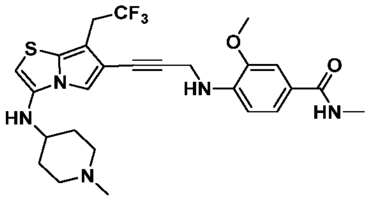,
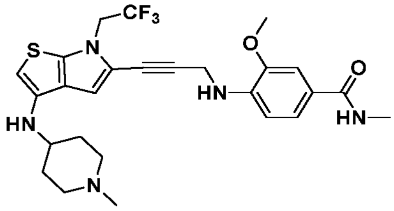,
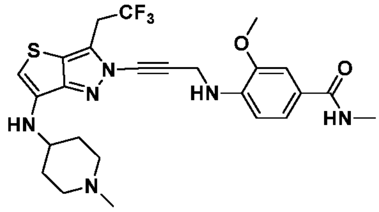,

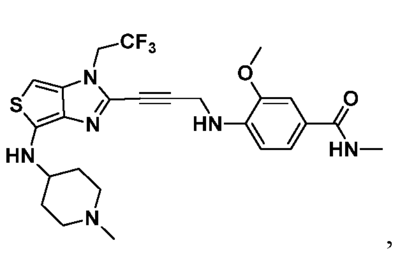 , 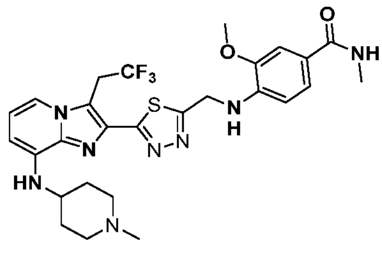 , 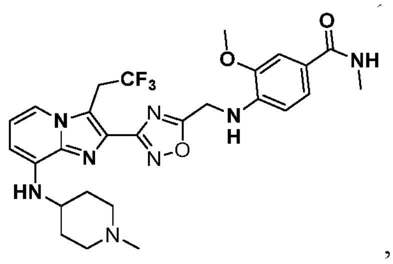 ,

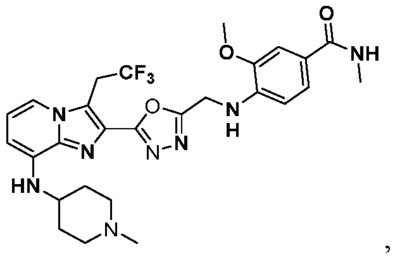 , 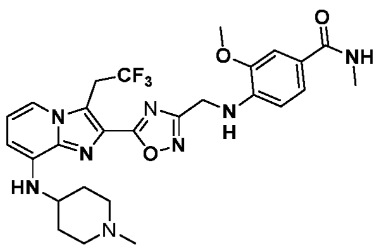 , 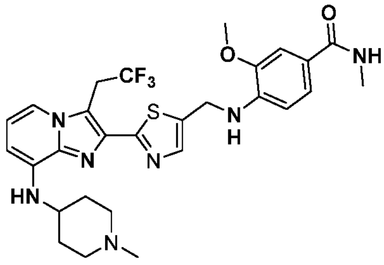 ,

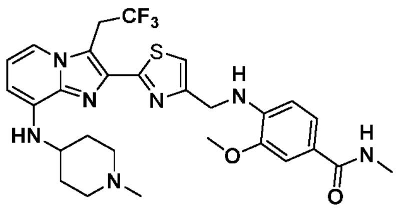 , 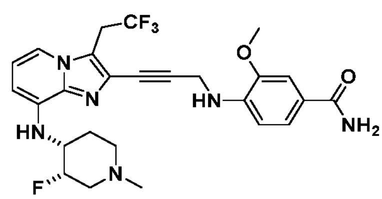 , 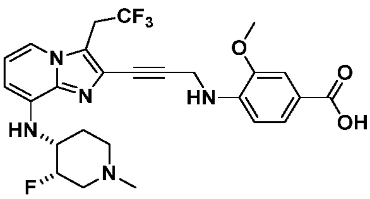 ,

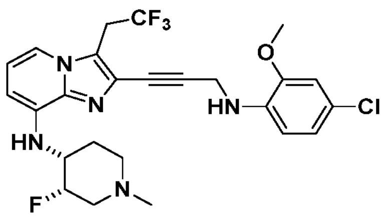 , 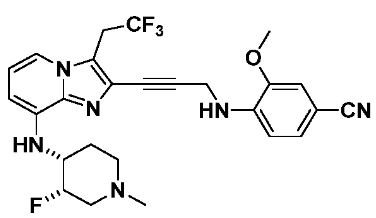 , 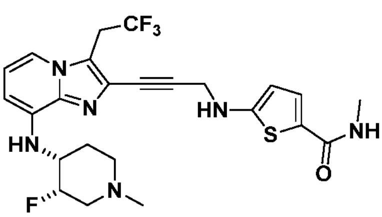 ,

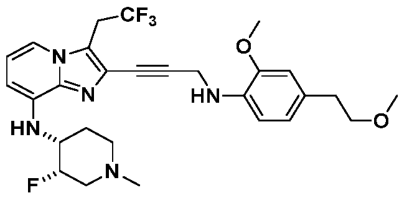 , 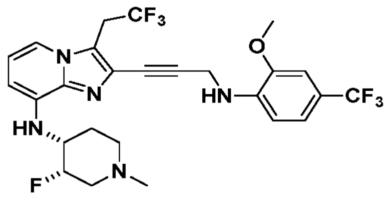 , 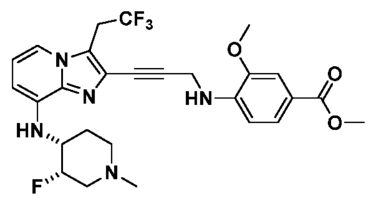 ,

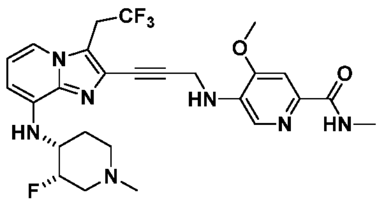 , 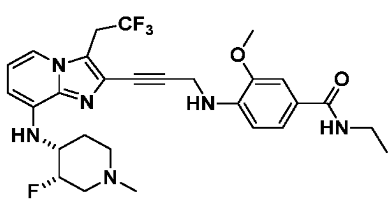 , 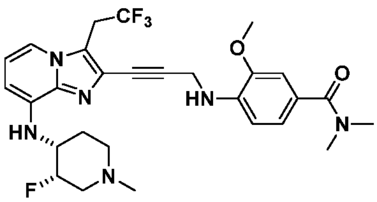 ,

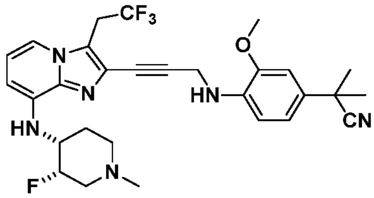,
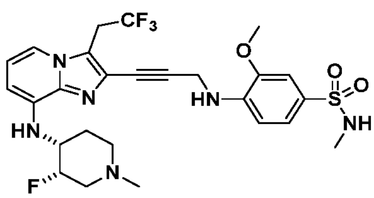,
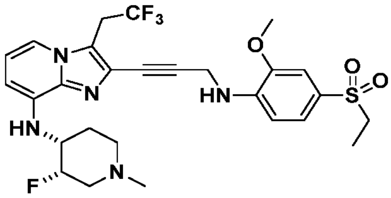,

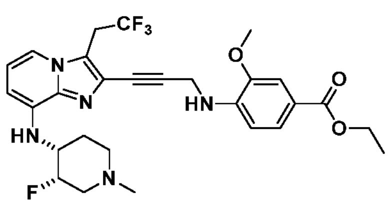,
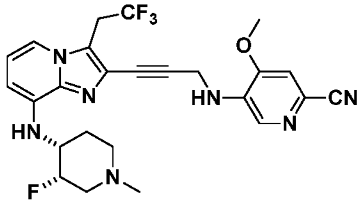,
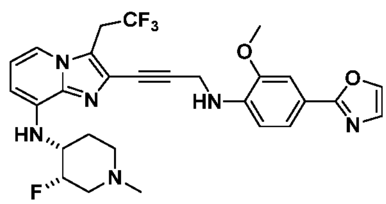,

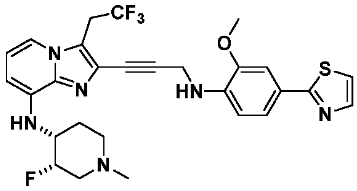,
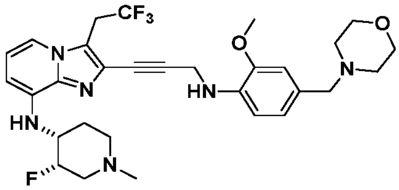,
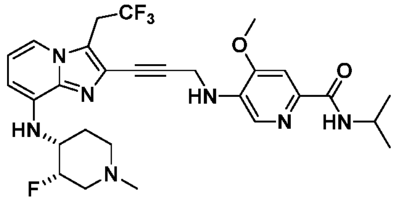,

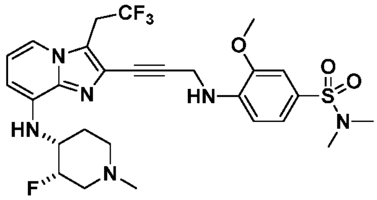,
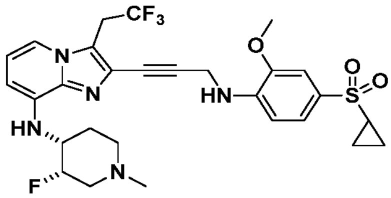,
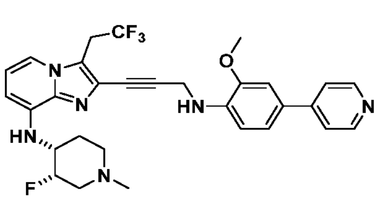,

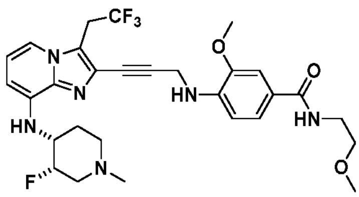,
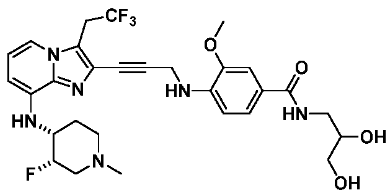,
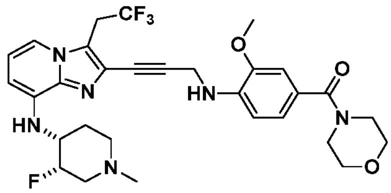,

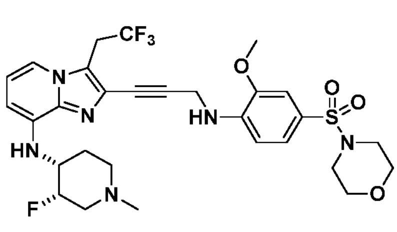,
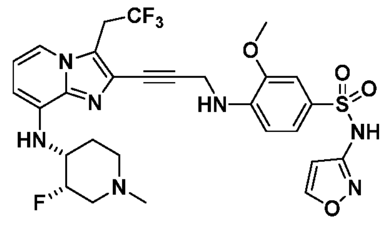,
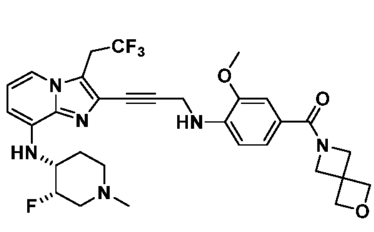,

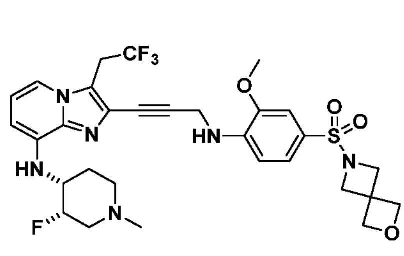,
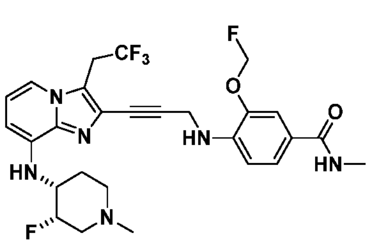,
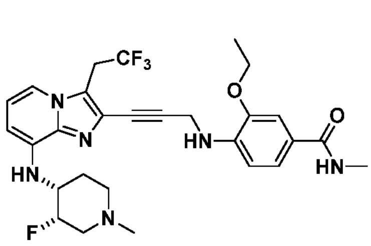,

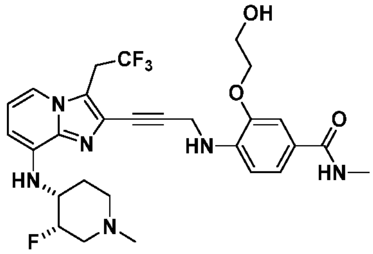,
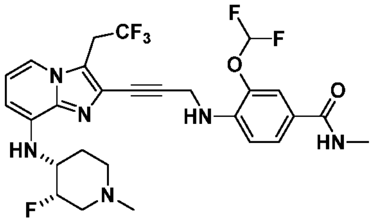,
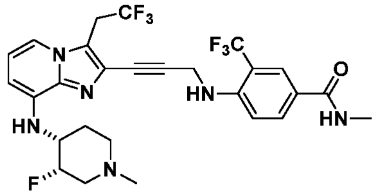,

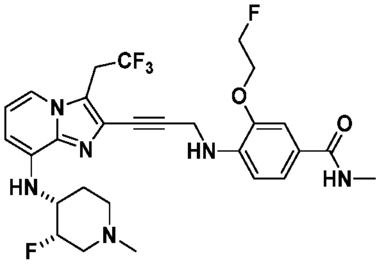,
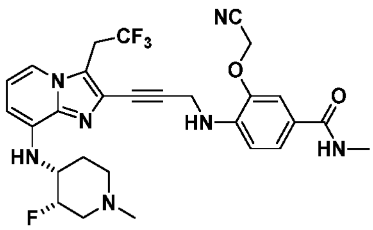,
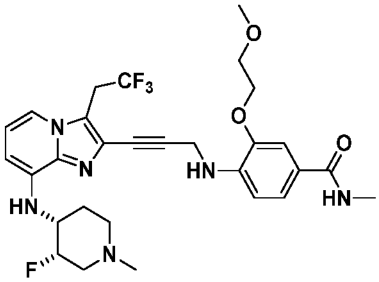,

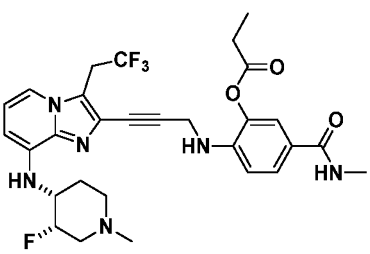,
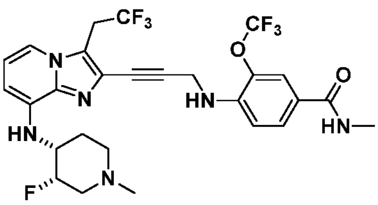,
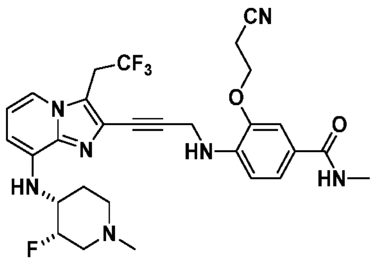,

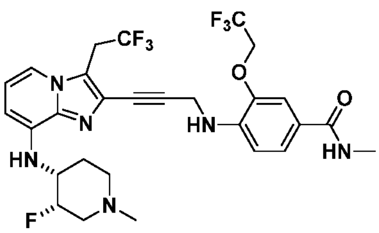,
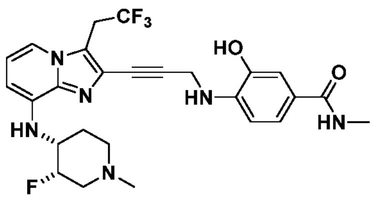,
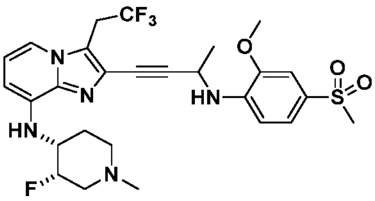,

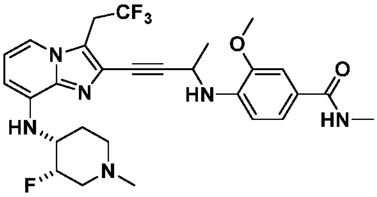,
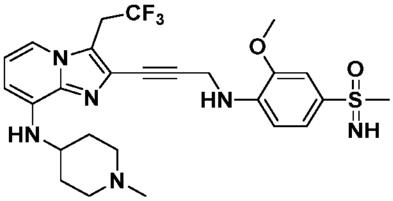,
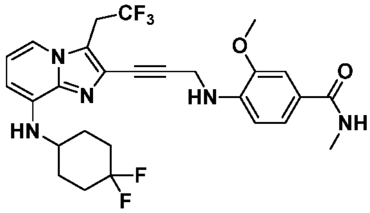,

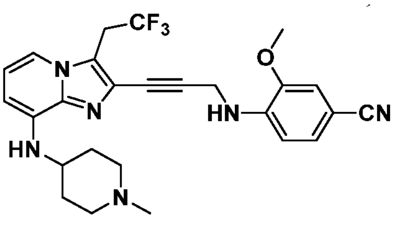,
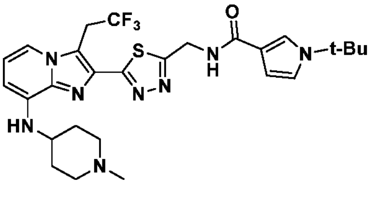,
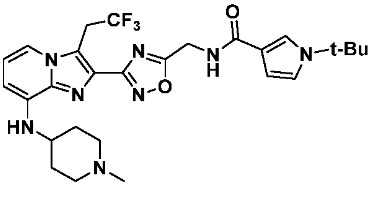,

, , ,

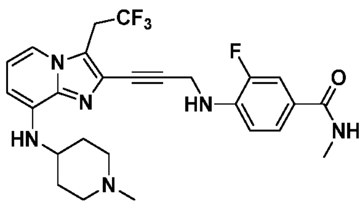
,
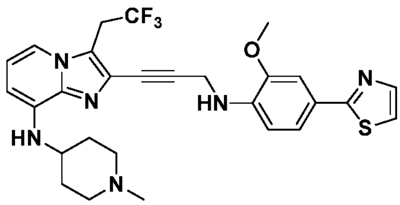
,
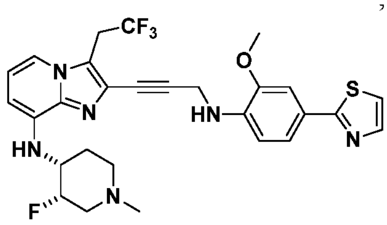
,

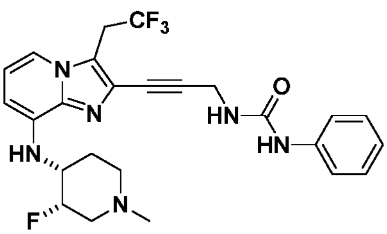
,
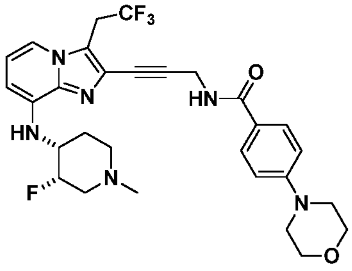
,
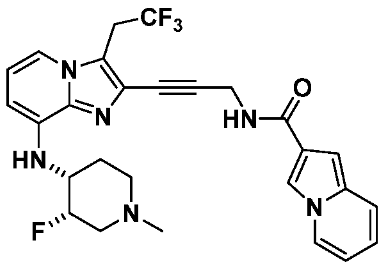
,

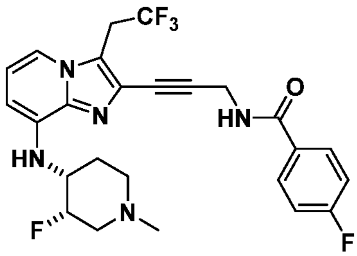
,
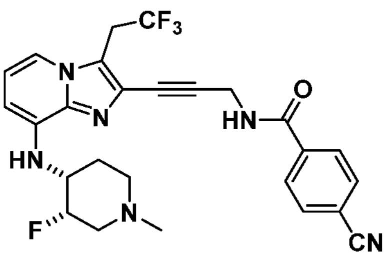
,
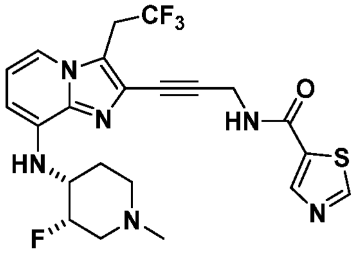
,

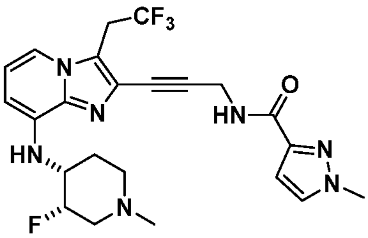
,
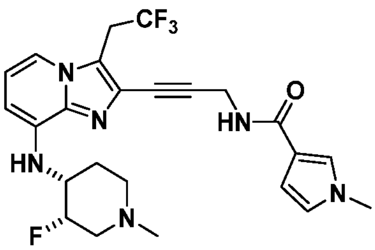
,
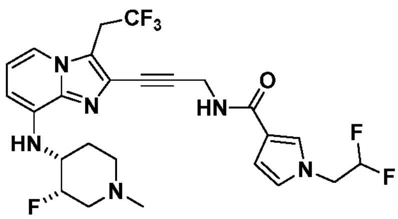
,

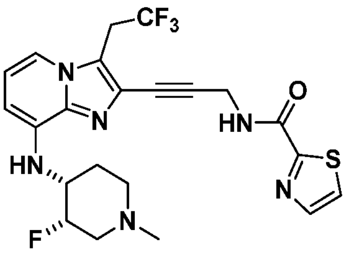
,
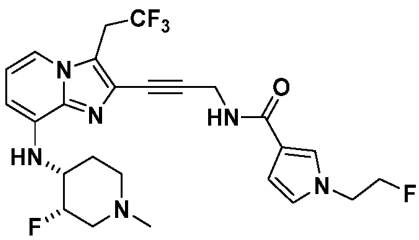
,
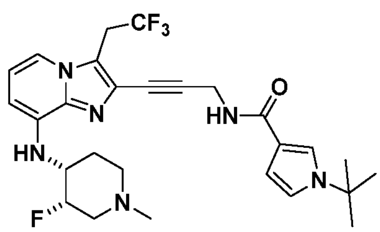
,

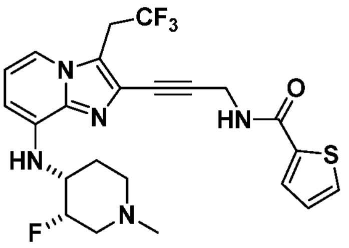
,
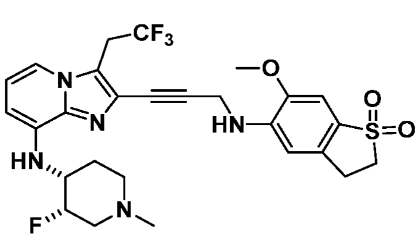
,
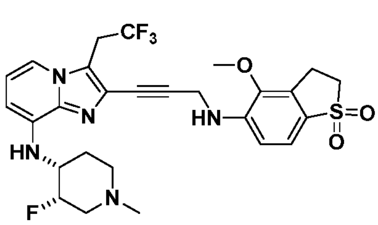
,

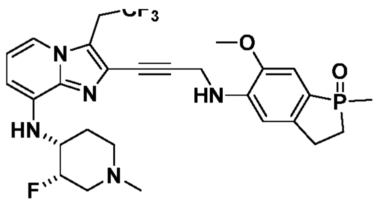 , 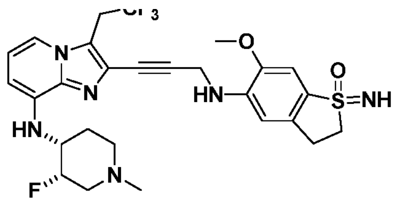 , 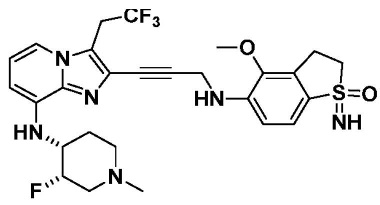 ,

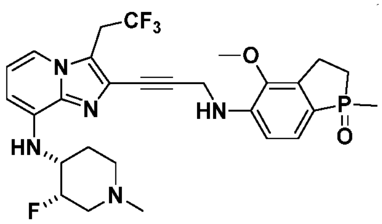 , 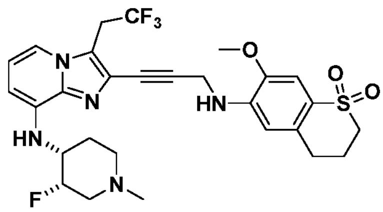 , 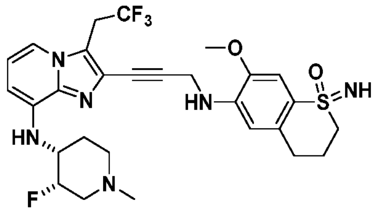 ,

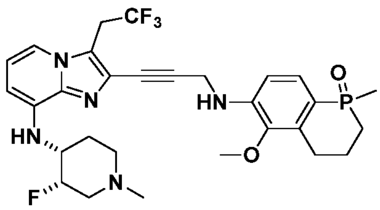 , 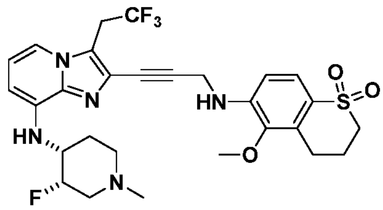 , 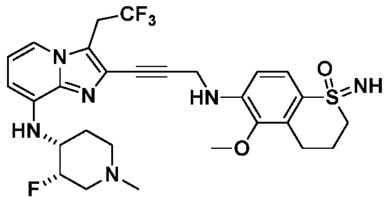 ,

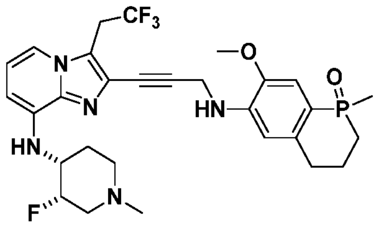 , 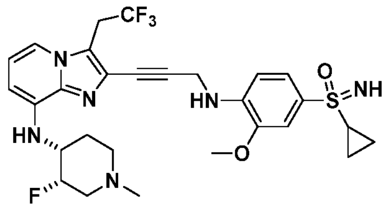 , 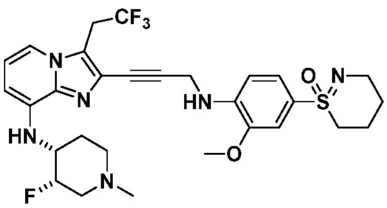 ,

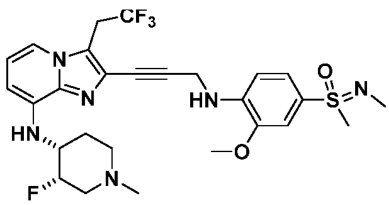 , 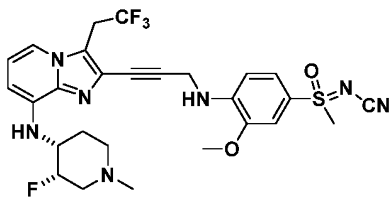 , 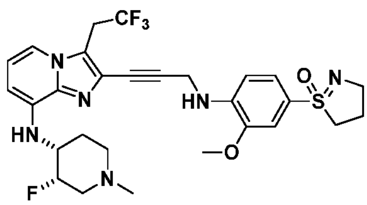 ,

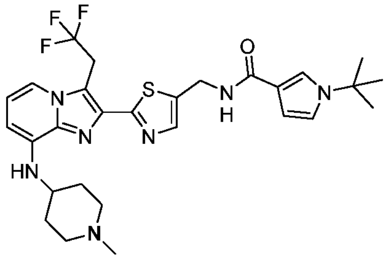 , 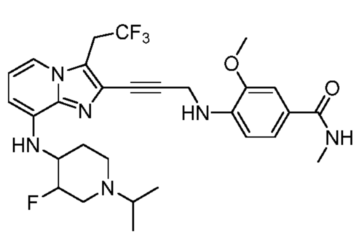 , 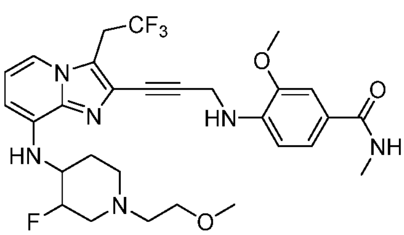 ,

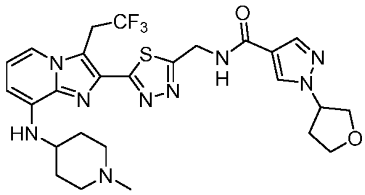, 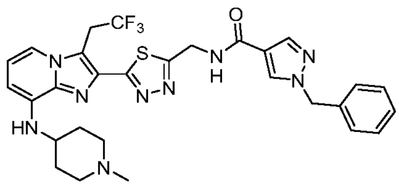, 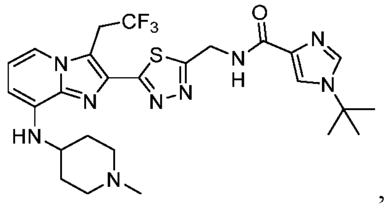,

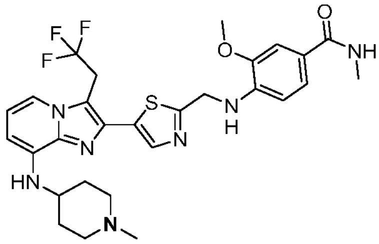, 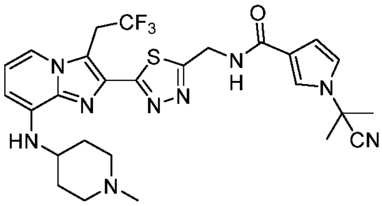, 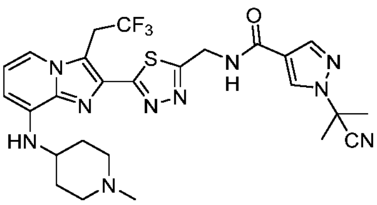,

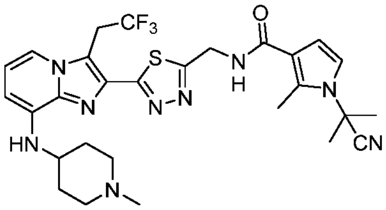, 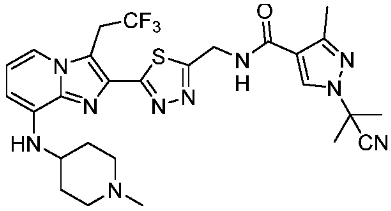,

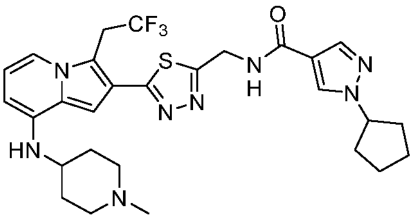, 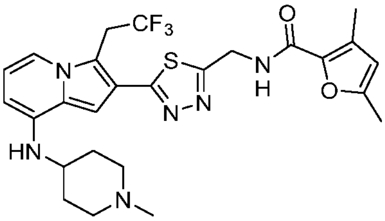,

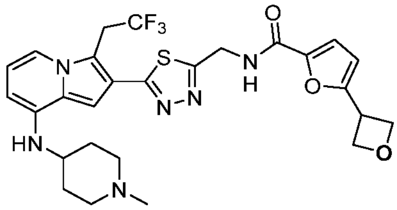, 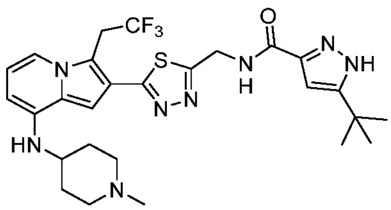, 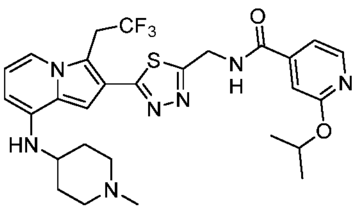,

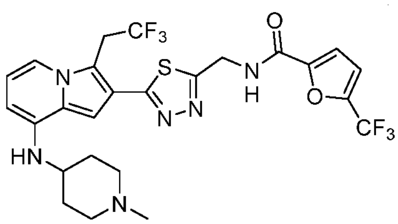, 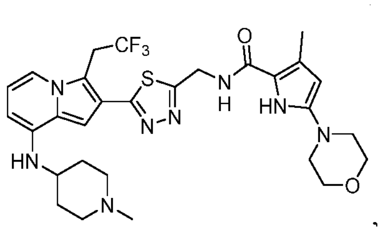, 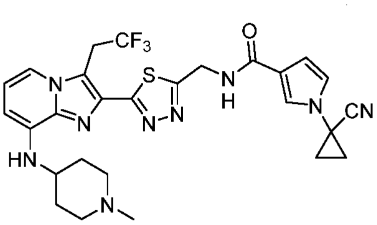,

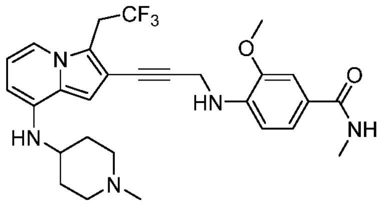,
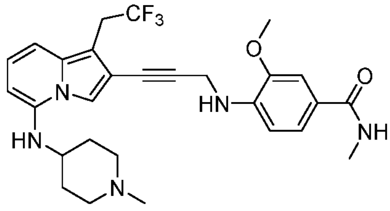,
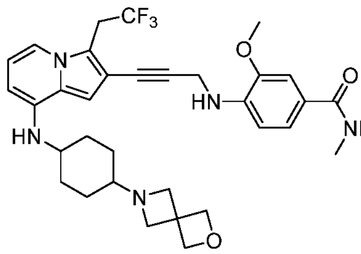,

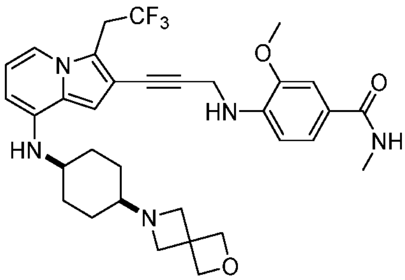,
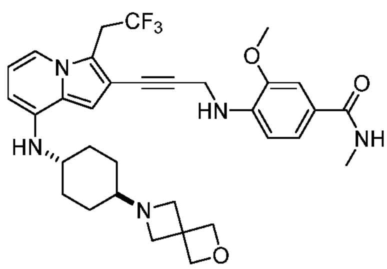,

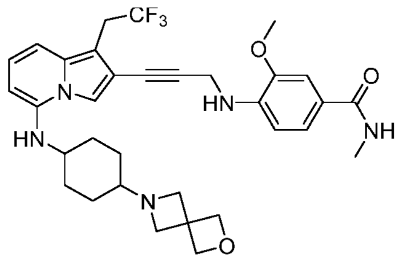,
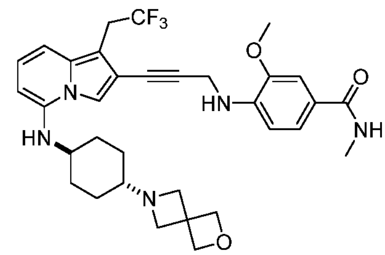,
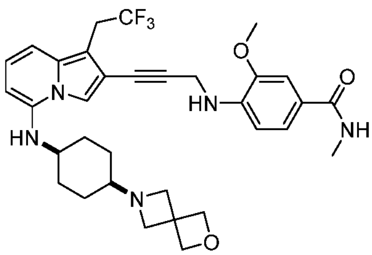,

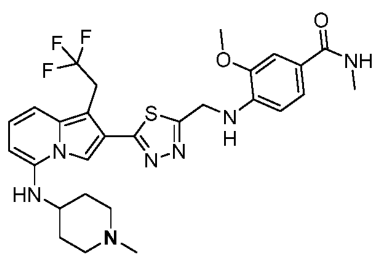,
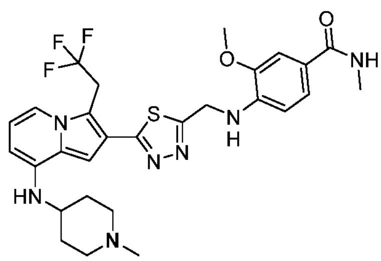,
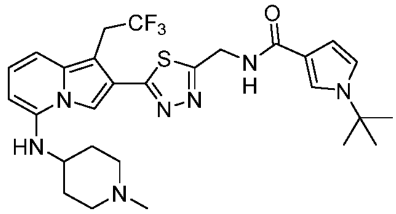,

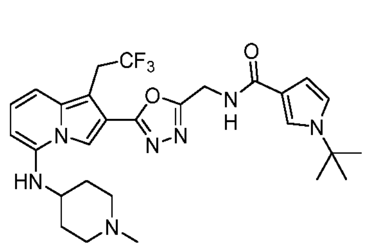,
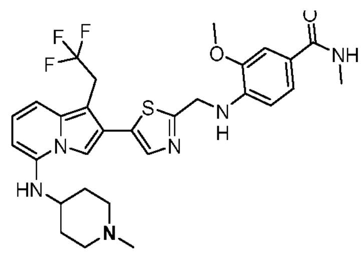,
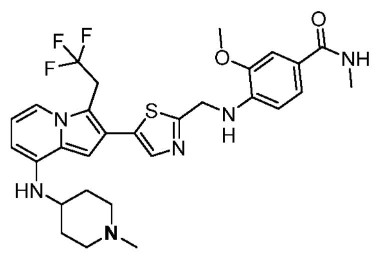,

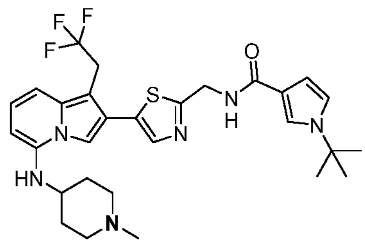,
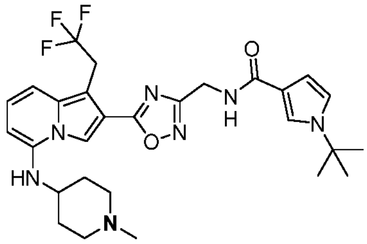,
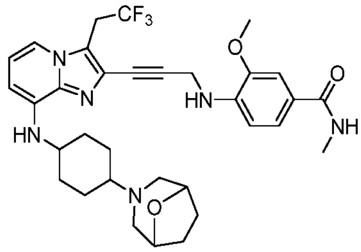,

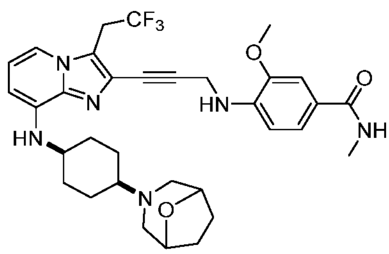,
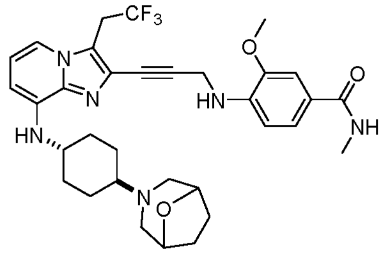,
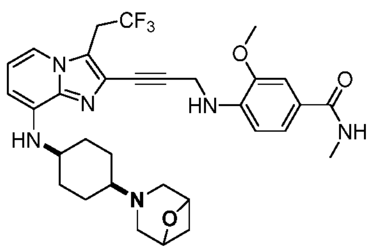,

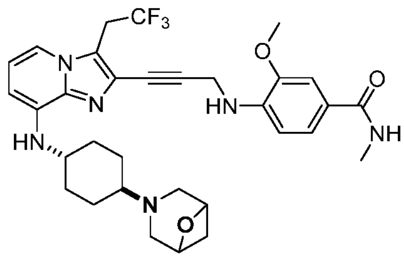,
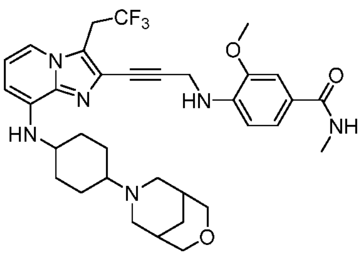,
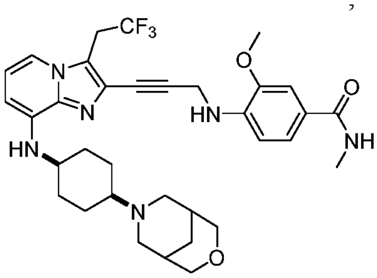,

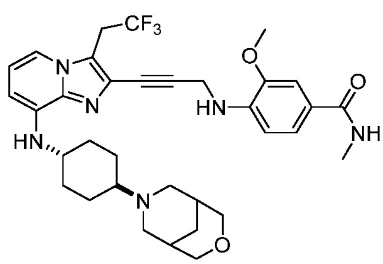,
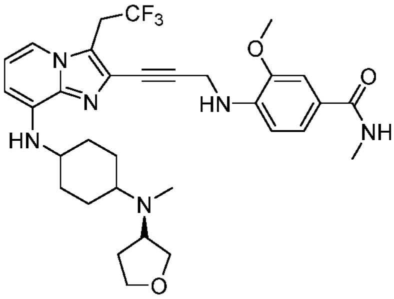,
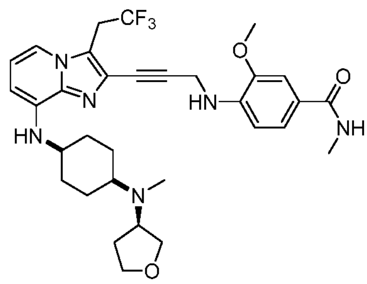,

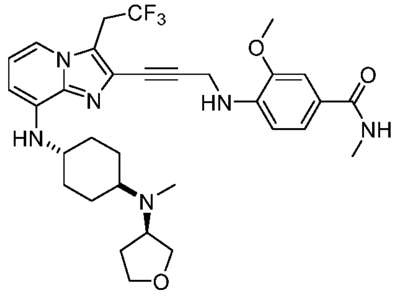,
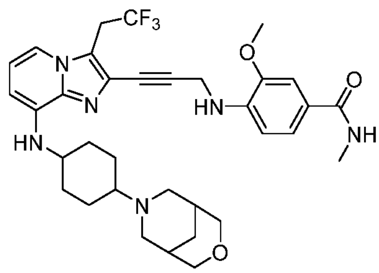,
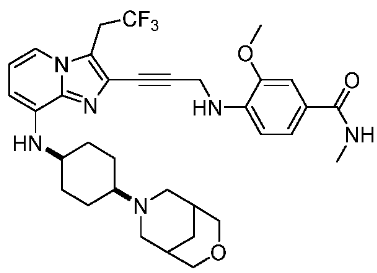,

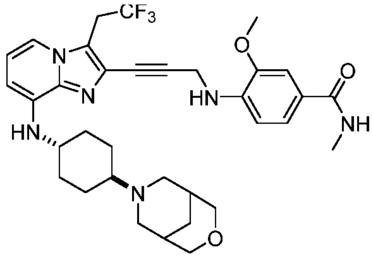,
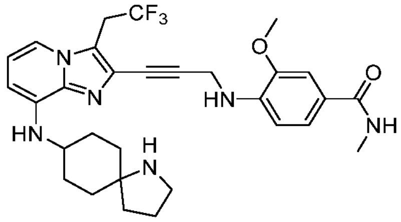,
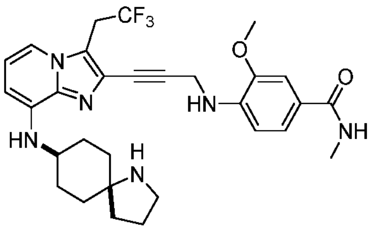,

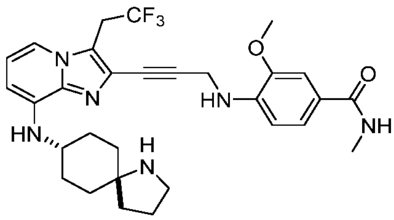,
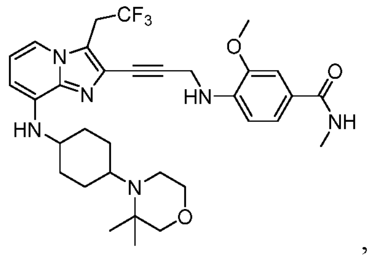,
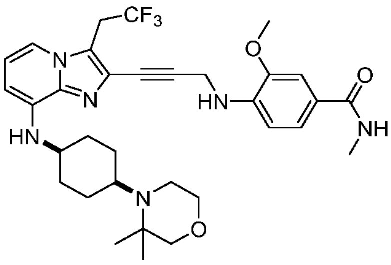,

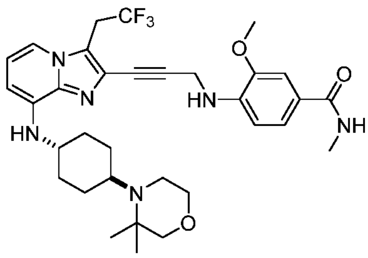,
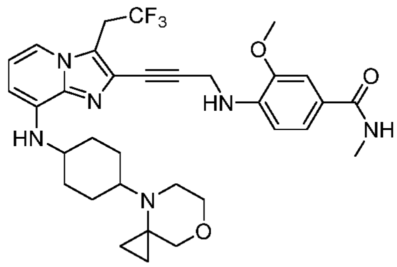,
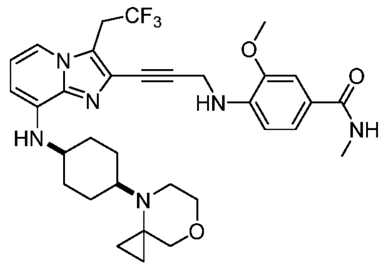,

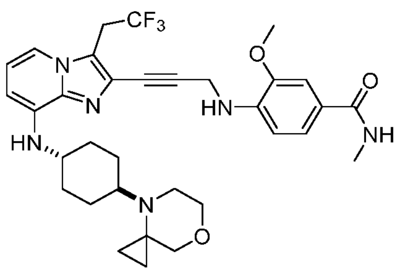,
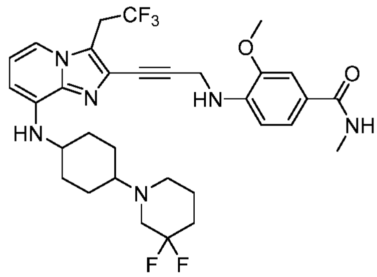,
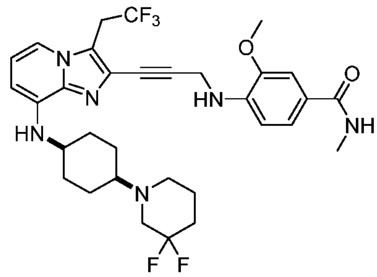,

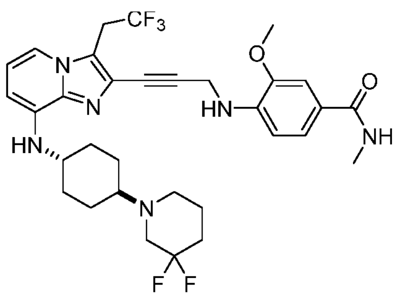,
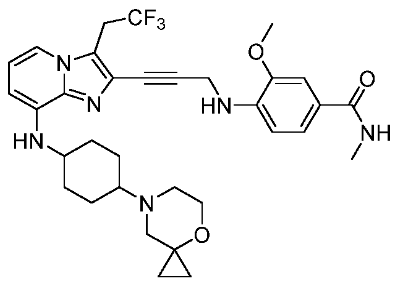,
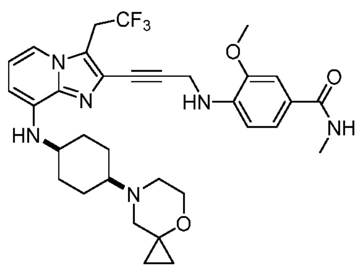,

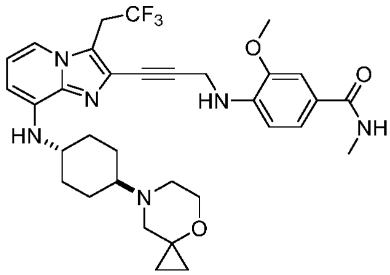 , 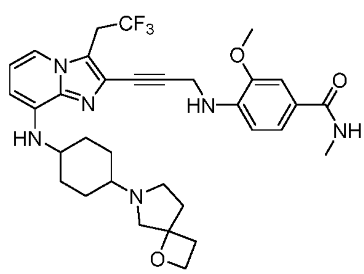 , 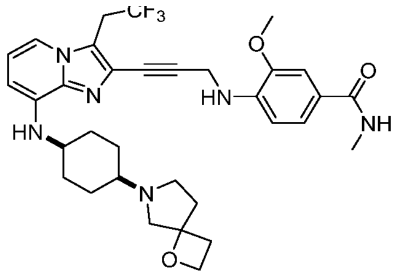 ,

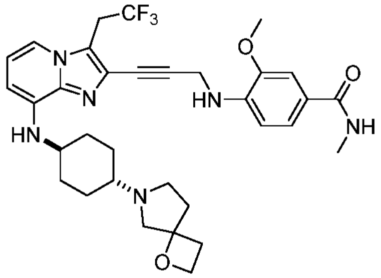 , 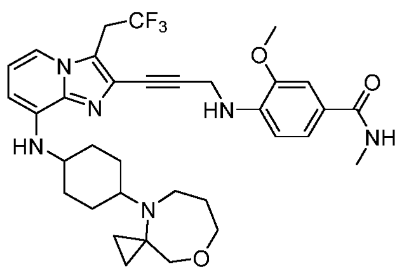 , 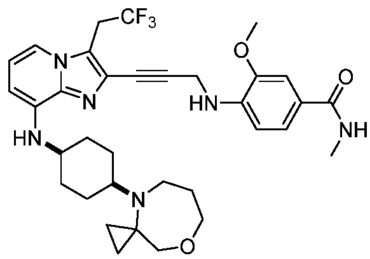 ,

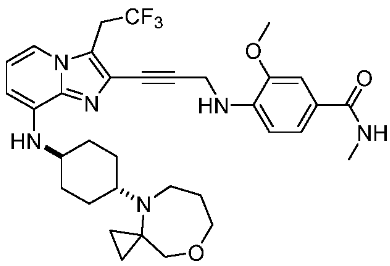 , 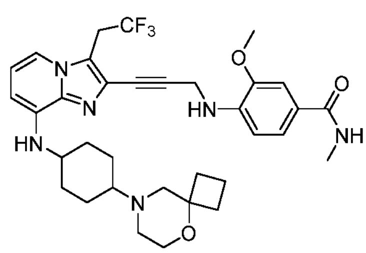 , 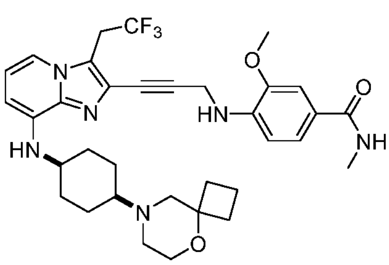 ,

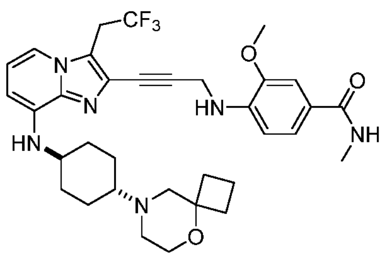 , 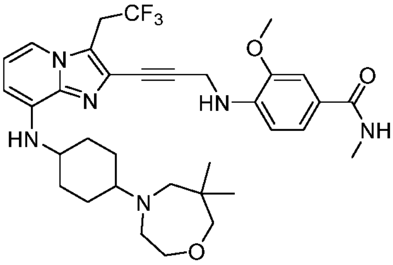 , 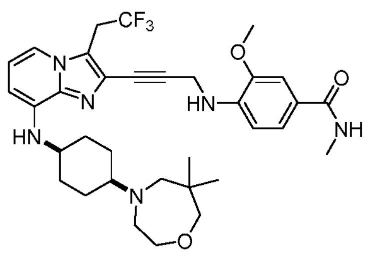 ,

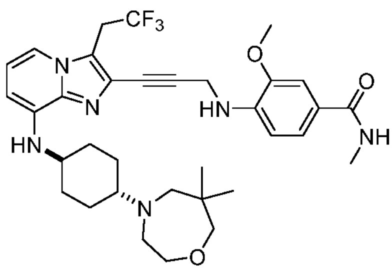 , 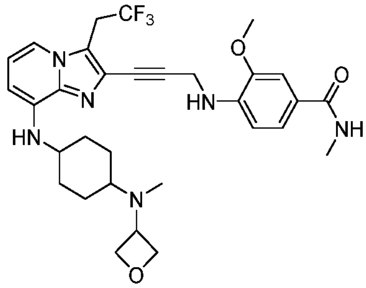 , 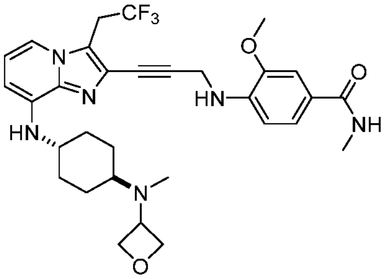 ,

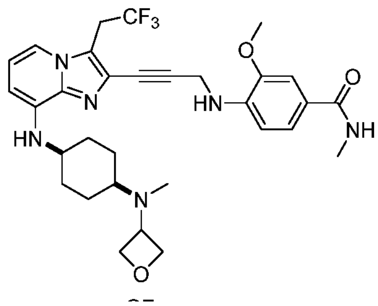,
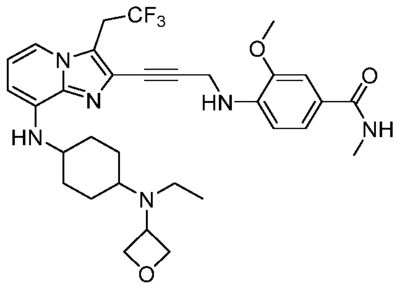,
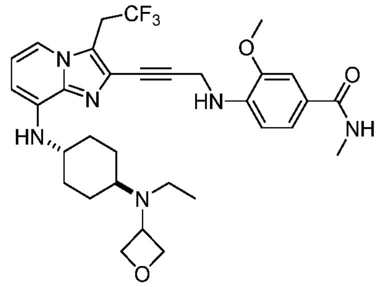,
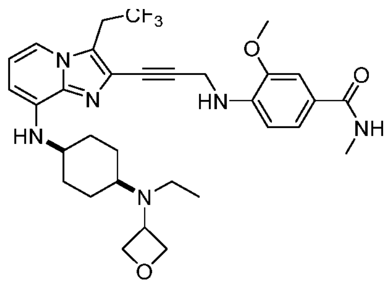,
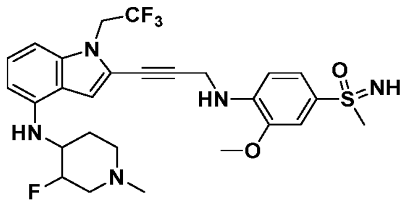,
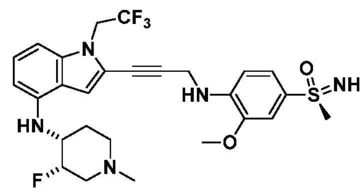,
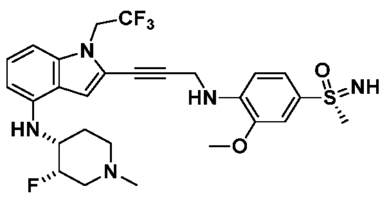,
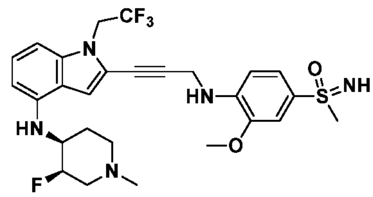,
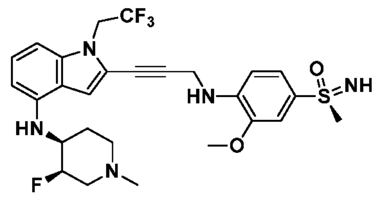,
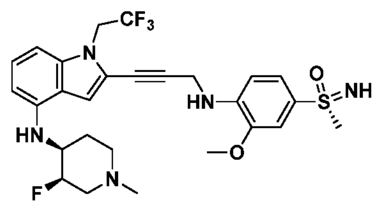,
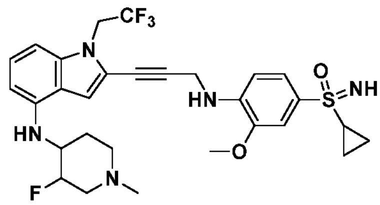,
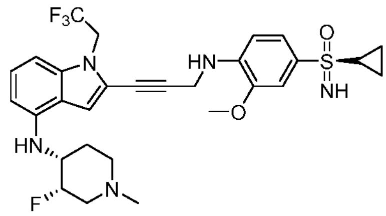,
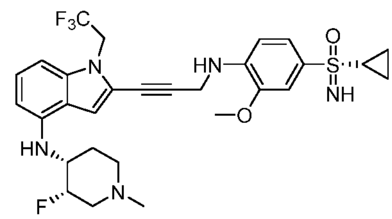,
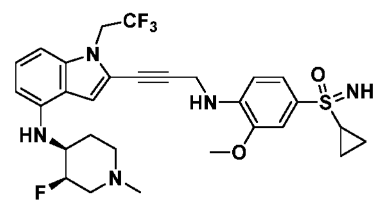,
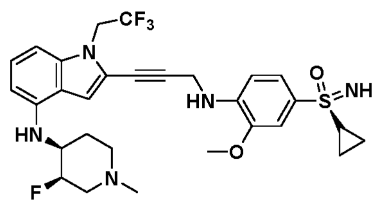,
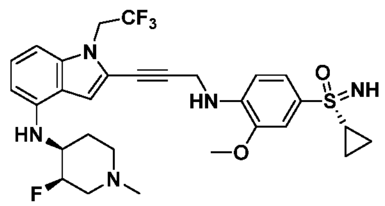,
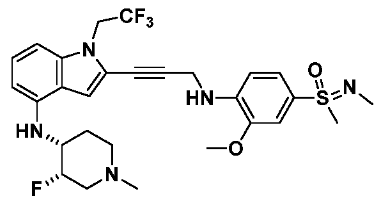,
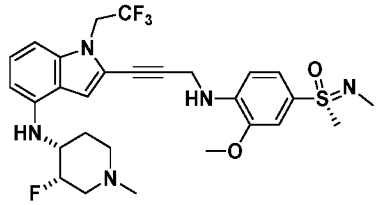,

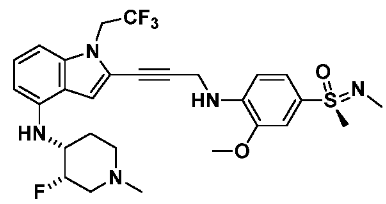,
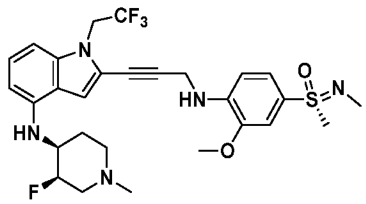,
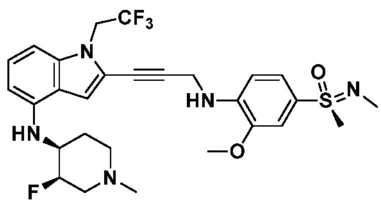,

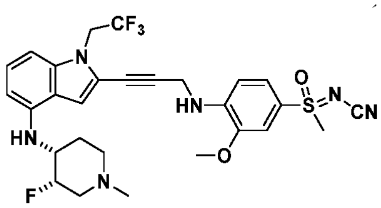,
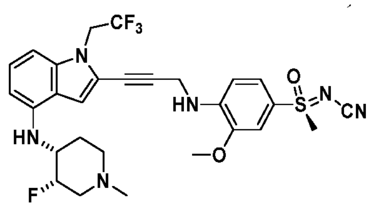,
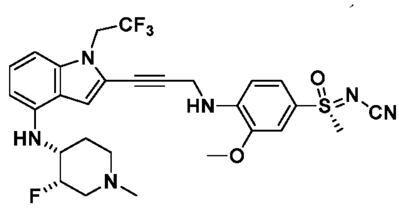,

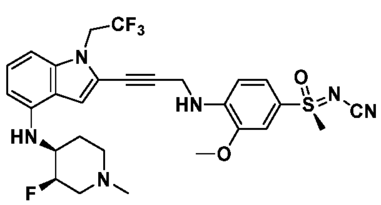,
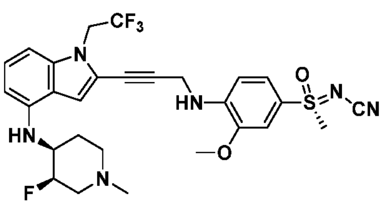,
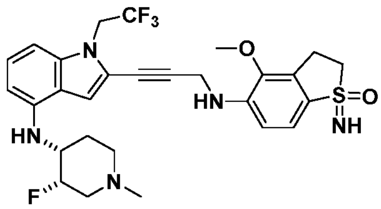,

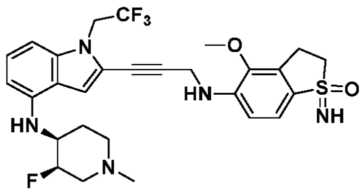,
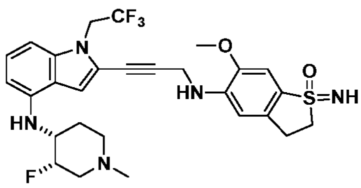,
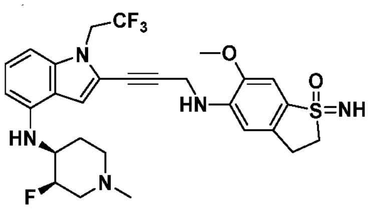,

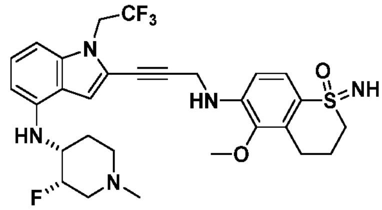,
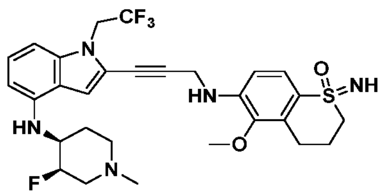,
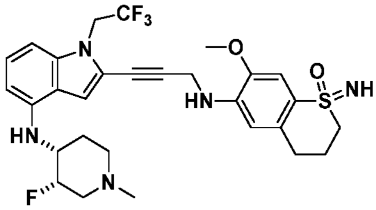,

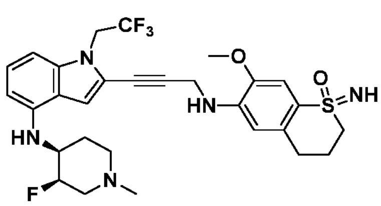,
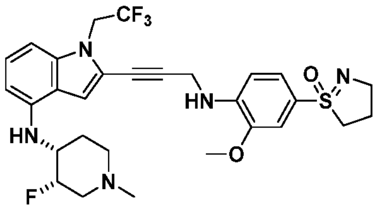,
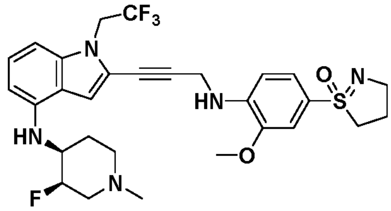,

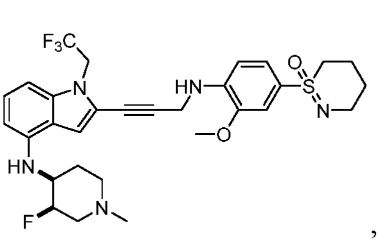,
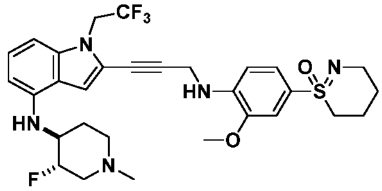,
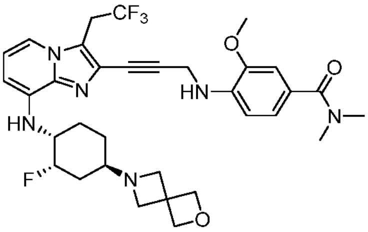,
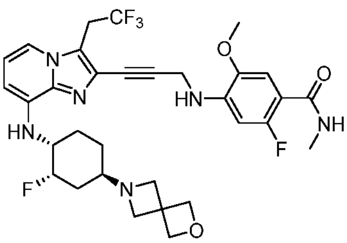,
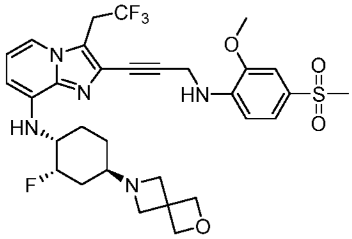,
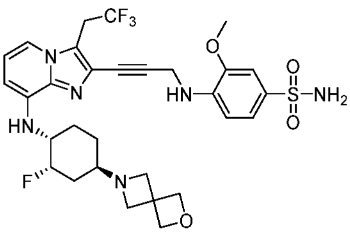,
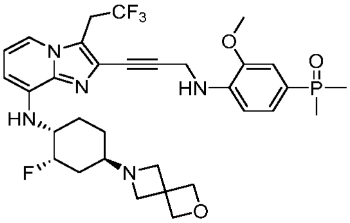,
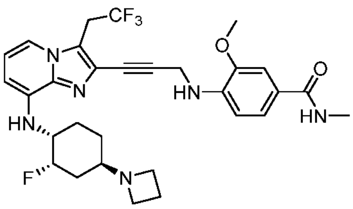,
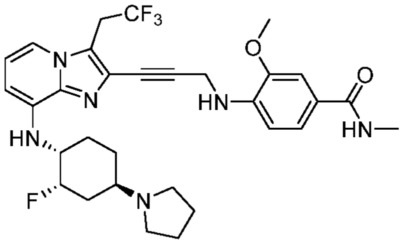,
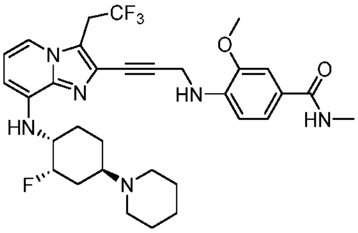,
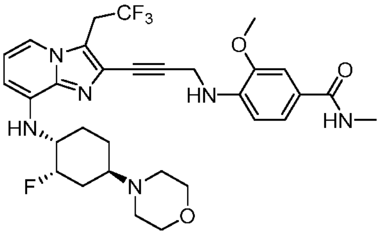,
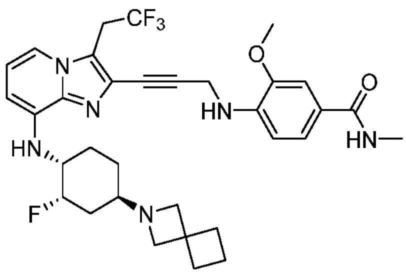,
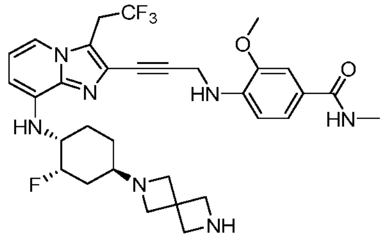,
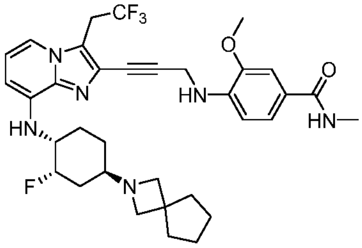,
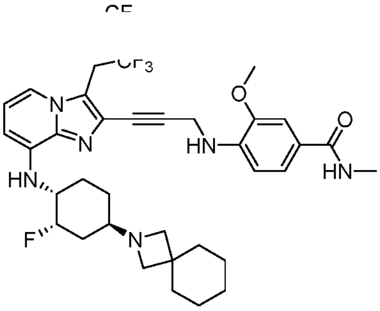,
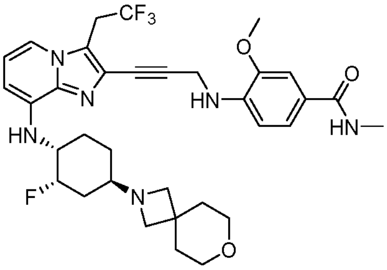,
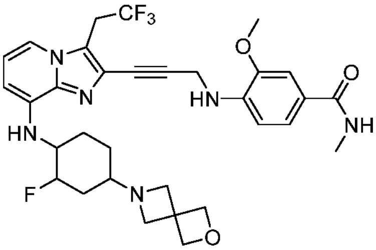,
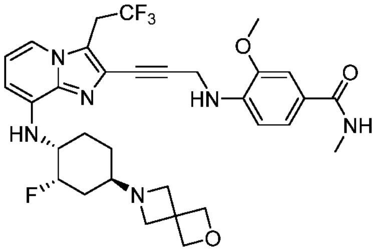,

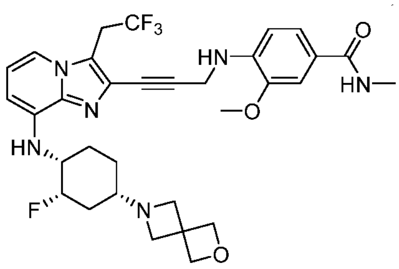,
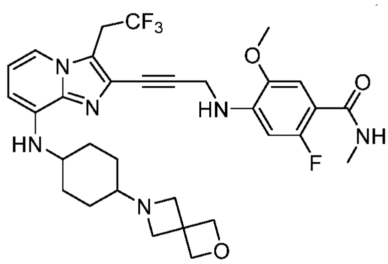,
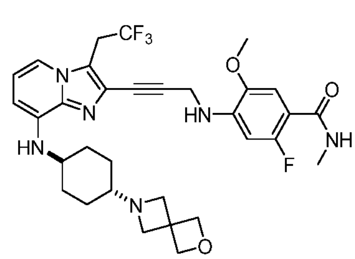,

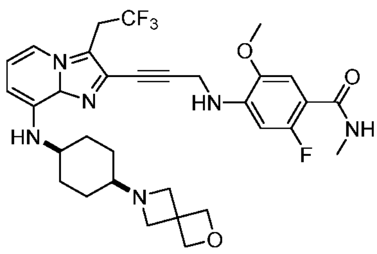,
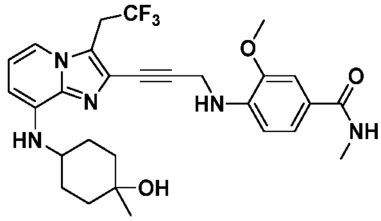,
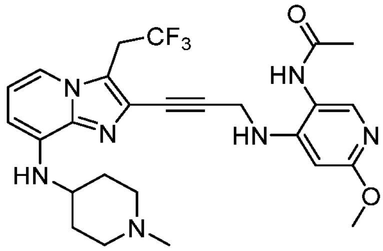,

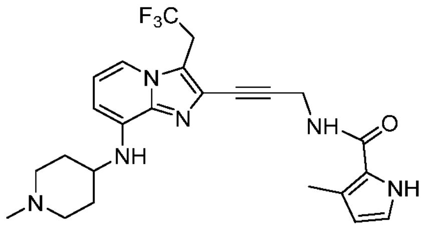,
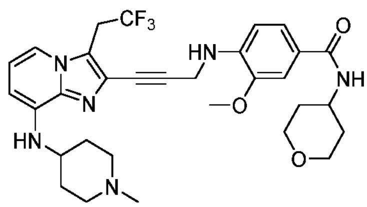,
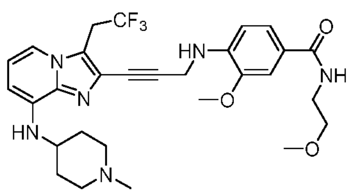,

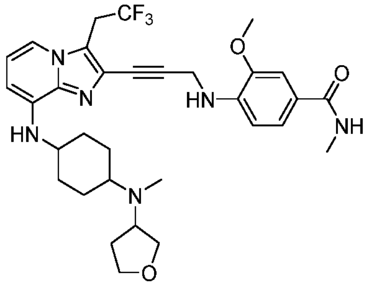,
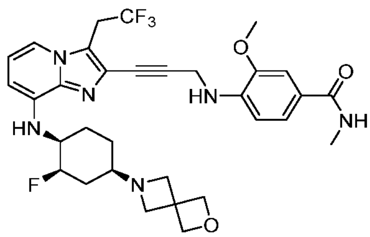,
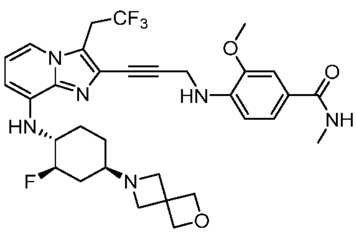,

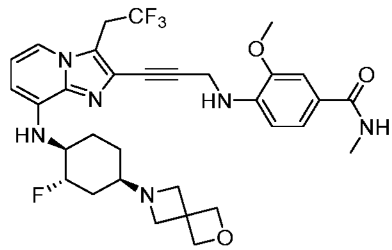,
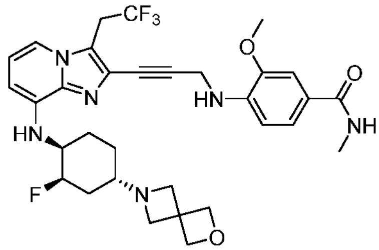,
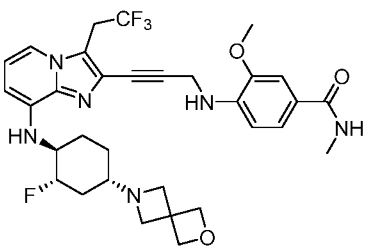,

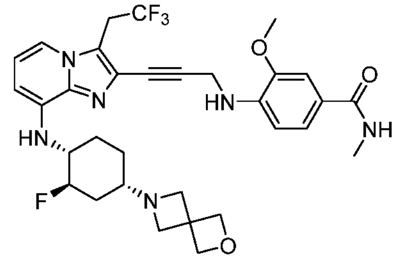,
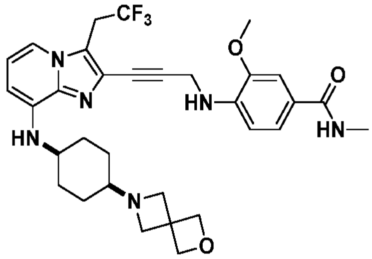,
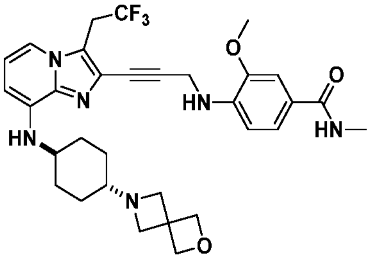,

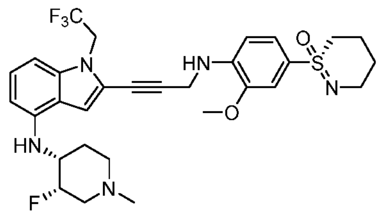,
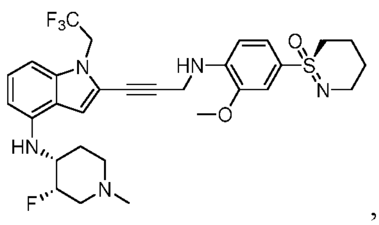,
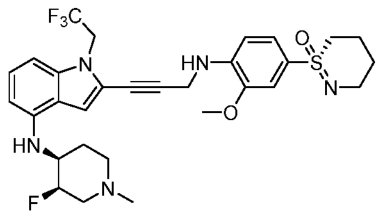,

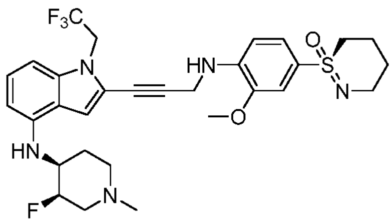,
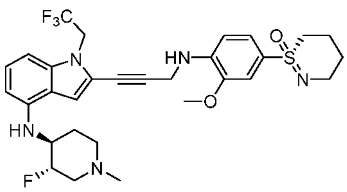,
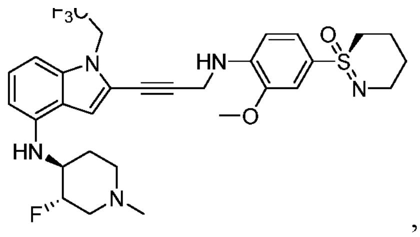,

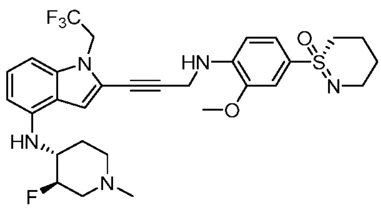,
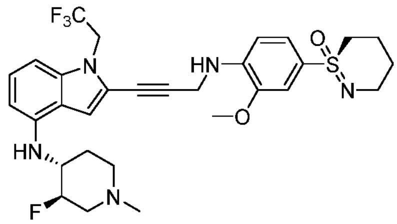,
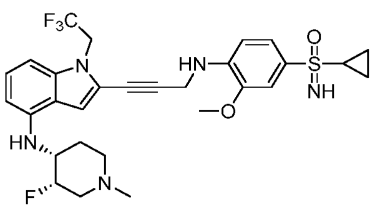,

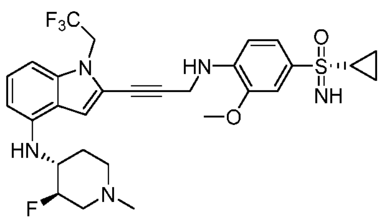,
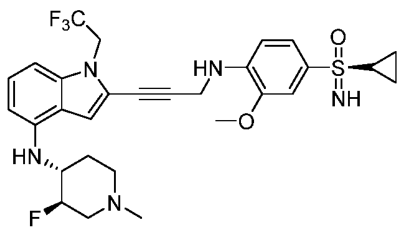,
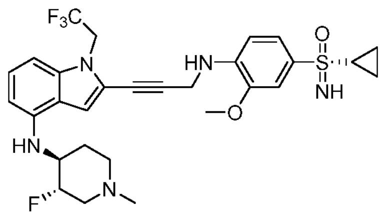,

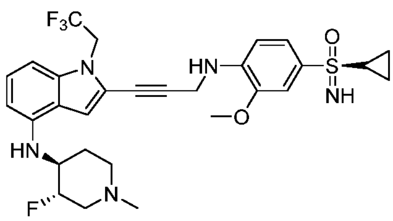,
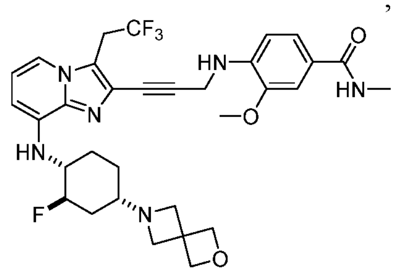,
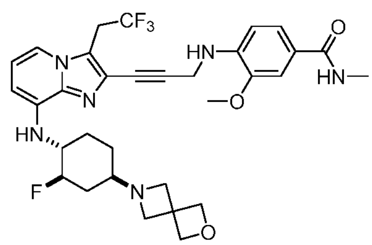,

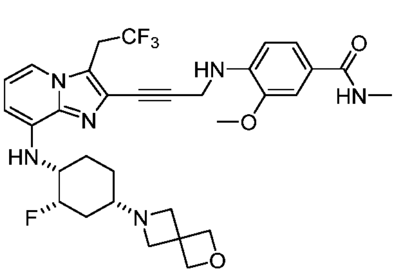,
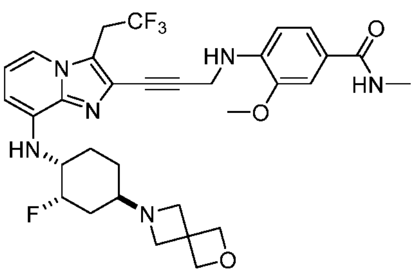,

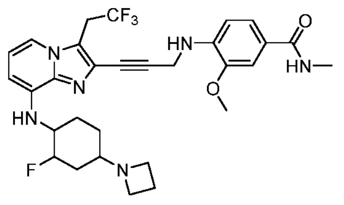
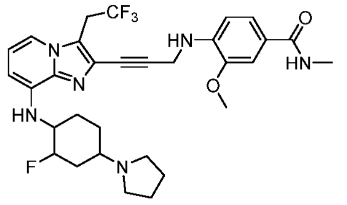
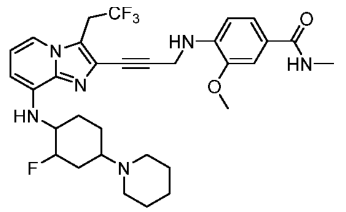
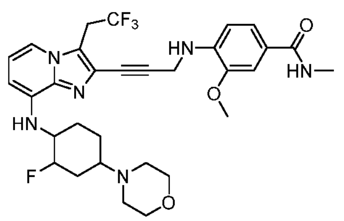
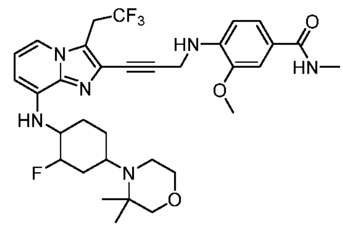
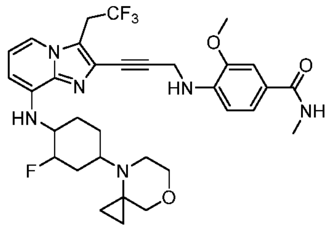
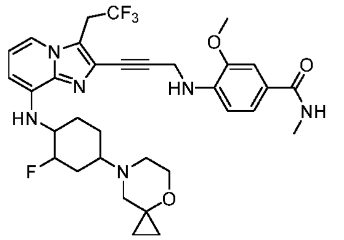
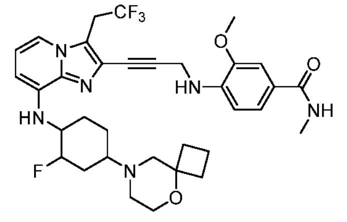
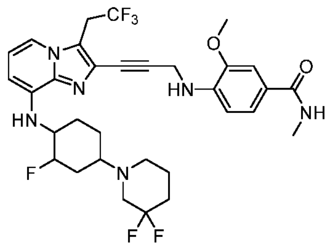
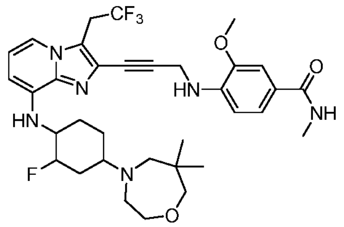
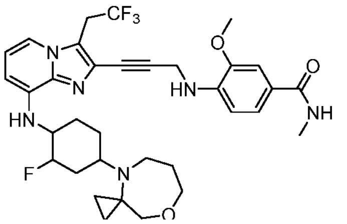
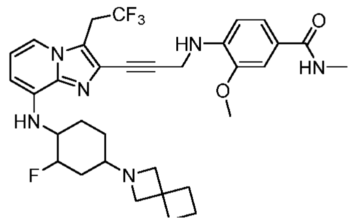
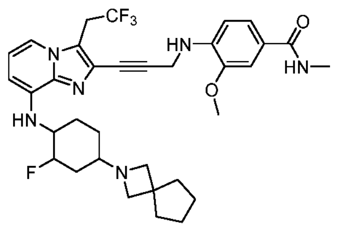
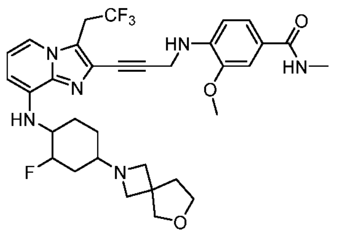
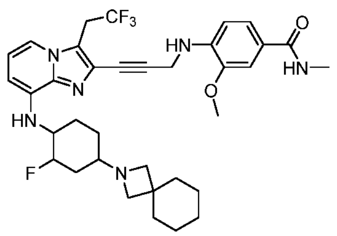
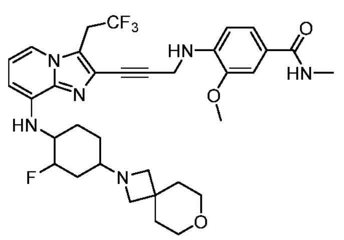
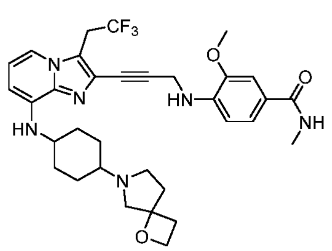
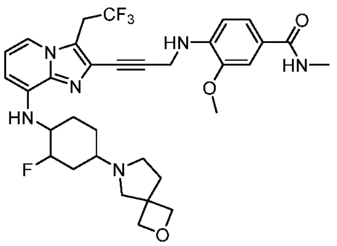

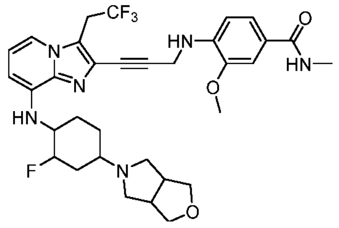

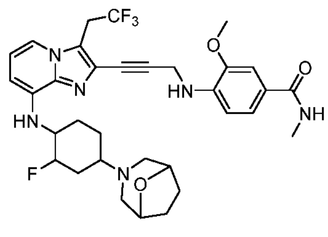

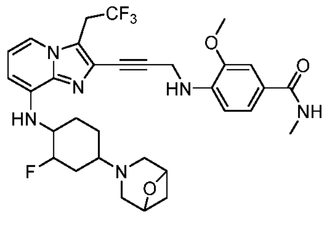

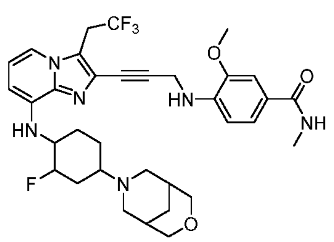

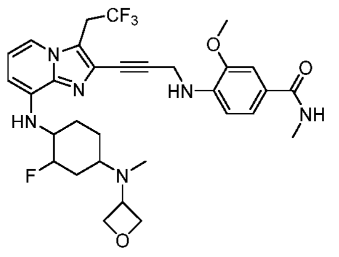

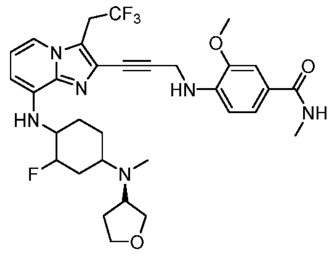

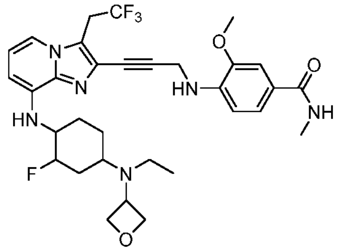

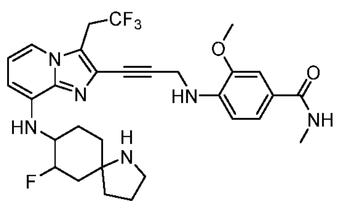

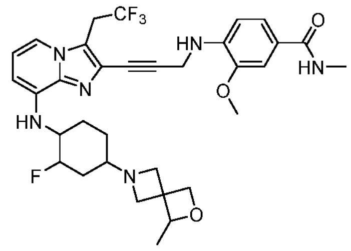

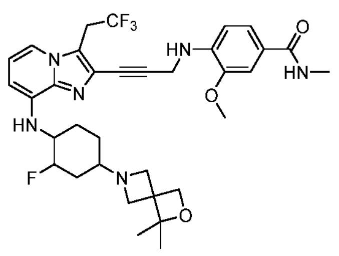

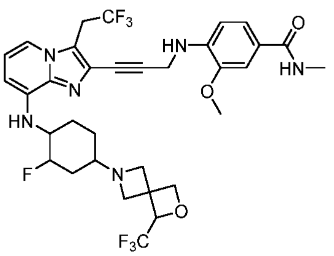

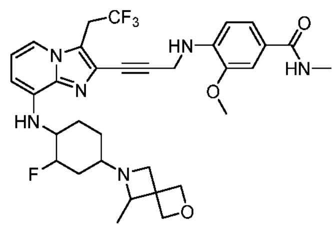

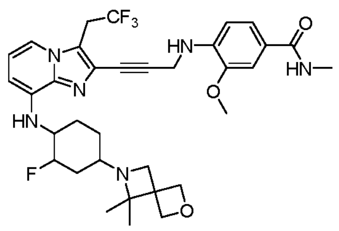

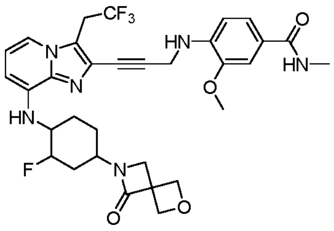

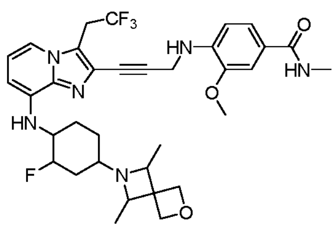

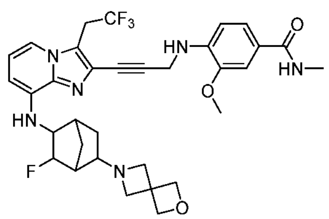

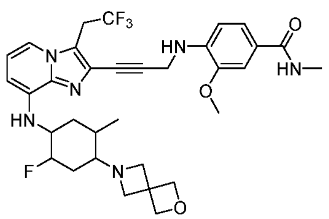

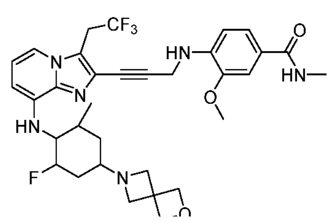

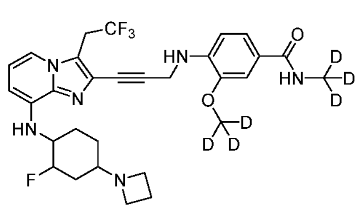
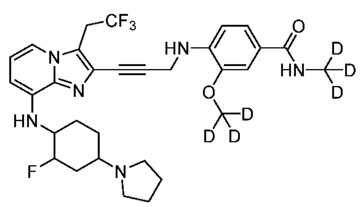
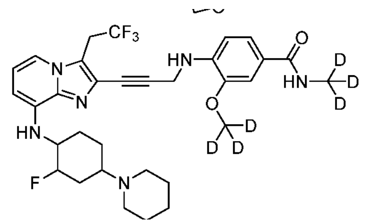

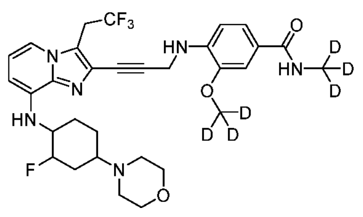
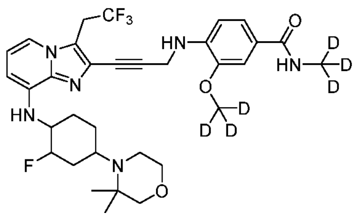
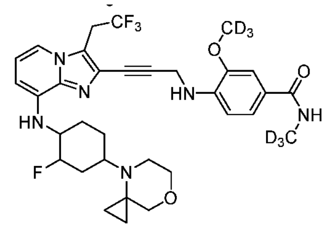

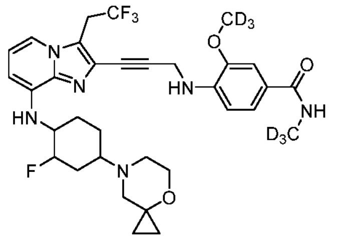
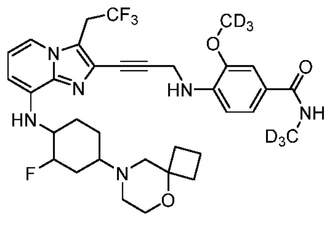
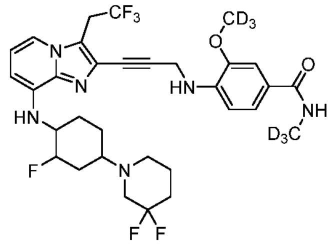

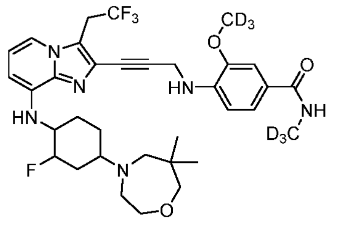
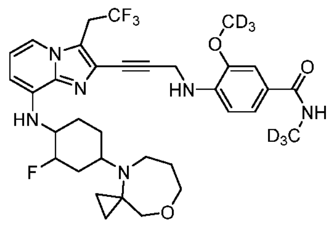
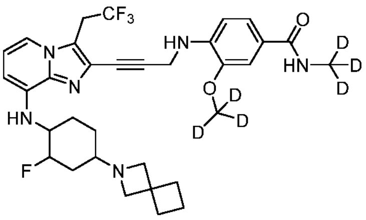

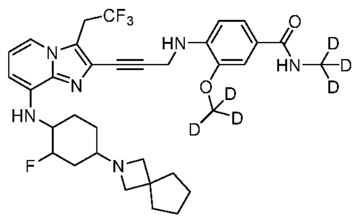
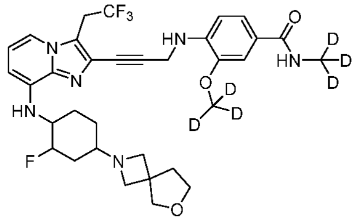
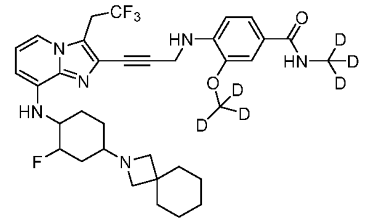

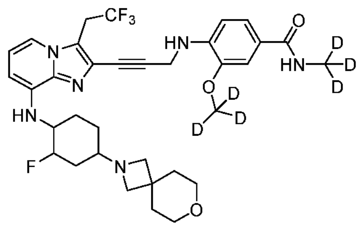
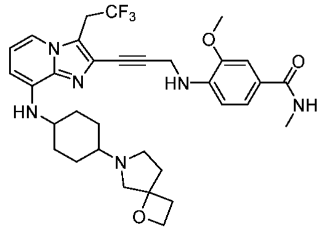
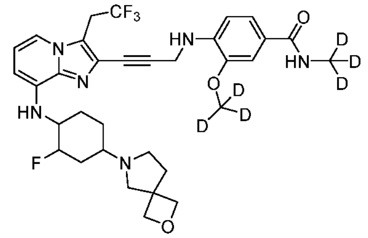

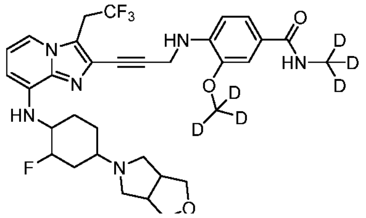
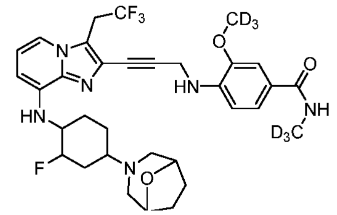
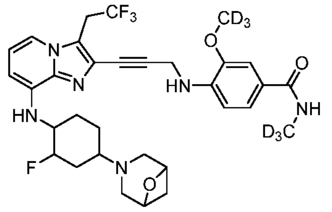

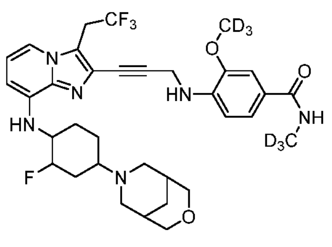
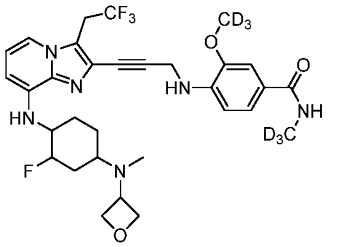
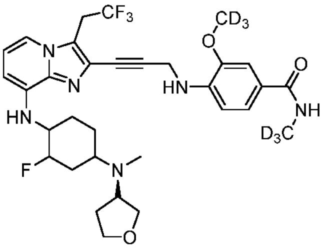

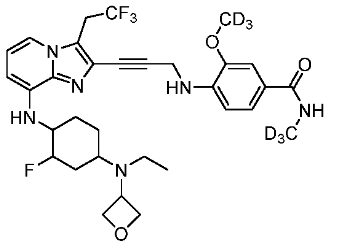
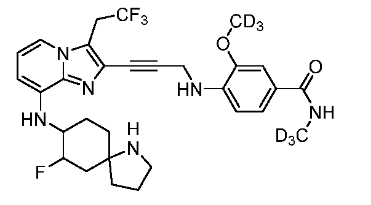
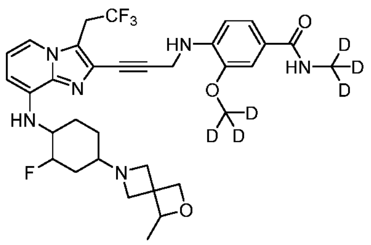

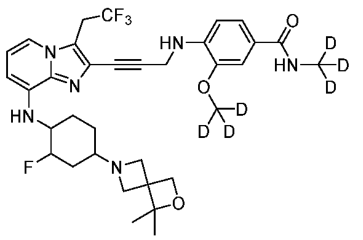
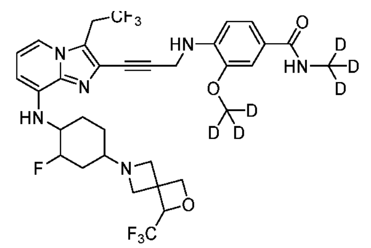
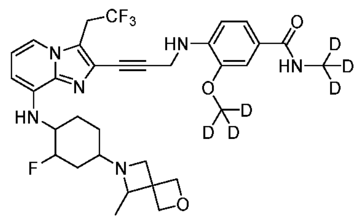

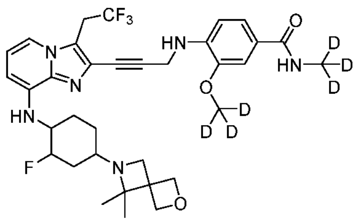
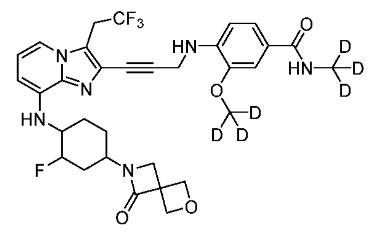
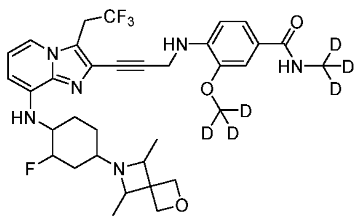

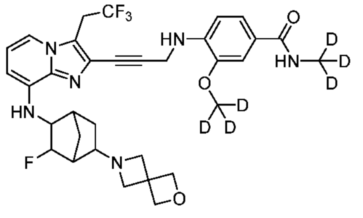
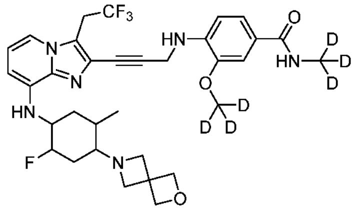
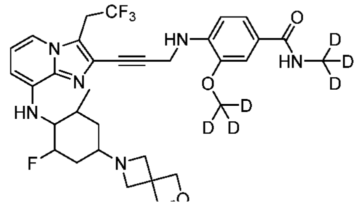

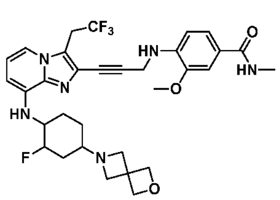 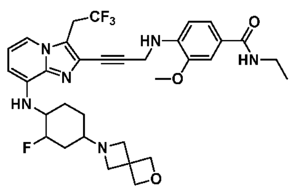 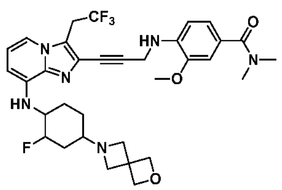 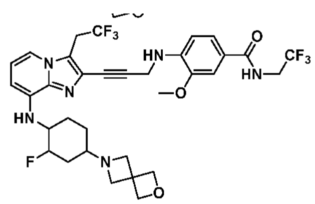

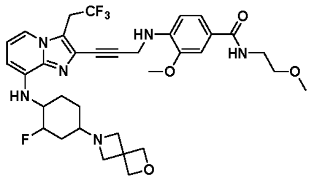 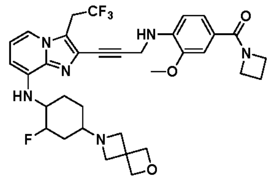 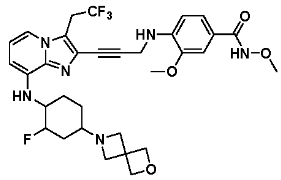 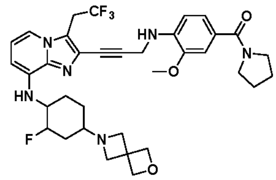

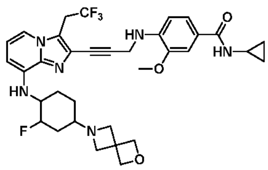 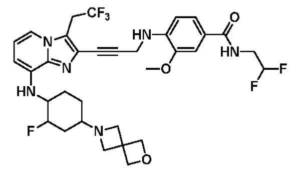 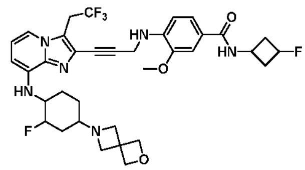 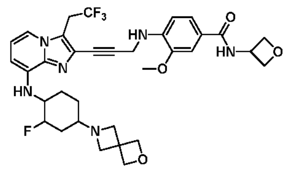

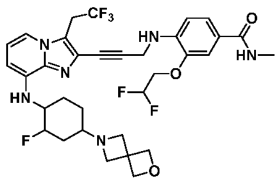 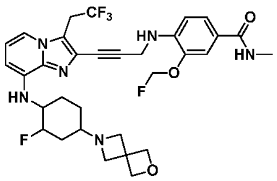 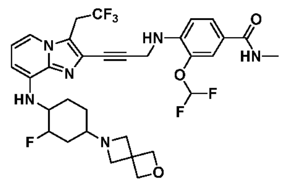 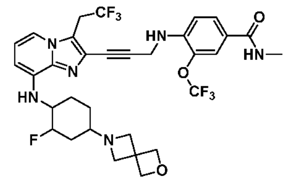

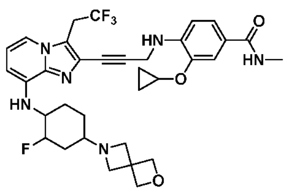 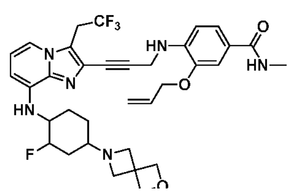 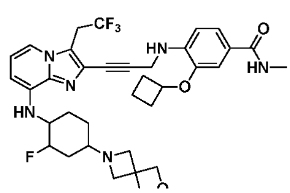 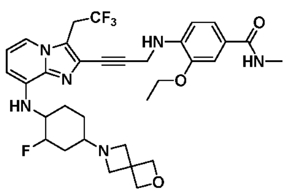

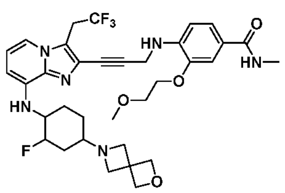 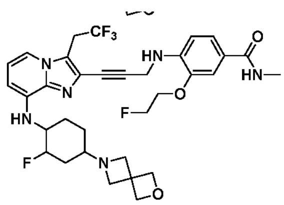 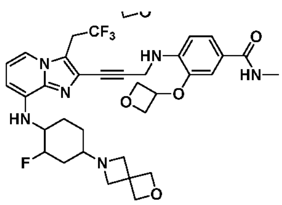 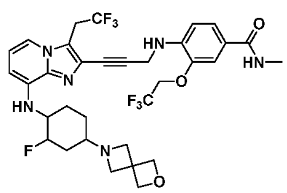

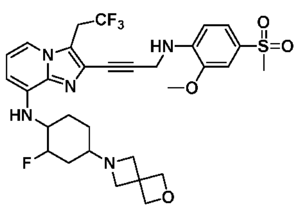 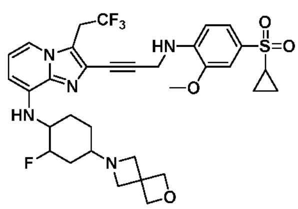 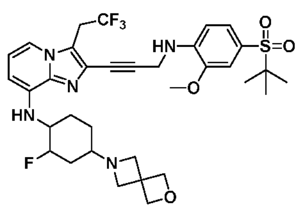

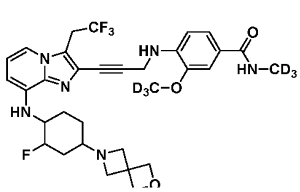 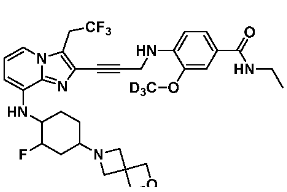 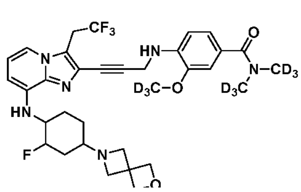 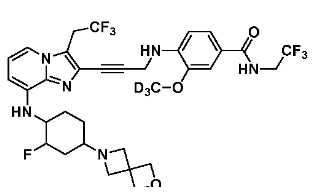

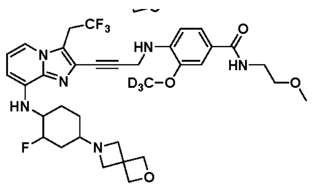
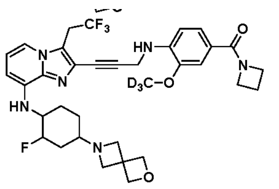
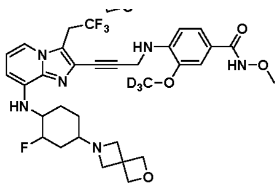
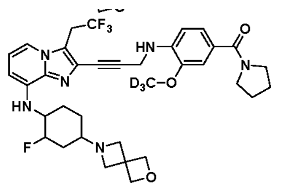

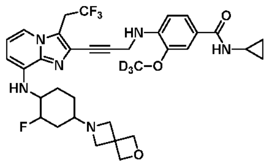
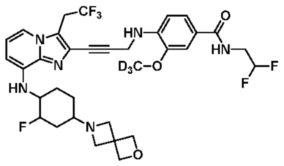
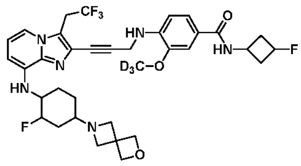
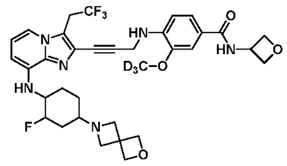

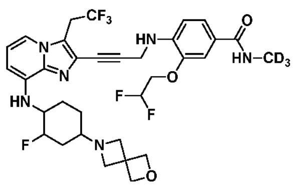
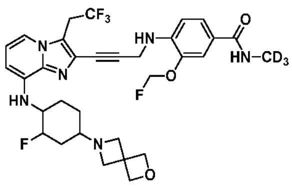
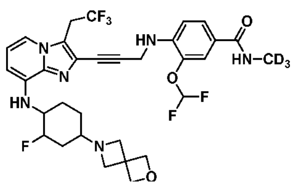
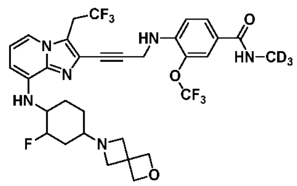

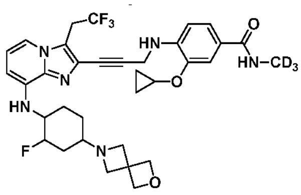
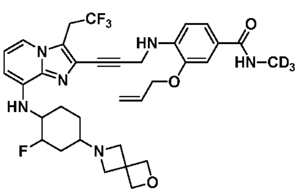
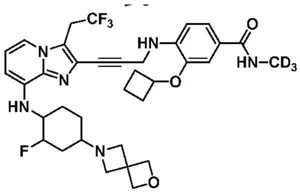
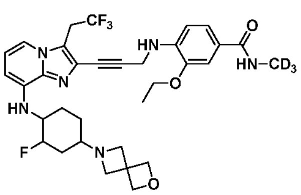

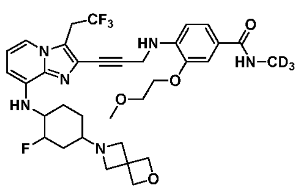
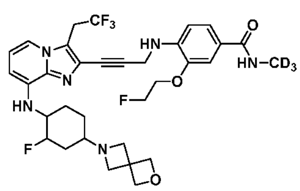
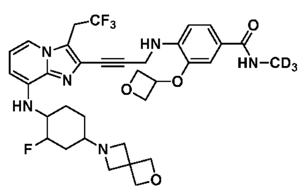
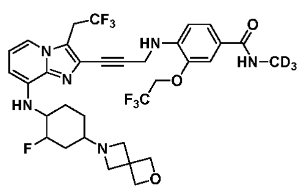

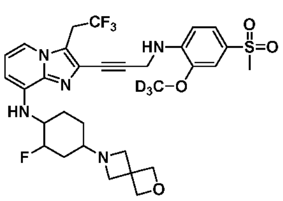
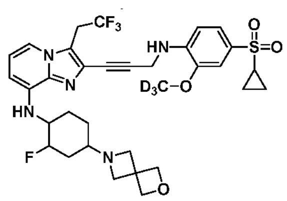
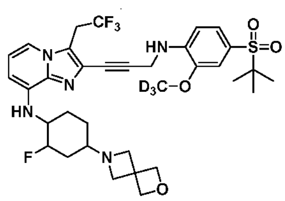

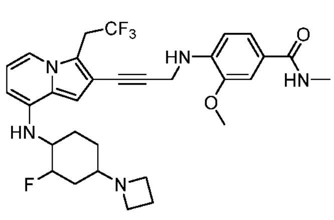
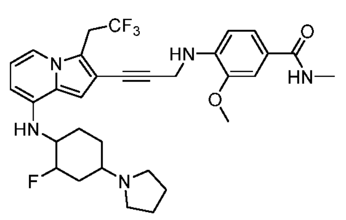
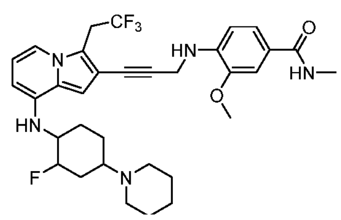

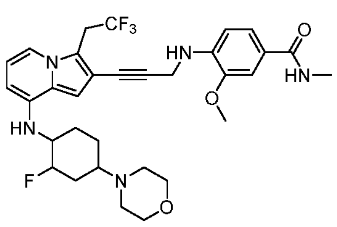
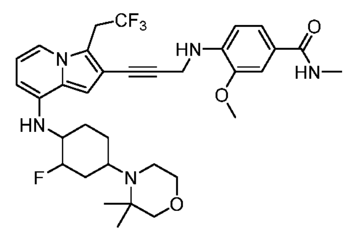
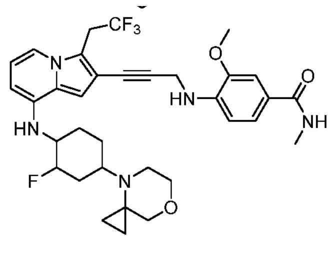
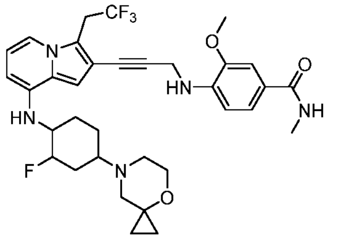
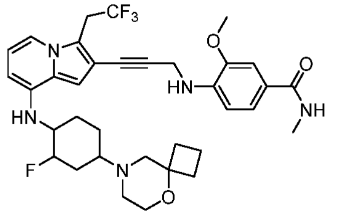
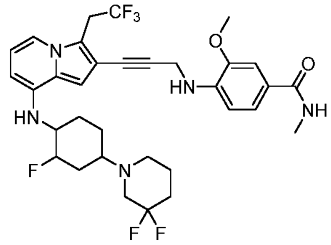
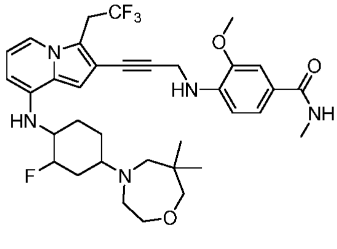
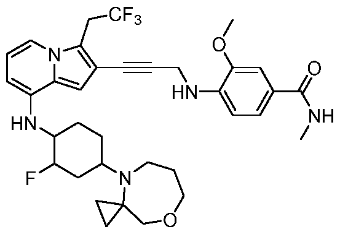
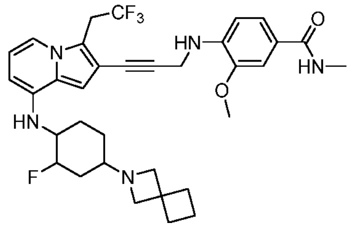
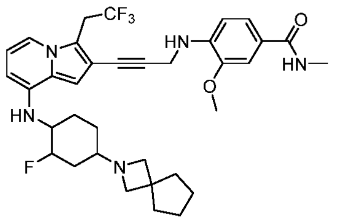
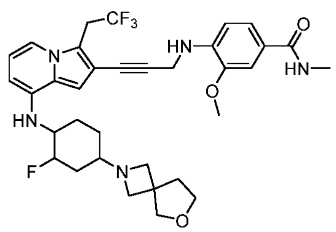
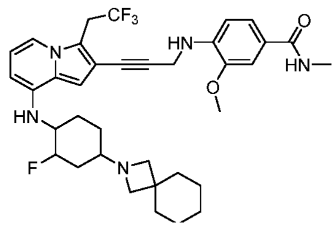
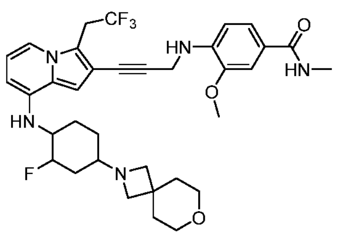
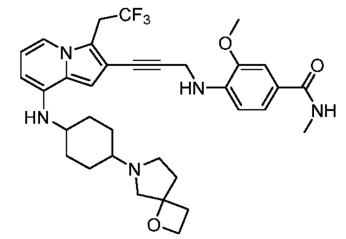
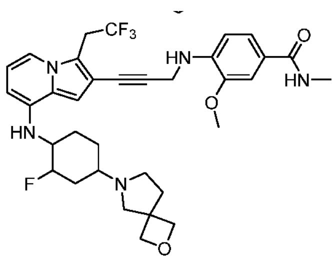
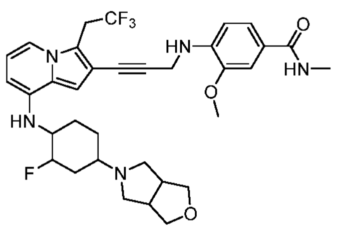
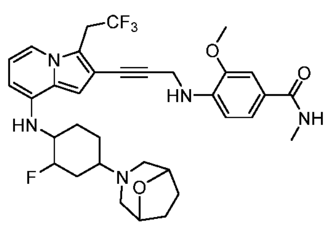
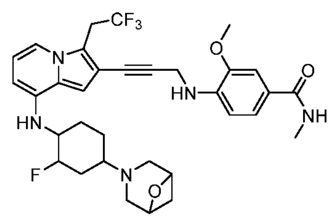

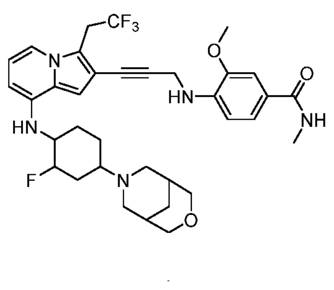
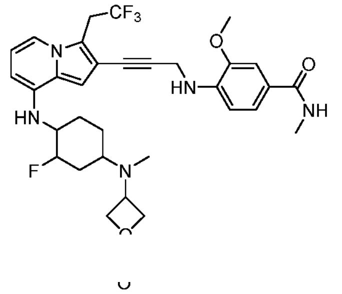
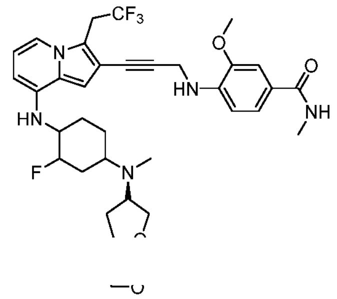
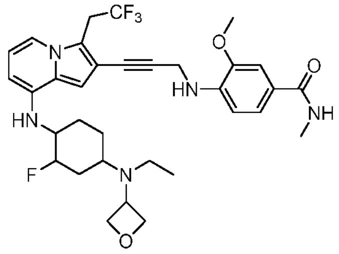
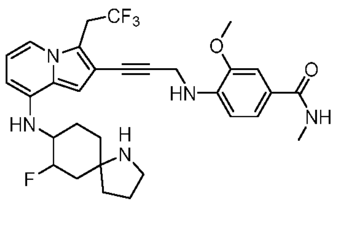
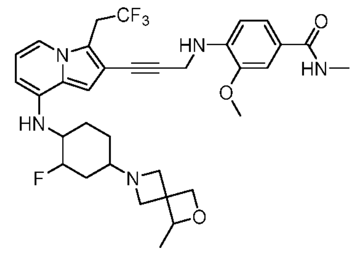
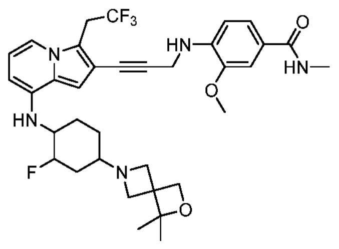
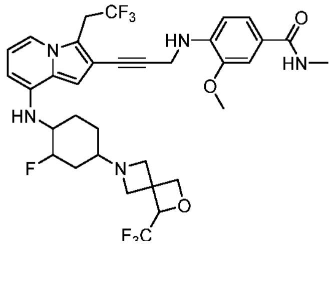
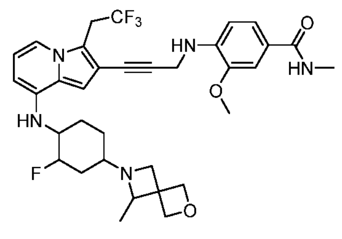
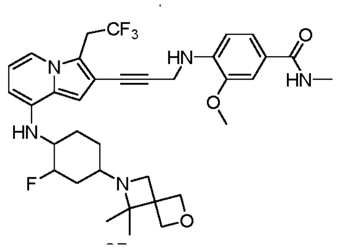
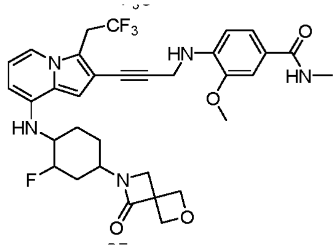
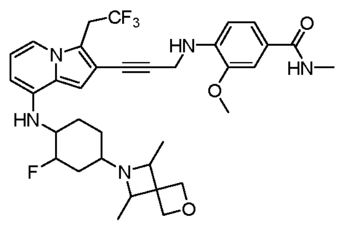
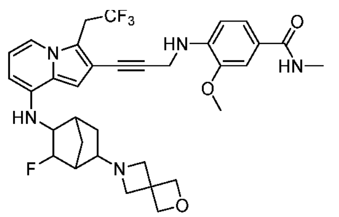
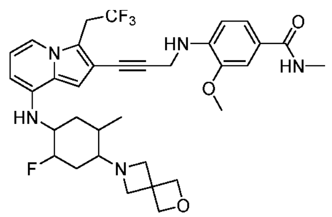
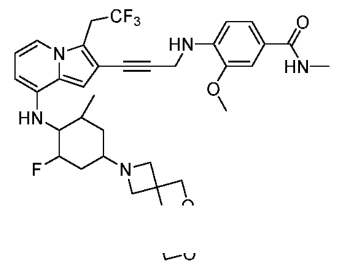
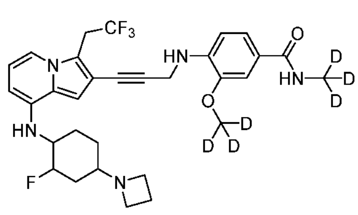
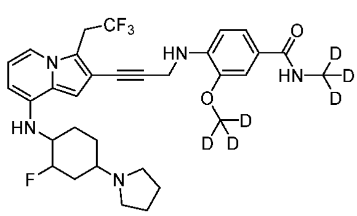
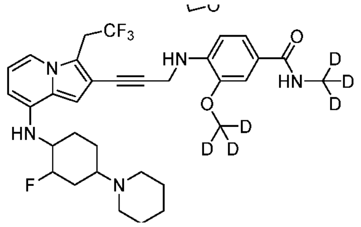

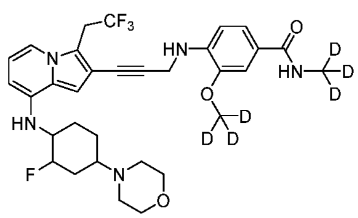
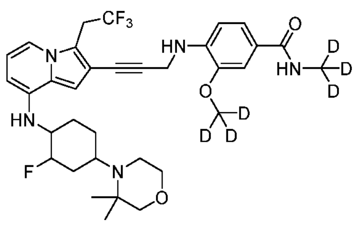
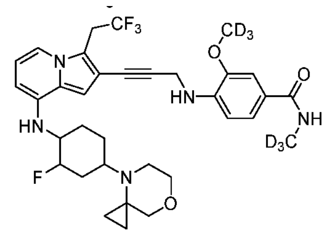

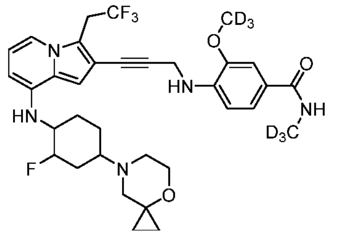
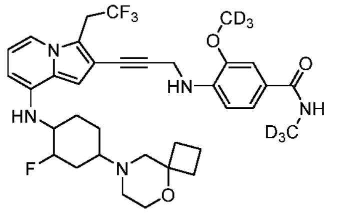
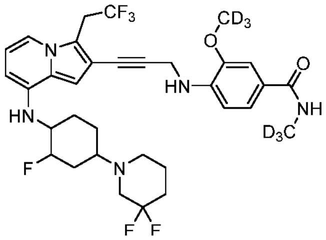

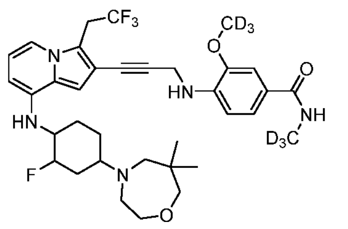
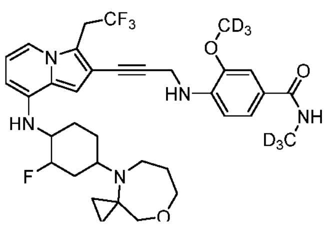
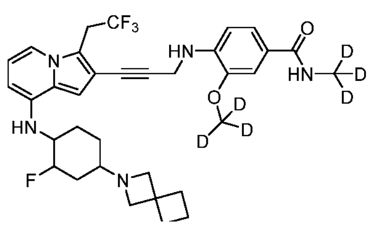

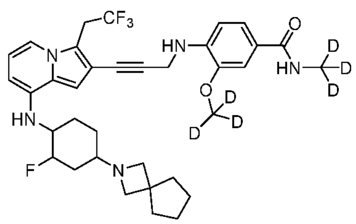
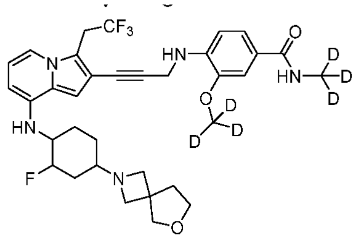
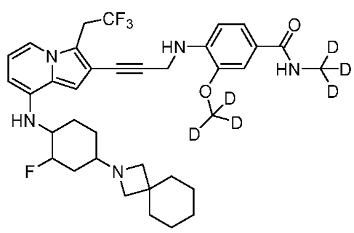

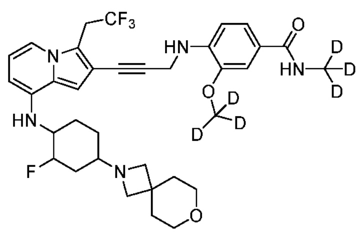
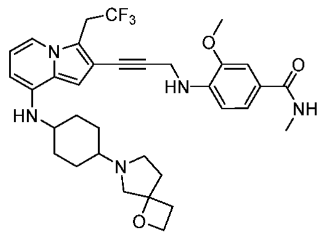
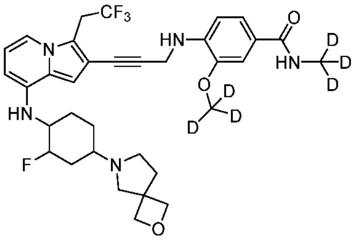

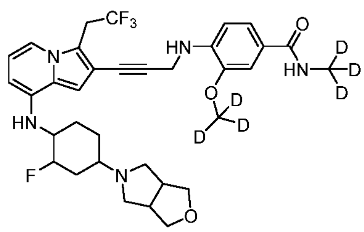
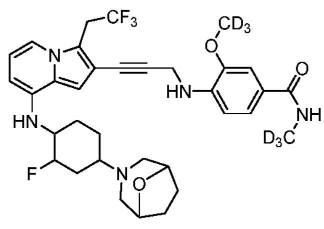
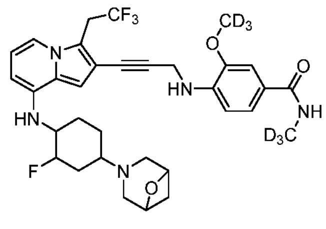

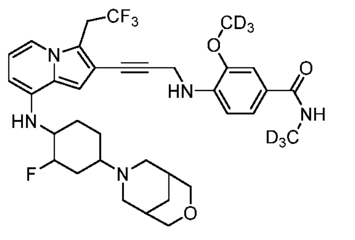
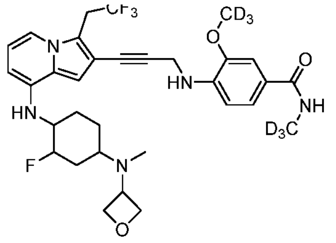
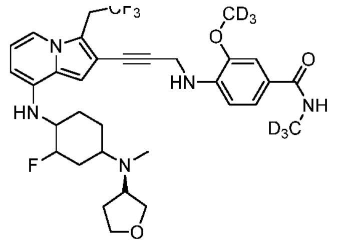

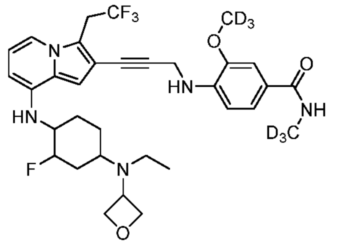
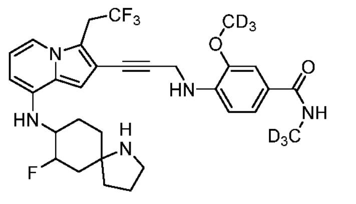
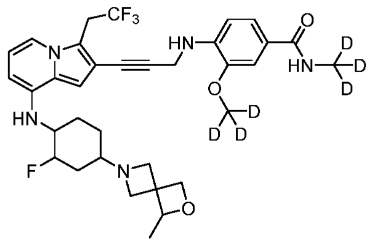

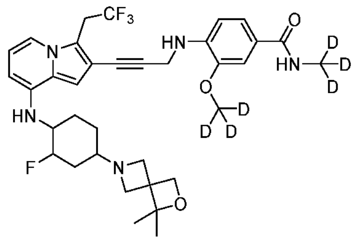
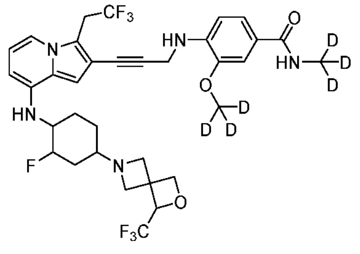
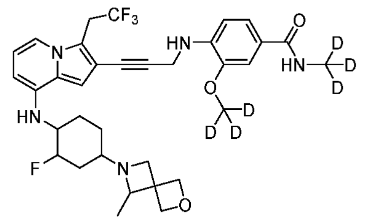

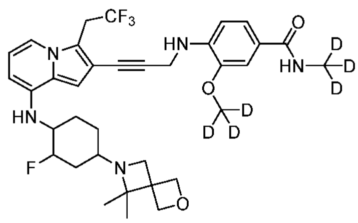
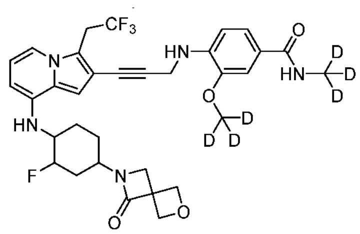
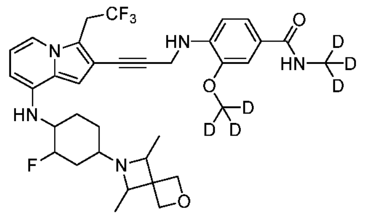

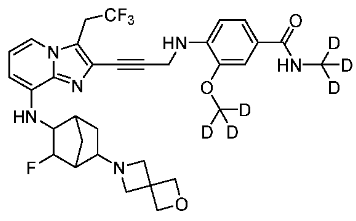
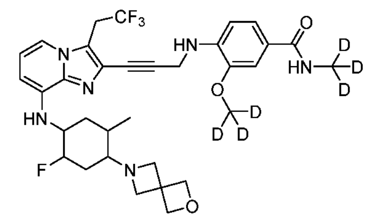
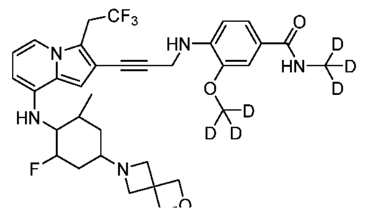

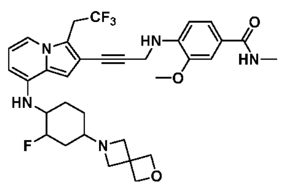
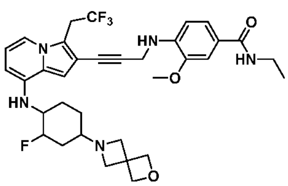
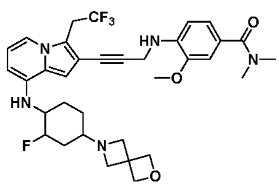
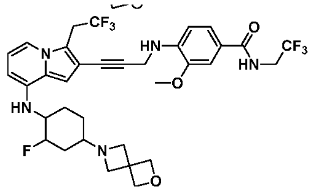

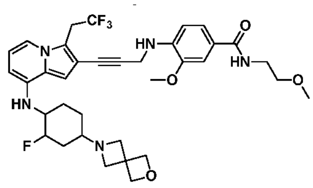
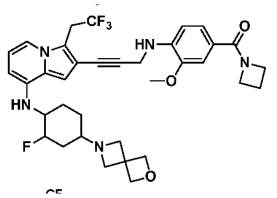
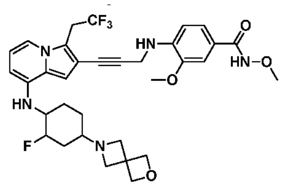
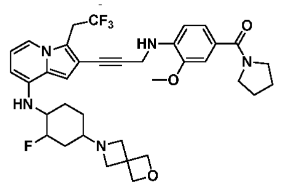

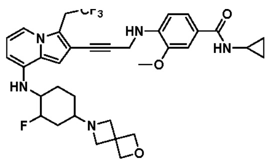
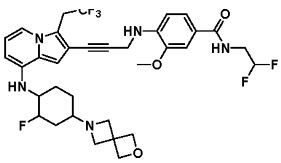
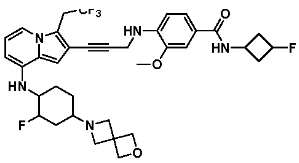
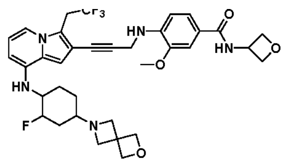

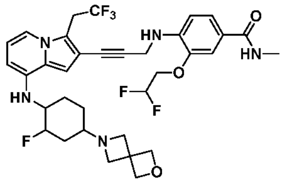
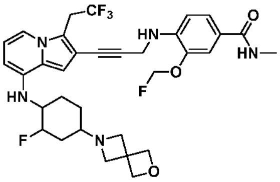
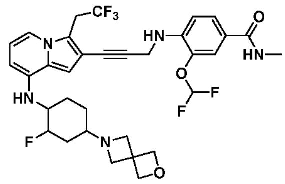
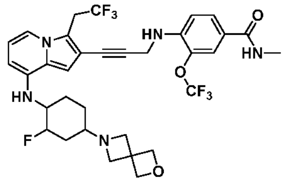

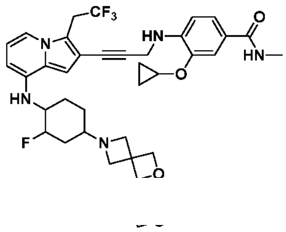
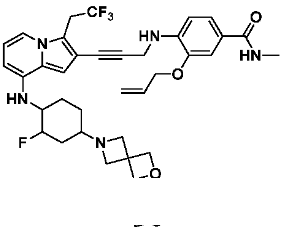
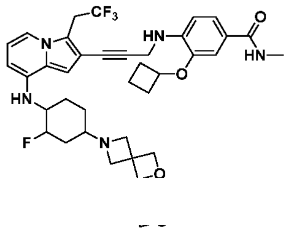
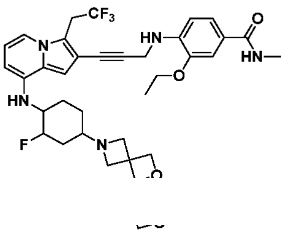

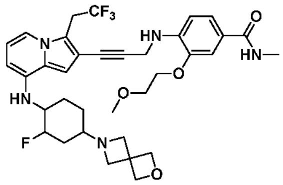
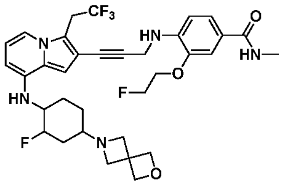
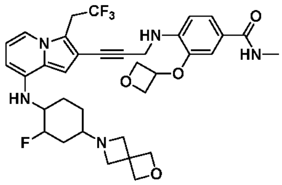
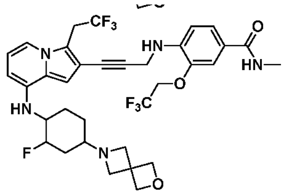

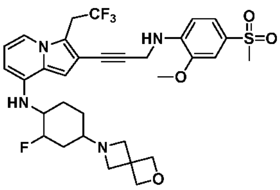
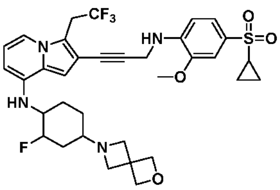
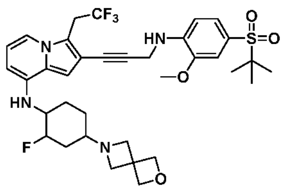

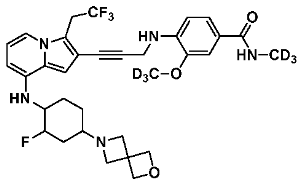
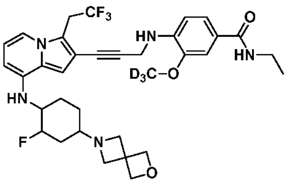
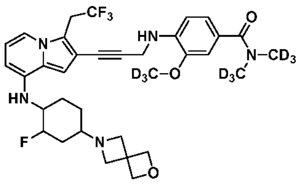
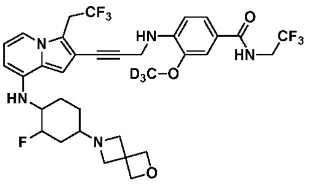

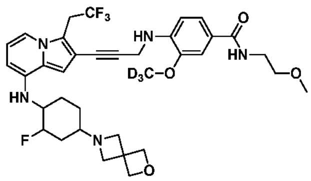
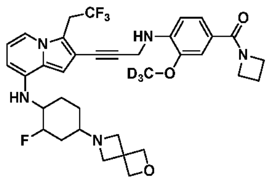
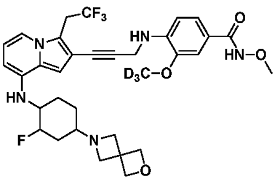
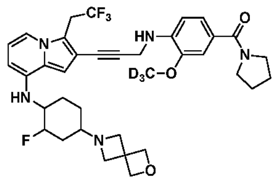

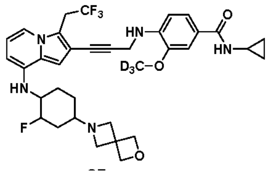
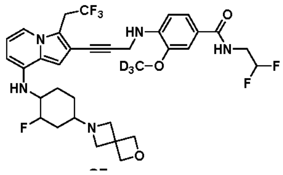
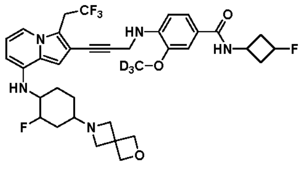
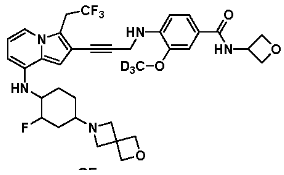

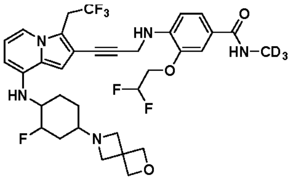
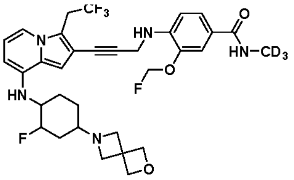
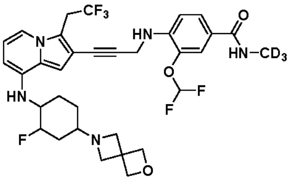
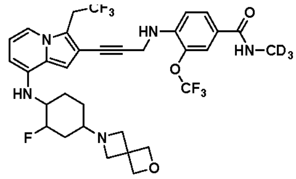

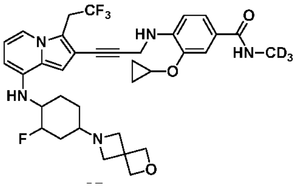
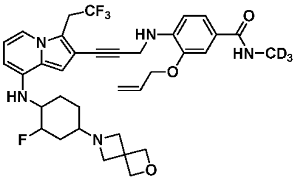
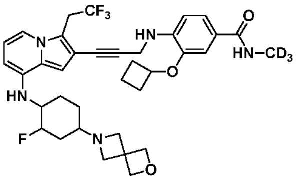
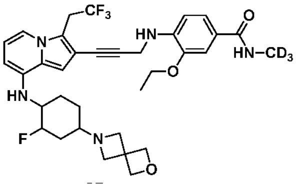

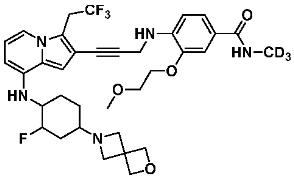
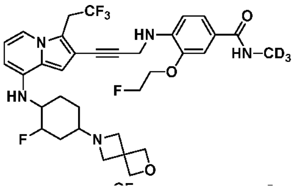
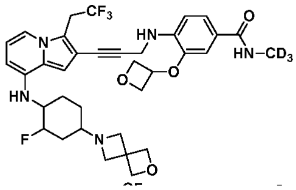
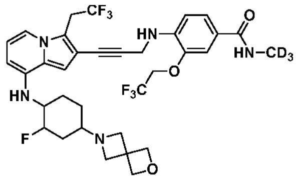

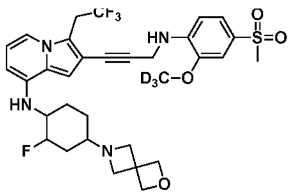
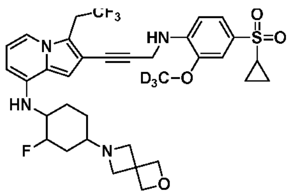
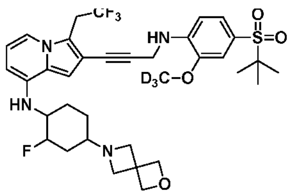

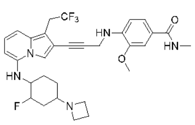
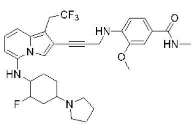
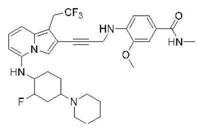
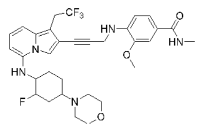
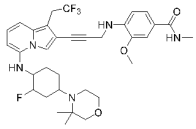

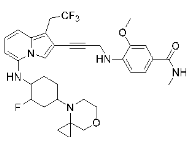
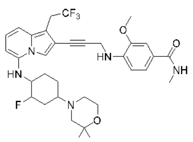
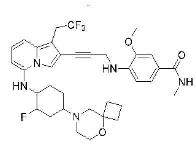
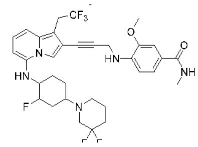
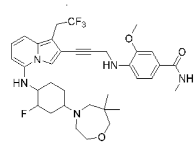

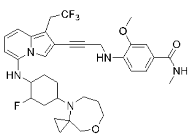
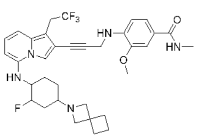
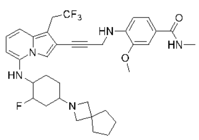
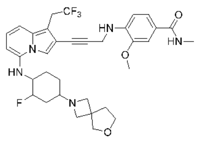
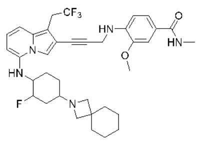

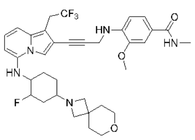
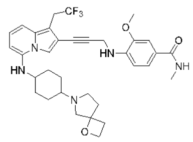
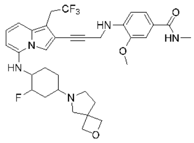
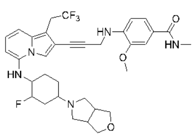
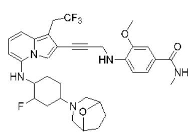

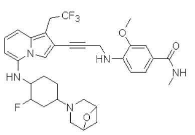
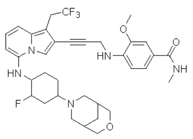
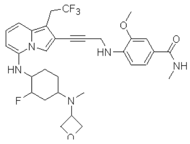
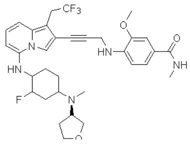
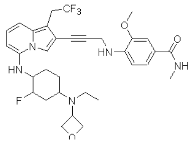

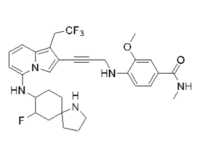 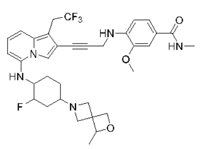 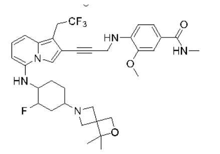 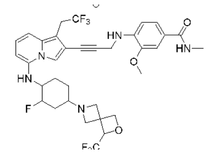 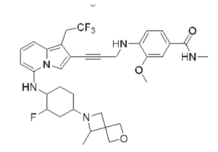

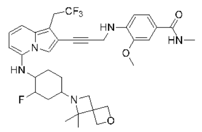 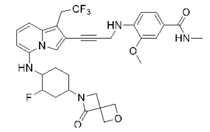 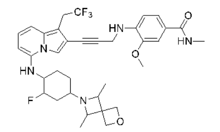 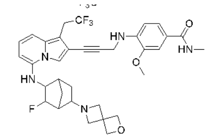 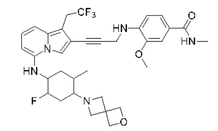

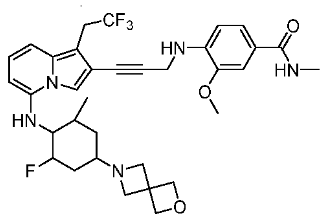

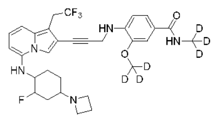 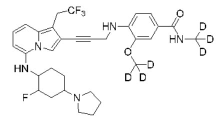 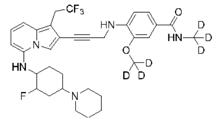 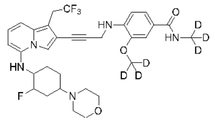 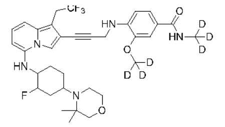

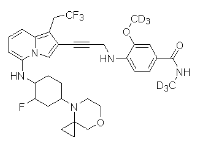 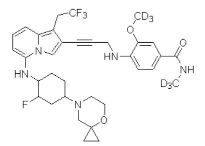 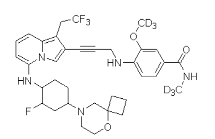 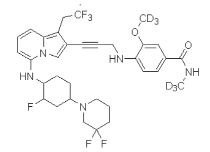 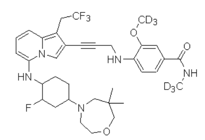

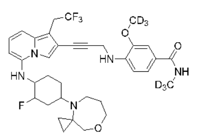 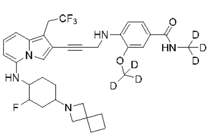 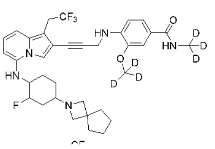 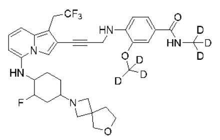 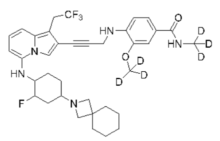

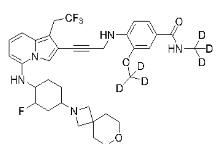 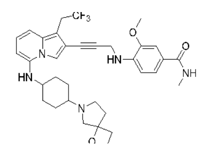 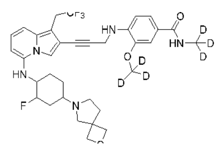 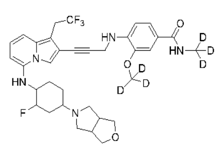 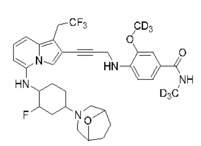

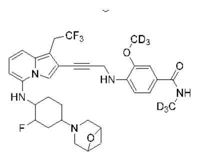 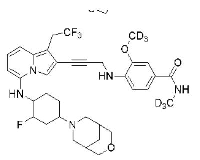 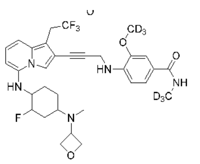 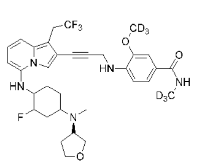 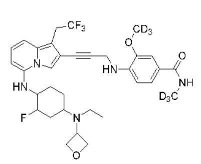

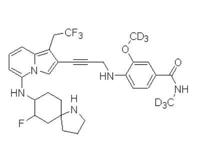 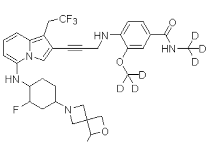 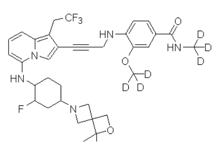 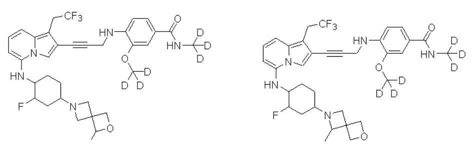

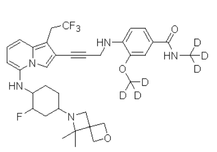
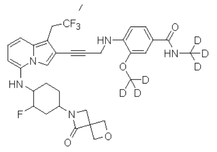
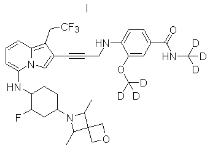
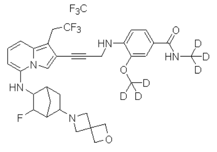
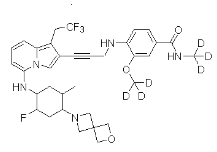

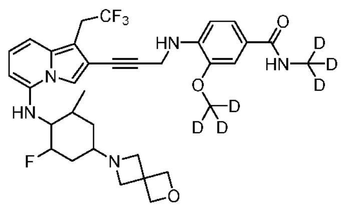

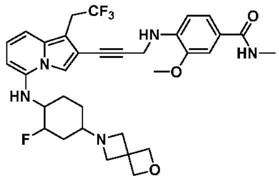
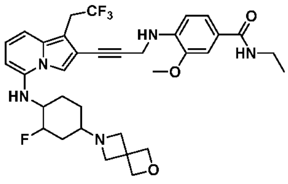
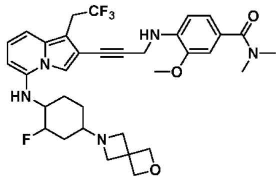
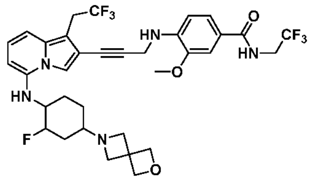

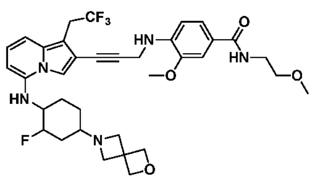
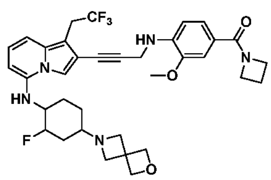
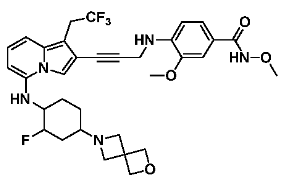
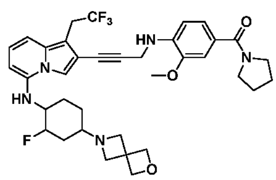

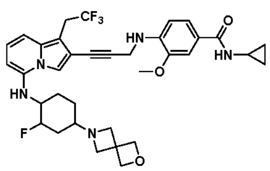
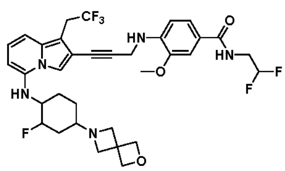
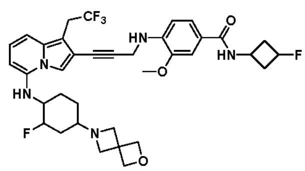
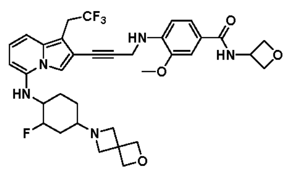

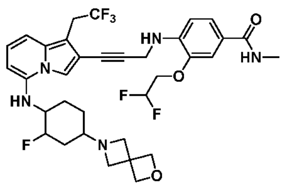
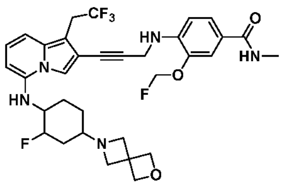
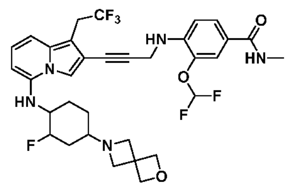
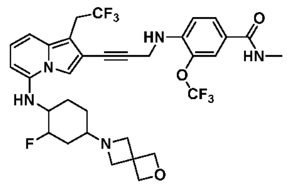

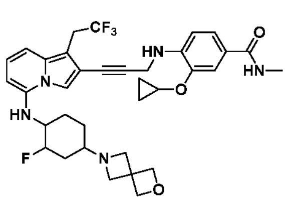
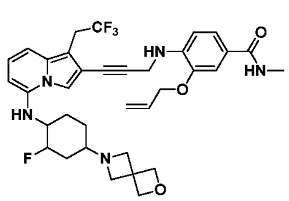
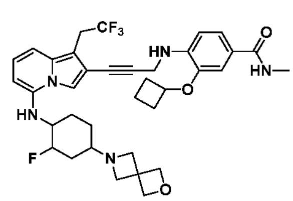
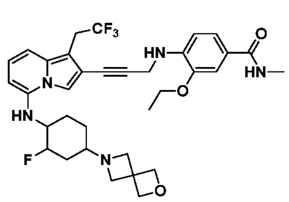

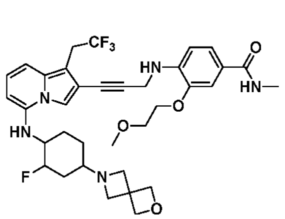
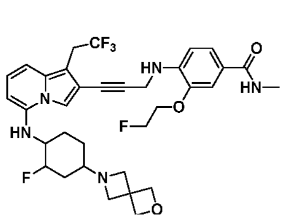
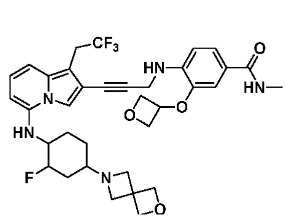
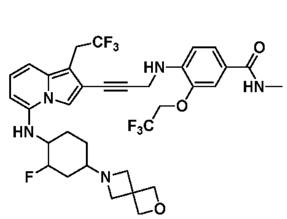

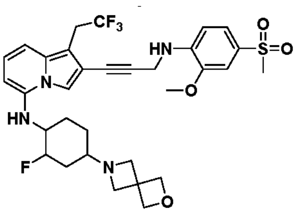
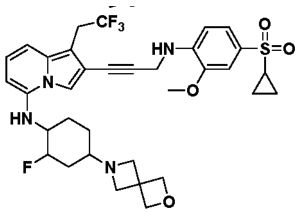
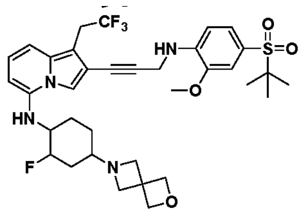

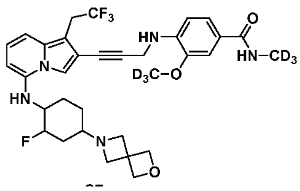
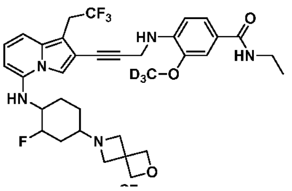
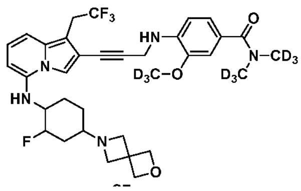
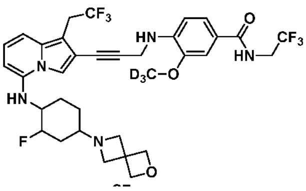

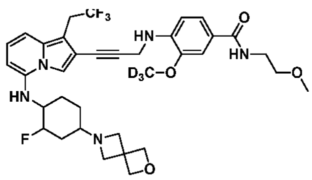
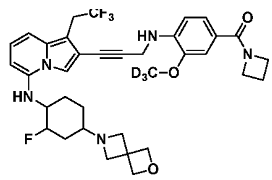
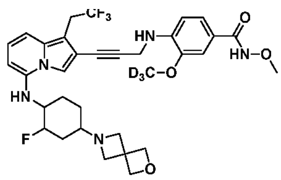
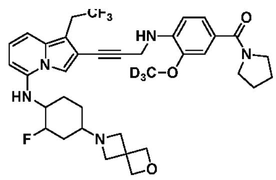

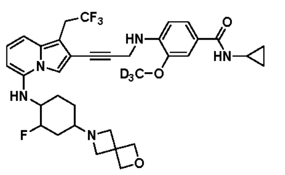
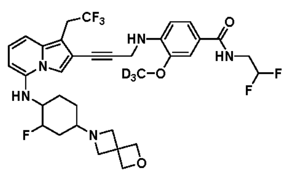
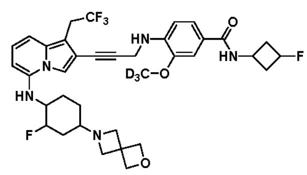
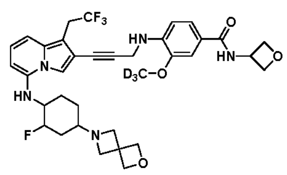

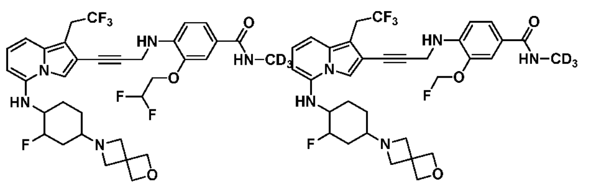
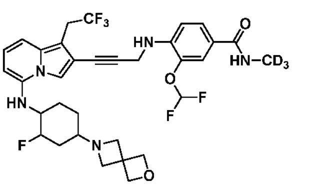
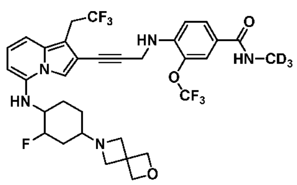

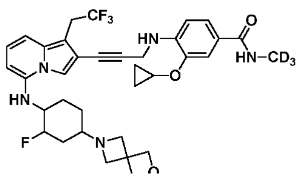
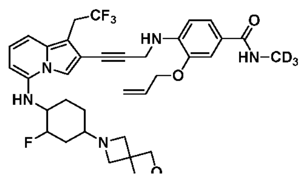
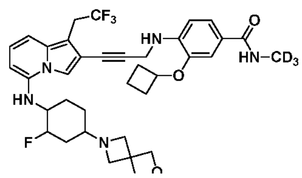
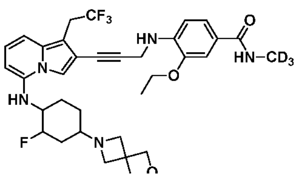

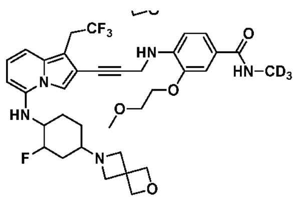
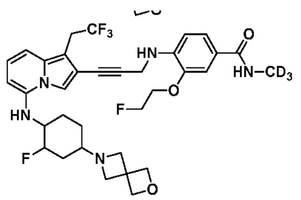
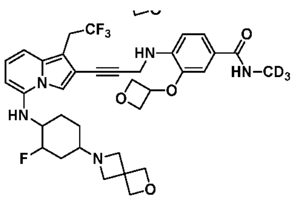
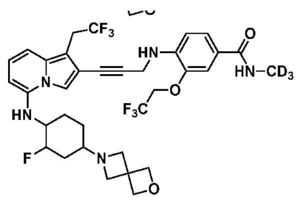

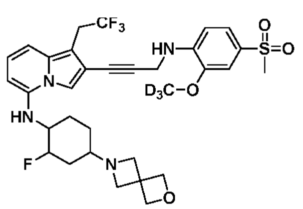
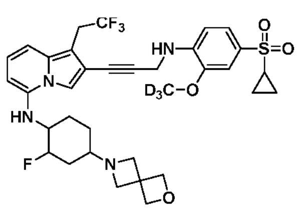
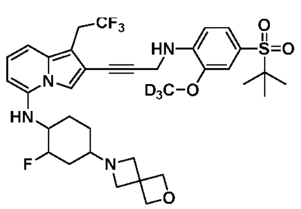

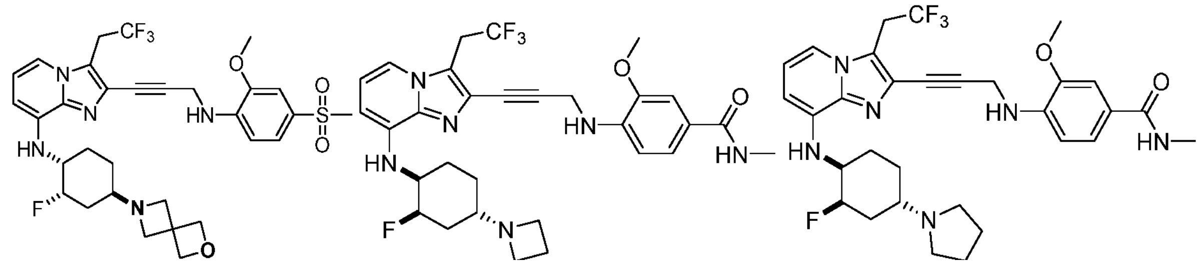

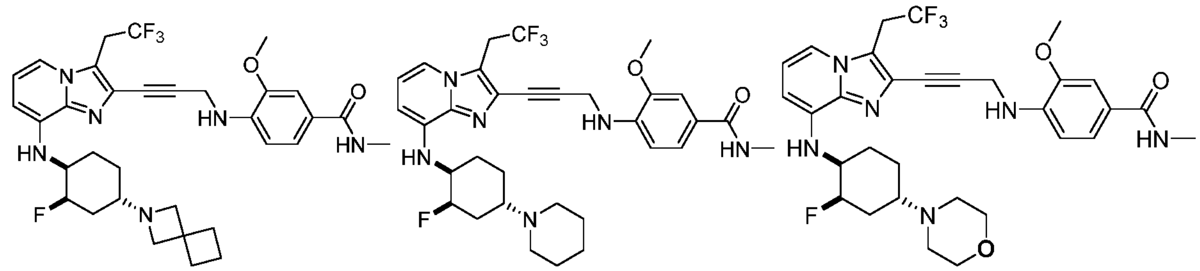

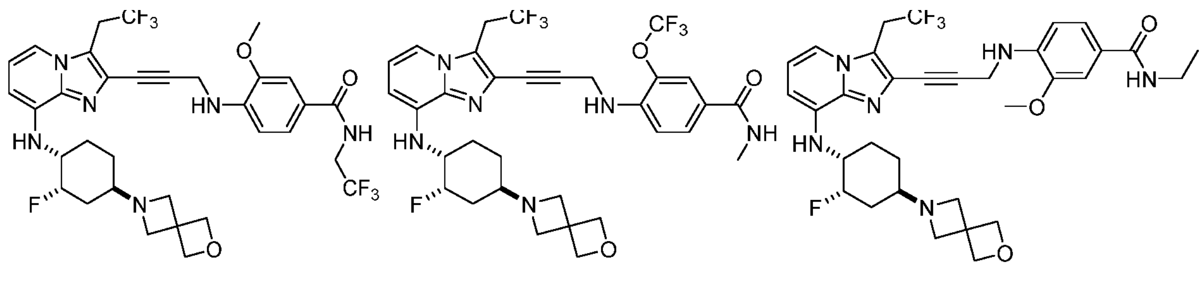

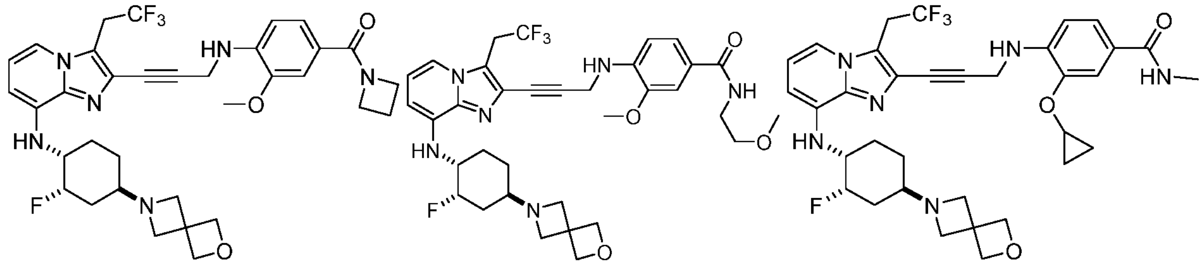

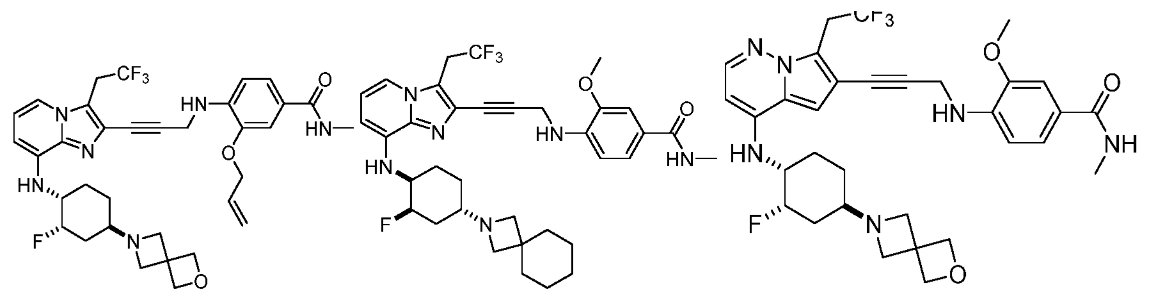

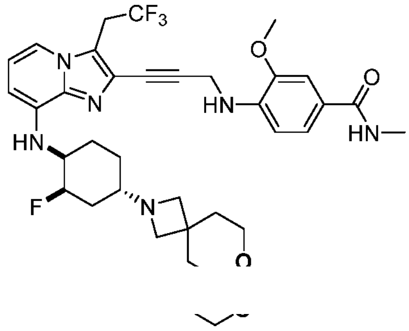
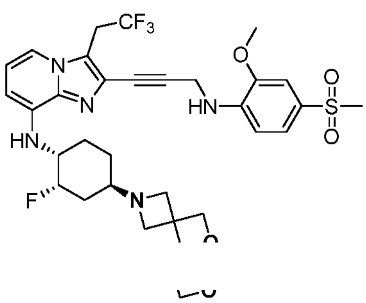
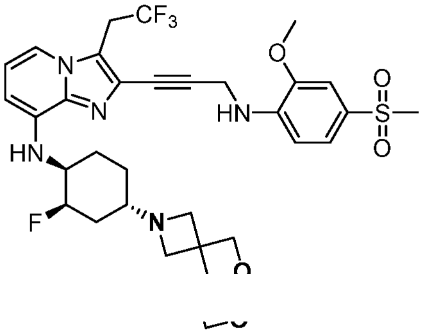

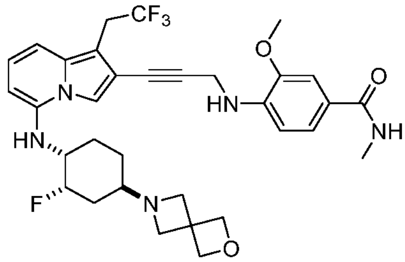
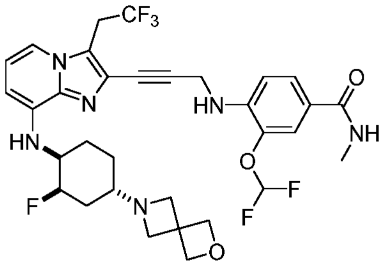
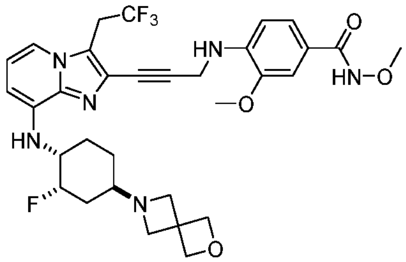

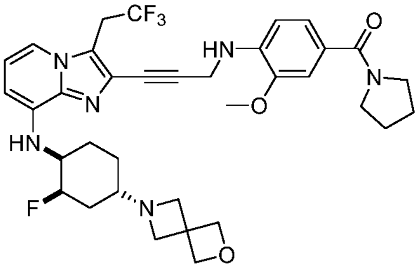
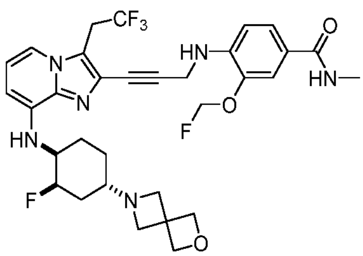
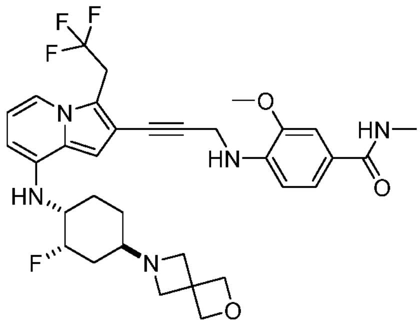

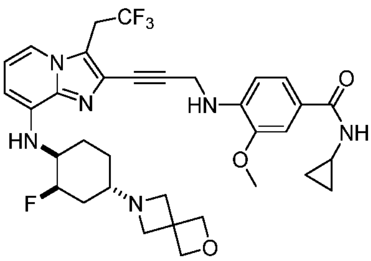
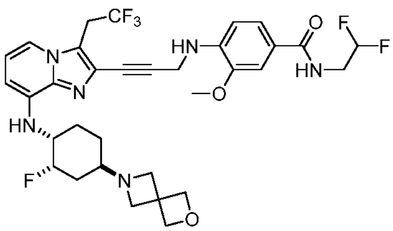
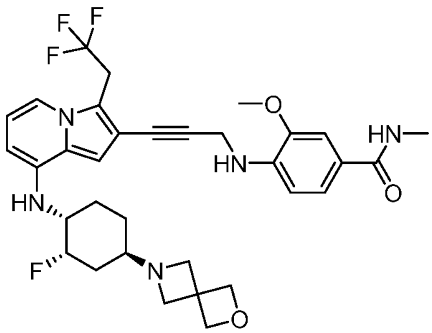

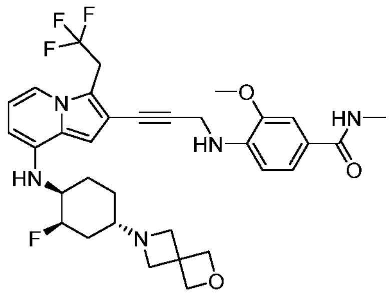
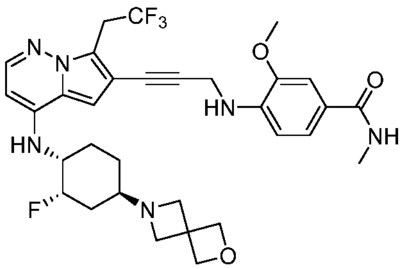
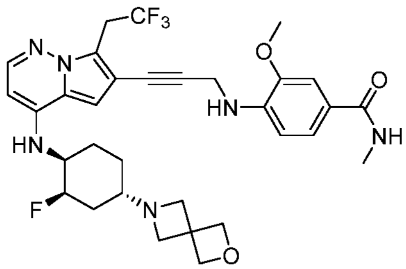

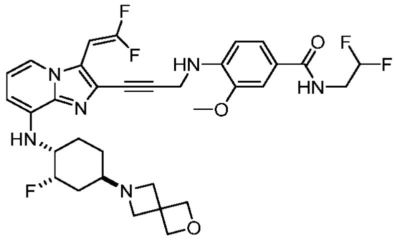

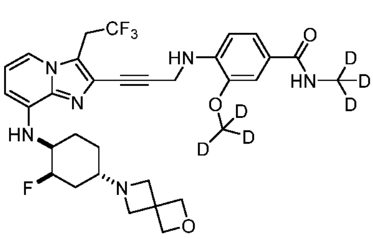

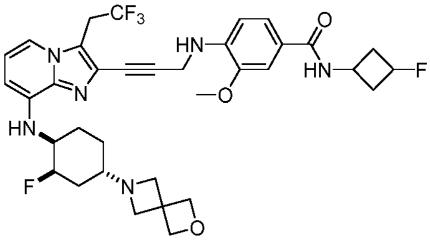

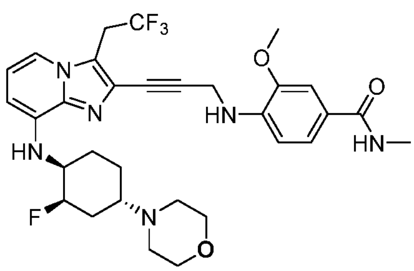

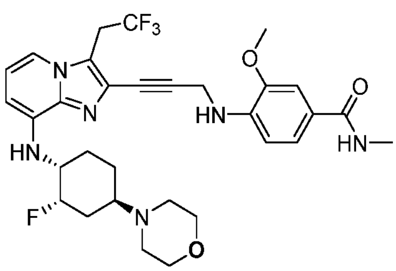

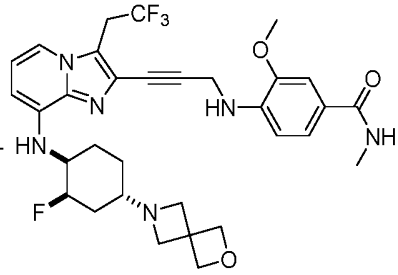

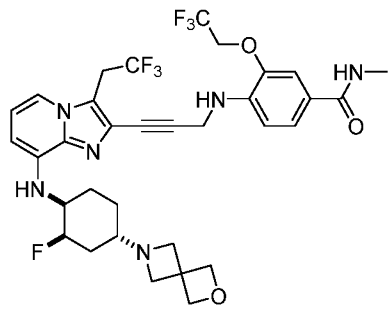

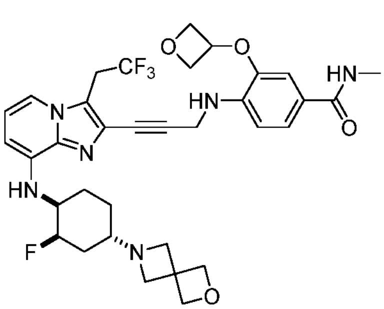

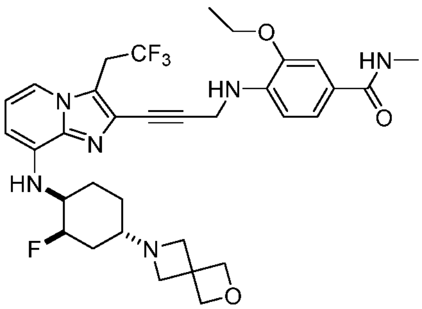

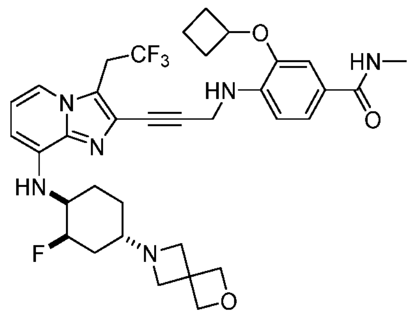

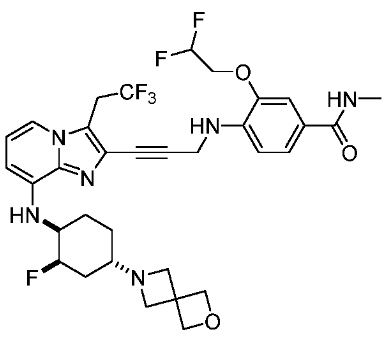

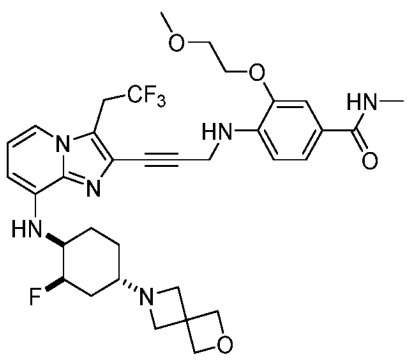

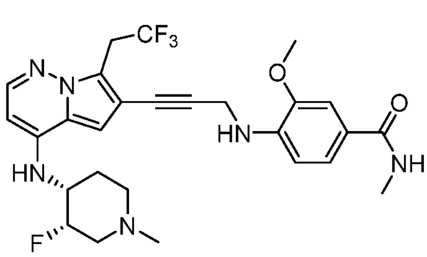

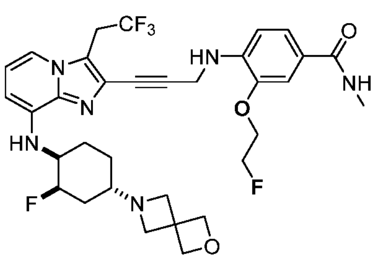

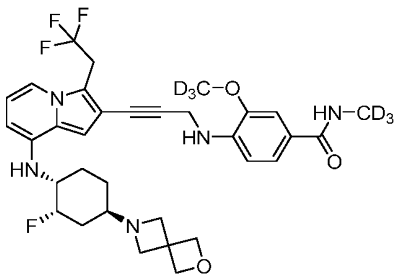

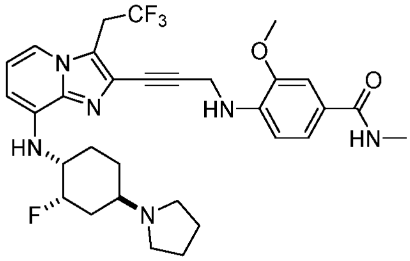
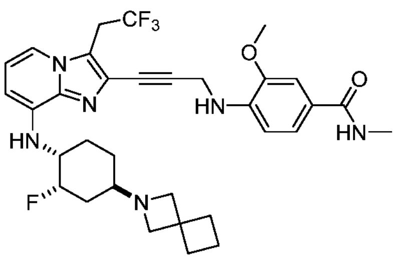
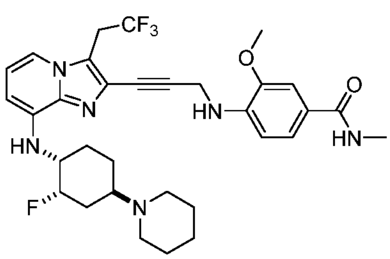

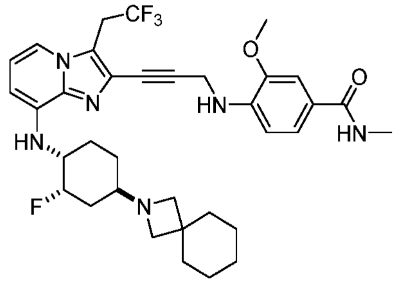
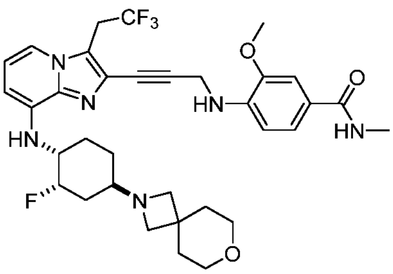
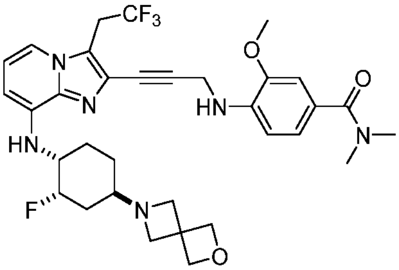

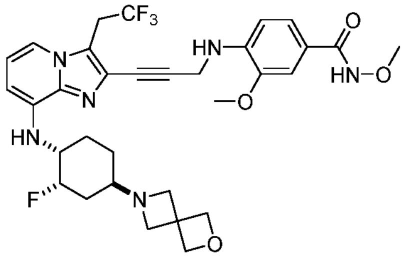
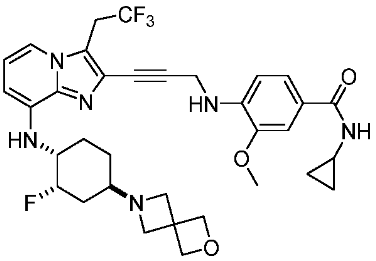
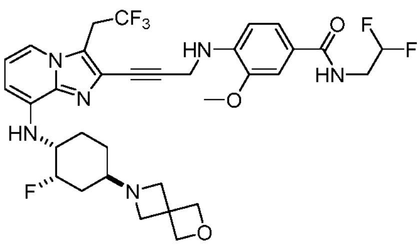

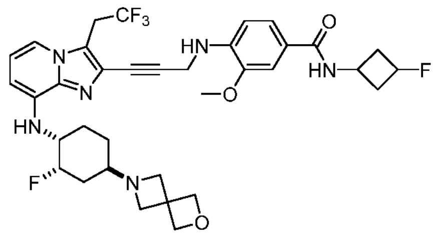
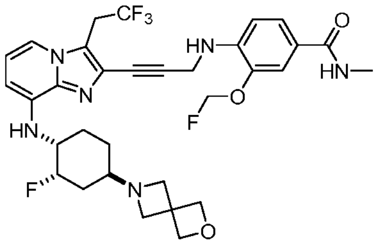
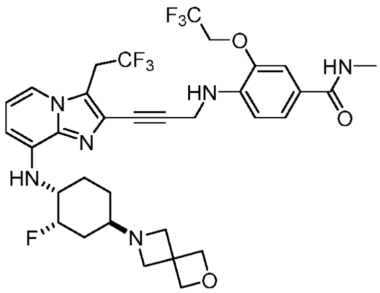

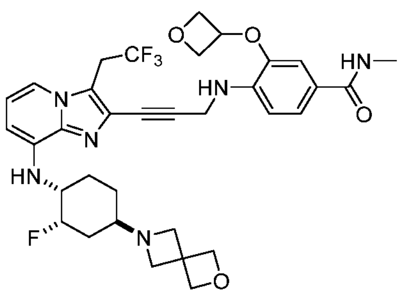
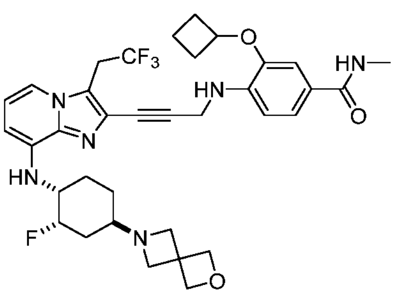
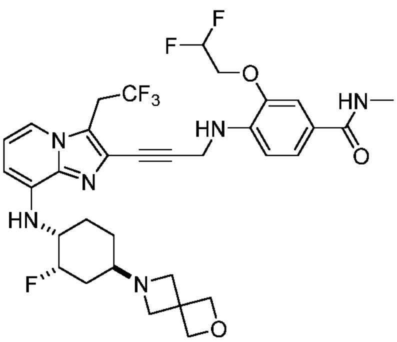

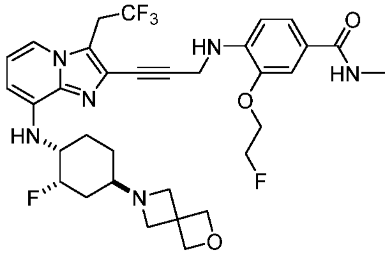 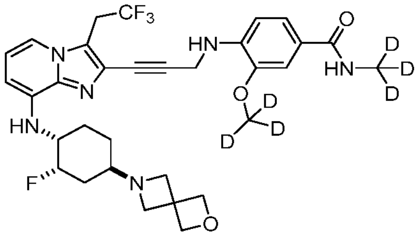 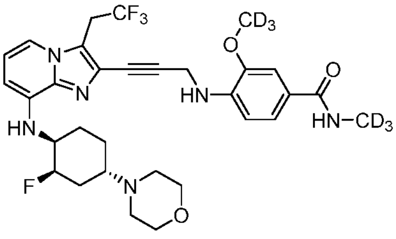

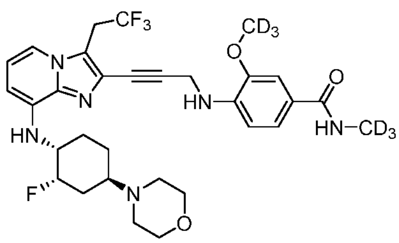 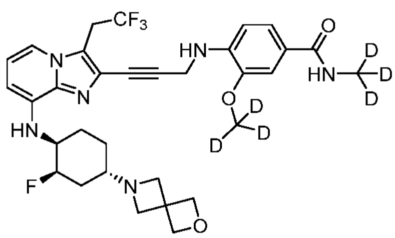 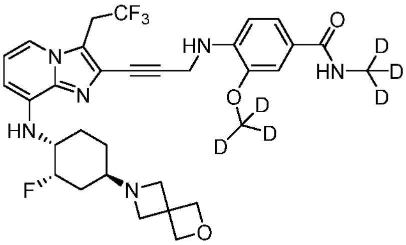

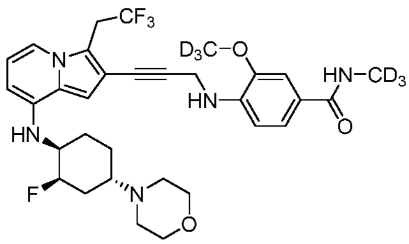 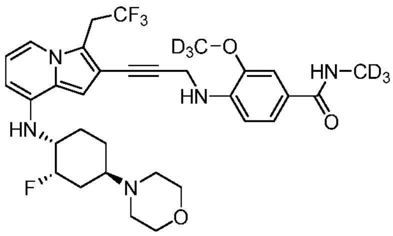 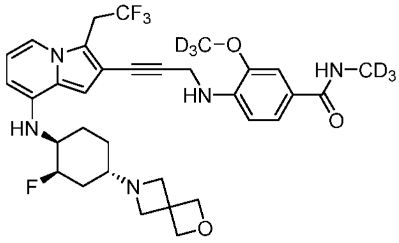

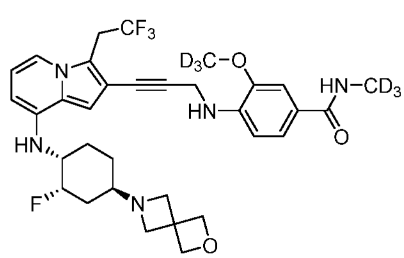 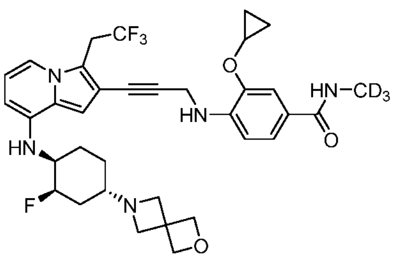 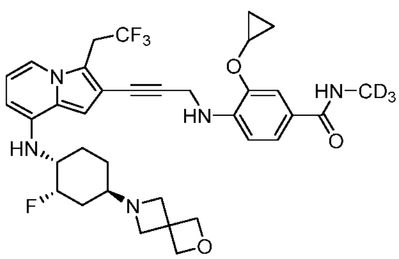

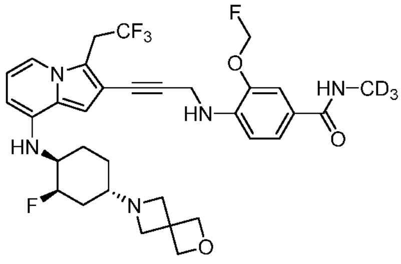 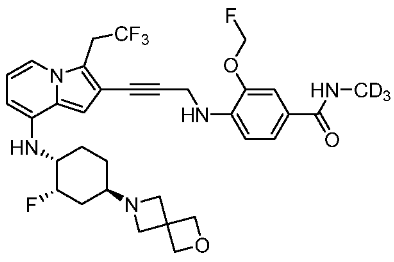 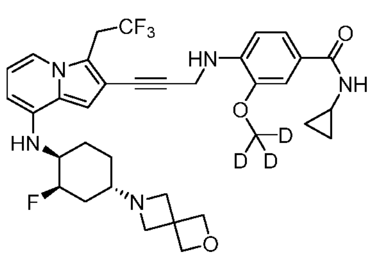

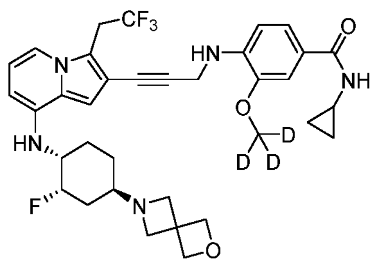 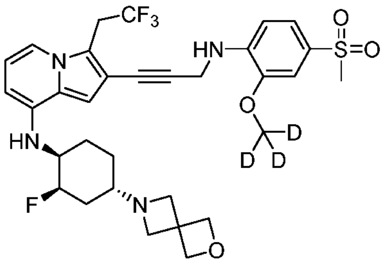 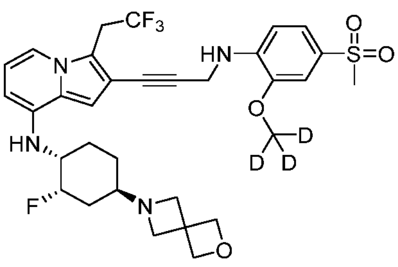

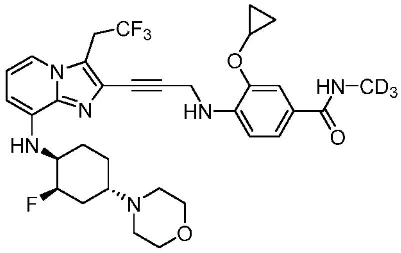 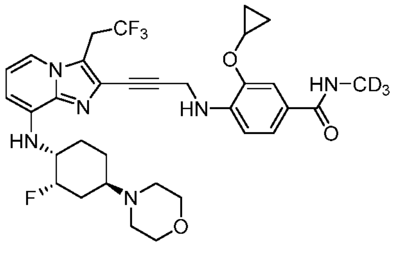 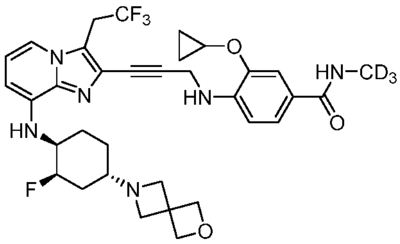

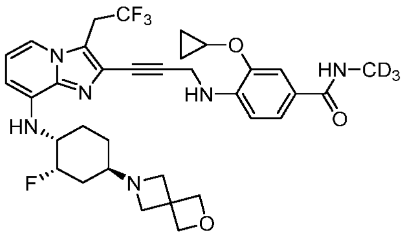 .

**21.** A pharmaceutical composition, which comprises the compound, or enantiomer, diastereomer, racemate, tautomer, stereoisomer, geometric isomer, crystalline form, N-oxide, metabolite thereof, or pharmaceutically acceptable salt, ester, solvate, hydrate, isotope-labeled compound (preferably deuterated compound) or prodrug thereof according to any one of claims 1 to 20, and optional a pharmaceutically acceptable excipient.

**22.** A combination drug, which comprises:

1) a first drug, in which the first drug is the compound, or enantiomer, diastereomer, racemate, tautomer, stereoisomer, geometric isomer, crystalline form, N-oxide, metabolite thereof, or pharmaceutically acceptable salt, ester, solvate, hydrate, isotope-labeled compound (preferably deuterated compound) or prodrug thereof according to any one of claims 1 to 20;
2) a second drug, in which the second drug is a PD-1 inhibitor or a PD-L1 inhibitor; preferably, the PD-1 inhibitor is selected from the group consisting of nivolumab, pembrolizumab, cemiplimab, tislelizumab, camrelizumab, sintilimab, toripalimab, penpulimab, zimberelimab, and pucotenlimab;

preferably, the PD-L1 inhibitor is selected from the group consisting of atezolizumab, avelumab, and durvalumab.

**23.** Use of the compound, or enantiomer, diastereomer, racemate, tautomer, stereoisomer, geometric isomer, crystalline form, N-oxide, metabolite thereof, or pharmaceutically acceptable salt, ester, solvate, hydrate, isotope-labeled compound (preferably deuterated compound) or prodrug thereof according to any one of claims 1 to 20, or the pharmaceutical composition according to claim 21, or the combination drug according to claim 22, in the manufacture of a medicament;

preferably, the medicament is used for the treatment and/or prevention of a disease;
preferably, the disease is a diseases caused by p53 Y220C mutation;
preferably, the disease is cancer;
preferably, the cancer is selected from the group consisting of gastric adenocarcinoma, pancreatic cancer, breast cancer, liver cancer, prostate cancer, cervical cancer, ovarian cancer, oral cancer, esophageal cancer, gastric cancer, colorectal cancer, nasopharyngeal cancer, lung cancer (e.g., non-small cell lung cancer, small cell lung cancer), bladder cancer, soft tissue sarcoma, brain tumor, lymphocyte tumor, osteogenic sarcoma, endometrial cancer, and head and neck cancer;
more preferably, the disease is selected from the group consisting of gastric adenocarcinoma, pancreatic cancer, prostate cancer, ovarian cancer, breast cancer, endometrial cancer, head and neck cancer, and small cell lung cancer.

## INTERNATIONAL SEARCH REPORT

International application No.
**PCT/CN2023/108762**

**A. CLASSIFICATION OF SUBJECT MATTER**

C07D 487/04(2006.01)i；C07D209/00(2006.01)i；C07D235/00(2006.01)i；A61K31/404(2006.01)i；A61K31/454(2006.01)i；A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS, CNTXT, ENTXTC, WPABS, WPABSC, VCN, CAPLUS(STN), REGISTRY(STN): 癌症, 深圳众格, 肿瘤, 突变体, 胃腺癌, 胰腺癌, 牟剑锋, 牛春意, 方小牛, 万晓, 董广平, 俞清方, 王绍晖, 陈斌, 张佩宇, p53, Y220C, cancer, tumor, mutants, gastric adenocarcinoma, pancreatic carcinoma, 结构式检索, search for structural formula

**C. DOCUMENTS CONSIDERED TO BE RELEVANT**

| Category* | Citation of document, with indication, where appropriate, of the relevant passages | Relevant to claim No. |
|---|---|---|
| PX | WO 2022213975 A1 (JACOBIO PHARMACEUTICALS CO., LTD.) 13 October 2022 (2022-10-13) description, table A, embodiment, section A, and page 3, paragraph 5 to page 6, paragraph 1, and claims 1-23 | 16, 20-23 |
| X | CN 109069481 A (PMV PHARMACEUTICALS INC.) 21 December 2018 (2018-12-21) description, paragraphs 0034-0055 and 0076-0088, and embodiments | 16, 20-23 |
| X | WO 2021061643 A1 (PMV PHARMACEUTICALS INC.) 01 April 2021 (2021-04-01) description, paragraphs 005-097, and table 1 | 16, 20-23 |
| X | WO 2021262483 A1 (PMV PHARMACEUTICALS INC.) 30 December 2021 (2021-12-30) description, paragraphs 0102-0176, and table 1, and claims 1-40 | 16, 20-23 |
| X | WO 2021262484 A1 (PMV PHARMACEUTICALS INC.) 30 December 2021 (2021-12-30) description, paragraphs 045 and 048-0107, and table 1, and claims 1-43 | 16, 20-22 |
| X | WO 2021262541 A1 (PMV PHARMACEUTICALS INC.) 30 December 2021 (2021-12-30) description, paragraphs 016-022, and tables 1-3, and claims 1-44 | 16, 20-23 |

☐ Further documents are listed in the continuation of Box C.　　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 October 2023** | **08 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/108762**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-15, 16 (in part), 17-19, 20-23 (in part)**
because they relate to parts of the international application that do not comply with the prescribed requirements to such an extent that no meaningful international search can be carried out, specifically:

General formula M involved in claim 1 comprises a plurality of substitution variables, and layer-by-layer substitution occurs in each substitution variable, so that it is difficult for the examiner to carry out an exhaustive search of the prior art with regard to the current scope of the claim. Meanwhile, the layer-by-layer substitution of a substituent in formula M causes said claim to cover a relatively large scope of protection; however, the description of the present application only verifies the pharmacological activity of specific compounds having limited substitution situations. According to the content disclosed in the present application, a person skilled in the art would not have been able to determine the technical problem of the present application to be solved by claim 1, as a whole technical solution. Therefore, claim 1 is not supported by the description. By the same reasoning, claims 2-15, 16 (in part), 17-19 and 20-23 (in part) also have the same defect. The present search report is provided within the following range: formulae MI-1-1-2, MI-1-1-2A and MI-1-1-2B of claim 16, wherein a B ring is selected from phenyl and 5-membered heteroaryl, and the definitions of other substituents are the same as those of claim 1; and specific compounds of claim 20 falling within the scope of claim 16 and the technical solutions of claims 21-23, when referring to claims 16 and 20.

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/108762**

| Patent document cited in search report | | | Publication date (day/month/year) | Patent family member(s) | | | Publication date (day/month/year) |
|---|---|---|---|---|---|---|---|
| WO | 2022213975 | A1 | 13 October 2022 | None | | | |
| CN | 109069481 | A | 21 December 2018 | AU | 2017221472 | A1 | 26 July 2018 |
| | | | | AU | 2017221472 | B2 | 05 January 2023 |
| | | | | KR | 20180138200 | A | 28 December 2018 |
| | | | | JP | 2019512465 | A | 16 May 2019 |
| | | | | JP | 6956098 | B2 | 27 October 2021 |
| | | | | US | 2021002252 | A1 | 07 January 2021 |
| | | | | US | 11339141 | B2 | 24 May 2022 |
| | | | | WO | 2017143291 | A1 | 24 August 2017 |
| | | | | IL | 261175 | A | 31 October 2018 |
| | | | | IL | 261175 | B | 31 October 2021 |
| | | | | IL | 286839 | A | 31 October 2021 |
| | | | | IL | 286839 | B | 01 August 2022 |
| | | | | MX | 2018009947 | A | 21 January 2019 |
| | | | | BR | 112018016890 | A2 | 12 February 2019 |
| | | | | US | 2019119249 | A1 | 25 April 2019 |
| | | | | US | 10640485 | B2 | 05 May 2020 |
| | | | | US | 2017240525 | A1 | 24 August 2017 |
| | | | | US | 10138219 | B2 | 27 November 2018 |
| | | | | US | 2022213062 | A1 | 07 July 2022 |
| | | | | EP | 3416638 | A1 | 26 December 2018 |
| | | | | EP | 3416638 | A4 | 17 July 2019 |
| | | | | CA | 3010847 | A1 | 24 August 2017 |
| WO | 2021061643 | A1 | 01 April 2021 | AU | 2020352516 | A1 | 14 April 2022 |
| | | | | CA | 3152160 | A1 | 01 April 2021 |
| | | | | MX | 2022003456 | A | 02 June 2022 |
| | | | | IL | 291593 | A | 01 May 2022 |
| | | | | EP | 4034104 | A1 | 03 August 2022 |
| | | | | EP | 4034104 | A4 | 13 September 2023 |
| | | | | KR | 20220070255 | A | 30 May 2022 |
| | | | | JP | 2022549278 | A | 24 November 2022 |
| | | | | US | 2022315564 | A1 | 06 October 2022 |
| | | | | BR | 112022005460 | A2 | 16 August 2022 |
| WO | 2021262483 | A1 | 30 December 2021 | US | 2023049952 | A1 | 16 February 2023 |
| WO | 2021262484 | A1 | 30 December 2021 | KR | 20230028484 | A | 28 February 2023 |
| | | | | AU | 2021297716 | A1 | 19 January 2023 |
| | | | | IL | 298592 | A | 01 January 2023 |
| | | | | US | 2023033324 | A1 | 02 February 2023 |
| | | | | EP | 4171548 | A1 | 03 May 2023 |
| | | | | CA | 3183081 | A1 | 30 December 2021 |
| | | | | JP | 2023533447 | A | 03 August 2023 |
| WO | 2021262541 | A1 | 30 December 2021 | US | 2023044826 | A1 | 09 February 2023 |
| | | | | KR | 20230028371 | A | 28 February 2023 |
| | | | | JP | 2023533446 | A | 03 August 2023 |
| | | | | CA | 3183025 | A1 | 30 December 2021 |
| | | | | EP | 4172143 | A1 | 03 May 2023 |
| | | | | AU | 2021296140 | A1 | 05 January 2023 |
| | | | | IL | 298549 | A | 01 January 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210866557
- CN 202310257457X


### Non-patent literature cited in the description


- Handbook of Chemistry and Physics. 1994
- **THOMAS SORRELL**. Organic Chemistry. University Science Books, 1999
- **MICHAEL B. SMITH** ; **JERRY MARCH**. March's Advanced Organic Chemistry. John Wiley & Sons, 2007
- Burger's Medicinal Chemistry and Drug Discovery. 1995, 172-178, 949-982
- McGraw-Hill Dictionary of Chemical Terms. McGraw-Hill, 1984
- **ELIEL, E.** ; **WILEN, S**. Stereochemistry of Organic Compounds. John Wiley & Sons, 1994
- **JACQUES et al.** Enantiomers, Racemates and Resolutions. Wiley Interscience, 1981
- **ROBERT E. GAWLEY** ; **JEFFREY AUBE**. Principles of Asymmetric Synthesis. Elsevier, 2012
- **ELIEL, E. L.** Stereochemistry of Carbon Compounds. McGraw-Hill, 1962
- **WILEN, S. H.** Tables of Resolving Agents and Optical Resolutions. Univ. of Notre Dame Press, 1972, 268
- Chiral Separation Techniques: A Practical Approach. Wiley-VCH Verlag GmbH & Co. KGaA, 2007
- **JERRY MARCH**. Advanced Organic Chemistry. Wiley Interscience
- **L.W. DEADY**. *Syn. Comm.*, 1977, vol. 7, 509-514